# EUROPÄISCHE PATENTSCHRIFT

(11) **EP 0 521 827 B1**
(45) Veröffentlichungstag und Bekanntmachung des Hinweises auf die Patenterteilung: **25.09.1996**
(21) Anmeldenummer: 92810490.0
(22) Anmeldetag: 25.06.1992
(51) Int. Cl.: C07C 243/12, C07C 243/24, C07C 243/34, C07C 271/12, C07C 281/02, C07D 295/182, C07D 213/56, C07D 215/48, A61K 31/175, A61K 31/395, C07D 257/04

(54) **Pharmakologisch wirksame Hydrazinderivate und Verfahren zu deren Herstellung**
Pharmacological active hydrazin derivatives and process for their preparation
Dérivés d'hydrazine pharmacologiquement actives et procédé pour leur préparation

(30) Priorität: 03.07.1991 CH 1962/91
(43) Veröffentlichungstag der Anmeldung: 07.01.1993
(73) Patentinhaber: CIBA-GEIGY AG, 4002 Basel (CH)
(72) Erfinder: Fässler, Alexander, Dr., CH-4104 Oberwil (CH); Bold, Guido, Dr., CH-5264 Gipf-Oberfrick (CH); Lang, Marc, Dr., F-68200 Mülhausen (FR); Schneider, Peter, Dr., CH-4103 Bottmingen (CH)

(56) Entgegenhaltungen:
- EP-A- 0 402 646
- DE-A- 1 254 461
- CHEM. BER. Band 108, 1975, Seiten 813- 819; W. STREICHER et al.: "Synthese eines Azaanalogen des Antibiotikums Negamycin"

## Beschreibung

Die Erfindung betrifft eine neue Klasse von nicht hydrolysierbaren Analoga für durch Aspartatproteasen spaltbare Peptide, nämlich Hydrazinderivate, Verfahren zu ihrer Herstellung, pharmazeutische Präparate, die diese Peptidanaloga enthalten, und deren Verwendung als Arzneimittel oder zur Herstellung pharmazeutischer Präparate zur Bekämpfung viral bedingter Erkrankungen, sowie neue Zwischenprodukte zur Herstellung dieser Verbindungen.

Bei der Immunschwäche AIDS ("Aquired Immunodeficiency Syndrome") handelt es sich um eine Erkrankung mit tödlichem Ausgang. Diese Erkrankung verbreitet sich weltweit in zunehmendem Ausmass vor allem innerhalb gewisser Risikogruppen, aber auch über diese Risikogruppen hinaus. Sie betrifft bereits Millionen von Menschen, und ihre kausale Bekämpfung ist eines der bedeutendsten Ziele für die moderne Medizin. Bisher konnten die Retroviren HIV-1 und HIV-2 (HIV steht für "Human Immunodeficiency Virus") als Ursache für die Erkrankung identifiziert und molekularbiologisch charakterisiert werden. Für die Therapie ist über die bisherigen Möglichkeiten zur Linderung der AIDS-Symptome und gewisse präventive Möglichkeiten hinaus insbesondere die Suche nach Präparaten interessant, welche die Vermehrung des Virus selbst beeinträchtigen, ohne die intakten Zellen und Gewebe der Patienten zu schädigen.

Besonders solche Verbindungen erscheinen als erfolgversprechend, welche die Prozessierung der in menschlichen Zellen biosynthetisierten Proteinbausteine des Virus und damit den korrekten Zusammenbau dieser Bausteine zu kompletten, infektiösen Virionen unterbinden.

HIV-1 und HIV-2 weisen jeweils in ihrem Genom einen Bereich auf, der für eine "gag-Protease" kodiert. Diese "gag-Protease" ist für die korrekte proteolytische Spaltung der Vorläuferproteine verantwortlich, die aus den für die "Group Specific Antigens" (gag) kodierenden Genomabschnitten hervorgehen. Hierbei werden die Struktwproteine des Viruskerns, englisch "Core", freigesetzt. Die "gag-Protease" selbst ist Bestandteil eines durch den pol-Genomabschnitt von HIV-1 und HIV-2 kodierten Vorläuferproteines, das auch die Abschnitte für die "Reverse Transcriptase" und die "Integrase" enthält und vermutlich autoproteolytisch gespalten wird.

Die "gag-Protease" spaltet das Hauptkernprotein ("Major Core Protein") p24 von HIV-1 und HIV-2 bevorzugt N-terminal von Prolinresten, z.B. in den zweiwertigen Radikalen Phe-Pro, Leu-Pro oder Tyr-Pro. Es handelt sich um eine Protease mit einem katalytisch aktiven Aspartatrest im aktiven Zentrum, eine sogenannte Aspartatprotease.

Wenn die Wirkung der "gag-Protease" unterbunden werden könnte, stünden dem Virus zum Zusammenbau des Viruskernes benötigte Proteine nicht mehr zur Verfügung. Dies hätte eine Einschränkung oder gar Unterbindung der Vermehrung des Virus zur Folge. Es besteht somit ein Bedürfnis nach Hemmstoffen für die "gag-Protease" zur Verwendung als antivirale Mittel gegen AIDS und weitere retrovirale Erkrankungen.

Es sind bereits eine Reihe von "gag-Protease"-Hemmern synthetisiert worden, die zentrale Gruppen enthalten, die nicht proteolytisch spaltbare Peptidisostere darstellen. Bisher ist es trotz intensiver Forschung jedoch noch nicht gelungen, zur Anwendung am Menschen geeignete Aspartatproteasehemmer zur Bekämpfung von AIDS bei einem Grossteil der Infizierten zum Einsatz zu bringen. Hierfür sind vor allem pharmakodynamische Probleme ausschlaggebend. Daneben weisen die meisten bisher bekannten "gag-Protease"-Hemmer mehr als zwei asymmetrische Kohlenstoffatome im genannten Zentralbaustein auf, was relativ aufwendige stereospezifische Synthesen oder Isomerentrennungen erforderlich macht. Das Ziel der hier vorliegenden Patentanmeldung ist daher, eine neue Klasse von Hemmstoffen viraler Aspartatproteasen mit einem neuen Zentralbaustein zugänglich zu machen. Die Synthese dieses Zentralbausteines soll darüber hinaus sterisch einfach möglich sein. Ferner weisen die neuen Zentralbausteine an beiden Enden Aminogruppen auf, so dass bei Wahl geeigneter Substituenten beispielsweise Retro-Inversopeptiden analoge Strukturen vorliegen.

Bei den erfindungsgemässen Verbindungen handelt es sich um Verbindungen der Formel 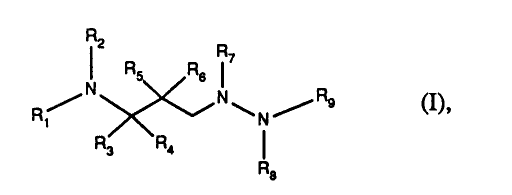 worin R₁ und R₉ unabhängig voneinander Wasserstoff, Acyl, unsubstituiertes oder substituiertes Alkyl, Alkenyl oder Alkinyl; Sulfo, durch unsubstituiertes oder substituiertes Alkyl, Aryl, Heterocyclyl, Alkoxy, das unsubstituiert oder substituiert ist, oder durch Aryloxy substituiertes Sulfonyl; Sulfamoyl, das am Stickstoff unsubstituiert oder substituiert ist; oder durch ein oder zwei unabhängig voneinander aus unsubstituiertem oder substituiertem Alkyl, aus unsubstituiertem oder substituiertem Cycloalkyl, aus Aryl, aus Hydroxy, aus unsubstituiertem oder substituiertem Alkoxy, aus Cycloalkoxy und aus Aryloxy ausgewählte Reste substituiertes Phosphoryl bedeuten, mit der Massgabe, dass höchstens einer der Reste R₁ und R₉ Wasserstoff bedeutet; und R₂ und R₈ jeweils unabhängig voneinander Wasserstoff oder einen der oben für R₁ und R₉ genannten Reste bedeuten; oder die Substituentenpaare R₁ und R₂ oder R₈ und R₉ jeweils unabhängig voneinander gemeinsam mit dem Stickstoffatom, an das sie gebunden sind, einen heterocyclischen Ring, bestehend aus dem bindenden Stickstoff zusammen mit einem aus Ethylen, Trimethylen, Tetramethylen und Pentamethylen ausgewählten Rest, worin ein Kohlenstoffatom durch Stickstoff, Sauerstoff, Schwefel oder mit Sauerstoff ein-oder zweifach substituierten Schwefel ersetzt sein kann und welcher ungesättigt sein kann, oder Naphthalin-1,8-dicarbonylimido bilden können;
R₃ und R₄ unabhängig voneinander Wasserstoff, unsubstituiertes oder substituiertes Alkyl oder Cycloalkyl; Aryl; Heterocyclyl; oder unsubstituiertes oder substituiertes Alkenyl bedeuten; oder R₃ und R₄ gemeinsam unsubstituiertes oder substituiertes Alkylen, Alkyliden oder benzokondensiertes Alkylen bedeuten;
R₅ Hydroxy bedeutet; R₆ Wasserstoff bedeutet;
oder R₅ und R₆ gemeinsam Oxo bedeuten;
und R₇ unsubstituiertes oder substituiertes Alkyl oder Cycloalkyl; Aryl; Heterocyclyl;
oder unsubstituiertes oder substituiertes Alkenyl bedeutet;
sowie die Salze der genannten Verbindungen, sofern salzbildende Gruppen vorliegen; mit Ausnahme der Verbindung der Formel I, worin R₁, R₂, R₃, R₄, R₆ und R₈ jeweils Wasserstoff bedeuten, R₅ Hydroxy bedeutet, R₇ einen Rest der Formel -[CH₂-(C=O)-NH-(N-CH₃)-CH₂-COOH] bedeutet und R₉ tert-Butoxycarbonyl bedeutet.

In der Beschreibung der vorliegenden Erfindung bedeutet der bei der Definition von Gruppen oder Resten, z. B. Niederalkyl, Niederalkoxy, Niederalkanoyl etc., verwendete Ausdruck "Nieder", dass die so definierten Gruppen oder Reste, falls nicht ausdrücklich anders definiert, bis einschliesslich 7 und bevorzugt bis einschliesslich 4 C-Atome enthalten. Sofern nichts anderes angegeben ist, liegen Substituenten innerhalb der Reste R₁, R₂, R₃, R₄, R₇, R₈ and/oder R₉ unabhängig voneinander ein- oder mehrfach, insbesondere ein-bis dreifach, z. B. einfach, vor.

Die durch R₃ und R₄ beziehungsweise durch R₅ und R₆ substituierten Kohlenstoffatome in Verbindungen der Formel I können, sofern sie asymmetrisch sind, ebenso wie gegebenenfalls vorhandene weitere asymmetrische Kohlenstoffatome in der (R)-, (S)- oder (R,S)-Konfiguration vorliegen. Somit können die vorliegenden Verbindungen als Isomeren-gemische oder als reine Isomeren, insbesondere als Diastereomerengemische, Enantiomerenpaare oder reine Enantiomere vorliegen. Bevorzugt sind Verbindungen der Formel I, worin das durch R₃ oder Hydroxy R₅ substituierten Kohlenstoffatom die (S)-Konfiguration aufweisen und weitere gegebenenfalls vorliegende asymmetrischen Kohlenstoffatome unabhängig voneinander in der (R)-, (S)- oder (R,S)-Konfiguration vorliegen.

Die in der Beschreibung der vorliegenden Erfindung verwendeten allgemeinen Ausdrücke und Bezeichnungen haben vorzugsweise die folgenden Bedeutungen, wobei auf den verschiedenen Definitionsebenen von den vor- oder nachstehend aufgeführten Resten beliebige Kombinationen oder Einzelreste anstelle der allgemeinen Definitionen verwendet werden können:

Acyl R₁, R₂, R₈ oder R₉ hat z.B. bis zu 25, vorzugsweise bis zu 19 C-Atome und ist in erster Linie die Acylgruppe einer Carbonsäure, eines Halbesters der Kohlensäure, einer unsubstituierten oder N-substituierten Carbaminsäure, eines unsubstituierten oder N-substituierten Oxalamids oder einer unsubstituierten oder substituierten Aminosäure, wobei anstelle von Carbonylgruppen in den genannten Acylresten jeweils auch Thiocarbonylgruppen möglich sind. Vorzugsweise ist nur höchstens jeweils einer der Reste R₁ und R₂ beziehungsweise der Reste R₈ und R₉ acyliert.

Bevorzugte Acylgruppen R₁, R₂, R₈ und R₉ einer Carbonsäure sind unsubstituiertes oder substituiertes Alkanoyl mit bis zu 19 Kohlenstoffatomen, z. B. n-Decanoyl, oder vorzugsweise
Niederalkanoyl, wie Formyl, Acetyl, Propionyl, Butyryl oder Pivaloyl, oder substituiertes Niederalkanoyl, insbesondere als
Cycloalkylniederalkanoyl, worin Cycloalkyl z. B. 3 - 7 Kohlenstoffatome hat und Niederalkanoyl wie oben definiert ist, z. B. Cycloalkylcarbonyl, insbesondere mit insgesamt 4 - 8 Kohlenstoffatomen, wie Cyclopropyl-, Cyclobutyl-, Cyclopentyl-oder Cyclohexylcarbonyl, oder 2-Cyclohexyl- oder 2-Cyclopentylacetyl,
Cycloalkenylniederalkanoyl, worin Cycloalkenyl z. B. 3 - 7 Kohlenstoffatome hat, wie Cycloalkenylcarbonyl, vorzugsweise mit 4 - 8 Kohlenstoffatomen, wie 1-Cyclohexenylcarbonyl, 1,4-Cyclohexadienylcarbonyl oder 1-Cyclohexenylacetyl oder 1,4-Cyclohexadienylacetyl,
Bicycloalkylniederalkanoyl, worin Bicylcoalkyl z. B. 5 - 10 Kohlenstoffatome enthält, z. B. Bicycloalkylcarbonyl, vorzugsweise mit 8 - 11 Kohlenstoffatomen, wie Decahydronaphthyl-2-carbonyl, endo- oder exo-Norbornyl-2-carbonyl, Bicyclo[2.2.2]oct-2-ylcarbonyl oder Bicyclo[3.3.1]-non-9-ylcarbonyl, ferner Bicyclohexyl-, -heptyl-, -octyl-, -nonyl- oder -decyl-acetyl oder -3-propionyl, wie Bicyclo[3.1.0]hex-1-, - 2- oder -3-yl-, Bicyclo[4.1.0]hept-1- oder -7-yl-, Bicyclo[2.2.1]hept-2-yl-, wie endo- oder exo-Nor-bornyl-, Bicyclo-[3.2.1]oct-2-yl-, Bicyclo[3.3.0]oct-3-yl- oder Bicyclo[3.3.1]non-9-yl-, ferner α- oder β-Decahydronaphthyl-acetyl oder -3-propionyl,
Bicycloalkenylcarbonyl, vorzugsweise mit 8 - 12 Kohlenstoffatomen, wie 5-Nor-bornen-2-ylcarbonyl oder Bicyclo[2.2.2]octen-2-ylcarbonyl,
Tricycloalkylniederalkanoyl, worin Tricycloalkyl z. B. 8 - 10 Kohlenstoffatome enthält, z. B. Tricycloalkylcarbonyl, vorzugsweise mit 8 - 11 Kohlenstoffatomen, wie 1-oder 2-Adamantylcarbonyl, ferner Tricyclo[5.2.1.0^{2,6}]dec-8-yl- oder Adamantyl-, wie 1-Adamantylacetyl,
Arylniederalkanoyl, worin Aryl 6 - 14 Kohlenstoffatome hat, wie in Phenyl, Indenyl, Indanyl, Naphthyl, Anthryl, Phenanthryl, Acenaphthyl oder Fluorenyl, und unsubstituiert oder insbesondere durch Niederalkyl, z. B. Methyl, Ethyl oder Propyl, Halogenniederalkyl, z. B. Trifluormethyl, Phenyl, 1- oder 2-Naphthyl, Hydroxy, Niederalkoxy, z. B. Methoxy, Carbamoylniederalkoxy, N-Niederalkylcarbamoylniederalkoxy oder N,N-Diniederalkylcarbamoylniederalkoxy, Amino, Mono- oder Diniederalkylamino, Niederalkanoylamino, z. B. Pivaloylamino, Halogen, z. B. Fluor, Chlor oder Brom, Carboxy, Niederalkoxycarbonyl, wie tert-Butoxycarbonyl, Phenyl-, Naphthyl- oder Fluorenylniederalkoxycarbonyl, wie Benzyloxycarbonyl, Niederalkanoyl, Sulfo, Niederalkylsulfonyl, z. B. Methylsulfonyl, Phosphono, Hydroxyniederalkoxy-phosphoryl oder Diniederalkoxyphosphoryl, Carbamoyl, Mono- oder Diniederalkylcarbamoyl, Sulfamoyl, Mono- oder Diniederalkylaminosulfonyl, Nitro und/oder Cyano ein- oder bis zu dreifach substituiert sein kann, wobei Phenyl bis zu dreimal vorliegen kann, z. B. in Diphenyl-, Dibenzyl- oder Triphenylniederalkanoyl, wie Diphenyl-, Dibenzyl- oder Triphenylacetyl, und Niederalkanoyl unsubstituiert oder substituiert z. B. durch Niederalkyl, z. B. Methyl, Heterocyclyl ausgewählt aus Pyrrolyl, Furyl, Thienyl, Imidazolyl, Pyrazolyl, Oxazolyl, Thiazolyl, Pyridyl, Pyrazinyl, Pyrimidinyl, Indolyl, Chinolyl, Isochinolyl, Chinoxalinyl, β-Carbolinyl oder einem benzanellierten, cyclopenta-, cyclohexa- oder cyclohepta-anellierten Derivat dieser Reste, die auch ganz oder teilweise gesättigt vorliegen können, Hydroxy, Niederalkoxy, Niederakanoyloxy, z. B. Acetoxy, Propionyloxy, Butyroxy, Isobutyroxy oder Pivaloyloxy, Acetoacetoxy, Amino- oder Benzyloxycarbonylamino-niederalkanoyloxy, z. B. 2-Amino- oder 2-Benzyloxycarbonylamino-2-methyl-propionyloxy, Arylniederalkanoyloxy, worin Aryl 6 - 10 Kohlenstoffatome hat, z. B. in Benzoyloxy, Phenylacetyloxy, 1- oder 2-Naphthoyloxy, Niederalkoxycarbonyloxy, z. B. Methoxy-, Ethoxy-, n-Propoxy-, Isopropoxy-, n-Butoxy-, Isobutoxy-, sec-Butoxy-, tert-Butoxy-, n-Pentoxy-, Isopentoxy-, Neopentoxy-, tert-Pentoxy-, n-Hexoxy-, Isohexoxy- oder n- Heptoxy-carbonyloxy, Mono- oder Diniederalkyl-aminocarbonyloxy, z. B. Ethylaminocarbonyloxy oder Diethylaminocarbonyloxy, Aryloxycarbonyloxy, worin Aryl 6 - 10 Kohlenstoffatome hat, z. B. Phenyloxycarbonyloxy oder 1- oder 2-Naphthyloxycarbonyloxy, Arylniederalkoxycarbonyloxy mit Aryl von 6 - 14 Kohlenstoffatomen, insbesondere Phenylniederalkoxycarbonyloxy, z. B. Benzyloxycarbonyloxy, ferner 1-oder 2-Naphthylmethoxycarbonyloxy oder 9-Fluorenylmethoxycarbonyloxy, Sulfonyloxy, Niederalkylsulfonyloxy, z. B. Methyl-, Ethyl-, Propyl-, Isopropyl-, n-Butyl-, Isobutyl-, sec-Butyl-, tert-Butyl-, n-Pentyl-, Isopentyl-, Neopentyl-, tert-Pentyl-, n-Hexyl-, Isohexyl- oder n-Heptylsulfonyloxy, Phenylsulfonyloxy, 2- oder 4-Toluolsulfonyloxy, 1- oder 2-Naphthylsulfonyloxy, Carboxy, verestertes Carboxy ausgewählt aus Niederalkoxy-carbonyl, z. B. Methoxy-, Ethoxy-, n-Propoxy-, Isopropoxy-, n-Butoxy-, Isobutoxy-, sec-Butoxy-, tert-Butoxy-, n-Pentoxy-, Isopentoxy-, Neopentoxy-, tert-Pentoxy-, n-Hexoxy-, Isohexoxy- oder n- Heptoxycarbonyl, Aryloxycarbonyl, worin Aryl 6 - 10 Kohlenstoffatome hat, z. B. Phenyloxycarbonyl oder 1- oder 2-Naphthyloxycarbonyl, Arylniederalkoxycarbonyl, worin Aryl 6 - 12 Kohlenstoffatome hat, z. B. Benzyloxycarbonyl, 1- oder 2-Naphthylmethyloxycarbonyl oder 9-Fluorenylmethoxycarbonyl, Niederalkanoyl, Niederalkylsulfonyl, z. B. Methyl- oder tert-Butylsulfonyl, Hydroxy-niederalkoxyphosphoryl oder Diniederalkoxyphosphoryl, Carbamoyl, durch einen oder zwei aus Niederalkyl, wie Methyl, Ethyl, Propyl, Isopropyl, n-Butyl, Isobutyl, sec-Butyl, tert-Butyl, n-Pentyl, Isopentyl, Neopentyl, tert-Pentyl, n-Hexyl, Isohexyl oder n-Heptyl, z. B. in N-Methylcarbamoyl, N-n-Butylcarbamoyl oder N,N-Dimethylcarbamoyl, aus Carboxyniederalkyl oder Niederalkoxycarbonyl-niederalkyl, z. B. als Carboxymethylcarbamoyl (Glycinylcarbonyl) oder als tert-Butoxycarbonylmethylcarbamoyl, aus Diniederalkylaminoniederalkyl, z. B. 2-Dimethylaminoethyl, Aminocarboxyniederalkyl, z. B. 5-Amino-5-carboxypentyl, aus Hydroxyniederalkyl, z. B. Hydroxymethyl oder Hydroxyethyl, und aus Diniederalkoxyniederalkyl, z. B. 2-(2,2-Dimethoxyethyl), ausgewählte Reste oder einen aus Ethylen, Trimethylen, Tetramethylen und Pentamethylen, worin ein Kohlenstoffatom durch Stickstoff, Sauerstoff, Schwefel oder mit Sauerstoff ein- oder zweifach substituierten Schwefel ersetzt sein kann und das ungesättigt sein kann, ausgewählten Rest, wie Piperidin-1-yl-, Pyrazin-1-yl-, Piperazin-1-yl-, Pyrimidin-1-yl-, Pyridazin-1-yl-, Morpholino-, Thiomorpholino- oder S,S-Dioxothiomorpholinocarbonyl, substituiertes Carbamoyl, Sulfamoyl, Phosphono, Benzofuranyl, Oxo und/oder Cyano sein kann und unverzweigt oder verzweigt ist, beispielsweise ausgewählt aus Benzoyl, unsubstituiert, mono- oder mehrfachsubstituiert durch Niederalkyl, z. B. Methyl, Phenyl, Halogen, z. B. Fluor oder Chlor, Hydroxy, Niederalkoxy, z. B. Methoxy, und/oder Nitro, wie 4-Chlor-, 4-Methoxy- oder 4-Nitrobenzoyl, Naphthylcarbonyl, wie α-oder β-Naphthylcarbonyl oder über beide Carbonylgruppen an die Aminogruppe gebundenem 1,8-Naphthalindicarbonyl, Indenylcarbonyl, wie 1-, 2- oder 3-Indenylcarbonyl, Indanylcarbonyl, wie 1- oder 2-Indanylcarbonyl, Phenanthrenylcarbonyl, wie 9-Phenanthrenylcarbonyl, Phenylacetyl, α-Naphthylacetyl, β-Naphthylacetyl, Niederalkyl-phenyl-acetyl, wie 4-Methylphenylacetyl, Niederalkoxyphenylacetyl, wie 4-Methoxy-phenylacetyl, 3-Phenylpropionyl, 3-(p-Hydroxyphenyl)-propionyl, Diphenylacetyl, Di-(4-methoxyphenyl)-acetyl, Triphenylacetyl, 2,2-Dibenzylacetyl, im Phenylrest durch einen oder zwei aus Niederalkyl, z. B. Methyl oder Ethyl, Hydroxy, Niederalkoxy, z. B. Methoxy, Amino, Mono- oder Diniederalkylamino, z. B. Ethylamino oder Dimethyl-amino, Halogen, z. B. Fluor oder Chlor, Carboxy, Sulfo, Carbamoyl, Sulfamoyl und Cyano, und/oder an der Aminogruppe aus Niederalkyl und Benzyl ausgewählten Resten substituiertem Anilinophenylacetyl, wie 2-(o,o-Dichloranilino)-phenylacetyl oder 2-(o,o-Dichloro-N-benzylanilino)-phenylacetyl, 3-α- oder 3-β-Naphthylpropionyl, 2-Benzyl-3-(1-pyrazolyl-)-propionyl, 3-Phenyl- oder 3-α-Naphthyl-2-hydroxy-propionyl, 3-Phenyl- oder 3-α-Naphthyl-2-niederalkoxy-propionyl, wie 3-Phenyl- oder 3-α-Naph-thyl-2-neopentyloxy-propionyl, 3-Phenyl- oder 3-α-Naphthyl-2-niederalkanoyloxy-propionyl, wie 3-Phenyl-2-pivaloyloxy- oder -2-acetoxy-propionyl, 2-Benzyl- oder 1- oder 2-Naphthyl-3-(N-methoxy-N-methylamino)propionyl, 3-α-Naphthyl-2-acetoacetoxy-propionyl, 3-α-Naphthyl-2-ethylaminocarbonyloxy-propionyl oder 3-α-Naphthyl-2-(2-amino- oder 2-benzyloxycarbonylamino-2-methylpropionyloxy)-propionyl, 3-Phenyl- oder 3-α-Naphthyl-2-carboxymethylpropionyl, 3-Phenyl- oder 3-α-Naphthyl-2-nieder-alkoxycarbonyl-propionyl, wie 3-α-Naphthyl-2-ethoxycarbonyl-propionyl, 3-Phenyl- oder 3-α-Naphthyl-2-benzyloxycarbonylmethyl-propionyl, 2-(S)Benzyl-3-tert-butylsulfonylpropionyl, 3-Phenyl-2-phosphono- oder -phosphonomethylpropionyl, 3-Phenyl-2-di-methoxyphosphoryl- oder -dimethoxyphosphorylmethyl-propionyl, 3-Phenyl-2-diethoxy-phosphoryl- oder -diethoxyphosphorylmethyl-propionyl, 3-Phenyl-2-ethoxy- oder -methoxy-hydroxyphosphorylpropionyl, Phenylniederalkanoyl, worin der Niederalkanoylrest durch Carbamoyl substituiert ist, wie 2(R,S)-Carbamoyl-3-phenylpropionyl, 3-Phenyl- oder 3-α-Naphthyl-2-carbamoylpropionyl, 3-Phenyl- oder 3-α-Naphthyl-2-tert-butylcarbamoyl-propionyl, 3-Phenyl- oder 3-α-Naphthyl-2-(2-dimethylaminoethyl)carbamoylpropionyl, 3-α-Naphthyl-2-(carboxy- oder tert-butoxycarbonyl)methylcarbamoylpropionyl, 3-Phenyl- oder 3-α-Naphthyl-2-(3-hydroxy-2-propyl)carbamoyl-propionyl, 3-Phenyl- oder 3-α-Naphthyl-2-(2,2-dimethoxyethyl)-carbamoylpropionyl, 3-Phenyl- oder 3-α-Naphthyl-2(5-amino-5-carboxypentyl)-carbamoylpropionyl, 3-Phenyl- oder 3-α-Naphthyl-2-cyano-propionyl, 3-Phenyl- oder 3-α-Naphthyl-2-cyanomethyl-propionyl, 3-Phenyl- oder 3-α-Naphthyl-2-acetonyl-propionyl, 4-Hydroxyphenylbutyryl, 4-Phenyl- oder 4-α-Naphthyl-3-carboxy-butyryl, 4-Phenyl- oder 4-α-Naphthyl-3-benzyloxycarbonyl-butyryl, 2-Benzyl- oder 2-α-Naphthylmethyl-4-cyano-butyryl, 2-Benzy-4-(2-benzofuranyl)-4-oxobutyryl, 2-Benzyl- oder 2-α-Naphthylmethyl-5-dimethylamino-pentanoyl, 2-Benzyl- oder 2-α-Naphthylmethyl-4-oxopentanoyl, 2-Benzyl- oder 2-α-Naphthylmethyl-4,4-dimethyl-3-oxo-pentanoyl, 2-Benzyl- oder 2-α-Naphthylmethyl-5-dimethylamino-4-oxopentanoyl, und 2-Benzyl- oder 2-α-Naphthylmethyl-5,5-dimethyl-4-oxo-hexanoyl, in erster Linie Phenylniederalkanyol, wie Phenylacetyl, oder Phenyl-niederalkanoyl, worin der Niederalkanoylrest durch Carbamoyl substituiert ist, wie 2(R,S)-Carbamoyl-3-phenylpropionyl,
Phenylniederalkenoyl, wie β-Phenylacryloyl oder β-Phenylvinylacetyl,
Heterocyclylniederalkanoyl, worin Niederalkanoyl unsubstituiert oder wie oben unter Arylniederalkanoyl R₁, R₂, R₈ oder R₉ definiert substituiert ist und worin Heterocyclyl vorzugsweise ein einfaches oder doppeltes Ringsystem mit 3 - 10 Ringatomen ist, über ein Kohlenstoff- oder insbesondere Stickstoffatom gebunden ist und bis zu 3 weitere Heteroatome, ausgewählt aus Sauerstoff, Stickstoff, Schwefel, Selen und mit 1 oder 2 Sauerstoffatomen verknüpftem Schwefel, enthält, das genannte Ringsystem zusätzlich mit 1 oder 2 Phenyl- oder Naphthylresten anneliert sein kann, wobei Naphthyl auch über zwei Seiten ankondensiert sein kann, oder mit 1 oder 2 Cycloalkylresten anneliert sein kann, wobei Cycloalkyl vorzugsweise 5 - 7 Ringatome hat; und ungesättigt oder auch teilweise oder ganz gesättigt sein kann, z. B. Thienyl-, Furyl-, Pyranyl-, Pytrolyl-, Imidazolyl-, Pyrazolyl-, Oxazolyl-, Isoxazolyl-, Thiazolyl-, Furazanyl-, Pyridyl-, Pyrazinyl-, Pyrimidinyl-, Pyridazinyl-, Azepinyl-, Indolyl-, Benzimidazolyl-, 1H-Indazolyl-, Chinolyl-, Isochinolyl-, Chinoxalinyl-, Chinazolinyl-, Cinnolyl-, Purinyl-, Pteridinyl-, Naphthyridinyl-, 4H-Chinolizinyl-, 3,1-Benzfuranyl-, Benz[e]indolyl-, 4,1-Benzoxazinyl-, 4,1-Benzthiazinyl-, Carbazolyl-, β-Carbolinyl-, Phenazinyl-, Phenanthridyl-, Acridinyl-, Phenoxazinyl-, Phenothiazinyl-, 1-Azaacenaphtenyl-, Cyclohexa[b]pyrrolyl-, Cyclohepta[b]pyrrolyl-, Cyclohexa[d]pyrazolyl-, Cyclohexa[b]pyridyl-, Cyclohexa[b]pyrazinyl-, Cyclohexa[b]pyrimidinyl-, Cyclohexa[b]-1,4-oxazinyl-, Cyclohexa[b]-1,4-thiazinyl-, Pyrrolidinyl-, Pyrrolinyl-, Immidazolidyl-, 2-Immidazolinyl-, 2,3-Dihydropyridyl-, Piperidyl-, Piperazinyl-, 2,3,5,6-Tetrahydro-pyrazinyl-, Morpholinyl-, Thiomorpholinyl-, S,S-Dioxo-thiomorpholinyl-, Indolinyl-, Isoindolinyl-, 4,5,6,7-Tetrahydroindolyl-, 1,2,3,4-Tetrahydrochinolyl-, 1,2,3,4-Tetrahydroisochinolyl-, Chromanyl-, Thiochromanyl-, 1,2,3,4-Tetrahydro-3,1-benzdiazinyl-, 3,4-Dihydro-3H-4,1-benzoxazinyl-, 3,4-Dihydro-3H-4,1-benzthiazinyl-, 2,3,4,5-Tetrahydro-1H-5,1-benzazepinyl- oder 5,6-Dihydrophenanthridinyl-niederalkanoyl, wobei die genannten Heterocyclyl-Reste unsubstituiert oder durch Niederalkyl, z. B. Methyl, Phenyl, 1- oder 2-Naphthyl, Phenylniederalkyl, z. B. Benzyl, Hydroxyniederalkyl, z. B. Hydroxymethyl oder 2-Hydroxyethyl, Niederalkoxyniederalkyl, z. B. Methoxymethyl oder 2-Methoxyethyl, Phenoxy- oder Naphthoxyniederalkyl, z. B. 2-Phenoxyethyl, 1- oder 2-Naphthoxymethyl, Phenylniederalkoxy- oder Naphthylniederalkoxy-niederalkyl, z. B. Benzyloxyniederalkyl, Niederalkanoyloxy-niederalkyl, z. B. Acetyloxymethyl, Phenyl- oder Napthyl-niederalkanoyloxy-niederalkyl, z. B. Benzoyloxy-, Phenylacetyloxy- oder 1- oder 2-Naphthoyloxy-methyl, -2-ethyl oder -2-(2,2-dimethylethyl), Niederalkoxycar-bonyloxy-niederalkyl, z. B. tert-Butyloxycarbonyloxyniederalkyl, Phenyl-, Napthyl- oder Fluorenylniederalkoxycarbonyloxy-niederalkyl, z. B. 2-Benzyloxycarbonyloxyethyl oder 9-Fluorenylmethoxycarbonyloxyethyl, Aminoniederalkyl, z. B. Aminomethyl, 2-Amino-ethyl oder 2-Aminopropyl, Carboxyniederalkyl, z. B. Carboxymethyl oder 2-Carboxy-ethyl, Hydroxy, Niederalkoxy, z. B. Methoxy oder Ethoxy, Phenyl-oder Naphthylnieder-alkoxy, z. B. Benzyloxy oder 1- oder 2-Naphthyloxy, Amino, Niederalkylamino, z. B. Methyl-, Ethyl- oder tert-Butylamino, Diniederalkylamino, z. B. Dimethyl- oder Diethylamino, Carboxy, Niederalkoxycarbonyl, z. B. Methoxy-, Isopropoxy-, sec-Butoxy- oder tert-Butoxy-carbonyl, Phenyl- oder Napthyl-niederalkoxycarbonyl, z. B. Benzyl-oxycarbonyl, Halogen, z. B. Fluor, Chlor, Brom oder Jod, insbesondere Chlor oder Brom, Niederalkanoyl, z. B. Acetyl oder Pivaloyl, Niederalkylsulfonyl, z. B. Methyl- oder Ethyl-sulfonyl, Phosphono, Hydroxy-niederalkoxy-phosphoryl oder Dialkoxy-phosphoryl, z. B. Dimethoxy- oder Diethoxyphosphoryl, Carbamoyl, Mono- oder Diniederalkylcarbamoyl, z. B. N-Methylcarbamoyl, N-n-Butylcarbamoyl oder N,N-Dimethylcarbamoyl, Hydroxy- oder Carboxyniederalkylcarbamoyl, z. B. Hydroxy- oder Carboxymethylcarbamoyl oder Hydroxy- oder Carboxyethylcarbamoyl, Sulfamoyl, durch Nitro, Oxo und/oder durch Cyano substituiert sind, wobei Heterocyclylniederalkanoyl insbesondere ausgewählt ist aus gegebenenfalls durch Niederalkyl oder Phenyl substituiertem Pyrrolylcarbonyl, z. B. 2- oder 3-Pyrrolylcarbonyl, 4- oder 5-Methylpyrrolylcarbonyl oder 4- oder 5-Phenyl-pyrrolyl-2-carbonyl, Thienylcarbonyl, wie 2-Thienylcarbonyl, Furylcarbonyl, wie 2-Furylcarbonyl, Pyridylcarbonyl, wie 2-, 3- oder 4-Pyridylcarbonyl, pyrimidin-1-yl-carbonyl, gegebenenfalls durch Niederalkyl, wie Methyl, Phenylniederalkyl, wie Benzyl, Niederalkoxy, wie Methoxy, Phenylniederalkoxy, wie Benzyloxy oder Halogen, wie Chlor, substituiertem Indolylcarbonyl, wie 2-, 3- oder 5-Indolylcarbonyl, 1-Methyl-, 5-Methyl-, 5-Methoxy-, 5-Benzyloxy-, 5-Chlor-oder 4,5-Dimethylindolyl-2-carbonyl, 1-Benzyl-indolyl-2- oder -3-carbonyl, 4,5,6,7-Tetrabydroindolyl-2-carbonyl, gegebenenfalls durch Hydroxy substituiertem Chinolylcarbonyl, wie 2-, 3- oder 4-Chinolylcarbonyl oder 4-Hydroxychinolyl-2-carbonyl, gegebenenfalls durch Hydroxy substituiertem Isochinolylcarbonyl, wie 1-, 3- oder 4-Isochinolylcarbonyl oder 1-Oxo-1,2-dihydroisochinolyl-3-carbonyl, 2-Chinoxalinylcarbonyl, 2-(3,1-Benzfuranyl)carbonyl, Benz[e]indolyl-2-arbonyl, β-Carbolinyl-3-carbonyl, Cyclohepta[b]pyrrolyl-5-carbonyl, 3-Chromanylcarbonyl, 3-Thiochromanylcarbonyl, Pyrrolidinyl-3-carbonyl, Hydroxypyrrolidinylcarbonyl, wie 3- oder 4-Hydroxypyrrolidinyl-2-carbonyl, Oxopyrrolidinylcarbonyl, wie 5-Oxopyrrolidinyl-2-carbonyl, Piperidylcarbonyl, wie Piperidinocarbonyl oder 2-, 3- oder 4-Piperidylcarbonyl, Pyrazinylcarbonyl, wie Pyrazin-1-ylcarbonyl, Piperazinylcarbonyl, wie Piperazin-1-yl-carbonyl, Morpholinylcarbonyl, wie Morpholinocarbonyl, Thiomorpholinylcarbonyl, wie Thiomorpholinocarbonyl, S,S-Dioxothiomorpholinylcarbonyl, wie S,S-Dioxothiomorpholinocarbonyl, Indolinylcarbonyl, wie 2- oder 3-Indolinylcarbonyl, 1,2,3,4-Tetrahydrochinolylcarbonyl, wie 1,2,3,4-Tetrahydrochinolyl-2-, - 3- oder -4-carbonyl, 1,2,3,4-Tetrahydroisochinolylcarbonyl, wie 1,2,3,4-Tetrahydroisochinolyl-1-, -3- oder 4-carbonyl oder 1-Oxo-1,2,3,4-tetrahydroisochinolyl-3-carbonyl, und Pyridylniederalkanoyl, z. B. Pyridylacetyl, wie 2-, 3- oder 4-Pyridylacetyl, insbesondere ausgewählt aus Morpholinocarbonyl, Thiomorpholinocarbonyl, S,S-Dioxothiomorpholinocarbonyl, Pyridylacetyl, Indolylacetyl, Benzfuranylacetyl, 2-Morpholino-2-isopropyl-acetyl und 2-(S,S-Dioxothiomorpholino)-2-isopropyl-acetyl, ganz besonders bevorzugt Morpholinocarbonyl, Thiomorpholinocarbonyl, Chinolin-2-ylcarbonyl, oder 3-Pyridylacetyl,
Hydroxyniederalkanoyl, wie 3-Hydroxypropionyl oder 2-Hydroxy-3-methylpentanoyl,
Niederalkoxyniederalkanoyl, z. B. Niederalkoxyacetyl oder Niederalkoxypropionyl, wie Methoxyacetyl, Ethoxyacetyl oder 3-Methoxypropionyl,
Niederalkoxyniederalkoxy-niederalkanoyl, wie 2-Methoxymethoxy-3-methyl-pentanoyl,
Phenoxyniederalkanoyl oder Nitrophenoxyniederalkanoyl, wie Phenoxyacetyl oder 4-Nitrophenoxyacetyl,
Naphthoxyniederalkanoyl, wie α- oder β-Naphthoxyacetyl,
Niederalkanoyloxyniederalkanoyl, worin Niederalkanoyloxy z. B. Acetoxy, Propionyloxy, Butyroxy, Isobutyroxy oder Pivaloyloxy bedeutet, wie Acetoxyacetyl oder 3-Acetoxypropionyl,
Acetoacetoxyniederalkanoyl, wie 3-Acetoacetoxy-propionyl
Amino- oder Benzyloxycarbonylamino-niederalkanoyloxyniederalkanoyl, z. B. 2-Amino- oder 2-Benzyloxycarbonylamino-2-methylpropionyloxy-niederalkanoyl, wie -acetyl oder -3-propionyl,
Arylniederalkanoyloxyniederalkanoyl, worin Aryl 6 - 10 Kohlenstoffatome hat, wie in Benzoyloxy-, Phenylacetyloxy- oder 1- oder 2-Naphthoyloxy-niederalkanoyl,
Niederalkoxycarbonyloxyniederalkanoyl, z. B. Methoxy-, Ethoxy-, n-Propoxy-, Isopropoxy-, n-Butoxy-, Isobutoxy-, sec-Butoxy-, tert-Butoxy-, n-Pentoxy-, Isopentoxy-, Neopentoxy-, tert-Pentoxy-, n-Hexoxy-, Isohexoxy- oder n-Heptoxycarbonyloxyniederalkanoyl, wie -acetyl oder -3-propionyl,
Mono- oder Diniederalkyl-aminocarbonyloxyniederalkanoyl, z. B. Ethylaminocarbonyloxyniederalkanoyl oder Diethylaminocarbonyloxyniederalkanoyl, wie -acetyl oder -3-propionyl,
Aryloxycarbonyloxyniederalkanoyl, worin Aryl 6 - 10 Kohlenstoffatome hat, z. B. Phenyloxycarbonyloxy- oder 1- oder 2-Naphthyloxycarbonyloxy-niederalkanoyl, wie -acetyl oder -3-propionyl,
Arylniederalkoxycarbonyloxyniederalkanoyl mit Aryl von 6 - 12 Kohlenstoffatomen, z. B. Phenylniederalkoxycarbonyloxy-niederalkanoyl, wie Benzyloxycarbonyloxy-acetyl oder -3-propionyl, ferner 1- oder 2-Naphthylmethoxycarbonyloxyniederalkanoyl oder 9-Fluorenylmethoxycarbonyloxyniederalkanoyl, wie -acetyl oder -3-propionyl
Sulfonyloxyniederalkanoyl, wie -acetyl oder -3-propionyl,
Niederalkylsulfonyloxyniederalkanoyl, z. B. Methyl-, Ethyl-, Propyl-, Isopropyl-, n-Butyl-, Isobutyl-, sec-Butyl-, tert-Butyl-, n-Pentyl-, Isopentyl-, Neopentyl-, tert-Pentyl-, n-Hexyl-, Isohexyl- oder n-Heptylsulfonyloxyniederalkanoyl, wie -acetyl oder -3-propionyl,
Phenylsulfonyloxy-, 2- oder 4-Toluolsulfonyloxy- oder 1- oder 2- Naphthylsulfonyloxyniederalkanoyl,
Arylmercaptoniederalkanoyl, worin Aryl 6 - 10 Kohlenstoffatome hat und vorzugsweise Phenyl oder Naphthyl bedeutet, z. B. Phenylmercaptoniederalkanoyl, wie - acetyl oder -3-propionyl,
Aminoniederalkanoyl, worin die Aminogruppe nicht in α- oder β-Stellung vorliegt, wie 5-Aminopentanoyl,
Niederalkanoylarmino-niederalkanoyl, worin die Aminogruppe nicht in α-oder β-Stellung des Niederalkanoylrestes steht, wie 5-Amino-pentanoyl,
Niederalkoxycarbonylamino-niederaklanoyl, worin die Aminogruppe nicht in α-oder β-Stellung des Niederalkanoylrestes steht, wie 5-(tert-Butoxycarbonylamino)-pentanoyl,
Phenylniederalkoxycarbonylamino-niederalkanoyl, worin die Aminogruppe nicht in α-oder β-Stellung des Niederalkanoylrestes steht, wie 5-Benzyloxycarbonylaminopentanoyl oder 6-Benzyloxycarbonylaminohexanoyl,
Halogenniederalkanoyl, welches vorzugsweise bis zu 3 Halogenatome enthalt, z. B. α-Halogenacetyl, wie α-Fluor-, α-Chlor-, α-Brom-, α-Iod-, α,α,α Trifluor-oder α,α,α-Trichloracetyl, oder Halogenpropionyl, wie β-Chlor- oder β-Brompropionyl,
Carboxyniederalkanoyl, z. B. Carboxyacetyl oder β-Carboxypropionyl,
Niederalkoxycarbonylniederalkanoyl, z. B. Niederalkoxycarbonylacetyl oder Niederalkoxycarbonylpropionyl, wie Methoxycarbonylacetyl, 3-Methoxycarbonylpropionyl, Ethoxycarbonylacetyl, 3-Ethoxycarbonylpropionyl oder 3-tert-Butoxycarbonylpropionyl,
Niederalkylcarbonyl-halogen-niederalkanoyl, wie 3-Ethoxycarbonyl-2-difluormethylpropionyl,
2-Halogenniederalkoxycarbonyl-niederalkanoyl, wie 2-Chlor-, 2-Brom-, 2-Iod- oder 2,2,2-Trichlorethoxycarbonyl-acetyl oder 3-propionyl,
Phenyl- oder Naphthylniederalkyloxycarbonyl-niederalkanoyl, z. B. Benzyloxycarbonylniederalkanoyl, wie 3-Benzyloxycarbonyl-2,2-dimethylpropionyl,
Heterocyclylniederaikoxycarbonyl-niederalkanoyl, worin Heterocyclyl vorzugsweise ausgewählt ist aus Pyrrolyl, Furyl, Thienyl, Imidazolyl, Pyrazolyl, Oxazolyl, Thiazolyl, Pyridyl, Pyrazinyl, Pyrimidinyl, Indolyl, Chinolyl, Isochinolyl, Chinoxalinyl und β-Carbolinyl oder einem benzanellierten, cyclopenta-, cyclohexa- oder cyclohepta-anellierten Derivat dieser Reste, wobei die genannten Reste auch ganz oder teilweise gesättigt vorliegen können, wie in 4-Pyridylmethoxycarbonyl-acetyl oder -3-propionyl oder 2-Morpholinocarbonyloxy-4-methyl-pentanoyl,
Sulfonylniederalkanoyl, wie 3-Sulfonylpropionyl,
Niederalkylsulfonylniederalkanoyl, wie 2-Ethylsulfonyl- oder 2-tert-Butylsulfonylacetyl,
Arylsulfonylniederalkanoyl, worin Aryl vorzugsweise 6 - 10 Kohlenstoffatome hat, z. B. Phenyl oder Napthyl, wie Phenylsulfoacetyl
Carbamoylniederalkanoyl, wie Carbamoylacetyl oder 3-Carbamoylpropionyl,
Niederalkylcarbamoylniederalkanoyl, z. B. Niederalkylcarbamoylacetyl oder Methylcarbamoylniederalkanoyl, wie Methylcarbamoylacetyl,
Diniederalkylcarbamoylniederalkanoyl, z. B. Diniederalkylcarbamoylacetyl oder Dimethylcarbamoylniederalkanoyl, wie Dimethylcarbamoylacetyl,
Hydroxyniederalkylcarbamoyl- oder Di-(hydroxyniederalkyl)carbamoyl-niederalkanoyl, wie Hydroxymethylcarbamoyl- oder Di-(hydroxymethyl)carbamoyl-acetyl oder -propionyl,
N-Niederalkoxyniederalkoxyniederalkyl-carbamoyl-niederalkanoyl, wie 2-Isobutyl-3-(2-(2-methoxyethoxy)ethylaminocarbonyl)-propionyl,
Carboxyniederalkylcarbamoyl- oder Di-(carboxyniederalkyl)carbamoyl-niederalkanoyl, wie Carboxymethyl- oder Di-(carboxymethyl)carbamoyl-acetyl oder -propionyl,
durch einen aus Ethylen, Trimethylen, Tetramethylen und Pentamethylen ausgewählten Rest, worin ein Kohlenstoffatom durch Stickstoff, Sauerstoff, Schwefel oder mit Sauerstoff ein- oder zweifach substituiertem Schwefel ersetzt sein kann, am Stickstoff substituiertes Carbamoylniederalkanoyl, wobei der so gebildete Rest auch ganz oder teilweise ungesättigt sein kann, z. B. als Piperidino-, Pyrazin-1-yl-, Piperazin-1-yl-, Pyrimidin-1-yl-, Pyridazin-1-yl-, Morpholino-, Thiomorpholino- oder S,S-Dioxothio-morpholinocarbonylniederalkanoyl, wie in Morpholinocarbonyl-acetyl, 3-(Morpholino-carbonyl)-propionyl oder 3-(Morpholinocarbonyl)-2-isobutyl-propionyl,
N-Heterocyclylniederalkyl-carbamoyl-niederalkanoyl oder N-Niederalkyl-N-hetero-cyclylniederalkylcarbamoylniederalkanoyl, worin Heterocyclyl vorzugsweise aus Pyrrolyl, Furyl, Thienyl, Imidazolyl, pyrazolyl, Oxazolyl, Thiazolyl, Pyridyl, Pyrazinyl, Pyrimidinyl, Indolyl, Chinolyl, Isochinolyl, Chinoxalinyl, β-Carbolinyl und einem benzanellierten, cyclopenta-, cyclohexa- oder cyclohepta-anellierten Derivat dieser Reste, die auch ganz oder teilweise gesättigt sein können, aus Morpholinyl und Thiomorpholinyl ausgewählt ist, wie N-Methyl-2-(N-2-pyridylmethyl)-carbamoylacetyl, 2-(N-Morpholino-niederalkyl-carbamoyl)-niederalkanoyl, wie 2(R,S)-(N-(2-morpholinoethyl)-carbamoyl-3-methyl-butyryl, oder 2-(N-(Pyridylniederalkyl)-carbamoyl)-niederalkanoyl, wie (2(R,S)-(N-(2-pyridylmethyl)-carbamoyl)-3-methyl)-butyryl
Sulfamoylniederalkanoyl, wie 2-Sulfamoylacetyl,
N-(Phenyl- oder Naphthylniederalkyl)sulfamoylniederalkanoyl, wie 3-Benzylaminosulfonyl-2-isopropyl-propionyl,
am Stickstoffatom von einem aus Ethylen, Trimethylen, Tetramethylen und Penta-methylen ausgewählten Rest, worin ein Kohlenstoffatom durch Stickstoff, mit Niederalkyl, wie Methyl, substituierten Stickstoff, Sauerstoff, Schwefel oder mit Sauerstoff ein-oder zweifach substituiertem Schwefel ersetzt sein kann, substituiertes Sulfamoylniederalkanoyl, wobei der so gebildete Rest auch ganz oder teilweise ungesättigt sein kann, z. B. als Piperidino-, Pyrazin-1-yl-, Piperazin-1-yl-, 4-Methylpiperazin-1-yl, Pyrimidin-1-yl-, Pyridazin-1-yl-, Morpholino-, Thiomorpholino- oder S,S-Dioxothiomorpholino-sulfonylniederalkanoyl, wie 3-(4-Methylpiperazinylsulfonyl)-2-isopropylpropionyl oder 3-(Morpholinosulfonyl)-2-isopropyl-propionyl,
Oxoniederalkanoyl, wie Acetoacetyl oder Propionylacetyl,
Cyanoniederalkanoyl, wie Cyanoacetyl, 2- oder 3-Cyanopropionyl oder 2-, 3- oder 4-Cyanobutyryl,
Hydroxy-carboxy-niederalkanoyl, wie 2-Hydroxy-2-carboxy-acetyl oder 2-Hydroxy-3-carboxypropionyl,
α-Naphthoxy-carboxy-niederalkanoyl, wie 2-α-Naphthoxy-4-carboxy-butyryl,
Hydroxy-niederalkoxycarbonyl-niederalkanoyl, z. B. Hydroxy-niederalkoxycarbonylacetyl oder -propionyl oder Hydroxy-ethoxy- oder Hydroxy-methoxy-carbonyl-niederalkanoyl, wie 2-Hydroxy-2-ethoxy- oder -methoxycarbonylacetyl oder 2-Hydroxy-3-eth-oxy- oder -methoxycarbonyl-propionyl,
α-Naphthoxy-niederalkoxycarbonylniederalkanoyl, z. B. α-Naphthoxy-niederalk-oxycarbonyl-acetyl, -propionyl oder -butyryl oder α-Naphthoxy-ethoxycarbonyl-niederalkanoyl, wie α-Naphthoxy-ethoxycarbonyl-acetyl, 2-α-Naphthoxy-3-ethoxycarbonyl-propionyl oder 2-α-Naphthoxy-4-tert-butoxycarbonylbutyryl,
α-Naphthoxy-benzyloxycarbonyl-niederalkanoyl, wie 2-α-Naphthoxy-3-benzyloxy-carbonyl-propionyl,
verestertes Hydroxy-niederalkoxycarbonyl-niederalkanoyl, worin die Hydroxygruppe durch Niederalkanoyl, z. B. Acetyl, Propionyl oder Pivaloyl, Cycloalkylniederalkanoyl, worin Cycloalkyl 3 - 7 Kohlenstoffatome hat und Niederalkanoyl vorzugsweise wie zuletzt definiert ist, z. B. Cyclohexylcarbonyl oder 2-Cyclohexyl- oder 2-Cyclopentylacetyl, Bicycloalkylniederalkanoyl, worin Bicylcoalkyl z. B. 5 - 10, insbesondere 6 - 9, Kohlenstoffatome enthält, wie in Bicyclohexyl-, -heptyl-, -octyl-, -nonyl-oder -decyl-acetyl oder -3-propionyl, z. B. Bicyclo[3.1.0]hex-1-, -2- oder -3-yl-, Bicyclo[4.1.0]hept-1- oder -7-yl-, Bicyclo[2.2.1]hept-2-yl-, wie endo- oder exo-Norbornyl-, Bicyclo[3.2.1]-oct-2-yl-, Bicyclo[3.3.0]oct-3-yl- oder Bicyclo[3.3.1]non-9-yl-, ferner α- oder β-Deca-hydronaphthyl-acetyl oder -3-propionyl, Tricycloalkylniederalkanoyl, worin Tricycloalkyl z. B. 8 - 10 Kohlenstoffatome enthält, z. B. in Tricyclo[5.2.1.0^{2,6}]dec-8-yl- oder Adamantyl-, wie 1-Adamantyl-acetyl, Arylniederalkanoyl, worin Aryl 6 - 14 Kohlenstoffatome hat, z. B. Phenyl, Indenyl, Indanyl, Naphthyl, Anthryl, Phenanthryl, Acenaphthyl oder Fluorenyl, das unsubstituiert oder durch Niederalkyl, wie Isopropyl, Halogenniederalkyl, wie Trifluormethyl, Hydroxy, Niederalkoxy, Carbamoylniederalkoxy, N-Nieder-alkylcarbamoylniederalkoxy oder N,N-Diniederalkylcarbamoylniederalkoxy, Amino, Mono- oder Diniederalkylamino, Halogen, wie Fluor, Chlor oder Brom, Carboxy, Nieder-alkoxycarbonyl, Phenyl-, Naphthyl- oder Fluorenylniederalkoxycarbonyl, Niederalkanoyl, Niederalkylsulfonyl, z. B. Methylsulfonyl, Phosphono, Hydroxy-niederalkoxy-phosphoryl oder Diniederalkoxyphosphoryl, Carbamoyl, Mono- oder Diniederalkylaminocarbamoyl, Sulfamoyl, Mono- oder Diniederalkylsulfamoyl, Nitro und/oder Cyano ein- oder bis zu dreifach substituiert sein kann, Niederalkoxycarbonyl, z. B. tert-Butoxycarbonyl, 2-Halogenniederalkoxycarbonyl, wie oben definiert, oder Phenyl- oder Fluorenyl-niederalkoxy-carbonyl, z. B. Benzyloxycarbonyl oder 9-Fluorenylmethoxycarbonyl, verestert ist, wie α-Acetoxy-α-methoxycarbonyl-acetyl, α-Benzoyloxy-, α-(1- oder 2-Naphthoyloxy)-, α-(Phenyl-2-acetyloxy)-, α-(1- oder 2-Napthyl-2-acetyloxy)-, α-(4-Methylphenyl-2-acetyloxy)-, α-(4-Methoxyphenyl-2-acetyloxy)- oder α-(2-(o,o-Dichlorophenyl)-2-acetyl-oxy)-α-methoxycarbonyl-acetyl,
Dihydroxy-carboxy-niederalkanoyl, wie 2,3-Dihydroxy-3-carboxy-propionyl,
Dihydroxy-niederalkoxycarbonyl-niederalkanoyl, wie 2,3-Dihydroxy-3-ethoxy- oder -methoxycarbonyl-propionyl,
durch Niederalkanoyl, wie Acetyl, Propionyl oder Butyryl, Niederalkoxycarbonyl, z. B. tert-Butoxycarbonyl, Phenyl- oder Fluorenyl-niederalkoxycarbonyl, wie Benzyloxy-carbonyl oder 9-Fluorenylmethoxycarbonyl, Niederalkylsulfonyl oder Toluolsulfonyl verestertes Dihydroxy-niederalkoxycarbonyl-niederalkanoyl, z. B. Diniederalkanoyl-oxy-niederalkoxy-propionyl, wie 2,3-Diacetoxy-3-methoxycarbonyl-propionyl,
α-Naphthoxy-diniederalkylamino-niederalkanoyl, wie 2-α-Naphthoxy-5-dimethyl-amino-pentanoyl,
α-Naphthoxy-carbamoyl-niederalkanoyl, wie 2-α-Naphthoxy-4-carbamoyl-butyryl,
α-Naphthoxy-oxo-niederalkanoyl, wie 2-α-Naphthoxy-4-oxo-pentanoyl,
α-Naphthoxy-cyanoniederalkanoyl, wie α-Naphthoxy-cyano-acetyl oder 2-α-Naphthoxy-4-cyanobutyryl,
Niederalkenoyl mit 3 - 7 Kohlenstoffatomen, bevorzugt mit 3 - 4 Kohlenstoffatomen, wobei Niederalkenoyl unsubstituiert oder durch die gleichen Substituenten wie Niederalkanoyl alkanoyl substituiert sein kann, insbesondere durch Phenyl, Hydroxy, Niederalkoxy, wie Methoxy, Phenylniederalkoxy, wie Benzyloxy, Niederalkanoyloxy, wie Acetoxy, Niederalkanoylamino, wie Acetylamino, Niederalkyloxycarbonyloxy, wie tert-Butoxycarbonyloxy, Phenyl- oder Naphthylniederalkoxycarbonyloxy, wie Benzyloxycarbonyloxy, Carboxy, Niederalkoxycarbonyl, wie tert-Butoxycarbonyl, Phenyl- oder Napthylniederalkoxycarbonyl, Halogen, wie Chlor oder Brom, Carbamoyl und/oder Mono- oder Diniederalkylcarbamoyl, wie in Acryloyl, Vinylacetyl, Crotonoyl oder 3- oder 4-Pentenoyl, 3-Phenylacryloyl, 3-Phenylvinylacetyl oder 5-Phenyl-4-acetylaminopenten-2-oyl,
Cycloalkylniederalkenoyl, worin Cycloalkyl vorzugsweise 3 - 7 Kohlenstoffatome hat, z. B. Cyclohexylacryloyl, oder
Niederalkinoyl mit 3 - 7, vorzugsweise 3 oder 4 Kohlenstoffatomen, z. B. Propioloyl oder 2- oder 3-Butinoyl.

Bevorzugte Acylgruppen R₁, R₂, R₈ und R₉ eines Halbesters der Kohlensäure sind
Niederalkoxycarbonyl, z. B. Methoxy-, Ethoxy-, Isopropoxy- oder tert-Niederalkoxycarbonyl, wie tert-Butoxycarbonyl,
2-Halogenniederalkoxycarbonyl, wie 2-Chlor-, 2-Brom-, 2-Iod- oder 2,2,2-Trichlorethoxycarbonyl,
Aryloxycarbonyl, worin Aryl 6 bis 14 Kohlenstoffatome hat und beispielsweise Phenyl, Biphenylyl, 1- oder 2-Naphthyl, Fluorenyl oder durch Niederalkyl, z. B. Methyl oder tert-Butyl, Hydroxy, Niederalkoxy, z. B. Methoxy, Ethoxy oder tert-Butoxy, Halogen, z. B. Chlor oder Brom, und/oder Nitro mono- oder mehrfach substituiertes Phenyl ist, wie Phenyloxycarbonyl,
Arylniederalkoxycarbonyl, z. B. Arylmethoxy-carbonyl, worin Aryl 6 bis 14 Kohlenstoffatome hat und beispielsweise Phenyl, Biphenylyl, 1- oder 2-Naphthyl, Fluorenyl oder durch Niederalkyl, z. B. Methyl oder tert-Butyl, Hydroxy, Niederalkoxy, z. B. Methoxy, Ethoxy oder tert-Butoxy, Halogen, z. B. Chlor oder Brom, und/oder Nitro mono-oder mehrfach substituiertes Phenyl ist, z. B. Phenylniederalkoxycarbonyl, wie Benzyloxycarbonyl, 4-Methoxybenzyloxycarbonyl, 4-Nitrobenzyloxycarbonyl, Diphenylniederalkoxycarbonyl, wie Diphenylmethoxycarbonyl, Di-(4-methoxyphenyl)-methoxycarbonyl, Trityloxycarbonyl oder Fluorenylniederalkoxycarbonyl, wie 9-Fluorenylmethoxycarbonyl, besonders bevorzugt Benzyloxycarbonyl,
Heterocyclylniederalkoxycarbonyl, worin Heterocyclyl vorzugsweise aus Pyrrolyl, Furyl, Thienyl, Imidazolyl, Pyrazolyl, Oxazolyl, Thiazolyl, Pyridyl, Pyrazinyl, Pyrimidinyl, Indolyl, Chinolyl, Isochinolyl, Chinoxalinyl, β-Carbolinyl und einem benzanellierten, cyclopenta-, cyclohexa- oder cyclohepta-anellierten Derivat dieser Reste, die auch ganz oder teilweise gesättigt vorliegen können, ausgewählt ist, wie 2-Tetrahydrofuranylniederalkoxycarbonyl, wie 2-Tetrahydrofuranyl-methoxycarbonyl oder 2-, 3- oder 4-Pyridylmethoxycarbonyl,
2-Triniederalkylsilylniederalkoxycarbonyl, wie 2-Trimethylsilyloxycarbonyl, oder
2-Triarylsilylniederalkoxycarbonyl, worin Aryl Phenyl oder 1- oder 2-Naphthyl ist, wie Triphenylsilylethoxycarbonyl.

Bevorzugte Acylgruppen R₁, R₂, R₈ und R₉ einer unsubstituierten oder substituierten Carbaminsäure sind neben geeigneten bereits als bevorzugte Acylgruppen R₁, R₂, R₈ und R₉ genannten Resten
Carbamoyl oder
unsubstituiertes oder substituiertes N-Alkyl- oder N,N-Dialkylcarbamoyl, worin der Alkylrest bis zu 12 Kohlenstoffatome aufweist, vorzugsweise unsubstituiertes oder substituietes Niederalkyl- oder Diniederalkylcarbamoyl, wie Methyl-, Ethyl-, Propyl-, tert-Butyl-, Dimethyl, Diethyl oder Di-n-propyl-carbamoyl, wobei die Substituenten aus Phenyl, z. B. in Benzylcarbamoyl, N-Phenylniederalkyl-N-niederalkylcarbarnoyl, wie N-Benzyl-N-methylcarbamoyl, oder Dibenzylcarbamoyl, Heterocyclyl, welches wie unter Heterocyclylniederalkanyol R₁, R₂, R₈ und R₉ definiert ist, vorzugsweise ausgewählt aus Thienyl, Furyl, Pyranyl, Pyrrolyl, Imidazolyl, Pyrazolyl, Oxazolyl, Isoxazolyl, Thiazolyl, Furazanyl, Pyridyl, Pyrazinyl, Pyrimidinyl, Pyridazinyl, Azepinyl, Indolyl, Benzimidazolyl, 1H-Indazolyl, Chinolyl, Isochinolyl, Chinoxalinyl, Chinazolinyl, Cinnolyl, Purinyl, Pteridinyl, Naphthyridinyl, 4H-Chinolizinyl, 3,1-Benzfuranyl, Benz[e]indolyl, 4,1-Benzoxazinyl, 4,1-Benzthiazinyl, Carbazolyl, β-Carbolinyl, Phenazinyl, Phenanthridyl, Acridyl, Phenoxazinyl, Phenothiazinyl, 1-Azaacenaphtenyl, Cyclohexa[b]pyrrolyl, Cyclohepta[b]pyrrolyl, Cyclohexa[d]pyrazolyl, Cyclohexa[b]pyridyl, Cyclohexa[b]pyrazinyl, Cyclohexa[b]pyrimidinyl, Cyclohexa[b]-1,4-oxazinyl, Cyclohexa[b]-1,4-thiazinyl, Pyrrolidinyl, Pyrrolinyl, Imidazolidinyl, 2-Imidazolinyl, 2,3-Dihydropyridyl, Piperidyl, Piperazinyl, 2,3,5,6-Tetrahydropyrazinyl, Morpholinyl, Thiomorpholinyl, S,S-Dioxothiomorpholinyl, Indolinyl, Isoindolinyl, 4,5,6,7-Tetrahydroindolyl, 1,2,3,4-Tetrahydrochinolyl, 1,2,3,4-Tetrahydroisochinolyl, Chromanyl, Thiochromanyl, 1,2,3,4-Tetrahydro-3,1-benzdiazinyl, 3,4-Dihydro-3H-4,1-benzoxazinyl, 3,4-Dihydro-3H-4,1-benzthiazinyl, 2,3,4,5-Tetrahydro-1H-5,1-benzazepinyl und 5,6-Dihydrophenanthridinyl, wobei die genannten Reste unsubstituiert oder durch Niederalkyl, z. B. Methyl, Phenyl, 1- oder 2-Naphthyl, Phenylniederalkyl, z. B. Benzyl, Hydroxyniederalkyl, z. B. Hydroxymethyl oder 2-Hydroxyethyl, Niederalkoxyniederalkyl, z. B. Methoxymethyl oder 2-Methoxyethyl, Phenoxy- oder Naphthoxyniederalkyl, z. B. 2-Phenoxyethyl, 1- oder 2-Naphthoxymethyl, Phenylniederalkoxy- oder Naphthylniederalkoxy-niederalkyl, z. B. Benzyloxyniederalkyl, Niederalkanoyloxy-niederalkyl, z. B. Acetyloxymethyl, Phenyl- oder Napthyl-nieder-alkanoyloxy-niederalkyl, z. B. Benzoyloxy-, Phenylacetyloxy- oder 1-oder 2-Naphthoyl-oxy-methyl, -2-ethyl oder -2-(2,2-dimethylethyl), Niederalkoxycarbonyloxy-niederalkyl, z. B. tert-Butyloxycarbonyloxyniederalkyl, Phenyl-, Napthyl- oder Fluorenylniederalkoxy-carbonyloxy-niederalkyl, z. B. 2-Benzyloxycarbonyloxyethyl oder 9-Fluorenylmethoxycarbonyloxyethyl, Aminoniederalkyl, z. B. Aminomethyl, 2-Aminoethyl oder 2-Aminopropyl, Carboxyniederalkyl, z. B. Carboxymethyl oder 2-Carboxyethyl, Hydroxy, Niederalkoxy, z. B. Methoxy oder Ethoxy, Phenyl- oder Naphthylniederalkoxy, z. B. Benzyloxy oder 1- oder 2-Naphthylmethoxy, Amino, Niederalkylamino, z. B. Methyl-, Ethyl- oder tert-Butylamino, Diniederalkylamino, z. B. Dimethyl- oder Diethylamino, Carboxy, Niederalkoxycarbonyl, z. B. Methoxy-, Isopropoxy-, sec-Butoxy- oder tert-Butoxy-carbonyl, Phenyl- oder Napthylniederalkoxycarbonyl, z. B. Benzyloxycarbonyl, Halogen, z. B. Fluor, Chlor, Brom oder Jod, insbesondere Chlor oder Brom, Nieder-alkanoyl, z. B. Acetyl oder Pivaloyl, Niederalkylsulfonyl, z. B. Methyl- oder Ethylsulfonyl, Phosphono, Hydroxy-niederalkoxyphosphoryl oder Dialkoxy-phosphoryl, z. B. Dimethoxy- oder Diethoxyphosphoryl, Carbamoyl, Mono- oder Diniederalkylcarbamoyl, z. B. N-Methylcarbamoyl, N-n-Butylcarbamoyl oder N,N-Dimethylcarbamoyl, Hydroxy- oder Carboxyniederalkylcarbamoyl, z. B. Hydroxy- oder Carboxymethyl-carbamoyl oder Hydroxy- oder Carboxyethylcarbamoyl, Sulfamoyl, durch Nitro, Oxo und/oder durch Cyano substituiert sind, insbesondere Pyridyl, wie 2-, 3-oder 4-Pyridyl, in erster Linie in N-Heterocyclylniederalkyl-N-niederalkylcarbamoyl, z. B. N-Pyridylniederalkyl-N-niederalkyl-carbamoyl, wie N-(2-, 3- oder 4-Pyridylmethyl)-N-methyl-carbamoyl, oder in N-Heterocylclyl-niederalkyl-carbamoyl, z. B. 2- oder 3-Pyridylniederalkylaminocarbonyl, wie 2- oder 3-Pydylmethylamino-carbonyl, Hydroxy, z. B. in Hydroxymethyl, 2-Hydroxyethyl, 3-Hydroxypropyl, 2-Hydroxypropyl, N-Dihydroxyniederalkyl, wie 2,3-Dihydroxy-n-propyl oder 2-Hydroxy-2,2-dimethylethyl, Niederalkoxy, bevorzugt in Niederalkoxyniederalkyl, z. B. Methoxymethyl oder 2-Methoxyethyl, Niederalkanoyloxy, bevorzugt in Niederalkanoyloxyniederalkyl, z. B. Niederalkanoyloxymethyl oder Niederalkanoyloxyethyl, wie Acetoxymethyl, 2-Acetoxyethyl, 3-Propionyloxymethyl, 2-Propionyloxyethyl, 4-Butyroxymethyl oder 2- Butyroxyethyl, Aryloxy oder Aryloxy und Hydroxy mit Aryl von 6 - 14 Kohlenstoffatomen, wie Phenyl, Naphthyl oder Fluorenyl, bevorzugt in Aryloxyniederalkyl oder Aryloxyhydroxyniederalkyl, wie Phenyloxymethyl, 2-Phenyloxyethyl, 1- oder 2-Naphthyloxymethyl oder 1- oder 2-Naphthyloxy-ethyl, oder 2-Phenyl-2-hydroxyethyl, wobei Aryl unsubstituiert oder beispielsweise mit Niederalkyl, z. B. Methyl, Ethyl, Propyl, Isopropyl, Butyl, sec-Butyl oder tert-Butyl, mit Hydroxy, mit Niederalkoxy, z. B. Methoxy, Ethoxy, Propoxy, Isopropoxy, Butoxy, sec-Butoxy oder tert-Butoxy, mit Carboxy, mit Niederalkoxycarbonyl, z. B. Isopropoxycarbonyl, sec-Butoxycarbonyl oder tert-Butoxycarbonyl, oder mit Carbamoyl, Niederalkyl-oder Diniederalkyl-carbamoyl und/oder Mono- oder Di-(hydroxy- oder carboxyniederalkyl)carbamoyl ein- oder zweifach substituiert ist und die genannten Substituenten in verschiedenen Ringstellungen vorliegen können, z. B. als 4-Methylphenyloxy, 2,4,5-Trimethylphenyloxy, 4-Hydroxyphenyloxy, 4- Methoxyphenyloxy, 3,5-Dimethoxyphenyloxy, 2-Carboxyphenyloxy, 2-tert-Butoxycarbonylphenyloxy, 2- oder 4-Carbamoylphenyloxy, Carbamoyl, Carboxyniederalkylcarbamoyl oder Hydroxyniederalkyl-carbamoyl, wie in 4-Carbamoyl-n-butyl, 7-Carbamoyl-n-heptyl, 2-Hydroxyethylcarbamoyl-n-butyl oder 4-(Tris[hydroxymethyl]-methyl)-carbamoyl-n-butyl, ferner Amino, z. B. in 2-Aminoethyl oder 3-Aminopropyl, Niederalkylamino, z. B. in Methyl-oder Ethylaminomethyl, Diniederalkylamino, z. B. in Dimethylaminomethyl, Halogen, insbesondere Fluor, Chlor oder Brom, z. B. in 2,2,2-Trichlorethyl, Sulfo, z. B. in Sulfomethyl oder 4-Sulfobutylamino, und Sulfamoyl, z. B. in 2-Sulfamoylethyl, ausgewählt sind.

Bevorzugte Acylgruppen R₁, R₂, R₈ und R₉ eines unsubstituierten oder substituierten N-substituierten Oxalamids sind Oxamoyl oder Niederalkyloxamoyl, wie Methyl-oder Ethyloxamoyl.

Bevorzugte Acylgruppen R₁, R₂, R₈ und R₉ einer unsubstituierten oder substituierten Aminosäure werden gebildet durch die Aminosäurereste einer α- oder β-Aminosäure, insbesondere
einer natürlichen α-Aminosäure mit der L-Konfiguration, wie sie normalerweise in Proteinen vorkommen, oder eines Epimeren einer solchen Aminosäure, d. h. mit der unnatürlichen D-Konfiguration, oder deren D,L-Isomerengemisch, eines Homologen einer solchen Aminosäure, z. B. worin die Aminosäurenseitenkette um eine oder zwei Methylengruppen verlängert oder verkürzt ist, worin die Aminogruppe in β-Stellung vorliegt und/oder worin eine Methylgruppe durch Wasserstoff ersetzt ist, einer substituierten aromatischen Aminosäure, worin der aromatische Rest 6 - 14 Kohlenstoffatome hat, z. B. eines substituierten Phenylalanins oder Phenylglycins, worin der Phenyl-Substituent Niederalkyl, z. B. Methyl, Hydroxy, Niederalkoxy, z. B. Methoxy, Niederalkanoyloxy, z B. Acetoxy, Amino, Niederalkylamino, z. B. Methylamino, Diniederalkylamino, z. B. Dimethylamino, Niederalkanoylamino, z. B. Acetylamino oder Pivaloylamino, Niederalkoxycarbonylamino, z. B. tert-Butoxycarbonylamino, Arylmethoxycarbonyl-amino, worin Aryl vorzugsweise 6 - 14 Kohlenstoffatome hat, z. B. Benzyloxy-carbonylamino oder 9-Fluorenylmethoxycarbonylamino, Halogen, z. B. Fluor, Chlor, Brom oder Jod, Carboxy und/oder Nitro sein kann und ein- oder mehrfach vorkommt, eines benzannelierten Phenylalanins oder Phenylglycins, wie α-Naphthylalanins, oder eines hydrierten Phenylalanins oder Phenylglycins, wie Cyclohexylalanins oder Cyclohexylglycins.

Diese Aminosäuren können an freien Amino- oder Hydroxyfunktionen, vorzugsweise an einer freien Aminofunktion, durch einen der oben unter Acyl R₁ als Acylgruppe einer Carbonsäure, eines Halbesters der Kohlensäure, einer unsubstituierten oder N-substituierten Carbaminsäure oder eines unsubstituierten oder N-substituierten Oxalamids genannten Reste oder einen der unten unter unsubstituiertes oder substituiertes Alkyl, Arylniederalkyl, Heterocyclyl, Heterocyclylniederalkyl, Sulfo, durch Alkyl, Aryl, Arylniederakyl, Heterocyclylniederalkyl, Alkoxy, Aryloxy, Arylniederakoxy oder Heterocyclylniederalkoxy substituiertes Sulfonyl, durch ein oder zwei unabhängig voneinander aus Alkyl, Cycloalkyl, Cycloalkylniederalkyl, Aryl, Arylniederalkyl, Hydroxy, Alkoxy, Cycloalkoxy, Cycloalkylniederalkoxy, Aryloxy und Arylniederalkoxy ausgewählte Reste substituiertes Phosphoryl R₁, R₂, R₈ oder R₉ oder Sulfamoyl R₁, R₂, R₈ oder R₉, das am Stickstoff unsubstituiert oder substituiert ist, oder einen der im Abschnitt über Verfahren als Schutzgruppen genannten Reste substituiert sein.

Besonders bevorzugt ist das über eine Carboxygruppe gebundene Radikal einer Aminosäure ausgewählt aus Glycin (H-Gly-OH), Alanin (H-Ala-OH), Valin (H-Val-OH), Norvalin (α-Aminovaleriansäure), Leucin, (H-Leu-OH), Isoleucin (H-Ile-OH), Norleucin (α-Aminohexansäure, H-Nle-OH), Serin (H-Ser-OH), Homoserin (α-Amino-γ-hydroxybutter-säure), Threonin (H-Thr-OH), Methionin (H-Met-OH), Cystein (H-Cys-OH), Prolin (H-Pro-OH), trans-3- und trans-4-Hydroxyprolin, Phenylalanin (H-Phe-OH), Tyrosin (H-Tyr-OH), 4-Aminophenylalanin, 4-Chlorphenylalanin, 4-Carboxyphenylalanin, β-Phenylserin (β-Hydroxyphenylalanin), Phenylglycin, α-Naphthylalanin (H-Nal-OH), Cyclohexylalanin (H-Cha-OH), Cyclohexylglycin, Tryptophan (H-Trp-OH), Indolin-2-carbonsäure, 1,2,3,4-Tetrahydroisochinolin-3-carbonsäure, Asparaginsäure (H-Asp-OH), Asparagin (H-Asn-OH), Aminomalonsäure, Aminomalonsäure-monoamid, Glutaminsäure (H-Glu-OH), Glutamin (H-Gln-OH), Histidin (H-His-OH), Arginin (H-Arg-OH), Lysin (H-Lys-OH), δ-Hydroxylysin, Omithin (α,δ-Diaminovaleriansäure), 3-Aminopropansäure, α,γ-Diaminobuttersäure und α,β-Diaminopropionsäure, besonders bevorzugt der Rest einer aliphatischen Aminosäure ausgewählt aus Valin, Alanin, Leucin und Isoleucin, oder einer Aminosäure ausgewählt aus Glycin, Glutaminsäure und Asparagin, wobei (ausser Glycin) jede der genannten Aminosäuren in der D-, L- oder (D,L)-, vorzugsweise (ausser Val, das auch in der (D)- oder (D,L)-Form vorliegen kann) in der L-Form vorliegen kann,
die α-Aminogruppe unsubstituiert oder ein- oder zweifach N-alkyliert, z. B. durch Niederalkyl, wie Methyl oder n-Propyl, durch Aminoniederalkyl, wie 3-Aminopropyl, durch Phenyl- oder Naphthylaminoniederalkyl, wie 3-Phenylaminopropyl, oder durch Niederalkyl, wie Methyl, am Stickstoff substituiertes Piperazinylcarbonylniederalkyl, wie 4-Methylpiperazinylcarbonylmethyl, oder N-acyliert, z. B. durch Niederalkanoyl, wie Acetyl, durch Arylniederalkanoyl, worin Aryl ausgewählt ist aus Phenyl, Indenyl, Indanyl, Naphthyl, Anthryl, Phenanthryl, Acenaphthyl und Fluorenyl, und unsubstituiert oder insbesondere durch Niederalkyl, z. B. Methyl, Ethyl oder Propyl, Halogenniederalkyl, z. B. Trifluormethyl, Phenyl, 1- oder 2-Naphthyl, Hydroxy, Niederalkoxy, z. B. Methoxy, Carbamoylniederalkoxy, N-Niederalkylcarbamoylniederalkoxy oder N,N-Di-niederalkylcarbamoylniederalkoxy, Amino, Mono- oder Diniederalkylamino, Nieder-alkanoylamino, z. B. Pivaloylamino, Halogen, z. B. Fluor, Chlor oder Brom, Carboxy, Niederalkoxycarbonyl, wie tert-Butoxycarbonyl, Phenyl-, Naphthyl- oder Fluorenylniederalkoxycarbonyl, wie Benzyloxycarbonyl, Niederalkanoyl, Sulfo, Niederalkylsulfonyl, z. B. Methylsulfonyl, Phosphono, Hydroxy-niederalkoxy-phosphoryl oder Diniederalkoxyphosphoryl, Carbamoyl, Mono- oder Diniederalkylcarbamoyl, Sulfamoyl, Mono- oder Diniederalkylaminosulfonyl, Nitro und/oder Cyano ein- oder bis zu dreifach substituiert sein kann, wobei Phenyl bis zu dreimal vorliegen kann, z. B. in Diphenyl-, Dibenzyl- oder Triphenylniederalkanoyl, wie Diphenyl-, Dibenzyl- oder Triphenylacetyl, und Niederalkanoyl unsubstituiert oder substituiert z. B. durch Niederalkyl, z. B. Methyl, Heterocyclyl ausgewählt aus Pyrrolyl, Furyl, Thienyl, Imidazolyl, Pyrazolyl, Oxazolyl, Thiazolyl, Pyridyl, Pyrazinyl, Pyrimidinyl, Indolyl, Chinolyl, Isochinolyl, Chinoxalinyl, β-Carbolinyl und einem benz-, cyclopenta-, cyclohexa- oder cyclohepta-anellierten Derivat dieser Reste, die auch ganz oder teilweise gesättigt vorliegen können, Hydroxy, Niederalkoxy, Niederalkanoyloxy, z. B. Acetoxy, Propionyloxy, Butyroxy, Isobutyroxy oder Pivaloyloxy, Acetoacetoxy, Amino- oder Benzyloxycarbonylami-no-niederalkanoyloxy, z. B. 2-Amino- oder 2-Benzyloxycarbonylamino-2-methylpro-pionyloxy, Arylniederalkanoyloxy, worin Aryl 6 - 10 Kohlenstoffatome hat, z. B. in Benzoyloxy, Phenylacetyloxy, 1- oder 2-Naphthoyloxy, Niederalkoxycarbonyloxy, z. B. Methoxy-, Ethoxy-, n-Propoxy-, Isopropoxy-, n-Butoxy-, Isobutoxy-, sec-Butoxy-, tert-Butoxy-, n-Pentoxy-, Isopentoxy-, Neopentoxy-, tert-Pentoxy-, n-Hexoxy-, Isohex-oxy- oder n- Heptoxy-carbonyloxy, Mono- oder Diniederalkyl-aminocarbonyloxy, z. B. Ethylaminocarbonyloxy oder Diethylaminocarbonyloxy, Aryloxycarbonyloxy, worin Aryl 6 - 10 Kohlenstoffatome hat, z. B. Phenyloxycarbonyloxy oder 1- oder 2-Naphthyloxycarbonyloxy, Arylniederalkoxycarbonyloxy mit Aryl von 6 - 14 Kohlenstoffatomen, insbesondere Phenylniederalkoxycarbonyloxy, z. B. Benzyloxycarbonyloxy, ferner 1- oder 2-Naphthylmethoxycarbonyloxy oder 9-Fluorenylmethoxycarbonyloxy, Sulfonyloxy, Niederalkylsulfonyloxy, z. B. Methyl-, Ethyl-, Propyl-, Isopropyl-, n-Butyl-, Isobutyl-, sec-Butyl-, tert-Butyl-, n-Pentyl-, Isopentyl-, Neopentyl-, tert-Pentyl-, n-Hexyl-, Isohexyl- oder n-Heptylsulfonyloxy, oder Phenylsulfonyloxy, 2- oder 4-Toluolsulfonyloxy oder 1- oder 2- Naphthylsulfonyloxy, Carboxy, verestertes Carboxy ausgewählt aus Niederalkoxycarbonyl, z. B. Methoxy-, Ethoxy-, n-Propoxy-, Isopropoxy-, n-Butoxy-, Isobutoxy-, sec-Butoxy-, tert-Butoxy-, n-Pentoxy-, Isopentoxy-, Neopentoxy-, tert-Pentoxy-, n-Hexoxy-, Isohexoxy- oder n- Heptoxycarbonyl, Aryloxycarbonyl, worin Aryl 6 - 10 Kohlenstoffatome hat, z. B. Phenyloxycarbonyl oder 1- oder 2-Naphthyloxycarbonyl, Arylniederalkoxycarbonyl, worin Aryl 6 - 12 Kohlenstoffatome hat, z. B. Benzyloxy-carbonyl, 1- oder 2-Naphthylmethyloxycarbonyl oder 9-Fluorenylmethoxycarbonyl, Niederalkanoyl, Niederalkylsulfonyl, z. B. Methyl- oder tert-Butylsulfonyl, Hydroxy-niederalkoxyphosphoryl und Diniederalkoxyphosphoryl, Carbamoyl, durch einen oder zwei aus Niederalkyl, wie Methyl, Ethyl, Propyl, Isopropyl, n-Butyl, Isobutyl, sec-Butyl, tert-Butyl, n-Pentyl, Isopentyl, Neopentyl, tert-Pentyl, n-Hexyl, Isohexyl oder n-Heptyl, z. B. in N-Methylcarbamoyl, N-n-Butylcarbamoyl oder N,N-Dimethylcarba-moyl, aus Carboxyniederalkyl oder Niederalkoxycarbonyl-niederalkyl, z. B. als Carboxymethylcarbamoyl (Glycinylcarbonyl) oder als tert-Butoxycarbonylmethyl-carbamoyl, aus Diniederalkylaminoniederalkyl, z. B. 2-Dimethylaminoethyl, Amino-carboxyniederalkyl, z. B. 5-Amino-5-carboxypentyl, aus Hydroxyniederalkyl, z. B. Hydroxymethyl oder Hydroxyethyl, und aus Diniederalkoxyniederalkyl, z. B. 2-(2,2-Dimethoxyethyl), ausgewählte Reste, oder einen aus Ethylen, Trimethylen, Tetramethylen und Pentamethylen, worin ein Kohlenstoffatom durch Stickstoff, Sauerstoff, Schwefel und mit Sauerstoff ein- oder zweifach substituierten Schwefel ersetzt sein kann und das ungesättigt sein kann, ausgewählten Rest, wie als Piperidino-, pyrazin-1-yl-, Piperazin-1-yl-, Pyrimidin-1-yl-, Pyridazin-1-yl-, Morpholino-, Thiomorpholino- oder S,S-Di-oxothiomorpholinocarbonyl, substituiertes Carbamoyl, Sulfamoyl, Phosphono, Benzo-furanyl, Oxo und/oder Cyano sein kann und unverzweigt oder verzweigt ist, vorzugsweise durch Arylniederalkanoyl, worin Aryl 6 - 14 Kohlenstoffatome hat und unsubstituiert oder substituiert z. B. mit Niederalkanoyl, wie in 2-Benzyl-3-pivaloylpropionyl, oder mit Niederalkysulfonyl, wie in 2-Benzyl-3-tertbutylsulfonylpropionyl, wobei Phenylniederalkanoyl, z. B. Phenylacetyl, besonders bevorzugt ist, durch Heterocyclylniederalkanoyl ausgewählt aus Thienyl-, Furyl-, Pyranyl-, Pyrrolyl-, Imidazolyl-, Pyrazolyl-, Oxazolyl-, Isoxazolyl-, Thiazolyl-, Furazanyl-, Pyridyl-, Pyrazinyl-, Pyrimidinyl-, Pyridazinyl-, Azepinyl-, Indolyl-, Benzimidazolyl-, 1H-Indazolyl-, Chinolyl-, Isochinolyl-, Chinoxalinyl-, Chinazolinyl-, Cinnolyl-, Purinyl-, Pteridinyl-, Naphthyridinyl-, 4H-Chinolizinyl-, 3,1-Benzfuranyl-, Benz[e]indolyl-, 4,1-Benzoxazinyl-, 4,1-Benzthiazinyl-, Carbazolyl-, β-Carbolinyl-, Phenazinyl-, Phenanthridyl-, Acridyl-, Phenoxazinyl-, Phenothiazinyl-, 1-Azaacenaphtenyl-, Cyclohexa[b]pyrrolyl-, Cyclohepta[b]pyrrolyl-, Cyclohexa[d]pyrazolyl-, Cyclohexa[b]pyridyl-, Cyclohexa[b]pyrazinyl-, Cyclohexa[b]pyrimidinyl-, Cyclohexa[b]-1,4-oxazinyl-, Cyclohexa[b]-1,4-thiazinyl-, Pyrrolidinyl-, Pynolinyl-, Imidazolidinyl-, 2-Imidazolinyl-, 2,3-Dihydropyridyl-, Piperidyl-, Piperazinyl-, 2,3,5,6-Tetrahydropyrazinyl-, Morpholinyl-, Thiomorpholinyl-, S,S-Dioxothiomorpholinyl-, Indolinyl-, Isoindolinyl-, 4,5,6,7-Tetrahydroindolyl-, 1,2,3,4-Tetrahydrochinolyl-, 1,2,3,4-Tetrahydroisochinolyl-, Chromanyl-, Thiochromanyl-, 1,2,3,4-Tetrahydro-3,1-benzdiazinyl-, 3,4-Dihydro-3H-4,1-benzoxazinyl-, 3,4-Dihydro-3H-4,1-benzthiazinyl-, 2,3,4,5-Tetrahydro-1H-5,1-benzazepinyl- und 5,6-Dihydrophenanthridinyl-niederalkanoyl, wobei die genannten Heterocylyl-Reste unsubstituiert oder durch Niederalkyl, z. B. Methyl, Phenyl, 1- oder 2-Naphthyl, Phenylniederalkyl, z. B. Benzyl, Hydroxyniederalkyl, z. B. Hydroxymethyl oder 2-Hydroxyethyl, Niederalkoxyniederalkyl, z. B. Methoxymethyl oder 2-Methoxyethyl, Phenoxy- oder Naphthoxyniederalkyl, z. B. 2-Phenoxyethyl, 1- oder 2-Naphthoxymethyl, Phenylniederalkoxy- oder Naphthylniederalkoxy-niederalkyl, z. B. Benzyloxyniederalkyl, Niederalkanoyloxy-niederalkyl, z. B. Acetyloxymethyl, Phenyl- oder Napthyl-niederalkanoyloxy-niederalkyl, z. B. Benzoyloxy-, Phenylacetyloxy- oder 1- oder 2-Naphthoyloxy-methyl, -2-ethyl oder -2-(2,2-dimethylethyl), Niederalkoxycarbonyloxy-niederalkyl, z. B. tert-Butyloxyniederalkyl, Phenyl-, Napthyl- oder Fluorenylniederalkoxycarbonyloxy-niederalkyl, z. B. 2-Benzyloxycarbonyloxyethyl oder 9-Fluorenylmethoxycarbonyloxyethyl, Aminoniederalkyl, z. B. Aminomethyl, 2-Aminoethyl oder 2-Aminopropyl, Carboxyniederalkyl, z. B. Carboxymethyl oder 2-Carboxyethyl, Hydroxy, Niederalkoxy, z. B. Methoxy oder Ethoxy, Phenyl- oder Naphthylniederalkoxy, z. B. Benzyloxy oder 1- oder 2-Naphthylmethoxy, Amino, Niederalkylamino, z. B. Methyl-, Ethyl- oder tert-Butylamino, Diniederalkylamino, z. B. Dimethyl- oder Diethylamino, Carboxy, Niederalkoxycarbonyl, z. B. Methoxy-, Isopropoxy-, sec-Butoxy- oder tert-Butoxy-carbonyl, Phenyl- oder Napthylniederalkoxycabonyl, z.B. Benzyloxycarbonyl, Halogen, z. B. Fluor, Chlor, Brom oder Jod, insbesondere Chlor oder Brom, Niederalkanoyl, z. B. Acetyl oder Pivaloyl, Niederalkylsulfonyl, z. B. Methyl-oder Ethylsulfonyl, Phosphono, Hydroxy-niederalkoxyphosphoryl oder Dialkoxy-phosphoryl, z. B. Dimethoxy- oder Diethoxyphosphoryl, Carbamoyl, Mono- oder Diniederalkylcarbamoyl, z. B. N-Methylcarbamoyl, N-n-Butylcarbamoyl oder N,N-Dimethylcarbamoyl, Hydroxy- oder Carboxyniederalkylcarbamoyl, z. B. Hydroxy- oder Carboxymethylcarbamoyl oder Hydroxy- oder Carboxyethylcarbamoyl, Sulfamoyl, durch Nitro, Oxo und/oder durch Cyano substituiert sind, z. B. Pyridylcarbonyl, wie 2-, 3-oder 4-Pyridylcarbonyl, 3,4-Dihydroxypyrrolidinylcarbonyl, N-Benzyloxycarbonyl-piperidin-4-ylcarbonyl, 1-Methylpiperazin-4-ylcarbonyl, Morpholinocarbonyl, Thiomorpholinocarbonyl, S,S-Dioxothiomorpholinocarbonyl, Indol-2-ylcarbonyl, Chinol-2-ylcarbonyl, Pyridylacetyl, wie 2- oder 3-Pyridylacetyl, Imidazolylacetyl, wie Imidazol-1-ylacetyl, Morpholinylacetyl, wie Morpholinoacetyl, Pyridylpropionyl, wie 3-(2- oder 3-Pyrridyl)Propionyl, Pyrrolidinylpropionyl, wie 3-(4-Pyrrolidinyl)propionyl, Morpholinylpropionyl, wie 3-Morpholinopropionyl, oder durch Heterocyclylniederalkenoyl, worin Heterocyclyl insbesondere ausgewählt ist aus Pyrrolyl, Furyl, Thienyl, Imidazolyl, Pyrazolyl, Oxazolyl, Thiazolyl, Pyridyl, Pyrazinyl, Pyrimidinyl, Indolyl, Chinolyl, Isochinolyl, Chinoxalinyl, β-Carbolinyl und einem benzanellierten, cyclopenta-, cyclohexa- oder cyclohepta-anellierten Derivat dieser Reste, die auch ganz oder teilweise gesättigt sein können, z. B. Pyrrolidylniederalkenoyl, wie N-Pyrrolidyl-acryloyl, Halogenniederalkanoyl, welches bis zu 3 Halogenatome enthält, z. B. α-Halogenacetyl, wie α-Fluor-, α-Chlor-, α-Brom-, α-Iod-, α,α,α-Trifluor- oder α,α,α-Trichloracetyl, oder Halogenpropionyl, wie β-Chlor- oder β-Brompropionyl, durch Niederalkoxycarbonyl, wie tert-Butoxycarbonyl, durch Arylniederalkoxycarbonyl, worin Aryl 6 - 14 Kohlenstoffatome hat und z. B. ausgewählt ist aus Phenyl, Naphthyl und Fluorenyl, z. B. Phenylniederalkoxycarbonyl, wie Benzyloxycarbonyl, oder 9-Fluorenylmethoxycarbonyl, durch Heterocyclylniederalkoxycarbonyl, worin Heterocyclyl insbesondere ausgewählt ist aus Pyrrolyl, Furyl, Thienyl, Imidazolyl, Pyrazolyl, Oxazolyl, Thiazolyl, Pyridyl, Pyrazinyl, Pyrimidinyl, Indolyl, Chinolyl, Isochinolyl, Chinoxalinyl, β-Carbolinyl und einem benzanellierten, cyclopenta-, cyclohexa- oder cyclohepta-anellierten Derivat dieser Reste, die auch ganz oder teilweise gesättigt und unsubstituiert oder substituiert insbesondere durch Niederalkyl, wie Methyl, sein können, z.B. 1-Methylpyrrolidin-2-yl-methoxycarbonyl, 2-Furylmethoxycarbonyl, Tetrahydrofuranyl-niederalkoxycarbonyl, wie 2-Tetrahydrofuranyl-methoxycarbonyl, 1-Methyl-2-piperidyl-methoxycarbonyl oder 2-Morpholino-ethoxycarbonyl, durch Carboxyniederalkanoyl, wie 3-Carboxypropionyl, 5-Carboxypentanoyl oder 6-Carboxyhexanoyl, durch Niederalkoxycarbonylniederalkanoyl, wie 5-Methoxycarbonylpentanoyl oder 6-Methoxycarbonylhexanoyl, durch Hydroxyniederalkoxy-niederalkanoyl, wie 3-Hydroxy-n-propoxycarbonyl, durch Aminoniederalkanoyl, worin die Aminogruppe nicht in α- oder β-Stellung des Niederalkanoylrestes steht, wie 5-Aminopentanoyl, durch Phenylniederalkoxycarbonylamino-niederalkanoyl, worin die Aminogruppe nicht in α-oder β-Stellung des Niederalkanoylrestes steht, wie 5-Benzyloxycarbonylaminopentanoyl oder 6-Benzyloxycarbonylaminohexanoyl,
durch Carbamoyl, durch Phenylniederalkylaminocarbonyl, wie Benzylaminocarbonyl, durch N-Diniederalkylaminoniederalkyl-N-niederalkylaminocarbonyl, wie N-(2-Dimethylamino)ethyl-N-methylaminocarbonyl, durch N-Dihydroxyniederalkyl-N-niederalkylaminocarbonyl, wie N-(2,3-Dihydroxy-n-propyl)-N-methylaminocarbonyl, durch N-Heterocyclylniederalkyl-N-niederalkylcarbamoyl mit Heterocyclyl ausgewählt aus Thienyl, Furyl, Pyranyl, Pyrrolyl, Imidazolyl, Pyrazolyl, Oxazolyl, Isoxazolyl, Thiazolyl, Furazanyl, Pyridyl, Pyrazinyl, Pyrimidinyl, Pyridazinyl, Azepinyl, Indolyl, Benzimidazolyl, 1H-Indazolyl, Chinolyl, Isochinolyl, Chinoxalinyl, Chinazolinyl, Cinnolyl, Purinyl, Pteridinyl, Naphthyridinyl, 4H-Chinolizinyl, 3,1-Benzfuranyl, Benz[e]indolyl, 4,1-Benzoxazinyl, 4,1-Benzthiazinyl, Carbazolyl, β-Carbolinyl, Phenazinyl, Phenanthridyl, Acridyl, Phenoxazinyl, Phenothiazinyl, 1-Azaacenaphtenyl, Cyclohexa[b]pyrrolyl, Cyclohepta[b]pyrrolyl, Cyclohexa[d]pyrazolyl, Cyclohexa[b]pyridyl, Cyclohexa[b]pyrazinyl, Cyclohexa[b]pyrimidinyl, Cyclohexa[b]-1,4-oxazinyl, Cyclohexa[b]-1,4-thiazinyl, Pyrrolidinyl, Pyrrolinyl, Imidazolidinyl, 2-Imidazolinyl, 2,3-Dihydropyridyl, Piperidyl, Piperazinyl, 2,3,5,6-Tetrahydropyrazinyl, Morpholinyl, Thiomorpholinyl, S,S-Dioxothiomorpholinyl, Indolinyl, Isoindolinyl, 4,5,6,7-Tetrahydroindolyl, 1,2,3,4-Tetrahydoochinolyl, 1,2,3,4-Tetrahydroisochinolyl, Chromanyl, Thiochromanyl, 1,2,3,4-Tetrahydro-3,1-benzdiazinyl, 3,4-Dihydro-3H-4,1-benzoxazinyl, 3,4-Dihydro-3H-4,1-benzthiazinyl, 2,3,4,5-Tetrahydro-1H-5,1-benzazepinyl und 5,6-Dihydrophenanthridinyl, wobei die genannten Reste unsubstituiert oder durch Niederalkyl, z. B. Methyl, Phenyl, 1- oder 2-Naphthyl, Phenylniederalkyl, z. B. Benzyl, Hydroxyniederalkyl, z. B. Hydroxymethyl oder 2-Hydroxyethyl, Niederalkoxyniederalkyl, z. B. Methoxymethyl oder 2-Methoxyethyl, Phenoxy- oder Naphthoxyniederalkyl, z. B. 2-Phenoxyethyl, 1- oder 2-Naphthoxymethyl, Phenylniederalkoxy- oder Naphthylniederalkoxy-niederalkyl, z. B. Benzyloxyniederalkyl, Niederalkanoyloxy-niederalkyl, z. B. Acetyloxymethyl, Phenyl- oder Napthyl-niederalkanoyloxy-niederalkyl, z. B. Benzoyloxy-, Phenylacetyloxy- oder 1- oder 2-Naphthoyloxy-methyl, -2-ethyl oder -2-(2,2-dimethylethyl), Niederalkoxycarbonyloxy-niederalkyl, z. B. tert-Buryloxycarbonyloxyniederalkyl, Phenyl-, Napthyl- oder Fluorenylniederalkoxycarbonyloxy-niederalkyl, z. B. 2-Benzyloxycarbonyloxyethyl oder 9-Fluorenylmethoxycarbonyloxyethyl, Aminoniederalkyl, z. B. Aminomethyl, 2-Aminoethyl oder 2-Aminopropyl, Carboxyniederalkyl, z. B. Carboxymethyl oder 2-Carboxyethyl, Hydroxy, Niederalkoxy, z. B. Methoxy oder Ethoxy, Phenyl- oder Naphthylniederalkoxy, z. B. Benzyloxy oder 1- oder 2-Naphthylmethoxy, Amino, Niederalkylamino, z. B. Methyl-, Ethyl- oder tert-Butylamino, Diniederalkylamino, z. B. Dimethyl- oder Diethylamino, Carboxy, Niederalkoxycarbonyl, z. B. Methoxy-, Isopropoxy-, sec-Butoxy- oder tert-Butoxy-carbonyl, Phenyl- oder Napthylniederalkoxycarbonyl, z. B. Benzyloxycarbonyl, Halogen, z. B. Fluor, Chlor, Brom oder Jod, insbesondere Chlor oder Brom, Niederalkanoyl, z. B. Acetyl oder Pivaloyl, Niederalkylsulfonyl, z. B. Methyl- oder Ethylsulfonyl, Phosphono, Hydroxy-niederalkoxyphosphoryl oder Dialkoxy-phosphoryl, z. B. Dimethoxy- oder Diethoxyphosphoryl, Carbamoyl, Mono- oder Diniederalkylcarbamoyl, z. B. N-Methylcarbamoyl, N-n-Butylcarbamoyl oder N,N-Dimethylcarbamoyl, Hydroxy-oder Carboxyniederalkylcarbamoyl, z. B. Hydroxy- oder Carboxymethylcarbamoyl oder Hydroxy- oder Carboxyethylcarbamoyl, Sulfamoyl, durch Nitro, Oxo und/oder durch Cyano substituiert sind, insbesondere Pyridyl, wie 2-, 3- oder 4-Pyridyl z. B. 2- oder 3-Pyridylniederalkylaminocarbonyl, wie 2- oder 3-Pyridylmethylaminocarbonyl, durch N-2-, N-3- oder N-4-Pyridylniederalkyl-N-niederalkylaminocarbonyl, wie N-2-, N-3- oder N-4-Pyridylmethyl-N-methylaminocarbonyl,
durch Heterocyclylniederalkyl-carbamoyl-niederalkanoyl, wie oben für Heterocyclylniederallryl-carbarnoyl-niederalkanoyl R₁, R₂, R₈ oder R₉ definiert, z. B. 2-(N-Morpholinoniederalkyl-carbamoyl)-niederalkanoyl, wie 2(R,S)-(N-(2-Morpholinoethyl)carbamoyl-3-methyl-butyryl, oder 2-(N-(Pyridylniederalkyl)-carbamoyl)-niederalkanoyl, wie (2(R,S)-(N-(2-pyridylmethyl)-carbamoyl)-3-methyl)-butyryl,
durch Sulfonyl, durch Niederalkylsulfonyl, wie Methyl- oder Ethylsulfonyl, durch Arylsulfonyl, worin Aryl 6 - 10 Kohlenstoffatome hat und z. B. aus Phenyl und Naphthyl ausgewählt ist, und unsubstituiert oder insbesondere durch Niederalkyl, wie Methyl, oder Niederalkoxy, wie Methoxy, substituiert ist, wie p-Toluolsulfonyl, durch Sulfamoyl oder durch mit Heterocyclylniederalkyl, worin Heterocyclyl wie zuletzt definiert ist, und/oder durch Niederalkyl substituiertes Sulfamoyl, wie N-2-Pyridylmethyl-N-methylaminosulfonyl, vorliegt
eine Carboxygruppe der Seitenkette in veresterter oder amidierter Form, z. B. als Niederalkylestergruppe, wie Methoxycarbonyl oder tert-Butoxycarbonyl, als Arylestergruppe oder als Arylniederalkylestergruppe, wobei Aryl Phenyl, 4-Nitrophenyl, Naphthyl oder Biphenylyl bedeutet, z. B. als 4-Nitrophenyloxycarbonyl, Benzyloxycarbonyl oder 9-Fluorenylmethoxycarbonyl, oder als Carbamoyl-, als Niederalkylcarbamoyl-, wie Methylcarbamoyl-, als Diniederalkylaminocarbamoyl-, wie Dimethylcarbamoyl-, als Mono oder Di-(hydroxyniederalkyl)carbamoyl-, wie Hydroxymethylcarbamoyl- oder Di-(hydroxymethyl)carbamoyl-, oder als Mono- oder Di-(carboxyniederalkyl)carbamoyl-, wie Carboxymethylcarbamoyl- oder Di-(carboxymethyl)carbamoylgruppe vorliegt,
eine Aminogruppe der Seitenkette in alkylierter Form, z. B. als Mono-oder Diniederalkylamino, wie n-Butylamino oder Dimethylamino, oder in acylierter Form, z. B. als Niederalkanoylamino, wie Acetylamino oder Pivaloylamino, als Aminoniederalkanoylamino, wie 3-Amino-3,3-dimethylpropionylamino, als Arylniederalkanoylamino, worin Aryl 6 - 14 Kohlenstoffatome hat, z. B. als Phenyl, Naphthyl oder Fluorenyl, und unsubstituiert oder durch Niederalkyl, Hydroxy, Niederalkoxy, Carboxy, Carbamoyl oder Sulfamoyl substituiert ist, wie 4-Hydroxyphenylbutyryl, als Niederalkoxycarbonylamino, wie tert-Butoxycarbonylamino, als Arylmethoxycarbonylamino, worin Aryl 6 - 14 Kohlenstoffatome hat, wie als Benzyloxycarbonylamino oder 9-Fluorenylmethoxycarbonylamino, als Piperidyl-1-carbonyl, Morpholinocarbonyl, Thiomorpholinocarbonyl oder als S,S-Dioxothiomorpholinocarbonyl vorliegt, und/oder
eine Hydroxygruppe der Seitenkette in veretherter oder veresterter Form, z. B. als Niederalkoxy-, wie Methoxy oder tert-Butoxy, als Arylniederalkoxy-, wie Benzyloxy, als Niederalkanoyloxy-, wie Acetoxy, oder als Niederalkyloxycarbonyloxygruppe, z. B. tert-Buryloxycarbonyloxygruppe, vorliegt.

Insbesondere sind Acylgruppen R₁, R₂, R₈ und R₉ einer unsubstituierten oder substituierten Aminosäure ausgewählt aus Phenylalanin, N-(Benzyloxycarbonyl)-phenylalanin, N-(2(R,S)-Benzyl-3-pivaloyl-propionyl)-phenylalanin, N-(9-Fluorenylmethoxycarbonyl)-phenylalanin, Tyrosin, N-Propyltyrosin, Tyrosin-O-methylether, N-(3-Amino-3,3-dimethylpropionyl)-tyrosin-O-methylether, N-(2(S)-Benzyl-3-tert-butylsulfonylpropionyl)-tyrosin-O-tert-butylether, N-(9-Fluorenylmethoxycarbonyl)-tyrosin-O-methylether, N-(9-Fluorenylmethoxycarbonyl)-tyrosin-O-tert-butylether, N-Morpholinocarbonylglycin, N-(N-(2-, 3- oder 4-pyridyl)methyl-N-methylaminocarbonyl)-glycin, Valin, N-(3-Phenylaminopropyl)-valin, N-(4-Methylpiperazinylcarbonylmethyl)-valin, N-(Trifluoracetyl)-valin, N-Phenylacetyl-valin, N-Acetyl-valin, N-(3-Phenylpropionyl)-valin, N-(2(R,S)- oder -(2S)-Benzyl-3-pivaloyl-propionyl)-valin, N-(2-Carbamoyl-3-phenylpropionyl)-valin, N-(2(S)-Benzyl-3-tert-butylsulfonylpropionyl)-valin, N-(2(R,S)-Carbamoyl-3-phenylpropionyl)-valin, N-(2-, 3- oder 4-Pyridylcarbonyl)-valin, N-(1-Imidazolylacetyl)-valin, N-(2- oder 3-Pyridylacetyl)-valin, N-(Morpholinoacetyl)-valin, N-(3-(2- oder 3-Pyridyl)-propionyl)-valin, N-(3-(4-Pyrrolidinyl)-propionyl)-valin, N-(3-(Morpholino)-propionyl)-valin, N-(N-Benzyloxycarbonylpiperidin-4-ylcarbonyl)-valin, N-Tetrahydrofurylmethoxycarbonyl-valin, N-(Indol-2-ylcarbonyl)-valin, N-(Chinolin-2-carbonyl)-valin, N-(1-Methylpiperazin-4-ylcarbonyl)-valin, N-(3,4-Dihydroxypyrrolidinylcarbonyl)-valin, N-Methoxycarbonyl-valin, N-tert-Butoxycarbonyl-valin, N-Benzyloxycarbonyl-valin, N-(2-Furylmethoxycarbonyl)valin, N-(1-Methylpyrrolidin-2-yl-methoxycarbonyl)-valin, N-(1-Methyl-2-piperidylmethoxycarbonyl)-valin, N-(1-Methyl-3-piperidyl-methoxycarbonyl)-valin, N-(2-(Morpholino)-ethoxycarbonyl)-valin, N-(3-Carboxypropionyl)-valin, N-(5-Carboxypentanoyl)-valin, N-(6-Carboxyhexanoyl)-valin, N-(5-Methoxycarbonylpentanoyl)-valin, N-(6-Methoxycarbonylhexanoyl)-valin, N-(3-Aminopropionyl)-valin, N-(4-Aminobutyryl)-valin, N-(5-Benzyloxycarbonylaminopentanoyl)-valin, N-(6-Benzyloxycarbonylaminohexanoyl)-valin, N-(Morpholinocarbonyl)-valin, N-(Thiomorpholinocarbonyl)-valin, N-(S,S-Dioxothiomorpholinocarbonyl)-valin, N-(N-Benzylaminocarbonyl)-valin, N-(N-2-Pyridylmethyl-N-methylaminocarbonyl)-valin, N-(N-3-Pyridylmethyl-aminocarbonyl)valin, N-(N-2-Pyridylmethyl-aminocarbonyl)-valin, N-(2-Pyrrolidylacryloyl)-valin, N-Methylsulfonyl-valin, N-(p-Toluolsulfonyl)-valin, N-(4-Methylpiperazinylsulfonyl)-valin, N-(N-(2-Pyridylmethyl)-N-methyl-sulfamoyl)-valin, N-(N-2-Pyridylmethyl-N-methyl-aminocarbonyl)-valin, N-(3-Aminopropyl)-leucin, N-Acetyl-leucin, N-(3-Aminopropionyl)-leucin, N-(2(R,S)- oder N-(2S)-Benzyl-3-pivaloyl-propionyl)-leucin, N-(2(S)-Benzyl-3-tert-butylsulfonylpropionyl)-leucin, N-(2-, 3- oder 4-Pyridylcarbonyl)-leucin, N-(4-Thiomorpholinocarbonyl)-leucin, N-(4-(S,S-Dioxothiomorpholino)carbonyl)-leucin, N-(4-Aminobutyryl)-leucin, N-(3-Hydroxy-n-propoxycarbonyl)-leucin, N-(Benzyloxycarbonyl)-leucin, N-(N-(2-(Dimethylamino)ethyl)-N-methyl-aminocarbonyl)-leucin, N-(N-(2,3-Dihydroxy-n-propyl)-N-methyl-amino-carbonyl)-leucin, N-Acetyl-isoleucin, N-Propionyl-isoleucin, N-(Benzyloxycarbonyl)-isoleucin, N-(2(R,S)-Benzyl-3-pivaloyl-propionyl)-norleucin, N-(2(S)-Benzyl-3-tert-butyl-sulfonylpropionyl)-norleucin, N-(tert-Butoxycarbonyl)-norleucin, N-(tert-Butoxycarbonyl)-serin, N-(Benzyloxycarbonyl)-serin, N-Acetyl-serin-O-methylether, N-(Benzyloxycarbonyl)-serin-O-methylether, N-(2(R,S)-Benzyl-3-pivaloyl-propionyl)-serin, N-Benzyloxycarbonyl-glutaminsäure, Asparagin, N-Benzyloxycarbonyl-asparagin, Chinolin-2-carbonyl-asparagin, und N-(Morpholinocarbonyl)-asparagin bevorzugt, wobei die Aminosäurereste vorzugsweise in der (L)- oder (D,L)-Form, im Falle von Valin auch in der (D)-Form vorliegen.

Unsubstituiertes oder substituiertes Alkyl R₁, R₂, R₈ oder R₉ enthält einen Alkylrest mit 1 -20, vorzugsweise bis zu 10 Kohlenstoffatomen, ist verzweigt oder unverzweigt, kann anstelle einer Methylengruppe ein Heteroatom ausgewählt aus Thia, Aza und Selena enthalten und ist z. B. Methyl, Ethyl, n-Propyl, Isopropyl, n-Butyl, Isobutyl, sec-Butyl, tert-Butyl, n-Pentyl, Isopentyl, Neopentyl, tert-Pentyl, n-Hexyl, Isohexyl, n-Heptyl, n-Octyl, n-Nonyl oder n-Decyl. Bevorzugt ist Niederalkyl, z. B. Methyl, Ethyl, n-Propyl, Isopropyl, n-Butyl, Isobutyl, sec-Butyl, tert-Butyl, n-Pentyl, Isopentyl, Neopentyl, tert-Pentyl, n-Hexyl, Isohexyl oder n- Heptyl, das unsubstituiert oder substituiert ist.

Als Substituenten in substituiertem Alkyl, vorzugsweise substituiertem Niederalkyl, kommen die für Niederalkanoyl R₁, R₂, R₈ und R₉ genannten Reste in Frage.

Substituiertes Niederalkyl ist vorzugssweise
Cycloalkylniederalkyl, worin Cycloalkyl 3 - 7 Kohlenstoffatome hat und Niederalkyl wie oben definiert ist, z. B. Cycloalkylmethyl oder -ethyl, vorzugsweise mit insgesamt 4 - 13 Kohlenstoffatomen, z. B. Cyclopropyl-, Cyclobutyl-, Cyclopentyl- oder Cyclohexylniederalkyl, wie -methyl, oder -ethyl,
Cycloalkenylniederalkyl, z.B. Cycloalkenylmethyl, worin Cycloalkyl vorzugsweise 4 - 8 Kohlenstoffatome hat, wie 1-Cyclohexenylmethyl, 1,4-Cyclohexadienylmethyl oder 1-Cyclohexenylethyl oder 1,4-Cyclohexadienylethyl,
Bicycloalkylniederalkyl, worin Bicylcoalkyl z.B. 5 - 10 Kohlenstoffatome enthält, z.B. Bicycloalkylmethyl oder -ethyl, vorzugsweise mit 8 - 11 Kohlenstoffatomen, wie Decahydronaphthyl-2-methyl, endo- oder exo-Norbornyl-2-methyl, Bicyclo[2.2.2]oct-2-ylmethyl oder Bicyclo[3.3.1]-non-9-ylmethyl, ferner Bicyclohexyl-, -heptyl-, -octyl-, -nonyl- oder -decyl-ethyl oder -3-propyl, z.B. Bicyclo[3.1.0]hex-1-, -2-oder -3-yl-, Bicyclo[4.1.0]hept-1- oder -7-yl-, Bicyclo[2.2.1]hept-2-yl-, z.B. endo- oder - exo-Nor-bornyl-, Bicyclo-[3.2.1]oct-2-yl-, Bicyclo[3.3.0]oct-3-yl- oder Bicyclo[3.3.1]non-9-yl-, ferner α- oder β-Decahydronaphthyl-ethyl oder -3-propyl,
Tricycloalkylniederalkyl, worin Tricycloalkyl z.B. 8 - 10 Kohlenstoffatome enthält, z.B. Tricycloalkylmethyl oder -ethyl, vorzugsweise mit 8 - 11 Kohlenstoffatomen, wie 1-oder 2-Adamantylmethyl, ferner Tricyclo[5.2. 1.0^{2,6}]dec- 8-yl- oder Adamantyl-, wie 1-Adamantyl-ethyl,
Arylniederalkyl, worin insbesondere
- Aryl 6 - 14 Kohlenstoffatome hat, wie in Phenyl, Indenyl, Indanyl, Naphthyl, Anthryl, Phenanthryl, Acenaphthyl oder Fluorenyl, das unsubstituiert oder insbesondere durch Nie-deralkyl, z. B. Methyl, Ethyl oder Isopropyl, Halogenniederalkyl, wie Trifluormethyl, Phenyl, 1- oder 2-Naphthyl, Hydroxy, Niederalkoxy, z. B. Methoxy, Carbamoylnieder-alkoxy, N-Niederalkylcarbamoylniederalkoxy oder N,N-Diniederalkylcarbamoylnieder-alkoxy, Amino, Mono- oder Diniederalkylamino, Niederalkanoylamino, z. B. Pivaloyl-amino, Halogen, z. B. Fluor oder Chlor, Carboxy, Niederalkoxycarbonyl, Benzyl-, Naphthyl- oder Fluorenylniederalkoxycarbonyl, Niederalkanoyl, Sulfo, Niederalkylsulfonyl, Phosphono, Hydroxy-niederalkoxy-phosphoryl oder Diniederalkoxyphosphoryl, Carbamoyl, Mono- oder Diniederalkylcarbamoyl, Sulfamoyl, Mono- oder Diniederalkylsulfamoyl, Nitro und/oder Cyano ein- oder bis zu dreifach substituiert sein kann, wobei Phenyl bis zu dreimal vorliegen kann, wie in Diphenyl-, Dibenzyl- oder Triphenylniederalkyl, z. B. Diphenyl-, Dibenzyl- oder Triphenyl-2-ethyl, und
- worin Niederalkyl unsubstituiert oder substituiert ist, z. B. durch Niederalkyl, wie Methyl, Heterocyclyl ausgewählt aus Pyrrolyl, Furyl, Thienyl, Imidazolyl, Pyrazolyl, Oxazolyl, Thiazolyl, Pyridyl, Pyrazinyl, Pyrimidinyl, Indolyl, Chinolyl, Isochinolyl, Chin-oxalinyl, β-Carbolinyl und einem benzanellierten, cyclopenta-, cyclohexa- oder cyclohepta-anellierten Derivat dieser Reste, die auch ganz oder teilweise gesättigt vorliegen können, Hydroxy, Niederalkoxy, Niederalkanoyloxy, wie Acetoxy, Propionyloxy, Butyroxy, Isobutyroxy oder Pivaloyloxy, Acetoacetoxy, Amino- oder Benzyloxycarbonyl-amino-niederalkanoyloxy, wie 2-Amino- oder 2-Benzyloxycarbonylamino-2-methylpropi-onyloxy, Arylniederalkanoyloxy, worin Aryl 6 - 10 Kohlenstoffatome hat, wie in Benzoyloxy, Phenylacetyloxy, 1- oder 2-Naphthoyloxy, Niederalkoxycarbonyloxy, z. B. Methoxy-, Ethoxy-, n-Propoxy-, Isopropoxy-, n-Butoxy-, Isobutoxy-, sec-Butoxy-, tert-Butoxy-, n-Pentoxy-, Isopentoxy-, Neopentoxy-, tert-Pentoxy-, n-Hexoxy-, Isohexoxy- oder n- Heptoxycarbonyloxy, Mono- oder Diniederalkyl-aminocarbonyloxy, wie Ethylaminocarbonyloxy oder Diethylaminocarbonyloxy, Aryloxycarbonyloxy, worin Aryl 6 - 10 Kohlenstoffatome hat, z. B. Phenyloxycarbonyloxy oder 1- oder 2-Naphthyloxy carbonyloxy, Arylniederalkoxycarbonyloxy mit Aryl von 6 - 12 Kohlenstoffatomen, z. B. Phenylniederalkoxycarbonyloxy, wie Benzyloxycarbonyloxy, ferner 1- oder 2-Naphthylmethoxycarbonyloxy oder 9-Fluorenylmethoxycarbonyloxy, Sulfonyloxy, Niederalkylsulfonyloxy, z. B. Methyl-, Ethyl-, Propyl-, Isopropyl-, n-Butyl-, Isobutyl-, sec-Butyl-, tert-Butyl-, n-Pentyl-, Isopentyl-, Neopentyl-, tert-Pentyl-, n-Hexyl-, Isohexyl- oder n-Heptylsulfonyloxy, Phenylsulfonyloxy, 2- oder 4-Toluolsulfonyloxy, 1- oder 2-Naphthylsulfonyloxy, Amino, Mono- oder Diniederalkylamino, N-Niederalkoxy-N-nieder-alkylamino, wie N-Methoxy-N-methylamino, Mono- oder Di-(phenyl- oder -naphthylniederalkyl)amino, wie Benzylamino, Niederalkanoylamino, wie Pivaloylamino, Carboxy, verestertes Carboxy ausgewählt aus Niederalkoxycarbonyl, z. B. Methoxy-, Ethoxy-, n-Propoxy-, Isopropoxy-, n-Butoxy-, Isobutoxy-, sec-Butoxy-, tert-Butoxy-, n-Pentoxy-, Isopentoxy-, Neopentoxy-, tert-Pentoxy-, n-Hexoxy-, Isohexoxy- oder n- Heptoxy-carbonyl, Aryloxycarbonyl, worin Aryl 6 - 10 Kohlenstoffatome hat, z. B. Phenyloxycarbonyl oder 1- oder 2-Naphthyloxycarbonyl, Arylniederalkoxycarbonyl, worin Aryl 6 - 12 Kohlenstoffatome hat, z. B. Benzyloxycarbonyl, 1- oder 2-Naphthylmethyloxy-carbonyl oder 9-Fluorenylmethoxycarbonyl, Niederalkanoyl, wie Pivaloyl oder Acetyl, Niederalkylsulfonyl, wie tert-Butylsulfonyl, Hydroxy-niederalkoxyphosphoryl und Diniederalkoxyphosphoryl, Carbamoyl, durch einen oder zwei aus Niederalkyl, z. B. Methyl, Ethyl, Propyl, Isopropyl, n-Butyl, Isobutyl, sec-Butyl, tert-Butyl, n-Pentyl, Isopentyl, Neopentyl, tert-Pentyl, n-Hexyl, Isohexyl oder n-Heptyl, wie in N-Methylcarbamoyl, N-n-Butylcarbamoyl oder N,N-Dimethylcarbamoyl, aus Carboxyniederalkyl oder Niederalkoxycarbonyl-niederalkyl, z. B. als Carboxymethylcarbamoyl (Glycinylcarbonyl) oder als tert-Butoxycarbonylmethylcarbamoyl, aus Diniederalkylaminonieder-alkyl, z. B. 2-Dimethylaminoethyl, aus Aminocarboxyniederalkyl, wie 5-Amino-5-carboxypentyl, aus Hydroxyniederalkyl, z. B. Hydroxymethyl oder Hydroxyethyl, und aus Diniederalkoxyniederalkyl, z. B. 2-(2,2-Dimethoxyethyl) ausgewählte Reste oder einen aus Ethylen, Trimethylen, Tetramethylen und Pentamethylen ausgewählten Rest, worin ein Kohlenstoffatom durch Stickstoff, Sauerstoff, Schwefel oder mit Sauerstoff ein- oder zweifach substituierten Schwefel ersetzt sein kann und das ungesättigt sein kann, z. B. als Piperidino-, Pyrazin-1-yl-, Piperazin-1-yl-, Pyrimidin-1-yl-, Pyridazin-1-yl-, Morpholino-, Thiomorpholino- oder S,S-Dioxothiomorpholino-carbonyl, substituiertes Carbamoyl, Sulfamoyl, Phosphono, Benzfuranyl, Oxo (welches nicht an dem Kohlenstoff, der mit dem Aminostickstoff, der mit R₁, R₂, R₈ oder R₉ verknüpft ist, gebunden ist) und/oder Cyano sein kann und unverzweigt oder verzweigt ist, ist insbesondere ausgewählt aus Phenylniederalkyl, wie Benzyl, das im Benzylrest unsubstituiert oder mono- oder mehrfachsubstituiert ist durch Niederalkyl, z.B. Methyl, Phenyl, Hydroxy, Niederalkoxy, z.B. Methoxy, Halogen, z.B. Chlor, Nitro, und/oder Cyano, wie 4-Methoxy-, 4-Fluor, 4-Chlor-, 4-Nitro- oder 4-Cyano-benzyl, Naphthylmethyl, wie α- oder β-Naphthylmethyl, Indenylmethyl, z.B. 1-, 2- oder 3-Indenylmethyl, Indanylmethyl, wie 1- oder 2-Indanylmethyl, und Phenanthrenylmethyl, wie 9-Phenanthrenylmethyl, 2-Phenylethyl, 2-α-Naphthylethyl, 2-β-Naphthylethyl, 2-Niederalkylphenyl-ethyl, wie 2-(4-Methylphenyl)ethyl, 2-Niederalkoxyphenylethyl, wie 2-(4-Methoxyphenyl)ethyl, 2,2-Diphenylethyl, 2,2-Di-(4-methoxyphenyl)-ethyl, 2,2,2-Triphenylethyl, 2,2-Dibenzylethyl, aus durch zwei aus Phenylniederalkoxycarbonyl, wie Benzyloxycarbonyl, oder Niederalkanoyl-amino, wie Pivaloylamino, ausgewählte Reste in 2- und p-Stellung substituiertes Phenylniederalkyl, wie 2,p-Dibenzyloxycarbonylamino-phenylethyl oder 2-Pivaloyl-amino-p-benzyloxycarbonylamino-phenylethyl, 2,p-Diamino-phenylethyl, 3-Phenyl-propyl, 3-(p-Hydroxyphenyl)-propyl, 3-α- oder 3-β-Naphthylpropyl, 2-Benzyl-3-(1-pyrazolyl-)-propyl, 3-Phenyl- oder 3-α-Naphthyl-2-hydroxy-propyl, 3-Phenyl- oder 3-α-Naphthyl-2-niederalkoxy-propyl, wie 3-Phenyl- oder 3-α-Naphthyl-2-neopentyloxy-propyl, 3-Phenyl- oder 3-α-Naphthyl-2-niederalkanoyloxy-propyl, wie 3-Phenyl-2-pivaloyloxy oder -2-acetoxy-propyl, 2-Benzyl- oder 1- oder 2-Naphthyl-3-(N-methoxyl-N-methylamino)-propyl, 3-Phenyl- oder 3-α-Naphthyl-2-dimethylamino-methyl-propyl, 3-α-Naphthyl-2-pivaloyloxy- oder -2-acetoxy-propyl, 3-αNaphthyl-2-acetoacetoxy-propyl, 3-α-Naphthyl-2-ethylaminocarbonyloxy-propyl oder 3-α-Naphthyl-2-((2-amino- oder 2-benzyloxycarbonylamino)-2-methylpropionyloxy)-propyl, 3-Phenyl- oder 3-α-Naphthyl-2-carboxymethylpropyl, 3-Phenyl- oder 3-α-Naphthyl2-niederalkoxycarbonyl-propyl, wie 3-α-Naphthyl-2-ethoxycarbonyl-propyl, 3-Phenyl- oder 3-α-Naphthyl-2-benzyloxycarbonylmethyl-propyl, 2-(S)-Benzyl-3-tert-butylsulfonyl-propyl, 3-Phe-nyl-2-phosphono- oder -phosphonomethyl-propyl, 3-Phenyl-2-dimethoxyphosphoryl- oder -dimethoxyphosphorylmethyl-propyl, 3-Phenyl-2-diethoxyphosphoryl- oder -diethoxyphosphorylmethyl-propyl, 3-Phenyl-2-ethoxy- oder methoxyhydroxyphosphoryl-propyl, 3-Phenyl- oder 3-α-Naphthyl-2-carbamoyl-propyl, 3-Phenyl- oder 3-α-Naphthyl-2-tert-butylcarbamoyl-propyl, 3-Phenyl- oder 3-α-Naphthyl-2-(2-dimethylaminoethyl)-carbamoyl-propyl, 3-α-Naphthyl-2-(carboxy- oder tert-butoxycarbonyl)methylcarbamoylpropyl, 3-Phenyl- oder 3-α-Naphthyl-2-(3-hydroxy-2-propyl)carbamoyl-propyl, 3-Phenyl- oder 3-α-Naphthyl-2-(2,2-dimethoxyethyl)-carbamoylpropyl, 3-Phenyl- oder 3-α-Naphthyl-2-(5-amino-5-carboxypentyl)-carbamoyl-propyl, 3-Phenyl- oder 3-α-Naphthyl-2-cyano-propyl, 3-Phenyl- oder 3-α-Naphthyl-2-cyanomethyl-propyl, 3-Phenyl- oder 3-α-Naphthyl-2-acetonyl-propyl, 4-Hydroxyphenylbutyl, 4-Phenyl- oder 4-α-Naphthyl-3-carboxy-butyl, 4-Phenyl- oder 4-α-Naphthyl-3-benzyloxycarbonyl-butyl, 2-Benzyl-4-(2-benzofuranyl)-4-oxobutyl, 2-Benzyl- oder 2-α-Naphthylmethyl-4-cyano-butyl, 2-Benzyl- oder 2-α-Naphthylmethyl-5-dimethylamino-pentyl, 2-Benzyl- oder 2-α-Naphthylmethyl-4-oxo-pentyl, 2-Benzyl- oder 2-α-Naphthylmethyl4,4-dimethyl-3-oxo-pentyl, 2-Benzyl- oder 2-α-Naphthylmethyl-5-dimethylamino-4oxo-pentyl oder 2-Benzyl- oder 2-α-Naphthylmethyl-5,5-dimethyl-4-oxo-hexyl, bevorzugt Phenylniederalkyl, wie Benzyl, 2-Phenylethyl oder 3-Phenylpropyl, 4-Hydroxybenzyl, 1- oder 2-Napthylmethyl oder 1- oder 2-Naphthyl-2-ethyl, besonders bevorzugt Phenylniederalkyl wie zuletzt definiert,
Heterocyclylniederalkyl, welches insbesondere unter Heterocyclylniederalkanoyl R₁, R₂, R₈ und R₉ genanntes unsubstituiertes oder substituiertes Heterocyclyl und unsubstituiertes oder wie Niederalkanoyl in Heterocyclylniederalkanoyl R₁, R₂, R₈ und R₉ substituiertes Niederalkyl enthält (wobei Oxo nicht an dem Kohlenstoffatom, welches mit dem den Rest R₁, R₂, R₈ oder R₉ tragenden Stickstoffatom verbunden ist, vorliegt), z. B. Methyl, 2-Ethyl oder 3-Propyl gebunden an unsubstituiertes oder durch Niederalkyl oder Phenyl substituiertes Pyrrolyl, wie 2- oder 3-Pyrrolyl, 4- oder 5-Methylpyrrolyl oder 4- oder 5-Phenylpyrrolyl, Thienyl, wie 2-Thienyl, Furyl, wie 2-Furyl, Pyrazolyl, wie 1-Pyrazolyl, Pyridyl, wie 2-, 3- oder 4-Pyridyl, unsubstituiertes oder durch Niederalkyl, z. B. Methyl, Phenylniederalkyl, z. B. Benzyl, Niederalkoxy, z. B. Methoxy, Phenylniederalkoxy, z. B. Benzyloxy, oder Halogen, z. B. Chlor, substituiertes Indolyl, wie 2-, 3-oder 5-Indolyl, 1-Methyl-, 2-Methyl-, 5-Methoxy, 5-Benzyloxy-, 5-Chlor- oder 4,5-Dimethylindol-2-yl, 1-Benzylindol-2-yl oder -3-yl, 4,5,6,7-Tetrahydroindol-2-yl, Cyclohepta[b]pyrrol-5-yl, unsubstituiertes oder durch Hydroxy substituiertes Chinolyl, wie 2-, 3- oder 4-Chinolyl oder 4-Hydroxychinol-2-yl, unsubstituiertes oder durch Hydroxy substituiertes Isochinolyl, wie 1-, 3- oder 4-Isochinolyl oder 1-Oxo1,2-dihydroisochinol-3-yl, 2-Chinoxalinyl, 3,1-Benzfuran-2-yl, Benz[e]indol-2-yl, β-Carbolin-3-yl, 3-Chromanyl, 3-Thiochromanyl, 3-Pyrrolidinyl, Hydroxypyrrolidinyl, wie 3- oder 4-Hydroxypyrrolidin-2-yl, Oxopyrrolidinyl, wie 5-Oxopytrolidin2-yl, Piperidinyl, wie 2-, 3- oder 4-Piperidinyl, Morpholinyl, wie 2- oder 3-Morpholinyl, Thiomorpholinyl, wie 2-oder 3-Thiomorpholinyl, S,S-Dioxothiomorpholinyl, wie S,S-Dioxothiomorpholin2- oder -3-yl, Indolinyl, wie 2- oder 3-Indolinyl, 1,2,3,4-Tetrahydrochinolyl, wie 1,2,3,4-Tetrahydrochinol-2-, -3- oder -4-yl, 1,2,3,4-Tetrahydroisochinolyl, wie 1,2,3,4-Tetrahydroisochinol- 1-,-2- oder -3-yl, oder 1-Oxo- 1,2,3,4-tetrahydroisochinol-3-yl,
Hydroxyniederalkyl, wie 3-Hydroxypropyl oder 2-Hydroxy-3-methylpentyl,
Niederalkoxyniederalkyl, z.B. Niederalkoxyethyl oder Niederalkoxypropyl, wie 2-Methoxyethyl, 2-Ethoxyethyl oder 3-Methoxypropyl,
Niederalkoxyniederalkoxy-niederalkyl, wie 2-Methoxymethoxy-3-methyl-pentyl,
Phenoxyniederalkyl oder Nitrophenyoxyniederalkyl, wie Phenoxymethyl, Phenoxyethyl oder 4-Nitrophenoxymethyl,
Naphthoxyniederalkyl, z.B. α- oder β-Naphthoxyethyl,
Niederalkanoyloxyniederalkyl, z.B. Niederalkanoyloxyethyl oder Niederalkanoyloxy-propyl, wie Acetoxyethyl oder 3-Acetoxypropyl,
Acetoacetoxyniederalkyl,
Arylmercaptoniederalkyl, worin Aryl 6 - 10 Kohlenstoffatome hat, z. B. Phenyl oder Naphthyl, wie Phenylmercaptomethyl,
Aminoniederalkyl, wie 3-Aminopropyl oder 5-Aminopentyl,
Mono- oder Diniederalkylaminoniederalkyl, wie Dimethylaminoethyl oder 2-Dimethyl-amino-2-isopropylethyl,
Phenyl- oder Naphthylaminoniederalkyl, wie 3-Phenylaminopropyl,
Niederalkanoylamino-niederalkyl, wie 4-Acetylaminopentyl, durch Niederalkyl, wie Methyl, am Stickstoff substituiertes Piperazinylcarbonylnieder-alkyl, wie 4-Methylpiperazinylcarbonylmethyl,
Niederalkoxycarbonylamino-niederalkyl, wie 5-(tert-Butoxycarbonylamino)-pentyl oder 3-Ethoxycarbonylamino-2-isobutyl-propyl,
Phenylniederalkoxycarbonylamino-niederalkyl, wie 5-(Benzyloxycarbonylamino)-pentyl,
Aminocarbonylamino-niederalkyl, wie Aminocarbonylamino-ethyl,
N-Phenylniederalkyl-N-niederalkylaminocarbonylamino-niederalkyl, z. B. 2-Isobutyl-3-(N-benzyl-N-methylaminocarbonylamino)propyl,
Halogenniederalkyl, z.B. 2-Halogenethyl, wie 2-Fluor-, 2-Chlor-, 2-Brom-, 2-Iod-, 2,2,2,-Trifluor- oder 2,2,2-Trichlorethyl, Trifluorniederalkyl, wie Trifluormethyl, oder Halogenpropyl, wie 3-Chlor- oder 3-Brompropyl,
Carboxyniederalkyl, z.B. Carboxyethyl oder 3-Carboxypropyl,
Niederalkoxycarbonylniederalkyl, z.B. Niederalkoxycarbonylethyl oder Niederalkoxycarbonylpropyl, wie Methoxycarbonylethyl, 3-Methoxycarbonylpropyl, Ethoxycarbonylethyl oder 3-Ethoxycarbonylpropyl,
2-Halogenniederalkoxycarbonyl-niederalkyl, wie 2-Chlor-, 2-Brom-, 2-Iod- oder 2,2,2-Trichlorethoxycarbonyl-2-ethyl oder 3-propyl,
Phenyl- oder Naphthylniederalkyloxycarbonyl-niederalkyl, z. B. Benzyloxycarbonyl-niederalkyl, wie 3-Benzyloxycarbonyl-2,2-dimethylpropyl,
Heterocyclylniederalkoxycarbonyl-niederalkyl, worin Heterocyclyl vorzugsweise ausgewählt ist aus Pyrrolyl, Furyl, Thienyl, Imidazolyl, Pyrazolyl, Oxazolyl, Thiazolyl, Pyridyl, Pyrazinyl, Pyrimidinyl, Indolyl, Chinolyl, Isochinolyl, Chinoxalinyl, β-Carbolinyl und einem benzanellierten, cyclopenta-, cyclohexa- oder cyclohepta-anellierten Derivat dieser Reste, wobei die genannten Reste auch ganz oder teilweise gesättigt vorliegen können, wie in 4-Pyridylmethyloxycarbonyl-2-ethyl oder -3-propyl oder 2-Morpholino-carbonyloxy-4-methyl-pentyl,
Niederalkylsulfonylniederalkyl, wie 2-Ethylsulfonyl- oder 2-tert-Butylsulfonylmethyl,
Arylsulfonylniederalkyl, worin Aryl vorzugsweise 6 - 10 Kohlenstoffatome hat, z. B. Phenyl oder Napthyl, wie Phenylsulfonylmethyl,
Carbamoylniederalkyl, wie Carbamoylethyl oder 3-Carbamoylpropyl,
Niederalkylcarbamoylniederalkyl, z.B. Niederalkylcarbamoylethyl oder Methyl-carbamoylniederalkyl, wie 2-Methylcarbamoylethyl,
Diniederalkylcarbamoylniederalkyl, z.B. 2-Diniederalkylcarbamoylethyl oder Dimethylcarbamoylniederalkyl, wie 2-Dimethylcarbamoylethyl,
Hydroxyniederalkylcarbamoyl- oder Di-(hydroxyniederalkyl)carbamoyl-niederalkyl, wie 2-Hydroxymethylcarbamoyl- oder Di-(hydroxymethyl)carbamoyl-2-ethyl oder -3-propyl,
N-Niederalkoxyniederalkoxyniederalkyl-carbamoyl-niederalkyl, wie 2-Isobutyl-3-(2-(2-methoxyethoxy)ethylaminocarbonyl)-propyl,
Carboxyniederalkylcarbamoyl- oder Di-(carboxyniederalkyl)carbamoyl-niederalkyl, wie Carboxymethyl- oder Di-(carboxymethyl)carbamoyl-2-ethyl oder -3-propyl,
durch einen aus Ethylen, Trimethylen, Tetramethylen und Pentamethylen ausgewählten Rest, worin ein Kohlenstoffatom durch Stickstoff, Sauerstoff, Schwefel oder mit Sauerstoff ein- oder zweifach substituiertem Schwefel ersetzt sein kann, am Stickstoff substituiertes Carbamoylniederalkyl, wobei der so gebildete Rest auch ganz oder teilweise ungesättigt sein kann, z. B. als Piperidino-, Pyrazin-1-yl-, Piperazin-1-yl-, Pyrimidin-1-yl-, Pyridazin-1-yl-, Morpholino-, Thiomorpholino- oder S,S-Dioxothiomorpholino-car-bonylniederalkyl, wie in 2-Morpholinocarbonyl-ethyl, 3-(Morpholinocarbonyl)-propyl oder 3-(Morpholinocarbonyl)-2-isobutyl-propyl,
N-Niederalkyl-N-heterocyclylniederalkylcarbamoylniederalkyl, worin Heterocyclyl vorzugsweise aus Pyrrolyl, Furyl, Thienyl, Imidazolyl, Pyrazolyl, Oxazolyl, Thiazolyl, Pyridyl, Pyrazinyl, Pyrimidinyl, Indolyl, Chinolyl, Isochinolyl, Chinoxalinyl, β-Carbolinyl und einem benzanellierten, cyclopenta-, cyclohexa- oder cyclohepta-anellierten Derivat dieser Reste, die auch ganz oder teilweise gesättigt sein können, ausgewählt ist, wie 2-(N-2-Pyridylmethyl)-N-methylcarbamoyl-ethyl,
Sulfamoylniederalkyl, wie 2-Sulfamoylethyl,
N-(Phenyl- oder Naphthylniederalkyl)sulfamoylniederalkyl, wie 3-Benzylamino-sulfonyl-2-isopropyl-propyl, oder
am Stickstoffatom von einem aus Ethylen, Trimethylen, Tetramethylen und Penta-methylen ausgewählten Rest, worin ein Kohlenstoffatom durch Stickstoff, mit Nieder-alkyl, wie Methyl, substituierten Stickstoff, Sauerstoff, Schwefel und mit Sauerstoff ein-oder zweifach substituierten Schwefel ersetzt sein kann, ausgewählten Rest substituiertes Sulfamoylniederalkyl, wobei der so gebildete Rest auch ganz oder teilweise ungesättigt sein kann, z. B. als Piperidino-, Pyrazin-1-yl-, Piperazin-1-yl-, 4-Methylpiperazin-1-yl, Pyrimidin-1-yl-, Pyridazin-1-yl-, Morpholino-, Thiomorpholino- oder S,S-Dioxothio-morpholino-sulfonylniederalkyl, wie 3-(4-Methylpiperazinylsulfonyl)-2-isopropyl-propyl oder 3-(4-Morpholinylsulfonyl)-2-isopropyl-propyl,
Oxoniederalkyl (worin Oxo nicht an dem Kohlenstoffatom, welches mit dem den Rest R₁, R₂, R₈ oder R₉ tragenden Stickstoffatom verbunden ist, vorliegt), wie 3-Oxo-n-butyl oder 3-Oxo-n-pentyl,
Cyanoniederalkanoyl, wie Cyanomethyl, 2-Cyanoethyl, 2- oder 3-Cyano-n-propyl oder 2-, 3- oder 4-Cyano-n-butyl,
Hydroxy-carboxy-niederalkyl, wie 2-Hydroxy-2-carboxy-ethyl oder 2-Hydroxy-3-carboxypropyl,
α-Naphthoxy-carboxy-niederalkyl, wie 2-α-Naphthoxy-4-carboxy-n-butyl,
Hydroxy-niederalkoxycarbonyl-niederalkyl, z. B. 2-Hydroxy-2-niederalkoxycarbonyl-ethyl oder -propyl oder Hydroxy-ethoxy- oder Hydroxy-methoxy-carbonyl-nieder-alkyl, wie 2-Hydroxy-2-ethoxy- oder -methoxy-carbonylethyl oder 2-Hydroxy-3-ethoxy- oder -methoxycarbonyl-propyl,
α-Naphthoxy-niederalkoxycarbonylniederalkyl, z.B. α-Naphthoxy-niederalkoxycarbonyl-2-ethyl, -2-propyl oder -2-butyryl oder α-Naphthoxyethoxycarbonyl-niederalkyl, wie α-Naphthoxy-ethoxycarbonyl-2-ethyl, 2-α-Naphthoxy-3-ethoxycarbonyl-propyl oder 2-α-Naphthoxy-4-tert-butoxycarbonylbutyl,
α-Naphthoxy-benzyloxycarbonyl-niederalkyl, wie 2-α-Naphthoxy-3-benzyloxy-carbonyl-propyl,
verestertes Hydroxy-niederalkoxycarbonyl-niederalkyl, worin die Hydroxygruppe durch Niederalkanoyl, z. B. Acetyl, Propionyl oder Pivaloyl, Cycloalkylniederalkanoyl, worin Cycloalkyl 3 - 7 Kohlenstoffatome hat und Niederalkanoyl wie zuletzt definiert ist, z. B. Cyclohexylcarbonyl oder 2-Cyclohexyl- oder 2-Cyclopentylacetyl, Bicycloalkylnieder-alkanoyl, worin Bicylcoalkyl z.B. 5 - 10, insbesondere 6 - 9, Kohlenstoffatome enthält, wie in Bicyclohexyl-, -heptyl-, -octyl-, -nonyl- oder -decyl-acetyl oder - 3-propionyl, z.B. Bicyclo[3.1.0]hex- 1-, -2- oder -3-yl-, Bicyclo[4.1.0]hept-1- oder -7-yl-, Bicyclo-[2.2.1]hept-2-yl-, z.B. endo- oder exo-Norbornyl-, Bicyclo-[3.2.1]oct-2-yl-, Bicyclo-[3.3.0]oct-3-yl- oder Bicyclo[3.3.1]non-9-yl-, ferner α- oder β-Decahydronaphthyl-acetyl oder -3-propionyl, Tricycloalkylniederalkanoyl, worin Tricycloalkyl z.B. 8 - 10 Kohlenstoffatome enthält, z.B. in Tricyclo[5.2.1.0^{2,6}]dec-8-yl- oder Adamantyl-, wie 1-Adamantyl-acetyl, Arylniederalkanoyl, worin Aryl 6 - 14 Kohlenstoffatome hat, z. B. Phenyl, Indenyl, Indanyl, Naphthyl, Anthryl, Phenanthryl, Acenaphthyl oder Fluorenyl, das unsubstituiert oder durch Niederalkyl, Hydroxy, Niederalkoxy, Carbamoylniederalkoxy, N-Nie-deralkylcarbamoylniederalkoxy oder N,N-Diniederalkylcarbamoylniederalkoxy, Amino, Mono- oder Diniederalkylamino, Halogen, wie Fluor, Chlor oder Brom, Carboxy, Nieder-alkoxycarbonyl, Phenyl-, Naphthyl- oder Fluorenylniederalkoxycarbonyl, Niederalkanoyl, Niederalkylsulfonyl, z. B. Methylsulfonyl, Phosphono, Hydroxy-niederalkoxy-phosphoryl oder Diniederalkoxyphosphoryl, Carbamoyl, Sulfamoyl, Nitro und/oder Cyano ein-oder bis zu dreifach substituiert sein kann, Niederalkoxycarbonyl, z. B. tert-Butoxycarbonyl, 2-Halogenniederalkoxycarbonyl, wie oben definiert, oder Phenyl- oder Fluorenyl-niederalkoxycarbonyl, z. B. Benzyloxycarbonyl oder 9-Fluorenylmethoxycarbonyl, verestert ist, wie 2-Acetoxy-2-methoxycarbonyl-ethyl, 2-Benzoyloxy-, 2-(1- oder 2-Naphthoyloxy)-, 2-(Phenyl-2-acetyloxy)-, 2-(1- oder 2-Napthyl-2-acetyloxy)-, 2-(4-Methylphenyl-2-acetyloxy)-, 2-(4-Methoxyphenyl-2-acetyloxy)- oder 2-(2-(o,o-Dichlorophenyl)-2-acetyloxy)-2-methoxycarbonyl-ethyl oder -3-propyl,
Dihydroxy-carboxy-niederalkyl, wie 2,3-Dihydroxy-3-carboxy-propyl,
Dihydroxy-niederalkoxycarbonyl-niederalkyl, wie 2,3-Dihydroxy-3-ethoxy- oder -methoxycarbonyl-propyl,
durch Niederalkanoyl, wie Acetyl, Propionyl oder Butyryl, Niederalkoxycarbonyl, z. B. tert-Butoxycarbonyl, Phenyl- oder Fluorenyl-niederalkoxycarbonyl, wie Benzyloxy-carbonyl oder 9-Fluorenylmethoxycarbonyl, Niederalkylsulfonyl oder Toluolsulfonyl verestertes Dihydroxy-niederalkoxycarbonyl-niederalkyl, z.B. Diniederalkanoyloxy-niederalkoxy-propyl, wie 2,3-Diacetoxy-3-methoxycarbonyl-propyl,
α-Naphthoxy-diniederalkylamino-niederalkyl, wie 2-α-Naphthoxy-5-dimethylamino-pentyl,
α-Naphthoxy-carbamoyl-niederalkyl, wie 2-α-Naphthoxy-4-carbamoyl-butyl,
α-Naphthoxy-oxo-niederalkyl (wobei Oxo nicht an dem Kohlenstoffatom, welches mit dem den Rest R₁, R₂, R₈ oder R₉ tragenden Stickstoffatom verbunden ist, vorliegt), wie 2-α-Naphthoxy-4-oxo-pentyl, oder
α-Naphthoxy-cyanoniederalkyl, wie 2-α-Naphthoxy-cyano-ethyl oder 2-α-Naph-thoxy-4-cyanobutyl.

Besonders bevorzugt ist Niederalkyl, wie Methyl, Ethyl, Propyl, Isopropyl, n-Butyl, Isobutyl oder tert-Butyl.

Alkenyl R₁, R₂, R₈ oder R₉ enthält vorzugsweise 2 - 10 Kohlenstoffatome, ist bevorzugt Niederalkenyl mit 2 - 7, insbesondere 2 - 4, Kohlenstoffatomen und ist z.B. Vinyl, Allyl oder 2- oder 3-Butenyl. Niederalkenyl R₁, R₂, R₈ oder R₉ kann durch die gleichen Substi-tuenten substituiert sein wie Niederalkyl, z.B. durch Cycloalkyl, wie bei Cycloalkylnie-deralkanoyl R₁, R₂, R₈ oder R₉ definiert, welches unsubstituiert oder durch Niederalkyl, wie Isopropyl, Halogenniederalkyl, wie Trifluormethyl, Hydroxy, Niederalkoxy, Carbamoylniederalkoxy, N-Niederalkylcarbamoylniederalkoxy oder N,N-Diniederalkyl-carbamoylniederalkoxy, Amino, Mono- oder Diniederalkylamino, Halogen, wie Fluor, Chlor oder Brom, Carboxy, Niederalkoxycarbonyl, Phenyl-, Naphthyl- oder Fluorenyl-niederalkoxycarbonyl, Niederalkanoyl, Niederalkylsulfonyl, z. B. Methylsulfonyl, Phosphono, Hydroxy-niederalkoxy-phosphoryl oder Diniederalkoxyphosphoryl, Carba-moyl, Mono- oder Diniederalkylaminocarbamoyl, Sulfamoyl, Mono- oder Diniederalkyl-sulfamoyl, Nitro und/oder Cyano ein- oder bis zu dreifach substituiert ist und insbesondere am endständigen Kohlenstoffatom mit Niederalkenyl verbunden ist, wie in Cyclobutyl-, Cyclopentyl-, Cyclohexyl- oder Cycloheptyl-methyl-2-vinyl, -2- oder -3-allyl oder -2-, -3- oder -4-but-2-enyl, Aryl, wie unter Arylniederalkanoyl R₁, R₂, R₈ oder R₉ definiert, das unsubstituiert oder substituiert ist und vorzugsweise endständig an Niederalkenyl gebunden ist, wie in Styryl, 3-Phenylallyl (Cinnamyl), 2-(α-Naphthyl)-vinyl oder 2-(β-Naphthyl)-vinyl, unsubstituiertes oder substituiertes Heterocyclyl, wie unter Heterocyclylniederalkanoyl R₁, R₂, R₈ oder R₉ definiert, vorzugsweise ausgewählt aus Pyrrolyl, Furyl, Thienyl, Imidazolyl, Pyrazolyl, Oxazolyl, Thiazolyl, Pyridyl, Pyrazinyl, Pyrimidinyl, Indolyl, Chinolyl, Isochinolyl, Chinoxalinyl, β-Carbolinyl und einem benz-, cyclopenta-, cyclohexa- oder cyclohepta-anellierten Derivat dieser Reste, die auch ganz oder teilweise gesättigt vorliegen können und unsubstituiert oder wie oben substituiert sind und über ein Stickstoff- oder ein Kohlenstoffatom mit Niederalkenyl, vorzugsweise am endständigen Kohlenstoffatom des Niederalkenylrestes, der z. B. aus Vinyl, Allyl und 2- oder 3-Butenyl ausgewählt ist, gebunden ist, z. B. als Pymidin-1-yl-, Piperidino-, Pyrazin-1-yl-, Piperazin-1-yl-, Pyridazin-1-yl-, Morpholino-, Thiomorpholino- oder S,S -Dioxothiomorpholino-niederalkenyl, wie in 2-Morpholino-vinyl, 3-Morpholino-allyl oder 4-Morpholino-2- oder -3-butenyl, unsubstituiertes oder durch Niederalkyl oder Phenyl substituiertes Pyrrolylniederalkenyl, wie 2- oder 3-Pyrrolylvinyl oder -allyl, 4- oder 5-Methylpyrrolylvinyl oder -allyl oder 4- oder 5-Phenylpyrrolylvinyl oder -allyl, Thienylniederalkenyl, wie 2-Thienylvinyl oder -allyl, Furylniederalkenyl, wie 2-Furyl-vinyl oder - allyl, Pyridylniederalkenyl, wie 2-, 3- oder 4-Pyridylvinyl oder - allyl, unsubstituiertes oder durch Niederalkyl, z. B. Methyl, Phenylniederalkyl, z. B. Benzyl, Niederalkoxy, z. B. Methoxy, Phenylniederalkoxy, z. B. Benzyloxy, oder Halogen, z. B. Chlor, substituiertes Indolylniederalkenyl, wie 2-, 3- oder 5-Indolylvinyl oder -allyl, 1-Methyl-, 2-Methyl-, 5-Methoxy-, 5-Benzyloxy-, 5-Chlor- oder 4,5-Dimethylindol-2-yl-, 1-Benzylindol-2-yl- oder -3-ylvinyl oder -allyl, 4,5,6,7-Tetrahydroindol-2-ylmethyl, -ethyl oder -n-propyl, Cyclohepta[b]pyrrol-5-ylvinyl oder -allyl, unsubstituiertes oder durch Hydroxy substituiertes Chinolylniederalkenyl, z. B. 2-, 3- oder 4-Chinolyl- oder 4-Hydroxychinol-2-ylvinyl oder -allyl, unsubstituiertes oder durch Hydroxy substituiertes Isochinolylniederalkenyl, wie 1-, 3- oder 4-Isochinolyl- oder 1-Oxo-1,2-dihydroiso-chinol-3-ylvinyl oder -allyl, 2-Chinoxalinylvinyl oder -allyl, 3,1-Benzfuran-2-ylvinyl oder -allyl, Benz[e]indol-2-ylvinyl oder -allyl, β-Carbolin-3-ylvinyl oder -allyl, 3-Chromanylvinyl oder -allyl, 3-Thiochromanylvinyl oder -allyl, 3-Pyrrolidinylvinyl oder - allyl, Hydroxypyrrolidinylniederalkenyl, wie 3- oder 4-Hydroxypyrrolidin-2-ylvinyl oder -allyl, Oxopyrrolidinylniederalkenyl, wie 5-Oxopyrrolidin-2-ylvinyl oder -allyl, Piperidinyl-niederalkenyl, wie 2-, 3- oder 4-Piperidinylvinyl oder -allyl, Morpholinylniederalkenyl, wie 2- oder 3-Morpholinylvinyl oder -allyl, Thiomorpholinylniederalkenyl, wie 2-oder 3-Thiomorpholinylvinyl oder -allyl, S,S-Dioxothiomorpholinylniederalkenyl, wie S,S-Di-oxothiomorpholin-2- oder -3-ylvinyl oder -allyl, Indolinylniederalkenyl, wie 2-oder 3-Indolinylvinyl oder -allyl, 1,2,3,4-Tetrahydrochinolylniederalkenyl, wie 1,2,3,4-Tetrahydrochinol-2-, -3- oder -4-ylvinyl oder -allyl, 1,2,3,4-Tetrahydroisochinolylnieder-alkenyl, wie 1,2,3,4-Tetrahydroisochinol-1-, -2- oder-3-ylvinyl oder -allyl, oder 1-Oxo-1,2,3,4-tetrahydroisochinol-3-ylvinyl oder -allyl, ferner Hydroxy, verethertes Hydroxy ausgewählt aus Niederalkoxy, wie Methoxy oder Ethoxy, Phenoxy oder Naphthoxy, Phenyl- oder Napthylniederalkoxy, wie Benzyloxy, und Heterocyclylniederalkoxy, worin Heterocyclyl vorzugsweise aus Pyrrolyl, Furyl, Thienyl, Imidazolyl, Pyrazolyl, Oxazolyl, Thiazolyl, Pyridyl, Pyrazinyl, Pyrimidinyl, Indolyl, Chinolyl, Isochinolyl, Chinoxalinyl, β-Carbolinyl und einem benzanellierten, cyclopenta-, cyclohexa- oder cyclohepta-anellierten Derivat dieser Reste, die auch ganz oder teilweise gesättigt sein können, ausgewählt ist, z.B. 4-Pyrrolidinylmethoxy, 1-Imidazolylmethoxy, 2-Pyridylmethoxy, 3-Pyridylmethoxy, Chinolin-2-ylmethoxy oder 1ndol-2-ylmethoxy, verestertes Hydroxy ausgewählt aus Niederalkanoyloxy, wie Acetoxy, Propionyloxy, Butyroxy, Isobutyroxy oder Pivaloyloxy, Acetoacetoxy, Amino- oder Benzyloxycarbonylamino-niederalkano-yloxy, wie 2-Amino- oder 2-Benzyloxycarbonylamino-2-methylpropionyloxy, Arylniederalkanoyloxy, worin Aryl 6 - 10 Kohlenstoffatome hat, wie in Benzoyloxy, Phenyl-acetyloxy, 1- oder 2-Naphthoyloxy, Niederalkoxycarbonyloxy, z. B. Methoxy-, Ethoxy-, n-Propoxy-, Isopropoxy-, n-Butoxy-, Isobutoxy-, sec-Butoxy-, tert-Butoxy-, n-Pentoxy-, Isopentoxy-, Neopentoxy-, tert-Pentoxy-, n-Hexoxy-, Isohexoxy- oder n- Heptoxycarbonyloxy, Mono- oder Diniederalkyl-aminocarbonyloxy, wie Ethylaminocarbonyloxy oder Diethylaminocarbonyloxy, Aryloxycarbonyloxy, worin Aryl 6 - 10 Kohlenstoffatome hat, z. B. Phenyloxycarbonyloxy oder 1- oder 2-Naphthyloxycarbonyloxy, Arylniederalkoxycarbonyloxy mit Aryl von 6 - 12 Kohlenstoffatomen, z. B. Phenylniederalk-oxycarbonyloxy, wie Benzyloxycarbonyloxy, ferner 1- oder 2-Naphthylmethoxycarbonyl-oxy oder 9-Fluorenylmethoxycarbonyloxy, Sulfonyloxy, Niederalkylsulfonyloxy, z. B. Methyl-, Ethyl-, Propyl-, Isopropyl-, n-Butyl-, Isobutyl-, sec-Butyl-, tert-Butyl-, n-Pentyl-, Isopentyl-, Neopentyl-, tert-Pentyl-, n-Hexyl-, Isohexyl- oder n-Heptylsulfonyloxy, Phenylsulfonyloxy, 2- oder 4-Toluolsulfonyloxy und 1- oder 2- Naphthylsulfonyloxy, Halogen, z.B. Chlor oder Brom, Carboxy, verestertes Carboxy ausgewählt aus Niederalkoxycarbonyl, wie Methoxycarbonyl, Ethoxycarbonyl, tert-Butoxycarbonyl, 2-Halogenniederalkoxycarbonyl, z. B. 2-Chlor-, 2-Brom-, 2-Iod- oder 2,2,2-Trichlorethoxycarbonyl, Phenyl- oder Naphthylniederalkyloxycarbonyl, z. B. Benzyloxycarbonyl, und Heterocyclylniederalkoxycarbonyl, worin Heterocyclyl vorzugsweise ausgewählt ist aus Pyrrolyl, Furyl, Thienyl, Imidazolyl, Pyrazolyl, Oxazolyl, Thiazolyl, Pyridyl, Pyrazinyl, Pyrimidinyl, Indolyl, Chinolyl, Isochinolyl, Chinoxalinyl, β-Carbolinyl und einem benz-anellierten, cyclopenta-, cyclohexa- oder cyclohepta-anellierten Derivat dieser Reste, wobei die genannten Reste auch ganz oder teilweise gesättigt vorliegen können, z. B. in 4-Pyridylmethoxycarbonyl, oder amidiertes Carboxy ausgewählt aus Carbamoyl, Niederalkylcarbamoyl, wie Methylcarbamoyl, Diniederalkylcarbamoyl, wie Dimethyl-carbamoyl, Hydroxyniederalkylcarbamoyl oder Di-(hydroxyniederalkyl)carbamoyl, wie Hydroxymethylcarbamoyl oder Di-(hydroxymethyl)carbamoyl, N-Niederalkoxyniederalkoxyniederalkyl-carbamoyl, wie 2-(2-Methoxyethoxy)ethylaminocarbonyl, Carboxyniederalkylcarbamoyl oder Di-(carboxyniederalkyl)carbamoyl-, z. B. Carboxymethyl- oder Di-(carboxymethyl)carbamoyl, und durch einen aus Ethylen, Trimethylen, Tetramethylen und Pentamethylen ausgewählten Rest, worin ein Kohlenstoffatom durch Stickstoff, Sauerstoff, Schwefel oder mit Sauerstoff ein- oder zweifach substituierten Schwefel ersetzt sein kann, am Stickstoff substituiertes Carbamoyl, wobei der so gebildete Rest auch ganz oder teilweise ungesättigt sein kann, z. B. als Piperidino-, Pyrazin-1-yl-, Piperazin-1-yl-, Pyrimidin-1-yl-, Pyridazin-1-yl-, Morpholino-, Thiomorpholino- oder S,S-Dioxothio-morpholinocarbonyl, N-Niederalkyl-N-heterocyclylniederalkylcarbamoyl, worin Hetero-cyclyl vorzugsweise aus Pyrrolyl, Furyl, Thienyl, Imidazolyl, Pyrazolyl, Oxazolyl, Thiazolyl, Pyridyl, Pyrazinyl, Pyrimidinyl, Indolyl, Chinolyl, Isochinolyl, Chinoxalinyl, β-Carbolinyl und einem benzanellierten, cyclopenta-, cyclohexa- oder cyclohepta-anel-lierten Derivat dieser Reste, die auch ganz oder teilweise gesättigt sein können, ausgewählt ist, z. B. N-2-Pyridylmethyl-N-methylcarbamoyl.

Alkinyl R₁, R₂, R₈ oder R₉ enthält in erster Linie 2 - 10, vorzugsweise als Niederalkinyl 2 -7, insbesondere 2 - 4, C-Atome und ist beispielsweise Ethinyl, 1-Propinyl oder 2-Propinyl, wobei die genannten Reste unsubstituiert oder durch die für Niederalkenyl R₁, R₂, R₈ oder R₉ genannten Reste substituiert sein können.

Heterocyclyl R₁ R₂, R₈ und R₉ ist über ein Kohlenstoffatom gebunden und enthält in erster Linie ein unter Heterocyclylniederalkanoyl R₁ R₂, R₈ und R₉ genanntes unsubstituiertes oder substituiertes Heterocyclyl und ist vorzugsweise unsubstituiertes oder durch Niederalkyl oder Phenyl substituiertes Pyrrolyl, wie 2- oder 3-Pyrrolyl, 4- oder 5-Methylpyrrolyl oder 4- oder 5-Phenylpyrrolyl, Thienyl, wie 2-Thienyl, Furyl, wie 2-Furyl, Pyridyl, wie 2-, 3- oder 4-Pyridyl, unsubstituiertes oder durch Niederalkyl, z. B. Methyl, Phenylniederalkyl, z. B. Benzyl, Niederalkoxy, z. B. Methoxy, Phenylnieder-alkoxy, z. B. Benzyloxy, oder Halogen, z. B. Chlor, substituiertes Indolyl, wie 2-, 3- oder 5-Indolyl, 1-Methyl-, 2-Methyl-, 5-Methoxy, 5-Benzyloxy, 5-Chlor oder 4,5-Dimethylindol-2-yl, 1-Benzylindol-2-yl oder -3-yl, 4,5,6,7-Tetrahydroindol-2-yl, unsubstituiertes oder durch Hydroxy substituiertes Chinolyl, wie 2-, 3- oder 4-Chinolyl oder 4-Hydroxychinol-2-yl, unsubstituiertes oder durch Hydroxy substituiertes Isochinolyl, wie 1-, 3- oder 4-Isochinolyl oder 1-Oxo-1,2-dihydroisochinol-3-yl, 2-Chinoxalinyl, 3,1-Benzfuran-2-yl, Benz[e]indol-2-yl, β-Carbolin-3-yl, Cyclohepta[b]pyrrol-5-yl, 3-Chromanyl, 3-Thiochromanyl, 3-Pyrrolidinyl, Hydroxypyrrolidinyl, wie 3-oder 4-Hydroxypyrrolidin-2-yl, Oxopyrrolidinyl, wie 5-Oxopyrrolidin-2-yl, Piperidyl, wie 2-, 3- oder 4-Piperidyl, Morpholinyl, wie 2- oder 3-Morpholinyl, Thiomorpholinyl, wie 2-oder 3-Thiomorpholinyl, S,S-Dioxothiomorpholinyl, wie S,S-Dioxothiomorpholin-2- oder -3-yl, Indolinyl, wie 2- oder 3-Indolinyl, 1,2,3,4-Tetrahydrochinolyl, wie 1,2,3,4-Tetrahydrochinol-2-, -3- oder -4-yl, 1,2,3,4-Tetrahydroisochinolyl, wie 1,2,3,4-Tetrahydroisochinol-1-, -2- oder -3-yl, oder 1-Oxo- 1,2,3,4-tetrahydroisochinol-3yl.

Durch Alkyl substituiertes Sulfonyl R₁ R₂, R₈ und R₉ enthält vorzugsweise einen unter Alkyl R₁, R₂, R₈ und R₉ genannten unsubstituierten oder substituierten Alkylrest und ist insbesondere
Niederalkylsulfonyl, wie Methylsulfonyl, Ethylsulfonyl, n-Propylsulfonyl oder tert-Butylsulfonyl,
durch Arylniederalkyl substituiertes Sulfonyl, das beispielsweise einen unter Arylniederalkyl R₁, R₂, R₈ und R₉ genannten unsubstituierten oder substituierten Rest enthält und insbesondere ausgewählt ist aus den Resten Benzyl-, 4-Chlor-, 4-Methoxy-oder 4-Nitrobenzyl-, Naphthylmethyl-, z.B. α- oder β-Naphthylmethyl-, 2-Phenylethyl-, 2-α-Naphthylethyl-, 2-β-Naphthylethyl-, 2-(4-Methylphenyl)ethyl-, 2-(4-Methoxyphenyl)ethyl- 3-Phenylpropyl-, 3-(p-Hydroxyphenyl)-propyl-, 2,2-Diphenylethyl- und 2,2-Di-(4-methoxyphenyl)-ethyl-sulfonyl, oder
durch Heterocyclylniederalkyl substituiertes Sulfonyl, das beispielsweise einen unter Heterocyclyiniederalkyl R_{1,} R₂, R₈ und R₉ genannten unsubstituierten oder substituierten Rest enthält und insbesondere ausgewählt ist aus Resten wie 2- oder 3-Pyrrolyl-, 2-Thienyl-, 2-Furyl-, 1-Pyrazolyl-, 2-, 3- oder 4-Pyriddyl, 2-, 3- oder 5-Indolyl-, (1-Methyl-, 2-Methyl-, 5-Methoxy-, 5-Benzyloxy-, 5-Chlor- oder 4,5-Dimethyl-indol-2-yl)-, (1-Benzylindol-2-yl oder -3-yl)-, 4,5,6,7-Tetrahydroindol-2-yl-, (2-, 3-oder 4-Chinolyl oder 4-Hydroxychinol-2-yl)-, (1-, 3- oder 4-Isochinolyl oder 1-Oxo-1,2-dihydroisochinol-3-yl)-, 3-Pyrrolidinyl-, (3- oder 4-Hydroxypyrrolidin-2-yl)-, 5-Oxopyrrolidin-2-yl-, (2- oder 3-Morpholinyl)-, (2- oder 3-Thiomorpholinyl)-, (S,S-Dioxothiomorpholin-2- oder -3-yl)-, (2- oder 3-Indolinyl)-, (1,2,3,4-Tetrahydrochinol-2-, -3- oder -4-yl)- und (1,2,3,4-Tetrahydroisochinol-1-, -2- oder -3-yl)-methylsulfonyl.

Durch Aryl substituiertes Sulfonyl R₁, R₂, R₈ und R₉ enthält vorzugsweise einen unter Arylniederalkanoyl R₁, R₂, R₈ und R₉ genannten unsubstituierten oder substituierten Arylrest und ist insbesondere unsubstituiertes oder durch Niederalkyl ein- oder zweifach substituiertes Phenyl- oder 1- oder 2-Naphthyl-sulfonyl, wie Phenylsulfonyl, 2- oder 4-Toluolsulfonyl oder 1- oder 2- Naphthylsulfonyl.

Durch Alkoxy substituiertes Sulfonyl R₁, R₂, R₈ und R₉ enthält vorzugsweise einen unter Alkyl R₁, R₂, R₈ und R₉ genannten unsubstituierten oder substituierten Alkylrest und ist insbesondere ausgewählt aus
Niederalkoxy-, wie Methoxy-, Ethoxy- oder tert-Butoxy-sulfonyl,
Heterocyclylniederalkoxy-sulfonyl, welches beispielsweise einen unter Heterocyclylniederalkryl R₁, R₂, R₈ und R₉ genannten unsubstituierten oder substituierten Heterocyclylniederalkylrest enthält und insbesondere ausgewählt ist aus 2- oder 3-Pyrrolyl-, 2-Thienyl-, 2-Furyl-, 1-Pyrazolyl-, 2-, 3- oder 4-Pyridyl-, 2-, 3- oder 5-Indolyl-, (1-Methyl-, 2-Methyl-, 5-Methoxy-, 5-Benzyloxy-, 5-Chlor- oder 4,5-Dimethyl-indol-2-yl)-, (1-Benzylindol-2-yl oder -3-yl)-, 4,5,6,7-Tetrahydroindol-2-yl-, (2-, 3- oder 4-Chinolyl oder 4-Hydroxychinol-2-yl)-, (1-, 3- oder 4-Isochinolyl oder 1-Oxo1,2-dihydroisochinol-3-yl)-, 3-Pyrrolidinyl-, (3- oder 4-Hydroxypyrrolidin-2-yl)-, 5-Oxopyrrolidin-2-yl-, (2- oder 3-Morpholinyl)-, (2- oder 3-Thiomorpholinyl)-, (S,S-Dioxothiomorpholin-2- oder -3-yl)-, (2- oder 3-Indolinyl)-, (1,2,3,4-Tetrahydrochinol-2-, -3- oder -4-yl)- und (1,2,3,4-Tetrahydroisochinol-1-, -2- oder -3-yl)-methoxysulfonyl oder -ethoxysulfonyl,
Arylniederalkoxysulfonyl, das beispielsweise einen unter Arylniederalkyl R₁, R₂, R₈ und R₉ genannten unsubstituierten oder substituierten Arylniederalkylrest enthält, wie Benzyloxysulfonyl,
Hydroxyniederalkoxy-, wie 3-Hydroxypropoxy- oder 2-Hydroxy-3-methylpentoxy-sulfonyl,
Niederalkoxyniederalkoxy-, z. B. Niederalkoxyethoxy- oder Niederalkoxypropoxy-, wie Methoxyethoxy- oder 3-Methoxypropoxy-sulfonyl,
Niederalkoxyniederalkoxy-niederalkoxy-, wie 2-Methoxymethoxy-3-methyl-pentoxysulfonyl,
Phenoxyniederalkoxy- oder Nitrophenoxyniederalkoxy-, wie Phenoxymethoxy-, Phenoxyethoxy- oder 4-Nitrophenoxymethoxy-sulfonyl,
Naphthoxyniederalkoxy-, wie α- oder β-Naphthoxyethoxy-sulfonyl,
Niederalkanoyloxyniederalkoxy-, z.B. Niederalkanoyloxyethoxy- oder Niederalkanoyloxypropoxy-, wie Acetoxyethoxy- oder 3-Acetoxypropoxy-sulfonyl,
Aminoniederalkoxy-, wie 5-Aminopentoxy-sulfonyl,
Mono- oder Diniederalkylaminoniederalkoxy-, wie Dimethylaminoethoxy- oder 2-Dimethylamino-2-isopropylethoxy-sulfonyl,
Niederalkanoylamino-niederalkoxy-, wie 4-Acetylaminopentoxy-sulfonyl,
Niederalkoxycarbonylamino-niederalkoxy-, wie 5-(tert-Butoxycarbonylamino)-pentoxy- oder 3-Ethoxycarbonylamino-2-isobutyl-propoxy-sulfonyl,
Phenylniederalkoxycarbonylamino-niederalkoxy-, wie 5-(Benzyloxycarbonylamino)pentoxy-sulfonyl,
Acetoacetoxy-sulfonyl,
Aminocarbonylamino-niederalkoxy-, wie Aminocarbonylamino-ethoxy-sulfonyl,
N-Phenylniederalkyl-N-niederalkyl-aminocarbonylamino-niederalkoxy-, z. B. 2-Iso-butyl-3-(N-benzyl-N-methylaminocarbonylamino)propoxy-sulfonyl,
Halogenniederalkoxy-, z.B. 2-Halogenethoxy-, wie (2-Chlor-, 2-Brom-, 2-Iod- oder 2,2,2-Trichlorethoxy)-, oder Halogenpropoxy-, wie (3-Chlor- oder 3-Brompropoxy)-sulfonyl,
Carboxyniederalkoxy-, wie Carboxyethoxy- oder 3-Carboxypropoxy-sulfonyl,
Niederalkoxycarbonylniederalkoxy-, z. B. Niederalkoxycarbonylethoxy- oder Niederalkoxycarbonylpropoxy-, wie Methoxycarbonylethoxy-, 3-Methoxycarbonylpropoxy-, Ethoxycarbonylethoxy- oder 3-Ethoxycarbonylpropoxy-sulfonyl,
2-Halogenniederalkoxycarbonyl-niederalkoxy-, wie (2-Chlor-, 2-Brom-, 2-Iod- oder 2,2,2-Trichlorethoxycarbonyl-2-ethoxy oder 3-propoxy)-sulfonyl,
Niederalkylsulfonylniederalkoxy-, wie (2-Ethylsulfonyl- oder 2-tert-Butylsulfonyl-methoxy)-sulfonyl,
Carbamoylniederalkoxy-, wie Carbamoylethoxy- oder 3-Carbamoylpropoxy-sulfonyl, und
durch einen aus Ethylen, Trimethylen, Tetramethylen und Pentamethylen ausgewählten Rest, worin ein Kohlenstoffatom durch Stickstoff, Sauerstoff, Schwefel oder mit Sauerstoff ein- oder zweifach substituierten Schwefel ersetzt sein kann, am Stickstoff substituiertem Carbamoylniederalkoxy-sulfonyl, wobei der so gebildete Rest auch ganz oder teilweise ungesättigt sein kann, z. B. als (Piperidino-, Pyrazin-1-yl-, Piperazin-1-yl-, Pyrimidin-1-yl-, Pyridazin- 1-yl-, Morpholino-, Thiomorpholino- oder S,S-Dioxothiomorpholino-carbonylniederalkoxy)-, wie in 2-Morpholinocarbonyl-ethoxy, 3-(Morpholinocarbonyl)-propoxy oder 3-(Morpholinocarbonyl)-2-isobutyl-propoxy- sulfonyl.

Durch Aryloxy substituiertes Sulfonyl R₁, R₂, R₈ und R₉ enthält vorzugsweise einen unter Arylniederalkanoyl R₁, R₂, R₈ und R₉ genannten unsubstituierten oder substituierten Arylrest und ist insbesondere ausgewählt aus Benzyloxysulfonyl und 1- oder 2-Napthoxysulfonyl.

Am Stickstoff substituiertes Sulfamoyl R₁, R₂, R₈ und R₉ kann vorzugsweise durch dieselben Reste wie Carbamoyl in Carbamoylniederalkanoyl R₁, R₂, R₈ und R₉ substituiert sein und ist insbesondere ausgewählt aus Mono- oder Diniederalkylsulfamoyl, wie N,N-Dimethylsulfamoyl, N-(Phenyl- oder Naphthylniederalkyl)sulfamoyl, wie 3-Benzylsulfamoyl, und am Stickstoffatom von einem aus Ethylen, Trimethylen, Tetramethylen oder Pentamethylen ausgewählten Rest, worin ein Kohlenstoffatom durch Stickstoff, mit Niederalkyl, wie Methyl, substituierten Stickstoff, Sauerstoff, Schwefel oder mit Sauerstoff ein- oder zweifach substituierten Schwefel ersetzt sein kann, substituiertes Sulfamoyl, wobei der so gebildete Rest auch ganz oder teilweise ungesättigt sein kann, z. B. als Piperidino-, Pyrazin-1-yl-, Piperazin-1-yl-, Methylpiperazin-1-yl, Pyrimidin- 1-yl-, Pyridazin-1-yl-, Morpholino-, Thiomorpholino- oder S,S-Dioxothiomorpholino-sulfonyl, wie 4-Methylpiperazinylsulfonyl oder Morpholinosulfonyl)-2-isopropyl-propyl.

Durch einen oder zwei unabhängig voneinander aus Alkyl, Cycloalkyl, Cycloalkylnieder-alkyl, Aryl, Arylniederalkyl, Hydroxy, Alkoxy, Cycloalkoxy, Cycloalkylniederalkoxy, Aryloxy und Arylniederalkoxy ausgewählte Reste substituiertes Phosphoryl R₁, R₂, R₈ und R₉ enthält vorzugsweise
als unsubstituiertes oder substituiertes Alkyl einen oder zwei der unter Alkyl, insbesondere Niederalkyl R₁, R₂, R₈ und R₉ genannten unsubstituierten oder substituierten Reste, z. B.
-Niederalkyl, wie Methyl, Ethyl, n-Propyl, Isopropyl, n-Butyl, tert-Butyl,
-Cycloalkylniederalkyl, welches einen insbesondere endständig durch einen der unter Cycloalkylniederalkanoyl R₁, R₂, R₈ und R₉ genannten Cycloalkylreste substituierten Niederalkylrest bedeutet, wie Cyclopropylmethyl, Cyclobutylmethyl, Cyclopentylmethyl, 2-Cyclopentylethyl, 3-Cyclopentylpropyl, Cyclohexylmethyl, 2-Cyclohexylethyl oder 3-Cyclohexylpropyl,
-Arylniederalkyl, definiert wie unter Arylniederalkyl R₁, R₂, R₈ oder R₉, insbesondere Benzyl, unsubstituiert, mono- oder mehrfachsubstituiert vorzugsweise durch Niederalkyl, z.B. Methyl, Phenyl, Hydroxy, Niederalkoxy, z.B. Methoxy, Halogen, z.B. Chlor, und/oder Nitro, wie 4-Methoxy-, 4-Chlor- oder 4-Nitrobenzyl, Naphthylmethyl, wie α-oder β-Naphthylmethyl, 2-Phenylethyl, 2-α-Naphthylethyl, 2-β-Naphthylethyl, 2-Niederalkyl-phenyl-ethyl, wie 2-(4-Methylphenyl)ethyl, 2-Niederalkoxyphenylethyl, wie 2-(4-Methoxyphenyl)ethyl, 2,2-Diphenylethyl, 2,2-Di-(4-methoxyphenyl)-ethyl, 2,2,2-Tri-phenylethyl, 2,2-Dibenzylethyl, 2,p-Diamino-phenylethyl, oder aus durch zwei aus Phe-nylniederalkoxycarbonyl, z. B. Benzyloxycarbonyl, und Niederalkanoylamino, z. B. Pivaloylamino, ausgewählte Reste in 2- und p-Stellung substituiertes Phenylniederalkyl, wie 2,p-Dibenzyloxycarbonylamino-phenylethyl oder 2-Pivaloylamino-p-benzyloxycar-bonylamino-phenylethyl, 3-Phenylpropyl, 3-(p-Hydroxyphenyl)-propyl, 3-α- oder 3-β-Naphthylpropyl, 2-Benzyl-3-(1-pyrazolyl-)-propyl, 3-Phenyl- oder 3-α-Naphthyl-2-hyd-roxy-propyl, 3-Phenyl- oder 3-α-Naphthyl-2-niederalkoxy-propyl, wie 3-Phenyl- oder 3-α-Naphthyl-2-neopentyloxy-propyl, 3-Phenyl- oder 3-α-Naphthyl-2-niederalkanoyl-oxy-propyl, wie 3-Phenyl- oder 3-α-Naphthyl-2-pivaloyloxy- oder -2-acetoxypropyl, 2-Benzyl- oder 1- oder 2-Naphthyl-3-(N-methoxyl-N-methylamino)-propyl, 3-Phenyl-oder 3-α-Naphthyl-2-dimethylaminomethyl-propyl, 3-α-Naphthyl-2-acetoacetoxy-propyl, 3-α-Naphthyl-2-ethylaminocarbonyloxy-propyl oder 3-α-Naphthyl-2-(2-amino- oder 2-benzyloxycarbonylamino-2-methylpropionyloxy)-propyl, 3-Phenyl- oder 3-α-Naph-thyl-2-carboxymethylpropyl, 3-Phenyl- oder 3-α-Naphthyl-2-niederalkoxycarbonyl-pro-pyl, wie 3-α-Naphthyl-2-ethoxycarbonyl-propyl, 3-Phenyl- oder 3-α-Naphthyl-2-benzyl-oxycarbonylmethyl-propyl, 2-(S)-Benzyl-3-tert-butylsulfonyl-propyl, 3-Phenyl-2-phos-phono- oder phosphonomethyl-propyl, 3-Phenyl-2-dimethoxyphosphoryl- oder dimeth-oxyphosphorylmethyl-propyl, 3-Phenyl-2-diethoxyphosphoryl- oder diethoxyphosphoryl-methyl-propyl, 3-Phenyl-2-ethoxy- oder -methoxy-hydroxyphosphorylpropyl, 3-Phenyl- oder 3-α-Naphthyl-2-carbamoyl-propyl, 3-Phenyl- oder 3-α-Naphthyl-2-tert-butyl-carbamoyl-propyl, 3-Phenyl- oder 3-α-Naphthyl-2-(2-dimethylaminoethyl)carbamoyl-propyl, 3-α-Naphthyl-2-(carboxy- oder tert-butoxycarbonyl)methylcarbamoyl-propyl, 3-Phenyl- oder 3-α-Naphthyl-2-(3-hydroxy-2-propyl)-carbamoylpropyl, 3-Phenyl- oder 3-α-Naphthyl-2-(2,2-dimethoxyethyl)-carbamoyl-propyl, 3-Phenyl- oder 3-α-Naphthyl-2-(5-amino-5-carboxypentyl)-carbamoyl-propyl, 3-Phenyl- oder 3-α-Naphthyl-2-cyano-pro-pyl, 3-Phenyl- oder 3-α-Naphthyl-2-cyanomethyl-propyl, 3-Phenyl- oder 3-α-Naphthyl-2-acetonyl-propyl, 4-Hydroxyphenylbutyl, 4-Phenyl- oder 4-α-Naphthyl-3-carboxy-butyl, 4-Phenyl- oder 4-α-Naphthyl-3-benzyloxycarbonyl-butyl, 2-Benzyl- oder 2-α-Naphthyl-methyl-4-cyano-butyl, 2-Benzyl-4-(2-benzofuranyl)-4-oxobutyl, 2-Benzyl- oder 2-α-Naphthylmethyl-5-dimethylamino-pentyl, 2-Benzyl- oder 2-α-Naphthylmethyl-4-oxo-pen-tyl, 2-Benzyl- oder 2-α-Naphthylmethyl-4,4-dimethyl-3-oxo-pentyl, 2-Benzyl- oder 2-α-Naphthylmethyl-5-dimethylamino-4-oxo-pentyl, oder 2-Benzyl- oder 2-α-Naphthyl-methyl5,5-dimethyl-4-oxo-hexyl,
als Cycloalkyl einen der unter Cycloalkylniederalkanoyl R₁, R₂, R₈ und R₉ genannten Reste, insbesondere Cyclopropyl, Cyclobutyl, Cyclopentyl oder Cyclohexyl,
als Aryl einen der unter Arylniederalkanoyl R₁, R₂, R₈ und R₉ genannten unsubstituierten oder substituierten Reste, vorzugsweise Phenyl, unsubstituiert, mono- oder mehrfachsubstituiert durch Niederalkyl, z. B. Methyl, Halogenniederalkyl, z. B. Trifluormethyl, Phenyl, Hydroxy, Niederalkoxy, z. B. Methoxy, Halogen, z. B. Fluor oder Chlor, und/oder Nitro, wie 4-Methyl-, 3-Hydroxy-, 4-Methoxy-, 4-Chlor- oder 4-Nitrophenyl, Naphthyl, wie α- oder β-Naphthyl, oder im Phenylrest durch einen oder zwei aus Niederalkyl, wie Methyl oder Ethyl, Hydroxy, Niederalkoxy, wie Methoxy, Amino, Mono- oder Dinieder-alkylamino, wie Ethylamino oder Dimethylamino, Halogen, wie Fluor oder Chlor, Carboxy, Sulfo, Carbamoyl, Sulfamoyl und Cyano, und/oder an der Aminogruppe aus Niederalkyl und Benzyl ausgewählten Resten substituiertes Anilinophenyl, wie 2-(o,o-Dichloranilino)-phenyl oder 2-(o,o-Dichloro-N-benzylanilino)-phenyl,
als Alkoxy einen der bei durch Alkoxy substituiertes Sulfonyl R₁, R₂, R₈ und R₉ genannten unsubstituierten oder substituierten Reste, insbesondere Niederalkoxy, wie Methoxy, Ethoxy oder tert-Butoxy, Cycloalkylniederalkoxy mit einem über Sauerstoff gebundenen der unter dwch Cycloalkylniederalkyl substituiertes Phosphoryl R₁, R₂, R₈ und R₉ genannten Reste, wie Cyclopropylmethoxy, Cyclobutylmethoxy, Cyclopentylmethoxy, 2-Cyclo-pentylethoxy, 3-Cyclopentylpropoxy, Cyclohexylmethoxy, 2-Cyclohexylethoxy oder 3-Cyclohexylpropoxy, Arylniederalkoxy mit einem über Sauerstoff gebundenen der unter durch Arylniederalkoxy substituiertes Sulfonyl R₁, R₂, R₈ und R₉ genannten Reste, insbesondere Benzyloxy, Hydroxyniederalkoxy, wie 3-Hydroxypropoxy oder 2-Hydroxy-3-methylpentoxy, Niederalkoxyniederalkoxy, z.B. Niederalkoxyethoxy oder Niederalkoxypropoxy, wie Methoxyethoxy oder 3-Methoxypropoxy, Niederalkoxyniederalkoxy-niederalkoxy, wie 2-Methoxymethoxy-3-methyl-pentoxy, Phenoxyniederalkoxy oder Nitrophenyloxyniederalkoxy, wie Phenoxymethoxy, Phenoxyethoxy oder 4-Nitrophenyloxymethoxy, Naphthoxyniederalkoxy, wie α- oder β-Naphthoxyethoxy, Niederalkanoyloxyniederalkoxy, wie Niederalkanoyloxyethoxy oder Niederalkanoyloxypropoxy, wie Acetoxyethoxy oder 3-Acetoxypropoxy, Aminoniederalkoxy, wie 5-Aminopentoxy, Mono- oder Diniederalkylaminoniederalkoxy, wie Dimethylaminoethoxy oder 2-Dimethylamino-2-isopropylethoxy, Niederalkanoylamino-niederalkoxy, wie 4-Acetylaminopentoxy, Nie-deralkoxycarbonylamino-niederalkoxy, wie 5-(tert-Butoxycarbonylamino)-pentoxy oder 3-Ethoxycarbonylamino-2-isobutyl-propoxy, Phenylniederalkoxycarbonylamino-nie-deralkoxy, wie 5-(Benzyloxycarbonylamino)-pentoxy, Aminocarbonylamino-niederalk-oxy, wie Aminocarbonylamino-ethoxy, N-Phenylniederalkyl-N-niederalkyl-aminocar-bonylamino-niederalkoxy, wie 2-Isobutyl-3-(N-benzyl-N-methylaminocarbonylami-no)propoxy, Halogenniederalkoxy, z.B. 2-Halogenethoxy, wie 2-Chlor-, 2-Brom-, 2-Iod- oder 2,2,2-Trichlorethoxy, oder Halogenpropoxy, wie 3-Chlor- oder 3-Brompropoxy, Carboxyniederalkoxy, wie Carboxyethoxy oder 3-Carboxypropoxy, Niederalkoxycar-bonylniederalkoxy, z.B. Niederalkoxycarbonylethoxy oder Niederalkoxycarbonylpropoxy, wie Methoxycarbonylethoxy, 3-Methoxycarbonylpropoxy, Ethoxycarbonylethoxy oder 3-Ethoxycarbonylpropoxy, 2-Halogenniederalkoxycarbonyl-niederalkoxy, wie 2-Chlor-, 2-Brom-, 2-Iod- oder 2,2,2-Trichlorethoxycarbonyl-2-ethoxy oder 3-propoxy, Nieder-alkylsulfonylniederalkoxy, wie 2-Ethylsulfonyl- oder 2-tert-Butylsulfonylmethoxy, Carbamoylniederalkoxy, wie Carbamoylethoxy oder 3-Carbamoylpropoxy, oder durch einen aus Ethylen, Trimethylen, Tetramethylen und Pentamethylen ausgewählten Rest, worin ein Kohlenstoffatom durch Stickstoff, Sauerstoff, Schwefel oder mit Sauerstoff ein-oder zweifach substituierten Schwefel ersetzt sein kann, am Stickstoff substituiertes Carbamoylniederalkoxy, wobei der gebildete Ring auch ganz oder teilweise ungesättigt sein kann, z. B. als (Piperidino-, Pyrazin-1-yl-, Piperazin- 1-yl-, Pyrimidin-1-yl, Pyridazin-1-yl-, Morpholino-, Thiomorpholino- oder S,S-Dioxothiomorpholino-carbonyl)niederalkoxy-, wie in 2-Morpholinocarbonyl-ethoxy, 3-(Morpholinocarbonyl)propoxy oder 3-(Morpholinocarbonyl)-2-isobutyl-propoxy,
als Cycloalkoxy einen über Sauerstoff gebundenen der unter Cykloalkylniederalkanoyl R₁, R₂, R₈ und R₉ genannten Cycloalkylreste, wie Cyclobutyloxy, Cyclopentyloxy oder Cyclohexyloxy, oder
als Aryloxy einen der unter durch Aryloxy substituiertes Sulfonyl R₁, R₂, R₈ und R₉ genannten Reste, insbesondere Benzyloxy oder 1- oder 2-Napthyloxy,
und ist in besonders bevorzugter Weise Niederalkylphosphoryl, wie tert-Butylphosphoryl, Hydroxy-niederalkoxyphosphoryl, wie Hydroxy-methoxy-phosphoryl oder Hydroxyethoxy-phosphoryl, oder Diniederalkoxyphosphoryl, wie Dimethoxy-phosphoryl oder Diethoxy-phosphoryl.

Die Reste Phosphono und die für substituiertes Phosphoryl genannten einzelnen Definitionen der Reste R₁, R₂, R₈ oder R₉ können auch unabhängig voneinander bei der Definition der Reste R₁, R₂, R₈ oder R₉ in den Verbindungen der Formel I weggelassen werden.

Vorzugsweise ist nur maximal jeweils einer der Reste R₁ und R₂ beziehungsweise der Reste R₈ und R₉ durch die unter Acyl, Sulfo, substituiertes Sulfo, Phosphono oder substituiertes Phosphoryl genannten Reste definiert, während der jeweils andere Rest aus den restlichen genannten Substituenten ausgewählt ist.

Ein unabhängig voneinander durch die Substituentenpaare R₁ und R₂ oder R₈ und R₉ gemeinsam mit dem Stickstoffatom, an das sie gebunden sind, gebildeter heterocyclischer Ring bestehend aus dem bindenden Stickstoff zusammen mit einem aus Ethylen, Trimethylen, Tetramethylen und Pentamethylen ausgewählten Rest, worin ein Kohlenstoffatom durch Stickstoff, Sauerstoff, Schwefel oder mit Sauerstoff ein-oder zweifach substituierten Schwefel ersetzt sein kann und welcher ungesättigt sein kann, ist vorzugsweise Piperidino, Pyrazin-1-yl, Piperazin-1-yl, Pyrimidin-1-yl, Pyridazin-1-yl, Morpholino, Thiomorpholino oder S,S-Dioxothiomorpholino.

Naphthatin-1,8-dicarbonylimido wird vorzugsweise nur durch eines der Substituentenpaare R₁ und R₂ oder R₈ und R₉ gebildet.

Unsubstituiertes oder substituiertes Alkyl R₃, R₄ oder R₇ ist vorzugsweise einer der unter Alkyl R₁, R₂, R₈ und R₉ genannten unsubstituierten oder substituierten Reste, und ist insbesondere ausgewählt aus
Niederalkyl, z. B. Methyl, Ethyl, Propyl, Isopropyl, n-Butyl, Isobutyl oder tert-Butyl,
Cycloalkylniederalkyl, welches beispielsweise die unter Cycloalkylniederalkanoyl R₁, R₂, R₈ oder R₉ genannten Cycloalkylreste enthält, wie Cyclopropyl, Cyclobutyl, Cyclopentyl, Cyclohexyl oder Cycloheptyl, wobei Cycloalkyl unsubstituiert oder durch Niederalkyl, wie Isopropyl, Halogenniederalkyl, wie Trifluormethyl, Hydroxy, Niederalkoxy, Carbamoylniederalkoxy, N-Niederalkylcarbamoylniederalkoxy oder N,N-Diniederalkylcarbamoylniederalkoxy, Amino, Mono- oder Diniederalkylamino, Halogen, wie Fluor, Chlor oder Brom, Carboxy, Niederalkoxycarbonyl, Phenyl-, Naphthyl-oder Fluorenylniederalkoxycarbonyl, Niederalkanoyl, Niederalkylsulfonyl, z. B. Methylsulfonyl, Phosphono, Hydroxy-niederalkoxy-phosphoryl oder Diniederalkoxyphosphoryl, Carbamoyl, Mono- oder Diniederalkylaminocarbamoyl, Sulfamoyl, Mono- oder Diniederalkylsulfamoyl, Nitro und/oder Cyano ein- oder bis zu dreifach substituiert ist und an Niederalkyl, insbesondere Methyl, Ethyl, n-Propyl, Isopropyl, N-Butyl, Isobutyl, tert-Butyl, vorzugsweise endständig, gebunden ist, wie Cyclobutyl-, Cyclopentyl-, Cyclohexyl-oder Cycloheptyl-niederalkyl, wie -methyl oder -ethyl, besonders bevorzugt Cyclohexylniederalkyl, wie Cyclohexylmethyl,
Bicycloalkylniederalkyl, worin Bicylcoalkyl z.B. 5 - 10, insbesondere 6 - 9, Kohlenstoffatome enthält, z.B. Bicycloalkylmethyl oder -ethyl, vorzugsweise mit 8 - 11 Kohlenstoffatomen, wie Decahydronaphthyl-2-methyl, endo- oder exo-Norbornyl-2-methyl, Bicyclo[2.2.2]oct-2-ylmethyl oder Bicyclo[3.3.1]-non-9-ylmethyl, ferner Bicyclohexyl-, -heptyl-, -octyl-, -nonyl- oder -decyl-ethyl oder -3-propyl, z.B. Bicyclo[3.1.0]hex-1-,-2- oder -3-yl-, Bicyclo[4.1.0]hept-1- oder -7-yl-, Bicyclo[2.2.1]hept-2-yl-, z.B. endo-oder exo-Norbornyl-, Bicyclo[3.2.1]oct-2-yl-, Bicyclo[3.3.0]oct-3-yl- oder Bicyclo[3.3.1]non-9-yl-, ferner α- oder β-Decahydronaphthyl-ethyl oder -3-propyl,
Tricycloalkylniederalkyl, worin Tricycloalkyl z.B. 8 - 10 Kohlenstoffatome enthält, z.B. Tricycloalkylmethyl oder -ethyl, vorzugsweise mit 8 - 11 Kohlenstoffatomen, wie 1-oder 2-Adamantylmethyl, ferner Tricyclo[5.2.1.0^{2,6}]dec-8-yl- oder Adamantyl-, wie 1-Adamantyl-ethyl,
Arylniederalkyl, beispielsweise definiert wie unter Arylniederalkyl R₁, R₂, R₈ oder R₉, welches unsubstituiert oder wie dort substituiert ist, z. B. Phenylniederalkyl, wie Benzyl, 2-Phenylethyl, 3-Phenylpropyl, 4- Fluor-, 4-Cyano-, 4-Methoxy- oder 4-Hydroxybenzyl, 1- oder 2-Naphthylmethyl oder -2-ethyl, besonders bevorzugt Phenylniederalkyl, wie zuletzt definiert,
Heterocyclylniederalkyl, beispielsweise definiert wie unter Heterocyclylniederalkyl R₁, R₂, R₈ oder R₉, das unsubstituiert oder wie dort substituiert ist, insbesondere ausgewählt aus Pyrimidin-1-yl-, Piperidino-, Pyrazin-1-yl-, Piperazin-1-yl-, Pyridazin-1-yl-, Morpholino-, Thiomorpholino- oder S,S-Dioxothiomorpholino-niederalkyl, wie in 2-Morpholino-ethyl, 3-Morpholino-propyl oder 3-Morpholino2-isobutyl-propyl, unsubstituiertes oder durch Niederalkyl oder Phenyl substituiertes Pyrrolylniederalkyl, wie 2- oder 3-Pyrrolylmethyl, -ethyl oder -n-propyl, 4- oder 5-Methylpyrrolylmethyl, -ethyl oder -n-propyl oder 4- oder 5-Phenylpyrrolylmethyl, -ethyl oder -n-propyl, Thienyl-niederalkyl, wie 2-Thienylmethyl, -ethyl oder -n-propyl, 1-Imidazolylmethyl, Furyl-niederalkyl, wie 2-Furylmethyl, -ethyl oder -n-propyl, Pyridylniederalkyl, wie 2-, 3- oder 4-Pyridylmethyl, -ethyl oder -n-propyl, unsubstituiertes oder durch Niederalkyl, z. B. Methyl, Phenylniederalkyl, z. B. Benzyl, Niederalkoxy, z. B. Methoxy, Phenylnieder-alkoxy, z. B. Benzyloxy, oder Halogen, z. B. Chlor, substituiertes Indolylniederalkyl, wie 2-, 3- oder 5-Indolylmethyl, -ethyl oder -n-propyl, 1-Methyl-, 2-Methyl-, 5-Methoxy-, 5-Benzyloxy-, 5-Chlor- oder 4,5-Dimethylindol-2-yl-, 1-Benzylindol-2-yl- oder -3-yl-methyl, -ethyl oder -n-propyl, 4,5,6,7-Tetrahydroindol-2-ylmethyl, -ethyl oder - n-propyl, Cyclohepta[b]pyrrol-5-yl-methyl, -ethyl oder -n-propyl, unsubstituiertes oder durch Hydroxy substituiertes Chinolylniederalkyl, z. B. 2-, 3- oder 4-Chinolyl- oder 4-Hydroxychinol-2-yl-methyl, -ethyl oder -n-propyl, unsubstituiertes oder durch Hydroxy substituiertes Isochinolylniederalkyl, wie 1-, 3- oder 4-Isochinolyl- oder 1-Oxo-1,2-dihydroisochinol-3-yl-methyl, -ethyl oder -n-propyl, 2-Chinoxalinylmethyl, -ethyl oder -n-propyl, 3,1-Benzfuran-2-yl-methyl, -ethyl oder -n-propyl, Benz[e]-indol-2-yl-methyl, -ethyl oder -n-propyl, β-Carbolin-3-yl-methyl, -ethyl oder - n-propyl, 3-Chromanyl-methyl, -ethyl oder -n-propyl, 3-Thiochromanyl-methyl, -ethyl oder - n-pro-pyl, 3- oder 4-Pyrrolidinyl-methyl, -ethyl oder -n-propyl, Hydroxypyrrolidinylniederalkyl, wie 3- oder 4-Hydroxypyrrolidin-2-yl-methyl, -ethyl oder -n-propyl, Oxopyrroli-dinylniederalkyl, wie 5-Oxopyrrolidin-2-yl-methyl, -ethyl oder -n-propyl, Piperidinyl-niederalkyl, wie 2-, 3- oder 4-Piperidinyl-methyl, -ethyl oder -n-propyl, Morpholinyl-niederalkyl, wie 2- oder 3-Morpholinyl-methyl, -ethyl oder -n-propyl, Thiomorpholinyl-niederalkyl, wie 2- oder 3-Thiomorpholinyl-methyl, -ethyl oder -n-propyl, S,S-Dioxo-thiomorpholinylniederalkyl, wie S,S-Dioxothiomorpholin-2- oder -3-yl-methyl, -ethyl oder -n-propyl, Indolinylniederalkyl, wie 2- oder 3-Indolinyl-methyl, -ethyl oder -n-propyl, 1,2,3,4-Tetrahydrochinolylniederalkyl, wie 1,2,3,4-Tetrahydrochinol-2-, -3- oder -4-yl-methyl, -ethyl oder -n-propyl, 1,2,3,4-Tetrahydroisochinolylniederalkyl, wie 1,2,3,4-Tetrahydroisochinol-1-, -2- oder -3-yl-methyl, -ethyl oder -n-propyl, und 1-Oxo-1,2,3,4-tetrahydroisochinol-3-yl-methyl, -ethyl oder -n-propyl.
Hydroxyniederalkyl, wie 3-Hydroxypropyl oder 2-Hydroxy-3-methylpentyl,
Niederalkoxyniederalkyl, z.B. Niederalkoxyethyl oder Niederalkoxypropyl, wie 2-Methoxyethyl, 2-Ethoxyethyl oder 3-Methoxypropyl,
Phenoxyniederalkyl oder Nitrophenoxyniederalkyl, wie Phenoxymethyl, Phenoxyethyl oder 4-Nitrophenoxymethyl,
Naphthoxyniederalkyl, wie α- oder β-Naphthoxyethyl,
Niederalkanoyloxyniederalkyl, z.B. Niederalkanoyloxyethyl oder Niederalkanoyloxy-propyl, wie Acetoxyethyl oder 3-Acetoxypropyl,
Acetoacetoxyniederalkanoyl,
Arylmercaptoniederalkyl, worin Aryl 6 - 10 Kohlenstoffatome hat, z. B. Phenyl oder Naphthyl, wie Phenylmercaptomethyl,
Aminoniederalkyl, wie 5-Aminopentyl,
Mono- oder Diniederalkylaminoniederalkyl, wie Dimethylaminoethyl oder 2-Dimethyl-amino-2-isopropylethyl,
Phenyl- oder Napthylaminoniederalkyl, wie 3-Phenylaminopropyl
Niederalkanoylamino-niederalkyl, wie 4-Acetylaminopentyl,
durch Niederalkyl, wie Methyl, am Stickstoff substituiertes Piperazinylcarbamoylnieder-alkyl, wie 4-Methylpiperazinylcarbonylmethyl
Niederalkoxycarbonylamino-niederalkyl, wie 5-(tert-Butoxycabonylamino)-pentyl oder 3-Ethoxycarbonylamino-2-isobutyl-propyl,
Phenylniederalkoxycarbonylamino-niederalkyl, wie 5-(Benzyloxycarbonylamino)-pentyl,
Aminocarbonylamino-niederalkyl, wie 2-Aminocarbonylamino-ethyl,
N-Phenylniederalkyl-N-niederalkylaminocarbonylamino-niederalkyl, wie 2-Isobutyl-3-(N-benzyl-N-methylaminocarbonylamino)propyl,
Halogenniederalkyl, worin Halogen aus Fluor, Chlor, Brom oder Iod ausgewählt ist, z.B. 2-Halogenethyl, wie 2-Fluor-, 2-Chlor-, 2-Brom-, 2-Iod-, 2,2,2-Trifluor-oder 2,2,2-Trichlorethyl, Trifluorniederalkyl, wie Trifluormethyl, oder Halogenpropyl, wie 3-Fluor-, 2-Chlor- oder 3-Brompropyl,
Carboxyniederalkyl, wie Carboxyethyl oder 3-Carboxypropyl,
Niederalkoxycarbonylniederalkyl, z.B. Niederalkoxycarbonylethyl oder Niederalkoxy-carbonylpropyl, wie Methoxycarbonylethyl, 3-Methoxycarbonylpropyl, Ethoxycarbonyl-ethyl oder 3-Ethoxycarbonylpropyl,
2-Halogenniederalkoxycarbonyl-niederalkyl, wie 2-Chlor-, 2-Brom-, 2-Iod- oder 2,2,2-Trichlorethoxycarbonyl-2-ethyl oder 3-propyl,
Phenyl- oder Naphthylniederalkyloxycarbonyl-niederalkyl, z. B. Benzyloxycarbonyl-niederalkyl, wie 3-Benzyloxycarbonyl-2,2-dimethylpropyl,
Heterocyclylniederalkoxycarbonyl-niederalkyl, worin Heterocyclyl vorzugsweise ausge-wählt ist aus Pyrrolyl, Furyl, Thienyl, Imidazolyl, Pyrazolyl, Oxazolyl, Thiazolyl, Pyridyl, Pyrazinyl, Pyrimidinyl, Indolyl, Chinolyl, Isochinolyl, Chinoxalinyl, β-Carbolinyl und einem benzanellierten, cyclopenta-, cyclohexa- oder cyclohepta-anellierten Derivat dieser Reste, wobei die genannten Reste auch ganz oder teilweise gesättigt vorliegen können, wie in 4-Pyridylmethyl-oxycarbonyl-2-ethyl oder -3-propyl oder 2-Morpholinocarbonyl-oxy-4-methyl-pentyl,
Niederalkylsulfonylniederalkyl, z. B. 2-Ethylsulfonyl- oder 2-tert-Butylsulfonylmethyl,
Arylsulfonylniederalkyl, worin Aryl vorzugsweise 6 - 10 Kohlenstoffatome hat, z. B. Phenyl oder Napthyl, wie Phenylsulfomethyl,
Carbamoylniederalkyl, wie Carbamoylethyl oder 3-Carbamoylpropyl,
Niederalkylcarbamoylniederalkyl, z.B. Niederalkylcarbamoylethyl oder Methyl-carbamoylniederalkyl, wie 2-Methylcarbamoylethyl,
Diniederalkylcarbamoylniederalkyl, z.B. 2-Diniederalkylcarbamoylethyl oder Dimethyl-carbamoylniederalkyl, wie 2-Dimethylcarbamoylethyl,
Hydroxyniederalkylcarbamoyl- oder Di-(hydroxyniederalkyl)carbamoyl-niederalkyl, wie 2-Hydroxymethylcarbamoyl- oder Di-(hydroxymethyl)carbamoyl-2-ethyl oder -3-propyl,
N-Niederalkoxyniederalkoxyniederalkyl-carbamoyl-niederalkyl, wie 2-Isobutyl-3-(2-(2-methoxyethoxy)ethylaminocarbonyl)-propyl,
Carboxyniederalkylcarbamoyl- oder Di-(carboxyniederalkyl)carbamoyl-niederalkyl, z. B. Carboxymethyl- oder Di-(carboxymethyl)carbamoyl-2-ethyl oder -3-propyl,
durch einen aus Ethylen, Trimethylen, Tetramethylen und Pentamethylen ausgewählten Rest, worin ein Kohlenstoffatom durch Stickstoff, Sauerstoff, Schwefel oder mit Sauer-stoff ein- oder zweifach substituierten Schwefel ersetzt sein kann, am Stickstoff substi-tuiertes Carbamoylniederalkyl, wobei der so gebildete Rest auch ganz oder teilweise unge-sättigt sein kann, z. B. als Piperidino-, Pyrazin-1-yl-, Piperazin-1-yl-, Pymidin-1-yl-, Pyridazin-1-yl-, Morpholino-, Thiomorpholino- oder S,S-Dioxothiomorpholino-carbonyl-niederalkyl, wie in 2-Morpholinocarbonyl-ethyl, 3-(Morpholinocarbonyl)-propyl oder 3-(Morpholinocarbonyl)-2-isobutyl-propyl,
N-Niederalkyl-N-heterocyclylniederalkylcarbamoylniederalkyl, worin Heterocyclyl vor-zugsweise aus Pyrrolyl, Furyl, Thienyl, Imidazolyl, Pyrazolyl, Oxazolyl, Thiazolyl, Pyridyl, Pyrazinyl, Pyrimidinyl, Indolyl, Chinolyl, Isochinolyl, Chinoxalinyl, β-Carbolinyl und einem benzanellierten, cyclopenta-, cyclohexa- und cyclohepta-anellierten Derivat dieser Reste, die auch ganz oder teilweise gesättigt sein können, ausgewählt ist, wie 2-(N-2-Pyridylmethyl)-N-methylcarbamoyl-ethyl,
Sulfamoylniederalkyl, wie 2-Sulfamoylethyl,
N-(Phenyl- oder Naphthylniederalkyl)sulfamoylniederalkyl, wie 3-Benzylamino-sulfonyl-2-isopropyl-propyl, oder
am Stickstoffatom von einem aus Ethylen, Trimethylen, Tetramethylen und Pentamethy-len ausgewählten Rest, worin ein Kohlenstoffatom durch Stickstoff, mit Niederalkyl, wie Methyl, substituierten Stickstoff, Sauerstoff, Schwefel oder mit Sauerstoff ein-oder zweifach substituierten Schwefel ersetzt sein kann, substituiertes Sulfamoylniederalkyl, wobei der so gebildete Rest auch ganz oder teilweise ungesättigt sein kann, z. B. als Piperidino-, Pyrazin-1-yl-, Piperazin-1-yl-, 4-Methylpiperazin-1-yl, Pyrimidin-1-yl-, Pyridazin-1-yl-, Morpholino-, Thiomorpholino- oder S,S-Dioxothiomorpholino-sulfonylnie-deralkyl, z. B. 3-(4-Methylpiperazinylsulfonyl)-2-isopropyl-propyl oder 3-(Morpholino-sulfonyl)-2-isopropyl-propyl,
Oxoniederalkyl (worin Oxo nicht an dem Kohlenstoffatom, welches mit dem den Rest R₇ bindenden Stickstoffatom verbunden ist, vorliegt), wie 3-Oxo-n-butyl oder 3-Oxo-n-pentyl,
Cyanoniederalkyl, wie Cyanomethyl, 2-Cyanoethyl, 2- oder 3-Cyano-n-propyl oder 2-, 3- oder 4-Cyano-n-butyl,
Hydroxy-carboxy-niederalkyl, wie 2-Hydroxy-2-carboxy-ethyl oder 2-Hydroxy-3-carboxypropyl,
α-Naphthoxy-carboxy-niederalkyl, wie 2-α-Naphthoxy-4-carboxy-n-butyl,
Hydroxy-niederalkoxycarbonyl-niederalkyl, z.B. 2-Hydroxy-2-niederalkoxycarbonyl-ethyl oder -propyl oder Hydroxy-ethoxy- oder Hydroxy-methoxy-carbonyl-niederalkyl, wie 2-Hydroxy-2-ethoxy- oder -methoxycarbonylethyl oder 2-Hydroxy-3-ethoxy- oder -methoxycarbonyl-propyl,
α-Naphthoxy-niederalkoxycarbonylniederalkyl, z.B. α-Naphthoxy-niederalkoxy-carbonyl-2-ethyl, -2-propyl oder -2-butyryl oder α-Naphthoxyethoxycarbonyl-niederalkyl, wie α-Naphthoxy-ethoxycarbonyl-2-ethyl, 2-α-Naphthoxy-3-ethoxycarbonyl-propyl oder 2-α-Naphthoxy-4-tert-butoxycarbonylbutyl,
Niederalkylcarbonyl-halogen-niederalkyl, wie 3-Ethoxycarbonyl-2-difluormethyl,
α-Naphthoxy-benzyloxycarbonyl-niederalkyl, wie 2-α-Naphthoxy-3-benzyloxy-carbonyl-propyl,
verestertes Hydroxy-niederalkoxycarbonyl-niederalkyl, worin die Hydroxygruppe durch Niederalkanoyl, z. B. Acetyl, Propionyl oder Pivaloyl, Cycloalkylniederalkanoyl, worin Cycloalkyl 3 - 7 Kohlenstoffatome hat und Niederalkanoyl wie zuletzt definiert ist, z. B. Cyclohexylcarbonyl oder 2-Cyclohexyl- oder 2-Cyclopentylacetyl, Bicycloalkylniederal-kanoyl, worin Bicylcoalkyl z. B. 5 - 10, insbesondere 6 - 9, Kohlenstoffatome enthält, wie in Bicyclohexyl-, -heptyl-, -octyl-, -nonyl- oder -decyl-acetyl oder -3-propionyl, z. B. Bicyclo[3.1.0]hex-1-, -2-oder -3-yl-, Bicyclo[4.1.0]hept-1- oder -7-yl-, Bicyclo[2.2.1]-hept-2-yl-, z.B. endo- oder exo-Norbornyl-, Bicyclo[3.2.1]oct-2-yl-, Bicyclo[3.3.0]-oct-3-yl- oder Bicyclo[3.3.1]non-9-yl-, ferner α- oder β-Decahydronaphthyl-acetyl oder -3-propionyl, Tricycloalkylniederalkanoyl, worin Tricycloalkyl z.B. 8 - 10 Kohlenstoffatome enthält, z.B. in Tricyclo[5.2.1.0^{2,6}]dec-8-yl- oder Adamantyl-, wie 1-Adamantyl-acetyl, Arylniederalkanoyl, worin Aryl 6 - 14 Kohlenstoffatome hat, z. B. Phenyl, Indenyl, Indanyl, Naphthyl, Anthryl, Phenanthryl, Acenaphthyl oder Fluorenyl, und unsubstituiert oder durch Niederalkyl, Hydroxy, Niederalkoxy, Carbamoylniederalkoxy, N-Niederalkyl-carbamoylniederalkoxy oder N,N-Diniederalkylcarbamoylniederalkoxy, Amino, Mono- oder Diniederalkylamino, Halogen, wie Fluor, Chlor oder Brom, Carboxy, Niederalk-oxycarbonyl, Phenyl-, Naphthyl- oder Fluorenylniederalkoxycarbonyl, Niederalkanoyl, Niederalkylsulfonyl, z. B. Methylsulfonyl, Phosphono, Hydroxy-niederalkoxy-phosphoryl Diniederalkoxyphosphoryl, Carbamoyl, Sulfamoyl, Nitro und/oder Cyano ein-oder bis zu dreifach substituiert sein kann, Niederalkoxycarbonyl, z. B. tert-Butoxycarbonyl, 2-Halogenniederalkoxycarbonyl, wie oben definiert, oder Phenyl- oder Fluorenyl-nie-deralkoxycarbonyl, z. B. Benzyloxycarbonyl oder 9-Fluorenylmethoxycarbonyl, verestert ist, wie 2-Acetoxy-2-methoxycarbonyl-ethyl, 2-Benzoyloxy-, 2-(1- oder 2-Naphthoyl-oxy)-, 2-(Phenyl-2-acetyloxy)-, 2-(1- oder 2-Napthyl-2-acetyloxy)-, 2-(4-Methylphe-nyl-2-acetyloxy)-, 2-(4-Methoxyphenyl-2-acetyloxy)- oder 2-(2-(o,o-Dichloro-phenyl)-2-acetyloxy)-2-methoxycarbonyl-ethyl oder -3-propyl,
Dihydroxy-carboxy-niederalkyl, wie 2,3-Dihydroxy-3-carboxy-propyl,
Dihydroxy-niederalkoxycarbonyl-niederalkyl, wie 2,3-Dihydroxy-3-ethoxy- oder -methoxycarbonyl-propyl,
durch Niederalkanoyl, wie Acetyl, Propionyl oder Butyryl, Niederalkoxycarbonyl, z. B. tert-Butoxycarbonyl, Phenyl- oder Fluorenyl-niederalkoxycarbonyl, wie Benzyloxycar-bonyl oder 9-Fluorenylmethoxycarbonyl, Niederalkylsulfonyl oder Toluolsulfonyl verestertes Dihydroxy-niederalkoxycarbonyl-niederalkyl, z.B. Diniederalkanoyloxy-nie-deralkoxy-propyl, wie 2,3-Diacetoxy-3-methoxycarbonyl-propyl,
α-Naphthoxy-diniederalkylamino-niederalkyl, wie 2-α-Naphthoxy-5-dimethylamino-pentyl,
α-Naphthoxy-carbamoyl-niederalkyl, wie 2-α-Naphthoxy-4-carbamoyl-butyl,
α-Naphthoxy-oxo-niederalkyl (wobei Oxo nicht an dem Kohlenstoffatom, welches mit dem den Rest R₇ tragenden Stickstoffatom verbunden ist, vorliegt), wie 2-α-Naphthoxy-4-oxo-pentyl, oder
α-Naphthoxy-cyanoniederalkyl, wie 2-α-Naphthoxy-cyano-ethyl oder 2-α-Naphthoxy-4-cyanobutyl.

Bevorzugt ist unsubstituiertes oder durch Hydroxy, Niederalkoxy, Amino, Mono-oder Diniederalkylamino, Fluor, Chlor oder Cyano ein- oder mehrfach substituiertes Niederalkyl, ganz besonders bevorzugt ist Niederalkyl, wie Methyl, Ethyl, Propyl, Isopropyl, n-Butyl, Isobutyl oder tert-Butyl.

Cycloalkyl R₃, R₄ oder R₇ ist vorzugsweise wie unter Cycloalkylniederalkanoyl R₁, R₂, R₈ oder R₉ definiert, z. B. Cyclopropyl, Cyclobutyl, Cyclopentyl, Cyclohexyl oder Cyclo-heptyl, wobei Cycloalkyl unsubstituiert oder durch Niederalkyl, wie Isopropyl, Halogen-niederalkyl, wie Trifluormethyl, Hydroxy, Niederalkoxy, Carbamoylniederalkoxy, N-Niederalkylcarbamoylniederalkoxy oder N,N-Diniederalkylcarbamoylniederalkoxy, Amino, Mono- oder Diniederalkylamino, Halogen, wie Fluor, Chlor oder Brom, Carboxy, Nieder-alkoxycarbonyl, Phenyl-, Naphthyl- oder Fluorenylniederalkoxycarbonyl, Niederalkanoyl, Niederalkylsulfonyl, z. B. Methylsulfonyl, Phosphono, Hydroxy-niederalkoxy-phosphoryl oder Diniederalkoxyphosphoryl, Carbamoyl, Mono- oder Diniederalkylaminocarbamoyl, Sulfamoyl, Mono- oder Diniederalkylsulfamoyl, Nitro und/oder Cyano ein- oder bis zu dreifach substituiert ist, wie Cyclobutyl, Cyclopentyl, Cyclohexyl oder Cycloheptyl, besonders bevorzugt Cyclohexyl.

Aryl R₃, R₄ oder R₇ ist vorzugsweise einer der unter Arylniederalkanoyl R₁, R₂, R₈ oder R₉ genannten unsubstituierten oder substituierten Aryl-Reste und ist insbesondere Phenyl, Naphthyl oder Fluorenyl, das unsubstituiert oder durch Niederalkyl, wie Isopropyl, Halo-genniederalkyl, wie Trifluormethyl, Hydroxy, Niederalkoxy, Carbamoylniederalkoxy, N-Niederalkylcarbamoylniederalkoxy oder N,N-Diniederalkylcarbamoylniederalkoxy, Ami-no, Mono- oder Diniederalkylamino, Halogen, wie Fluor, Chlor oder Brom, Carboxy, Nie-deralkoxycarbonyl, Phenyl-, Naphthyl- oder Fluorenylniederalkoxycarbonyl, Niederalka-noyl, Niederalkylsulfonyl, z. B. Methylsulfonyl, Phosphono, Hydroxy-niederalkoxy-phos-phoryl oder Diniederalkoxyphosphoryl, Carbamoyl, Mono-oder Diniederalkylaminocar-bamoyl, Sulfamoyl, Mono- oder Diniederalkylsulfamoyl, Nitro und/oder Cyano ein- oder bis zu dreifach substituiert ist, wie Phenyl, 4-Metoxyphenyl, 4-Fluorphenyl, 4-Nitrophenyl, 4-Cyanophenyl oder 1- oder 2-Naphthyl.

Heterocyclyl R₃, R₄ oder R₇ ist vorzugsweise definiert wie unter Heterocyclyl R₁, R₂, R₈ oder R₉, das unsubstituiert oder wie dort substituiert ist, und ist insbesondere ausgewählt aus 2- oder 3-Pyrrolyl, 4- oder 5-Methylpyrrolyl oder 4- oder 5-Phenylpyrrolyl, 2-Thienyl, 2-Furyl, 2-, 3- oder 4-Pyridyl, 2-, 3- oder 5-Indolyl, 1-Methyl-, 2-Methyl-, 5Methoxy-, 5-Benzyloxy-, 5-Chlor- oder 4,5-Dimethylindol-2-yl, 1-Benzylindol-2- oder - 3-yl, 4,5,6,7-Tetrahydroindol-2-yl, Cyclohepta[b]pyrrol-5-yl, 2-, 3- oder 4-Chinolyl oder 4-Hydroxychinol-2-yl, 1-, 3- oder 4-Isochinolyl, 1-Oxo- 1,2-dihydroisochinol-3-yl, 2-Chinox-alinyl, 3,1-Benzfuran-2-yl, Benz[e]indol-2-yl, β-Carbolin-3-yl, 3-Chromanyl, 3-Thiochro-manyl, 3-Pyrrolidinyl, 3- oder 4-Hydroxypyrrolidin-2-yl, wie 5-Oxopyrrolidin-2-yl, 2-, 3- oder 4-Piperidinyl, 2- oder 3-Morpholinyl, 2- oder 3-Thiomorpholinyl, S,S-Dioxothiomorpholin-2- oder -3-yl, 2- oder 3-Indolinyl, 1,2,3,4-Tetrahydrochinol-2-, -3- oder - 4-yl, 1,2,3,4-Tetrahydroisochinol-1-, -2- oder -3-yl, oder 1-Oxo-1,2,3,4-tetrahydroisochinol-3-yl.

Unsubstituiertes oder substituiertes Alkenyl R₃, R₄ oder R₇ ist vorzugsweise wie unter Alkenyl R₁, R₂, R₈ oder R₉ definiert, insbesondere unsubstituiertes oder wie dort substi-tuiertes Niederalkenyl, z.B.
Niederalkenyl, wie Vinyl, Allyl oder 2- oder 3-Butenyl,
Cycloalkylniederalkenyl, worin Cycloalkyl wie bei Cycloalkylniederalkanoyl R₁, R₂, R₈ oder R₉ definiert ist und unsubstituiert oder durch Niederalkyl, wie Isopropyl, Halogennie-deralkyl, wie Trifluormethyl, Hydroxy, Niederalkoxy, Carbamoylniederalkoxy, N-Nieder-alkylcarbamoylniederalkoxy oder N,N-Diniederalkylcarbamoylniederalkoxy, Amino, Mono- oder Diniederalkylamino, Halogen, wie Fluor, Chlor oder Brom, Carboxy, Nieder-alkoxycarbonyl, Phenyl-, Naphthyl- oder Fluorenylniederalkoxycarbonyl, Niederalkanoyl, Niederalkylsulfonyl, z. B. Methylsulfonyl, Phosphono, Hydroxy-niederalkoxy-phosphoryl oder Diniederalkoxyphosphoryl, Carbamoyl, Mono- oder Diniederalkylaminocarbamoyl, Sulfamoyl, Mono- oder Diniederalkylsulfamoyl, Nitro und/oder Cyano ein- oder bis zu dreifach substituiert ist und insbesondere am endständigen Kohlenstoffatom von Niederal-kenyl gebunden ist, wie Cyclobutyl-, Cyclopentyl-, Cyclohexyl- oder Cycloheptyl-methyl-2-vinyl, -2- oder -3-allyl oder -2-, -3- oder -4-but-2-enyl,
Arylniederalkenyl mit einem unter Aryl R₃ oder R₄ definierten unsubstituierten oder substituierten Arylrest, der vorzugsweise endständig an Niederalkenyl gebunden ist, und einem unter Niederalkenyl R₁, R₂, R₈ oder R₉ definierten Niederalkenylrest, wie Styryl, 3-Phenylallyl (Cinnamyl), 2-(α-Naphthyl)-vinyl oder 2-(β-Naphthyl)-vinyl, oder
unsubstituiertes oder substituiertes Heterocyclylniederalkenyl, welches beispielsweise einen unter Alkenyl R₁, R₂, R₈ oder R₉ genannten unsubstituierten oder substituierten Niederalkenylrest enthält, z.B. Vinyl, Allyl oder 2- oder 3-Butenyl, der vorzugsweise am endständigen Kohlenstoffatom von einem unter Heterocylclyl R₁, R₂, R₈ oder R₉ genannten unsubstituierten oder substituierten Heterocyclylrest, substituiert ist, z. B. als Pyrimidin-1-yl-, Piperidino-, Pyrazin-1-yl-, Piperazin-1-yl-, Pyridazin-1-yl-, Morpholino-, Thiomorpholino- oder S,S-Dioxothiomorpholino-niederalkenyl, wie in 2-Morpholino-vinyl, 3-Morpholino-al1yl oder 4-Morpholino-2- oder -3-butenyl, unsubstituiertes oder durch Niederalkyl oder Phenyl substituiertes Pyrrolylniederalkenyl, wie 2- oder 3-Pyrrolylvinyl oder -allyl, 4- oder 5-Methylpyrrolylvinyl oder -allyl oder 4- oder 5-Phenylpyrrolylvinyl oder -allyl, Thienylniederalkenyl, wie 2-Thienylvinyl oder -allyl, Fwylniederalkenyl, wie 2-Furylvinyl oder -allyl, Pyridylniederalkenyl, wie 2-, 3- oder 4-Pyridylvinyl oder -allyl, unsubstituiertes oder durch Niederalkyl, z. B. Methyl, Phenylniederalkyl, z. B. Benzyl, Niederalkoxy, z. B. Methoxy, Phenylniederalkoxy, z. B. Benzyloxy, oder Halogen, z. B. Chlor, substituiertes Indolylniederalkenyl, wie 2-, 3- oder 5-1ndolylvinyl oder -allyl, 1-Methyl-, 2-Methyl-, 5-Methoxy-, 5-Benzyloxy-, 5-Chlor- oder 4,5-Dimethylindol-2-yl-, 1-Benzylindol-2-yl- oder -3-ylvinyl oder -allyl, 4,5,6,7-Tetrahydroindol-2-ylmethyl, -ethyl oder -n-propyl, Cyclohepta[b]pyrrol-5-ylvinyl oder -allyl, unsubstituiertes oder durch Hydroxy substituiertes Chinolylniederalkenyl, z. B. 2-, 3- oder 4-Chinolyl- oder 4-Hydroxychinol-2-ylvinyl oder -allyl, unsubstituiertes oder durch Hydroxy substituiertes Iso-chinolylniederalkenyl, wie 1-, 3- oder 4-Isochinolyl- oder 1-Oxo- 1,2-dihydroisochi-nol-3-ylvinyl oder -allyl, 2-Chinoxanylvinyl oder -allyl, 3, 1-Benzfuran-2-ylvinyl oder -allyl, Benz[e]indol2-ylvinyl oder -allyl, β-Carbolin-3-ylvinyl oder -allyl, 3-Chromanyl-vinyl oder -allyl, 3-Thiochromanylvinyl oder -allyl, 3-Pyrrolidinylvinyl oder -allyl, Hydroxypyrrolidinylniederalkenyl, wie 3- oder 4-Hydroxypyrrolidin-2-ylvinyl oder -allyl, Oxopyrrolidinylniederalkenyl, wie 5-Oxopyrrolidin-2-ylvinyl oder -allyl, Piperidinylnie-deralkenyl, wie 2-, 3- oder 4-Piperidinylvinyl oder -allyl, Morpholinylniederalkenyl, wie 2- oder 3-Morpholinylvinyl oder -allyl, Thiomorpholinylniederalkenyl, wie 2- oder 3-Thiomorpholinylvinyl oder -allyl, S,S-Dioxothiomorpholinylniederalkenyl, wie S,S-Di-oxothiomorpholin-2- oder -3-ylvinyl oder -allyl, Indolinylniederalkenyl, wie 2- oder 3-Indolinylvinyl oder -allyl, 1,2,3,4-Tetrahydrochinolylniederalkenyl, wie 1,2,3,4-Tetrahydro-chinol-2-, -3- oder -4-ylvinyl oder -allyl, 1,2,3,4-Tetrahydroisochinolylniederalkenyl, wie 1,2,3,4-Tetrahydroisochinol-1-, -2- oder -3-ylvinyl oder -allyl, oder 1-Oxo-1,2,3,4-tetra-hydroisochinol-3-ylvinyl oder -allyl.

Durch R₃ und R₄ gemeinsam gebildetes unsubstituiertes oder substituiertes Alkylen ent-hält in erster Linie einen Alkylerest mit bis zu 20 Kohlenstoffatomen, wobei die genannten Reste auch eine oder mehr Doppelbindungen enthalten können, vorzugsweise Nieder-alkylen, z. B. Ethylen, Trimethylen, Tetramethylen, Pentamethylen, Hexamethylen oder Heptamethylen, der unsubstituiert ist oder substituiert ist, insbesondere mit unsubstituiertem oder substituiertem Aryl, wie unter Arylniederalkanoyl R₁, R₂, R₈ oder R₉ definiert, vorzugsweise mit Phenyl, Indenyl, Indanyl, Naphthyl, Anthryl, Phenanthryl, Acenaphthyl oder Fluorenyl, welche unsubstituiert oder durch Reste wie Niederalkyl, z. B. Methyl, Ethyl oder Propyl, Halogenniederalkyl, z. B. Trifluormethyl, Phenyl, 1-oder 2-Naphthyl, Hydroxy, Niederalkoxy, z. B. Methoxy, Carbamoylniederalkoxy, N-Niederalkylcarbamoylniederalkoxy oder N,N-Diniederalkylcarbamoylniederalkoxy, Amino, Mono- oder Diniederalkylamino, Niederalkanoylamino, z. B. Pivaloylamino, Halogen, z. B. Fluor, Chlor oder Brom, Carboxy, Niederalkoxycarbonyl, Phenyl-, Naphthyl-oder Fluorenylniederalkoxycarbonyl, Niederalkanoyl, Sulfo, Niederalkylsulfonyl, z. B. Methylsulfonyl, Phosphono, Hydroxy-niederalkoxy-phosphoryl oder Diniederalkoxy-phosphoryl, Carbamoyl, Mono- oder Diniederalkylcarbamoyl, Sulfamoyl, Mono- oder Diniederalkylaminosulfonyl, Nitro und/oder Cyano ein- oder bis zu dreifach substituiert sind, und/oder wie Niederalkanoyl in Arylniederalkanoyl R₁, R₂, R₈ oder R₉ substituiert ist, insbesondere durch Niederalkyl, z. B. Methyl, Hydroxy, Niederalkoxy, z. B. Methoxy oder Ethoxy, Niederalkanoyloxy, z. B. Acetoxy oder Propionyloxy, Benzoyloxy, Phenyl-acetyloxy oder 1- oder 2-Naphthoyloxy, Niederalkoxycarbonyloxy, z. B. Isopropoxy-carbonyloxy oder tert-Butoxycarbonyloxy, Phenylniederalkoxycarbonyloxy, z. B. Benzyl-oxycarbonyloxy, 9-Fluorenylmethoxycarbonyloxy, Sulfonyloxy, Niederalkylsulfonyloxy, z. B. Methyl- oder Ethylsulfonyloxy, Phenylsulfonyloxy, 2- oder 4-Toluolsulfonyloxy, Amino, Mono- oder Diniederalkylamino, z. B. Mono- oder Di-methyl-amino oder - ethylamino, Niederalkanoylamino, z. B. Acetylamino oder Pivaloylamino, Carboxy, Nieder-alkoxycarbonyl, z. B. Isopropoxy- oder tert-Butoxycarbonyl, Benzyloxycarbonyl, 9-Fluorenylmethoxycarbonyl, Niederalkanoyl, z. B. Acetyl oder Propionyl, Niederalkylsul-fonyl, wie Methylsulfonyl oder tert-Butylsulfonyl, Phosphono, Carbamoyl, Mono- oder Diniederalkylcarbamoyl, z. B. N-Methylcarbamoyl oder N,N-Dimethylcarbamoyl, Piperidino, Pyrazin-1-yl, Pyrimidin-1-yl, Pyridazin-1-yl, Morpholino, Thiomorpholino oder S,S-Dioxothiomorpholino, Sulfamoyl, Oxo und/oder Cyano, wie Ethylen, Ethylethylen, Trimethylen, Propylen oder Tetramethylen.

Durch R₃ und R₄ gemeinsam gebildetes unsubstituiertes oder substituiertes Alkyliden enthält bis zu 20 Kohlenstoffatome und keine, eine oder mehrere Doppelbindungen zu-sätzlich zur bindenden Doppelbindung, und ist vorzugsweise Niederalkyliden, z. B. Methylen, Ethyliden, Propyliden, Butyliden oder Pentyliden, unsubstituiert oder substituiert insbesondere mit Cycloalkyl, wie unter Cycloalkylniederalkanoyl R₁, R₂, R₈ oder R₉ genannt, z. B. Cyclopropyl, Cyclobutyl, Cyclopentyl oder Cyclohexyl, Cycloalkenyl, wie unter Cycloalkenylniederalkanoyl R₁, R₂, R₈ oder R₉ genannt, z. B. Cyclohexen-1-yl oder 1,4-Cyclohexadienyl, unsubstituiertem oder substituiertem Aryl, wie unter Arylniederalkanoyl R₁, R₂, R₈ oder R₉, definiert, vorzugsweise mit Phenyl, Indenyl, Indanyl, Naphthyl, Anthryl, Phenanthryl, Acenaphthyl oder Fluorenyl, welche unsubstituiert oder durch Reste wie Niederalkyl, z. B. Methyl, Ethyl oder Propyl, Halogenniederalkyl, z. B. Tri-fluormethyl, Phenyl, 1- oder 2-Naphthyl, Hydroxy, Niederalkoxy, z. B. Methoxy, Carba-moylniederalkoxy, N-Niederalkylcarbamoylniederalkoxy oder N,N-Diniederalkyl-carbamoylniederalkoxy, Amino, Mono- oder Diniederalkylamino, Niederalkanoylamino, z. B. Pivaloylamino, Halogen, z. B. Fluor, Chlor oder Brom, Carboxy, Niederalkoxy-carbonyl, Phenyl-, Naphthyl- oder Fluorenylniederalkoxycarbonyl, Niederalkanoyl, Sulfo, Niederalkylsulfonyl, z. B. Methylsulfonyl, Phosphono, Hydroxy-niederalkoxy-phosphoryl oder Diniederalkoxyphosphoryl, Carbamoyl, Mono- oder Diniederalkylcarbamoyl, Sul-famoyl, Mono- oder Diniederalkylaminosulfonyl, Nitro und/oder Cyano ein- oder bis zu dreifach substituiert sind, und/oder wie Niederalkanoyl in Arylniederalkanoyl R₁, R₂, R₈ oder R₉ substituiert, insbesondere durch Niederalkyl, z. B. Methyl, Hydroxy, Nieder-alkoxy, z. B. Methoxy oder Ethoxy, Niederalkanoyloxy, z. B. Acetoxy oder Propionyloxy, Benzoyloxy, Phenylacetyloxy oder 1- oder 2-Naphthoyloxy, Niederalkoxycarbonyloxy, z. B. Isopropoxycarbonyloxy oder tert-Butoxycarbonyloxy, Phenylniederalkoxycarbonyloxy, z. B. Benzyloxycarbonyloxy, 9-Fluorenylmethoxycarbonyloxy, Sulfonyloxy, Niederalkyl-sulfonyloxy, z. B. Methyl- oder Ethylsulfonyloxy, Phenylsulfonyloxy, 2- oder 4-Toluol-sulfonyloxy, Amino, Mono- oder Diniederalkylamino, z. B. Mono- oder Di-methyl-amino oder -ethyl-amino, Niederalkanoylamino, z. B. Acetylamino oder Pivaloylamino, Carboxy, Niederalkoxycarbonyl, z. B. Isopropoxy- oder tert-Butoxycarbonyl, Benzyloxy-carbonyl, 9-Fluorenylmethoxycarbonyl, Niederalkanoyl, z. B. Acetyl oder Propionyl, Nie-deralkylsulfonyl, wie Methylsulfonyl oder tert-Butylsulfonyl, Phosphono, Carbamoyl, Mono- oder Diniederälkylcarbamoyl, z. B. N-Methylcarbamoyl oder N,N-Dimethylcarba-moyl, Piperidino, Pyrazin-1-yl, Pyrimidin-1-yl, Pyridazin-1-yl, Morpholino, Thiomorpholino oder S,S-Dioxothiomorpholino, Sulfamoyl, Oxo und/oder Cyano, wie Ethy-liden, Propyliden, Butyliden, Benzyliden oder Cinnamyliden.

Durch R₃ und R₄ gemeinsam gebildetes unsubstituiertes oder substituiertes benzkonden-siertes Alkylen enthält bis zu 20 Kohlenstoffatome und ist vorzugsweise Niederalkylen, z B. Ethylen, Trimethylen, Tetramethylen, Pentamethylen, Hexamethylen oder Hepta-methylen, an welches ein Benzolring ankondensiert ist, und ist unsubstituiert oder substituiert insbesondere mit unsubstituiertem oder substituiertem Aryl, wie unter Arylniederalkanoyl R₁, R₂, R₈ oder R₉, definiert, vorzugsweise mit Phenyl, Indenyl, Indanyl, Naphthyl, Anthryl, Phenanthryl, Acenaphthyl oder Fluorenyl, welche unsubstituiert oder durch Reste wie Niederalkyl, z. B. Methyl, Ethyl oder Propyl, Halogenniederalkyl, z. B. Trifluormethyl, Phenyl, 1- oder 2-Naphthyl, Hydroxy, Niederalkoxy, z. B. Methoxy, Carbamoylniederalkoxy, N-Niederalkylcarbamoylniederalkoxy oder N,N-Dinie-deralkylcarbamoylniederalkoxy, Amino, Mono- oder Diniederalkylamino, Niederalka-noylamino, z. B. Pivaloylamino, Halogen, z. B. Fluor, Chlor oder Brom, Carboxy, Niederalkoxycarbonyl, Phenyl-, Naphthyl- oder Fluorenylniederalkoxycarbonyl, Nie-deralkanoyl, Sulfo, Niederalkylsulfonyl, z. B. Methylsulfonyl, Phosphono, Hydroxy-nie-deralkoxy-phosphoryl oder Diniederalkoxyphosphoryl, Carbamoyl, Mono-oder Diniederalkylcarbamoyl, Sulfamoyl, Mono- oder Diniederalkylaminosulfonyl, Nitro und/oder Cyano ein- oder bis zu dreifach substituiert sind, ferner mit Niedeialkyl, z. B. Methyl, Hydroxy, Niederalkoxy, z. B. Methoxy oder Ethoxy, Niederalkanoyloxy, z. B. Acetoxy oder Propionyloxy, Benzoyloxy, Phenylacetyloxy oder 1- oder 2-Naphthoyloxy, Niederalkoxycarbonyloxy, z. B. Isopropoxycarbonyloxy oder tert-Butoxycarbonyloxy, Phenylniederalkoxycarbonyloxy, z. B. Benzyloxycarbonyloxy, 9-Fluorenylmethoxycarbonyloxy, Sulfonyloxy, Niederalkylsulfonyloxy, z. B. Methyl- oder Ethylsulfonyloxy, Phenylsul-fonyloxy, 2- oder 4-Toluolsulfonyloxy, Amino, Mono- oder Diniederalkylamino, z. B. Mono- oder Di-methyl-amino oder -ethyl-amino, Niederalkanoylamino, z. B. Acetylamino oder Pivaloylamino, Carboxy, Niederalkoxycarbonyl, z. B. Isopropoxy- oder tert-Butoxycarbonyl, Benzyloxycarbonyl, 9-Fluorenylmethoxycarbonyl, Niederalkanoyl, z. B. Acetyl oder Propionyl, Niederalkylsulfonyl, wie Methylsulfonyl oder tert-Butylsulfonyl, Phosphono, Carbamoyl, Mono- oder Diniederalkylcarbamoyl, z. B. N-Methylcarbamoyl oder N,N-Dimethylcarbamoyl, Piperidino, Pyrazin-1-yl, Pyrimidin-1-yl, Pyridazin-1-yl, Morpholino, Thiomorpholino oder S,S-Dioxothiomorpholino, Sulfamoyl, Oxo und/oder Cyano, wie ortho-Phenylen.

Wenn bei den mit den genannten Resten substituierten Verbindungen der Formel I Stickstoffatome mit freiem Wasserstoff und/oder Hydroxygruppen vicinal zu Doppel-oder Dreifachbindungen stehen (wie bei unsubstituiertem oder substituiertem Alkenyl oder Alkyl R₁, R₂, R₈ oder R₉), sind stets auch die entsprechenden tautomeren Imino-beziehungsweise Oxo-Verbindungen mit umfasst.

Salze von Verbindungen der Formel I sind insbesondere Säureadditionssalze, Salze mit Basen oder bei Vorliegen mehrerer salzbildender Gruppen gegebenenfalls auch Mischsalze oder innere Salze.

Salze sind in erster Linie die pharmazeutisch verwendbaren, nichttoxischen Salze von Verbindungen der Formel I.

Solche Salze werden beispielsweise von Verbindungen der Formel I mit einer sauren Gruppe, z. B. einer Carboxy-, Sulfo- oder durch eine oder zwei Hydroxygruppen substi-tuierten Phosphorylgruppe, gebildet und sind beispielsweise deren Salze mit geeigneten Basen, wie nicht-toxische, von Metallen der Gruppe Ia, Ib, IIa und IIb des Periodensystems der Elemente abgeleitete Metallsalze, in erster Linie geeignete Alkalimetall-, z. B. Lithium-, Natrium- oder Kalium-, oder Erdalkalimetallsalze, z. B. Magnesium-oder Calciumsalze, ferner Zinksalze oder Ammoniumsalze, auch solche Salze, welche mit or-ganischen Aminen, wie gegebenenfalls dwch Hydroxy substituierten Mono-, Di- oder Trialkylaminen, insbesondere Mono-, Di- oder Triniederalkylaminen, oder mit quater-nären Ammoniumverbindungen gebildet werden, z. B. mit N-Methyl-N-ethylamin, Di-ethylamin, Triethylamin, Mono-, Bis- oder Tris-(2-hydroxyniederalkyl)-aminen, wie Mono-, Bis- oder Tris-(2-hydroxyethyl)-amin, 2-Hydroxy-tert-butylamin oder Tris-(hydroxymethyl)-methylamin, N,N-Diniederalkyl-N-(hydroxyniederalkyl)-aminen, wie N,N-Dimethyl-N-(2-hydroxyethyl)-amin oder Tri-(2-hydroxyethyl)-amin, N-Methyl-D-glucamin oder quaternären Ammoniumsalzen, wie Tetrabutylammoniumsalzen. Die Ver-bindungen der Formel I mit einer basischen Gruppe, z. B. einer Aminogruppe, können Säureadditionssalze bilden, beispielsweise mit anorganischen Säuren, z. B. Halogen-wasserstoffsäure, wie Salzsäure, Schwefelsäure oder Phosphorsäure, oder mit organischen Carbon-, Sulfon-, Sulfo- oder Phosphosäuren oder N-substituierter Sulfaminsäuren, wie, z. B. Essigsäure, Propionsäure, Glykolsäure, Bernsteinsäure, Maleinsäure, Hydroxymaleinsäure, Methylmaleinsäure, Fumarsäure, Aepfelsäure, Weinsäure, Gluconsäure, Glucarsäure, Glucuronsäure, Zitronensäure, Benzoesäure, Zimtsäure, Mandelsäure, Salicylsäure, 4-Aminosalicylsäure, 2-Phenoxybenzoesäure, 2-Acetoxybenzoesäure, Embonsäure, Nico-tinsäure oder Isonicotinsäure, ferner mit Aminosäuren, wie z. B. den weiter vorn genannten α-Aminosäuren, insbesondere Glutaminsäure und Asparaginsäure, sowie mit Methansulfonsäure, Ethansulfonsäure, 2-Hydroxyethansulfonsäure, Ethan-1,2-disulfonsäure, Benzolsulfonsäure, 4-Methylbenzolsulfonsäure, Naphthalin-2-sulfonsäure, 2- oder 3-Phosphoglycerat, Glucose-6-phosphat, N-Cyclohexylsulfaminsäure (unter Bildung der Cyclamate) oder mit anderen sauren organischen Verbindungen, wie Ascorbinsäure. Verbindungen der Formel I mit sauren und basischen Gruppen können auch innere Salze bilden.

Zur Isolierung oder Reinigung können auch pharmazeutisch ungeeignete Salze Verwendung finden.

Die Verbindungen der vorliegenden Erfindung weisen Hemmwirkung auf virale Aspartat-proteasen, insbesondere gag-Protease-hemmende Wirkungen auf. In erster Linie hemmen sie in den nachfolgend beschriebenen Tests in Konzentrationen von 10⁻⁶ bis 10⁻⁹ mol/l die Wirkung der gag-Protease von HIV-1 und HIV-2 und sind daher geeignet als Mittel gegen durch diese oder verwandte Retroviren verursachte Krankheiten, wie z. B. gegen AIDS.

Die Fähigkeit der Verbindungen der Formel I, die proteolytische Aktivität von z. B. HIV-1 Protease zu inhibieren, lässt sich beispielsweise gemäss dem von J. Hansen et al., The EMBO Journal 7, 1785-1791 (1988), beschriebenen Verfahren demonstrieren. Dabei wird die Hemmung der Wirkung der gag-Protease auf ein Substrat gemessen, das ein in E. coli exprimiertes Fusionsprotein aus dem gag-Vorläuferprotein und MS-2 ist. Das Substrat und seine Spaltprodukte werden durch Polyacrylamid-Gelelektrophorese getrennt und durch Immunoblotting mit monoklonalen Antikörpern gegen MS-2 sichtbar gemacht.

In einem noch einfacher zu handhabenden Test, der genaue quantitative Aussagen ermög-licht, wird als Substrat für die gag-Protease ein synthetisches Peptid eingesetzt, das der Spaltstelle des gag-Vorläuferproteins entspricht. Dieses Substrat und seine Spaltprodukte können durch Hochdruckflüssig-Chromatographie (HPLC) gemessen werden.

Beispielsweise wird als Substrat für eine rekombinante HIV-1-Protease (Herstellung gemäss Billich, S., et al., J. Biol. Chem. 263(34), 17905 - 17908 (1990)) ein synthetisches chromophores Peptid (z. B. HKARVL[NO₂]FEANleS (Bachem, Schweiz) oder ein Icosapeptid wie RRSNQVSQNYPIVQNIQGRR (hergestellt durch Peptidsynthese nach bekannten Verfahren) eingesetzt, das einer der Spaltstellen des gag-Vorläuferproteins entspricht. Dieses Substrat und seine Spaltprodrrkte können durch Hochdruckflüssig-Chromatographie (HPLC) gemessen werden.

Hierzu wird ein zu testender Hemmstoff der Formel I in Dimethylsulfoxid gelöst; der Enzymtest wird düchgeführt, indem geeignete Verdünnungen des Hemmstoffes in 20 mM β-Morpholinoethansulfonsäure (MFS)-Puffer pH 6,0 zum Assay-Mix aus 67,2 µM des oben genannten chromophoren Peptides in 0,3 M Natriumacetat, 0,1 M NaCl pH 7,4 oder 122 µM des oben genannten Icosapeptids in 20 mM MFS-Puffer pH 6,0 zugegeben werden. Die Grösse der Ansätze beträgt 100 µl. Die Reaktion wird gestartet durch Zugabe von im ersten Fall 2 µl, im zweiten Fall 10 µl HIV-I-Protease und im ersten Fall nach 15 min durch Zugabe von 100 µl 0,3 M HClO₄, im zweiten Fall nach einer Stunde Inkubation bei 37 °C durch Zugabe von 10 µl 0,3 M HClO₄ gestoppt. Die Reaktionsprodukte werden nach Abzentrifugieren der Probe für 5 min bei 10000 x g in 100 µl (Ansatz mit chromophorem Peptid) bzw. 20 µl (Icosapeptid-Ansatz) des erhaltenen Überstandes und nach Auftragen auf eine 125 x 4,6 mm Nucleosil® C18-5µ-HPLC-Säule (Macherey & Nagel, Düren) und Elution quantifiziert anhand der Peak-Höhe des Spaltproduktes bei 280 (Ansatz mit chromophorem Peptid) oder bei 215 nm (Ansatz mit Icosapeptid), Gradient: 100 % El.1-> 50 % El.1/50 % El.2 (El.1: 75 % Acetonitril, 90 % H₂O, 0,1 % Trifluoressigsäure (TFA); E1.2: 75 % Acetonitril, 25 % H₂O, 0,08 % TFA) innerhalb von 15 min; Durchflussrate 1 ml/min.

Hierbei werden für Verbindungen der Formel I vorzugsweise IC₅₀-Werte (IC₅₀ = diejenige Konzentration, welche die Aktivität der HIV-1-Protease gegenüber einer Kontrolle ohne Hemmstoff um 50 % senkt) von etwa 10⁻⁶ bis 10⁻⁹ M, insbesondere von etwa 10⁻⁷ bis etwa 10⁻⁸ M, ermittelt.

In einem weiteren Test kann gezeigt werden, dass die Verbindungen der vorliegenden Erfindung Zellen, die normalerweise von HIV infiziert werden, vor einer solchen Infektion schützt oder zumindest eine solche Infektion verlangsamt. Dabei wird die menschliche T-Zell-Leukämie Zellinie MT-2 (Science 229, 563 (1985)), die extrem empfindlich für den zytopathogenen Effekt von HIV ist, mit HIV allein oder mit HIV in Gegenwart der erfindungsgemässen Verbindungen inkubiert und nach einigen Tagen die Lebensfähigkeit der so behandelten Zellen beurteilt.

Hierzu werden die MT-2-Zellen in RPMI 1640-Medium (Gibco, Schweiz; RPMI 1640 enthält ein Aminosäurengemisch ohne L-Gln), das mit lO%o hitzeinaktiviertem fetalem Kälberserum, L-Glutamin, Hepes (2-(4-(2-Hydroxyethyl)- 1-piperazino]-ethansulfonsäure) und Standardantibiotika supplementiert ist, bei 37 °C in befeuchteter Luft mit 5% CO₂ gehalten. 50 µl derjeweiligen Testverbindung in Kulturmedium und 100 µl HIV-1 in Kulturmedium (800 TCID50/ml) (TCID50 = Tissue Culture Infectious Dose 50 = Dosis, die 50% der MT-2-Zellen infiziert) werden zu 4x10³ exponentiell wachsenden MT-2-Zellen in 50 µl Kulturmedium pro Vertiefung auf 96-Loch-Mikrotiterplatten gegeben. Parallele Ansätze auf einer weiteren Mikrotiterplatte mit Zellen und Testverbindung erhalten 100 µl Kulturmedium ohne Virus. Nach 4 Tagen Inkubation wird in 10 µl Zellüberstand die Reverse-Transkriptase (RT)-Aktivität ermittelt. Die RT-Aktivität wird bestimmt in 50 mM Tris (α,α,α-Tris(hydroxymethyl)-methylamin, Ultra pur, Merck, Bundesrepublik Deutschland) pH 7,8; 75 mM KCl, 2 mM Dithiothreitol, 5 mM MgCl₂; 0,05% Nonidet P-40 (Detergens; Sigma, Schweiz); 50 µg/ml Polyadenylic Acid (Pharmacia, Schweden); 1,6 µg/ml dT(12-18) (Sigma, Schweiz). Die Mischung wird durch einen 0,45 µ Acrodisc-Filter (Gellman Science Inc, Ann Arbor) abfiltriert und bei -20 °C aufbewahrt. Zu Aliquoten dieser Lösung werden 0,1 % (v/v) (alpha-³²P]dTTP zum Erzielen einer radioaktiven Endaktivität von 10 µCi/ml zugegeben. 10 µl des Kulturüberstandes werden auf eine neue 96-Loch-Mikrotiterplatte übertragen und hierzu 30 µl des genannten RT-Cocktails gegeben. Nach Mischen wird die Platte für 1,5 bis 3 h bei 37 °C inkubiert. 5 µl dieser Reaktionsmischung werden auf Whatman DE8 1-Papier (Whatman) überführt. Die getrockneten Filter werden 3-mal für 5 min mit 300 mM NaCl/25 mM Tri-Natriumcitrat und 1-mal mit 95% Ethanol gewaschen und erneut luftgetrocknet. Die Auswertung erfolgt in einem Matrix Packard 96well counter (Packard). Die ED90-Werte werden errechnet und als die niedrigste Konzentration derjeweiligen Testverbindung definiert, welche die RT-Aktivität um 90% im Vergleich zu nicht mit der Testsubstanz behandelten Zellansätzen senkt. Die RT-Aktivität ist dabei ein Mass für die HIV-1-Vermehrung.

Die erfindungsgemässen Verbindungen zeigen hierbei eine ED90 von etwa 10⁻⁵ bis 10⁻⁸ M, vorzugsweise von etwa 5 x 10⁻⁷ bis 5 x 10⁻⁸ M.

Bei den im folgenden genannten Gruppen von Verbindungen der Formel I können in sinnvoller Weise, z. B. zur Ersetzung allgemeinerer durch speziellere Definitionen, Definitionen von Resten aus den oben genannten allgemeinen Definitionen eingesetzt werden oder Definitionen aus den anderen Gruppen eingefügt oder weggelassen werden.

Bevorzugt ist eine Verbindung der Formel I gemäss Anspruch 1, worin
R₁ und R₉ unabhängig voneinander Wasserstoff; Niederalkanoyl, wie Formyl, Acetyl, Propionyl, Butyryl oder Pivaloyl, insbesondere Acetyl; Arylniederalkanoyl, worin Aryl vorzugsweise wie oben unter den allgemeinen Definitionen für Arylniederalkanoyl definiert und unsubstituiert oder wie dort substituiert ist, d. h. 6 - 14 Kohlenstoffatome hat, wie in Phenyl, Indenyl, Indanyl, Naphthyl, Anthryl, Phenanthryl, Acenaphthyl oder Fluorenyl, und unsubstituiert oder insbesondere durch Niederalkyl, z. B. Methyl, Ethyl oder Propyl, Halogenniederalkyl, z. B. Trifluormethyl, Phenyl, 1 - oder 2-Naphthyl, Hydroxy, Niederalkoxy, z. B. Methoxy, Carbamoylniederalkoxy, N-Niederalkylcarb amoylniederalkoxy oder N,N-Diniederalkylcarbamoylniederalkoxy, Amino, Mono-oder Diniederalkylamino, Niederalkanoylamino, z. B. Pivaloylamino, Halogen, z. B. Fluor, Chlor oder Brom, Carboxy, Niederalkoxycarbonyl, wie tert-Butoxycarbonyl, Phenyl-, Naphthyl- oder Fluorenylniederalkoxycarbonyl, wie Benzyloxycarbonyl, Niederalkanoyl, Sulfo, Niederalkylsulfonyl, z. B. Methylsulfonyl, Phosphono, Hydroxyniederalkoxy-phosphoryl oder Diniederalkoxyphosphoryl, Carbamoyl, Mono- oder Diniederalkylcarbamoyl, Sulfamoyl, Mono- oder Diniederalkylaminosulfonyl, Nitro und/oder Cyano ein-oder bis zu dreifach substituiert sein kann, wobei Phenyl bis zu dreimal vorliegen kann, z. B. in Diphenyl-, Dibenzyl- oder Triphenylniederalkanoyl, wie Diphenyl-, Dibenzyl-oder Triphenylacetyl, und worin Niederalkanoyl unsubstituiert oder substituiert ist durch Carbamoyl oder durch einen oder zwei aus Niederalkyl, Carboxyniederalkyl, Niederalkoxycarbonyl-niederalkyl, Diniederalkylamino-niederalkyl, Amino-carboxyniederalkyl, Hydroxyniederalkyl und Diniederalkoxyniederalkyl ausgewählte Reste am Stickstoff substituiertes Carbamoyl, vorzugsweise, wie unter Arylniederalkanoyl oben bei den allgemeinen Definitionen beschrieben, beispielsweise 4-Chlor-, 4-Methoxy- oder 4-Nitrobenzoyl, Naphthylcarbonyl, wie α- oder β-Naphthylcarbonyl oder über beide Carbonylgruppen an die Aminogruppe gebundenes 1,8-Naphthalindicarbonyl, Indenylcarbonyl, wie 1-,2- oder 3-Indenylcarbonyl, Indanylcarbonyl, wie 1- oder 2-Indanylcarbonyl, Phenanthrenylcarbonyl, wie 9-Phenanthrenylcarbonyl, Phenylniederalkanoyl, wie Phenylacetyl, α-Naphthylacetyl, β-Naphthylacetyl, Niederalkylphenyl-acetyl, wie 4-Methylphenylacetyl, Niederalkoxyphenylacetyl, wie 4-Methoxyphenylacetyl, 3-Phenylpropionyl, 3-(p-Hydroxyphenyl)-propionyl, Diphenylacetyl, Di-(4-methoxyphenyl)-acetyl, Triphenylacetyl, 2,2-Dibenzylacetyl, 3-α- oder 3-β-Naphthylpropionyl, Phenylniederalkanoyl, worin der Niederalkanoylrest durch Carbamoyl substituiert ist, wie 2(R,S)-Carbamoyl-3-phenylpropionyl, 3-Phenyl- oder 3-α-Naphthyl-2-carbamoylpropionyl, 3-Phenyl- oder 3-α-Naphthyl-2-tert-butylcarbamoyl-propionyl, 3-Phenyl- oder 3-α-Naphthyl-2-(2-dimethylaminoethyl)carbamoylpropionyl, 3-α-Naphthyl-2-(carboxy- oder tert-butoxycarbonyl)methylcarbamoylpropionyl, 3-Phenyl- oder 3-α-Naphthyl-2-(3-hydroxy-2-propyl)carbamoyl-propionyl, 3-Phenyl- oder 3-α-Naphthyl-2-(2,2-dimethoxyethyl)-carbamoylpropionyl, 3-Phenyl- oder 3-α-Naphthyl-2(5-amino-5-carboxypentyl)-carbamoylpropionyl, in erster Linie Phenylniederalkanyol, wie Phenylacetyl, oder Phenyl-niederalkanoyl, worin der Niederalkanoylrest durch Carbamoyl substituiert ist, wie 2(R,S)-Carbamoyl-3-phenylpropionyl; Heterocyclylniederalkanoyl, welches vorzugsweise wie oben für Heteroarylniederalkanoyl R₁, R₂, R₈ und R₉ definiert ist, insbesondere Heterocyclylniederalkanoyl, worin Niederalkanoyl unsubstituiert ist und worin Heterocyclyl vorzugsweise ein einfaches oder doppeltes Ringsystem mit 3 - 10 Ringatomen ist, über ein Kohlenstoff- oder insbesondere Stickstoffatom gebunden ist, bis zu 3 weitere Heteroatome, ausgewählt aus Sauerstoff, Stickstoff, Schwefel, Selen und mit 1 oder 2 Sauerstoffatomen verknüpftem Schwefel, enthält, und ungesättigt oder auch teilweise oder ganz gesättigt sein kann, z. B. Thienyl-, Furyl-, Pyranyl-, Pyrrolyl-, Imidazolyl-, Pyrazolyl-, Oxazolyl-, Isoxazolyl-, Thiazolyl-, Furazanyl-, Pyridyl-, Pyrazinyl-, Pyrimidinyl-, Pyridazinyl-, Azepinyl-, Indolyl-, Benzimidazolyl-, 1H-Indazolyl-, Chinolyl-, Isochinolyl-, Chinoxalinyl-, Chinazolinyl-, Cinnolyl-, Purinyl-, Pteridinyl-, Naphthyndinyl-, 4H-Chinolizinyl-, 3,1-Benzfuranyl-, 4,1-Benzoxazinyl-, 4,1-Benzthiazinyl-, Cyclohexa[b]pyrrolyl-, Cyclohepta[b]pyrrolyl-, Cyclohexa[d]pyrazolyl-, Cyclohexa[b]pyridyl-, Cyclohexa[b]pyrazinyl-, Cyclohexa[b]pyrimidinyl-, Cyclohexa[b]-1,4-oxazinyl-, Cyclohexa[b]-1,4-thiazinyl-, Pyrrolidinyl-, Pyrrolinyl-, Imidazolidyl-, 2-Imidazolinyl-, 2,3-Dihydropyndyl-, Piperidyl-, Piperazinyl-, 2,3,5,6-Tetrahydropyrazinyl-, Morpholinyl-, Thiomorpholinyl-, S,S-Dioxo-thiomorpholinyl-, Indolinyl-, Isoindolinyl-, 4,5,6,7-Tetrahydroindolyl-, 1,2,3,4-Tetrahydrochinolyl-, 1,2,3,4-Tetrahydroisochinolyl-, Chromanyl-, Thiochromanyl-, 1,2,3,4-Tetrahydro-3,1-benzdiazinyl, 3,4-Dihydro-3H-4,1-benzoxazinyl- oder 3,4-Dihydro-3H-4,1-benzthiazinyl-niederalkanoyl, wobei die genannten Heterocyclyl-Reste unsubstituiert oder durch Niederalkyl, z. B. Methyl, Phenyl, 1- oder 2-Naphthyl, Phenylniederalkyl, z. B. Benzyl, Hydroxyniederalkyl, z. B. Hydroxymethyl oder 2-Hydroxyethyl, Niederalkoxyniederalkyl, z. B. Methoxymethyl oder 2-Methoxyethyl, Phenoxy- oder Naphthoxyniederalkyl, z. B. 2-Phenoxyethyl, 1- oder 2-Naphthoxymethyl, Phenylniederalkoxy- oder Naphthylniederalkoxy-niederalkyl, z. B. Benzyloxyniederalkyl, Niederalkanoyloxy-niederalkyl, z. B. Acetyloxymethyl, Phenyl- oder Napthyl-niederalkanoyloxy-niederalkyl, z. B. Benzoyloxy-, Phenylacetyloxy- oder 1- oder 2-Naphthoyloxy-methyl, -2-ethyl oder -2-(2,2-dimethylethyl), Niederalkoxycarbonyloxy-niederalkyl, z. B. tert-Butyloxycarbonyloxyniederalkyl, Phenyl-, Napthyl- oder Fluorenylniederalkoxycarbonyloxy-niederalkyl, z. B. 2-Benzyloxycarbonyloxyethyl oder 9-Fluorenylmethoxycarbonyloxyethyl, Aminoniederalkyl, z. B. Aminomethyl, 2-Aminoethyl oder 2-Aminopropyl, Carboxyniederalkyl, z. B. Carboxymethyl oder 2-Carboxyethyl, Hydroxy, Niederalkoxy, z. B. Methoxy oder Ethoxy, Phenyl- oder Naphthylniederalkoxy, z. B. Benzyloxy oder 1- oder 2-Naphthyloxy, Amino, Niederalkylamino, z. B. Methyl-, Ethyl- oder tert-Butylamino, Diniederalkylamino, z. B. Dimethyl- oder Diethylamino, Carboxy, Niederalkoxycarbonyl, z. B. Methoxy-, Isopropoxy-, sec-Butoxy- oder tert-Butoxy-carbonyl, Phenyl- oder Napthyl-niederalkoxycarbonyl, z. B. Benzyloxycarbonyl, Halogen, z. B. Fluor, Chlor, Brom oder Jod, insbesondere Chlor oder Brom, Niederalkanoyl, z. B. Acetyl oder Pivaloyl, Niederalkylsulfonyl, z. B. Methyl- oder Ethylsulfonyl, Phosphono, Hydroxy-niederalkoxy-phosphoryl oder Dialkoxy-phosphoryl, z. B. Dimethoxy- oder Diethoxyphosphoryl, Carbamoyl, Mono- oder Diniederalkylcarbamoyl, z. B. N-Methylcarbamoyl, N-n-Butylcarbamoyl oder N,N-Dimethylcarbamoyl, Hydroxy-oder Carboxyniederalkylcarbamoyl, z. B. Hydroxy- oder Carboxymethylcarbamoyl oder Hydroxy- oder Carboxyethylcarbamoyl, Sulfamoyl, durch Nitro, Oxo und/oder durch Cyano substituiert sind, wobei Heterocyclylniederalkanoyl insbesondere ausgewählt ist aus gegebenenfalls durch Niederalkyl oder Phenyl substituiertem Pyrrolylcarbonyl, z. B. 2- oder 3-Pyrrolylcarbonyl, 4- oder 5-Methylpyrrolylcarbonyl oder 4- oder 5-Phenylpyrrolyl-2-carbonyl, Thienylcarbonyl, wie 2-Thienylcarbonyl, Furylcarbonyl, wie 2-Furylcarbonyl, Pyridylcarbonyl, wie 2-, 3- oder 4-Pyridylcarbonyl, Pyrimidin-1-yl-carbonyl, gegebenenfalls durch Niederalkyl, wie Methyl, Phenylniederalkyl, wie Benzyl, Niederalkoxy, wie Methoxy, Phenylniederalkoxy, wie Benzyloxy oder Halogen, wie Chlor, substituiertem Indolylcarbonyl, wie 2-, 3- oder 5-Indolylcarbonyl, 1-Methyl-, 5-Methyl-, 5-Methoxy-, 5-Benzyloxy-, 5-Chlor- oder 4,5-Dimethylindolyl-2-carbonyl, 1-Benzyl-indolyl-2- oder -3-carbonyl, 4,5,6,7-Tetrahydroindolyl-2-carbonyl, gegebenenfalls durch Hydroxy substituiertem Chinolylcarbonyl, wie 2-, 3- oder 4-Chinolylcarbonyl oder 4-Hydroxychinolyl-2-carbonyl, gegebenenfalls durch Hydroxy substituiertem Isochinolylcarbonyl, wie 1-, 3- oder 4-Isochinolylcarbonyl oder 1-Oxo-1,2-dihydroisochinolyl-3-carbonyl, 2-Chinoxalinylcarbonyl, 2-(3,1-Benzfuranyl)carbonyl,Cyclohepta[b]pyrrolyl-5-carbonyl, 3-Chromanylcarbonyl, 3-Thiochromanylcarbonyl, Pyrrolidinyl-3-carbonyl, Hydroxypyrrolidinylcarbonyl, wie 3- oder 4-Hydroxypyrrolidinyl-2-carbonyl, Oxopyrrolidinylcarbonyl, wie 5-Oxopyrrolidinyl-2-carbonyl, Piperidylcarbonyl, wie Piperidinocarbonyl oder 2-, 3- oder 4-Piperidylcarbonyl, Pyrazinylcarbonyl, wie Pyrazin- 1-ylcarbonyl, Piperazinylcarbonyl, wie Piperazin-1-yl-carbonyl, Morpholinylcarbonyl, wie Morpholinocarbonyl, Thiomorpholinylcarbonyl, wie Thiomorpholinocarbonyl, S,S-Dioxothiomorpholinylcarbonyl, wie S,S-Dioxothiomorpholinocarbonyl, Indolinylcarbonyl, wie 2- oder 3-Indolinylcarbonyl, 1,2,3,4-Tetrahydrochinolylcarbonyl, wie 1,2,3,4-Tetrahydrochinolyl-2-, -3- oder -4-carbonyl, 1,2,3,4-Tetrahydroisochinolylcarbonyl, wie 1,2,3,4-Tetrahydroisochinolyl-1-, -3- oder 4-carbonyl oder 1-Oxo-1,2,3,4-tetrahydroisochinolyl-3-carbonyl, und Pyridylniederalkanoyl, z. B. Pyridylacetyl, wie 2-, 3- oder 4-Pyridylacetyl, in erster Linie ausgewählt aus Morpholinocarbonyl, Thiomorpholino-carbonyl, Pyridylniederalkanoyl, wie 2-, 3- oder 4-Pyridylacetyl, und Chinolin-2-carbonyl; Halogenniederalkanoyl, welches bis zu drei Halogenatome enthält, in erster Linie α-Halogenacetyl, wie α-Fluor-, α-Chlor-, α-Brom-, α-Iod-, α,α,α Trifluor- oder α,α,α-Trichloracetyl, oder Halogenpropionyl, wie β-Chlor- oder β-Brompropionyl, insbesondere Trifluoracetyl; (N-Heterocyclylniederalkyl-carbamoyl)-niederalkanoyl, worin Heterocyclyl vorzugsweise aus Pyrroyl, Furyl, Thienyl, Imidazolyl, Pyrazolyl, Oxazolyl, Thiazolyl, Pyridyl, Pyrazinyl, Pyrimidinyl, Indolyl, Chinolyl, Isochinolyl, Chinoxalinyl, β-Carbolinyl und einem benzanellierten, cyclopenta-, cyclohexa-oder cyclohepta-anellierten Derivat dieser Reste, die auch ganz oder teilweise gesättigt sein können, aus Morpholinyl und aus Thiomorpholinyl ausgewählt ist, in erster Linie 2-(N-Morpholinoniederalkyl-carbamoyl)-niederalkanoyl, wie 2(R,S)-(N-(2-morpholinoethyl)-carbamoyl)-3-methyl-butyryl, oder 2-(N-(Pyridylniederalkyl)-carbamoyl)-niederalkanoyl, wie 2(R,S)-(N-(2-pyridylmethyl)-carbamoyl)-3-methyl-butyryl; Niederalkoxycarbonyl, in erster Linie Methoxy-, Ethoxy-, Isopropoxy- oder tert-Niederalkoxycarbonyl, z.B. Methoxy-carbonyl oder tert-Butoxycarbonyl; Arylniederalkoxycarbonyl, worin Aryl vorzugsweise 6 bis 14 Kohlenstoffatome hat und beispielsweise Phenyl, Biphenylyl, 1- oder 2-Naphthyl, Fluorenyl oder durch Niederalkyl, z. B. Methyl oder tert-Butyl, Hydroxy, Niederalkoxy, z. B. Methoxy, Ethoxy oder tert-Butoxy, Halogen, z. B. Chlor oder Brom, und/oder Nitro mono- oder mehrfach substituiertes Phenyl ist, z. B. Phenylniederalkoxycarbonyl, wie Benzyloxycarbonyl, 4-Methoxybenzyloxycarbonyl, 4-Nitrobenzyloxycarbonyl oder Fluorenylniederalkoxycarbonyl, wie 9-Fluorenylmethoxycarbonyl, in erster Linie Phenylniederalkoxycarbonyl, wie Benzyloxycarbonyl; Heterocyclylniederalkoxycarbonyl, worin Heterocyclyl vorzugsweise aus Pyrrolyl, Furyl, Thienyl, Imidazolyl, Pyrazolyl, Oxazolyl, Thiazolyl, Pyridyl, Pyrazinyl, Pyrimidinyl, Indolyl, Chinolyl, Isochinolyl, Chinoxalinyl, β-Carbolinyl und einem benzanellierten, cyclopenta-, cyclohexa- oder cycloheptaanellierten Derivat dieser Reste, die auch ganz oder teilweise gesättigt vorliegen können, ausgewählt ist, z.B.

2-Tetrahydrofuranylniederalkoxycarbonyl, wie 2-Tetrahydrofuryl-methoxycarbonyl, oder 2-, 3- oder 4-Pyridylmethoxycarbonyl, in erster Linie Tetrahydrofuranyl-niederalkyloxycarbonyl, wie 2(R,S)-Tetrahydrofuranylmethoxycarbonyl; Niederalkylsulfonyl, z.B. Methyl- oder Ethylsulfonyl, wie Methylsulfonyl;
N-Heterocyclylniederalkyl-N-niederalkyl-carbamoyl, welches wie oben unter unsubstituiertes oder substituiertes N-Alkyl- oder N,N-Dialkyl-carbamoyl R₁, R₂, R₈ oder R₉ definiert ist, worin vorzugsweise Heterocyclyl ausgewählt ist aus Thienyl, Furyl, Pyranyl, Pyrrolyl, Imidazolyl, Pyrazolyl, Oxazolyl, Isoxazolyl, Thiazolyl, Furazanyl, Pyridyl, Pyrazinyl, Pyrimidinyl, Pyridazinyl, Azepinyl, Indolyl, Benzimidazolyl, 1H-Indazolyl, Chinolyl, Isochinolyl, Chinoxalinyl, Chinazolinyl, Cinnolyl, Purinyl, Pteridinyl, Naphthyridinyl, 4H-Chinolizinyl, 3,1-Benzfuranyl, Benz[e]indolyl, 4,1-Benzoxazinyl, 4,1-Benzthiazinyl, Carbazolyl, β-Carbolinyl, Phenazinyl, Phenanthridyl, Acridyl, Phenoxazinyl, Phenothiazinyl, 1-Azaacenaphtenyl, Cyclohexa[b]pyrrolyl, Cyclohepta[b]pyrrolyl, Cyclohexa[d]pyrazolyl, Cyclohexa[b]pyridyl, Cyclohexa[b]pyrazinyl, Cyclohexa[b]pyrimidinyl, Cyclohexa[b]-1,4-oxazinyl, Cyclohexa[b]-1,4-thiazinyl, Pyrrolidinyl, Pyrrolinyl, Imidazolidinyl, 2-Imidazolinyl, 2,3-Dihydropyridyl, Piperidyl, Piperazinyl, 2,3,5,6-Tetrahydropyrazinyl, Morpholinyl, Thiomorpholinyl, S,S-Dioxothiomorpholinyl, Indolinyl, Isoindolinyl, 4,5,6,7-Tetrahydroindolyl, 1,2,3,4-Tetrahydrochinolyl, 1,2,3,4-Tetrahydroisochinolyl, Chromanyl, Thiochromanyl, 1,2,3,4-Tetrahydro-3,1-benzdiazinyl, 3,4-Dihydro-3H-4,1-benzoxazinyl, 3,4-Dihydro-3H-4,1-benzthiazinyl, 2,3,4,5-Tetrahydro-1H-5,1-benzazepinyl und 5,6-Dihydrophenanthridinyl, wobei die genannten Reste unsubstituiert oder durch Niederalkyl, z. B. Methyl, Phenyl, 1- oder 2-Naphthyl, Phenylniederalkyl, z. B. Benzyl, Hydroxyniederalkyl, z. B. Hydroxymethyl oder 2-Hydroxyethyl, Niederalkoxyniederalkyl, z. B. Methoxymethyl oder 2-Methoxyethyl, Phenoxy- oder Naphthoxyniederalkyl, z. B. 2-Phenoxyethyl, 1- oder 2-Naphthoxymethyl, Phenylniederalkoxy- oder Naphthylniederalkoxy-niederalkyl, z. B. Benzyloxyniederalkyl, Niederalkanoyloxy-niederalkyl, z. B. Acetyloxymethyl, Phenyl- oder Napthyl-niederalkanoyloxy-niederalkyl, z. B. Benzoyloxy-, Phenylacetyloxy- oder 1- oder 2-Naphthoyloxy-methyl, -2-ethyl oder -2-(2,2-dimethylethyl), Niederalkoxycarbonyloxy-niederalkyl, z. B. tert-Butyloxycarbonyloxyniederalkyl, Phenyl-, Napthyl- oder Fluorenylniederalkoxy-carbonyloxy-niederalkyl, z. B. 2-Benzyloxycarbonyloxyethyl oder 9-Fluorenylmethoxy-carbonyloxyethyl, Aminoniederalkyl, z. B. Aminomethyl, 2-Aminoethyl oder 2-Aminopropyl, Carboxyniederalkyl, z. B. Carboxymethyl oder 2-Carboxyethyl, Hydroxy, Nieder-alkoxy, z. B. Methoxy oder Ethoxy, Phenyl- oder Naphthylniederalkoxy, z. B. Benzyloxy oder 1- oder 2-Naphthylmethoxy, Amino, Niederalkylamino, z. B. Methyl-, Ethyl-oder tert-Butylamino, Diniederalkylamino, z. B. Dimethyl- oder Diethylamino, Carboxy, Niederalkoxycarbonyl, z. B. Methoxy-, Isopropoxy-, sec-Butoxy- oder tert-Butoxy-carbonyl, Phenyl- oder Napthylniederalkoxycarbonyl, z. B. Benzyloxycarbonyl, Halogen, z. B. Fluor, Chlor, Brom oder Jod, insbesondere Chlor oder Brom, Niederalkanoyl, z. B. Acetyl oder Pivaloyl, Niederalkylsulfonyl, z. B. Methyl- oder Ethylsulfonyl, Phosphono, Hydroxy-niederalkoxyphosphoryl oder Dialkoxy-phosphoryl, z. B. Dimethoxy- oder Diethoxyphosphoryl, Carbamoyl, Mono- oder Diniederalkylcarbamoyl, z. B. N-Methylcarbamoyl, N-n-Butylcarbamoyl oder N,N-Dimethylcarbamoyl, Hydroxy- oder Carboxyniederalkylcarbamoyl, z. B. Hydroxy- oder Carboxymethyl-carbamoyl oder Hydroxy- oder Carboxyethylcarbamoyl, Sulfamoyl, durch Nitro, Oxo und/oder durch Cyano substituiert sind, und insbesondere Pyridyl, wie 2-, 3- oder 4-Pyridyl, bedeutet, in erster Linie N-Pyridylniederalkyl-N-niederalkyl-carbamoyl, wie N-(2-, 3- oder 4-Pyridylmethyl)-N-methyl-carbamoyl; oder einen Acylrest einer Aminosäure, deren Aminofunktion frei oder durch einen der übrigen bisher für R₁ und R₉ genannten Reste acyliert ist, bedeuten, wobei die Aminosäurereste vorzugsweise unabhängig voneinander wie für unsubstituierte oder substituierte Aminosäuren als Acyl R₁, R₂, R₈ und R₉ definiert sind, insbesondere den Rest einer natürlichen α-Aminosäure mit der L-Konfiguration, wie sie normalerweise in Proteinen vorkommen, oder eines Epimeren einer solchen Aminosäure, d. h. mit der unnatürlichen D-Konfiguration, oder deren D,L-Isomerengemisch, eines Homologen einer solchen Aminosäure, z. B. worin die Aminosäurenseitenkette um eine oder zwei Methylengruppen verlängert oder verkürzt ist, worin die Aminogruppe in β-Stellung vorliegt und/oder worin eine Methylgruppe durch Wasserstoff ersetzt ist, einer substituierten aromatischen Aminosäure, worin der aromatische Rest 6 - 14 Kohlenstoffatome hat, z. B. eines substituierten Phenylalanins oder Phenylglycins, worin der Phenyl-Substituent Niederalkyl, z. B. Methyl, Hydroxy, Niederalkoxy, z. B. Methoxy, Niederalkanoyloxy, z. B. Acetoxy, Amino, Niederalkylamino, z. B. Methylamino, Diniederalkylamino, z. B. Dimethylamino, Niederalkanoylamino, z. B. Acetylamino oder Pivaloylamino, Niederalkoxycarbonylamino, z. B. tert-Butoxycarbonylamino, Arylmethoxycarbonylamino, worin Aryl vorzugsweise 6 - 14 Kohlenstoffatome hat, z. B. Benzyloxycarbonylamino oder 9-Fluorenylmethoxycarbonylamino, Halogen, z. B. Fluor, Chlor, Brom oder Jod, Carboxy und/oder Nitro sein kann und ein- oder mehrfach vorkommt, eines benzannelierten Phenylalanins oder Phenylglycins, wie α-Naphthylalanins, oder eines hydrierten Phenylalanins oder Phenylglycins, wie Cyclohexylalanins oder Cyclohexylglycins, besonders bevorzugt das das über eine Carboxygruppe gebundene Radikal einer Aminosäure ausgewählt aus Glycin (H-Gly-OH), Alanin (H-Ala-OH), Valin (H-Val-OH), Norvalin (α-Aminovaleriansäure), Leucin, (H-Leu-OH), Isoleucin (H-Ile-OH), Norleucin (α-Aminohexansäure, H-Nle-OH), Serin (H-Ser-OH), Homoserin (α-Amino-γ-hydroxybuttersäure), Threonin (H-Thr-OH), Methionin (H-Met-OH), Cystein (H-Cys-OH), Prolin (H-Pro-OH), trans-3- und trans-4-Hydroxyprolin, Phenylalanin (H-Phe-OH), Tyrosin (H-Tyr-OH), 4-Aminophenylalanin, 4-Chlorphenylalanin, 4-Carboxyphenylalanin, β-Phenylserin (β-Hydroxyphenylalanin), Phenylglycin, α-Naphthylalanin (H-Nal-OH), Cyclohexylalanin (H-Cha-OH), Cyclohexylglycin, Tryptophan (H-Trp-OH), Indolin-2-carbonsäure, 1,2,3,4-Tetrahydroisochinolin-3-carbonsäure, Asparaginsäure (H-Asp-OH), Asparagin (H-Asn-OH), Aminomalonsäure, Aminomalonsäure-monoamid, Glutaminsäure (H-Glu-OH), Glutamin (H-Gln-OH), Histidin (H-His-OH), Arginin (H-Arg-OH), Lysin (H-Lys-OH), δ-Hydroxylysin, Ornithin (α,δ-Diaminovaleriansäure), 3-Aminopropansäure, α,γ-Diaminobuttersäure und α,β-Diaminopropionsäure, noch stärker bevorzugt der Rest einer Aminosäure ausgewählt aus Valin, Alanin, Leucin, Isoleucin, Glycin, Glutaminsäure und Asparagin, wobei (ausser Glycin) jede der genannten Aminosäuren in der D-, L- oder (D,L)-, vorzugsweise (ausser Val, das auch in der (D)- oder (D,L)-Form vorliegen kann) in der L-Form vorliegen kann, die α-Aminogruppe unsubstituiert oder durch einen der oben für R₁ und R₉ genannten Reste N-acyliert ist, vor allem durch Niederalkanoyl, Phenylniederalkanyol, wie Phenylacetyl, Phenyl-niederalkanoyl, worin der Niederalkanoylrest durch Carbamoyl substituiert ist, wie 2(R,S)-Carbamoyl-3-phenylpropionyl, Morpholinocarbonyl, Thiomorpholino-carbonyl, Pyridylniederalkanoyl, wie 2-oder 3-Pyridylacetyl, Chinolinyl-niederalkanoyl, wie Chinolin-2-carbonyl, Halogenniederalkanoyl, welches bis zu drei Halogenatome enthält, z. B. α-Halogenacetyl, wie α-Fluor-, α-Chlor-, α-Brom-, α-Iod-, α,α,α-Trifluor- oder α,α,α-Trichloracetyl, oder Halogenpropionyl, wie β-Chlor- oder β-Brompropionyl, insbesondere Trifluoracetyl, 2-(N-Morpholinoniederalky-carbamoyl)-niederalkanoyl, wie 2(R,S)-(N-(2-morpholinoethyl)-carbamoyl-3-methyl-butyryl, 2-(N-(Pyridylniederalkyl)-carbamoyl)-niederalkanoyl, wie 2(R,S)-(N-(2-pyridylmethyl)-carbamoyl)-niederalkanoyl, Niederalkoxycarbonyl, Phenylniederalkoxycarbonyl, Tetrahydrofuranyl-niederalkyloxycarbonyl, wie 2(R,S)-Tetrahydrofuranylmethoxycarbonyl, Niederalkylsulfonyl oder N-Pyridylniederalkyl-N-niederalkyl-carbamoyl, wie N-(2-, 3- oder 4-Pyridylmethyl)-N-methyl-carbamoyl, substituiert ist, wobei N-Morpholinocarbonyl-glycin, N-(N-(2-, 3- oder 4-Pyridyl)methyl-N-methylaminocarbonyl)-glycin, Valin, N-(Trifluoracetyl)-valin, N-Phenylacetyl-valin, N-Acetyl-valin, N-(2-Carbamoyl-3-phenyl-propionyl)-valin, N-(2(R,S)-Carbamoyl-3-phenyl-propionyl)-valin, N-(2- oder 3-Pyridylacetyl)-valin, N-2-Tetrahydrofurylmethoxycarbonyl-valin, N-(Chinolin-2-carbonyl)-valin, N-Methoxycarbonyl-valin, N-tert-Butoxycarbonyl-valin, N-Benzyloxycarbonyl-valin, N-(Morpholinocarbonyl)-valin, N-(Thiomorpholinocarbonyl)valin, N-(S,S-Dioxotiomorpholinocarbonyl)-valin, N-(N-2-Pyridylmethyl-N-methylaminocarbonyl)-valin, N-Methylsulfonyl-valin, N-Acetyl-isoleucin, N-Propionyl-isoleucin, N-(Benzyloxycarbonyl)-isoleucin, N-Benzyloxycarbonyl-glutaminsäure, Asparagin, N-Benzyloxycarbonyl-asparagin oder Chinolin-2-carbonyl-asparagin am stärksten bevorzugt sind, wobei die genannten Aminosäurereste vorzugsweise in der (L)-oder (D,L)-Form, im Falle von Valin auch in der (D)-Form, vorliegen, mit der Massgabe, dass höchstens einer der beiden Reste R₁ oder R₉ Wasserstoff bedeuten kann,
R₂, R₄, R₆ und R₈ Wasserstoff bedeuten,
R₃ Niederalkyl, wie Isobutyl oder n-Butyl, bedeutet; Cycloalkylniederalkyl, wie oben für Cycloalkylniederalkyl R₃, R₄ und R₇ definiert, bedeutet, worin vorzugsweise Cycloalkyl 3 bis 7 Kohlenstoffatome hat und unsubstituiert oder durch Niederalkyl, wie Isopropyl, Halogenniederalkyl, wie Trifluormethyl, Hydroxy, Niederalkoxy, Carbamoylniederalkoxy, N-Niederalkylcarbamoylniederalkoxy oder N,NDiniederalkylcarbamoylniederalkoxy, Amino, Mono- oder Diniederalkylamino, Halogen, wie Fluor, Chlor oder Brom, Carboxy, Niederalkoxycarbonyl, Phenyl-, Naphthyl- oder Fluorenylniederalkoxycarbonyl, Niederalkanoyl, Niederalkylsulfonyl, z. B. Methylsulfonyl, Phosphono, Hydroxy-niederalkoxy-phosphoryl oder Diniederalkoxyphosphoryl, Carbamoyl, Mono- oder Diniederalkylaminocarbamoyl, Sulfamoyl, Mono- oder Diniederalkylsulfamoyl, Nitro und/oder Cyano ein- oder bis zu dreifach substituiert ist und an Niederalkyl, insbesondere Methyl, Ethyl, n-Propyl, Isopropyl, N-Butyl, Isobutyl oder tert-Butyl, vorzugsweise endständig, gebunden ist, wie Cyclobutyl-, Cyclopentyl-, Cyclohexyl- oder Cycloheptyl-niederalkyl, wie -methyl oder -ethyl, in erster Linie Cyclohexylniederalkyl, vor allem Cyclohexyl-methyl; oder Arylniederalkyl bedeutet, welches vorzugsweise wie unter Arylniederalkyl R₃, R₄ und R₇ definiert ist, worin Aryl insbesondere 6 - 14 Kohlenstoffatome enthält, wie in Phenyl, Indenyl, Indanyl, Naphthyl, Anthryl, Phenanthryl, Acenaphthyl oder Fluorenyl, und unsubstituiert oder substituiert ist, insbesondere durch Niederalkyl, z. B. Methyl, Ethyl oder Isopropyl, Halogenniederalkyl, wie Trifluormethyl, Phenyl, 1 - oder 2-Naphthyl, Hydroxy, Niederalkoxy, z. B. Methoxy, Carbamoylniederalkoxy, N-Niederalkylcarbamoylniederalkoxy oder N,N-Diniederalkylcarbamoylniederalkoxy, Amino, Mono- oder Diniederalkylamino, Niederalkanoylamino, z. B. Pivaloylamino, Halogen, z. B. Fluor oder Chlor, Carboxy, Niederalkoxycarbonyl, Benzyl-, Naphthyl- oder Fluorenylniederalkoxycarbonyl, Niederalkanoyl, Sulfo, Niederalkylsulfonyl, Phosphono, Hydroxy-niederalkoxy-phosphoryl oder Diniederalkoxyphosphoryl, Carbamoyl, Mono-oder Diniederalkylcarbamoyl, Sulfamoyl, Mono- oder Diniederalkylsulfamoyl, Nitro und/oder Cyano ein- oder bis zu dreifach substituiert sein kann, wobei Phenyl bis zu dreimal vorliegen kann, wie in Diphenyl-, Dibenzyl- oder Triphenylniederalkyl, z. B. Diphenyl-, Dibenzyl- oder Triphenyl-2-ethyl, in erster Linie Phenylniederalkyl, welches unsubstituiert oder durch die genannten Substituenten substituiert ist, vor allem Benzyl, 2-Phenylethyl, 3-Phenylpropyl, 4- Fluor-, 4-Cyano-, 4-Methoxy- oder 4-Hydroxybenzyl,
R₅ Hydroxy bedeutet, und
R₇ unsubstituiertes oder substituiertes Niederalkyl bedeutet, vorzugsweise unsubstituiert oder wie oben für unsubstituiertes oder substituiertes Alkyl R₃, R₄ oder R₇ beschrieben substituiert, in erster Linie Niederalkyl, vor allem Isobutyl oder n-Butyl, Cycloalkylniederalkyl, wie zuletzt für Cycloalkylniederalkyl R₃ beschrieben, vor allem Cyclohexylniederalkyl, wie Cyclohexylmethyl, oder Arylniederalkyl, wie zuletzt für Arylniederalkyl R₃ beschrieben, vor allem Phenylniederalkyl, welches unsubstituiert oder durch die genannten Substituenten substituiert ist, wie Benzyl, 2-Phenylethyl, 3-Phenylpropyl, 4- Fluor-, 4-Cyano-, 4-Methoxy- oder 4-Hydroxybenzyl,
oder ein Salz davon, sofern eine salzbildende Gruppe vorliegt.

Von den zuletzt genannten Verbindungen der Formel I sind solche besonders bevorzugt, worin
R₁ und R₉ unabhängig voneinander Wasserstoff; Niederalkanoyl; Arylniederalkanoyl, worin der Niederalkanoylrest unsubstituiert oder substituiert ist durch Carbamoyl oder durch am Stickstoff durch einen oder zwei Reste ausgewählt aus Niederalkyl, Carboxyniederalkyl, Niederalkoxycarbonyl-niederalkyl, Diniederalkylaminoniederalkyl, Amino-carboxy-niederalkyl, Hydroxyniederalkyl, oder Diniederalkoxy-niederalkyl substituiertes Carbamoyl, und worin Aryl 6 - 14 Kohlenstoffatome enthält; Heterocyclylniederalkanoyl, worin Heterocyclyl 3 - 10 Ringatome und bis zu 4 Heteroatoem ausgewählt aus O, N, S, Se oder mit 1 oder 2 O-Atomen verknüpftem S (S=O, O=S=O) enthält; Halogenniederalkanoyl mit 1 - 3 Halogenatomen; N-Heterocyclylniederalkylcarbamoyl-niederalkanoyl; Niederalkoxycarbonyl; Arylniederalkoxycarbonyl, worin Aryl 6 - 14 Kohlenstoffatome enthält; Heterocyclylniederalkoxycarbonyl, worin Heterocyclyl ausgewählt ist aus Pyrrolyl, Furyl, Thienyl, Imidazolyl, Pyrazolyl, Oxazolyl, Thiazolyl, Pyridyl, Pyrazinyl, Pyrizimidinyl, Indolyl, Chinolyl, Isochinolyl, Chinoxalinyl, β-Carbolinyl und einem benzannelierten, cyclopenty-, cyclohexa- oder cyclohepta-annelierten Derivat dieser Reste, die auch ganz oder teilweise gesättigt vorliegen können;
Niederalkylsulfonyl; N-(Heterocyclylniederalkyl)-N-niederalkyl-carbamoyl; oder das über die Carboxygruppe gebundene Radikal einer Aminosäure ausgewählt aus Glycin, Alanin, Valin, Norvalin, Leucin, Isoleucin, Norleucin, Serin, Homoserin, Threonin, Methionin, Cystein, Prolin, trans-3 und trans-4-Hydroxyprolin, Phenylalanin, Tyrosin, 4-Aminophenylalanin, 4-Chlorphenylalanin, 4-Carboxyphenylalanin, β-Hydroxyphenylalanin, Phenylglycin, α-Naphthylalanin, Cyclohexylalanin, Cyclohexylglycin, Tryptophan, Asparaginsäure, Asparagin, Aminomalonsäure, Aminomalonsäure-monoamid, Glutaminsäure, Glutamin, Histidin, Arginin, Lysin, δ-Hydroxylysin, Ornithin, α,γ-Diaminobuttersäure oder α,β-Diaminopropionsäure, wobei die Aminosäureradikale unsubstituiert oder durch einen der genannten Reste R₁ oder R₉ ausser dem Radikal einer der Aminosäuren selbst substituiert sind, bedeuten;
R₂, R₄, R₆ und R₉ Wasserstoff bedeuten,
R₃ Cycloalkylniederalkyl, worin Cycloalkyl 3 - 7 Kohlenstoffatome hat, oder Arylniederalkyl, worin Aryl 6 - 14 Kohlenstoffatome hat, bedeutet,
R₅ Hydroxy bedeutet, und
R₇ Niederalkyl, Cycloalkylniederalkyl, worin Cycloalkyl 3 - 7 Kohlenstoffatome hat, oder Arylniederalkyl, worin Aryl 6 - 14 Kohlenstoffatome hat, bedeutet,
und Salze von solchen Verbindungen mit salzbildenden Gruppen, wobei vorzugsweise die genannten allgemeinen Ausdrücke und Definitionen die im letzten Absatz genannten bevorzugten Bedeutungen haben.

Bevorzugt sind auch die Verbindungen der Formel I, worin
R₁ und R₉ unabhängig voneinander
- Wasserstoff,
- Niederalkoxycarbonyl,
- 2-Halogenniederalkoxycarbonyl,
- Aryloxycarbonyl, worin Aryl 6 bis 14 Kohlenstoffatome hat,
- Arylniederalkoxycarbonyl, worin Aryl 6 bis 14 Kohlenstoffatome hat,
- Heterocyclylniederalkoxycarbonyl, worin Heterocyclyl aus Pyrrolyl, Furyl, Thienyl, Imidazolyl, Pyrazolyl, Oxazolyl, Thiazolyl, Pyridyl, Pyrazinyl, Pyrimidinyl, Indolyl, Chinolyl, Isochinolyl, Chinoxalinyl, β-Carbolinyl und einem benzanellierten, cyclopenta-, cyclohexa- oder cyclohepta-anellierten Derivat dieser Reste, die auch ganz oder teilweise gesättigt vorliegen können, ausgewählt ist,
- 2-Triniederalkylsilylniederalkoxycarbonyl,
- 2-Triarylsilylniederalkoxycarbonyl, worin Aryl Phenyl oder 1- oder 2-Naphthyl ist, - das über die Carboxygruppe gebundene Radikal einer einer Aminosäure ausgewählt aus Glycin, Alanin, Valin, Norvalin, Leucin, Isoleucin, Norleucin, Serin, Homoserin, Threonin, Methionin, Cystein, Prolin, trans-3- und trans-4-Hydroxyprolin, Phenylalanin, Tyrosin, 4-Aminophenylalanin, 4-Chlorphenylalanin, 4-Carboxyphenylalanin, β-Phenylserin, Phenylglycin, α-Naphthylalanin, Cyclohexylalanin, Cyclohexylglycin, Typtophan, Indolin-2-carbonsäure, 1,2,3,4-Teträhydroisochinolin-3-carbonsäure, Asparaginsäure, Asparagin, Aminomalonsäure, Aminomalonsäure-monoamid, Glutaminsäure, Glutamin, Histidin, Arginin, Lysin, δ-Hydroxylysin, Ornithin, α,γ-Diaminobuttersäure und α,β-Diaminopropionsäure, wobei jede dieser Aminosäuren in der D-, L- oder (D,L)-Form vorliegen kann, bevorzugt in der L-Form, und
die α-Aminogruppe unsubstituiert oder ein- oder zweifach N-alkyliert durch Niederalkyl, durch Aminoniederalkyl, durch Phenyl- oder Naphthylaminoniederalkyl, oder durch Niederalkyl am Stickstoff substituiertes Piperazinylcarbonylniederalkyl, oder N-acyliert durch Niederalkanoyl, durch Arylniederalkanoyl, worin Aryl ausgewählt ist aus Phenyl, Indenyl, Indanyl, Naphthyl, Anthryl, Phenanthryl, Acenaphthyl und Fluorenyl, und unsubstituiert oder durch Niederalkyl, Halogenniederalkyl, Phenyl, 1- oder 2-Naphthyl, Hydroxy, Niederalkoxy, Carbamoylniederalkoxy, N-Niederalkylcarbamoylniederalkoxy oder N,N-Diniederalkylcarbamoylniederalkoxy, Amino, Mono- oder Diniederalkylamino, Niederalkanoylamino, Halogen, Carboxy, Niederalkoxycarbonyl, Phenyl-, Naphthyl-oder Fluorenylniederalkoxycarbonyl, Niederalkanoyl, Sulfo, Niederalkylsulfonyl, Phosphono, Hydroxy-niederalkoxy-phosphoryl oder Diniederalkoxyphosphoryl, Carbamoyl, Mono-oder Diniederalkylcarbamoyl, Sulfamoyl, Mono- oder Diniederalkylaminosulfonyl, Nitro und/oder Cyano ein- oder bis zu dreifach substituiert sein kann, wobei Phenyl bis zu dreimal vorliegen kann, und Niederalkanoyl unsubstituiert oder substituiert durch Niederalkyl, Heterocyclyl ausgewählt aus Pyrrolyl, Furyl, Thienyl, Imidazolyl, Pyrazolyl, Oxazolyl, Thiazolyl, Pyridyl, Pyrazinyl, Pyrimidinyl, Indolyl, Chinolyl, Isochinolyl, Chinoxalinyl, β-Carbolinyl und einem benz-, cyclopenta-, cyclohexa- oder cyclohepta-anellierten Derivat dieser Reste, die auch ganz oder teilweise gesättigt vorliegen können, Hydroxy, Niederalkoxy, Niederalkanoyloxy, Acetoacetoxy, Amino- oder Benzyl-oxycarbonylamino-niederalkanoyloxy, Arylniederalkanoyloxy, worin Aryl 6 - 10 Kohlenstoffatome hat, Niederalkoxycarbonyloxy, Mono- oder Diniederalkyl-aminocarbonyloxy, Aryloxycarbonyloxy, worin Aryl 6 - 10 Kohlenstoffatome hat, Arylniederalkoxycarbo-nyloxy mit Aryl von 6 - 14 Kohlenstoffatomen, Sulfonyloxy, Niederalkylsulfonyloxy, Phenylsulfonyloxy, 2- oder 4-Toluolsulfonyloxy oder 1 - oder 2- Naphthylsulfonyloxy, Carboxy, verestertes Carboxy ausgewählt aus Niederalkoxycarbonyl, Aryloxycarbonyl, worin Aryl 6 - 10 Kohlenstoffatome hat, Arylniederalkoxycarbonyl, worin Aryl 6 - 12 Kohlenstoffatome hat, Niederalkanoyl, Niederalkylsulfonyl, Hydroxy-niederalkoxy-phosphoryl und Diniederalkoxyphosphoryl, Carbamoyl, durch einen oder zwei aus Niederalkyl, Carboxyniederalkyl, Niederalkoxycarbonyl-niederalkyl, Diniederalkylamino-niederalkyl, Aminocarboxyniederalkyl, Hydroxyniederalkyl und aus Diniederalkoxyniederalkyl ausgewählte Reste oder einen aus Ethylen, Trimethylen, Tetramethylen und Pentamethylen ausgewählten Rest, worin ein Kohlenstoffatom durch Stickstoff, Sauerstoff, Schwefel oder mit Sauerstoff ein- oder zweifach substituierten Schwefel ersetzt sein kann und das ungesättigt sein kann, substituiertes Carbamoyl, ferner Sulfamoyl, Phosphono, Benzfuranyl, Oxo und/oder Cyano sein kann und unverzweigt oder verzweigt ist durch Heterocyclylniederalkanoyl ausgewählt aus Thienyl-, Furyl-, Pyranyl-, Pyrrolyl-, Imidazolyl-, Pyrazolyl-, Oxazolyl-, Isoxazolyl-, Thiazolyl-, Furazanyl-, Pyridyl-, Pyrazinyl-, Pyrimidinyl-, Pyridazinyl-, Azepinyl-, Indolyl-, Benzimidazolyl-, 1H-Indazolyl-, Chinolyl-, Isochinolyl-, Chinoxalinyl-, Chinazolinyl-, Cinnolyl-, Purinyl-, Pteridinyl-, Naphthyridinyl-, 4H-Chinolizinyl-, 3,1Benzfuranyl, Benz[e]indolyl-, 4,1-Benzoxazinyl-, 4,1-Benzthiazinyl-, Carbazolyl-, β-Carbolinyl-, Phenazinyl-, Phenanthridyl-, Acridyl-, Phenoxazinyl-, Phenothiazinyl-, 1-Azaacenaphtenyl-, Cyclohexa[b]pyrrolyl-, Cyclohepta[b]pyrrolyl-, Cyclohexa[d]pyrazolyl-, Cyclohexa[b]pyridyl-, Cyclohexa[b]pyrazinyl-, Cyclohexa[b]pyrimidinyl-, Cyclohexa[b]-1,4-oxazinyl-, Cyclohexa[b]- 1,4-thiazinyl-, Pyrrolidinyl, Pyrrolinyl-, Imidazolidinyl-, 2-Imidazolinyl-, 2,3-Dihydropyridyl-, Piperidyl-, Piperazinyl-, 2,3,5,6-Tetrahydropyrazinyl-, Morpholinyl-, Thiomorpholinyl-, S,S -Dioxo-thiomorpholinyl, Indolinyl-, Isoindolinyl-, 4,5,6,7-Tetrahydroindolyl-, 1,2,3,4-Tetrahydrochinolyl-, 1,2,3,4-Tetrahydroisochinolyl-, Chroman-, Thiochroman-, 1,2,3,4-Tetrahydro-3,1-benzdiazinyl-, 3,4-Dihydro-3H-4,1-benzoxazinyl-, 3,4-Dihydro-3H-4,1benzthiazinyl-, 2,3,4,5-Tetrahydro-1H-5,1 -benzazepinyl- und 5,6-Dihydrophenanthridinyl-niederalkanoyl, wobei die genannten Heterocyclyl-Reste unsubstituiert oder durch Niederalkyl, Phenyl, 1 - oder 2-Naphthyl, Phenylniederalkyl, Hydroxyniederalkyl, Niederalkoxyniederalkyl, Phenoxy-oder Naphthoxyniederalkyl, Phenylniederalkoxy- oder Naphthylniederalkoxy-niederalkyl, Nieder-alkanoyloxy-niederalkyl, Phenyl- oder Napthyl-niederalkanoyloxy-niederalkyl, Niederalkoxycarbonyloxy-niederalkyl, Phenyl-, Napthyl- oder Fluorenylniederalkoxycarbonyloxy-niederalkyl, Aminoniederalkyl, Carboxyniederalkyl, Hydroxy, Niederalkoxy, Phenyl- oder Naphthylniederalkoxy, Amino, Niederalkylamino, Diniederalkylamino, Carboxy, Niederalkoxycarbonyl, Phenyl- oder Napthyl-niederalkoxycarbonyl, Halogen, Niederalkanoyl, Niederalkylsulfonyl, Phosphono, Hydroxy-niederalkoxy-phosphoryl, Dialkoxy-phosphoryl, Carbamoyl, Mono- oder Di-niederalkylcarbamoyl, Hydroxy-oder Carboxyniederalkylcarbamoyl, Sulfamoyl, durch Nitro, Oxo und/oder durch Cyano substituiert sind, durch Heterocyclylniederalkenoyl, worin Heterocyclyl ausgewählt ist aus Pyrrolyl, Furyl, Thienyl, Imidazolyl, Pyrazolyl, Oxazolyl, Thiazolyl, Pyridyl, Pyrazinyl, Pyrimidinyl, Indolyl, Chinolyl, Isochinolyl, Chinoxalinyl, β-Carbolinyl und einem benzanellierten, cyclopenta-, cyclohexa- oder cyclohepta-anellierten Derivat dieser Reste, die auch ganz oder teilweise gesättigt sein können, durch Niederalkoxycarbonyl, durch Arylniederalkoxycarbonyl, worin Aryl 6 - 14 Kohlenstoffatome hat, durch Heterocyclylniederalkoxycarbonyl, worin Heterocyclyl ausgewählt ist aus Pyrrolyl, Furyl, Thienyl, Imidazolyl, Pyrazolyl, Oxazolyl, Thiazolyl, Pyridyl, Pyrazinyl, Pyrimidinyl, Indolyl, Chinolyl, Isochinolyl, Chinoxalinyl, β-Carbolinyl und einem benzanellierten, cyclopenta-, cyclohexa- oder cyclohepta-anellierten Derivat dieser Reste, die auch ganz oder teilweise gesättigt und unsubstituiert oder substituiert durch Niederalkyl sein können, durch Carboxyniederalkanoyl, durch Niederalkoxycarbonylniederalkanoyl, durch Hydroxy-niederalkoxy-niederalkanoyl, durch Aminoniederalkanoyl oder durch Benzyloxycarbonylaminoniederalkanoyl, worin die Aminogruppe nicht in α- oder β-Stellung gebunden ist, durch Carbamoyl, durch Phenylniederalkylaminocarbonyl, durch N-Diniederalkylaminoniederalkyl-N-niederalkylaminocarbonyl, durch N-Dihydroxyniederalkyl-N-niederalkylaminocarbonyl, durch 2- oder 3-Pyridylniederalkylaminocarbonyl, durch N-2-Pyridyl-niederalkyl-N-niederalkylaminocarbonyl, durch Sulfonyl, durch Niederalkylsulfonyl, durch Arylsulfonyl, worin Aryl 6 - 10 Kohlenstoffatome hat und unsübstituiert oder durch Niederalkyl oder Niederalkoxy substituiert ist, durch Heterocyclylsulfonyl, worin Heterocyclyl ausgewählt ist aus Pyrrolyl, Furyl, Thienyl, Imidazolyl, Pyrazolyl, Oxazolyl, Thiazolyl, Pyridyl, Pyrazinyl, Pyrimidinyl, Indolyl, Chinolyl, Isochinolyl, Chinoxalinyl, β-Carbolinyl und einem benzanellierten, cyclopenta-, cyclohexa- oder cyclohepta-anel-lierten Derivat dieser Reste, die auch ganz oder teilweise gesättigt sein können, durch Sulfamoyl, oder durch mit Heterocyclylniederalkyl, worin Heterocyclyl wie zuletzt defi-niert ist, und/oder mit Niederalkyl substituiertes Sulfamoyl vorliegen kann,
eine Carboxygruppe der Seitenkette frei oder in veresterter Form als Nie-deralkylestergruppe, als Arylestergruppe oder als Arylniederalkylestergruppe, wobei Aryl Phenyl, 4-Nitrophenyl, Naphthyl oder Biphenylyl bedeutet, oder in amidierter Form als Carbamoyl-, als Niederalkylcarbamoyl-, als Diniederalkylaminocarbamoyl-, als Mono- oder Di-(hydroxyniederalkyl)carbamoyl- oder als Mono- oder Di-(carboxynieder-alkyl)carbamoylgruppe vorliegt,
eine Aminogruppe der Seitenkette frei oder in alkylierter Form als Mono-oder Diniederalkylamino oder in acylierter Form als Niederalkanoylamino, als Aminoniederalkano-ylamino, als Arylniederalkanoylamino, worin Aryl 6 - 14 Kohlenstoffatome hat und unsubstituiert oder durch Niederalkyl, Hydroxy, Niederalkoxy, Carboxy, Carbamoyl oder Sulfamoyl substituiert ist, als Niederalkoxycarbonylamino-, als Aryl-methoxycarbonylaminogruppe, worin Aryl 6 - 14 Kohlenstoffatome hat, als Piperidyl-1-carbonyl, Morpholinocarbonyl, Thiomorpholinocarbonyl oder als S,S-Dioxo-thiomorpholinocarbonyl vorliegt und/oder
eine Hydroxygruppe der Seitenkette frei oder in veretherter oder veresterter Form als Niederalkoxy-, als Arylniederalkoxy-, als Niederalkanoyloxy-, oder als Nieder-alkyloxycarbonyloxygruppe vorliegt,
- Niederalkylsulfonyl,
- 2- oder 3-Pyrrolyl-, 2-Thienyl-, 2-Furyl-, 1-Pyrazolyl-, 2-, 3- oder 4-Pyridyl-, 2-, 3- oder 5-Indolyl-, (1-Methyl-, 2-Methyl-, 5-Methoxy-, 5-Benzyloxy-, 5-Chlor- oder 4,5-Dimethyl-indol-2-yl)-, (1-Benzylindol-2-yl oder -3-yl)-, 4,5,6,7-Tetrahydroindol-2-yl-, (2-, 3- oder 4-Chinolyl oder 4-Hydroxychinol-2-yl)-, (1-, 3- oder 4-Isochinolyl oder 1-Oxo-1,2-dihydroisochinol-3-yl)-, 3-Pyrrolidinyl-, (3- oder 4-Hydroxypyrrolidin-2-yl)-, 5-Oxo-pyrrolidin-2-yl-, (2- oder 3-Morpholinyl)-, (2- oder 3-Thiomorpholinyl)-, (S,S-Dioxothio-morpholin-2- oder -3-yl)-, (2- oder 3-Indolinyl)-, (1,2,3,4-Tetrahydrochinol-2-, -3- oder -4-yl)- oder (1,2,3,4-Tetrahydroisochinol-1-, -2- oder -3-yl)-methylsulfonyl
- unsubstituiertes oder durch Niederalkyl ein- oder zweifach substituiertes Phenyl-oder 1 - oder 2-Napthyl-sulfonyl,
- Niederalkoxysulfonyl, oder
- Benzyloxysulfonyl oder 1 - oder 2-Naphthyloxysulfonyl
bedeuten, so dass höchstens einer der Reste R₁ oder R₉ Wasserstoff bedeuten kann, und R₂ und R₈ unabhängig voneinander Wasserstoff oder die gleichen Reste wie R₁ und R₉ bedeuten,
oder die Substituentenpaare R₁ und R₉ oder R₂ und R₈ jeweils unabhängig voneinander gemeinsam mit dem bindenden Stickstoff und einem aus Ethylen, Trimethylen, Tetra-methylen und Pentamethylen ausgewählten Rest, worin ein Kohlenstoffatom durch Stick-stoff, Sauerstoff, Schwefel oder mit Sauerstoff ein- oder zweifach substituierten Schwefel ersetzt sein kann und das ungesättigt sein kann, einen heterocyclischen Ring bilden,
R₃
- Cycloalkylniederalkyl, worin Cycloalkyl 3 - 7 Kohlenstoffatome hat und unsubstituiert oder durch Niederalkyl, Halogenniederalkyl, Hydroxy, Niederalkoxy, Carbamoylniederalkoxy, N-Niederalkylcarbamoylniederalkoxy oder N,N-Diniederalkylcarbamoylniederalkoxy, Amino, Mono- oder Diniederalkylamino, Halogen, Carboxy, Niederalkoxycarbonyl, Phenyl-, Naphthyl- oder Fluorenylniederalkoxycarbonyl, Niederalkanoyl, Niederalkylsulfonyl, Phosphono, Hydroxy-niederalkoxy-phosphoryl oder Diniederalkoxyphosphoryl, Carbamoyl, Mono- oder Diniederalkylaminocarbamoyl, Sulfamoyl, Mono- oder Diniederalkylsulfamoyl, Nitro und/oder Cyano ein- oder bis zu dreifach substituiert ist und an Niederalkyl gebunden ist, oder
- Arylniederalkyl mit Aryl ausgewählt aus Phenyl, Indenyl, Indanyl, Naphthyl, Anthryl, Phenanthryl, Acenaphthyl und Fluorenyl, das unsubstituiert oder durch Niederalkyl, Halogenniederalkyl, Phenyl, 1 - oder 2-Naphthyl, Hydroxy, Niederalkoxy, Carbamoylniederalkoxy, N-Niederalkylcarbamoylniederalkoxy oder N,N-Diniederalkylcarbamoylnie-deralkoxy, Amino, Mono- oder Diniederalkylamino, Niederalkanoylamino, Halogen, Carboxy, Niederalkoxycarbonyl, Phenyl-, Naphthyl- oder Fluorenylniederalkoxycarbonyl, Niederalkanoyl, Sulfo, Niederalkylsulfonyl, Phosphono, Hydroxy-niederalkoxy-phosphoryl oder Diniederalkoxyphosphoryl, Carbamoyl, Mono- oder Diniederalkylcarbamoyl, Sulfamoyl, Mono- oder Diniederalkylsulfamoyl, Nitro und/oder Cyano ein- oder bis zu dreifach substituiert sein kann, wobei Phenyl bis zu dreimal vorliegen kann, und mit Niederalkyl, welches unsubstituiert oder substituiert ist durch Niederalkyl, Heterocyclyl ausgewählt aus Pyrrolyl, Furyl, Thienyl, Imidazolyl, Pyrazolyl, Oxazolyl, Thiazolyl, Pyridyl, Pyrazinyl, Pyrimidinyl, Indolyl, Chinolyl, Isochinolyl, Chinoxalinyl, β-Carbolinyl oder einem benzanellierten, cyclopenta-, cyclohexa- oder cyclohepta-anellierten Derivat dieser Reste, die auch ganz oder teilweise gesättigt vorliegen können, Hydroxy, Niederalkoxy, Niederalkanoyloxy, Acetoacetoxy, Amino- oder Benzyloxycarbonylamino-niederalka noyloxy, Arylniederalkanoyloxy, worin Aryl 6 - 10 Kohlenstoffatome hat Niederalkoxy-carbonyloxy, Mono- oder Diniederalkyl-aminocarbonyloxy, Aryloxycarbonyloxy, worin Aryl 6 - 10 Kohlenstoffatome hat, Arylniederalkoxycarbonyloxy mit Aryl von 6 - 12 Koh-lenstoffatomen, Sulfonyloxy, Niederalkylsulfonyloxy, Phenylsulfonyloxy, 2- oder 4-Toluolsulfonyloxy oder 1 - oder 2- Naphthylsulfonyloxy, Amino, Mono- oder Diniederalkylamino, N-Niederalkoxy-N-niederalkylamino, Mono- oder Di-(Phenyl- oder Naphthylniederalkyl)amino, Niederalkanoylamino, Carboxy, verestertes Carboxy ausgewählt aus Niederalkoxycarbonyl, Aryloxycarbonyl, worin Aryl 6 - 10 Kohlenstoffatome hat, Arylniederalkoxycarbonyl, worin Aryl 6 - 12 Kohlenstoffatome hat, Niederalkanoyl, Nie-deralkylsulfonyl, Hydroxy-niederalkoxyphosphoryl und Diniederalkoxyphosphoryl, Carbamoyl, durch einen oder zwei aus Niederalkyl, aus Carboxyniederalkyl oder Niederalkoxycarbonyl-niederalkyl, aus Diniederalkylaminoniederalkyl, aus Aminocarboxyniederalkyl, aus Hydroxyniederalkyl, und aus Diniederalkoxyniederalkyl ausgewählte Reste oder einen aus Ethylen, Trimethylen, Tetramethylen und Pentamethylen ausgewählten Rest, worin ein Kohlenstoffatom durch Stickstoff, Sauerstoff, Schwefel oder mit Sauerstoff ein-oder zweifach substituierten Schwefel ersetzt sein kann und das ungesättigt sein kann, substituiertes Carbamoyl, ferner Sulfamoyl, Phosphono, Benzofwanyl, Oxo und/oder Cyano sein kann und unverzweigt oder verzweigt ist,
R₄ Wasserstoff bedeutet,
R₅ Hydroxy bedeutet und
R₆ Wasserstoff bedeutet,
oder R₅ und R₆ zusammen Oxo bedeuten, und
R₇
- Niederalkyl,
- Cycloalkylniederalkyl, worin Cycloalkyl 3 - 7 Kohlenstoffatome hat und unsubstituiert oder durch Niederalkyl, Halogenniederalkyl, Hydroxy, Niederalkoxy, Carbamoylniederalkoxy, N-Niederalkylcarbamoylniederalkoxy oder N,N-Diniederalkylcarbamoylniederalkoxy, Amino, Mono- oder Diniederalkylamino, Halogen, Carboxy, Niederalkoxycarbonyl, Phenyl-, Naphthyl- oder Fluorenylniederalkoxycarbonyl, Niederalkanoyl, Niederalkylsulfonyl, Phosphono, Hydroxy-niederalkoxy-phosphoryl oder Diniederalkoxyphosphoryl, Carbamoyl, Mono- oder Diniederalkylaminocarbamoyl, Sulfamoyl, Mono- oder Diniederalkylsulfamoyl, Nitro und/oder Cyano ein- oder bis zu dreifach substituiert ist und an Niederalkyl gebunden ist,
- Bicycloalkylniederalkyl, worin Bicylcoalkyl 5 - 10, Kohlenstoffatome enthält,
- Tricycloalkylniederalkyl, worin Tricycloalkyl 8 - 10 Kohlenstoffatome enthält,
- Arylniederalkyl mit Aryl ausgewählt aus Phenyl, Indenyl, Indanyl, Naphthyl, Anthryl, Phenanthryl, Acenaphthyl und Fluorenyl, das unsubstituiert oder durch Niederalkyl, Halogenniederalkyl, Phenyl, 1 - oder 2-Naphthyl, Hydroxy, Niederalkoxy, Carbamoylniederalkoxy, N-Niederalkylcarbamoylniederalkoxy, N,N-Diniederalkylcarbamo-ylniederalkoxy, Amino, Mono- oder Diniederalkylamino, Niederalkanoylamino, Halogen, Carboxy, Niederalkoxycarbonyl, Benzyl-, Naphthyl- oder Fluorenylniederalkoxycarbonyl, Niederalkanoyl, Sulfo, Niederalkylsulfonyl, Phosphono, Hydroxy-niederalkoxy-phosphoryl, Diniederalkoxyphosphoryl, Carbamoyl, Mono- oder Diniederalkylcarbamoyl, Sulfamoyl, Mono- oder Diniederalkylsulfamoyl, Nitro und/oder Cyano ein- oder bis zu dreifach substituiert sein kann, wobei Phenyl bis zu dreimal vorliegen kann, und mit Niederalkyl, welches unsubstituiert oder substituiert ist durch Niederalkyl, Heterocyclyl ausgewählt aus Pyrrolyl, Furyl, Thienyl, Imidazolyl, Pyrazolyl, Oxazolyl, Thiazolyl, Pyridyl, Pyrazinyl, Pyrimidinyl, Indolyl, Chinolyl, Isochinolyl, Chinoxalinyl, β-Carbolinyl und einem benzanellierten, cyclopenta-, cyclohexa- oder cyclohepta-anellierten Derivat dieser Reste, die auch ganz oder teilweise gesättigt vorliegen können, Hydroxy, Niederalkoxy, Niederalkanoyloxy, Acetoacetoxy, Amino- oder Benzyloxycarbonylamino-niederalkanoyloxy, Arylniederalkanoyloxy, worin Aryl 6 - 10 Kohlenstoffatome hat, Niederalkoxycarbonyloxy, Mono- oder Diniederalkyl-aminocarbonyloxy, Aryloxycarbonyloxy, worin Aryl 6 - 10 Kohlenstoffatome hat, Arylniederalkoxycarbonyloxy mit Aryl von 6 - 12 Kohlenstoffatomen, Sulfonyloxy, Niederalkylsulfonyloxy, Phenylsulfonyloxy, 2-oder 4-Toluolsulfonyloxy, 1- oder 2- Naphthylsulfonyloxy, Amino, Mono- oder Diniederalkylamino, N-Niederalkoxy-N-niederalkylamino, Mono- oder Di-(Phenyl- oder Naphthylniederalkyl)amino, Niederalkanoylamino, Carboxy, verestertes Carboxy ausgewählt aus Niederalkoxycarbonyl, Aryloxycarbonyl, worin Aryl 6 - 10 Kohlenstoffatome hat, Arylniederalkoxycarbonyl, worin Aryl 6 - 12 Kohlenstoffatome hat, Niederalkanoyl, Niederalkylsulfonyl, Hydroxy-niederalkoxyphosphoryl und Diniederalkoxyphosphoryl, Carbamoyl, durch einen oder zwei aus Niederalkyl, aus Carboxyniederalkyl, aus Niederalkoxycarbonyl-niederalkyl, aus Diniederalkylaminoniederalkyl, aus Aminocarboxy-niederalkyl, aus Hydroxyniederalkyl und aus Diniederalkoxyniederalkyl ausgewählte Reste oder einen aus Ethylen, Trimethylen, Tetramethylen und Pentamethylen ausgewählten Rest, worin ein Kohlenstoffatom durch Stickstoff, Sauerstoff, Schwefel oder mit Sauerstoff ein- oder zweifach substituierten Schwefel ersetzt sein kann und das ungesättigt sein kann, substituiertes Carbamoyl, ferner Sulfamoyl, Phosphono, Benzfuranyl, Oxo (welches nicht an das Kohlenstoffatom gebunden ist, das mit dem den Rest R₇ bindenden Stickstoffatom verknüpft ist) und/oder Cyano sein kann und unverzweigt oder verzweigt ist,
- Heterocyclylniederalkyl, worin Heterocyclyl vorzugsweise aus Pyrrolyl, Furyl, Thienyl, Imidazolyl, Pyrazolyl, Oxazolyl, Thiazolyl, Pyridyl, Pyrazinyl, Pyrimidinyl, Indolyl, Chinolyl, Isochinolyl, Chinoxalinyl, β-Carbolinyl und einem benzanellierten, cyclopenta-, cyclohexa- oder cyclohepta-anellierten Derivat dieser Reste, die auch ganz oder teilweise gesättigt sein können, ausgewählt ist, z.B. 4-Pyrrolidinylmethyl, 1-Imidazolylmethyl, 2-Pyridylmethyl, 3-Pyridylmethyl, Chinolin-2-ylmethyl oder Indol-2-ylmethyl,
- Hydroxyniederalkyl,
- Niederalkoxyniederalkyl,
- Phenoxyniederalkyl oder Nitrophenoxyniederalkyl,
- Naphthoxyniederalkyl,
- Niederalkanoyloxyniederalkyl,
- Acetoacetoxyniederalkyl,
- Arylmercaptoniederalkyl, worin Aryl 6 - 10 Kohlenstoffatome hat,
- Aminoniederalkyl,
- Mono- oder Diniederalkylaminoniederalkyl,
- Phenyl- oder Naphthylaminoniederalkyl,
- Niederalkanoylamino-niederalkyl,
- durch Niederalkyl am Stickstoff substituiertes Piperazinylcarbonylniederalkyl,
- Niederalkoxycarbonylamino-niederalkyl,
- Phenylniederalkoxycarbonylamino-niederalkyl,
- Aminocarbonylamino-niederalkyl,
- N-Phenylniederalkyl-N-niederalkylaminocarbonylamino-niederalkyl,
- Halogenniederalkyl,
- Carboxyniederalkyl,
- Niederalkoxycarbonylniederalkyl,
- 2-Halogenniederalkoxycarbonyl-niederalkyl,
- Phenyl- oder Naphthylniederalkyloxycarbonyl-niederalkyl,
- Heterocyclylniederalkoxycarbonyl-niederalkyl, worin Heterocyclyl ausgewählt ist aus Pyrrolyl, Furyl, Thienyl, Imidazolyl, Pyrazolyl, Oxazolyl, Thiazolyl, Pyridyl, Pyrazinyl, Pyrimidinyl, Indolyl, Chinolyl, Isochinolyl, Chinoxalinyl, β-Carbolinyl und einem benzanellierten, cyclopenta-, cyclohexa- oder cyclohepta-anellierten Derivat dieser Reste, wobei die genannten Reste auch ganz oder teilweise gesättigt vorliegen können,
- Niederalkylsulfonylniederalkyl,
- Arylsulfonylniederalkyl, worin Aryl 6 - 10 Kohlenstoffatome hat,
- Carbamoylniederalkyl,
- Niederalkylcarbamoylniederalkyl,
- Diniederalkylcarbamoylniederalkyl,
- Hydroxyniederalkylcarbamoyl- oder Di-(hydroxyniederalkyl)carbamoyl-niederalkyl,
- N-Niederalkoxyniederalkoxyniederalkyl-carbamoyl-niederalkyl,
- Carboxyniederalkylcarbamoyl- oder Di-(carboxyniederalkyl)carbamoyl-niederalkyl,
- durch einen aus Ethylen, Trimethylen, Tetramethylen und Pentamethylen ausgewählten Rest, worin ein Kohlenstoffatom durch Stickstoff, Sauerstoff, Schwefel oder mit Sauerstoff ein- oder zweifach substituierten Schwefel ersetzt sein kann, am Stickstoff substituiertes Carbamoylniederalkyl, wobei der so gebildete Rest auch ganz oder teilweise ungesättigt sein kann,
- N-Niederalkyl-N-heterocyclylniederalkylcarbamoylniederalkyl, worin Heterocyclyl aus Pyrrolyl, Furyl, Thienyl, Imidazolyl, Pyrazolyl, Oxazolyl, Thiazolyl, Pyridyl, Pyrazinyl, Pyrimidinyl, Indolyl, Chinolyl, Isochinolyl, Chinoxalinyl, β-Carbolinyl und einem benz-anellierten, cyclopenta-, cyclohexa- oder cyclohepta-anellierten Derivat dieser Reste, die auch ganz oder teilweise gesättigt sein können, ausgewählt ist,
- Sulfamoylniederalkyl,
- N-(Phenyl- oder Naphthylniederalkyl)sulfamoylniederalkyl,
- am Stickstoffatom von einem aus Ethylen, Trimethylen, Tetramethylen und Penta-methylen ausgewählten Rest, worin ein Kohlenstoffatom durch Stickstoff, mit Niederalkyl substituierten Stickstoff, Sauerstoff, Schwefel oder mit Sauerstoff ein-oder zweifach substituierten Schwefel ersetzt sein kann, substituiertes Sulfamoylniederalkyl, wobei der so gebildete Rest auch ganz oder teilweise ungesättigt sein kann,
- Oxoniederalkyl (wobei Oxo nicht an das Kohlenstoffatom gebunden ist, welches mit dem R₇ bindenden Stickstoffatom verknüpft ist),
- Cyanoniederalkyl,
- Hydroxy-carboxy-niederalkyl,
- α-Naphthoxy-carboxy-niederalkyl,
- Hydroxy-niederalkoxycarbonyl-niederalkyl,
- α-Naphthoxy-niederalkoxycarbonylniederalkyl,
- Niederalkylcarbonyl-halogen-niederalkyl,
- α-Naphthoxyethoxycarbonyl-niederalkyl,
- α-Naphthoxy-benzyloxycarbonyl-niederalkyl,
- verestertes Hydroxy-niederalkoxycarbonyl-niederalkyl, wobei die Hydroxygruppe durch Niederalkanoyl, Cycloalkylniederalkanoyl, worin Cycloalkyl 3 - 7 Kohlenstoffatome hat, Bicycloalkylniederalkanoyl, worin Bicylcoalkyl 5 - 10 Kohlenstoffatome enthält, Tricycloalkylniederalkanoyl, worin Tricycloalkyl 8 - 10 Kohlenstoffatome enthält, Arylniederalkanoyl, worin Aryl 6 - 14 Kohlenstoffatome hat und unsubstituiert oder durch Niederalkyl, Hydroxy, Niederalkoxy, Carbamoylniederalkoxy, N-Niederalkylcarbamoyl- niederalkoxy oder N,N-Diniederalkylcarbamoylniederalkoxy, Amino, Mono- oder Diniederalkylamino, Halogen, Carboxy, Niederalkoxycarbonyl, Phenyl-, Naphthyl- oder Fluorenylniederalkoxycarbonyl, Niederalkanoyl, Niederalkylsulfonyl, Phosphono, Hydroxyniederalkoxy-phosphoryl, Diniederalkoxyphosphoryl, Carbamoyl, Sulfamoyl, Nitro und/oder Cyano ein- oder bis zu dreifach substituiert sein kann, Niederalkoxy-carbonyl, 2-Halogenniederalkoxycarbonyl, oder Phenyl- oder Fluorenyl-niederalkoxy-carbonyl, verestert ist,
- Dihydroxy-carboxy-niederalkyl,
- Dihydroxy-niederalkoxycarbonyl-niederalkyl,
- durch Niederalkanoyl, Niederalkoxycarbonyl, Phenyl- oder Fluorenyl-niederalkoxycar-bonyl,- Niederalkylsulfonyl oder Toluolsulfonyl verestertes Dihydroxy-niederalkoxycar-bonyl-niederalkyl,
- α-Naphthoxy-diniederalkylamino-niederalkyl,
- α-Naphthoxy-carbamoyl-niederalkyl,
- α-Naphthoxy-oxo-niederalkyl (wobei Oxo nicht an das Kohlenstoffatom gebunden ist,
welches mit dem R₇ bindenden Stickstoffatom verknüpft ist), oder - α-Naphthoxy-cyanoniederalkyl bedeutet, sowie die Salze der genannten Verbindungen, sofern salzbildende Gruppen vorliegen.

Besonders bevorzugt sind die Verbindungen der Formel I, worin R₁ und R₉ unabhängig voneinander Wasserstoff; Niederalkanoyl, wie Formyl, Acetyl, Propionyl, Butyryl oder Pivaloyl, insbesondere Acetyl; Arylniederalkanoyl, worin Aryl Phenyl, Indenyl, Indanyl, Naphthyl, Anthryl, Phenanthryl, Acenaphthyl oder Fluorenyl bedeutet und unsubstituiert oder insbesondere durch Niederalkyl, z. B. Methyl, Ethyl oder Propyl, Halogennieder-alkyl, z. B. Trifluormethyl, Phenyl, 1 - oder 2-Naphthyl, Hydroxy, Niederalkoxy, z. B. Methoxy, Carbamoylniederalkoxy, N-Niederalkylcarbamoylniederalkoxy, N,N-Dinieder-alkylcarbamoylniederalkoxy, Amino, Mono- oder Diniederalkylamino, Niederalkanoyl-amino, z. B. Pivaloylamino, Halogen, z. B. Fluor, Chlor oder Brom, Carboxy, Niederalkoxycarbonyl, wie tert-Butoxycarbonyl, Phenyl-, Naphthyl- oder Fluorenylnieder-alkoxycarbonyl, wie Benzyloxycarbonyl, Niederalkanoyl, Sulfo, Niederalkylsulfonyl, z. B. Methylsulfonyl, Phosphono, Hydroxyniederalkoxy-phosphoryl, Diniederalkoxy-phosphoryl, Carbamoyl, Mono- oder Diniederalkylcarbamoyl, Sulfamoyl, Mono- oder Diniederalkylaminosulfonyl, Nitro und/oder Cyano ein- oder bis zu dreifach substituiert sein kann, wobei Phenyl bis zu dreimal vorliegen kann, z. B. in Diphenyl-, Dibenzyl- oder Triphenylniederalkanoyl, wie Diphenyl-, Dibenzyl- oder Triphenylacetyl, und worin Niederalkanoyl unsubstituiert oder substituiert ist durch Carbamoyl oder durch einen oder zwei aus Niederalkyl, Carboxyniederalkyl, Niederalkoxycarbonyl-niederalkyl, Diniederalkylamino-niederalkyl, Amino-carboxy-niederalkyl, Hydroxyniederalkyl und Diniederalkoxyniederalkyl ausgewählte Reste am Stickstoff substituiertes Carbamoyl ist, beispielsweise durch Carbamoyl, durch einen oder zwei aus Methyl, Ethyl, Propyl, Isopropyl, n-Butyl, Isobutyl, sec-Butyl, tert-Butyl, n-Pentyl, Isopentyl, Neopentyl, tert-Pentyl, n-Hexyl, Isohexyl und n-Heptyl ausgewählte Reste substituiertes Carbamoyl, z. B. in N-Methylcarbamoyl, N-n-Butylcarbamoyl oder N,N-Dimethylcarbamoyl, durch Carboxymethylcarbamoyl (Glycinylcarbonyl), durch tert-Butoxycarbonylmethylcarbamoyl, durch 2-Dimethylaminoethyl, durch 5-Amino-5-carboxypentyl, durch Hydroxymethyl, durch Hydroxyethyl, oder durch 2-(2,2-Dimethoxyethyl)carbamoyl, beispielsweise 4-Chlor-, 4-Methoxy- oder 4-Nitrobenzoyl, Naphthylcarbonyl, wie α-oder β-Naphthylcarbonyl, Indenylcarbonyl, wie 1-, 2- oder 3-Indenylcarbonyl, Indanylcarbonyl, wie 1- oder 2-Indanylcarbonyl, Phenanthrenylcarbonyl, wie 9-Phenathrenylcarbonyl, Phenylniederalkanoyl, wie Phenylacetyl oder 3-Phenylpropionyl, Phenylniederalkanoyl, worin der Niederalkanoylrest durch Carbamoyl substituiert ist, wie 2(R,S)-Carbamoyl-3-phenylpropionyl, α-Naphthylacetyl, β-Naphthylacetyl, Niederalkylphenyl-acetyl, wie 4-Methylphenylacetyl, Niederalkoxyphenylacetyl, wie 4-Methoxyphenylacetyl, 3-(p-Hydroxyphenyl)-propionyl, Diphenylacetyl, Di-(4-methoxyphenyl)-acetyl, Triphenylacetyl, 2,2-Dibenzylacetyl, 3-α- oder 3-β-Naphthylpropionyl, 3-Phenyl- oder 3-α-Naphthyl-2-carbamoylpropionyl, 3-Phenyl- oder 3-α-Naphthyl-2-tert-butylcarbamoyl-propionyl, 3-Phenyl- oder 3-α-Naphthyl-2-(2-dimethylaminoethyl)carbamoylpropionyl, 3-α-Naphthyl-2-(carboxy- oder tert-butoxycarbonyl)methylcarbamoylpropionyl, 3-Phenyl- oder 3-α-Naphthyl-2-(3-hydroxy-2-propyl)carbamoyl-propionyl, 3-Phenyl- oder 3-α-Naphthyl-2-(2,2-dimethoxyethyl)-carbamoylpropionyl, 3-Phenyl- oder 3-α-Naphthyl-2(5-amino-5-carboxypentyl)-carbamoylpropionyl, in erster Linie Phenylniederalkanyol, wie Phenylacetyl, oder Phenyl-niederalkanoyl, worin der Niederalkanoylrest durch Carbamoyl substituiert ist, wie 2(R,S)-Carbamoyl-3-phenylpropionyl; Heterocyclylniederalkanoyl, worin Niederalkanoyl unsubstituiert ist und worin Heterocyclyl aus Thienyl, Furyl, Pyranyl, Pyrrolyl, Imidazolyl, Pyrazolyl, Oxazolyl, Isoxazolyl, Thiazolyl, Furazanyl, Pyridyl, Pyrazinyl, Pyrimidinyl, Pyridazinyl, Azepinyl, Indolyl, Benzimidazolyl, 1H-Indazolyl, Chinolyl, Isochinolyl, Chinoxalinyl, Chinazolinyl, Cinnolyl, Purinyl, Pteridinyl, Naphthyridinyl, 4H-Chinolizinyl, 3,1Benzfuranyl, 4,1-Benzoxazinyl, 4,1-Benzthiazinyl, Cyclohexa[b]pyrrolyl, Cyclohepta[b]pyrrolyl, Cyclohexa[d]pyrazolyl, Cyclohexa[b]pyridyl, Cyclohexa[b]pyrazinyl, Cyclohexa[b]pyrimidinyl, Cyclohexa[b]-1,4-oxazinyl, Cyclohexa[b]-1,4-thiazinyl, Pyrrolidinyl, Pyrrolinyl, Imidazolidyl, 2-Imidazolinyl, 2,3-Dihydropyridyl, Piperidyl, Piperazinyl, 2,3,5,6-Tetrahydropyrazinyl, Morpholinyl, Thiomorpholinyl, S,S-Dioxo-thiomorpholinyl, Indolinyl, Isoindolinyl, 4,5,6,7-Tetrahydroindolyl, 1,2,3,4-Tetrahydrochinolyl, 1,2,3,4-Tetrahydroisochinolyl, Chromanyl, Thiochromanyl, 1,2,3,4-Tetrahydro-3,1 -benzdiazinyl, 3,4-Dihydro-3H-4,1 - benzoxazinyl und 3,4-Dihydro-3H-4,1 -benzthiazinyl ausgewählt ist, wobei die genannten Heterocyclyl-Reste unsubstituiert oder durch Niederalkyl, z. B. Methyl, Phenyl, 1- oder 2-Naphthyl, Phenylniederalkyl, z. B. Benzyl, Hydroxyniederalkyl, z. B. Hydroxymethyl oder 2-Hydroxyethyl, Niederalkoxyniederalkyl, z. B. Methoxymethyl oder 2-Methoxy-ethyl, Phenoxy-oder Naphthoxyniederalkyl, z. B. 2-Phenoxyethyl, 1 - oder 2-Naph-thoxymethyl, Phenylniederalkoxy-oder Naphthylniederalkoxy-niederalkyl, z. B. Benzyloxyniederalkyl, Niederalkanoyloxy-niederalkyl, z. B. Acetyloxymethyl, Phenyl- oder Napthyl-niederalkanoyloxy-niederalkyl, z. B. Benzoyloxy-, Phenylacetyloxy-oder 1 - oder 2-Naphthoyloxy-methyl, -2-ethyl oder -2-(2,2-dimethylethyl), Niederalkoxycar-bonyloxy-niederalkyl, z. B. tert-Butyloxyniederalkyl, Phenyl-, Napthyl- oder Fluorenyl-niederalkoxycarbonyloxy-niederalkyl, z. B. 2-Benzyloxycarbonyloxyethyl oder 9-Fluorenylmethoxycarbonyloxyethyl, Aminoniederalkyl, z. B. Aminomethyl, 2-Aminoethyl oder 2-Aminopropyl, Carboxyniederalkyl, z. B. Carboxymethyl oder 2-Carboxyethyl, Hydroxy, Niederalkoxy, z. B. Methoxy oder Ethoxy, Phenyl- oder Naphthylniederalkoxy, z. B. Benzyloxy oder 1- oder 2-Naphthyloxy, Amino, Niederalkylamino, z. B. Methyl-, Ethyl- oder tert-Butylamino, Diniederalkylamino, z. B. Dimethyl- oder Diethylamino, Carboxy, Niederalkoxycarbonyl, z. B. Methoxy-, Isopropoxy-, sec-Butoxy- oder tert-Butoxy-carbonyl, Phenyl- oder Napthyl-niederalkoxycarbonyl, z. B. Benzyloxycarbonyl, Halogen, z. B. Fluor, Chlor, Brom oder Jod, insbesondere Chlor oder Brom, Nieder-alkanoyl, z. B. Acetyl oder Pivaloyl, Niederalkylsulfonyl, z. B. Methyl- oder Ethylsulfonyl, Phosphono, Hydroxy-niederalkoxy-phosphoryl oder Dialkoxy-phosphoryl, z. B. Dimethoxy- oder Diethoxyphosphoryl, Carbamoyl, Mono- oder Diniederalkylcarbamoyl, z. B. N-Methylcarbamoyl, N-n-Butylcarbamoyl oder N,N-Dimethylcarbamoyl, Hydroxy- oder Carboxyniederalkylcarbamoyl, z. B. Hydroxy- oder Carboxymethylcarbamoyl oder Hydroxy- oder Carboxyethylcarbamoyl, Sulfamoyl, durch Nitro, Oxo und/oder durch Cyano substituiert sind, wobei Heterocyclylniederalkanoyl insbesondere ausgewählt ist aus gegebenenfalls durch Niederalkyl oder Phenyl substituiertem Pyrrolylcarbonyl, z. B. 2- oder 3-Pyrrolylcarbonyl, 4- oder 5-Methylpyrrolylcarbonyl oder 4- oder 5-Phenylpyrrolyl-2-carbonyl, Thienylcarbonyl, wie 2-Thienylcarbonyl, Furylcarbonyl, wie 2-Furylcarbonyl, Pyridylcarbonyl, wie 2-, 3- oder 4-Pyridylcarbonyl, Pyrimidin-1 -yl-carbonyl, gegebenenfalls durch Niederalkyl, wie Methyl, Phenylniederalkyl, wie Benzyl, Niederalkoxy, wie Methoxy, Phenylniederalkoxy, wie Benzyloxy oder Halogen, wie Chlor, substituiertem Indolylcarbonyl, wie 2-, 3- oder 5-Indolylcarbonyl, 1-Methyl-, 5-Methyl-, 5-Methoxy-, 5-Benzyloxy-, 5-Chlor- oder 4,5-Dimethylindolyl-2-carbonyl, 1-Benzyl-indolyl-2- oder -3-carbonyl, 4,5,6,7-Tetrahydroindolyl-2-carbonyl, gegebenenfalls durch Hydroxy substituiertem Chinolylcarbonyl, wie 2-, 3- oder 4-Chinolylcarbonyl oder 4-Hydroxychinolyl-2-carbonyl, gegebenenfalls durch Hydroxy substituiertem Isochinolylcarbonyl, wie 1-, 3- oder 4-Isochinolylcarbonyl oder 1-Oxo-1,2-dihydroisochinolyl-3-carbonyl, 2-Chinoxalinylcarbonyl, 2-(3,1-Benzfuranyl)carbonyl, Cyclohepta[b]pyrrolyl-5-carbonyl, 3-Chromanylcarbonyl, 3-Thiochromanylcarbonyl, Pyrrolidinyl-3-carbonyl, Hydroxypyrrolidinylcarbonyl, wie 3- oder 4-Hydroxypyrrolidinyl-2-carbonyl, Oxopyrrolidinylcarbonyl, wie 5-Oxopyrrolidinyl-2-carbonyl, Piperidylcarbonyl, wie Piperidinocarbonyl oder 2-, 3- oder 4-Piperidylcarbonyl, Pyrazinylcarbonyl, wie Pyrazin- 1-ylcarbonyl, Piperazinylcarbonyl, wie Piperazin-1-yl-carbonyl, Morpholinylcarbonyl, wie Morpholinocarbonyl, Thiomorpholinylcarbonyl, wie Thiomorpholinocarbonyl, S,S-Dioxothiomorpholinylcarbonyl, wie S,S-Dioxothiomorpholinocarbonyl, Indolinylcarbonyl, wie 2- oder 3-Indolinylcarbonyl, 1,2,3,4-Tetrahydrochinolylcarbonyl, wie 1,2,3,4-Tetrahydrochinolyl-2-, - 3- oder -4-carbonyl, 1,2,3,4-Tetrahydroisochinolylcarbonyl, wie 1,2,3,4-Tetrahydroisochinolyl-1-, -3- oder 4-carbonyl oder 1-Oxo-1,2,3,4-tetrahydroisochinolyl-3-carbonyl, und Pyridylniederalkanoyl, z. B. Pyridylacetyl, wie 2-, 3- oder 4-Pyridylacetyl, in erster Linie ausgewählt aus Morpholinocarbonyl, Thiomorpholino-carbonyl, Pyridylniederalkanoyl, wie 2-, 3- oder 4-Pyridylacetyl, und Chinolin-2-carbonyl; Halogenniederalkanoyl, welches bis zu drei Halogenatome enthält, in erster Linie α-Halogenacetyl, wie α-Fluor-, α-Chlor-, α-Brom-, α-Iod-, α,α,α-Trifluor- oder α,α,α-Trichloracetyl, oder Halogenpropionyl, wie β-Chlor- oder β-Brompropionyl, z.B. Trifluoracetyl; (N-Heterocyclylniederalkylcarbamoyl)-niederalkanoyl, worin Heterocyclyl aus Pyrrolyl, Furyl, Thienyl, Imidazolyl, Pyrazolyl, Oxazolyl, Thiazolyl, Pyridyl, Pyrazinyl, Pyrimidinyl, Indolyl, Chinolyl, Isochinolyl, Chinoxalinyl, β-Carbolinyl und einem benzanellierten, cyclopenta-, cyclohexa- oder cyclohepta-anellierten Derivat dieser Reste, die auch ganz oder teilweise gesättigt sein können, aus Morpholin und aus Thiomorpholin ausgewählt ist, in erster Linie 2-(N-Morpholinoniederalkyl-carbamoyl)niederalkanoyl, wie 2(R,S)-(N-(2-morpholinoethyl)-carbamoyl)-3-methyl-butyryl, oder 2-(N-(Pyridylniederalkyl)-carbamoyl)-niederalkanoyl, wie 2(R,S)-(N-(2-pyridylmethyl)-carbamoyl)-3-methyl-butyryl; Niederalkoxycarbonyl, in erster Linie Methoxy-, Ethoxy-, Isopropoxy- oder tert-Niederalkoxycarbonyl, z.B. Methoxy-carbonyl oder tert-Butoxycarbonyl; Arylniederalkoxycarbonyl, worin Aryl Phenyl, Biphenylyl, 1- oder 2-Naphthyl, Fluorenyl oder durch Niederalkyl, z. B. Methyl oder tert-Butyl, Hydroxy, Niederalkoxy, z. B. Methoxy, Ethoxy oder tert-Butoxy, Halogen, z. B. Chlor oder Brom, und/oder Nitro mono- oder mehrfach substituiertes Phenyl ist, z. B. Phenylniederalkoxycarbonyl, wie Benzyloxycarbonyl, 4-Methoxybenzyloxycarbonyl, 4-Nitrobenzyloxycarbonyl oder Fluorenylniederalkoxycarbonyl, wie 9-Fluorenylmethoxycarbonyl, in erster Linie Phenylniederalkoxycarbonyl, wie Benzyloxycarbonyl; Heterocyclylniederalkoxycarbonyl, worin Heterocyclyl aus Pyrrolyl, Furyl, Thienyl, Imidazolyl, Pyrazolyl, Oxazolyl, Thiazolyl, Pyridyl, Pyrazinyl, Pyrimidinyl, Indolyl, Chinolyl, Isochinolyl, Chinoxalinyl, β-Carbolinyl und einem benzanellierten, cyclopenta-, cyclohexa- oder cyclohepta-anellierten Derivat dieser Reste, die auch ganz oder teilweise gesättigt vorliegen können, ausgewählt ist, wie 2-Tetrahydrofuryl-methoxycarbonyl oder 2-Morpholino-ethoxycarbonyl, oder 2-, 3- oder 4-Pyridylmethoxycarbonyl, in erster Linie Tetrahydrofuranyl-niederalkyloxycarbonyl, wie 2(R,S)-Tetrahydrofuranylmethoxycarbonyl; Niederalkylsulfonyl, z.B. Methyl- oder Ethylsulfonyl, wie Methylsulfonyl; N-Heterocyclylniederalkyl-N-niederalkyl-carbamoyl, worin Heterocyclyl ausgewählt ist aus Thienyl, Furyl, Pyranyl, Pyrrolyl, Imidazolyl, Pyrazolyl, Oxazolyl, Isoxazolyl, Thiazolyl, Furazanyl, Pyridyl, Pyrazinyl, Pyrimidinyl, Pyridazinyl, Azepinyl, Indolyl, Benzimidazolyl, 1H-Indazolyl, Chinolyl, Isochinolyl, Chinoxalinyl, Chinazolinyl, Cinnolyl, Purinyl, Pteridinyl, Naphthyridinyl, 4H-Chinolizinyl, 3,1-Benzfuranyl, Benz[e]indolyl, 4,1-Benzoxazinyl, 4,1-Benzthiazinyl, Carbazolyl, β-Carbolinyl, Phenazinyl, Phenanthridyl, Acridyl, Phenoxazinyl, Phenothiazinyl, 1-Azaacenaphtenyl, Cyclohexa[b]pyrrolyl, Cyclohepta[b]pyrrolyl, Cyclohexa[d]pyrazolyl, Cyclohexa[b]pyridyl, Cyclohexa[b]pyrazinyl, Cyclohexa[b]pymidinyl, Cyclohexa-[b]-1,4-oxazinyl, Cyclohexa[b]-1,4-thiazinyl, Pyrrolidinyl, Pyrrolinyl, Imidazolidinyl, 2-Imidazolinyl, 2,3-Dihydropyridyl, Piperidyl, Piperazinyl, 2,3,5,6-Tetrahydropyrazinyl, Morpholinyl, Thiomorpholinyl, S,S-Dioxothiomorpholinyl, Indolinyl, Isoindolinyl, 4,5,6,7-Tetrahydroindolyl, 1,2,3,4-Tetrahydrochinolyl, 1,2,3,4-Tetrahydroisochinolyl, Chromanyl, Thiochromanyl, 1,2,3,4-Tetrahydro-3,1-benzdiazinyl, 3,4-Dihydro-3H-4,1-benzoxazinyl, 3,4-Dihydro-3H-4,1-benzthiazinyl, 2,3,4,5-Tetrahydro-1H-5,1 -benzazepinyl und 5,6-Dihydrophenanthridinyl, wobei die genannten Reste unsubstituiert oder durch Niederalkyl, z. B. Methyl, Phenyl, 1- oder 2-Naphthyl, Phenylniederalkyl, z. B. Benzyl, Hydroxyniederalkyl, z. B. Hydroxymethyl oder 2-Hydroxyethyl, Niederalkoxyniederalkyl, z. B. Methoxymethyl oder 2-Methoxyethyl, Phenoxy- oder Naphthoxyniederalkyl, z. B. 2-Phenoxyethyl, 1- oder 2-Naphthoxymethyl, Phenylniederalkoxy- oder Naphthylniederalkoxy-niederalkyl, z. B. Benzyloxyniederalkyl, Niederalkanoyloxy-niederalkyl, z. B. Acetyloxymethyl, Phenyl- oder Napthyl-niederalkanoyloxy-niederalkyl, z. B. Benzoyloxy-, Phenylacetyloxy- oder 1- oder 2-Naphthoyloxy-methyl, -2-ethyl oder -2-(2,2-dimethylethyl), Niederalkoxycarbonyloxy-niederalkyl, z. B. tert-Butyloxyniederalkyl, Phenyl-, Napthyl- oder Fluorenylniederalkoxycarbonyl-oxy-niederalkyl, z. B. 2-Benzyloxycarbonyloxyethyl oder 9-Fluorenylmethoxycarbonyl-oxyethyl, Aminoniederalkyl, z. B. Aminomethyl, 2-Aminoethyl oder 2-Aminopropyl, Carboxyniederalkyl, z. B. Carboxymethyl oder 2-Carboxyethyl, Hydroxy, Niederalkoxy, z. B. Methoxy oder Ethoxy, Phenyl- oder Naphthylniederalkoxy, z. B. Benzyloxy oder 1-oder 2-Naphthylmethoxy, Amino, Niederalkylamino, z. B. Methyl-, Ethyl- oder tert-Butylamino, Diniederalkylamino, z. B. Dimethyl- oder Diethylamino, Carboxy, Niederalkoxycarbonyl, z. B. Methoxy-, Isopropoxy-, sec-Butoxy- oder tert-Butoxy-carbonyl, Phenyl- oder Napthylniederalkoxycarbonyl, z. B. Benzyloxycarbonyl, Halogen, z. B. Fluor, Chlor, Brom oder Jod, insbesondere Chlor oder Brom, Niederalkanoyl, z. B. Acetyl oder Pivaloyl, Niederalkylsulfonyl, z. B. Methyl- oder Ethylsulfonyl, Phosphono, Hydroxy-niederalkoxyphosphoryl oder Dialkoxy-phosphoryl, z. B. Dimethoxy- oder Diethoxyphosphoryl, Carbamoyl, Mono- oder Diniederalkylcarbamoyl, z. B. N-Methylcarbamoyl, N-n-Butylcarbamoyl oder N,N-Dimethylcarbamoyl, Hydroxy- oder Carboxyniederalkylcarbamoyl, z. B. Hydroxy- oder Carboxymethyl-carbamoyl oder Hydroxy- oder Carboxyethylcarbamoyl, Sulfamoyl, durch Nitro, Oxo und/oder durch Cyano substituiert sind, und insbesondere Pyridyl, wie 2-, 3- oder 4-Pyridyl, bedeutet, in erster Linie N-Pydylniederalkyl-N-niederalkyl-carbamoyl, wie N-(2-, 3- oder 4-Pyridylmethyl)-N-methyl-carbamoyl; oder einen Acylrest einer Aminosäure, deren Aminofunktion frei oder durch einen der übrigen bisher für R₁ und R₉ genannten Reste acyliert ist, bedeuten, wobei die Aminosäuren aus Glycin (H-Gly-OH), Alanin (H-Ala-OH), Valin (H-Val-OH), Norvalin (α-Aminovaleriansäure), Leucin, (H-Leu-OH), Isoleucin (H-Ile-OH), Norleucin (α-Aminohexansäure, H-Nle-OH), Serin (H-Ser-OH), Homoserin (α-Amino-γ-hydroxybuttersäure), Threonin (H-Thr-OH), Methionin (H-Met-OH), Cystein (H-Cys-OH), Prolin (H-Pro-OH), trans-3- und trans-4-Hydroxyprolin, Phenylalanin (H-Phe-OH), Tyrosin (H-Tyr-OH), 4-Aminophenylalanin, 4-Chlorphenylalanin, 4-Carboxyphenylalanin, β-Phenylserin (β-Hydroxyphenylalanin), Phenylglycin, α-Naphthylalsnin (H-Nal-OH), Cyclohexylalanin (H-Cha-OH), Cyclohexylglycin, Tryptophan (H-Trp-OH), Indolin-2-carbonsäure, 1,2,3,4-Tetrahydroisochinolin-3-carbonsäure, Asparaginsäure (H-Asp-OH), Asparagin (H-Asn-OH), Aminomalonsäure, Aminomalonsäure-monoamid, Glutaminsäure (H-Glu-OH), Glutamin (H-Gln-OH), Histidin (H-His-OH), Arginin (H-Arg-OH), Lysin (H-Lys-OH), δ-Hydroxylysin, Ornithin (α,δ-Diaminovaleriansäure), 3-Aminopropansäure, α,γ-Diaminobuttersäure und α,β-Diaminopropionsäure, besonders bevorzugt der Rest einer Aminosäure ausgewählt aus Valin, Alanin, Leucin, Isoleucin, Glycin, Glutaminsäure und Asparagin, wobei (ausser Glycin) jede der genannten Aminosäuren in der D-, L- oder (D,L)- vorzugsweise (ausser Val, das auch in der (D)- oder (D,L)-Form vorliegen kann) in der L-Form vorliegen kann, die α-Aminogruppe unsubstituiert oder durch einen der oben für R₁ und R₉ genannten Reste N-acyliert ist, vor allem durch Niederalkanoyl, Phenylniederalkanyol, wie Phenylacetyl, Phenyl-niederalkanoyl, worin der Niederslkanoylrest durch Carbamoyl substituiert ist, wie 2(R,S)-Carbamoyl-3-phenylpropionyl, Morpholinocarbonyl, Thiomorpholino-carbonyl, Pyridylniederalkanoyl, wie 2-oder 3-Pyridylacetyl, Chinolinyl-niederalkanoyl, wie Chinolin-2-carbonyl, Halogenniederalkanoyl, welches bis zu drei Halogenatome enthält, z. B. α-Halogenacetyl, wie α-Fluor-, α-Chlor-, α-Brom-, α-Iod-, α,α,α-Trifluor- oder α,α,α-Trichloracetyl, oder Halogenpropionyl, wie β-Chlor- oder β-Brompropionyl, insbesondere Trifluoracetyl, 2-(N-Morpholinoniederalkyl-carbamoyl)-niederalkanoyl, wie 2(R,S)-(N-(2-morpholinoethyl)-carbamoyl-3-methyl-butyryl, 2-(N-(Pyridylniederalkyl)-carbamoyl)-niederalkanoyl, wie 2(R,S)-(N-(2-pyridylmethyl)carbamoyl)-niederalkanoyl, Niederalkoxycarbonyl, Phenylniederalkoxycarbonyl, Tetrahydrofuranyl-niederalkyloxycarbonyl, wie 2(R,S)-Tetrahydrofuranylmethoxycarbonyl, Niederalkylsulfonyl oder N-Pyridylniederalkyl-N-niederalkyl-carbamoyl, wie N-(2-, 3- oder 4-Pyridylmethyl)-N-methyl-carbamoyl, substituiert ist, wobei N-Morpholinocarbonylglycin, N-(N-(2-, 3- oder 4-pyridyl)methyl-N-methylaminocarbonyl)-glycin, Valin, N-(Trifluoracetyl)-valin, N-Phenylacetyl-valin, N-Acetyl-valin, N-(2-Carbamoyl-3-phenyl-propionyl)-valin, N-(2(R,S)-Carbamoyl-3-phenyl-propionyl)-valin, N-(2- oder 3-Pyridylacetyl)-valin, N-2-Tetrahydrofurylmethoxycarbonyl-valin,N-(Chinolin-2-carbonyl)-valin, N-Methoxycarbonyl-valin, N-tert-Butoxycarbonyl-valin, N-Benzyloxycarbonyl-valin, N-(Morpholinocarbonyl)-valin, N-(Thiomorpholinocarbonyl)valin, N-(S,S-Dioxothiomorpholinocarbonyl)-valin, N-(N-2-Pyridylmethyl-N-methylaminocarbonyl)-valin, N-Methylsulfonyl-valin, Morpholinosulfonyl-valin, N-Acetyl-isoleucin, N-Propionylisoleucin, N-(Benzyloxycarbonyl)-isoleucin, N-Benzyloxycarbonyl-glutaminsäure, Asparagin, N-Benzyloxycarbonyl-asparagin oder Chinolin-2-carbonyl-asparagin am stärksten bevorzugt sind, worin die Aminosäurereste jeweils vorzugsweise in der (L)-oder (D,L)-Form, im Falle von Valin auch in der (D)-Form, vorliegen; mit der Massgabe, dass höchstens einer der Reste R₁ oder R₉ Wasserstoff bedeutet,
R₂, R₄, R₆ und R₈ Wasserstoff bedeuten,
R₃ Niederalkyl, wie Isobutyl oder n-Butyl; Cycloalkylniederalkyl, worin Cycloalkyl 3 bis 7 Kohlenstoffatome hat und unsubstituiert oder durch Niederalkyl, wie Isopropyl, Halogenniederalkyl, wie Trifluormethyl, Hydroxy, Niederalkoxy, Carbamoylniederalkoxy, N-Niederalkylcarbamoylniederalkoxy oder N,N-Diniederalkylcarbamoylniederalkoxy, Amino, Mono- oder Diniederalkylamino, Halogen, wie Fluor, Chlor oder Brom, Carboxy, Niederalkoxycarbonyl, Phenyl-, Naphthyl- oder Fluorenylniederalkoxycarbonyl, Niederalkanoyl, Niederalkylsulfonyl, z. B. Methylsulfonyl, Phosphono, Hydroxy-niederalkoxy-phosphoryl oder Diniederalkoxyphosphoryl, Carbamoyl, Mono-oder Diniederalkylaminocarbamoyl, Sulfamoyl, Mono- oder Diniederalkylsulfamoyl, Nitro und/oder Cyano ein- oder bis zu dreifach substituiert ist und an Niederalkyl, insbesondere Methyl, Ethyl, n-Propyl, Isopropyl, N-Butyl, Isobutyl, tert-Butyl, vorzugsweise endständig, gebunden ist, wie Cyclobutyl-, Cyclopentyl-, Cyclohexyl-oder Cycloheptyl-niederalkyl, wie -methyl oder -ethyl, in erster Linie Cyclohexylniederalkyl, vor allem Cyclohexylmethyl; oder Arylniederalkyl bedeutet, worin Aryl Phenyl, Indenyl, Indanyl, Naphthyl, Anthryl, Phenanthryl, Acenaphthyl oder Fluorenyl bedeutet und unsubstituiert oder substituiert ist durch Niederalkyl, z. B. Methyl, Ethyl oder Isopropyl, Halogenniederalkyl, wie Trifluormethyl, Phenyl, 1- oder 2-Naphthyl, Hydroxy, Niederalkoxy, z. B. Methoxy, Carbamoylniederalkoxy, N-Niederalkylcarbamoylniederalkoxy oder N,N-Diniederalkylcarbamoylniederalkoxy, Amino, Mono- oder Diniederalkylamino, Niederalkanoylamino, z. B. Pivaloylamino, Halogen, z. B. Fluor oder Chlor, Carboxy, Niederalkoxycarbonyl, Benzyl-, Naphthyl- oder Fluorenylniederalkoxycarbonyl, Niederalkanoyl, Sulfo, Niederalkylsulfonyl, Phosphono, Hydroxy-niederalkoxy-phosphoryl oder Diniederalkoxyphosphoryl, Carbamoyl, Mono-oder Diniederalkylcarbamoyl, Sulfamoyl, Mono- oder Diniederalkylsulfamoyl, Nitro und/oder Cyano ein- oder bis zu dreifach substituiert sein kann, wobei Phenyl bis zu dreimal vorliegen kann, wie in Diphenyl-, Dibenzyl- oder Triphenylniederalkyl, z. B. Diphenyl-, Dibenzyl- oder Triphenyl-2-ethyl, in erster Linie Phenylniederalkyl, welches unsubstituiert oder durch die genannten Substituenten substituiert ist, vor allem Benzyl, 4-Fluor- oder 4-Cyano-benzyl,
R₅ Hydroxy bedeutet, und
R₇ unsubstituiertes Niederalkyl bedeutet, vor allem Isobutyl oder n-Butyl; oder Cycloalkylniederalkyl, wie zuletzt für Cycloalkylniederalkyl R₃ beschrieben, in erster Linie Cyclohexylniederalkyl, vor allem Cyclohexylmethyl; oder Arylniederalkyl, wie zuletzt für Arylniederalkyl R₃ beschrieben, in erster Linie Phenylniederalkyl, welches unsubstituiert oder durch die genannten Substituenten substituiert ist, vor allem Benzyl, 4-Fluor- oder 4-Cyano-benzyl, bedeutet,
oder ein Salz davon, sofern wenigstens eine salzbildende Gruppe vorliegt.

Besonders bevorzugt sind auch die Verbindungen der Formel I, worin R₁ Niederalkoxycarbonyl, Phenylniederalkoxycarbonyl, das über die Carboxygruppe gebun-dene monovalente Radikal einer aliphatischen Aminosäure ausgewählt aus Valin, Alanin, Leucin und Isoleucin oder das über die Carboxygruppe gebundene Radikal einer am Ami-nostickstoff durch einen der Reste Phenylniederalkanoyl, Morpholinylniederalkanoyl, Thiomorpholinylniederalkanoyl, S,S-Dioxothiomorpholinylniederalkanoyl, Pyridylnieder-alkanoyl, Niederalkoxycarbonyl oder Phenylniederalkoxycarbonyl acylierten, wie oben definierten aliphatischen Aminosäure, wobei alle genannten Aminosäuren in der D-, D,L- oder L-Form vorliegen, bevorzugt in der L-Form, bedeutet, R₂ Wasserstoff, R₃ Phenylnie-deralkyl, R₄ Wasserstoff, R₅ Hydroxy, R₆ Wasserstoff, R₇ Niederalkyl, Cyclohexylnieder-alkyl oder Phenylniederalkyl, R₈ Wasserstoff und R₉ einen der für R₁ genannten Reste be-deuten und die die Reste R₃ und R₅ tragenden asymmetrischen Kohlenstoffatome in der S-Konfiguration vorliegen, sowie die pharmakologisch verwendbaren Salze dieser Verbindungen.

In erster Linie bevorzugt sind die Verbindungen der Formel I, worin R₁ tert-Butoxycarbonyl, Benzyloxycarbonyl, das über die Carboxygruppe gebundene monovalente Radikal der Aminosäure Valin oder das über die Carboxygruppe gebundene Radikal des am Aminostickstoff durch einen der Reste Phenylacetyl, 3-Pyridylacetyl, Morpholinocarbonyl, Thiomorpholinocarbonyl, tert-Butoxycarbonyl oder Benzyloxycarbonyl acylierten Alanins bedeutet, R₂ Wasserstoff, R₃ Benzyl, R₄ Wasserstoff, R₅ Hydroxy, R₆ Wasserstoff, R₇ Isobutyl, Cyclohexylmethyl oder Benzyl, R₈ Wasserstoff und R₉ einen der für R₁ genannten Reste bedeuten und die die Reste R₃ und R₅ tragenden asymmetrischen Kohlenstoffatome in der S-Konfiguration vorliegen, sowie die pharmakologisch verwendbaren Salze dieser Verbindungen.

Zuallererst bevorzugt sind die in den Beispielen genannten Verbindungen und ihre Salze.

Die Verbindungen der Formel I und Salze von solchen Verbindungen mit mindestens einer salzbildenden Gruppe werden nach an sich bekannten Verfahren erhalten, z. B. indem man
a) ein Hydrazinderivat der Formel 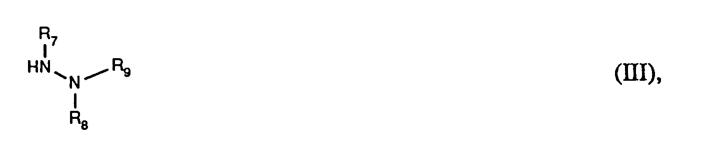 worin die Reste die oben genannten Bedeutungen haben, an ein Epoxid der Formel 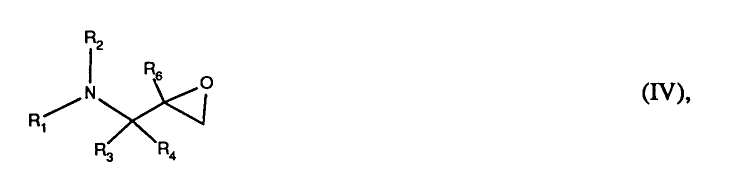 worin die Reste die oben genannten Bedeutungen haben, addiert, wobei freie funktionelle Gruppen mit Ausnahme der an der Reaktion beteiligten gegebenenfalls in geschützter Form vorliegen, und gegebenenfalls vorhandene Schutzgruppen abspaltet, oder
b) zur Herstellung von Verbindungen der Formel I, worin R₁ und R₉ Acyl, Sulfo, durch unsubstituiertes oder substituiertes Alkyl, Aryl, Alkoxy, das unsubstituiert oder substituiert ist, oder Aryloxy substituiertes Sulfonyl, Sulfamoyl, das am Stickstoff unsubstituiert oder substituiert ist, oder durch ein oder zwei unabhängig voneinander aus substituiertem oder unsubstituiertem Alkyl, unsubstituiertem oder substituiertem Cycloalkyl, Aryl, Hydroxy, unsubstituiertem oder substituiertem Alkoxy, Cycloalkoxy und Aryloxy ausgewählte Reste substituiertes Phosphoryl bedeuten, R₂ und R₈ Wasserstoff, unsubstituiertes oder substituiertes Alkyl, Alkenyl oder Alkinyl, oder Heterocyclyl bedeuten und die übrigen Reste die genannten Bedeutungen haben, eine Aminoverbindung der Formel 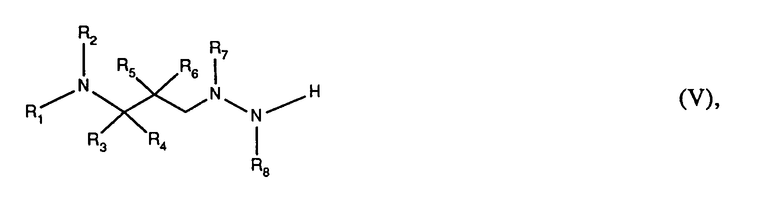 worin die Reste die gerade genannten Bedeutungen haben, mit einer Säure der Formel

   R₉-OH (VI)

   oder reaktionsfähigen Säurederivaten davon, worin R₉ die gerade genannten Bedeutungen hat, kondensiert, wobei freie funktionelle Gruppen mit Ausnahme der an der Reaktion beteiligten gegebenenfalls in geschützter Form vorliegen, und gegebenenfalls vorhandene Schutzgruppen abspaltet, oder
c) zur Herstellung von Verbindungen der Formel I, worin R₁ und R₉ Acyl, Sulfo, durch unsubstituiertes oder substituiertes Alkyl, Aryl, Alkoxy, das unsubstituiert oder substituiert ist, oder Aryloxy substituiertes Sulfonyl, Sulfamoyl, das am Stickstoff unsubstituiert oder substituiert ist, oder durch ein oder zwei unabhängig voneinander aus substituiertem oder unsubstituiertem Alkyl, unsubstituiertem oder substituiertem Cycloalkyl, Aryl, Hydroxy, unsubstituiertem oder substituiertem Alkoxy, Cycloalkoxy und Aryloxy ausgewählte Reste substituiertes Phosphoryl bedeuten, R₂ und R₈ Wasserstoff, unsubstituiertes oder substituiertes Alkyl, Alkenyl oder Alkinyl, oder Heterocyclyl bedeuten und die übrigen Reste die genannten Bedeutungen haben, eine Aminoverbindung der Formel 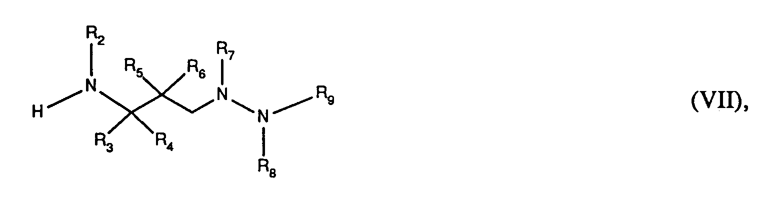 worin die Reste die gerade genannten Bedeutungen haben, mit einer Säure der Formel

   R₁-OH (VIII)

   oder reaktionsfähigen Säurederivaten davon, worin R₁ die gerade genannten Bedeutungen hat, kondensiert, wobei freie funktionelle Gruppen mit Ausnahme der an der Reaktion beteiligten gegebenenfalls in geschützter Form vorliegen, und gegebenenfalls vorhandene Schutzgruppen abspaltet, oder
d) zur Herstellung von Verbindungen der Formel I, worin R₁ und R₉ zwei gleiche Reste ausgewählt aus Acyl, Sulfo, durch unsubstituiertes oder substituiertes Alkyl, Aryl, Alkoxy, das unsubstituiert oder substituiert ist, oder Aryloxy substituiertes Sulfonyl, Sulfamoyl, das am Stickstoff unsubstituiert oder substituiert ist, und durch ein oder zwei oder zwei unabhängig voneinander aus substituiertem oder unsubstituiertem Alkyl, unsubstituiertem oder substituiertem Cycloalkyl, Aryl, Hydroxy, unsubstituiertem oder substituiertem Alkoxy, Cycloalkoxy und Aryloxy ausgewählte Reste substituiertes Phosphoryl bedeuten, R₂ und R₈ Wasserstoff, unsubstituiertes oder substituiertes Alkyl, Alkenyl oder Alkinyl, oder Heterocyclyl bedeuten und die übrigen Reste die genannten Bedeutungen haben, eine Diaminoverbindung der Formel 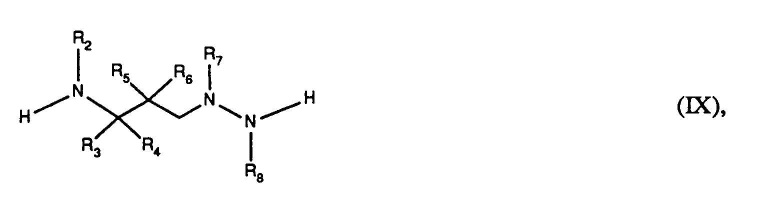 worin die Reste die gerade genannten Bedeutungen haben, mit einer zur Einführung der identischen Reste R₁ und R₉ geeigneten Säure oder reaktionsfähigen Säurederivaten davon, worin R₁ und R₉ die gerade genannten Bedeutungen haben, kondensiert, wobei freie funktionelle Gruppen mit Ausnahme der an der Reaktion beteiligten gegebenenfalls in geschützter Form vorliegen, und gegebenenfalls vorhandene Schutzgruppen abspaltet, oder
e) zur Herstellung einer Verbindung der Formel I, worin an Stelle von R₇ der Rest R₇˝ vorliegt, welcher unsubstituiertes oder substituiertes Alkyl oder Cycloalkyl bedeutet, in einer Verbindung der Formel I′, 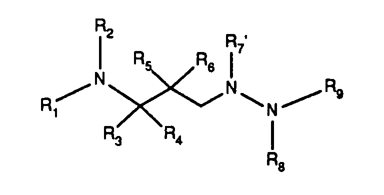 worin R₇′ Wasserstoff bedeutet und die übrigen Reste die genannten Bedeutungen haben, den Rest R₇˝ einführt durch Substitution mit einer Verbindung der Formel XII,

   R₇''-X (XII)

   worin X eine Abgangsgruppe ist und R₇˝ unsubstituiertes oder substituiertes Alkyl oder Cycloalkyl bedeutet, wobei freie funktionelle Gruppen mit Ausnahme der an der Reaktion beteiligten gegebenenfalls in geschützter Form vorliegen, und gegebenenfalls vorhandene Schutzgruppen abspaltet, oder
f) in einer Verbindung der Formel I, worin die Substituenten die oben genannten Bedeutungen haben mit der Massgabe, dass in der betreffenden Verbindung der Formel I mindestens eine funktionelle Gruppe durch Schutzgruppen geschützt ist, vorhandene Schutzgruppen abspaltet
und gewünschtenfalls eine nach einem der vorstehenden Verfahren a) bis f) erhältliche Verbindung der Formel I mit mindestens einer salzbildenden Gruppe in ihr Salz oder ein erhältliches Salz in die freie Verbindung oder in ein anderes Salz überführt und/oder gegebenenfalls erhältliche Isomerengemische auftrennt und/oder eine erfindungsgemässe Verbindung der Formel I in eine andere erfindungsgemässe Verbindung der Formel I umwandelt.

Die obigen Verfahren werden nachfolgend näher beschrieben:

### Verfahren a) (Addition eines Amins an ein Epoxid):

Die an der Reaktion teilnehmende Aminogruppe der Hydrazinderivate der Formel III hat je nach Bedeutung von R₇ bevorzugt mindestens ein freies Wasserstoffatom; sie kann aber auch selbst derivatisiert sein, um die Reaktionsfähigkeit des Hydrazinderivates zu steigern.

Das Epoxid der Formel IV hat insbesondere eine Struktur, die die endständige Addition des Hydrazinderivates bevorzugt ablaufen lässt.

Funktionelle Gruppen in Ausgangsmaterialien, deren Umsetzung vermieden werden soll, insbesondere Carboxy-, Amino-, Hydroxy-, Mercapto- und Sulfogruppen, können durch geeignete Schutzgruppen (conventional protecting groups) geschützt sein, die üblicherwei-se bei der Synthese von Peptid-Verbindungen, aber auch von Cephalosporinen und Peni-cillinen sowie Nucleinsäurederivaten und Zuckern verwendet werden. Diese Schutzgrup-pen können bereits in den Vorstufen vorhanden sein und sollen die betreffenden funk-tionellen Gruppen gegen unerwünschte Nebenreaktionen wie Acylierungen, Veretherun-gen, Veresterungen, Oxidationen, Solvolyse etc. schützen. In bestimmten Fällen können die Schutzgruppen darüber hinaus einen selektiven, beispielsweise stereoselektiven Verlauf von Umsetzungen bewirken. Charakteristisch für Schutzgruppen ist, dass sie leicht, d. h. ohne unerwünschte Nebenreaktionen abspaltbar sind, beispielsweise sol-volytisch, reduktiv, photolytisch oder auch enzymatisch, z. B. auch unter physiologischen Bedingungen. Schutzgruppen analoge Reste können aber auch in den Endstoffen vorhanden sein. Verbindungen der Formel I mit geschützten funktionellen Gruppen können eine höhere metabolische Stabilität oder anderweitig verbesserte pharmakodynamische Eigenschaften aufweisen als die entsprechenden Verbindungen mit freien funktionellen Gruppen. Von Schutzgruppen im engeren Sinne ist vor-und nachstehend die Rede, wenn die betreffenden Reste in den Endstoffen nicht mehr vorhanden sind.

Der Schutz von funktionellen Gruppen durch solche Schutzgruppen, die Schutzgruppen selbst, sowie ihre Abspaltungsreaktionen sind beispielsweise in Standardwerken wie J. F. W. McOmie, "Protective Groups in Organic Chemistry", Plenum Press, London und New York 1973, in Th. W. Greene, "Protective Groups in Organic Synthesis", Wiley, New York 1981, in "The Peptides"; Band 3 (E. Gross und J. Meienhofer, Herausg.), Academic Press, London und New York 1981, in "Methoden der organischen Chemie", Houben-Weyl, 4. Auflage, Band 15/I, Georg Thieme Verlag, Stuttgart 1974, in H.-D. Jakubke und H. Jescheit, "Aminosäuren, Peptide, Proteine", Verlag Chemie, Weinheim, Deerfield Beach und Basel 1982, und in Jochen Lehmann, "Chemie der Kohlenhydrate: Mono-saccharide und Derivate", Georg Thieme Verlag, Stuttgart 1974, beschrieben.

Eine Carboxygruppe ist z. B. als eine Estergruppe geschützt, die unter schonenden Bedingungen selektiv spaltbar ist. Eine in veresterter Form geschützte Carboxygruppe ist in erster Linie durch eine Niederalkylgruppe verestert, welche bevorzugt in 1-Stellung der Niederalkylgruppe verzweigt oder in 1- oder 2-Stellung der Niederalkylgruppe durch geeignete Substituenten substituiert ist.

Eine geschützte Carboxygruppe, welche durch eine Niederalkylgruppe verestert ist, ist beispielsweise Methoxycarbonyl oder Ethoxycarbonyl.

Eine geschützte Carboxygruppe, welche durch eine Niederalkylgruppe verestert ist, die in 1-Stellung der Niederalkylgruppe verzweigt ist, ist beispielsweise tert-Niederalkoxy-carbonyl, z. B. tert-Butoxycarbonyl.

Eine geschützte Carboxygruppe, welche durch eine Niederalkylgruppe verestert ist, welche in 1- oder 2-Stellung der Niederalkylgruppe durch geeignete Substituenten substituiert ist, ist beispielsweise Arylmethoxycarbonyl mit einem oder zwei Arylresten, worin Aryl unsubstituiertes oder z. B. durch Niederalkyl, z. B. tert-Niederalkyl, wie tert-Butyl, Niederalkoxy, z. B. Methoxy, Hydroxy, Halogen, z. B. Chlor, und/oder Nitro mono-, di- oder trisubstituiertes Phenyl bedeutet, beispielsweise Benzyloxycarbonyl, durch die genannten Substituenten substituiertes Benzyloxycarbonyl, z. B. 4-Nitro-benzyloxycarbonyl oder 4-Methoxybenzyloxycarbonyl, Diphenylmethoxycarbonyl oder durch die genannten Substituenten substituiertes Diphenylmethoxycarbonyl, z. B. Di-(4-methoxyphenyl)-methoxycarbonyl, ferner durch eine Niederalkylgruppe verestertes Carboxy, wobei die Niederalkylgruppe in 1- oder 2-Stellung durch geeignete Substituenten substituiert ist, wie 1-Niederalkoxyniederalkoxycarbonyl, z. B. Methoxymethoxy-carbonyl, 1-Methoxyethoxycarbonyl oder 1-Ethoxyethoxycarbonyl, 1-Niederalkylthioniederalkoxycarbonyl, z. B. 1-Methylthiomethoxycarbonyl oder 1-Ethylthioethoxycarbonyl, Aroylmethoxycarbonyl, worin die Aroylgruppe gegebenenfalls beispielsweise durch Halogen, wie Brom, substituiertes Benzoyl darstellt, z. B. Phenacyloxycarbonyl, 2-Halogenniederalkoxycarbonyl, z. B. 2,2,2-Trichlorethoxycarbonyl, 2-Bromethoxycarbonyl oder 2-Jodethoxycarbonyl, sowie 2-(trisubstituiertes Silyl)-niederalkoxycarbonyl, worin die Substituenten unabhängig voneinander je einen gegebenenfalls, z. B. durch Niederalkyl, Niederalkoxy, Aryl, Halogen, und/oder Nitro substituierten aliphatischen, araliphatischen, cycloaliphatischen oder aromatischen Kohlenwasserstoffrest bedeuten, beispiels-weise gegebenenfalls wie oben substituiertes Niederalkyl, Phenylniederalkyl, Cycloalkyl oder Phenyl, z. B. 2-Triniederalkylsilylniederalkoxycarbonyl, wie 2-Triniederalkyl-silylethoxycarbonyl, z. B. 2-Trimethylsilylethoxycarbonyl oder 2-(Di-n-butyl-methyl-silyl)-ethoxycarbonyl, oder 2-Triarylsilylethoxycarbonyl, wie Triphenylsilylethoxycarbonyl.

Eine Carboxygruppe kann auch als organische Silyloxycarbonylgruppe geschützt sein. Ei-ne organische Silyloxycarbonylgruppe ist beispielsweise eine Triniederalkylsilyloxycarbo-nylgruppe, z. B. Trimethylsilyloxycarbonyl. Das Siliciumatom der Silyloxycarbonylgrup-pe kann auch durch zwei Niederalkylgruppen, z. B. Methylgruppen, und die Aminogruppe oder die Carboxygruppe eines zweiten Moleküls der Formel I substituiert sein. Verbindungen mit solchen Schutzgruppen lassen sich z. B. mit Dimethylchlorsilan als Silylierungs-mittel herstellen.

Eine geschützte Carboxygruppe ist bevorzugt tert-Niederalkoxycarbonyl, z. B. tert-But-oxycarbonyl, Benzyloxycarbonyl, 4-Nitrobenzyloxycarbonyl, 9-Fluorenylmethoxycarbonyl oder Diphenylmethoxycarbonyl.

Eine geschützte Aminogruppe kann durch eine Aminoschutzgruppe geschützt, z. B. in Form einer Acylamino-, Arylmethylamino-, veretherten Mercaptoamino-, 2-Acyl-niederalk-1-enylamino oder Silylaminogruppe oder als Azidogruppe, vorliegen.

In einer entsprechenden Acylaminogruppe ist Acyl beispielsweise der Acylrest einer organischen Carbonsäure mit z. B. bis zu 18 Kohlenstoffatomen, insbesondere einer gegebenenfalls, z.B durch Halogen oder Aryl, substituierten Niederalkancarbonsäure oder gegebenenfalls, z. B. durch Halogen, Niederalkoxy oder Nitro, substituierten Benzoesäure, oder bevorzugt eines Kohlensäurehalbesters. Solche Acylgruppen sind beispielsweise Nie-deralkanoyl, wie Formyl, Acetyl, Propionyl oder Pivaloyl, Halogenniederalkanoyl, z. B. 2-Halogenacetyl, wie 2-Chlor-, 2-Brom-, 2-Jod-, 2,2,2-Trifluor- oder 2,2,2-Trichloracetyl, gegebenenfalls z. B. durch Halogen, Niederalkoxy oder Nitro substituiertes Benzoyl, wie Benzoyl, 4-Chlorbenzoyl, 4-Methoxybenzoyl oder 4-Nitrobenzoyl, Niederalkoxycarbonyl, vorzugsweise in 1-Stellung des Niederalkylrestes verzweigtes oder in 1- oder 2-Stellung geeignet substituiertes Niederalkoxycarbonyl, z.B. tert-Niederalkoxycarbonyl, wie tert-Butoxycarbonyl, Arylmethoxycarbonyl mit einem, zwei oder drei Arylresten, die ge-gebenenfalls, z. B. durch Niederalkyl, insbesondere tert-Niederalkyl, wie tert-Butyl, Nie-deralkoxy, wie Methoxy, Hydroxy, Halogen, wie Chlor, und/oder Nitro mono- oder polysubstituiertes Phenyl darstellen, z. B. Benzyloxycarbonyl, 4-Nitrobenzyloxycarbonyl, Diphenylmethoxycarbonyl, 9-Fluorenylmethoxycarbonyl oder Di-(4-methoxyphenyl)-methoxycarbonyl, Aroylmethoxycarbonyl, worin die Aroylgruppe vorzugsweise gegebenenfalls, z. B. durch Halogen, wie Brom, substituiertes Benzoyl darstellt, z. B. Phenacyloxycarbonyl, 2-Halogen-niederalkoxycarbonyl, z. B. 2,2,2-Trichlorethoxycarbonyl, 2-Bromethoxycarbonyl oder 2-Jodethoxycarbonyl, 2-(trisubstituiertes Silyl)-niederalkoxycarbonyl, z. B. 2-Triniederalkylsilylniederalkoxycarbonyl, wie 2-Trimethylsilylethoxycarbonyl oder 2-(Di-n-butyl-methyl-silyl)-ethoxycarbonyl, oder Triarylsilylniederalkoxycarbonyl, z. B. 2-Triphenylsilylethoxycarbonyl.

In einer Arylmethylaminogruppe, die z. B. eine Mono-, Di- oder insbesondere Triarylmethylaminogruppe darstellt, sind die Arylreste insbesondere gegebenenfalls substituierte Phenylreste. Solche Gruppen sind z. B. Benzyl-, Diphenylmethyl- oder insbesondere Tritylamino.

In einer veretherten Mercaptoaminogruppe liegt die Mercaptogruppe in erster Linie als substituiertes Arylthio oder Arylniederalkylthio, worin Aryl beispielsweise gegebenenfalls z. B. durch Niederalkyl, wie Methyl oder tert.-Butyl, Niederalkoxy, wie Methoxy, Halogen, wie Chlor, und/oder Nitro substituiertes Phenyl ist, z. B. 4-Nitrophenylthio, vor.

In einem als Aminoschutzgruppe verwendbaren 2-Acyl-niederalk-1-enylrest ist Acyl z. B. der entsprechende Rest einer Niederalkancarbonsäure, einer gegebenenfalls z. B. durch Niederalkyl, wie Methyl oder tert-Butyl, Niederalkoxy, wie Methoxy, Halogen, wie Chlor, und/oder Nitro substituierten Benzoesäure, oder insbesondere eines Kohlensäurehalb-esters, wie eines Kohlensäure-niederalkylhalbesters. Entsprechende Schutzgruppen sind in erster Linie 1 -Niederalkanoyl-niederalk-1-en-2-yl, z.B. 1 -Niederalkanoyl-prop-1-en-2-yl, wie 1-Acetyl-prop-1-en-2-yl, oder Niederalkoxycarbonyl-niederalk-1-en-2-yl, z. B. Niederalkoxycarbonyl-prop-1-en-2-yl, wie 1-Ethoxycarbonyl-prop-1-en-2-yl.

Eine Silylaminogruppe ist beispielsweise eine Triniederalkylsilylaminogruppe, z. B. Trimethylsilylamino oder tert-Butyl-dimethylsilylamino. Das Siliciumatom der Silylaminogruppe kann auch nur durch zwei Niederalkylgruppen, z. B. Methylgruppen, und die Aminogruppe oder Carboxylgruppe eines zweiten Moleküls der Formel I substituiert sein. Verbindungen mit solchen Schutzgruppen lassen sich z. B. mit den entsprechenden Chlorsilanen, wie Dimethylchlorsilan, als Silylierungsmittel herstellen.

Eine Aminogruppe kann auch durch Überführung in die protonierte Form geschützt werden; als entsprechende Anionen kommen in erster Linie diejenigen von starken anorganischen Säuren, wie von Schwefelsäure, Phosphorsäure oder Halogenwasserstoffsäuren, z. B. das Chlor- oder Bromanion, oder von organischen Sulfonsäuren, wie p-Toluolsulfonsäure, in Frage.

Bevorzugte Aminoschutzgruppen sind Niederalkoxycarbonyl, Phenylniederalkoxycarbonyl, Fluorenylniederalkoxycarbonyl, 2-Niederalkanoyl-niederalk-1-en-2-yl oder Niederalkoxycarbonyl-niederalk-1-en-2-yl.

Eine Hydroxygruppe kann beispielsweise durch eine Acylgruppe, z. B. durch Halogen, wie Chlor, substituiertes Niederalkanoyl, wie 2,2-Dichloracetyl, oder insbesondere durch einen für geschützte Aminogruppen genannten Acylrest eines Kohlensäurehalbesters geschützt sein. Eine bevorzugte Hydroxyschutzgruppe ist beispielsweise 2,2,2-Trichlorethoxycarbonyl, 4-Nitrobenzyloxycarbonyl, Diphenylmethoxycarbonyl oder Trityl. Eine Hydroxygruppe kann ferner durch Triniederalkylsilyl, z. B. Trimethylsilyl, Triisopropylsilyl oder tert-Butyl-dimethylsilyl, eine leicht abspaltbare verethernde Gruppe, z. B. eine Alkylgruppe, wie tert-Niederalkyl, z. B. tert-Butyl, einen oxa- oder einen thiaaliphatischen oder -cycloaliphatischen, insbesondere 2-oxa- oder 2-thiaaliphatischen oder -cycloaliphatischen, Kohlenwasserstoffrest, beispielsweise 1-Niederalkoxyniederalkyl oder 1-Niederalkylthioniederalkyl, wie Methoxymethyl, 1-Methoxyethyl, 1-Ethoxyethyl, Methylthiomethyl, 1-Methylthioethyl oder 1-Ethylthioethyl, oder 2-Oxa- oder 2-Thiacycloalkyl mit 5-7 Ringatomen, wie 2-Tetrahydrofuryl oder 2-Tetrahydropyranyl, oder ein entsprechendes Thiaanaloges, sowie durch I-Phenylniederalkyl, wie Benzyl, Diphenylmethyl oder Trityl, wobei die Phenylreste beispielsweise durch Halogen, z.B. Chlor, Niederalkoxy, z. B. Methoxy, und/oder Nitro substituiert sein können, geschützt sein.

Zwei in einem Molekül vorkommende, insbesondere benachbarte Hydroxylgruppen oder eine benachbarte Hydroxy- und Aminogruppe können beispielsweise durch bivalente Schutzgruppen, wie eine vorzugsweise, etwa durch ein oder zwei Niederalkylreste oder Oxo, substituierte Methylengruppe, geschützt sein, z. B. durch unsubstituiertes oder substituiertes Alkyliden, z. B. Niederalkyliden, wie Isopropyliden, Cycloalkyliden, wie Cyclohexyliden, eine Carbonylgruppe oder Benzyliden.

Eine Mercaptogruppe, wie z. B. in Cystein, kann insbesondere durch S-Alkylierung mit gegebenenfalls substituierten Alkylresten, Silylierung, Thioacetalbildung, S-Acylierung oder durch die Bildung asymmetrischer Disulfidgruppierungen geschützt sein. Bevorzugte Mercaptoschutzgruppen sind beispielsweise gegebenenfalls im Phenylrest, z. B. durch Methoxy oder Nitro, substituiertes Benzyl, wie 4-Methoxybenzyl, gegebenenfalls im Phenylrest, z. B. durch Methoxy, substituiertes Diphenylmethyl, wie Di-(4-methoxyphenyl)-methyl, Triphenylmethyl, Pyridyldiphenylmethyl, Trimethylsilyl, Benzylthiomethyl, Tetrahydropyranyl, Acylaminomethyl, wie Acetamidomethyl, iso-Butyrylacetamido-methyl oder 2-Chloracetamidomethyl, Benzoyl, Benzyloxycarbonyl oder Alkyl-, insbesondere Niederalkylaminocarbonyl, wie Ethylaminocarbonyl, sowie Niederalkylthio, wie S-Ethylthio oder S-tert-Butylthio, oder S-Sulfo.

Eine Sulfogruppe kann beispielsweise durch Niederalkyl, z. B. Methyl oder Ethyl, durch Phenyl oder als Sulfonamid, beispielsweise als Imidazolid, geschützt sein.

Als Schutzgruppe, beispielsweise Carboxyschutzgruppe, im Sinne dieser Anmeldung ist ausdrücklich auch ein in leicht abspaltbarer Weise mit der zu schützenden funktionellen Gruppe, beispielsweise Carboxygruppe, verbundener polymerer Träger zu verstehen, wie er z. B. für die Merrifield-Synthese geeignet ist. Ein solcher geeigneter polymerer Träger ist beispielsweise ein durch Copolymerisation mit Divinylbenzol schwach vernetztes Polystyrolharz, das zur reversiblen Bindung geeignete Brückenglieder trägt.

Die Addition der Verbindungen der Formel III an die Epoxide der Formel IV erfolgt vorzugsweise unter den zur Addition von Nukleophilen an Epoxide üblichen Reaktionsbedingungen.

Die Addition erfolgt insbesondere in wässriger Lösung und/oder in Gegenwart von polaren Lösungsmitteln, wie Alkoholen, z. B. Methanol, Ethanol oder Ethylenglykol, Ethern, wie Dioxan, Amiden, wie Dimethylformamid, oder Phenolen, wie Phenol, auch unter wasserfreien Bedingungen, in apolaren Lösungsmitteln, wie Benzol und Toluol, oder in Benzol/Wasser-Emulsionen, gegebenenfalls in Gegenwart von sauren oder basischen Katalysatoren, z. B. von Laugen, wie Natronlauge, oder in Gegenwart von mit dem Hydrazin dotierten Festphasenkatalysatoren, wie Aluminiumoxid, in Ethern, z. B. Diethyl-ether, allgemein bei Temperaturen von etwa 0 °C bis zur Siedetemperatur des entsprechenden Reaktionsgemisches, vorzugsweise zwischen 20 ° und 130 °C, gegebenenfalls unter Rückfluss, unter erhöhtem Druck, z. B. im Bombenrohr, wobei die Siedetemperatur auch überschritten werden kann, und/oder unter Inertgas, wie Stickstoff oder Argon, wobei jede einzelne der beiden Verbindungen der Formel III und IV im Überschuss vorliegen kann, beispielsweise im Molverhältnis 1: 1 bis 1: 100, vorzugsweise im Molverhältnis 1:1 bis 1:10, besonders bevorzugt im Verhältnis 1:1 bis 1:3.

Die Freisetzung von geschützten Gruppen erfolgt gegebenenfalls nach den unter Verfahren f) (Schutzgruppenabspaltung) beschriebenen Methoden.

### Verfahren b) (Herstellung einer Amidbindung)

In Ausgangsmaterialien der Formel V und VI sind funktionelle Gruppen mit Ausnahme der Gruppen, die an der Reaktion teilnehmen sollen oder die unter den Reaktionsbedin-gungen nicht reagieren, unabhängig voneinander durch eine der unter Verfahren a) ge-nannten Schutzgruppen geschützt.

Die Verbindungen der Formel VI enthalten eine freie Carboxy-, Sulfo- oder Phosphoryl-gruppe oder reaktionsfähige Säurederivate davon, z. B. die abgeleiteten aktivierten Ester oder reaktionsfähigen Anhydride, ferner reaktionsfähige cyclische Amide. Die reaktions-fähigen Säurederivate können auch in situ gebildet werden.

Aktivierte Ester von Verbindungen der Formel VI mit endständiger Carboxygruppe sind insbesondere am Verknüpfungskohlenstoffatom des veresternden Restes ungesättigte Ester, z. B. vom Vinylester-Typ, wie Vinylester (erhältlich z. B. durch Umesterung eines entsprechenden Esters mit Vinylacetat; Methode des aktivierten Vinylesters), Carbamoylester (erhältlich z. B. durch Behandeln der entsprechenden Säure mit einem Isoxazoliumreagens; 1,2-Oxazolium- oder Woodward-Methode), oder 1-Niederalkoxyvinylester (erhältlich z. B. durch Behandeln der entsprechenden Säure mit einem Niederalkoxyacetylen; Ethoxyacetylen-Methode), oder Ester vom Amidinotyp, wie N,N′-disubstituierte Amidinoester (erhältlich z. B. durch Behandeln der entsprechenden Säure mit einem geeigneten N,N′-disubstituierten Carbodiimid, z. B. N,N′-Dicyclohexylcarbodiimid; Carbodiimid-Methode), oder N,N-disubstituierte Amidinoester (erhältlich z. B. durch Behandeln der entsprechenden Säure mit einem N,N-disubstituierten Cyananmid; Cyanamid-Methode), geeignete Arylester, insbesondere durch elektronenanziehende Substituenten geeignet substituierte Phenylester (erhältlich z. B. durch Behandeln der entsprechenden Säure mit einem geeignet substituierten Phenol, z. B. 4-Nitrophenol, 4-Methylsulfonylphenol, 2,4,5-Trichlorphenol, 2,3,4,5,6-Pentachlorphenol oder 4-Phenyldiazophenol, in Gegenwart eines Kondensationsmittels, wie N,N′-Dicyclohexylcarbodiimid; Methode der aktivierten Arylester), Cyanmethylester (erhältlich z. B. durch Behandeln der entsprechenden Säure mit Chloracetonitril in Gegenwart einer Base; Cyanmethylester-Methode), Thioester, ins-besondere gegebenenfalls, z. B. durch Nitro, substituierte Phenylthioester (erhältlich z. B. durch Behandeln der entsprechenden Säure mit gegebenenfalls, z. B. durch Nitro, substituierten Thiophenolen, u. a. mit Hilfe der Anhydrid- oder Carbodiimid-Methode; Methode der aktivierten Thiolester), oder insbesondere Amino- oder Amidoester (erhältlich z. B. durch Behandeln der entsprechenden Säure mit einer N-Hydroxyamino- bzw. N-Hydroxyamidoverbindung, z. B. N-Hydroxysuccinimid, N-Hydroxypiperidin, N-Hydroxyphthalimid, N-Hydroxy-5-norbornen-2,3-dicarbonsäureimid, 1-Hydroxybenztriazol oder 3-Hydroxy-3,4-dihydro-1,2,3-benztriazin-4-on, z. B. nach der Anhydrid- oder Carbodiimid-Methode; Methode der aktivierten N-Hydroxyester). Es sind auch innere Ester, z. B. γ-Lactone, einsetzbar.

Anhydride von Säuren können symmetrische oder vorzugsweise gemischte Anhydride dieser Säuren sein, z. B. Anhydride mit anorganischen Säuren, wie Säurehalogenide, insbesondere Säurechloride (erhältlich z. B. durch Behandeln der entsprechenden Säure mit Thionylchlorid, Phosphorpentachlorid, Phosgen oder Oxalylchlorid; Säurechloridmethode), Azide (erhältlich z. B. aus einem entsprechenden Säureester über das entsprechende Hydrazid und dessen Behandlung mit salpetriger Säure; Azidmethode), Anhydride mit Kohlensäurehalbestern, z. B. Kohlensäureniederalkylhalbestern (erhältlich z. B. durch Behandeln der entsprechenden Säure mit Chlorameisensäureniederalkylestern oder mit einem 1-Niederalkoxycarbonyl-2-niederalkoxy-1,2-dihydrochinolin; Methode der gemischten O-Alkylkohlensäureanhydride), oder Anhydride mit dihalogenierter, insbeson-dere dichlorierter Phosphorsäure (erhältlich z. B. durch Behandeln der entsprechenden Säure mit Phosphoroxychlorid; Phosphoroxychloridmethode), Anhydride mit anderen Phosphorsäurederivaten (z. B. solchen, die man mit Phenyl-N-Phenylphosphoramido-chloridat erhalten kann oder durch Umsetzung von Alkylphosphorsäureamiden in Gegen-wart von Sulfonsäureanhydriden und/oder racemisierungssenkenden Additiven, wie N-Hydroxybenztriazol, oder in Gegenwart von Cyanphosphonsäurediethylester) oder mit Phosphorigsäurederivaten, oder Anhydride mit organischen Säuren, wie gemischte An-hydride mit organischen Carbonsäuren (erhältlich z. B. durch Behandeln der entspre-chenden Säure mit einem gegebenenfalls substituierten Niederalkan- oder Phenylnieder-alkancarbonsäurehalogenid, z. B. Phenylessigsäure-, Pivalinsäure- oder Trifluoressig-säurechlorid; Methode der gemischten Carbonsäureanhydride) oder mit organischen Sul-fonsäuren (erhältlich z. B. durch Behandeln eines Salzes, wie eines Alkalimetallsalzes, der entsprechenden Säure mit einem geeigneten organischen Sulfonsäurehalogenid, wie Nie-deralkan- oder Aryl-, z. B. Methan- oder p-Toluolsulfonsäurechlorid; Methode der ge-mischten Sulfonsäureanhydride) sowie symmetrische Anhydride (erhältlich z. B. durch Kondensation der entsprechenden Säure in Gegenwart eines Carbodiimides oder von 1-Diethylaminopropin; Methode der symmetrischen Anhydride).

Geeignete cyclische Amide sind insbesondere Amide mit fünfgliedrigen Diazacyclen aromatischen Charakters, wie Amide mit Imidazolen, z. B. Imidazol (erhältlich z. B. durch Behandeln der entsprechenden Säure mit N,N′-Carbonyldiimidazol; Imidazol-Methode), oder Pyrazol, z. B. 3,5-Dimethylpyrazol (erhältlich z. B. über das Säurehydrazid durch Behandeln mit Acetylaceton; Pyrazolid-Methode).

Wie erwähnt, können Derivate von Carbonsäuren, die als Acylierungsmittel verwendet werden, auch in situ gebildet werden. So kann man z. B. N,N′-disubstituierte Amidino-ester in situ bilden, indem man das Gemisch des Ausgangsmaterials der Formel V und der als Acylierungsmittel verwendeten Säure in Gegenwart eines geeigneten N,N′-disubstitu-ierten Carbodiimids, z. B. N,N′-Cyclohexylcarbodiimid, zur Reaktion bringt. Weiter kann man Amino- oder Amidoester der als Acylierungsmittel verwendeten Säuren in Gegen-wart des zu acylierenden Ausgangsmaterials der Formel V bilden, indem man das Gemisch der entsprechenden Säure- und Amino-Ausgangsstoffe in Gegenwart eines N,N′-disubstituierten Carbodiimids, z. B. N,N′-Dicyclohexylcarbodiimid, und eines N-Hydroxy-amins oder N-Hydroxy-amids, z B. N-Hydroxysuccinimid, gegebenenfalls in Anwesenheit einer geeigneten Base, z. B. 4-Dimethylamino-pyridin, umsetzt. Ferner kann man durch Umsetzung mit N,N,N′,N′-Tetraalkyluroniumverbindungen, wie O-Benztriazol- 1-yl-N,N,N′,N′-tetra-methyl-uronium-hexafluorphosphat, O-(1,2-Dihydro-2-oxo-1-pyridyl)-N,N,N′,N′-tetramethyluronium-tetrafluorborat oder O-(3,4-Dihydro-4-oxo-1,2,3-benz-triazolin-3-yl)N,N,N′,N′-tetramethyluronium-tetrafluorborat, Aktivierung in situ erreichen. Schliesslich können Phosphorsäureanhydride der Carbonsäuren der Formel VI oder VII in situ hergestellt werden, indem man ein Alkylphosphorsäureamid, wie Hexamethylphosphorsäuretriamid, in Gegenwart eines Sulfonsäureanhydrides, wie 4-Toluolsulfonsäureanhydrid, mit einem Salz, wie einem Tetrafluoroborat, z. B. Natriumtetrafluoroborat, oder mit einem anderen Abkömmling des Hexamethylphosphorsäuretriamides, wie Benzotriazol-1-yl-oxy-tris-(dimethylamino)-phosphonium-hexafluorid, vorzugsweise in Gegenwart eines racemisierungssenkenden Additives, wie N-Hydroxybenztriazol, umsetzt.

In analoger Weise lassen sich viele der oben für Carbonsäuren der Formeln VI aufgeführten Reaktionstypen auch für Verbindungen der Formel III mit endständiger Sulfonyl-oder Phosphorylgruppe bei der Kondensation mit Verbindungen der Formel V zu Sulfonamiden durchführen.

So können beispielsweise aktivierte Sulfonsäureester eingesetzt werden, z. B. die entsprechenden, insbesondere durch Nitrogruppen substituierten, Arylester, wie Phenylester, wobei die Aminkomponente der Formel V auch als Alkalimetallamid, z. B. Alkalimetallarylamid, wie Natriumanilinamid, oder Alkalimetallsalz von stickstoffhaltigen Heterocyclen, z. B. Kalium-pyrrolid, eingesetzt werden kann.

Ferner können reaktionsfähige Anhydride zum Einsatz kommen, wie etwa die entsprechenden symmetrischen (herstellbar z. B. durch Reaktion der alkylsulfonsauren Silbersalze mit Alkylsulfonylchloriden) oder vorzugsweise asymmetrischen Säureanhydride, z. B. Anhydride mit anorganischen Säuren, wie Sulfonylhalogenide, insbesondere Sulfonylchloride (erhältlich z. B. durch Umsetzung der entsprechenden Sulfonsäuren mit anorganischen Säurechloriden, z. B Thionylchlorid, Phosphorpentachlorid), mit organischen Carbonsäuren (erhältlich z. B. durch Behandeln eines Sulfonsäurehalogenides mit dem Salz einer Carbonsäure, wie einem Alkalimetallsalz, analog der oben genannten Methode der gemischten Sulfonsäureanhydride), oder Azide (erhältlich z. B. aus einem entspre-chenden Sulfonsäurechlorid und Natriumazid oder über das entsprechende Hydrazid und dessen Behandlung mit salpetriger Säure analog der oben genannten Azidmethode).

Die Phosphorylreste R₉ mit den oben genannten Substituenten können durch analoge Verfahren, wie durch aktivierte Phosphorderivate, z. B. entsprechend substituierte Phosphorylhalogenide mit einem oder mehr Halogenatomen, wie Phosphoroxychlorid, in Abwesenheit oder Gegenwart von Basen, wie sterisch gehinderten Aminen, in wässrigen oder nichtwässrigen Lösungsmitteln an Verbindungen der Formel V kondensiert werden, wobei gegebenenfalls anschliessend überschüssige Halogenatome durch Hydrolyse oder Umsetzung mit den entsprechenden Alkoholen mit geeigneten Substituenten vertauscht werden.

Die durch Hydroxy, Alkoxy, Cycloalkoxy, Cycloalkylniederalkoxy, Aryloxy oder Arylniederalkoxy substituierten Phosphorylreste lassen sich beispielsweise durch Umsetzung der entsprechend substituierten Phosphite, z. B. Diaryloxyphosphit oder Dialkoxyphosphit, oder der entsprechend substituierten Mono- oder Dihalogeno-, wie Mono- oder Di-chloro-phosphate, in Gegenwart einer Base, wie 2,6-Dimethylpyridin, Triethylamin oder Imidazol, in wässrigen oder wasserfreien Lösungsmitteln, wie den betreffenden Alkoholen, z. B. Ethanol, entprechenden Estern, oder Dichlormethan oder Tetrachlormethan, gegebenenfalls unter Schutzgas, wie Argon, bei Temperaturen von - 50 bis 100 °C, vorzugsweise von -10 bis 50 °C, mit den Verbindungen der Formel V umgesetzt werden, insbesondere unter den in der am 04.07.90 publizierten Europäischen Patentanmeldung EP-A 0 376 040 beschriebenen Bedingungen.

Die entsprechenden, durch die genannten, über Oxy gebundenen Reste symmetrisch oder vorzugsweise unsymmetrisch substituierten Phosphorylreste lassen sich durch Umsetzung von Phosphortrihalogeniden, wie Phosphortrichlorid, mit den entsprechenden Aminen der Formel V und in Gegenwart organischer Amine, wie Triethylamin, wobei die entsprechenden Dichlorphosphorylverbindungen entstehen, Umsetzung dieser Verbindungen mit dem ersten entsprechenden Alkohol oder mit Wasser, welche vorzugsweise in stöchiometrischer Menge eingesetzt werden, in Gegenwart von einem tertiären Amin, und gegebenenfalls anschliessender Umsetzung des zweiten Halogenatoms mit einem weiteren Alkohol oder Wasser in Gegenwart eines tertiären Amins unter Erhalt der entsprechenden disubstituierten Phosphit-Verbindungen, die dann oxidiert werden, z. B. mit Halogenen, wie Jod, Peroxiden, wie Wasserstoffperoxid, Persäuren, wie m-Chlorperbenzoesäure, oder molekularem Sauerstoff, gewinnen.

Die Aminogruppe von Verbindungen der Formel V, die an der Reaktion teilnimmt, trägt vorzugsweise mindestens ein reaktionsfähiges Wasserstoffatom, insbesondere, wenn die damit reagierende Carboxy-, Sulfonyl- oder Phosphorylgruppe in reaktionsfähiger Form vorliegt; sie kann aber auch selbst derivatisiert sein, z. B. durch Reaktion mit einem Phosphit, wie Diethylchlorphosphit, 1,2-Phenylenchlorphosphit, Ethyldichlorphosphit, Ethylenchlorphosphit oder Tetraethylpyrophosphit. Ein Derivat einer solchen Verbindung mit einer Aminogruppe ist z. B. auch ein Carbaminsäurehalogenid oder ein Isocyanat, wobei die an der Reaktion teilnehmende Aminogruppe durch Halogencarbonyl, z. B. Chlorcarbonyl, substituiert bzw. als Isocyanatgruppe abgewandelt ist, wobei im letzteren Falle nur Verbindungen der Formel I zugänglich sind, die am Stickstoffatom der durch die Reaktion gebildeten Amidgruppe ein Wasserstoffatom tragen.

Ist die Verbindung der Formel V an der Aminogruppe durch Niederalkyl oder Arylniederalkyl einfach substituiert, so stellt auch eine entsprechende Harnstoffverbindung ein reaktionsfähiges Derivat dar. Beispielsweise erhält man beim Erhitzen equimolarer Mengen dieser Harnstoffverbindung und der Verbindung der Formel VI oder VIII mit freier Carboxygruppe entsprechende Verbindungen der Formel I.

Die Kondensation zur Herstellung einer Amidbindung kann in an sich bekannter Weise durchgeführt werden, beispielsweise wie in Standardwerken, wie "Houben-Weyl, Methoden der organischen Chemie", 4. Auflage, Band 15/II (1974), Band IX (1955) Band E 11 (1985), Georg Thieme Verlag, Stuttgart, "The Peptides" (E. Gross und J. Meienhofer, Hg.), Band 1 und 2, Academic Press, London und New York, 1979/1980, oder M. Bodansky, "Principles of Peptide Synthesis", Springer-Verlag, Berlin 1984, beschrieben.

Die Kondensation einer freien Carbonsäure mit dem entsprechenden Amin kann vorzugs-weise in Gegenwart eines der üblichen Kondensationsmittel durchgeführt werden, oder unter Verwendung von Carbonsäureanhydriden oder Carbonsäurehalogeniden, wie -chloriden, oder von aktivierten Carbonsäureestern, wie p-Nitrophenylestern. Übliche Kondensationsmittel sind z. B. Carbodiimide, beispielsweise Diethyl-, Dipropyl-, N-Ethyl-N′-(3-dimethylaminopropyl)-carbodiimid oder insbesondere Dicyclohexyl-carbodiimid, ferner geeignete Carbonylverbindungen, beispielsweise Carbonylimidazol, 1,2-Oxazoliumverbindungen, z. B. 2-Ethyl-5-phenyl- 1,2-oxazolium-3′-sulfonat und 2-tert-Butyl-5-methylisoxazoliumperchlorat, oder eine geeignete Acylaminoverbindung, z. B. 2-Ethoxy-1-ethoxycarbonyl- 1,2-dihydrochinolin, N,N,N′,N′-Tetraalkyluroniumverbin-dungen, wie O-Benztriazol-1-yl-N,N,N′,N′-tetra-methyluronium-hexafluorphosphat, ferner aktivierte Phosphorsäurederivate, z. B. Diphenylphosphorylazid, Diethylphos-phorylcyanid, Phenyl-N-phenylphosphoroamidochloridat, Bis-(2-oxo-3-oxazolidinyl)-phosphinsäurechlorid oder 1-Benztriazolyloxy-tris-(dimethylamino)-phosphonium-hexa-fluorophosphat.

Gewünschtenfalls wird eine organische Base zugegeben, vorzugsweise ein tertiäres Amin, z. B. ein Triniederalkylamin mit voluminösen Resten, z. B. Ethyldiisopropylamin, oder Triethylamin, und/oder eine heterocyclische Base, z. B. 4-Dimethylaminopyridin oder bevorzugt N-Methylmorpholin oder Pyridin.

Die Kondensation aktivierter Ester, reaktionsfähiger Anhydride oder reaktionsfähiger cyclischer Amide mit den entsprechenden Aminen wird üblicherweise in Gegenwart einer organischen Base, z. B. einfachen Triniederalkylaminen, z. B. Triethylamin oder Tributylamin, oder einer der vorstehend genannten organischen Basen durchgeführt. Gewünschtenfalls wird zusätzlich noch ein Kondensationsmittel verwendet, wie für freie Carbonsäuren beschrieben ist.

Die Kondensation von Säureanhydriden mit Aminen kann z. B. in Gegenwart von anorga-nischen Carbonaten, z. B. Ammonium- oder Alkalimetallcarbonaten oder -hydrogen-carbonaten, wie Natrium- oder Kaliumcarbonat oder -hydrogencarbonat (gewünschtenfalls zusammen mit einem Sulfat), erfolgen.

Carbonsäurechloride, beispielsweise die von der Säure der Formel VI abgeleiteten Chlorkohlensäurederivate, oder Sulfonsäurechloride werden mit den entsprechenden Aminen vorzugsweise in Gegenwart eines organischen Amins, z. B. der vorstehend genannten Triniederalkylamine oder heterocyclischen Basen, gegebenenfalls in Gegenwart eines Hydrogensulfates, kondensiert.

Die Kondensation wird vorzugsweise in einem inerten, aprotischen, vorzugsweise wasserfreien, Lösungsmittel oder Lösungsmittelgemisch durchgeführt, beispielsweise in einem Carbonsäureamid, z. B. Formamid oder Dimethylformamid, einem halogenierten Kohlenwasserstoff, z. B. Methylenchlorid, Tetrachlorkohlenstoff oder Chlorbenzol, einem Keton, z. B. Aceton, einem cyclischen Ether, z. B. Tetrahydrofuran, einem Ester, z. B. Essigsäureethylester, oder einem Nitril, z. B. Acetonitril, oder in einer Mischung davon, gegebenenfalls bei erniedrigter oder erhöhter Temperatur, z. B. in einem Temperaturbereich von etwa -40 °C bis etwa +100 °C, bevorzugt von etwa -10 °C bis etwa +50 °C, im Falle der Verwendung von Arylsulfonylestern auch bei etwa +100 °C bis +200 °C, und gegebenenfalls unter Inertgas-, z. B. Stickstoff- oder Argonatmosphäre.

Auch wässrige, beispielsweise alkoholische, z. B. Ethanol, oder aromatische Lösungs-mittel, z. B. Benzol oder Toluol, sind möglich. Bei Gegenwart von Alkalihydroxiden als Basen kann gegebenenfalls auch Aceton zugesetzt werden.

Die Kondensation kann auch gemäss der als Festphasen-Synthese bekannten Technik erfolgen, die auf R. Merrifield zurückgeht und beispielsweise in Angew. Chem. 97, 801 - 812 (1985), Naturwissenschaften 71, 252 - 258 (1984) oder in R. A. Houghten, Proc. Natl. Acad. Sci. USA 82, 5131 - 5135 ( 1985) beschrieben ist.

Je nach verwendeten Ausgangsverbindungen können die Reste R₁ und R₉ in den erhältlichen Verbindungen der Formel I identisch oder voneinander verschieden sein.

Die Freisetzung von *geschützten* Gruppen erfolgt gegebenenfalls nach den unter Verfahren f) (Schutzgruppenabspaltung) beschriebenen Methoden.

### Verfahren c) (Herstellung einer Amidbindung)

In Ausgangsmaterialien der Formel VII und VIII sind funktionelle Gruppen mit Ausnahme der Gruppen, die an der Reaktion teilnehmen sollen oder die unter den den Reaktionsbedingungen nicht reagieren, unabhängig voneinander durch eine der unter Verfahren a) genannten Schutzgruppen geschützt.

Das Verfahren ist vollständig analog zu dem unter Verfahren b) genannten, wenn man anstelle von Verbindungen der Formel V die der Formel VII und anstelle von Verbindungen der Formel VI die der Formel VIII einsetzt und bei der Acylierung R₁ anstelle von R₉ an Verbindungen der Formel VII anstelle der Verbindungen der Formel V bindet.

Je nach verwendeten Ausgangsverbindungen können die Reste R₁ und R₉ in den erhältlichen Verbindungen der Formel I identisch oder voneinander verschieden sein.

Die Freisetzung von geschützten Gruppen erfolgt gegebenenfalls nach den unter Verfahren f) (Schutzgruppenabspaltung) beschriebenen Methoden.

### Verfahren d) (Herstellung einer Amidbindung)

In Ausgangsmaterialien der Formel IX und der zur Einführung der identischen Reste R₁ und R₉ geeigneten Säure oder deren reaktionsfähigen Derivaten sind funktionelle Gruppen, die nicht an der Reaktion teilnehmen sollen oder die unter den Reaktionsbedingungen nicht reagieren, unabhängig voneinander durch eine der unter Verfahren a) genannten Schutzgruppen geschützt.

Die zur Einführung der identischen Reste R₁ und R₉ geeignete Säure hat vorzugsweise eine der Formeln VI oder VIII.

Bevorzugt als gegebenenfalls durch Schutzgruppen geschützte Ausgangsverbindungen der Formel IX sind diejenigen der Formel II, die unten im Abschnitt über Ausgangsverbin-dungen beschrieben sind.

Das Verfahren ist vollständig analog zu dem unter Verfahren b) genannten, wobei man anstelle von Verbindungen der Formel V die der Formel IX und anstelle von Verbindungen der Formel VI die der Formel VI oder VIII einsetzt.

Die Freisetzung von geschützten Gruppen erfolgt gegebenenfalls nach den unter Verfahren f) (Schutzgruppenabspaltung) beschriebenen Methoden.

### Verfahren e) (Alkylierung eines sekundären Stickstoffatomes)

In Ausgangsmaterialien der Formel I′ und der zur Einführung des Restes R₇˝ geeigneten Verbindung der Formel XII oder deren reaktionsfähigen Derivaten sind funktionelle Gruppen, die nicht an der Reaktion teilnehmen sollen oder die unter den Reaktionsbedingungen nicht reagieren, unabhängig voneinander durch eine der unter Verfahren a) genannten Schutzgruppen geschützt.

Eine Abgangsgruppe X ist insbesondere eine nukleofuge Abgangsgruppe ausgewählt aus mit einer starken anorganischen oder organischen Säure verestertem Hydroxy, wie mit einer Mineralsäure, z. B. Halogenwasserstoffsäure, wie Chlorwasserstoff-, Bromwasserstoff- oder Jodwasserstoffsäure, oder mit einer starken organischen Sulfonsäure, wie einer gegebenenfalls, z. B. durch Halogen, wie Fluor, substituierten Niederalkansulfonsäure oder einer aromatischen Sulfonsäure, z. B. einer gegebenenfalls durch Niederalkyl, wie Methyl, Halogen, wie Brom, und/oder Nitro substituierten Benzolsulfonsäure, z. B. einer Methansulfon-, p-Bromtoluolsulfonsäure oder p-Toluolsulfonsäure verestertes Hydroxy oder mit Stickstoffwasserstoffsäure verestertes Hydroxy.

Die Substitution kann unter den Bedingungen einer nukleophilen Substitution erster oder zweiter Ordnung ablaufen.

Beispielsweise kann man eine der Verbindungen der Formeln XII, worin X für eine Abgangsgruppe mit hoher Polarisierbarkeit der Elektronenhülle, z. B. für Jod, steht, in einem polaren aprotischen Lösungsmittel, z. B. Aceton, Acetonitril, Nitromethan, Dimethylsulfoxid oder Dimethylformamid, einsetzen. Die Reaktion kann auch in Wasser, dem gegebenenfalls als Lösungsvermittler ein organisches Lösungsmittel, z. B. Ethanol, Tetrahydrofuran oder Aceton, beigemischt ist durchgeführt werden. Die Substitutionsreaktion wird gegebenenfalls bei erniedrigter oder erhöhter Temperatur, z. B. in einem Temperaturbereich von etwa -40 ° bis etwa 100 °C, bevorzugt von etwa - 10 ° bis etwa 50 °C, und gegebenenfalls unter Inertgas, z. B. Stickstoff- oder Argonatmosphäre, durchgeführt.

Die Freisetzung von geschützten Gruppen erfolgt gegebenenfalls nach den unter Verfahren f) (Schutzgruppenabspaltung) beschriebenen Methoden.

### Verfahren f) (Schutzgruppenabspaltung)

Die Abspaltung der Schutzgruppen, die nicht Bestandteil des gewünschten Endproduktes der Formel I sind, z. B. der Carboxy-, Amino-, Hydroxy-, Mercapto- und/oder Sulfoschutzgruppen, erfolgt in an sich bekannter Weise, z. B. mittels Solvolyse, insbesondere Hydrolyse, Alkoholyse oder Acidolyse, oder mittels Reduktion, insbesondere Hydrogenolyse oder chemischer Reduktion, sowie Photolyse, gegebenenfalls stufenweise oder gleichzeitig, wobei auch enzymatische Methoden verwendet werden können. Die Abspal-tung der Schutzgruppen ist beispielsweise in den weiter vorn im Abschnitt über "Schutzgruppen" genannten Standardwerken beschrieben.

So kann beispielsweise geschütztes Carboxy, z. B. tert-Niederalkoxycarbonyl, in 2-Stellung durch eine trisubstituierte Silylgruppe oder in 1-Stellung durch Niederalkoxy oder Niederalkylthio substituiertes Niederalkoxycarbonyl oder gegebenenfalls substituiertes Diphenylmethoxycarbonyl durch Behandeln mit einer geeigneten Säure, wie Ameisensäure, Chlorwasserstoff oder Trifluoressigsäure, gegebenenfalls unter Zugabe einer nukleophilen Verbindung, wie Phenol oder Anisol, in freies Carboxy überführt werden. Aus Niederalkoxycarbonyl kann auch durch Basen, wie Hydroxide, z. B. Alkalimetallhydroxide, wie NaOH oder KOH, Carboxy freigesetzt werden. Gegebenenfalls substituiertes Benzyloxycarbonyl kann z. B. mittels Hydrogenolyse, d.h. durch Behandeln mit Wasserstoff in Gegenwart eines metallischen Hydrierkatalysators, wie eines Palladium-katalysators, freigesetzt werden. Ferner kann geeignet substituiertes Benzyloxycarbonyl, wie 4-Nitrobenzyloxycarbonyl, auch durch Reduktion, z. B. durch Behandeln mit einem Alkalimetall, wie Natrium-dithionit, oder mit einem reduzierenden Metall, z. B. Zink, oder einem reduzierenden Metallsalz, wie einem Chrom-II-salz, z. B. Chrom-II-chlorid, üblicherweise in Gegenwart eines Wasserstoff-abgebenden Mittels, das zusammen mit dem Metall nascierenden Wasserstoff erzeugen kann, wie einer Säure, in erster Linie einer geeigneten Carbonsäure, wie einer gegebenenfalls, z. B. durch Hydroxy, substituierten Niederalkancarbonsäure, z. B. Essigsäure, Ameisensäure, Glycolsäure, Diphenylglycolsäure, Milchsäure, Mandelsäure, 4-Chlormandelsäure oder Weinsäure, oder eines Alkohols oder Thiols, wobei man vorzugsweise Wasser zugibt, in freies Carboxy überführt werden. Durch Behandeln mit einem reduzierenden Metall oder Metallsalz, wie oben beschrieben, kann auch 2-Halogenniederalkoxycarbonyl (gegebenenfalls nach Umwandlung einer 2-Bromniederalkoxycarbonylgruppe in eine entsprechende 2-Iodnieder-alkoxycarbonylgruppe) oder Aroylmethoxycarbonyl in freies Carboxy umgewandelt werden. Aroylmethoxycarbonyl kann ebenfalls durch Behandeln mit einem nukleophilen, vorzugsweise salzbildenden Reagens, wie Natriumthiophenolat oder Natriumjodid, gespalten werden. 2-(trisubstituiertes Silyl)-niederalkoxycarbonyl, wie 2-Triniederalkylsilylniederalkoxycarbonyl, kann auch durch Behandeln mit einem das Fluoridanion liefernden Salz der Fluorwasserstoffsäure, wie einem Alkalïmetallfluorid, z. B. Natrium-oder Kaliumfluorid, gegebenenfalls in Anwesenheit eines macrocyclischen Polyethers ("Kronenether"), oder mit einem Fluorid einer organischen quaternären Base, wie Tetraniederalkylammoniumfluorid oder Triniederalkylarylniederalkylammoniumfluorid, z. B. Tetraethylammoniumfluorid oder Tetrabutylammoniumfluorid, in Gegenwart eines aprotischen, polaren Lösungsmittels, wie Dimethylsulfoxid oder N,N-Dimethylacetamid, in freies Carboxy überführt werden. Als organisches Silyloxycarbonyl, wie Triniederalkyl-silyloxycarbonyl, z. B. Trimethylsilyloxycarbonyl, geschütztes Carboxy kann in üblicher Weise solvolytisch, z. B. durch Behandeln mit Wasser, einem Alkohol oder Säure, oder ausserdem Fluorid, wie oben beschrieben, freigesetzt werden. Verestertes Carboxy kann auch enzymatisch freigesetzt werden, beispielsweise durch Esterasen oder geeignete Peptidasen, z. B. verestertes Arginin oder Lysin, wie Lysinmethylester, mittels Trypsin.

Eine geschützte Aminogruppe setzt man in an sich bekannter und je nach Art der Schutzgruppen in verschiedenartiger Weise, vorzugsweise mittels Solvolyse oder Reduktion, frei. Niederalkoxycarbonylamino, wie tert-Butoxycarbonylamino, kann in Gegenwart von Säuren, beispielsweise Mineralsäuren, z. B. Halogenwasserstoff, wie Chlorwasserstoff oder Bromwasserstoff, oder von Schwefel- oder oder Phosphorsäure, vorzugsweise von Chlorwasserstoff, oder von starken organischen Säuren, wie Trihalogenessigsäure, z. B. Trifluoressigsäure, oder Ameisensäure, in polaren Lösungsmitteln, wie Wasser, oder Ethern, bevorzugt cyclischen Ethern, wie Dioxan, 2-Halogenniederalkoxycarbonylamino (gegebenenfalls nach Umwandlung einer 2-Bromniederalkoxycarbonylaminogruppe in eine 2-Iodniederalkoxycarbonylaminogruppe), oder direkt gelöst in einer flüssigen organischen Carbonsäure, wie Ameisensäure, Aroylmethoxycarbonylamino oder 4-Nitrobenzyloxycarbonylamino kann z. B. durch Behandeln mit einem geeigneten Reduktionsmittel, wie Zink in Gegenwart einer geeigneten Carbonsäure, wie wässriger Essigsäure, gespalten werden. Aroylmethoxycarbonylamino kann auch durch Behandeln mit einem nucleophilen, vorzugsweise salzbildenden Reagenz, wie Natriumthiophenolat, und 4-Nitrobenzyloxycarbonylamino auch durch Behandeln mit einem Alkalimetall-, z. B. Natrium-dithionit, gespalten werden. Gegebenenfalls substituiertes Diphenylmethoxycarbonylamino, tertNiederalkoxycarbonylamino oder 2-(trisubstituiertes Silyl)-niederalkoxycarbonylamino, wie 2-Triniederalkylsilylniederalkoxycarbonylamino, kann durch Behandeln mit einer geeigneten Säure, z. B. Ameisen- oder Trifluoressigsäure, gegebenenfalls substituiertes Benzyloxycarbonylamino z. B. mittels Hydrogenolyse, d.h. durch Behandeln mit Wasserstoff in Gegenwart eines geeigneten Hydrierkatalysators, wie eines Platin- oder Palladium-katalysators, gegebenenfalls substituiertes Triarylmethylamino oder Formylamino z. B. durch Behandeln mit einer Säure, wie Mineralsäure, z. B. Chlorwasserstoffsäure, oder einer organischen Säure, z. B. Ameisen-, Essig- oder Trifluoressigsäure, gegebenenfalls in Gegenwart von Wasser, und eine als Silylamino geschützten Aminogruppe z. B. mittels Hydrolyse oder Alkoholyse freigesetzt werden. Eine durch 2-Halogenacetyl, z. B. 2-Chloracetyl, geschützte Aminogruppe kann durch Behandeln mit Thioharnstoff in Gegenwart einer Base, oder mit einem Thiolatsalz, wie einem Alkalimetallthiolat des Thioharnstoffs, und anschliessende Solvolyse, wie Alkoholyse oder Hydrolyse, des entstandenen Substitutionssproduktes freigesetzt werden, aus Trifluoracetylamino wird beispielsweise durch Hydrogenolyse mit Basen, wie Alkalimetallhydroxiden oder -carbonaten, wie Na₂CO₃ oder K₂CO₃, in polaren Lösungsmitteln, z. B. Alkoholen, wie Methanol, bei Temperaturen zwischen 0 und 100 °C, insbesondere bei 40 bis 80 °C, Amino freigesetzt. Eine durch 2-(trisubstituiertes Silyl)-niederalkoxycarbonyl, wie 2-Triniederalkylsilylniederalkoxycarbonyl, geschützte Aminogruppe kann auch durch Behandeln mit einem Fluoridanionen liefernden Salz der Fluorwasserstoffsäure, wie oben im Zusammenhang mit der Freisetzung einer entsprechend geschützten Carboxygruppe angegeben, in die freie Aminogruppe überführt werden. Ebenso kann man direkt an ein Heteroatom, wie Stickstoff, gebundenes Silyl, wie Trimethylsilyl, mittels Fluoridionen abspalten.

In Form einer Azidogruppe geschütztes Amino wird z. B. durch Reduktion in freies Amino überführt, beispielsweise durch katalytische Hydrierung mit Wasserstoff in Gegenwart eines Hydrierkatalysators, wie Platinoxid, Palladium oder Raney-Nickel, durch Reduktion mittels Mercaptoverbindungen, wie Dithiothreitol oder Mercaptoethanol, oder auch durch Behandeln mit Zink in Gegenwart einer Säure, wie Essigsäure. Die katalytische Hydrierung wird vorzugsweise in einem inerten Lösungsmittel, wie einem halogenierten Kohlenwasserstoff, z. B. Methylenchlorid, oder auch in Wasser oder einem Gemisch von Wasser und einem organischen Lösungsmittel, wie einem Alkohol oder Dioxan, bei etwa 20 °C bis 25 °C, oder auch unter Kühlen oder Erwärmen, durchgeführt.

Eine durch eine geeignete Acylgruppe, eine Triniederalkylsilylgruppe oder durch gegebenenfalls substituiertes 1-Phenylniederalkyl geschützte Hydroxy- oder Mercaptogruppe wird analog einer entsprechend geschützten Aminogruppe freigesetzt. Eine durch 2,2-Dichloracetyl geschützte Hydroxy- bzw. Mercaptogruppe wird z. B. durch basische Hydrolyse, eine durch tert-Niederalkyl oder durch einen 2-oxa- oder 2-thia-aliphatischen oder -cycloaliphatischen Kohlenwasserstoffrest geschützte Hydroxy- bzw. Mercaptogruppe durch Acidolyse, z. B. durch Behandeln mit einer Mineralsäure oder einer starken Carbonsäure, z. B. Trifluoressigsäure, freigesetzt. Durch Pyridyldiphenylmethyl geschütztes Mercapto kann z. B. durch Quecksilber-II-salze bei pH 2-6 oder durch Zink/Essigsäure oder elektrolytische Reduktion, Acetamidomethyl und iso-Butyrylamidomethyl z. B. durch Reaktion mit Quecksilber-II-salzen bei pH 2-6, 2-Chloracetamidomethyl z. B. durch 1-Piperidinothiocarboxamid, S-Ethylthio, S-tert-Butylthio und S-Sulfo z. B. durch Thio-lyse mit Thiophenol, Thioglycolsäure, Natrium-thiophenolat oder 1,4-Dithiothreitol frei-gesetzt werden. Zwei Hydroxygruppen oder eine benachbarte Amino- und Hydroxygrup-pe, die zusammen mittels einer bivalenten Schutzgruppe, vorzugsweise z. B. einer durch Niederalkyl ein- oder zweifach substituierten Methylengruppe, wie durch Niederalkyliden, z. B. Isopropyliden, Cycloyalkyliden, z. B. Cyclohexyliden, oder Benzyliden, geschützt sind, können durch saure Solvolyse, besonders in Gegenwart einer Mineralsäure oder einer starken organischen Säure, freigesetzt werden. Eine Triniederalkylsilylgruppe wird ebenfalls durch Acidolyse, z. B. durch Mineralsäure, bevorzugt Fluorwasserstoffsäure, oder eine starke Carbonsäure abgespalten. 2-Halogenniederalkoxycarbonyl wird durch die oben genannten Reduktionsmittel, z. B. reduzierendes Metall, wie Zink, reduzierende Metall-salze, wie Chrom-II-salze, oder durch Schwefelverbindungen, beispielsweise Natrium-dithionit oder bevorzugt Natriumsulfid und Schwefelkohlenstoff, entfernt.

Eine als Sulfonsäureester oder Sulfonamid geschützte Sulfogruppe wird beispielsweise durch saure Hydrolyse, z. B. in Gegenwart von Mineralsäure, oder bevorzugt durch basische Hydrolyse, z. B. mit Alkalimetallhydroxid oder Alkalimetallcarbonat, bei-spielsweise Natriumcarbonat, freigesetzt.

Beim Vorhandensein von mehreren geschützten funktionellen Gruppen können, wenn er-wünscht, die Schutzgruppen so gewählt werden, dass gleichzeitig mehr als eine solche Gruppe abgespalten werden kann, beispielsweise acidolytisch, wie durch Behandeln mit Trifluoressigsäure, oder mit Wasserstoff und einem Hydrierkatalysator, wie einem Palladium-Kohle-Katalysator. Umgekehrt können die Gruppen auch so gewählt werden, dass sie nicht alle gleichzeitig, sondern in gewünschter Reihenfolge abgespalten werden können, wobei die entsprechenden Zwischenprodukte erhalten werden.

### Zusätzliche Verfahrensmassnahmen

Bei den zusätzlichen Verfahrensmassnahmen, die gewünschtenfalls durchgeführt werden, können funktionelle Gruppen der Ausgangsverbindungen, die nicht an der Reaktion teilnehmen sollen, ungeschützt oder in geschützter Form, beispielsweise durch eine oder mehrere der oben unter Verfahren a) genannten Schutzgruppen, vorliegen. Die Schutzgruppen können in den Endprodukten erhalten bleiben oder ganz oder teilweise nach einer der unter Verfahren f) genannten Methoden abgespalten werden.

Salze von Verbindungen der Formel I mit mindestens einer salzbildenden Gruppe können in an sich bekannter Weise hergestellt werden. So kann man Salze von Verbindungen der Formel I mit sauren Gruppen z. B. durch Behandeln mit Metallverbindungen, wie Alkali-metallsalzen von geeigneten organischen Carbonsäuren, z. B. dem Natriumsalz der 2-Ethyl-hexansäure, mit organischen Alkali- oder Erdalimetallverbindungen, wie den entsprechenden Hydroxiden, Carbonaten oder Hydrogencarbonaten, wie Natrium-und Ka-liumhydroxid, -carbonat oder -hydrogencarbonat, mit entsprechenden Calciumverbindun-gen oder mit Ammoniak oder einem geeigneten organischen Amin bilden, wobei man vor-zugsweise stöchiometrische Mengen oder nur einen kleinen Überschuss des salzbildenden Mittels verwendet. Säureadditionssalze von Verbindungen der Formel I erhält man in übli-cher Weise, z. B. durch Behandeln mit einer Säure oder einem geeigneten Anionenaustau-scherreagenz. Innere Salze von Verbindungen der Formel I, welche saure und basische salzbildende Gruppen enthalten, z. B. eine freie Carboxygruppe und eine freie Amino-gruppe, können z.B. durch Neutralisieren von Salzen, wie Säureadditionssalzen, auf den isoelektrischen Punkt, z. B. mit schwachen Basen, oder durch Behandeln mit Ionenaustau-schern gebildet werden.

Salze können in üblicher Weise in die freien Verbindungen überführt werden, Metall-und Ammoniumsalze beispielsweise durch Behandeln mit geeigneten Säuren, und Säureadditionssalze beispielsweise durch Behandeln mit einem geeigneten basischen Mittel.

Stereoisomerengemische, also Gemische von Diastereomeren und/oder Enantiomeren, wie beispielsweise racemische Gemische, können in an sich bekannter Weise durch geeignete Trennverfahren in die entsprechenden Isomeren aufgetrennt werden. So können Diastereo-merengemische durch fraktionierte Kristallisation, Chromatographie, Lösungsmittelverteilung etc. in die einzelnen Diastereomeren aufgetrennt werden. Racemate können nach Überführung der optischen Antipoden in Diastereomere, beispielsweise durch Umsetzung mit optisch aktiven Verbindungen, z. B. optisch aktiven Säuren oder Basen, durch Chromatographie an mit optisch aktiven Verbindungen belegten Säulenmaterialien oder durch enzymatische Methoden, z. B. durch selektive Umsetzung nur eines der beiden Enantiomeren, voneinander getrennt werden. Diese Trennung kann sowohl auf der Stufe eines der Ausgangsprodukte als auch bei den Verbindungen der Formel I selbst erfolgen.

An einzelnen Chiralitätszentren in einer Verbindung der Formel I kann die Konfiguration gezielt umgekehrt werden. Beispielsweise kann man die Konfiguration asymmetrischer Kohlenstoffatome, welche nukleophile Substituenten, wie Amino oder Hydroxy, tragen, durch nukleophile Substitution zweiter Ordnung, gegebenenfalls nach Überführung des gebundenen nukleophilen Substituenten in eine geeignete nucleofuge Abgangsgruppe und Reaktion mit einem den ursprünglichen Substituenten einführenden Reagenz, umkehren, oder man kann die Konfiguration an Kohlenstoffatomen mit Hydroxygruppen, wie das R₅ tragende Kohlenstoffatom der Formel I, durch Oxidation und Reduktion von Verbindungen der Formel I, wie unten beschrieben, umkehren.

Die Reste Hydroxy R₅ und Wasserstoff R₆ in einer Verbindung der Formel I können zu einer Oxogruppe oxidiert werden, wobei vorzugsweise solche Oxidationsmittel verwendet werden, welche selektiv die Hydroxy- in eine Ketogruppe umwandeln, beispielsweise Chromsäure oder ihre Derivate, wie Pyridiniumchromat oder tert-Butylchromat, Dichromat/Schwefelsäure, Schwefeltrioxid in Gegenwart von heterocyclischen Basen, wie Pyridin/SO₃, ferner Salpetersäure, Braunstein oder Selendioxid, *oder Dimethylsulfoxid in Gegenwart von Oxalylchlorid*, in Wasser, wässrigen oder organischen Lösungsmitteln, wie halogenierten Lösungsmitteln, z. B. Methylenchlorid, Carbonsäureamiden, wie Dimethylformamid, oder Diniederalkylsulfoxiden, wie Dimethylsulfoxid, in Gegenwart oder Abwesenheit von basischen Aminen, z. B. Triniederalkylaminen, wie Triethylamin, bei Temperaturen zwischen -50 und 100 °C, vorzugsweise bei -10 bis 50 °C, beispielsweise wie in der Europäischen Patentanmeldung EP-A-0 236 734 beschrieben.

Umgekehrt kann in den so erhaltenen Verbindungen der Formel I, in denen R₅ und R₆ zu-sammen eine Oxogruppe bilden, die Oxo- zu einer Hydroxygruppe reduziert werden. Zur Reduktion der Oxogruppe in einer Verbindung der Formel I eignen sich Reduktionsmittel, die unter den Reaktionsbedingungen des Verfahrens eine isolierte Ketogruppe selektiv oder schneller als in Verbindungen der Formel I vorhandene Amidgruppen reduzieren.

In erster Linie zu nennen sind geeignete Borhydride, wie Alkalimetallborhydride, insbesondere Natriumborhydrid, Lithiumborhydrid oder Natriumcyanborhydrid, ferner Zinkborhydrid, oder geeignete Aluminiumhydride, wie Alkalimetallniederalkoxyaluminiumhydride mit voluminösen Resten, z.B. Lithium-tris-tert-butoxyaluminiumhydrid.

Die Reduktion kann auch mit Wasserstoff in Gegenwart geeigneter Schwermetallkatalysatoren, z.B. Raney-Nickel oder Platin- oder Palladiumkatalysatoren, z.B. Platin-oder Palladium-Aktivkohle, oder nach Meerwein-Ponndorf-Verley mit Hilfe von Aluminiumalkanolaten, bevorzugt Aluminium-2-propanolat oder -ethanolat, durchgeführt werden.

Die Reduktion kann vorzugsweise mit stöchiometrischen Mengen oder einem sinnvoll bemessenen Ueberschuss des Reduktionsmittels in einem inerten Lösungsmittel bei Temperaturen zwischen -80°C und dem Siedepunkt des Lösungsmittels, z. B. zwischen -20°C und +100°C, wenn nötig unter Schutzgas, z.B. Stickstoff oder Argon, durchgeführt werden. Ein Ueberschuss des Reduktionsmittels ist insbesondere dann nötig, wenn dieses auch mit dem Lösungsmittel, z.B. den Protonen eines protischen Lösungsmittels, reagiert.

Bei Verwendung von Natriumborhydrid eignen sich polare, protische Lösungsmittel, z.B Methanol, Ethanol oder Isopropanol; bei Verwendung der anderen Reduktionsmittel polare, aprotische Lösungsmittel, z.B. Tetrahydrofuran.

In einer Verbindung der Formel I, in welcher R₁, R₂, R₈ und R₉ keine oder nicht sehr reaktive Arylreste enthalten, kann man einen in R₇, R₃ und/oder R₄ vorhandenen Arylrest, insbesondere einen Phenylrest, hydrieren, beispielsweise durch katalytische Hydrierung, insbesondere in Gegenwart von Schwermetalloxiden, wie Rhodium/Platin-Mischoxiden, z. B. mit dem Nishimura-Katalysator, vorzugsweise in einem polaren Lösungsmittel, wie einem Alkohol, z. B. Methanol oder Ethanol, bei Temperaturen zwischen 0 und 80 °C, insbesondere zwischen 10 und 40 °C, und bei einem Wasserstoffdruck von I bis 10 atm, vorzugsweise etwa bei Normaldruck.

In einer erhältlichen Verbindung der Formel I kann man eine Amino- oder Carboxamid-gruppe substituieren, eine in freier oder in reaktionsfähiger Form vorliegende Carboxygruppe verestern oder amidieren bzw. eine veresterte oder amidierte Carboxygruppe in eine freie Carboxygruppe überführen.

Die Substitution einer Carboxamidgruppe oder einer anderen primären oder sekundären Aminogruppe, beispielsweise zur Einführung von Resten wie unsubsbtuiertem oder substituiertem Alkyl, Alkenyl oder Alkinyl, Arylniederalkyl, über Kohlenstoff gebundenes Heterocyclyl oder Heterocyclylniederalkyl R₁, R₂, R₈ oder R₉ in Verbindungen der Formel 1, in denen einer oder mehrere der genannten Reste Wasserstoff bedeuten, erfolgt z. B. durch Alkylierung.

Geeignete Mittel zur Alkylierung einer Carboxamidgruppe in einer Verbindung der Formel I sind z. B. Diazoverbindungen, z. B. Diazomethan. Man kann Diazomethan in einem inerten Lösungsmittel zersetzen, wobei das gebildete freie Methylen mit der Carboxamidgruppe in der Verbindung der Formel I reagiert. Die Zersetzung von Diazomethan erfolgt vorzugsweise katalytisch, z. B. in Gegenwart eines Edelmetalls in fein verteilter Form, z. B. Kupfer, oder eines Edelmetallsalzes, z. B. Kupfer(I)-chlorid oder Kupfer(II)-sulfat.

Alkylierungsmittel sind auch in der Deutschen Offenlegungsschrift 2 331133 genannt, z. B. Alkylhalogenide, Sulfonsäureester, Meerweinsalze oder 1-substituierte 3-Aryltriazene, welche man unter den dort genannten Reaktionsbedindungen mit einer Verbindung der Formel I mit einer Carboxamidgruppe umsetzen kann.

Weitere Alkylierungsmittel sind ausgewählt aus Verbindungen der Formeln

R₁-X (X),

R₂-X (XI),

R₈-X (XIII) und

R₉-X (XIV),

worin X eine Abgangsgruppe ist und die übrigen Reste die genannten Bedeutungen ausser Acyl, unsubstituiertes oder wie oben substituiertes Sulfo, Phosphono oder wie oben substituiertes Phosphoryl haben. Eine Abgangsgruppe ist insbesondere eine nukleofuge Abgangsgruppe ausgewählt aus mit einer starken anorganischen oder organischen Säure verestertem Hydroxy, wie mit einer Mineralsäure, z. B. Halogenwasserstoffsäure, wie Chlorwasserstoff-, Bromwasserstoff- oder Jodwasserstoffsäure, oder mit einer starken organischen Sulfonsäure, wie einer gegebenenfalls, z. B. durch Halogen, wie Fluor, substituierten Niederalkansulfonsäure oder einer aromatischen Sulfonsäure, z. B. einer gegebenenfalls durch Niederalkyl, wie Methyl, Halogen, wie Brom, und/oder Nitro substituierten Benzolsulfonsäure, z. B. einer Methansulfon-, Trimethansulfon- oder p-Toluolsulfonsäure verestertes Hydroxy oder mit Stickstoffwasserstoffsäure verestertes Hydroxy.

Die Reaktion kann unter den Bedingungen einer nukleophilen Substitution erster oder zweiter Ordnung ablaufen.

Beispielsweise kann man eine der Verbindungen der Formeln VIII bis XIII, worin X für eine Abgangsgruppe mit hoher Polarisierbarkeit der Elektronenhülle, z. B. für Jod, steht, in einem polaren aprotischen Lösungsmittel, z. B. Aceton, Acetonitril, Nitromethan, Di-methylsulfoxid oder Dimethylformamid einsetzen. Die Reaktion kann auch in Wasser, dem gegebenenfalls als Lösungsvermittler ein organisches Lösungsmittel, z. B. Ethanol, Tetrahydrofuran oder Aceton, beigemischt ist, durchgeführt werden. Die Substitutionsreaktion wird gegebenenfalls bei erniedrigter oder erhöhter Temperatur, z. B. in einem Temperaturbereich von etwa -40 ° bis etwa 100 °C, bevorzugt von etwa - 10 ° bis etwa 50 °C, und gegebenenfalls unter Inertgas, z. B. Stickstoff- oder Argonatmosphäre, durchgeführt.

Zur Veresterung oder Amidierung einer Carboxygruppe in einer Verbindung der Formel I kann man, wenn erwünscht, die freie Säure verwenden oder die freie Säure in eines der oben genannten reaktionsfähigen Derivate überführen und mit einem Alkohol, Ammoniak, einem primären oder einem sekundären Amin umsetzen, oder man kann zur Veresterung die freie Säure oder ein reaktionsfähiges Salz, z. B. das Cäsiumsalz, mit einem reaktionsfähigen Derivat eines Alkohols umsetzen. Beispielsweise kann man das Cäsiumsalz einer Carbonsäure mit einem dem Alkohol entsprechenden Halogenid oder Sulfonsäureester umsetzen. Die Veresterung der Carboxygruppe kann auch mit anderen üblichen Alkylierungsmitteln erfolgen, z. B. mit Diazomethan, Alkylhalogeniden, Sulfonsäureestern, Meerweinsalzen oder 1-substituierten 3-Aryltriazenen, etc.

Zur Uberführung einer veresterten oder amidierten Carboxygruppe in die freie Carboxygruppe kann eine der oben bei der Abspaltung der Carboxyschutzgruppen beschriebenen Methoden oder gewünschtenfalls eine alkalische Verseifung nach üblichen, wie den im Organikum, 17. Auflage, VEB Deutscher Verlag der Wissenschaften, Berlin 1988, genannten Reaktionsbedingungen angewendet werden.

In einer Verbindung der Formel I kann man eine veresterte Carboxygruppe durch Aminolyse mit Ammoniak oder einem primären oder sekundären Amin in eine gegebenenfalls substituierte Carboxamidgruppe überführen. Die Aminolyse kann nach den üblichen, wie den im Organikum, 15. Auflage, VEB Deutscher Verlag der Wissenschaften, Berlin (Ost) 1976, für derartige Umsetzungen genannten Reaktionsbedingungen erfolgen.

In einer Verbindung der Formel I kann man eine vorhandene freie Aminogruppe acylieren, beispielsweise zur Einführung eines der für R₁, R₂, R₈ oder R₉ genannten Reste Acyl, Sulfo, substituiertes Sulfonyl, Phosphono oder substituiertes Phosphoryl. Die Acylierung erfolgt nach einer der oben unter Verfahren b), c) oder d) für Kondensationen oder einer der für Schutzgruppen genannten Methoden oder beispielsweise nach einem der im Organikum, 17. Auflage, VEB Deutscher Verlag der Wissenschaften, Berlin (Ost) 1988, genannten Verfahren.

In einer erhältlichen Verbindung der Formel I, worin die Substituenten die genannten Bedeutungen haben und mindestens eine freie Hydroxygruppe vorhanden ist und die übrigen funktionellen Gruppen in geschützter Form vorliegen, kann man die freie Hydroxygruppe acylieren oder verethern.

Die Acylierung kann erfolgen mit acylierenden Reagentien nach einer der unter Verfahren b) bis d), nach einer der für Schutzgruppen genannten Methoden oder nach einem der im Organikum, 17. Auflage, VEB Deutscher Verlag der Wissenschaften, Berlin (Ost) 1988, genannten Verfahren.

Die Veretherung kann mit den oben genannten Alkylierungsmitteln und unter den gleichen Reaktionsbedingungen erfolgen, z. B. mit Diazomethan, Alkylhalogeniden, Sulfonsäureestem, Meerweinsalzen, 1-substituierten 3-Aryltriazenen, etc.

In einer erhältlichen Verbindung der Formel I kann man aus einer Thiogruppe durch Oxidation eine Sulfinyl- oder Sulfonylgruppe herstellen, aus einem Sulfid das entsprechende Sulfoxid oder Sulfon.

Die Oxidation zur Sulfonylgruppe oder zum Sulfon kann mit den meisten der üblichen Oxidationsmittel durchgeführt werden. Besonders bevorzugt verwendet man solche Oxidationsmittel, welche die Thiogruppe oder den Sulfidschwefel selektiv in Gegenwart anderer funktioneller Gruppen der jeweiligen Verbindung der Formel I, z. B. von Amino-oder Hydroxygruppen, oxidieren, beispielsweise aromatische oder aliphatische Peroxycar-bonsäuren, z. B. Peroxybenzoesäure, Monoperphthalsäure, m-Chlorperbenzoesäure, Peressigsäure, Perameisensäure oder Trifluorperessigsäure. Die Oxidation mit Peroxycarbon-säuren erfolgt in den üblichen dafür geeigneten Lösungsmitteln, beispielsweise Chlorkoh-lenwasserstoffen, z. B. Methylenchlorid oder Chloroform, Ethern, wie Diethylether, Estern, wie Essigester oder dergleichen, bei Temperaturen zwischen -78 °C und Raumtemperatur, z. B. zwischen -20 °C und +10 °C, bevorzugt um 0 °C. Die Peroxycarbonsäure kann auch in situ gebildet werden, z. B. mit Wasserstoffperoxid in Essigsäure oder Ameisensäure, die gegebenenfalls Essigsäureanhydrid enthält, z. B. mit 30 % oder 90 % Wasserstoffperoxid in Essigsäure/Essigsäureanhydrid. Geeignet sind auch andere Peroxoverbindungen, beispielsweise Kaliumperoxomonosulfat in Niederalkanol/Wasser-Mischungen, z. B. Methanol/Wasser oder Ethanol/Wasser, oder in wässriger Essigsäure bei Temperaturen zwischen -70 °C und +30 °C, z. B. zwischen -20 °C und Raumtemperatur, ferner Natriummetaperjodat in Methanol oder Methanol/Wasser-Gemischen bei Temperaturen zwischen 0 °C und 50 °C, z. B. um Raumtemperatur. Bei Einsatz stöchiometrischer Mengen der genannten Oxidationsmittel können auch die entsprechenden Sulfinsäuren bzw. Sulfoxide erhalten werden. Geeignet sind hierfür beispielsweise Natriummetaperiodat in Methanol oder Methanol-Wasser-Gemischen bei Temperaturen zwischen -15 °C und Raumtemperatur, z. B. um 0°C, m-Chlorperbenzoesäure in Methylenchlorid, Chloroform oder Essigester bei Temperaturen zwischen -78°C und 10°C, bevorzugt zwischen - 30°C und 0°C, ferner tert-Butylhypochlorit in Niederalkanolen, z. B. Methanol, oder Wasserstoffperoxid in Aceton oder Essigsäure bei Temperaturen um 0°C, oder das oben genannte Kaliumperoxomonosulfat bei tiefen Temperaturen.

Gewünschtenfalls kann durch Reduktion einer Sulfonylgruppe oder eines Sulfonrestes in einer erhältlichen Verbindung der Formel I die entsprechende Thioverbindung oder das entsprechende Sulfid erhalten werden, beispielsweise mit Diisobutylaluminiumhydrid in Ether oder Tetrahydrofuran.

In einer erhältlichen Verbindung der Formel I mit einer Sulfinylgruppe kann man diese Gruppe zu einer Thiogruppe reduzieren. Bevorzugt sind selektive Reduktionsmittel, die andere funktionelle Gruppen der Verbindung der Formel I, z. B. die Amidfunktion, unverändert lassen. Beispiele für solche selektiven Reduktionsmittel sind Dichlorboran, das vorzugsweise in Tetrahydrofuran oder Dimethoxyethan bei Temperaturen zwischen - 30°C und +10°C eingesetzt wird, Triphenylphosphin in siedendem Tetrachlorkohlenstoff, Tri-chlorsilan oder Hexachlordisilan, Eisenpentacarbonyl, ferner Natriumhydrogensulfit in wässrig-alkoholischen Lösungsmitteln, z. B. Wasser-Methanol, Wasser-Ethanol oder auch Wasser-Tetrahydrofuran, bei Temperaturen zwischen -10°C und +50°C, femer Natriumborhydrid in Gegenwart von Kobalt(II)chlorid oder auch Wasserstoff in Gegenwart von katalytischen Mengen Palladium, z. B. Palladium/Kohle in siedendem Ethanol.

In einer Verbindung der Formel I kann man vorhandene Schutzgruppen oder geeignete Reste R₁, R₂, R₈ oder R₉, d. h. solche, die Acyl, Sulfo, substituiertes Sulfo, Phosphono oder substituiertes Phosphoryl bedeuten, nach einem der unter Verfahren,*f*) genannten Verfahren abspalten, insbesondere durch Hydrolyse, beispielsweise in Gegenwart von Basen, wie Alkali- oder Erdalkalihydroxiden, z. B. Natriumhydroxid, oder Säuren, wie organischen Säuren oder Mineralsäuren, z. B. Halogenwasserstoff, wie Chlorwasserstoff. Die Hydrolyse erfolgt unter den üblichen Bedingungen, beispielsweise in wässriger Lösung oder in wasserfreien Lösungsmitteln, insbesondere in Ethern, wie Dioxan, bei Temperaturen zwischen -50 °C und der Rückflusstemperatur der entsprechenden Reaktionsgemische, z. B. zwischen 0 °C und 50 °C, vorzugsweise in Gegenwart eines Schutzgases, wie Argon oder Stickstoff.

Alle vorstehend angeführten Verfahrensschritte können unter an sich bekannten, vorzugsweise den spezifisch genannten, Reaktionsbedingungen durchgeführt werden, in Ab- oder üblicherweise Anwesenheit von Lösungs- oder Verdünnungsmitteln, vorzugsweise solchen, die gegenüber den verwendeten Reagenzien inert sind und diese lösen, in Ab- oder Anwesenheit von Katalysatoren, Kondensationsmitteln oder neutralisierenden Agentien, z. B. Ionenaustauschern, wie Kationenaustauschern, z. B. in der H⁺-Form, je nach Art der Reaktion und/oder der Reaktionsteilnehmer bei erniedrigter, normaler oder erhöhter Temperatur, z.B. im Temperaturbereich von etwa -100°C bis etwa 190°C, vorzugsweise von etwa -80°C bis etwa 150°C, z.B. bei - 80 bis -60 °C, bei Raumtemperatur, bei - 20 bis 40 °C oder beim Siedepunkt des verwendeten Lösungsmittels, unter atmosphärischem Druck oder in einem geschlossenen Gefäss, gegebenenfalls unter Druck, und/oder in einer inerten Atmosphäre, z.B. unter einer Argon-oder Stickstoffatmosphäre.

Bei allen Ausgangs- und Zwischenverbindungen können, sofern salzbildende Gruppen vorliegen, Salze vorliegen. Salze können auch während der Umsetzung derartiger Verbindungen vorliegen, sofern dadurch die Reaktion nicht gestört wird.

Auf allen Reaktionsstufen können auftretende Isomerengemische in die einzelnen Isomeren, z. B. Diastereomeren oder Enantiomeren, oder in beliebige Gemische von Isomeren, z. B. Racemate oder Diastereomerengemische, aufgetrennt werden, beispielsweise analog zu den Methoden, die unter den "Zusätzlichen Verfahrensmassnahmen" beschrieben sind.

In bestimmten Fällen, beispielsweise bei Hydrierungen, ist es möglich, stereoselektive Reaktionen zu erzielen, so dass z. B. eine erleichterte Gewinnung von einzelnen Isomeren möglich ist.

Zu den Lösungsmitteln, aus denen die für die jeweilige Reaktion geeigneten ausgewählt werden können, zählen beispielsweise Wasser, Ester, wie Niederalkylniederalkanoate, z. B. Essigsäurediethylester, Ether, wie aliphatische Ether, z. B. Diethylether, oder cyclische Ether, z. B. Tetrahydrofuran, flüssige aromatische Kohlenwasserstoffe, wie Benzol oder Toluol, Alkohole, wie Methanol, Ethanol oder 1- oder 2-Propanol, Nitrile, wie Acetonitril, Halogenkohlenwasserstoffe, wie Methylenchlorid, Säureamide, wie Dimethylformamid, Basen, wie heterocyclische Stickstoffbasen, z. B. Pyridin, Carbonsäureanhydride, wie Niederalkansäureanhydride, z. B. Acetanhydrid, cyclische, lineare oder verzweigte Kohlenwasserstoffe, wie Cyclohexan, Hexan oder Isopentan, oder Gemische dieser Lösungsmittel, z. B. wässrige Lösungen, soweit bei der Beschreibung der Verfahren nichts anderes angegeben ist. Derartige Lösungsmittelgemische können auch bei der Aufarbeitung, beispielsweise durch Chromatographie oder Verteilung, Verwendung finden.

Die Erfindung betrifft auch diejenigen Ausführungsformen des Verfahrens, bei denen man von einer auf irgend einer Stufe als Zwischenprodukt erhältlichen Verbindung ausgeht und die fehlenden Schritte durchführt oder das Verfahren auf irgendeiner Stufe abbricht oder einen Ausgangsstoff unter den Reaktionsbedingungen bildet oder in Form eines reaktionsfähigen Derivates oder Salzes verwendet oder eine nach dem erfindungsgemässen Verfahren erhältliche Verbindung unter den Verfahrensbedingungen erzeugt und in situ weiterverarbeitet. Dabei geht man vorzugsweise von solchen Ausgangsstoffen aus, die zu den Verbindungen führen, die oben als bevorzugt, insbesondere als besonders bevorzugt, in erster Linie bevorzugt und/oder zuallererst bevorzugt beschrieben sind.

### Pharmazeutische Präparate:

Die Erfindung betrifft auch pharmazeutische Präparate enthaltend Verbindungen der Formel I.

Die pharmakologisch verwendbaren Verbindungen der vorliegenden Erfindung können z. B. zur Herstellung von pharmazeutischen Präparaten verwendet werden, welche eine wirksame Menge des Wirkstoffes zusammen oder im Gemisch mit einer signifikanten Menge von anorganischen oder organischen, festen oder flüssigen, pharmazeutisch verwendbaren Trägerstoffen enthalten.

Zur Erfindung gehört auch eine pharmazeutische Zusammensetzung (Präparat), die geeignet ist zur Verabreichung an einen Warmblüter, insbesondere Menschen, zur Behandlung oder Verhütung einer Erkrankung, die auf eine Hemmung einer retroviralen Protease, insbesondere einer retroviralen Aspartatprotease, wie HIV-I- oder HIV-II-gag-Protease, anspricht, z. B. einer retroviralen Erkrankung, wie AIDS, umfassend eine zur Hemmung der retroviralen Protease wirksame Menge einer Verbindung der Formel I oder eines pharmazeutisch annehmbaren Salzes davon, zusammen mit wenigstens einem pharmazeutisch annehmbaren Trägermaterial.

Bei den erfindungsgemässen pharmazeutischen Präparaten handelt es sich um solche zur enteralen, wie nasalen, rektalen oder oralen, oder parenteralen, wie intramuskulären oder intravenösen, Verabreichung an Warmblüter (Menschen und Tiere), welche eine effektive Dosis des pharmakologischen Wirkstoffs allein oder zusammen mit einer signifikanten Menge eines pharmazeutisch anwendbaren Trägermaterials enthalten. Die Dosierung des Wirkstoffs hängt von der Warmblüter-Spezies, dem Körpergewicht, dem Alter und dem individuellen Zustand, individuellen pharmakokinetischen Gegebenheiten, der zu behandelnden Krankheit sowie der Applikationsweise ab.

Die Erfindung betrifft auch eine Methode zur Behandlung von durch Viren, insbesondere Retroviren verursachten Krankheiten, z. B. von AIDS, dadurch gekennzeichnet, dass eine therapeutisch wirksame Menge von erfindungsgemässen Verbindungen der Formel I, insbesondere an einen Warmblüter, z. B. Menschen, der wegen einer der genannten Erkrankungen, insbesondere AIDS, einer derartigen Behandlung bedarf, verabreicht wird. Die an Warmblüter, z. B. Menschen von etwa 70 kg Körpergewicht, zu verabreichenden Dosismengen liegen zwischen etwa 3 mg und etwa 3 g, vorzugsweise zwischen etwa 10 mg und etwa 1,5 g, z. B. bei ungefähr 300 mg bis 1000 mg pro Person und Tag, verteilt auf vorzugsweise 1 bis 3 Einzeldosen, die z. B. gleich gross sein können. Ueblicherweise erhalten Kinder die halbe Dosis von Erwachsenen.

Die pharmazeutischen Präparate enthalten von etwa 1 % bis etwa 95 %, vorzugsweise von etwa 20 % bis etwa 90 % des Wirkstoffes. Erfindungsgemässe pharmazeutische Präparate können z. B. in Dosiseinheitsform, wie Ampullen, Vials, Suppositorien, Dragées, Tabletten oder Kapseln, vorliegen.

Die pharmazeutischen Präparate der vorliegenden Erfindung werden in an sich bekannter Weise, z. B. mittels konventioneller Lösungs-, Lyophilisierungs-, Misch-, Granulier- oder Dragierverfahren, hergestellt.

Vorzugsweise verwendet man Lösungen des Wirkstoffes, daneben auch Suspensionen, und zwar insbesondere isotonische wässrige Lösungen oder Suspensionen, wobei diese z. B. bei lyophilisierten Präparaten, welche die Wirksubstanz allein oder zusammen mit einem Trägermaterial, z. B. Mannit, enthalten, vor Gebrauch hergestellt werden können. Die pharmazeutischen Präparate können sterilisiert sein und/oder Hilfsstoffe, z. B. Konservier-, Stabilisier-, Netz- und/oder Emulgiermittel, Löslichkeitsvermittler, Salze zur Regu-lierung des osmotischen Druckes und/oder Puffer enthalten und werden in an sich bekannter Weise, z. B. mittels konventioneller Lösungs- oder Lyophilisierungsverfahren, herge-stellt. Die genannten Lösungen oder Suspensionen können viskositätserhöhende Stoffe, wie Natriumcarboxymethylcellulose, Carboxymethylcellulose, Dextran, Polyvinylpyrrolidon oder Gelatine, enthalten.

Suspensionen in Öl enthalten als ölige Komponente die für Injektionszwecke üblichen vegetabilen, synthetischen oder halbsynthetischen Öle. Als solche sind insbesondere flüssige Fettsäureester zu nennen, die als Säurekomponente eine langkettige Fettsäure mit 8-22, insbesondere 12-22, Kohlenstoffatomen, wie z. B. Laurinsäure, Tridecylsäure, Myristinsäure, Pentadecylsäure, Palmitinsäure, Margarinsäure, Stearinsäure, Arachidinsäure, Be-hensäure oder entsprechende ungesättigte Säuren, wie z. B. Ölsäure, Elaidinsäure, Eruca-säure, Brasidinsäure oder Linolsäure, enthalten, gegebenenfalls unter Zusatz von Antioxidantien, wie z. B. Vitamin E, β-Carotin oder 3,5-Di-tert-butyl-4-hydroxytoluol. Die Alko-holkomponente dieser Fettsäureester hat maximal 6 Kohlenstoffatome und ist ein ein- oder mehrwertiger, z. B. ein-, zwei- oder dreiwertiger Alkohol, z. B. Methanol, Ethanol, Pro-panol, Butanol oder Pentanol oder deren Isomere, vor allem aber Glycol und Glycerin. Als Fettsäureester sind daher beispielsweise zu nennen: Ethyloleat, Isopropylmyristat, Isopropylpalmitat, "Labrafil M 2375 " (Polyoxyethylenglycerintrioleat der Firma Gattefossé, Paris), "Miglyol 812" (Triglycerid gesättigter Fettsäuren der Kettenlänge C₈ bis C₁₂ der Firma Hüls AG, Deutschland), besonders aber vegetabile Öle wie Baumwollsaatöl, Mandelöl, Olivenöl, Ricinusöl, Sesamöl, Sojabohnenöl und vor allem Erdnussöl.

Die Herstellung der Injektionspräparate erfolgt in üblicher Weise unter sterilen Bedingun-gen, ebenso das Abfüllen in Ampullen oder Vialen sowie das Verschliessen der Behälter.

Pharmazeutische Präparate zur oralen Anwendung können erhalten werden, indem man den Wirkstoff mit festen Trägerstoffen kombiniert, ein erhaltenes Gemisch gegebenenfalls granuliert und das Gemisch, wenn erwünscht oder notwendig, nach Zugabe von geeig-neten Hilfsstoffen, zu Tabletten, Dragée-Kernen oder Kapseln verarbeitet. Dabei kann man sie auch in Kunststoffträger einbauen, die die Wirkstoffe dosiert abgeben oder diffundieren lassen.

Geeignete Trägerstoffe sind insbesondere Füllstoffe, wie Zucker, z. B. Lactose, Saccharose, Mannit oder Sorbit, Cellulosepräparate, und/oder Calciumphosphate, z. B. Tricalciumphosphat oder Calciumhydrogenphosphat, ferner Bindemittel, wie Stärkekleister unter Verwendung z. B. von Mais-, Weizen-, Reis- oder Kartoffelstärke, Gelatine, Traganth, Methylcellulose, Hydroxypropylmethylcellulose, Natriumcarboxymethylcellulose und/oder Polyvinylpyrrolidon, und/oder, wenn erwünscht, Sprengmittel, wie die oben genannten Stärken, ferner Carboxymethylstärke, quervernetztes Polyvinylpyrrolidon, Agar, Alginsäure oder ein Salz davon, wie Natriumalginat. Hilfsmittel sind in erster Linie Fliessregulier- und Schmiermittel, z. B. Kieselsäure, Talk, Stearinsäure oder Salze davon, wie Magnesium- oder Calciumstearat, und/oder Polyethylenglycol. Dragée-Kerne werden mit geeigneten, gegebenenfalls magensaftresistenten Überzügen versehen, wobei man u.a. konzentrierte Zuckerlösungen, welche gegebenenfalls arabischen Gummi, Talk, Poly-vinylpyrrolidon, Polyethylenglycol und/oder Titandioxid enthalten, Lacklösungen in geeigneten organischen Lösungsmitteln oder, zur Herstellung von magensaftresistenten Überzügen, Lösungen von geeigneten Cellulosepräparaten, wie Ethylcellulosephthalat oder Hydroxypropylmethylcellulosephthalat, verwendet. Kapseln sind Steckkapseln aus Gelatine sowie weiche, geschlossene Kapseln aus Gelatine und einem Weichmacher, wie Glycerin oder Sorbit. Die Steckkapseln können den Wirkstoff in Form eines Granulats, z. B. mit Füllstoffen, wie Lactose, Bindemitteln, wie Stärken, und/oder Gleitmitteln, wie Talk oder Magnesiumstearat, und gegebenenfalls mit Stabilisatoren, enthalten. In weichen Kapseln ist der Wirkstoff vorzugsweise in geeigneten öligen Hilfsstoffen, wie fetten Ölen, Paraffinöl oder flüssigen Polyethylenglykolen, gelöst oder suspendiert, wobei ebenfalls Stabilisatoren und/oder antibakterielle Mittel zugefügt sein können. Den Tabletten oder Dragée-Überzügen und den Kapselhüllen können Farbstoffe oder Pigmente, z. B. zur Identifizierung oder zur Kennzeichnung verschiedener Wirkstoffdosen, beigefügt werden.

### Ausgangsmaterialien:

Neue Ausgangsmaterialien und/oder Zwischenprodukte sowie Verfahren zu ihrer Herstellung sind ebenfalls Gegenstand der vorliegenden Erfindung. Vorzugsweise werden solche Ausgangsstoffe verwendet und die Reaktionsbedingungen so gewählt, dass man zu den als bevorzugt aufgeführten Verbindungen gelangt.

Bei der Herstellung aller Ausgangsmaterialien können freie funktionelle Gruppen, die nicht an der jeweiligen Reaktion teilnehmen sollen, ungeschützt oder in geschützter Form vorliegen, beispielsweise geschützt durch die oben unter Verfahren a) genannten Schutzgruppen. Diese Schutzgruppen können zu geeigneten Zeitpunkten durch die unter Verfahren f) beschriebenen Reaktionen freigesetzt werden.

Die Ausgangsmaterialien des Verfahrens a) sind bekannt oder können, falls sie neu sind, nach an sich bekannten Verfahren hergestellt werden, z. B. aus Hydrazin oder dessen geeigneten Derivaten die Verbindungen der Formel III, aus geeigneten Aminosäuren oder deren Analoga, beispielsweise mit ein oder zwei der genannten Seitenketten R₃ und R₄, die Verbindungen der Formel IV.

Die Verbindungen der Formel III sind beispielsweise zugänglich aus Verbindungen der Formel

H₂N-NH-R₁₁ (XV),

worin R₁₁ Wasserstoff oder eine Aminoschutzgruppe, wie oben unter Verfahren b) beschrieben, insbesondere tert-Niederalkoxycarbonyl, wie tert-Butoxycarbonyl, Arylniederalkoxycarbonyl, wie Benzyloxycarbonyl oder 9-Fluorenylmethoxycarbonyl, oder eine der oben genannten Acyl-Aminoschutzgruppen bedeutet, indem man zur Herstellung einer Verbindung der Formel I, worin anstelle von R₇ der Rest R₇˝ steht, welcher für unsubstituiertes oder substituiertes Alkyl oder Cycloalkyl steht, unter Einführung von R₇˝ alkyliert mit einer Verbindung der Formel XII, wie oben unter Verfahren e) beschrieben, oder den Rest R₇ durch Reaktion geeigneter Carbonylverbindungen mit der freien Aminogruppe der Verbindung der Formel VIII oder ihrer acylierten Derivate und anschliessende Reduktion des erhaltenen Hydrazones einsetzt unter Bildung von Hydrazinderivaten der Formel

R₇-NH-NH-R₁₁ (XVI),

wobei die Reste in allen genannten Verbindungen die oben angegebenen Bedeutungen haben und funktionelle Gruppen in den beteiligten Reagenzien, die nicht an der Reaktion teilnehmen sollen, gegebenenfalls geschützt sind, gegebenenfalls die Schutzgruppe R₁₁, sofern sie nicht einem der Reste R₈ oder R₉ in den Verbindungen der Formel I entspricht, und/oder weitere Schutzgruppen abspaltet, und die Reste R₈ und R₉ ausser Wasserstoff durch Kondensation unter den oben unter Verfahren b) genannten Bedingungen mit Säuren der Formel VI oder der Formel

R₈-OH (XVII),

worin R₈ die genannten Bedeutungen hat, oder durch Alkylierung mit einer Verbindung der Formel XIII oder XIV, wie oben definiert, oder beiden nach den oben bei den zusätzlichen Verhafrensmassnahmen genannten Bedingungen umsetzt.

Die zur Herstellung der Verbindungen der Formel XVI verwendeten, zur Einführung von R₇ geeigneten Carbonylverbindungen sind Aldehyde oder Ketone, deren reaktive Carbonylgruppe nach der Reaktion mit Verbindungen der Formel XV und der anschliessenden Reduktion Bestandteil eines der genannten Reste R₇ sind, vorzugsweise Aldehyde, die zu Einführung von Niederalkyl, Cyclohexylniederalkyl oder Phenylniederalkyl geeignet sind.

Die Reaktion der Carbonylverbindungen mit den Verbindungen der Formel XVI zu den entsprechenden Hydrazonen erfolgt unter den zur Reaktion von Carbonylverbindungen mit Aminen üblichen Bedingungen, vorzugsweise in polaren organischen Lösungsmitteln, z. B. Ethern, wie Tetrahydrofwan oder Diethylether, Alkoholen, wie Methanol oder Ethanol, Carbonsäureamiden, wie Dimethylformamid, oder Estern, wie Essigsäureethylester, oder in wässriger Lösung, bevorzugt in Methanol, ferner in Anwesenheit oder Abwesenheit von sauren Katalysatoren, z. B. Carbonsäuren, wie Ameisensäure oder Essigsäure, oder Sulfonsäuren, wie p-Toluolsulfonsäure, bei Temperaturen zwischen 0 °C und der Rückflusstemperatur des Reaktionsgemisches, vorzugsweise bei Temperaturen von 20 °C bis zur Rückflusstemperatur des Reaktionsgemisches.

Die Reduktion der erhaltenen Hydrazone erfolgt vorzugsweise durch Hydrierung in Gegenwart eines geeigneten Katalysators. Als zur Hydrierung geeignete Katalysatoren werden Metalle, wie Nickel, Eisen, Cobalt oder Ruthenium, oder Edelmetalle bzw. deren Oxide, wie Palladium oder Rhodium bzw. deren Oxide, verwendet, gegebenenfalls z. B. auf geeignetem Trägermaterial, wie Bariumsulfat, Aluminiumoxid oder Aktivkohle aufgezogen oder als Skelettkatalysatoren, wie Raney-Nickel. Gebräuchliche Lösungsmittel für die katalytische Hydrierung sind beispielsweise Wasser, Alkohole, wie Methanol oder Ethanol, Ester, wie Essigsäureethylester, Ether, wie Dioxan, Chlorkohlenwasserstoffe, wie Dichlormethan, Carbonsäureamide, wie Dimethylformamid, oder Carbonsäuren, wie Eisessig, oder Gemische dieser Lösungsmittel. Die Hydrierung erfolgt bei Temperaturen von 10 bis 250 °C, bevorzugt von Raumtemperatur bis 100 °C, und bei Wasserstoffdrukken von 1 bis 200 bar, vorzugsweise von 1 bis 10 bar, in den üblichen Apparaturen.

Besonders bevorzugt für die Herstellung der Verbindungen der Formel XV sind Reaktionsbedingungen, welche den in J. Chem. Soc. Perkin I, 1712 (1975) beschriebenen analog sind.

Die Verbindungen der Formel IV sind beispielsweise zugänglich durch Reduktion von Aminosäuren der Formel 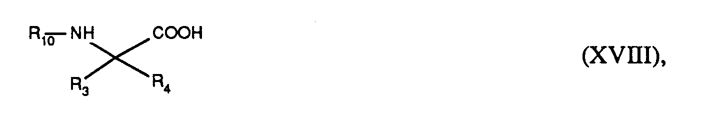
worin R₁₀ Wasserstoff oder eine der unter Verfahren a) genannten Aminoschutzgruppen, insbesondere tert-Niederalkoxycarbonyl, wie tert-Butoxycarbonyl, Arylniederalkoxy-carbonyl, wie Benzyloxycarbonyl oder 9-Fluorenylmethoxycarbonyl, oder eine der dort genannten Acyl-Aminoschutzgruppen, bedeutet und R₃ und R₄ die für Verbindungen der Formel I genannten Bedeutungen hat, vorzugsweise von Aminosäuren der Formel 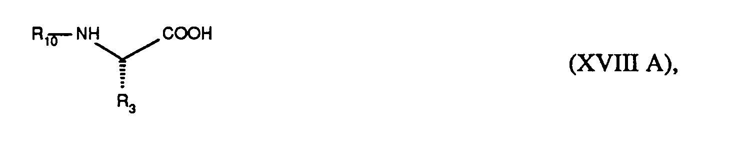
in denen die Reste die genannten Bedeutungen haben, zu Aldehyden der Formel 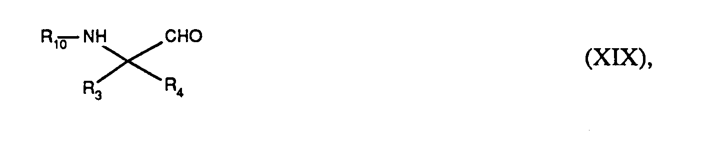
worin die Reste die genannten Bedeutungen haben, vorzugsweise zu den Aldehyden der Formel 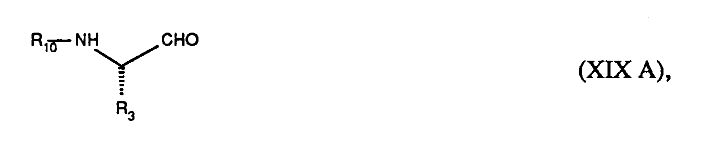
worin die Reste die genannten Bedeutungen haben (erhältlich beispielsweise aus Verbin-dungen der Formel XVIII A), durch Umsetzung dieser Aldehyde mit einer Ylid-Verbin-dung, vorzugsweise einer Schwefel-Ylid-Verbindung, zu einem Epoxid der Formel 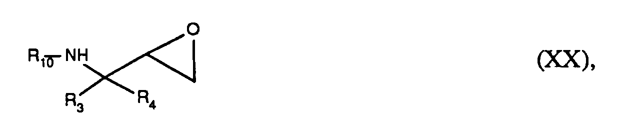
worin die Reste die genannten Bedeutungen haben, vorzugsweise zu Verbindungen der Formel 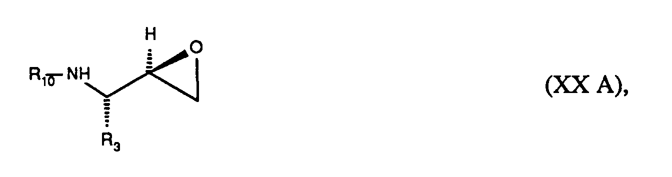
(erhältlich beispielsweise aus Verbindungen der Formel XIX A), worin die Reste die genannten Bedeutungen haben, gegebenenfalls Abspaltung der Schutzgruppe R₁₁, sofern sie nicht einem der Reste R₈ oder R₉ in den Verbindungen der Formel I entspricht, und Acylierung der Aminogruppe der resultierenden Verbindung mit Säuren der Formel VIII oder der Formel

R₂-OH (XXI),

wobei die Reste die genannten Bedeutungen haben, unter den für Verfahren b) beschrie-benen Bedingungen, und/oder Alkylierung der Aminogruppe der resultierenden Verbindung mit Reagentien mit nukleofugen Abgangsgruppen der Formeln X oder XI, worin die Reste die genannten Bedeutungen haben, unter den für zusätzliche Verfahrensmassnahmen beschriebenen Bedingungen.

Die Reduktion von Aminosäuren der Formel XVIII oder XVIII A zu den entsprechenden Aldehyden XIX und XIX A erfolgt beispielsweise durch Reduktion zu den entsprechenden Alkoholen und anschliessende Oxidation zu den genannten Aldehyden.

Die Reduktion zu den Alkoholen erfolgt beispielsweise durch Hydrogenierung der Säurehalogenide oder anderweitiger, unter Verfahren b) genannter aktivierter Carbonsäurederivate unter den für die Hydrogenierung von aus Verbindungen der Formel XVI erhaltenen Hydrazonen genannten Bedingungen oder mit komplexen Hydriden, wie Natriumborhydrid. Die anschliessende Oxidation der erhaltenen Alkohole ist beispielsweise unter den Bedingungen zur Oxidation von Verbindungen der Formel I, in denen R₅ Hydroxy und R₆ Wasserstoff bedeutet, zu solchen, in denen R₅ und R₆ gemeinsam Oxo bedeuten, wie bei den zusätzlichen Verfahrensmassnahmen beschrieben, möglich, oder durch Oxidation der Hydroxygruppe mit einem Sulfoxid, wie Dimethylsulfoxid, in Gegenwart eines die Hydroxygruppe aktivierenden Reagenzes, wie eine Carbonsäurechlorides, z. B. Oxalylchlorid, in einem inerten Lösungsmittel, z. B. einem halogenierten Kohlenwasserstoff, wie Dichlormethan, und/oder einem acyclischen oder cyclischen Ether, wie Tetrahydrofuran, bei - 80 bis 0 °C, z. B. - 78 bis - 50 °C.

Auch die direkte Reduktion der Aminosäuren zu den Aldehyden ist möglich, beispielsweise durch Hydrierung in Gegenwart eines partiell vergifteten Palladiumkatalysators oder durch Reduktion der entsprechenden Aminosäureester, z. B. der Niederalkylester, wie Ethylester, mit komplexen Hydriden, z. B. Borhydriden, wie Natriumborhydrid, oder vorzugsweise Aluminiumhydriden, z. B. Lithiumaluminiumhydrid, Lithium-tri-(tert-butoxy)aluminiumhydrid oder insbesondere Diisobutylaluminiumhydrid, in apolaren Lösungsmitteln, z. B. in Kohlenwasserstoffen oder aromatischen Lösungsmitteln, wie Toluol, bei -100 bis 0 °C, bevorzugt -70 bis -30 °C, und anschliessende Umsetzung zu den entsprechenden Semicarbazonen, z. B. mit den entsprechenden Säuresalzen von Semi-carbazonen, wie Semicarbazidhydrochlorid, in wässrigen Lösungsmittelsystemen, wie Alkohol/Wasser, z. B. Ethanol/Wasser, bei Temperatwen zwischen -20 und 60 °C, vorzugsweise 10 bis 30 °C, und Umsetzung des erhaltenen Semicarbazones mit einem reaktiven Aldehyd, z. B. Formaldehyd, in einem inerten Lösungsmittel, beispielsweise einem polaren organischen Lösungsmittel, z. B. einem Carbonsäureamid, wie Dimethyl-formamid, bei Temperaturen zwischen -30 und 60 °C, bevorzugt 0 bis 30 °C, und dann einer Säure, beispielsweise einer starken Mineralsäure, wie Halogenwasserstoff, in wässriger Lösung, gegebenenfalls in Gegenwart des vorher verwendeten Lösungsmittels, bei Temperaturen zwischen -40 und 50 °C, bevorzugt zwischen -10 und 30 °C, umsetzt. Die entsprechenden Ester werden durch Umsetzung der Aminosäuren mit den entspre-chenden Carbonsäuren, beispielsweise Ethanol, analog den bei der Kondensation unter Verfahren b) verwendeten Bedingungen gewonnen, beispielsweise durch Umsetzung mit anorganischen Säurehalogeniden, wie Thionylchlorid, in organischen Lösungsmittel-gemischen, wie Mischungen aus aromatischen und alkoholischen Lösungsmitteln, z. B. Toluol und Ethanol, bei Temperaturen zwischen -50 und 50 °C, bevorzugt zwischen - 10 und 20 °C.

Die Herstellung der Verbindungen der Formel XIX und XIX A erfolgt in besonders bevorzugter Weise unter Bedingungen analog den in J. Org. Chem. **47**, 3016 (1982) oder J. Org. Chem. **43**, 3624 (1978) genannten Reaktionsbedingungen.

Ein zur Umsetzung von Verbindungen der Formel XIX oder XIX A zu den Epoxiden der Formel XX oder XX A geeignetes Schwefel-Ylid ist beispielsweise ein Dialkylsulfoniummethylid, z. B. Dimethylsulfoniummethylid, ein Alkyl- oder Phenyl-dialkylaminosulfoxoniummethylid, z. B. Methyl- oder Phenyl-dimethylaminosulfoxoniummethylid, oder ein Dialkylsulfoxoniummethylid, z. B. Dimethyl- oder Diethylsulfoxoniummethylid.

Die betreffende Schwefel-Ylid-Verbindung wird zweckmässigerweise in situ aus dem entsprechenden Sulfonium- bzw- Sulfoxoniumsalz und einer Base, z. B. Natriumhydrid, in einem dipolar aprotischen Lösungsmittel, z. B. Dimethylsulfoxid, oder einem Ether, z. B. Tetrahydrofuran oder 1,2-Dimethoxyethan, hergestellt und anschliessend mit den Verbindungen der Formel XIX oder XIX A umgesetzt. Die Umsetzung erfolgt normalerweise bei Raumtemperatur, unter Kühlen z. B. bis auf -20 °C, oder unter leichtem Erwärmen z. B. bis auf 40 °C. Das gleichzeitig gebildete Sulfid, Sulfinamid bzw. Sulfoxid wird bei der anschliessenden wässrigen Aufarbeitung entfernt.

Die Umsetzung mit einem Schwefel-Ylid erfolgt in besonders bevorzugter Weise analog den in J. Org. Chem. **50**, 4615 ( 1985) genannten Bedingungen.

Die Verbindung der Formel XX (vorzugsweise XXA) kann auch aus einer Verbindung der Formel XIX (vorzugsweise XIXA), wie oben definiert, durch deren Umsetzung mit einer Triniederalkyl-silylmethyl-Grignard-Verbindung, z. B. hergestellt aus dem entsprechenden Halogen-methyl-Silan, wie Chlormethyl-trimethylsilan, in einem inerten Lösungsmittel, z. B. einem Ether, wie Dioxan oder Diethylether, bei Temperaturen zwischen 0 und 50 °C, z. B. zwischen Raumtemperatur und etwa 40 °C, anschliessende Elimination unter Entfernung des Silylrestes und Bildung einer Doppelbindung, z. B. mittels einer Lewis-Säure, wie BF₃, wobei vorzugsweise auch eine vorliegende Aminoschutzgruppe R₁₀ abgespalten wird, in einem inerten LM, z. B. einem Ether, wie Diethylether, oder einem Halogenkohlenwasserstoff, wie Dichlormethan, oder einem Gemisch davon, bei Temperaturen zwischen -50 °C und der Rückflusstemperatur, insbesondere zwischen 0 und 30 °C, wenn erforderlich, erneute Acylierung unter Einführung von einer Aminoschutzgruppe als R₁₀, wie oben definiert, und Oxidation der erhaltenen Doppelbindung zum Oxiran, vorzugsweise mit einer Percarbonsäure, z. B. m-Chlorperbenzoesäure, in einem inerten Lösungsmittel, z. B. halogenierten Kohlenwasserstoff, wie Dichlormethan, bei Temperaturen zwischen -20 °C und der Rückflusstemperatur des Gemisches, z. B. bei 10 bis 30 °C, erhalten werden.

Die Ausgangsmaterialien der Verfahren b), c) und d) sind bekannt oder können, falls sie neu sind, nach an sich bekannten Verfahren hergestellt werden, z. B. kann die Verbindung der Formel V aus geeigneten Hydrazinderivaten der Formel III, in der R₉ Wasserstoff bedeutet und die übrigen Reste die für Verbindungen der Formel V genannten Bedeutungen haben, und geeigneten Epoxiden der Formel IV, worin die Reste die für Verbindungen der Formel V genannten Bedeutungen haben (Verfahren b), die Verbindung der Formel VII aus geeigneten Hydrazinderivaten der Formel III, in der die Reste die für Verbindungen der Formel VII genannten Bedeutungen haben, und geeigneten Epoxiden der Formel IV, worin R₁ Wasserstoff bedeutet und die übrigen Reste die für Verbindungen der Formel VII genannten Bedeutungen haben (Verfahren c), und die Verbindung der Formel IX aus geeigneten Hydrazinderivaten der Formel III, in der R₉ Wasserstoff bedeutet und die übrigen Reste die für Verbindungen der Formel IX genannten Bedeutungen haben (Verfahren d) und geeigneten Epoxiden der Formel IV, worin R₁ Wasserstoff bedeutet und die übrigen Reste die für Verbindungen der Formel IX genannten Bedeutungen haben (Verfahren d), hergestellt werden in Analogie zu Verfahren a), gegebenenfalls unter Verwendung und Abspaltung von Schutzgruppen.

Die Verbindungen der Formel I′, in der die Substituenten die oben genannten Bedeutungen haben, lassen sich beispielsweise herstellen aus Verbindungen der Formel III′, 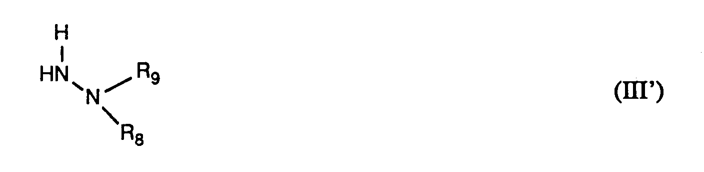
worin die Reste die für Verbindungen der Formel I genannten Bedeutungen haben, wie bei Verfahren b) beschrieben, durch Umsetzung mit einer Verbindung der Formel IV, wobei vorhandene funktionelle Gruppen, die nicht an der Reaktion teilnehmen sollen, wie dort geschützt vorliegen und nach der Reaktion freigesetzt werden können.

Bevorzugt für Verfahren d) sind die Ausgangsverbindungen der Formel 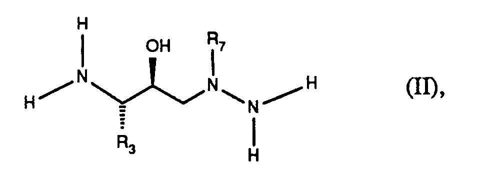
worin R₃ unsubstituiertes oder substituiertes Alkyl oder Cycloalkyl; Aryl; Heterocyclyl; oder unsubstituiertes oder substituiertes Alkenyl bedeutet; und R₇ unsubstituiertes oder substituiertes Alkyl oder Cycloalkyl; Aryl; Heterocyclyl; oder unsubstituiertes oder substituiertes Alkenyl bedeutet, sowie die Salze der genannten Verbindungen, sofern salzbildende Gruppen vorliegen, welches erfindungsgemässe Zwischenverbindungen sind.

Diese können insbesondere an einer oder beiden Aminogruppen geschützt sein, wobei im Falle des Vorliegens zweier Aminoschutzgruppen diese identisch oder voneinander ver-schieden sein können.

Als Aminoschutzgruppen finden beispielsweise die oben unter Verfahren a) genannten Aminoschutzgruppen Verwendung. Die für Verbindungen der Formel II genannten Reste R₃ und R₇ haben die oben für Verbindungen der Formel I bei der Definition der Reste R₃ und R₇ genannten Bedeutungen.

Sehr bevorzugt sind die Verbindungen der Formel II, worin R₃ Cyclohexylniederalkyl, Phenylniederalkyl oder p-Fluorphenylniederalkyl bedeutet und R₇ Niederalkyl, Cyclohexylniederalkyl, Phenylniederalkyl, p-Cyanophenylniederalkyl oder p-Fluorphenylniederalkyl bedeutet, sowie die Salze der genannten Verbindungen, sofern salzbildende Gruppen vorliegen.

Sehr bevorzugt sind insbesondere die Verbindungen der Formel II, worin R₃ Phenylniederalkyl bedeutet und R₇ Niederalkyl, Cyclohexylniederalkyl oder Phenylniederalkyl bedeutet, sowie die Salze der genannten Verbindungen, sofern salzbildende Gruppen vorliegen.

In erster Linie bevorzugt sind die Verbindungen der Formel II, worin R₃ Cyclohexyl-methyl, Benzyl oder p-Fluorbenzyl bedeutet und R₇ n-Butyl, Isobutyl, Cyclohexylmethyl, Benzyl, p-Fluorbenzyl oder p-Cyanobenzyl bedeutet, sowie die Salze der genannten Verbindungen, sofern salzbildende Gruppen vorliegen.

In erster Linie bevorzugt sind insbesondere die Verbindungen der Formel II, worin R₃ Benzyl bedeutet und R₇ Isobutyl, Cyclohexylmethyl oder Benzyl bedeutet, sowie die Salze der genannten Verbindungen, sofern salzbildende Gruppen vorliegen.

Zuallererst bevorzugt sind die in den Beispielen genannten Verbindungen der Formel II.

Die Verbindungen der Formel II, worin die Substituenten die genannten Bedeutungen haben, oder ihre Salze, sofern salzbildende Gruppen vorliegen, werden beispielsweise hergestellt indem man ein Hydrazinderivat der Formel

R₇-NH-NH-R₁₁ (XVI),

worin R₁₀ eine Aminoschutzgruppe bedeutet, an ein Epoxid der Formel 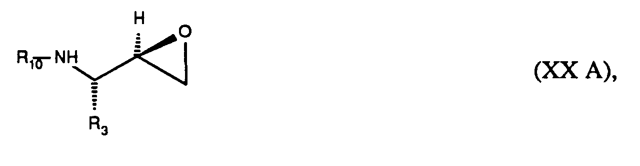 worin R₁₁ eine Aminoschutzgruppe bedeutet, addiert, und
gewünschtenfalls eine nach dem vorstehenden Verfahren erhältliche Verbindung der Formel II mit mindestens einer salzbildenden Gruppe in ihr Salz oder ein erhältliches Salz in die freie Verbindung oder in ein anderes Salz überführt und/oder gegebenenfalls erhältliche Isomerengemische auftrennt und/oder vorhandene Schutzgruppen in einer Verbindung der Formel II abspaltet und/oder eine erfindungsgemässe Verbindung der Formel II in eine andere erfindungsgemässe Verbindung der Formel II umwandelt.

Die Herstellung und Umwandlung von Salzen, die Auftrennung von Isomerengemischen, die Abspaltung von Schutzgruppen und die Umwandlung von Verbindungen der Formel II erfolgen analog den oben für Verbindungen der Formel I beschriebenen Verfahren.

Besonders bevorzugt ist die Herstellung von Ausgangsprodukten der Formel II, in denen die Substituenten die genannten Bedeutungen haben, durch Schutzgruppenabspaltung aus Verbindungen der Formel II, in denen eine oder beide Aminogruppen durch Aminoschutzgruppen geschützt sind, insbesondere unter den Bedingungen zur Hydrolyse von Verbindungen der Formel I, wie bei den zusätzlichen Verfährensmassnahmen beschrieben.

Die Methoden für die Addition von Verbindungen der Formel XVI an solche der Formel XX A sind oben unter Verfahren a) bei der Herstellung von Verbindungen der Formel I beschrieben.

Die Herstellung der geschützten Verbindungen der Formel I erfolgt beispielsweise nach irgendeinem der bisher genannten Verfahren, insbesondere aus Verbindungen der Formel III und IV, wobei funktionelle Gruppen in diesen Verbindungen gegebenenfalls geschützt sind durch Schutzgruppen, wie unter Verfahren a) beschrieben.

Die Säuren der Formeln VI, VIII, XVII und XXI sowie die Verbindungen mit nukleofugen Gruppen der Formel X, XI, XII, XIII, XIV und XV sind bekannt oder, wenn sie neu sind, nach an sich bekannten Verfahren herstellbar.

Die folgenden Beispiele dienen zur Illustration der Erfindung, schränken deren Umfang jedoch in keiner Weise ein.

Temperaturen werden in Celsiusgraden (°C) angegeben. Sofern keine Temperaturangabe erfolgt, findet die Reaktion bei Raumtemperatur statt. Die R_{f}-Werte, die das Verhältnis von Laufstrecke der jeweiligen Substanz zur Laufstrecke der Laufmittelfront angeben, werden auf Kieselgeldünnschichtplatten durch Dünnschichtchromatographie (DC) in folgenden Lösungsmittelsystemen ermittelt

### DC-Laufmittelsysteme:

| | | |
|---|---|---|
| A | Chloroform/Methanol/Wasser/Essigsäure | 75:27:5:0,5 |
| B | Chloroform/Methanol/Wasser/Essigsäure | 90:10:1:0,5 |
| C | Chloroform/Methanol/Wasser/Essigsäure | 85:13:1,5:0,5 |
| D | Chloroform/Methanol | 8:1 |
| E | Chloroform/Methanol | 95:5 |
| F | Hexan/Essigsäureethylester | 2:1 |
| G | Methylenchlorid/Diethylether/Methanol | 20:20:1 |
| H | Methylenchlorid/Diethylether | 1:1 |
| I | Toluol/Essigsäureethylester | 2:1 |
| K | Chloroform/Methanol | 5:1 |
| J | Methylenchlorid/diethylether | 5:1 |
| L | Hexan/Essigsäureethylester | 4:1 |
| M | Hexan/Essigsäureethylester | 5:1 |
| N | Hexan/Essigsäureethylester | 1:1 |
| O | Essigsäureethylester | - |
| P | Methylenchlorid/Ethanol/NH₃aq. | 90:10:1 |
| Q | Methylenchlorid/Diethylether | 10:1 |
| R | Hexan/Essigsäureethylester | 3:1 |
| S | Methylenchlorid/Diethylether | 20:1 |
| T: | Chloroform/Methanol | 30:1 |
| U: | Chloroform/Methanol | 15:1 |
| V: | Methylenchlorid/Diethylether/Hexan | 1:1:3 |
| W: | Methylenchlorid/Diethylether | 20:1 |
| X: | Methylenchlorid/Methanol | 40:1 |
| Y: | Toluol/Essigsäureethylester | 4:1 |

Die Abkürzung "R_{f}(A)" bedeutet beispielsweise, dass der R_{f}-Wert im Lösungsmittelsystem A ermittelt wurde. Das Mengenverhältnis von Lösungsmitteln zueinander ist stets in Volumenanteilen angegeben.

### HPLC-Gradienten:

| | |
|---|---|
| I: | 20 % ->100% Acetonitril/0,05 % Trifluoressigsäure in Wasser/0,05 % Trifluoressigsäure während 35 min. |
| II: | 0 % ->40 % Acetonitril/0,05 % Trifluoressigsäure in Wasser/0,05 % Trifluoressigsäure während 30 min. |
| III: | 20 % -> 60 % Acetonitril/0,05 % Trifluoressigsäure in Wasser/0,05 % Trifluoressigsäure während 60 min. |
| IV: | 10 % -> 50 % Acetonitril/0,05 % Trifluoressigsäure in Wasser/0,05 % Trifluoressigsäure während 60 min. |

Säule (250 x 4,6 mm) gefüllt mit "Reversed-Phase"-Material C₁₈-Nucleosil® (5 µm mittlere Korngrösse, mit Octadecylsilanen kovalent derivatisiertes Silicagel, Macherey & Nagel, Düren, BRD). Detektion durch UV-Absorption bei 215 nm. Die Retentionszeiten (t_{Ret}) werden in Minuten angegeben. Fliessgeschwindigkeit 1 ml/min.

Für die Bezeichnung der Fliessmittel-Systeme werden bei der Flash-Chromatographie und der Mitteldruckchromatographie die gleichen Abkürzungen verwendet.

Die weiteren verwendeten Kurzbezeichnungen und Abkürzungen haben die folgenden Bedeutungen:
- abs.: absolut (gibt an, dass Lösungsmittel wasserfrei ist)
- atm: physikalische Atmosphären (Druckeinheit) - 1 atm entspricht 1,013 bar
- Boc: tert-Butoxycarbonyl
- BOP: Benzotriazol- 1-yl-oxy-tris-(di-methyl-amino)-phosponium-hexafluor-phosphat
- DIPE: Diisopropylether
- DMF: Dimethylformamid
- DMSO: Dimethylsulfoxid
- Ether: Diethylether
- ges.: gesättigt
- h: Stunde(n)
- HBTU: O-Benztriazol-1-yl-N,N,N′,N′-tetra-methyl-uronium-hexafluorphosphat
- HOBt: 1-Hydroxy-benztriazol
- HV: Hochvakuum
- min: Minute(n)
- MS: Massenspektroskopie
- NMM: N-Methyl-morpholin
- RT: Raumtemperatur
- RV: Rotationsverdampfer
- Sole: gesättigte Natriumchloridlösung
- TFA: Trifluoressigsäure
- Z: Benzyloxycarbonyl

Massenspektroskopische Messwerte werden entweder durch konventionelle MS oder nach der "Fast-Atom-Bombardment" (FAB-MS)-Methode erhalten. Die Massenangaben bezie-hen sich im ersten Fall auf das unprotonierte Molekülion (M)⁺ oder das protonierte Molekülion (M+H)⁺.

Die Werte für Protonen-Kernresonanzspektroskopie (¹H-NMR) werden in ppm (parts per million) bezogen auf Tetramethylsilan als internen Standard angegeben. s = Singulett, d = Dublett, t = Triplett, q = Quartett, m = Multiplett, dd = Doppeldublett, br = breit.

Die Werte für IR-Spektren werden in cm⁻¹ angegeben, in runden Klammern findet sich das jeweilige Lösungsmittel. Falls angegeben, bedeutet s eine starke, m eine mittlere und w eine schwache Intensität der jeweiligen Bande.

Der Rest mit der Bezeichnung -[Phe^{NN}Phe] bedeutet das zweiwertige Radikal von 3(S)-Amino-4-phenyl-1-(N-benzylhydrazino)-butan-2(S)-ol und hat die Formel

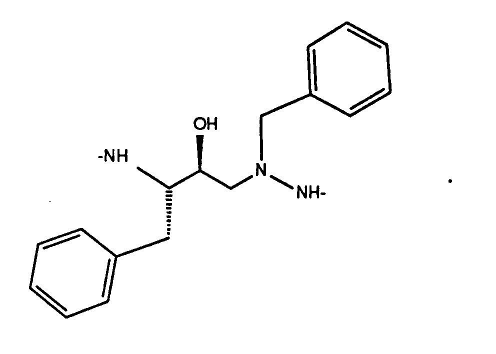

Der Rest mit der Bezeichnung -[Phe^{NN}Cha] bedeutet das zweiwertige Radikal von 3(S)-Amino-4-phenyl-1-(N-cyclohexylmethylhydrazino)-butan-2(S)-ol und hat die Formel

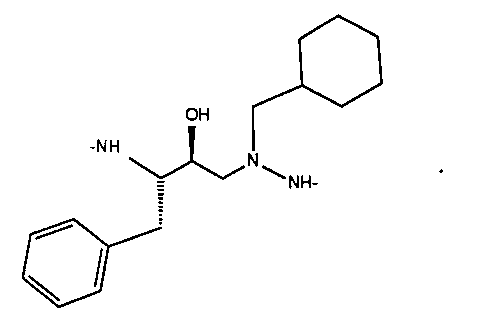

Der Rest mit der Bezeichnung -[Phe^{NN}Leu] bedeutet das zweiwertige Radikal von 3(S)-Amino-4-phenyl-1-(N-isobutylhydrazino)-butan-2(S)-ol und hat die Formel

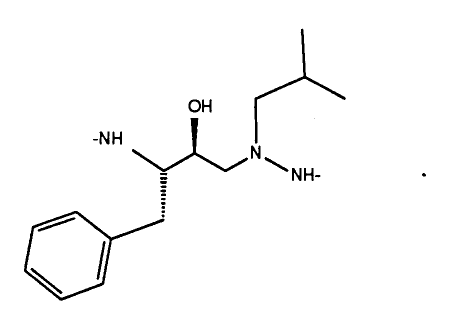

Der Rest mit der Bezeichnung -[Phe^{NN}Nle] bedeutet das Radikal von 3(S)-Amino-4-phenyl-1-(N-n-butylhydrazino)-butan-2(S)-ol und hat die Formel

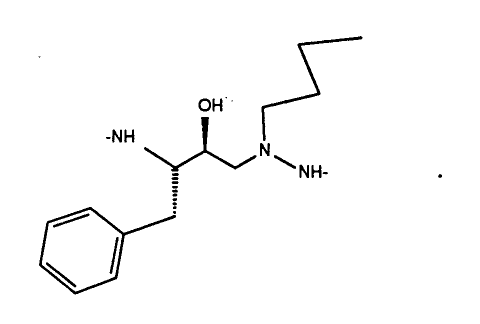

Der Rest mit der Bezeichnung -[Phe^{NN}(p-F)Phe] bedeutet das zweiwertige Radikal von 3(S)-Amino-4-phenyl-1-(N-(p-fluorphenylmethyl)-hydrazino)-butan-2(S)-ol und hat die Formel

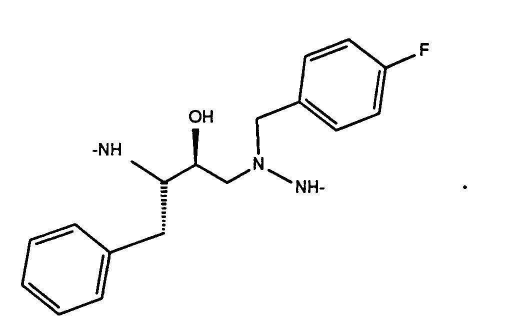

Der Rest mit der Bezeichnung - [(p-F)Phe^{NN}(p-F)Phe] bedeutet das zweiwertige Radikal von 3(S)-Amino-4-(p-fluorphenyl)-1 -(N-(p-fluorphenylmethyl)-hydrazino)-butan-2(S)-ol und hat die Formel

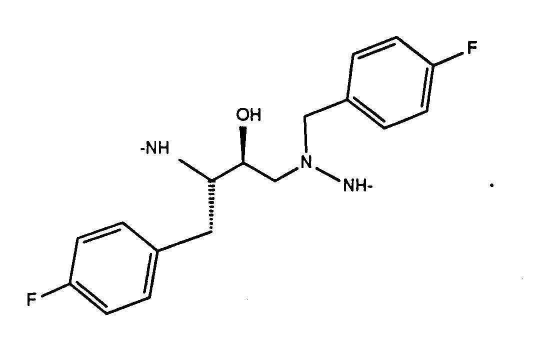

Der Rest mit der Bezeichnung -[Phe^{NN}(p-CN)Phe] bedeutet das zweiwertige Radikal von 3(S)-Amino-4-phenyl-1-(N-(p-cyanophenylmethyl)-hydrazino)-butan-2(S)-ol und hat die Formel 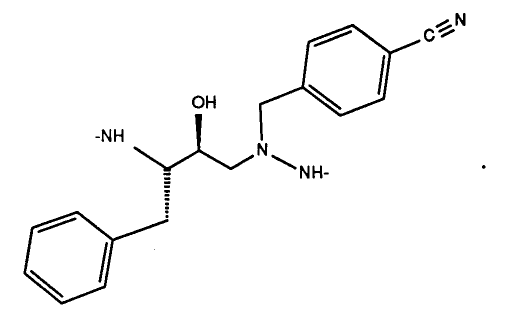
Der Rest mit der Bezeichnung -[Cha^{NN}Leu] bedeutet das zweiwertige Radikal von 3(S)-Amino-4-cyclohexyl-1-(N-isobutyl-hydrazino)-butan-2(S)-ol und hat die Formel

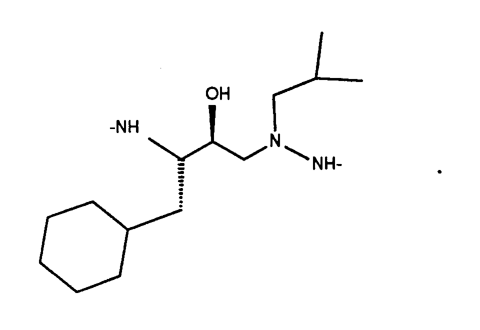

Zur Bezeichnung von zweiwertigen Radikalen von natürlichen α-Aminosäuren werden die in der Peptidchemie üblichen Abkürzungen verwendet. Hierbei haben jedoch Aminosäuren, die in den Verbindungsnamen rechts der Radikale -[Phe^{NN}Phe], -[Phe^{NN}Cha], -[Phe^{NN}Leu], -[Phe^{NN}Nle], -[Phe^{NN}(p-F)Phe], -[(p-F)Phe^{NN}(p-F)Phe], -[Phe^{NN}(p-CN)Phe] oder -[Cha^{NN}Leu] stehen, in Abweichung von der üblichen Peptidnomenklatur, worin links der Amino- und rechts der Carboxyterminus vorliegt, die bindende Carboxygruppe links, was durch einen Pfeil (←), der die Umkehrung der Bindungsrichtung symbolisiert, angedeutet wird. Die Konfiguration am α-Kohlen-stoffatom wird, wenn sie bekannt ist, durch Voranstellen von (L)-oder (D)-angegeben. An der phenolischen Hydroxygruppe mit dem Rest R veretherte Tyrosin-Radikale werden mit Tyr(OR) bezeichnet. Nle bedeutet das Radikal von Norleucin.

### Beispiel 1: Boc-[Phe^{NN}Phe]-Boc:

Eine Lösung von 300 mg (1,14 mMol) (2R)-[*1′(S)*-Boc-amino-2′-phenylethyl]oxiran (J. Org. Chem. **50**, 4615 (1985)) und 253 mg (1,14 mMol) tert-Butyl-3-benzyl-carbazat (J. Chem. Soc., Perkin I, 1712 (1975)) in 4 ml Methanol wird während 12 h unter Rückfluss erhitzt. Nach dem Abkühlen des Reaktionsgemisches auf 0° fällt ein grosser Teil der Titelverbindung aus. Die Mutterlauge wird eingedampft und der Rückstand in wenig Methylenchlorid gelöst. Nach Zutropfen von Hexan fällt weitere Titelverbindung als weisser Niederschlag aus. FAB-MS: (M+H)⁺=486, t_{Ret}(I)=26,8 min, R_{f}(E)=0,70.

### Beispiel 2: Z-(L)-Val-[Phe^{NN}Phe]←((L)-Val-Z):

Man löst 191 mg (0,76 mMol) Z-(L)-Valin, 336 mg (0,76 mMol) BOP und 103 mg (0,76 mMol) HOBt in 5 ml einer 0,3 M Lösung von NMM in DMF, fügt nach 10 min 100 mg (0,25 mMol) H-[Phe^{NN}Phe]-H · 3HCl zu und rührt während 2 h bei *RT* unter Stickstoffatmosphäre. Die Reaktionsmischung wird eingedampft, der Rückstand in Methylenchlorid gelöst und zweimal mit ges. Natriumbicarbonatlösung gewaschen. Die organischen Phasen werden durch Watte filtriert, eingedampft und der Rückstand mittels Chromatographie auf Kieselgel mit Methylenchlorid/Ether (1:1) gereinigt. Nach Lyophilisation der produkthaltigen Fraktionen aus Dioxan erhält man die Titelverbindung als weissen Festkörper. FAB-MS: (M+H)⁺=752, t_{Ret}(I)=27,8 min, R_{f}(E)=0,45.

Das Ausgangsmaterial wird folgendermassen hergestellt:

### a) H-[Phe^{NN}Phe]-H · 3HCl:

Eine Lösung von 280 mg (0,58 mMol) Boc-[Phe^{NN}Phe]-Boc aus Beispiel 1 in 10 ml 4 N Chlorwasserstoff in Dioxan wird während 2 h bei RT unter Stickstoffatmosphäre gerührt und anschliessend lyophilisiert. Erneute Lyophilisation aus Dioxan/tert-Butanol ergibt die Titelverbindung als flockigen Festkörper. FAB-MS: (M+H)⁺=286, t_{Ret}(II)=23,1 min, R_{f}(C)=0,17.

### Beispiel 3: Boc-(L)-Val-[Phe^{NN}Phe]←((L)-Val-Boc):

Auf analoge Weise wie in Beispiel 2 beschrieben erhält man aus 50 mg (0,13 mMol) H-[Phe^{NN}Phe]-H · 3HCl, 83 mg (0,83 mMol) Boc-(L)-Valin, 168 mg (0,38 mMol) BOP, 51 mg (0,38 mMol) HOBt und 2,5 ml 0,3 M NMM in DMF nach chromatographischer Reinigung an Kieselgel mit Chloroform/Methanol (95:5) und Lyophilisation aus Dioxan die Titelverbindung. FAB-MS: (M+H)⁺=684, t_{Ret}(I)=27,4 min, R_{f}(E)=0,38.

### Beispiel 4: Boc-[Phe^{NN}Cha]-Boc:

Analog Beispiel 1 erhält man aus 231 mg (0,88 mMol) (2R,3S)-1-[3-Boc-amino-2-phenylethyl]oxiran und 200 mg (0,88 mMol) tert-Butyl-3-cyclohexylmethyl-carbazat die Titelverbindung als weissen Niederschlag aus Hexan. FAB-MS: (M+H)⁺=492, t_{Ret}(I)=30,4 min, R_{f}(E)=0,78.

Das Ausgangsmaterial wird folgendermassen hergestellt:

### a) tert-Butyl-3-cyclohexylmethyl-carbazat:

10.2 g (45,1 mMol) Cyclohexylcarbaldehyd-tert-butoxycarbonylhydrazon, gelöst in 400 ml Methanol, werden in Gegenwart von 5,1 g 5 % Platin auf Kohle bei RT und 4 atm Wasserstoffdruck hydriert. Nach beendeter Reaktion wird vom Katalysator abfiltriert und das Filtrat eingedampft. Der Rückstand wird in Methylenchlorid gelöst und mit Wasser gewaschen. Nach dem Eindampfen der organischen Phase erhält man die Titelverbindung als farbloses Harz. ¹H-NMR (200 MHz, CDCl₃): 6,1 (s, br, 1H), 3,9 (s, br, 1H), 2,65 (d, 2H), 1,8-0,75 (m, 11H), 1,45 (s, 9H), t_{Ret}(I)=32,0 min, R_{f}(E)=0,75.

### b) Cyclohexylcarbaldehyd-tert-butoxycarbonylhydrazon:

Eine Lösung von 10,8 g (81,2 mMol) tert-Butylcarbazat und 10,1 g (90 mMol) Cyclohexylcarbaldehyd in 400 ml Ethanol wird während 2 h unter Rückfluss erhitzt. Anschliessend wird die Hälfte des Lösungsmittels abdestilliert und die Titelverbindung durch Zugabe von Wasser ausgefällt. Sie wird direkt weiterverwendet in a).

### Beispiel 5: H-(L)-Val-[Phe^{NN}Phe]←((L)-Val)-H · 3HCl:

Eine Lösung von 40 mg (0,06 mMol) Boc-(L)-Val-[Phe^{NN}Phe]←((L)-Val)-Boc aus Beispiel 3 in 4 ml 4 N Chlorwasserstoff in Dioxan wird während 1 h bei *RT* gerührt. Anschliessend verdünnt man mit Dioxan und erhält nach Lyophilisation die Titelverbindung als Hydrochlorid. FAB-MS: (M+H)⁺=484, t_{Ret}(II)=25,8 min, R_{f}(A)=0,45.

### Beispiel 6: N-Thiomorpholinocarbonyl-(L)-Val-[Phe^{NN}Phe]←(N-thiomorpholinocarbonyl-(L)-Val):

Eine Lösung von 20 mg (0,03 mMol) H-(L)-Val-[Phe^{NN}Phe]←((L)-Val)-H · 3HCl aus Beispiel 5 in 0,5 ml DMF wird bei RT nacheinander mit 35 µl (0,25 mMol) Triethylamin und 16 mg (0,1 mMol) (4-Thiomorpholinylcarbonyl)chlorid versetzt und während 1 h bei RT gerührt. Die Reaktionsmischung wird mit Chloroform verdünnt und mit ges. Natriumbicarbonatlösung gewaschen. Die organische Phase wird durch Watte filtriert, eingedampft und der Rückstand an Kieselgel mit einem Gradienten von Chloroform/Methanol ( 15:1 -> 8:1 ) chromatographiert. Die Produktfraktionen werden eingedampft und mit Methylenchlorid/DIPE gefällt. Sie ergeben nach Lyophilisation aus Dioxan die Titelverbindung als flockigen Festkörper. FAB-MS: (M+H)⁺=742, t_{Ret}(I)=21,6 min, R_{f}(D)=0,54.

Das Ausgangsmaterial wird folgendermassen hergestellt:

### a) (4-Thiomorpholinylcarbonyl)chlorid:

Zu einer Lösung von 85 ml (165 mMol) 20 % Phosgen in Toluol wird bei 0° eine Lösung von 10 g (97 mMol) Thiomorpholin in 200 ml Toluol zugetropft und die weisse Suspension während 1 h bei RT gerührt. Ueberschüssiges Phosgen wird durch Einleiten von Stickstoff vertrieben, die Suspension filtriert und das Filtrat eingedampft. Man erhält die Titelverbindung als gelbes Oel. IR (CH₂Cl₂, cm⁻¹): 1735, 1450, 1440, 1405, 1370, 1290, 1180.

### Beispiel 7: N-Morpholinocarbonyl-(L)-Val-[Phe^{NN}Phe]←(N-morpholinocarbonyl-(L)-Val):

Eine Lösung von 100 mg (0,25 mMol) H-[Phe^{NN}Phe]-H · 3HCl aus Beispiel 2a, 163 mg (0,76 mMol) N-Morpholinocarbonyl-(L)-valin und 288 mg (0,76 mMol) HBTU in 2 ml DMF wird mit 210 µl (1.52 mMol) Triethylamin versetzt und bei RT während 16 h unter Stickstoffatmosphäre gerührt. Die Reaktionsmischung wird vollständig eingedampft, der Rückstand in Methylenchlorid gelöst und mit ges. Natriumbicarbonatlösung gewaschen. Die organische Phase wird durch Watte filtriert, eingedampft und an Kieselgel mit Methylenchlorid/Methanol (15:1) chromatographiert. Die Titelverbindung wird aus Methylenchlorid/Hexan gefällt und nach Lyophilisation aus Dioxan/tert-Butanol als flockiger Festkörper erhalten. FAB-MS: (M+H)⁺=710, t_{Ret}(I)=16,3 min, R_{f}(E)=0,16.

Das Ausgangsmaterial wird folgendermassen hergestellt:

### a) N-Morpholinocarbonyl-(L)-valin:

2,7 g (8,4 mMol) N-Morpholinocarbonyl-(L)-valin-benzylester werden in 75 ml Essigester gelöst und in Gegenwart von 500 mg 10 % Palladium auf Kohle bei 1 atm Wasserstoffdruck und RT während 3 h hydriert. Der Katalysator wird abfiltriert, und man erhält nach dem Eindampfen des Lösungsmittels die Titelverbindung als farbloses Oel. ¹H-NMR (300 MHz, CD₃OD): 4,15 (m, 1H), 3,65 (m, 4H), 3,40 (m, 4H), 2,12 (m, 1H), 0,95 (2d, 6H).

### b) N-Morpholinocarbonyl-(L)-valin-benzylester:

Eine Lösung von 4 g (10,5 mMol) (L)-Valin-benzylester-4-toluolsulfonat in 56 ml Methylenchlorid wird mit 0,8 ml (8,1 mMol) (Morpholinocarbonyl)chlorid (Herstellung: J. Med. Chem. **31**, 2277 (1988)) und 4,1 ml (24,1 mMol) N-Ethyldiisopropylamin versetzt und während 24 h bei RT gerührt. Die Reaktionsmischung wird mit Essigester verdünnt und nacheinander mit 1 N Salzsäure, Wasser, ges. Natriumbicarbonatlösung und Sole gewaschen. Die organische Phase wird über Natriumsulfat getrocknet und eingedampft. Sie ergibt nach Chromatographie an Kieselgel mit Essigester den *N*-Morpholinocarbonyl-(L)-valin-benzylester als farbloses Oel. Der Ester wird sofort in a) weiterverwendet.

### Beispiel 8: Phenylacetyl-(L)-Val-[Phe^{NN}Phe]←(N-phenylacetyl-(L)-Val):

Analog Beispiel 7 erhält man aus 100 mg (0,25 mMol) H-[Phe^{NN}Phe]-H · 3HCl aus Beispiel 2a, 143 mg (0,61 mMol) Phenylacetyl-(L)-valin (Herstellung: Mem. Tokyo Univ. Agric. **20**, 51 (1978)), 230 mg (0,61 mMol) HBTU und 200 µl (1,42 mMol) Triethylamin nach chromatographischer Reinigung mit Methylenchlorid/Ether/Methanol (20:20:1)und Lyophilisation aus Dioxan/tert-Butanol die Titelverbindung. FAB-MS: (M+H)⁺=720, t_{Ret}(I)=23,7 min, R_{f}(G)=0,21.

### Beispiel 9: N-(3-Pyridylacetyl)-(L)-Val-[Phe^{NN}Phe]←(N-(3-pyridylacetyl)-(L)-Val):

Analog Beispiel 7 erhält man aus 100 mg (0,25 mMol) H-[Phe^{NN}Phe]-H · 3HCl aus Beispiel 2a, 576 mg (1,52 mMol) HBTU, 358 mg (1,52 mMol) N-(3-Pyridylacetyl)-(L)-valin und 316 µl (2,3 mMol) Triethylamin nach chromatographischer Reinigung mit Chloroform/Methanol (5:1) und Lyophilisation aus Dioxan/tert-Butanol die Titelverbindung als weissen Festkörper. FAB-MS: (M+H)⁺=722, t_{Ret}(II)=27,9 min, R_{f}(A)=0,71.

Das Ausgangsmaterial wird folgendermassen hergestellt:

### a) N-(3-Pyridylacetyl)-(L)-valin:

3,4 g N-(3-Pyridylacetyl)-(L)-valin-tert-butylester werden in 20 ml Trifluoressigsäure/Methylenchlorid (1:1) gelöst und bei RT während 16 h gerührt. Die Reaktionslösung wird vollständig eingedampft und der Rückstand mit DIPE digeriert. Man erhält die Titelverbindung als weissen, amorphen Festkörper. ¹H-NMR (200 MHz, CD₃OD): 8,9-8,6 (m, breit, 1H), 8,5 (m, 1H), 7,95 (m, 1H), 4,33 (m, 1H), 3,93 (s, 2H), 2,2 (m, 1H), 0,98 (2d, 6H).

### b) N-(3-Pyridylacetyl)-(L)-valin-tert-butylester:

Zu einer Lösung von 3,36 g (16 mMol) (L)-Valin-tert-butylester · HCl, 2 g (14,5 mMol) 3-Pyridylessigsäure und 2,17 ml (14,3 mMol) Cyanphosphonsäure-diethylester in 20 ml DMF werden bei 0° 4,2 ml Triethylamin zugetropft. Die Reaktionsmischung wird wäh-rend 48 h bei RT gerührt, anschliessend mit Methylenchlorid verdünnt und mit 10 % Citronensäure sowie ges. Natriumbicarbonatlösung gewaschen. Die organische Phase wird durch Watte filtriert und ergibt nach Abdampfen des Lösungsmittels N-(3-Pyridylacetyl)-(L)-valin-tert-butylester, der sofort unter a) weiterverwendet wird.

### Beispiel 10: Boc-(L)-Val-[Phe^{NN}Cha]←((L)-Val)-Boc:

Analog Beispiel 7 erhält man ausgehend von 500 mg (1,25 mMol) H-[Phe^{NN}Cha]-H· 3HCl, 1,08 g (4,98 mMol) Boc-(L)-Valin, 1,89 g (4,98 mMol) HBTU und 1,39 ml (9,96 mMol) Triethylamin nach chromatographischer Reinigung an Kieselgel mit Methylenchlorid/Ether (1:1) und Lyophilisation aus Dioxan die Titelverbindung als flockigen Festkörper. FAB-MS: (M+H)⁺=690, t_{Ret}(I)=29,3 min, R_{f}(H)=0,48.

Das Ausgangsmaterial wird folgendermassen hergestellt:

### a) H[Phe^{NN}Cha]-H · 3HCl:

1,10 g (2,2 mMol) Boc-[Phe^{NN}Cha]-Boc aus Beispiel 4 werden in 20 ml 4 N Chlorwasserstoff in Dioxan gelöst und während 3 h bei *RT* gerührt. Nach Lyophilisation der Reaktionslösung erhält man die Titelverbindung als Hydrochlorid. FAB-MS: (M+H)⁺=292, t_{Ret}(II)=27,3 min.

### Beispiel 11: Z-(L)-Val-[Phe^{NN}Cha]←((L)-Val)-Z:

Analog Beispiel 2 erhält man aus 50 mg (0,12 mMol) H-[Phe^{NN}Cha]-H · 3HCl aus Beispiel 10a, 94 mg (0,37 mMol) Z-(L)-Valin, 165 mg (0,37 mMol) BOP, 51 mg (0,37 mMol) HOBt und 2,5 ml 0,3 M *NMM* in DMF nach chromatographischer Reinigung an Kieselgel mit Methylenchlorid/Ether (1:1) und Lyophilisation aus Dioxan die Titelverbindung. FAB-MS: (M+H)⁺=758, t_{Ret}(I)=29,1 min, R_{f}(H)=0,55.

### Beispiel 12: Boc-[Phe^{NN}Leu]-Boc:

Analog Beispiel 1 erhält man ausgehend von 1,0 g (3,8 mMol) (2R)-[1′(S)-Boc-amino-2′-phenylethyl]oxiran und 715 mg (3,8 mMol) tert-Butyl-3-isobutyl-carbazat (Herstellung: J. Chem. Soc., Perkin I, 1712 (1975)) die Titelverbindung als Niederschlag aus Hexan. FAB-MS: (M+H)⁺=452, t_{Ret}(I)=27,2 min, R_{f}(I)=0,55.

### Beispiel 13: Z-(L)-Val-[Phe^{NN}Leu]←((L)-Val)-Z:

Analog Beispiel 2 erhält man ausgehend von 60 mg (0,17 mMol) H-[Phe^{NN}Leu]-H ·3HCl, 125 mg (0,50 mMol) Z-(L)-Valin, 221 mg (0,50 mMol) BOP, 67 mg (0,50 mMol) HOBt und 3,3 ml 0,3 M NMM in DMF nach chromatographischer Reinigung an Kieselgel mit Methylenchlorid/Ether (1:1) und Lyophilisation aus Dioxan die Titelverbindung. FAB-MS: (M+H)⁺= 718, t_{Ret}(I)=26,8 min, R_{f}(H)=0,38.

Das Ausgangsmaterial wird folgendermassen hergestellt:

### a) H-[Phe^{NN}Leu] -H · 3HCl:

Analog Beispiel 10a erhält man aus 1,21 g (2,48 mMol) Boc-[Phe^{NN}Leu]-Boc aus Beispiel 12 die Titelverbindung als Lyophilisat. FAB-MS: (M+H)⁺=252, t_{Ret}(II)=20,9 min , R_{f}(K)=0,23.

### Beispiel 14: H-(L)-Val-[Phe^{NN}Cha]←(L)-Val)-H · 3HCl:

Analog Beispiel 10a erhält man aus 632 mg (0,91 mMol) Boc-(L)-Val-[Phe^{NN}Cha]←((L)-Val)-Boc aus Beispiel 10 nach Lyophilisation die Titelverbindung als Hydrochlorid. FAB-MS: (M+H)⁺=490, t_{Ret}(II)=29,4 min, R_{f}(K)=0,23.

### Beispiel 15: N-(3-Pyridylacetyl)-(L)-Val-[Phe^{NN}Leu]←(N-(3-pyridylacetyl)-(L)-Val):

Analog Beispiel 9 erhält man aus 90 mg (0,25 mMol) H-[Phe^{NN}Leu]-H · 3 HCl aus Beispiel 13 a), 358 mg (1,52 mMol) N-(3-Pyridylacetyl)-(L)-valin, 576 mg (1,52 mMol) HBTU und 316 µl (2,5 mMol) Triethylamin nach chromatographischer Reinigung mit Methylenchlorid/Methanol (15:1) und Lyophilisation aus Dioxan/tert-Butanol/Wasser die Titelverbindung. FAB-MS: (M+H)⁺= 688, t_{Ret}(IV)= 15,5 min, R_{f}(D)= 0,37.

### Beispiel 16: N-Trifluoracetyl-[Phe^{NN}(p-F)Phe]-Boc:

Eine Lösung von 4,0 g (15,4 mMol) 2(R)-[1′(S)-(Trifluoracetyl-amino)-2′-phenylethyl]-oxiran und 3,89 g (16,2 mMol) tert-Butyl-3-(p-fluorphenyl-methyl)-carbazat in 35 ml Methanol werden während ca. 20 h im Bombenrohr auf 80°C erhitzt. Das Reaktions-gemisch wird eingedampft, der Rückstand in wenig Dichlormethan gelöst und daraus die Titelverbindung mit Hexan gefällt (Kühlschrank). Weiteres Produkt liefert eine Säulenchromatographie (SiO₂, Methylenchlorid/Ether 95:7). DC R_{f}(J)=0,57; t_{Ret}(I)=24,3 min; FAB-MS (M+H)⁺=500.

Die Ausgangsmaterialien werden wie folgt hergestellt:

### a) N-3(S)-(Boc-amino)-2(R,S)-hydroxy-4-phenyl-1-trimethylsilyl-butan:

Unter N₂-Atmosphäre werden 24,7 g (1,02 Mol) Magnesium in 100 ml abs. Ether vorgelegt und über 35 min mit wenig Jod und gleichzeitig mit 132,5 ml (0,95 Mol) Chlor-methyltrimethylsilan und 300 ml Ether versetzt, wobei die Temperatur mittels Eisbad bei 38°C gehalten wird. Das erhaltene Reaktionsgemisch wird dann 1,5 h bei RT gerührt. Nach Abkühlen auf -60°C wird mit einer Suspension von 48,6 g (0,195 Mol) N-Boc-phenylalaninal (Herstellung: D.J. Kempf, J. Org. Chem. 51, 3921 ( 1986)) in 1,11 Ether innerhalb 40 min versetzt. Über 90 min wird das Reaktionsgemisch auf RT erwärmt und weitere 90 min bei dieser Temperatur gerührt. Danach wird auf 21 Eiswasser und 1,5l 10 %-ige wässrige Zitronensäure gegossen. Die abgetrennte wässrige Phase wird zweimal mit 500 ml Ether extrahiert. Alle Etherextrakte werden mit 500 ml 10 %-iger Zitronensäure und zweimal mit Sole gewaschen. Nach Trocknen über Natriumsulfat wird unter Vakuum eingeengt und die erhaltene Titelverbindung ohne zusätzliche Reinigung weiterverwendet. DC R_{f}(L)= 0,6; FAB-MS (M+H)⁺= 338.

### b) 1-Phenyl-3-buten-2(S)-amin:

Eine Lösung von 18,8 g (0,055 Mol) 3(S)-(Boc-amino)-2(R,S)-hydroxy-4-phenyl-1-tri-methylsilyl-butan in 420 ml Methylenchlorid wird bei 5°C innerhalb von 10 min mit 35,6 ml (0,28 Mol) einer ungefähr 48 %-igen Lösung von Bortrifluorid in Ether versetzt. Das Reaktionsgemisch wird dann 16 h bei RT gerührt, auf 10°C gekühlt und innerhalb von 20 min mit 276 ml einer 4 N Natriumhydroxydlösung versetzt. Die wässrige Phase wird abgetrennt und zweimal mit je 400 ml Methylenchlorid extrahiert. Die vereinigten organischen Extrakte werden mit Sole gewaschen und über Natriumsulfat getrocknet. Das Titelprodukt wird ohne zusätzliche Reinigung weiterverwendet. DC R_{f} (C)= 0,15 ; IR (Methylenchlorid) (cm⁻¹): 3370, 3020, 2920, 1640, 1605.

### c) N-Trifluoracetyl-1-phenyl-3-buten-2(S)-amin:

Gelöst in 210 ml Methylenchlorid und 70 ml Pyridin, werden 11,9 g (81 mMol) 1-Phenyl-3-buten-2(S)-amin bei 0°C tropfenweise mit 17,0 ml (121 mMol) Trifluoressigsäureanhydrid versetzt. Nach 0,5 h Rühren bei 0 °C wird 2x mit verd. HCI, Wasser und Sole extrahiert. Die wässrigen Phasen wäscht man noch 2x mit Methylenchlorid, trocknet sie mit Natriumsulfat und dampft sie ein: DC R_{f}(M)=0,4.

### d) 2(R)-[1′(S)-(Trifluoracetyl-amino)-2′-phenylethyl]-oxiran:

Eine Lösung von 14,5 g (60 mMol) N-Trifluoracetyl-1-phenyl-3-buten-2(S)-amin in 600 ml Chloroform versetzt man mit 54,28 g (314 mMol) m-Chlorperbenzoesäure und rührt sie 24 h bei RT aus. Das Reaktionsgemisch wird 2x mit 10 %-iger Natriumsulfitlösung, 2x mit ges. Natriumcarbonatlösung, Wasser und Sole gewaschen. Die wässrigen Phasen werden noch 2x mit Methylenchlorid extrahiert, die vereinigten organischen Phasen mit Natriumsulfat getrocknet und eingedampft, was die Titelverbindung liefert, die ohne weitere Reinigung in der nächsten Stufe eingesetzt wird: DC R_{f}(N)=0,6.

### e) p-Fluorphenylcarbaldehyd-tert-butoxycarbonylhydrazon:

Analog Beispiel 4 b) werden 32 g (242 mMol) tert-Butylcarbazat und 30 g (242 mMol) p-Fluorbenzaldehyd in 300 ml Ethanol bei 80°C während 3 h zur Titelverbindung umgesetzt, die beim Abkühlen und Verdünnen mit Wasser auskristallisiert: DC R_{f}(N)=0,48; t_{Ret}(I)=19,4 min.

### f) tert-Butyl-3-(p-fluorphenyl-methyl)-carbazat:

Analog Beispiel 4 a) werden 55 g (231 mMol) p-Fluorphenylcarbaldehyd-tert-butoxycarbonylhydrazon in 500 ml THF mit 5,5 g Palladium (5 %) auf Kohle zur Titelverbindung hydriert: ¹H-NMR (200 MHz, CD₃OD): 7,35 (dd, 8 und 6 Hz, 2 H), 7,05 (t, 8 Hz, 2 H), 3,9 (s, 2 H), 1,45 (s, 9 H).

### Beispiel 17: N-Morpholinocarbonyl-(L)-Val-[Phe^{NN}(p-F)Phe]-Boc:

Eine Gemisch von 185 mg (0,80 mMol) N-Morpholinocarbonyl-(L)-valin (Herstellung siehe Beispiel 7 a), 270 mg (0,67 mMol) H-[Phe^{NN}(p-F)Phe]-Boc, 311 mg (0,70 mMol) BOP und 95 mg (0,70 mMol) HOBT wird bei RT in 6,8 ml NMM/DMF 0,3 M gelöst und 5 h bei RT gerührt. Das Reaktionsgemisch wird am HV eingedampft und der Rückstand verteilt zwischen 4 Portionen Methylenchlorid, 2 Portionen 1 M Natriumcarbonatlösung, Wasser und Sole. Die über Natriumsulfat getrockneten vereinigten organischen Phasen werden eingedampft und durch Säulenchromatographie (SiO₂, Essigsäureethylester) gereinigt: DC R_{f}(O)=0,38; t_{Ret}(I)=21,8 min; FAB-MS (M+H)⁺=616.

Das Ausgangsmaterial wird wie folgt hergestellt:

### a) H-[Phe^{NN}(p-F)Phe]-Boc:

Bei 70°C tropft man zu einer Lösung von 0,3 g (0,6 mMol) N-Trifluoracetyl-[Phe^{NN}(p-F)-Phe]-Boc (Herstellung siehe Beispiel 16) in 50 ml Methanol unter N₂-Atmosphäre 15 ml einer 1 M wässrigen Lösung von Kaliumcarbonat und rührt 25 h bei dieser Temperatur nach. Das Reaktionsgemisch wird am HV eingedampft, der Rückstand mit Methylenchlorid versetzt und 2x mit Wasser und Sole gewaschen. Die wässrigen Phasen werden 2x mit Methylenchlorid extrahiert, die organischen Phasen mit Natriumsulfat getrocknet und eingedampft. Das Rohprodukt setzt man ohne weitere Reinigung in der nächsten Stufe ein. t_{Ret}(I)= 16,2 min.

### Beispiel 18: N-Morpholinocarbonyl-(L)-Val-[Phe^{NN}(p-F)Phe]←((L)-Val)-Z:

Eine Lösung von 86 mg (0,34 mMol) Z-(L)-Val und 160 mg (0,31 mMol) N-Morpholinocarbonyl-(L)-Val- [Phe^{NN}(p-F)Phe]-H in 2,7 ml NMM/CH₃CN 0,25 M (0,25 M NMM in CH₃CN) wird mit 129 mg (0,34mMol) HBTU versetzt. Nach 4 h bei RT dampft man ein und verteilt den Rückstand zwischen 3 Portionen Methylenchlorid, 2 Portionen ges. Natriumbicarbonatlösung und Sole. Trocknen der organischen Phasen mit Natriumsulfat und Eindampfen liefert die Titelverbindung, die nach Digerieren aus Methylenchlorid/Ether 1:1 rein erhalten wird: DC R_{f}(P)=0,4; t_{Ret}(I)=22,4 min; FAB-MS (M+H)⁺=749.

Das Ausgangsmaterial wird wie folgt hergestellt:

### a) N-Morpholinocarbonyl-(L)-Val-[Phe^{NN}(p-F)Phe]-H:

Gelöst in 105 ml Ameisensäure, werden 210 mg (0,34 mMol) N-Morpholinocarbonyl-(L)-Val-[Phe^{NN}(p-F)Phe]-Boc (Beispiel 17) 4 h bei RT gerührt. Anschliessend wird eingedampft, der Rückstand in Methylenchlorid aufgenommen und die Lösung mit ges. Natriumbicarbonatlösung und Sole gewaschen. Extraktion der wässrigen Phasen mit 2 Portionen Methylenchlorid, Trocknen der organischen Phasen mit Natriumsulfat und Eindampfen liefert die Titelverbindung, die ohne weitere Reinigung in der nächsten Stufe eingesetzt wird: t_{Ret}(I)=12,9.

### Beispiel 19: N-Morpholinocarbonyl-(L)-Val-[Phe^{NN}(p-F)Phe]←((L)-Val)-H:

Bei Normaldruck werden 160 mg (0,21 mMol) N-Morpholinocarbonyl-(L)-Val-[Phe^{NN}(p-F)Phe]←((L)-Val)-Z (Beispiel 18) in 6 ml Ethanol mit 40 mg Palladium (10 %) auf Kohle hydriert. Nach Filtration durch Celite® (Kieselgur, Filterhilfsmittel von Fluka, Buchs, Schweiz), Eindampfen und Lyophilisieren aus Dioxan erhält man die Titel-verbindung: t_{Ret}(Hydrochlorid, I)=13,4 min; FAB-MS (M+H)⁺=615.

### Beispiel 20: N-Morpholinocarbonyl-(L)-Val-[Phe^{NN}(p-F)Phe]←((L)-Val)←(N-morpholinocarbonyl-Gly):

Eine Lösung von 26,9 mg (0,143 mMol) N-Morpholinocarbonyl-glycin und 80 mg (0,130 mMol) N-Morpholinocarbonyl-(L)-Val-[Phe^{NN}(p-F)Phe]←((L)-Val)-H in 1,1 ml NMM/CH₃CN 0,25 M wird mit 54 mg (0,143 mMol) HBTU versetzt und 16 h bei RT gerührt. Man dampft ein und verteilt den Rückstand zwischen 3 Portionen Essigsäureethylester, Wasser, 2 Portionen ges. Natriumbicarbonatlösung, Wasser und Sole. Trocknen der organischen Phasen mit Natriumsulfat und Eindampfen liefert die Titelverbindung, die nach Lösen in wenig DMF und Fällen mit DIPE rein anfällt: t_{Ret}(I)=15,1 min; FAB-MS (M+H)⁺=785.

Das Ausgangsmaterial wird wie folgt hergestellt:

### a) N-Morpholinocarbonyl-glycin-benzylester:

Analog Beispiel 7 b) werden 7,69 g (22,8 mMol) Glycin-benzylester-4-toluolsulfonat und 2,8 g (19 mMol) (Morpholinocarbonyl)chlorid in 118 ml Methylenchlorid und 9 ml (53 mMol) N-Ethyldiisopropylamin während 18 h umgesetzt. Die Titelverbindung fällt nach Extraktion mit Methylenchlorid und Digerieren aus Hexan rein an: t_{Ret}(I)=11,6 min.

### b) N-Morpholinocarbonyl-glycin:

Analog Beispiel 7 a) werden 4,8 g ( 18,3 mMol) N-Morpholinocarbonyl-glycin-benzylester in 100 ml Essigsäureethylester mit 1 g Palladium (10 %) auf Kohle zur Titelverbindung hydriert: ¹H-NMR (300 MHz, CDCl₃): 3,88 (s, 2 H), 3,64 (s, 4 H), 3,50 (s, 2 H), 3,35 (s , 4H).

### Beispiel 21: Z-(L)-Val-[Phe^{NN}(p-F)Phe]-Boc:

Eine Lösung von 335 mg ( 1,33 mMol) Z-(L)-Val und 448 mg (1,11 mMol) H-[Phe^{NN}(p-F)Phe]-Boc (Herstellung siehe Beispiel 17 a) in 9,4 ml NMM/CH₃CN 0,25 M (0,25 M NMM in CH₃CN) wird mit 463 mg (1,22mMol) HBTU versetzt. Nach 16 h Rühren bei RT dampft man ein und verteilt den Rückstand zwischen 3 Portionen Methylenchlorid, 2 Portionen ges. Natriumbicarbonatlösung und Sole. Trocknen der organischen Phasen mit Natriumsulfat und Eindampfen liefert die Titelverbindung, die durch Säulenchromatographie (SiO₂, Hexan/Essigsäureethylester 4:1→1:1) gereinigt wird: t_{Ret}(I)=26,6 min; FAB-MS (M+H)⁺=637.

### Beispiel 22: Z-(L)-Val-[Phe^{NN}(p-F)Phe]←((L)-Val)-Boc:

Analog Beispiel 18) werden 165 mg (0,76 mMol) Boc-(L)-Val und 371 mg (0,69 mMol) Z-(L)-Val-[Phe^{NN}(p-F)Phe]-H in 6 ml NMM/CH₃CN 0,25 M mit 289 mg (0,76 mMol) HBTU zur Titelverbindung umgesetzt, die direkt aus der Reaktionslösung kristallisiert und abfiltriert werden kann: t_{Ret}(I)=27,2 min; FAB-MS (M+H)+=736.

Das Ausgangsmaterial wird wie folgt hergestellt:

### a) Z-(L)-Val-[Phe^{NN}(p-F)Phe]-H:

Analog Beispiel 18 a) werden 440 mg (0,69 mMol) Z-(L)-Val-[Phe^{NN}(p-F)Phe]-Boc mit 212 ml Ameisensäure zur Titelverbindung entschützt: t_{Ret}(I)=17,8 min.

### Beispiel 23: Z-(L)-Val-[Phe^{NN}(p-F)Phe]←((L)-Val)-H:

Analog Beispiel 18 a) werden 250 mg (0,34 mMol) Z-(L)-Val-[Phe^{NN}(p-F)Phe]←((L)-Val)-Boc (Beispiel 22) mit 50 ml Ameisensäure zur Titelverbindung entschützt: t_{Ret}(I)=18,0 min; FAB-MS (M+H)⁺=636.

### Beispiel 24: Z-(L)-Val-[Phe^{NN}(p-F)Phe]←((L)-Val)←(N-morpholinocarbonyl-Gly):

Analog Beispiel 20) werden 32 mg (0,17 mMol) N-Morpholinocarbonyl-glycin (Beispiel 20 b) und 99 mg (0,16 mMol) Z-(L)-Val-[Phe^{NN}(p-F)Phe]←((L)-Val)-H in 1,3 ml NMM/CH₃CN 0,25 M mit 65 mg (0,17 mMol) HBTU zur Titelverbindung umgesetzt, die direkt aus der Reaktionslösung kristallisiert: t_{Ret}(I)=21,1 min; FAB-MS (M+H)⁺=806.

### Beispiel 25: Z-(L)-Asn-[Phe^{NN}(p-F)Phe]-Boc:

Zu einer Lösung von 2,09 g (5,2 mMol) H-[Phe^{NN}(p-F)Phe]-Boc (Herstellung siehe Beispiel 17 a) in 68 ml DMF und 2,7 ml (16 mMol) N-Ethyl-diisopropylamin gibt man 3,0 g (7,8 mMol) Z-(L)-Asparagin-p-nitrophenylester (Bachem, Bubendorf/Schweiz). Nach 16 h Rühren bei RT wird am HV eingedampft, der Rückstand in viel Methylenchlorid aufgenommen (schlecht löslich) und mit 2 Portionen 5 %-iger Kaliumcarbonatlösung gewaschen. Die wässrigen Phasen extrahiert man noch 2x mit viel Methylenchlorid, trocknet die vereinigten organischen Phasen mit Natriumsulfat und dampft sie ein. Nach Lösen des Rohproduktes in wenig Methanol und Fällen durch Zugabe von Toluol bei -20°C erhält man die Titelverbindung: t_{Ret}(I)=21,2 min.

### Beispiel 26: H-(L)-Asn-[Phe^{NN}(p-F)Phe]-Boc:

Analog Beispiel 19) werden 0,40 g (0,61 mMol) Z-(L)-Asn-[Phe^{NN}(p-F)Phe]-Boc in 20 ml Methanol zur Titelverbindung hydriert: t_{Ret}(I)= 14,9 min.

### Beispiel 27: Chinolin-2-carbonyl-(L)-Asn-[Phe^{NN}(p-F)Phe]-Boc:

Analog Beispiel 17) werden 134 mg (0,78 mMol) Chinolin-2-carbonsäure (Fluka, Buchs/Schweiz) in 4 ml NMM/DMF 0,3 M mit 344 mg (0,78 mMol) BOP, 105 mg (0,78 mMol) HOBT und 268 mg (0,52 mMol) H-(L)-Asn-[Phe^{NN}(p-F)Phe]-Boc umgesetzt. Da laut HPLC nach 16 h Rühren bei RT noch H-(L)-Asn-[Phe^{NN}(p-F)Phe]-Boc vorhanden ist, werden nochmals 299 mg BOP, 70 mg HOBT, 89 mg Chinaldinsäure und 113 µl NMM zugesetzt. Nach weiteren 16 h wird eingedampft und der Rückstand zwischen 3 Portionen Methylenchlorid, 2 Portionen ges. Natriumbicarbonatlösung und Sole verteilt. Die vereinigten organischen Phasen werden mit Natriumsulfat getrocknet und eingedampft. Lösen des Rohproduktes in wenig DMF, Fällen mit DIPE und Kühlen auf -20°C liefert die Titelverbindung: t_{Ret}(I)=22,8 min; FAB-MS (M+H)⁺=673.

### Beispiel 28: Z-(L)-Asn-[Phe^{NN}(p-F)Phe]←((L)-Val)-Z:

88 mg (0,35 mMol) Z-(L)-Val in 3,8 ml NMM/DMF 0,3 N werden mit 153 mg (0,35 mMol) BOP und 47 mg (0,35 mMol) HOBT aktiviert und nach 15 min mit 144 mg (0,23 mMol) Z-(L)-Asn-[Phe^{NN}(p-F)Phe]-H·2 HCl versetzt. Nach 14 h Rühren bei RT dampft man das Reaktionsgemisch ein, löst den Rückstand in 2 ml Methanol und verteilt zwischen 3 Portionen Methylenchlorid und 2 Portionen 1 M Natriumcarbonatlösung, trocknet die organischen Phasen mit Natriumsulfat und dampft sie ein. Wiederholtes Lösen des Rohproduktes in wenig DMF und Ausfällen mit DIPE liefert die Titelverbindung: t_{Ret}(I)=22,2 min; FAB-MS (M+H)⁺=785.

Das Ausgangsmaterial wird wie folgt hergestellt:

### a) Z-(L)-Asn-[Phe^{NN}(p-F)Phe]-H·2 HCl:

Unter N₂-Atmosphäre wird eine Lösung von 150 mg (0,23 mMol) Z-(L)-Asn-[Phe^{NN}(p-F)Phe]-Boc (Beispiel 25) in 1 ml Dioxan mit 2 ml HCl/Dioxan 4 N (Fluka, Buchs/Schweiz) versetzt. Nach 1,5 h Rühren bei RT nwird das Reaktionsgemisch lyophilisiert und das Lyophilisat sofort weiter umgesetzt.

### Beispiel 29: Trifluoracetyl-[Phe^{NN}(p-F)Phe]←((L)-Val)-Z:

Analog Beispiel 17) werden 239 mg (0,95 mMol) Z-(L)-Val in 10,5 ml NMM/DMF 0,3 M mit 421 mg (0,95 mMol) BOP, 129 mg (0,95 mMol) HOBT und 0,3 g (0,63 mMol) N-Tri-fluoracetyl-[Phe^{NN}(p-F)Phe]-H während 15 h umgesetzt. Säulenchromatographie (SiO₂, Methylenchlorid/Ether 10:1) und Fällen aus DMF-Lösung mit DIPE liefert die Titelverbindung: DC R_{f}(Q)=0,15; t_{Ret}(I)=25,9 min; FAB-MS (M+H)⁺=633.

Das Ausgangsmaterial wird wie folgt hergestellt:

### a) N-Trifluoracetyl-[Phe^{NN}(p-F)Phe]-H:

Bei 0°C werden 0,20 g (0,40 mMol) N-Trifluoracetyl-[Phe^{NN}(p-F)Phe]-Boc (Herstellung siehe Beispiel 16) in 5 ml Methylenchlorid mit 5 ml Trifluoressigsäure versetzt. Nach 4 h Rühren bei 0°C und 2 h bei RT wird das Reaktionsgemisch eingedampft. Lyophilisation des Rückstandes aus Dioxan liefert die Titelverbindung, die ohne Reinigung weiter umgesetzt wird: t_{Ret}(I)= 14,7 min.

### Beispiel 30: Z-(L)-Asn-[Phe^{NN}Phe]-Boc:

Analog Beispiel 25) werden 167 mg (0,34 mMol) H-[Phe^{NN}Phe]-Boc in 3,6 ml DMF und 0,18 ml (1 mMol) N-Ethyl-diisopropylamin mit 0,20 g (0,52 mMol) Z-(L)-Aspara-gin-p-nitrophenylester zur Titelverbindung umgesetzt, die nach Säulenchromatographie (SiO₂, Essigsäureethylester) rein anfällt: DC R_{f}(O)=0,19; t_{Ret}(I)=20,9 min.

Das Ausgangsmaterial wird wie folgt hergestellt:

### a) N-Trifluoracetyl-[Phe^{NN}Phe]-Boc:

Analog Beispiel 16) werden 1,82 g (7,0 mMol) 2(R)-[(1′(S)-(Trifluoracetyl-amino)-2′-phenylethyl]-oxiran (Beispiel 16 d) und 1,58 g (7,1 mMol) tert-Butyl-3-benzyl-carbazat (J. Chem., Perkin I, 1712 (1975)) in 15 ml Methanol im Bombenrohr zur Titelverbindung umgesetzt, die durch Säulenchromatographie (SiO₂, Methylenchlorid/Ether 50:1) isoliert wird: DC R_{f}(J)=0,38; t_{Ret}(I)=24,5 min.

### b) H-[Phe^{NN}Phe]-Boc:

Analog Beispiel 17 a) werden 258 mg (0,53 mMol) N-Trifluoracetyl-[Phe^{NN}Phe]-Boc in 60 ml Methanol mit 10,7 ml 1 M Kaliumcarbonatlösung zur Titelverbindung umgesetzt.

### Beispiel 31: Z-(L)-Val-[(p-F)Phe^{NN}(p-F)Phe]-Boc:

Analog Beispiel 21) werden 18 mg (0,070 mMol) Z-(L)-Val und 27 mg (0,064 mMol) H-[(p-F)Phe^{NN}(p-F)Phe]-Boc in 0,6 ml NMM/CH₃CN 0,25 M mit 26,6 mg (0,070m Mol) HBTU zur Titelverbindung umgesetzt, die durch Lösen in wenig Methylenchlorid und Ausfällen mit DIPE gereinigt wird: FAB-MS (M+H)⁺=655.

Das Ausgangsmaterial wird wie folgt hergestellt:

### a) N-Boc-(p-fluorphenylalanin):

In 0,4 l Dioxan/Wasser 1:1 werden 20 g (109 mMol) p-Fluorphenylalanin (Fluka, Buchs, Schweiz) mit 35,5 g (163 mMol) Boc-anhydrid und 150 g (1,09 Mol) Kaliumcarbonat umgesetzt. Nach 4 h wird das Reaktionsgemisch mit Zitronensäurelösung angesäuert und mit 3 Portionen Essigsäureethylester extrahiert. Die organischen Phasen wäscht man mit 10 %-iger Zitronensäure, Wasser und Sole, trocknet sie mit Natriumsulfat und dampft sie ein. Lösen des Rückstands in wenig Methylenchlorid und Kristallisieren durch Zusatz von Hexan liefert die Titelverbindung: t_{Ret}(I)= 16,9 min.

### b) N-Boc-(p-fluorphenylalaninol):

Bei -5°C bis -10°C versetzt man eine Lösung von 17,9 g (63 mMol) N-Boc-(p-fluorphenylalanin) in 73 ml abs. THF mit 9,66 ml (69 mMol) Triethylamin und tropft eine Lösung von 9,05 ml (69 mMol) Chlorameisensäureisobutylester in 44 ml abs. THF zu. Nach 0,5 h Rühren bei RT wird der gebildete Niederschlag abgenutscht. Das Filtrat tropft man unter Kühlen zu 4,77 g (126 mMol) Natriumborhydrid in 28 ml Wasser. Nach 4 h Rühren bei RT säuert man mit 10 %-iger Zitronensäure an, dampft das THF am RV teilweise ab und verteilt den Rückstand zwischen 3 Portionen Essigsäureethylester, 2 Portionen 2 N Natriumhydroxidlösung, Wasser, ges. Natriumhydrogencarbonatlösung und Sole. Die mit Natriumsulfat getrockneten und eingedampften organischen Phasen liefem nach Lösen in wenig Methylenchlorid und Kristallisieren durch Zugabe von Hexan die Titelverbindung: DC R_{f}(N)-=0,36; t_{Ret}(I)=16,8 min; ¹H-NMR (200 MHz, CD₃OD): 7,24 (dd, 8 und 5 Hz, 2 H), 6,98 (t, 8 Hz, 2 H), 3,73 (m, 1 H), 3,47 (d, 5 Hz, 2 H), 2,88 (dd, 13 und 6 Hz, 1 H), 2,62 (dd, 13 und 8 Hz, 1 H), 1,36 (s, 9 H).

### c) N-Boc-(p-fluorphenylalaninal):

Unter N₂-Atmosphäre werden zu einer auf -60°C abgekühlten Lösung von 4,0 ml (46,8 mMol) Oxalylchlorid in 44 ml Methylenchlorid 4,44 ml (62,4 mMol) DMSO gelöst in 76 ml Methylenchlorid getropft. Nach 15 min Rühren fügt man der klaren Reaktionslösung 8,4 g (31,2 mMol) N-Boc-(p-fluorphenylalaninol) als Lösung in 185 ml Methylenchlorid/THF 1:1 (→ Ausfällung) zu und rührt 25 min nach. Anschliessend setzt man 17,3 ml (124,8 mMol) Triethylamin gelöst in 38 ml Methylenchlorid zu. Nach 30 min Rühren werden 278 ml einer 20 %-igen Kaliumhydrogensulfatlösung zugetropft, gefolgt von 220 ml Hexan. Man lässt auf RT aufwärmen, trennt die wässrige Phase ab und extrahiert sie mit 2 Portionen Ether. Die organischen Phasen ergeben nach Waschen mit ges. Natriumbicarbonatlösung, Wasser und Sole, Trocknen mit Natriumsulfat und Eindampfen die Titelverbindung, die ohne weitere Reinigung in der nächsten Stufe eingesetzt wird: ¹H-NMR (200 MHz, CDCl₃): 9,63 (s, 1 H), 6,9-7,2 (2m, 4 H), 5,04 (m, 1 H), 4,42 (m, 1 H), 3,10 (m, 2 H), 1,43 (s, 9 H).

### d) N-3(S)-(Boc-amino)-2(R,S)-hydroxy-4-(p-fluorphenyl)-1-trimethylsilyl-butan:

Analog Beispiel 16 a) werden 1,63 g (67 mMol) Magnesium in 33 ml abs. Ether, mit 8,3 ml (60 mMol) Chlormethyltrimethylsilan zur Grignard-Verbindung umgesetzt, die nach Reaktion mit 13 mMol N-Boc-(p-fluorphenylalaninal), Extraktion und Säulenchromatographie (SiO₂, Hexan/Essigsäureethylester 5:1 → 4:1) das Diastereomerengemisch der Titelverbindungen liefert: DC R_{f}(L)=0,32; t_{Ret}(I)=24,9 min (22 %)/ 25,5 min (78 %); FAB-MS (M+H)⁺=356.

### e) 1-(p-Fluorphenyl)-3-buten-2(S)-amin:

Analog Beispiel 16 b) werden 1,1 g (3,1 mMol) N-3(S)-(Boc-amino)-2(R,S)-hydroxy-4-(p-fluorphenyl)-1-trimethylsilyl-butan in 22 ml Methylenchlorid mit 1,9 ml ( 15,5 mMol) einer ungefähr 48 %-igen Lösung von Bortrifluorid in Ether zur Titelverbindung umgesetzt: ¹H-NMR (300 MHz, CDCl₃): 7,2-7,10 und 7,05-6,9 (2m, je 2 H), 5,9-5,8 (m, 1 H), 5,2-5,0 (m, 2 H), 3,57 (m, 1 H), 2,79 (dd, 12 und 6 Hz, 1 H), 2,62 (dd, 12 und 8 Hz, 1 H), 1,7 (sb, 2 H).

### f) N-Trifluoracetyl-1-(p-fluorphenyl)-3-buten-2(S)-amin:

Analog Beispiel 16 c) werden 364 mg (2,2 mMol) 1-(p-Fluorphenyl)-3-buten-2(S)-amin in 1,8 ml Methylenchlorid und 5,4 ml Pyridin mit 460 µl (3,3 mMol) Trifluoressigsäureanhydrid zur Titelverbindung umgesetzt, die nach Digerieren in Hexan rein anfällt: DC R_{f}(F)=0,58; MS (M)⁺=261.

### g) 2(R)-[1′(S)-(Trifluoracetyl-amino)-2′-(p-fluorphenyl)ethyl]-oxiran:

Analog Beispiel 16 d) werden 359 mg (1,37 mMol) N-Trifluoracetyl-1-(p-fluor-phenyl)-3-buten-2(S)-amin in 9 ml Chloroform mit 1,18 g (6,87 mMol) m-Chlorperben-zoesäure zur Titelverbindung oxidiert: DC R_{f}(R)=0,45.

### h) N-Trifluoracetyl-[(p-F)Phe^{NN}(p-F)Phe]-Boc:

Analog Beispiel 16) werden 415 mg [1,49 mMol) 2(R)-(1′(S)-(Trifluoracetyl-amino)-2′-(p-fluorphenyl)ethyl]-oxiran und 377 mg (1,57 mMol) tert-Butyl-3-(p-fluorphenyl-methyl)-carbazat in 9 ml Methanol zur Titelverbindung umgesetzt: DC R_{f}(S)=0,53; FAB-MS (M+H)⁺=518; ¹H-NMR (300 MHz, CD₃OD): 7,4-7,3 und 7,3-7,2 (2m, je 2 H), 7,05-6,9 (m, 4 H), 4,23 (m, 1 H), 3,90-3,65 (m, 3 H), 3,03-2,78 und 2,74-2,60 (2m, je 2 H), 1,30 (s, 9 H).

### i) H-[(p-F)Phe^{NN}(p-F)Phe]-Boc:

Analog Beispiel 17 a) werden 285 mg (0,55 mMol) N-Trifluoracetyl-[(p-F)Phe^{NN}(p-F)Phe]-Boc in 45 ml Methanol mit 14 ml 1 M Kaliumcarbonatlösung zur Titelverbindung umgesetzt: t_{Ret}(I)= 16,4 min.

### Beispiel 32: Z-(L)-Val-[(p-F)Phe^{NN}(p-F)Phe]-H:

Analog Beispiel 18 a) werden 215 mg (0,33 mMol) Z-(L)-Val-[(p-F)Phe^{NN}(p-F)Phe]-Boc mit 100 ml Ameisensäure zur Titelverbindung entschützt: FAB-MS (M+H)⁺=555.

### Beispiel 33: Z-(L)-Val-[(p-F)Phe^{NN}(p-F)Phe]←(N-(N-(2-pyridylmethyl)-N-methyl-aminocarbonyl)-(L)-Val):

Analog Beispiel 18) werden 23,6 mg (0,089 mMol) N-(N-(2-pyridylmethyl)-N-methylaminocarbonyl)-(L)-valin (Herstellung siehe EP 402646 A1, 19. Dez. 1990) und 45 mg (0,081 mMol) Z-(L)-Val-[(p-F)Phe^{NN}(p-F)Phe]-H mit 33,8 mg (0,089 mMol) HBTU in 0,76 ml NMM/CH₃CN 0,25 M zur Titelverbindung umgesetzt und mit DMF/DIPE umkristallisiert: DC R_{f}(O)=0,39; FAB-MS (M+H)⁺=802.

### Beispiel 34: Z-(L)-Val-[(p-F)Phe^{NN}(p-F)Phe]←(N-(2(R,S)-carbamoyl-3-phenyl-propionyl)(L)-Val):

Analog Beispiel 18) werden 26,0 mg (0,089 mMol) N-(2(R,S)-carbamoyl-3-phenyl-propionyl)-(L)-valin (Herstellung: Synth., Struct., Funct., Proc. Am. Pept. Symp., 7^{th}, 85, (1981)) und 45 mg (0,081 mMol) Z-(L)-Val-[(p-F)Phe^{NN}(p-F)Phe]-H (Beispiel 32) mit 33,8 mg (0,089 mMol) HBTU in 0,76 ml NMM/CH₃CN 0,25 M zur Titelverbindung umgesetzt und mit DMF/DIPE umkristallisiert: R_{f}(P)=0,64; FAB-MS (M+H)⁺=829

### Beispiel 35: Acetyl-Val-[Phe^{NN}Phe]←(N-acetyl-Val):

Analog Beispiel 7 erhält man aus 100 mg (0,25 mMol) H-[Phe^{NN}Phe]-H·3 HCl aus Beispiel 2a, 121 mg (0,76 mMol) N-Acetyl-(L)-valin, 288 mg (0,76 mMol) HBTU und 0,211 ml (1,52 mMol) Triethylamin in DMF die Titelverbindung nach Lyophilisation aus Dioxan. FAB-MS: (M+H)⁺=568, t_{Ret}(I)=15,0 min., R_{f} (B)=0,46.

### Beispiel 36: Z-(D)-Val-[Phe^{NN}Phe]←((D)-Val)-Z:

Analog Beispiel 2 erhält man aus 50 mg (0,123 mMol) H-[Phe^{NN}Phe]-H·3 HCl aus Beispiel 2a, 95 mg (0,38 mMol) Z-(D)-Valin, 168 mg (0,38 mMol) BOP, 51 mg (0,38 mMol) HOBt und 2,53 ml 0,3M NMM in DMF die Titelverbindung nach Lyophilisation aus Dioxan. FAB-MS: (M+H)⁺=752, t_{Ret}(I)=26,4 min, R_{f} (H)=0,21.

### Beispiel 37: Chinolin-2-carbonyl-Val-[Phe^{NN}Phe]←(N-chinolin-2-carbonyl-Val):

Man löst 145 mg (0,53 mMol) N-(Chinolin-2-carbonyl)-(L)-valin, 235 mg (0,53 mMol) BOP und 72 mg (0,53 mMol) HOBt in 3,5 ml einer 0,3 M Lösung von NMM in DMF, fügt nach 10 min 70 mg (0,18 mMol) H-[Phe^{NN}Phe]-H·HCl (Beispiel 2a) zu und rührt während 5 h bei RT unter Stickstoffatmosphäre. Die Reaktionsmischung wird eingedampft, der Rückstand in Methylenchlorid gelöst und zweimal mit ges. Natriumbicarbonatlösung, einmal mit 10 % Zitronensäure und erneut einmal mit ges. Natriumbicarbonatlösung gewaschen. Die organischen Phasen werden durch Watte filtriert, eingedampft und der Rückstand zweimal aus Methylenchlorid/Methanol durch Zugabe von DIPE ausgefällt. Nach Lyophilisation aus Dioxan erhält man die Titelverbindung als weissen Festkörper (Gemisch von zwei im HPLC unterscheidbaren Diastereomeren). FAB-MS: (M+H)⁺=794, t_{Ret}(A)=29,1 und 29,3 min, R_{f}(B)=0,81.

### a) N-(Chinolin-2-carbonyl)-(L)-valin:

Eine Lösung von 2,5 g (14,5 mMol) (L)-Valyl-tert-butylester und 2,5 g (14,5 mMol) Chinolin-2-carbonsäure in 100 ml Methylenchlorid/THF (10:1) wird mit 3,28 g (15,9 mMol) N,N-Dicyclohexylcarbodiimid und 2,0 ml (14,5 mMol) Triethylamin versetzt und während 18h bei RT gerührt. Die Reaktionsmischung wird auf -18° abgekühlt und vom Hanstoff abfiltriert. Das Filtrat wird eingedampft, der Rückstand in Methylenchlorid gelöst und je einmal mit ges. Natriumbicarbonatlösung und Wasser gewaschen. Die organischen Phasen werden durch Watte filtriert, eingedampft und ergeben nach chromatographischer Reinigung auf Kieselgel mit Hexan/Essigsäureethylester (2:1) N-(Chinolin-2-carbonyl)-(L)-valyl-tert-butylester. 2,59 g (12,2 mMol) davon werden während 4,5h bei RT in Methylenchlorid/TFA (1:1) belas sen. Nach dem Eindampfen wird der Rückstand durch Chromatographie auf Kieselgel mit Hexan/Essigsäureethylester (2:1) gereinigt. Die produkthaltigen Fraktionen werden eingedampft, erneut in Methylenchlorid gelöst und durch Waschen mit 1N Natronlauge und 1N Salzsäure in das Hydrochlorid der Titelverbindung übergeführt. ¹H-NMR (200 MHz, CD₃OD): 1,05 und 1,07 (2d, J=6Hz, 6H) 2,40 (m, 1H) 4,65 (m, 1H) 7,70 (m, 1H) 7,85 (m, 1H) 8,00 (dxd, 1H) 8,20 (m, 2H) 8,48 (d, 1H).

### Beispiel 38: Acetyl-(L)-Val-[Phe^{NN}Cha]←(N-acetyl-(L)-Val):

Analog Beispiel 37 erhält man aus 160 mg (0,40 mMol) H-[Phe^{NN}Cha]-H·3 HCl aus Beispiel 10a, 190 mg (1,19 mMol) N-Acetyl-(L)-valin, 525 mg (1,19 mMol) BOP, 160 mg (1,19 mMol) HOBt und 7,9 ml 0,3M NMM in DMF die Titelverbindung nach Fällung aus Chloroform/Methanol mit DIPE und Lyophilisation aus Dioxan. FAB-MS: (M+H)⁺=574, t_{Ret}(I)= 18,1 min, R_{f} (B)=0,30.

### Beispiel 39: N-(3-Pyridylacetyl)-(L)-Val-[Phe^{NN}Cha]←(N-(3-pyridylacetyl)-(L)-Val) ·3 HCl:

Analog Beispiel 7 erhält man aus 100 mg (0,25 mMol) H-[Phe^{NN}Cha]-H·3 HCl aus Beispiel 10a, 358 mg (1,52 mMol) N-(3-Pyridylacetyl)-(L)-valin aus Beispiel 9a, 576 mg (1,52 mMol) HBTU und 0,316 ml (2,28 mMol) Triethylamin in DMF die Titelverbindung nach chromatographischer Reinigung auf Kieselgel mit Methylenchlorid/Methanol ( 15:1) und Lyophilisation der produkthaltigen Fraktionen aus Dioxan. FAB-MS: (M+H)⁺=728, t_{Ret}(I)=11,3 min, R_{f} (U)=0,21.

### Beispiel 40: Acetyl-Ile-[Phe^{NN}Cha]←(N-acetyl-Ile):

Analog Beispiel 37 erhält man aus 160 mg (0,40 mMol) H-[Phe^{NN}Cha]-H·3 HCl aus Beispiel 10a, 206 mg (1,19 mMol) N-Acetyl-(L)-isoleucin, 525 mg (1,19 mMol) BOP, 160 mg (1,19 mMol) HOBt und 7,9 ml 0,3M NMM in DMF die Titelverbindung nach Ausfällen aus Methylenchlorid/Methanol durch Zugabe von DIPE und Lyophilisation aus Dioxan/tert-Butanol (Gemisch von 2 im HPLC unterscheidbaren Diasteromeren). FAB-MS: (M+H)⁺=602, t_{Ret}(I)=20,4 und 20,7 min, R_{f} (D)=0,33.

### Beispiel 41: Thiomorpholinocarbonyl-(L)-Val-[Phe^{NN}Cha]←(N-thiomorpholinocarbony]-(L)-Val):

Analog Beispiel 6 erhält man ausgehend von 70 mg (0,12 mMol) H-(L-Val)-[Phe^{NN}Cha]←(N-(L)-Val)-H·3 HCl aus Beispiel 14, 58 mg (0,35 mMol) (4-Thiomorpholinylcarbonyl)chlorid aus Beispiel 6a und 0,127 ml Triethylamin in 2 ml DMF nach chromatographischer Reinigung auf Kieselgel mit Methylenchlorid/Methanol (95:5) und Lyophilisation der produkthaltigen Fraktionen aus Dioxan die Titelverbindung. FAB-MS: (M+H)⁺=748, t_{Ret}(I)=24,0 min, R_{f} (B)=0,70.

### Beispiel 42: Z-(L)-Glu-[Phe^{NN}(p-F)Phe]←((L)-Glu)-Z:

Man rührt eine Lösung von 130 mg (0,14 mMol) Z-(L)-Glu(O-tert-butyl)-[Phe^{NN}(p-F)Phe]←((L)-Glu(O-tert-butyl))-Z [(Glu(O-tert-butyl) bedeutet hierin das an der γ-Carboxygruppe mit einem tert-Butylrest veresterte Radikal von Glutaminsäure] in 8 ml Methylenchlorid/TFA (1:1) während 3 h bei RT Das Lösungsmittel wird unter vermindertem Drück abgedampft und der Rückstand aus Methylenchlorid durch Zugabe von DIPE ausgefällt. Man erhält die Titelverbindung nach Lyophilisation aus Dioxan/tert-Butanol. FAB-MS: (M+H)+=830, t_{Ret}(I)=19,6 min, R_{f} (B)=0,32.

### a) Z-(L)-Glu(O-tert-butyl)-[Phe^{NN}(p-F)Phe]←((L)-Glu(O-tert-butyl))-Z:

Analog Beispiel 37 erhält man aus 100 mg (0,24 mMol) H-[Phe^{NN}(p-F)Phe]-H·3HCl, 245 mg (0,73 mMol) Z-(L)-Glutaminsäure-tert-butylester, 321 mg (0,73 mMol) BOP, 98 mg (0,73 mMol) HOBt und 4,8 ml 0,3M NMM in DMF die Titelverbindung nach chromatographischer Reinigung auf Kieselgel mit Methylenchlorid/Ether (1:1). t_{Ret}(I)=30,2 min, R_{f} (H)=0,17.

### b) H-[Phe^{NN}(p-F)Phe]-H·3 HCl:

Analog Beispiel 2a erhält man ausgehend von 1,77 g (3,51 mMol) Boc-[Phe^{NN}(p-F)Phe]-Boc nach Lyophilisation die Titelverbindung. FAB-MS: (M+H)⁺=304, R_{f} (K)=0,19.

### c) Boc-[Phe^{NN}(p-F)Phe]-Boc:

Analog Beispiel 1 erhält man ausgehend von 2,0 g (7,60 mMol) (2R)-[1′(S)-Boc-amino-2′-phenylethyl]oxiran und 2,17 g (9,04 mMol) tert-Butyl-3-(4-fluorphenyl-methyl)-carbazat aus Beispiel 16 f die Titelverbindung nach chromatographischer Reinigung auf Kieselgel mit Hexan/Essigsäureethylester (2:1). FAB-MS: (M+H)⁺=504, t_{Ret}(I)=26,2 min, R_{f} (F)=0,26.

### Beispiel 43: N-(2-Pyridylmethyl)-N-methyl-aminocarbonyl(L)-Val-[Phe^{NN}(p-F)-Phe]←(N-(N-(2-pyridylmethyl)-N-methyl-aminocarbonyl)-(L)-Val):

Analog Beispiel 37 erhält man aus 70 mg (0,17 mMol) H-[Phe^{NN}(p-F)Phe]-H·3 HCl aus Beispiel 42b, 135 mg (0,51 mMol) N-(N-(2-Pydylmethyl)-N-methyl-aminocarbonyl)-(L)-valin (Herstellung wie in EP 0 402 646 A1 vom 19. Dec. 1990 beschrieben), 225 mg (0,51 mMol) BOP, 69 mg (0,51 mMol) HOBt und 3,4 ml 0,3M NMM in DMF die Titelverbindung nach Chromatographie auf Kieselgel mit Methylenchlorid/Methanol (15:1) und Lyophilisation der produkthaltigen Fraktionen aus Dioxan. FAB-MS: (M+H)⁺=798, t_{Ret}(IV)=35 min, R_{f} (U)=0,21.

### Beispiel 44: N-(3-(Tetrazol-1-yl)-propionyl)-Val-[(Phe^{NN}(p-F)Phe]←(N-(3-(tetrazol-1-yl)-propionyl)-Val):

Analog Beispiel 37 erhält man aus 100 mg (0,24 mMol) H-[Phe^{NN})p-F)Phe]-H·3 HCl (aus Beispiel 42b), 146 mg (0,61 mMol) N-(3-(Tetrazol-1-yl)-propionyl)-(L)-valin, 268 mg (0,61 mMol) BOP, 82 mg (0,61 mMol) HOBt und 4 ml 0,3M NMM in DMF die Titelverbindung nach Ausfällung aus Methylenchlorid durch Zugabe von DIPE und Lyophilisation aus Dioxan (im HPLC 4 Diastereomere unterscheidbar). FAB-MS: (M+H)⁺=750, t_{Ret}(III)=30,8; 31,4; 32,4 und 32,8 min, R_{f} (K)=0,5.

### Beispiel 44a: N-(3-(Tetrazol-1-yl)-propionyl)-(L)-valin:

Analog Beispiel 9b erhält man ausgehend von 4 g (16,4 mMol) (L)-Valin-benzylester·HCl, 2,1 g (14,9 mmol) 3-(Tetrazol-1-yl)-propionsäure (Herstellung: US 4,794,109 A vom 27. Dec. 1988), 2,4 ml Cyanphosphonsäure-diethylester und 4,4 ml Triethylamin in DMF nach chromatographischer Reinigung auf Kieselgel mit Methylenchlorid/Methanol (30:1) N-(3-(Tetrazol-1-yl)-propionyl)-(L)-valin-benzylester. 2,66 g (8,03 mMol) davon werden in Methanol/Wasser (9:1) in Gegenwart von 530 mg 10 % Palladium auf Kohle bei 1 atm Wasserstoffdruck hydriert und ergeben nach Fällung aus Methanol/DIPE die Titelver-bindung. ¹H-NMR (200 MHz, CD₃OD): 0,9 (d, J=7Hz, 6H) 2,1 (m, 1H) 2,95 (m, 2H) 4,29 (d, J=6Hz, 1H) 4,78 (m, 2H) 9,15 (s,1H).

### Beispiel 45: Z-(L)-Val-[Phe^{NN}(p-F)Phe]←((L)-Val)-Z:

Analog Beispiel 37 erhält man aus 100 mg (0,24 mMol) H-[Phe^{NN}(p-F)Phe]-H·3 HCI (aus Beispiel 42b), 182 mg (0,38 mMol) Z-(L)-Valin, 321 mg (0,73 mMol) BOP, 98 mg (0,73 mMol) HOBt und 4,8 ml 0,3M NMM in DMF die Titelverbindung nach Ausfällen aus Methylenchlorid durch Zugabe von DIPE und Lyophilisation aus Dioxan. FAB-MS: (M+H)⁺=770, t_{Ret}(I)=26,3 min, R_{f} (H)=0,25.

### Beispiel 46: Acetyl-Val-[Phe^{NN}(p-F)Phe]←(N-acetyl-Val):

Analog Beispiel 37 erhält man aus 80 mg (0,19 mMol) H-[Phe^{NN}(p-F)Phe]-H·3 HCl aus Beispiel 42b), 124 mg (0,78 mMol) N-Acetyl-(L)-valin, 344 mg (0,78 mMol) BOP, 105 mg (0,76 mMol) HOBt und 4,5 ml 0,3M NMM in DMF die Titelverbindung nach zweimaliger Umfällung aus Methylenchlorid/Methanol durch Zugabe von DIPE und Lyophilisation aus Dioxan/tert-Butanol. FAB-MS: (M+H)⁺=586, t_{Ret}(I)=15,8 min, R_{f} (E)=0,32.

### Beispiel 47: Acetyl-Val-[Phe^{NN}(p-CN)Phe]←(N-acetyl-Val):

Analog Beispiel 37 erhält man aus 80 mg (0,19 mMol) H-[Phe^{NN}(p-CN)Phe]-H·3 HCI, 124 mg (0,78 mMol) N-Acetyl-(L)-valin, 344 mg (0,78 mMol) BOP, 105 mg (0,78 mMol) HOBt und 4,5 ml 0,3M NMM in DMF die Titelverbindung nach Fällung aus Methylenchlorid/Methanol durch Zugabe von DIPE und Lyophilisation aus Dioxan als Gemisch von 2 im HPLC unterscheidbaren Diastereomeren. FAB-MS: (M+H)⁺=593, t_{Ret}(I)=14,4 und 14,6 min, R_{f} (D)=0,39.

### a) H-[Phe^{NN}(p-CN)Phe]-H·3 HCl:

Analog Beispiel 2a erhält man ausgehend von 2,69 g (5,27 mMol) Boc-[Phe^{NN}(p-CN)Phe]-Boc nach Lyophilisation die Titelverbindung. FAB-MS: (M+H)⁺=311, R_{f} (K)=0,16.

### b) Boc-[Phe^{NN}(p-CN)Phe]-Boc:

Analog Beispiel 1 erhält man ausgehend von 2,0 g (7,60 mMol) (2R)-(1′(S)-Boc-amino-2′-phenylethyl]oxiran und 1,87 g (7,6 mMol) tert-Butyl-3-(4-cyanphenyl-me-thyl)-carbazat die Titelverbindung nach Kristallisation aus Methanol/DIPE. FAB-MS: (M+H)⁺=511, t_{Ret}(I)=25 min, R_{f} (Y)=0,19.

### c) tert-Butyl-3-(4-cyanphenyl-methyl)-carbazat:

Analog Beispiel 4b werden 10 g (76,3 mMol) 4-Cyanbenzaldehyd und 10 g (76,3 mMol) tert-Butylcarbazat in Ethanol zum 4-Cyanphenylcarbaldehyd-tert-butoxycarbonylhydrazon umgesetzt. 11,1 g davon werden in 150 ml THF in Gegenwart von 2 g Palladium auf Kohle 10% bei 2 atm Wasserstoffdruck hydriert und ergeben die Titelverbindung. ¹H-NMR (200 MHz, CDCl₃): 7,65 (d, J=8Hz, 2H), 7,45 (d, J=8 Hz, 2H), 6,08 (s, br, 1H), 4,3 (s, br, 1H), 4,02 (s, 2H), 1,45 (s, 9H).

### Beispiel 48: Z-(L)-Val-[Phe^{NN}(p-CN)Phe]←((L)-Val)-Z:

Analog Beispiel 37 erhält man aus 70 mg (0,17 mMol) H-[Phe^{NN}(p-CN)Phe]-H· 3 HCl (aus Beispiel 47a), 125 mg (0,5 mMol) Z-(L)-Valin, 221 mg (0,5 mMol) BOP, 68 mg (0,5 mMol) HOBt und 3,33 ml 0,3M NMM in DMF die Titelverbindung nach Fällung aus Methylenchlorid durch Zugabe von Hexan und Lyophilisation aus Dioxan. FAB-MS: (M+H)⁺=777, t_{Ret}(I)=25,3 min, R_{f} (D)=0,69.

### Beispiel 49: Z-(L)-Ile-[Phe^{NN}Leu]←((L)-Ile)-Z:

Analog Beispiel 37 erhält man aus 70 mg (0,19 mMol) H-[Phe^{NN}Leu]-H·3 HCl (aus Beispiel 13a), 154 mg (0,58 mMol) Z-(L)-Isoleucin, 257 mg (0,58 mMol) BOP, 79 mg (0,58 mMol) HOBt und 3,88 ml 0,3M NMM in DMF die Titelverbindung nach Chromatographie auf Kieselgel mit Methylenchlorid/Ether (3:1) und Fällung der produkthaltigen Fraktionen aus Methylenchlorid/DIPE und Lyophilisation aus Dioxan. FAB-MS: (M+H)⁺=746, t_{Ret}(I)=28,2 min, R_{f} (H)=0,39.

### Beispiel 50: Isobutoxycarbonyl- (L)-Val-[Phe^{NN}Leu]←(N-isobutoxycarbonyl-(L)-Val):

Analog Beispiel 37 erhält man aus 70 mg (0,19 mMol) H-[Phe^{NN}Leu]-H·3 HCl (aus Beispiel 13a), 130 mg (0,58 mMol) N-(Isobutoxycarbonyl)-(L)-valin, 256 mg (0,58 mMol) BOP, 78 mg (0,58 mMol) HOBt und 3,9 ml 0,3M NMM in DMF die Titelverbindung nach Chromatographie auf Kieselgel mit Methylenchlorid/Ether (1:1) und Lyophilisation der produkthaltigen Fraktionen aus Dioxan. FAB-MS: (M+H)⁺=650, t_{Ret}(I)=26,4 min, R_{f} (H)=0,38.

### a) N-(Isobutoxycarbonyl)-(L)-valin:

Eine Lösung von 10 g (85,3 mMol) (L)-Valin in 100 ml 2N Natronlauge wird mit 11,2 ml (85,3 mMol) Isobutylchloroformiat versetzt und bei RT während 18h gerührt. Die Reaktionslösung wird mit Methylenchlorid gewaschen, mit 4N Salzsäure angesäuert und mit Methylenchlorid extrahiert. Die organischen Extrakte werden mit Sole gewaschen, durch Watte filtriert und ergeben nach dem Eindampfen die Titelverbindung als farbloses Harz. ¹H-NMR (200 MHz, CD₃OD): 0,95 (m, 12H) 1,9 (m, 1H) 2,15 (m, 1H) 3,85 (d, J=7Hz, 2H) 4,05 (d breit, 1H).

### Beispiel 51: N-(3-(Tetrazol-1-yl)-propionyl)-(L)-Val-[Phe^{NN}Leu]←(N-3-(tetrazol-1-yl)-propionyl-(L)-Val):

Analog Beispiel 37 erhält man aus 150 mg (0,42 mMol) H-[Phe^{NN}Leu]-H·3 HCl (aus Beispiel 13a), 251 mg (1,04 mMol) N-(3-(Tetrazol-1-yl-propionyl)-(L)-valin aus Beispiel 44a, 460 mg (1,04 mMol) BOP, 140 mg (1,04 mMol) HOBt und 6,9 ml 0,3M N-Methyl-morpholin in DMF die Titelverbindung nach Fällung aus Methylenchlorid/DIPE und Lyophilisation aus Dioxan/tert-Butanol/Wasser. FAB-MS: (M+H)⁺=689, t_{Ret}(I)= 14,7 min, R_{f} (K)-=0,36.

### Beispiel 52: Acetyl-Val-[Phe^{NN}Leu]←(N-acetyl-Val):

Analog Beispiel 37 erhält man aus 70 mg (0,19 mMol) H-[Phe^{NN}Leu]-H·3 HCl (aus Beispiel 13a), 184 mg (1,16 mMol) N-Acetyl-(L)-valin, 512 mg (1,16 mMol) BOP, 156 mg (1,16 mMol) HOBt und 7,8 ml 0,3M NMM in DMF die Titelverbindung nach Fällung aus Methylenchlorid/Methanol durch Zugabe von DIPE und Lyophilisation aus Dioxan/tert-Butanol/Wasser (gemäss HPLC 2 Diasteromere unterscheidbar). FAB-MS: (M+H)⁺=534, t_{Ret}(I)= 14,7 und 15,1 min, R_{f} (D)=0,35.

### Beispiel 53: Boc-(L)-Val-[Phe^{NN}Leu]←((L)-Val)-Boc:

Analog Beispiel 7 erhält man aus 300 mg (0,83 mMol) H-[Phe^{NN}Leu]-H·3 HCl (aus Beispiel 13a), 722 mg (3,33 mMol) Boc-(L)-valin, 1,262 g (3,33 mMol) HBTU und 0,927 ml (6,66 mMol) Triethylamin in DMF die Titelverbindung nach chromatographischer Reinigung auf Kieselgel mit Methylenchlorid/Ether (1:1), Fällung der produkthaltigen Fraktionen und Lyophilisation aus Dioxan. FAB-MS: (M+H)⁺=650, t_{Ret}(I)=26,3 min, R_{f} (H)=0,64.

### Beispiel 54: H-(L)-Val-[Phe^{NN}Leu]←((L)-Val)-H·3 HCl:

Analog Beispiel 5 erhält man aus 396 mg (0,61 mMol) Boc-(L)-Val--[Phe^{NN}Leu]←((L)-Val(-Boc aus Beispiel 53 und 10 ml 4N Chlorwasserstoff in Dioxan nach Lyophilisation der Reaktionslösung die Titelverbindung. FAB-MS: (M+H)⁺=450, t_{Ret}(II)=24,1 min, R_{f} (K)=0,25.

### Beispiel 55: N-Thiomorpholinocarbonyl-(L)-Val-[Phe^{NN}Leu←(N-thiomorpholinocarbonyl-(L)-Val):

Analog Beispiel 6 erhält man ausgehend von 100 mg (0,16 mMol) H-(L)-Val-[Phe^{NN}Leu]←(L)-Val-H·3 HCl, 78,5 mg (0,47 mMol) (4-Thiomorpholinyl-carbonyl)chlorid aus Beispiel 6a und 0,172 ml Triethylamin in DMF nach chromatographischer Reinigung auf Kieselgel mit Methylenchlorid/Methanol (95:5), Fällung der produkthaltigen Fraktionen aus Methylenchlorid/Hexan und Lyophilisation aus Dioxan die Titelverbindung als amorphen Festkörper. FAB-MS: (M+H)⁺=708, t_{Ret}(I)=21,4 min, R_{f} (E)=0,45.

### Beispiel 56: 2(R,S)-Tetrahydrofuryl-methoxycarbonyl-(L)-Val-[Cha^{NN}Leu]←(N-2(R,S)-tetrahydrofuryl-methoxycarbonyl-(L)-Val):

Analog Beispiel 37 erhält man aus 80 mg (0,22 mMol) H-[Cha^{NN}Leu]-H·3 HCl, 160 mg (0,65 mMol) N-(2(R,S)-Tetrahydrofuryl-methoxycarbonyl)-(L)-valin, 289 mg (0,65 mMol) BOP, 88 mg (0,65 mMol) HOBt und 4,35 ml 0,3M NMM in DMF die Titelverbindung nach chromatographischer Reinigung auf Kieselgel mit Essigsäureethylester und Lyophilisation der produkthaltigen Fraktionen aus Dioxan. FAB-MS: (M+H)⁺=712, t_{Ret}(I)=22,4 min, R_{f} (E)=0,21.

### a) H-[Cha^{NN}Leu]-H·3 HCl:

Analog Beispiel 5 erhält man aus 150 mg (0,33 mMol) Boc-[Cha^{NN}Leu]-Boc und 10 ml 4N Chlorwasserstoff in Dioxan nach Lyophilisation der Reaktionslösung 100 mg (83 %) der Titelverbindung. R_{f} (K)=0,26.

### b) Boc-[Cha^{NN}Leu]-Boc

Eine Lösung von 200 mg (0,24 mMol) Boc-[Phe^{NN}Leu]-Boc (Beispiel 12) in 15 ml Methanol wird in Gegenwart von 10 mg Nishimura-Katalysator (Rh(III)- und Pt(IV)-Oxid Monohydrat, Degussa) bei 1 atm Wasserstoffdruck während 4 h hydriert. Man filtriert vom Katalysator ab, dampft das Lösungsmittel vollständig ein und erhält die Titelver-bindung nach Kristallisation aus Methylenchlorid/Hexan. t_{Ret}(I)=26,7 min, R_{f} (V)=0,21.

### c) N-(2(R,S)-Tetrahydrofuryl-methoxycarbonyl)-(L)-valin:

Analog Beispiel 50a erhält man aus 7 g (60 mMol) (L)-Valin und 9,8 g (60 mMol) 2(R,S)-Tetrahydrofurylmethyl-chloroformiat (Heterocycles 27, 1155 (1988)) in 100 ml 2N Natronlauge und 30 ml Dioxan die Titelverbindung als Gemisch von 2 Diastereomeren. t_{Ret}(II)=23,5 und 23,8 min

### Beispiel 57: Z-Val-[Phe^{NN}Leu]←(N-(3-(tetrazol-1-yl)-propionyl)-Val):

Analog Beispiel 37 erhält man aus 100 mg (0,21 mMol) Z-(L)-Val-[Phe^{NN}Leu]-H, 75 mg (0,31 mMol) N-(3-(Tetrazol-1-yl)-propionyl)-(L)-valin aus Beispiel 44a, 137 mg (0,31 mMol) BOP, 42 mg (0,31 mMol) HOBt und 2 ml 0,3M NMM in DMF die Titelverbindung nach Fällung aus Methylenchlorid/Hexan und Lyophilisation aus Dioxan/tert-Butanol (2 Diasteromere im HPLC unterscheidbar). FAB-MS: (M+H)⁺=708, t_{Ret}(I)=21,1 und 21,1 min, R_{f} (D)=0,45.

### a) Z-(L)-Val-[Phe^{NN}Leu]-H:

Eine Lösung von 250 mg (0,43 mMol) Z-(L)-Val-[(Phe^{NN}Leu]-Boc in 5 ml Ameisensäure wird während 7,5 h bei RT gerührt. Nach dieser Zeit ist bei HPLC-Analyse kein Ausgangsmaterial mehr erkennbar (t_{Ret}(I)=27,5 min), und die Reaktionslösung wird eingedampft. Der Rückstand wird in Chloroform gelöst und mit ges. Natriumbicarbonat-lösung gewaschen. Die Chloroform-Phase wird durch Watte filtriert und ergibt die rohe Titelverbindung nach Abdampfen des Lösungsmittels. t_{Ret}(I)=16,7 min, R_{f} (K)=0,21.

### b) Z-(L)-Val-[Phe^{NN}Leu]-Boc:

Analog Beispiel 37 erhält man aus 230 mg (0,653 mMol) H-[Phe^{NN}Leu]-Boc, 247 mg (0,98 mMol) Z-(L)-Valin, 434 mg (0,98 mMol) BOP, 133 mg (0,98 mMol) HOBt und 6,5 ml 0,3M NMM in DMF die Titelverbindung nach Fällung aus Methylenchlorid/Methanol durch Zugabe von DIPE. FAB-MS: (M+H)⁺=585, t_{Ret}(I)=27,5 min, R_{f} (C)=0,71.

### c) H-[Phe^{NN}Leu]-Boc:

Analog Beispiel 17a erhält man ausgehend von 1,27 g (2,84 mMol) N-Trifluoracetyl-[Phe^{NN}Leu]-Boc und 24 ml 1N wässriger Natriumcarbonatlösung in 90 ml Methanol die Titelverbindung, welche aus Methylenchlorid durch Zugabe von DIPE gefällt wird. t_{Ret}(I)=14,9 min, R_{f} (K)=0,38.

### d) N-Trifluoracetyl-[Phe^{NN}Leu]-Boc:

Analog Beispiel 16 erhält man ausgehend von 3 g (11,57 mMol) 2(R)-[1′(S)-(Trifluoracetyl-amino)-2′-phenylethyl]-oxiran aus Beispiel 16d und 2,3 g (12,15 mMol) tert-Butyl-3-isobutyl-carbazat (Herstellung: J. Chem. Soc. Perkin 1,1712 ( 1975)) nach chromatographischer Reinigung auf Kieselgel mit Methylenchlorid/Ether (20:1) die Titelverbindung. t_{Ret}(I)=24,7 min, R_{f} (W)=0,36.

### Beispiel 58: Acetyl-Val-[Phe^{NN}Leu]←N-(2(R,S)-carbamoyl-3-phenyl-propionyl)-Val):

Analog Beispiel 37 erhält man aus 140 mg (0,3 mMol) Acetyl-(L)-Val-[Phe^{NN}Leu]-H·2HCl, 132 mg (0,45 mMol) N-(2(R,S)-Carbamoyl-3-phenyl-propionyl)-(L)-valin (Herstellung: Synth., Struct., Funct., Proc. Am. Pept. Symp., 7^{th}, 85, (1981)), 199 mg (0,45 mMol) BOP, 61 mg (0,45 mMol) HOBt und 3,5 ml 0,3M NMM in DMF die Titelverbindung nach Ausfällen aus Methylenchlorid/DIPE und Lyophilisation aus Dioxan (im HPLC 2 Diastereomere unterscheidbar). FAB-MS: (M+H)⁺=667, t_{Ret}(I)=17,9 und 18,4 min, R_{f} (D)=0,33.

### a) Acetyl-Val-[Phe^{NN}Leu]-H·2 HCl:

Analog Beispiel 2a erhält man ausgehend von 230 mg (0,46 mMol) Acetyl-(L)-Val-[Phe^{NN}Leu]-Boc die Titelverbindung nach Lyophilisation. t_{Ret}(I)= 10,5 min, R_{f} (D)=0,38.

### b) Acetyl-Val-[Phe^{NN}Leu]-Boc:

Analog Beispiel 37 erhält man aus 250 mg (0,71 mMol) H-[Phe^{NN}Leu]-Boc aus Beispiel 57c, 170 mg (1,07 mMol) N-Acetyl-(L)-valin, 471 mg (1,07 mMol) BOP, 144 mg (1,07 mMol) HOBt und 7,1 ml 0,3M NMM in DMF die Titelverbindung nach Ausfällen aus Methylenchlorid durch Zugabe von DIPE und Lyophilisation aus Dioxan. FAB-MS: (M+H)⁺=493, t_{Ret}(I)=20,5 min, R_{f} (D)=0,59.

### Beipiel 59: N-Morpholinocarbonyl-(L)-Val-[Phe^{NN}Leu]←(N-(3-(tetrazol-1-yl)-propionyl)-Val):

Analog Beispiel 37 erhält man aus 100 mg (0,19 mMol) N-Morpholinocarbonyl-(L)-Val-[Phe^{NN}Leu]-H·2 HCl, 67 mg (0,38 mMol) N-(3-(Tetrazol- 1-yl)-propionyl)-(L)-valin aus Beispiel 44a, 124 mg (0,28 mMol) BOP, 3 8 mg (0,28 mMol) HOBt und 2,1 ml 0,3M NMM in DMF die Titelverbindung nach Ausfällen aus Methylenchlorid durch Zugabe von DIPE und Lyophilisation aus Dioxan (im HPLC 2 Diastereomere unterscheidbar). FAB-MS: (M+H)⁺=687, t_{Ret}(I)=15,2 und 15,4 min, R_{f} (D)=0,25.

### a) N-Morpholinocarbonyl-(L)-Val-[Phe^{NN}Leu]-H·2 HCl:

Analog Beispiel 2a erhält man ausgehend von 279 mg (0,49 mMol) N-Morpholino-carbonyl-(L)-Val-[Phe^{NN}Leu]-Boc nach Lyophilisation die Titelverbindung. FAB-MS: (M+H)⁺=464, t_{Ret}(II)=30,3 min, R_{f} (D)=0,46.

### b) N-Morpholinocarbonyl-(L)-Val-[Phe^{NN}Leu]-Boc:

Analog Beispiel 37 erhält man aus 250 mg (0,71 mMol) H-[Phe^{NN}Leu]-Boc (aus Beispiel 57c), 265 mg (1,07 mMol) N-Morpholinocarbonyl-(L)-valin aus Beipiel 7a, 471 mg (1,07 mMol) BOP, 144 mg (1,07 mMol) HOBt und 7,1 ml 0,3M NMM in DMF die Titelverbindung nach Ausfällen aus Methylenchlorid/Hexan und Lyophilisation aus Dioxan. FAB-MS: (M+H)⁺=564, t_{Ret}(I)=21,5 min, R_{f} (K)=0,69.

### Beispiel 60: N-Trifluoracetyl-[Phe^{NN}Leu]←(N-(2(R,S)-carbamoyl-3-phenylpropionyl)-(L)-Val):

Analog Beispiel 37 erhält man aus 136 mg (0,32 mMol) N-Trifluoracetyl-[Phe^{NN}Leu]-H·2HCl, 142 mg (0,49 mMol) N-(2(R,S)-Carbamoyl-3-phenyl-propionyl)-(L)-valin (Herstellung: Synth., Struct., Funct., Proc. Am. Pept. Symp., 7^{th}, 85, (1981)), 215 mg (0,49 mMol) BOP, 66 mg (0,49 mMol) HOBt und 3,5 ml 0,3M NMM in DMF die Titelverbindung nach chromatographischer Reinigung auf Kieselgel mit Chloroform/Methanol (15:1), Fällung der produkthaltigen Fraktionen aus Methylenchlorid/DIPE und Lyophilisation aus Dioxan/tert-Butanol (im HPLC 2 Diasteromere unterscheidbar). FAB-MS: (M+H)⁺=622, t_{Ret}(I)=21,6 und 22,0 min, R_{f} (K)-=0,26.

### a) N-Trifluoracetyl-[Phe^{NN}Leu]-H·2 HCl:

Analog Beispiel 2a erhält man ausgehend von 300 mg (0,67 mMol) N-Trifluoracetyl-[Phe^{NN}Leu]-Boc aus Beispiel 57d nach Lyophilisation die Titelverbindung. R_{f} (W)<0,1.

### Beispiel 61: Z-(L)-Val-[Phe^{NN}Nle]←(N-(2(R,S)-(N-(2-morpholinoethyl)carbamoyl)-3-methyl)-butyryl):

Analog Beispiel 37 erhält man aus 100 mg (0,17 mMol) Z-(L)-Val-(Phe^{NN}Nle]-H·2HCl, 69 mg (0,27 mMol) 2(R,S)-(N-(2-Morpholinoethyl)-carbamoyl)-3-methyl-buttersäure (Isopropylmalonsäure-N-(2-morpholinoethyl)monoamid), 119 mg (0,27 mMol) BOP, 36 mg (0,27 mMol) HOBt und 2,1 ml 0,3M NMM in DMF die Titelverbindung nach Fällung aus Methylenchlorid/DIPE und Lyophilisation aus Dioxan (im HPLC 2 Diastereomere unterscheidbar). FAB-MS: (M+H)⁺=725, t_{Ret}(I)=17,2 und 17,6 min, R_{f} (D)=0,56.

### a) Z-(L)-Val-[Phe^{NN}Nle]-H·2 HCl:

Analog Beispiel 2a erhält man ausgehend von 310 mg (0,53 mMol) Z-(L)-Val-[Phe^{NN}Nle]-Boc nach Lyophilisation die Titelverbindung. t_{Ret}(I)= 16,4 min, R_{f} (U)=0,25.

### b) Z-(L)-Val-[Phe^{NN}Nle]-Boc:

Analog Beispiel 37 erhält man aus 250 mg (0,71 mMol) H-[Phe^{NN}Nle]-Boc, 268 mg (1,07 mMol) Z-(L)-Valin, 472 mg (1,07 mMol) BOP, 144 mg (1,07 mMol) HOBt und 7,1 ml 0,3M NMM in DMF die Titelverbindung nach chromatographischer Reinigung auf Kieselgel mit Methylenchlorid/Methanol (40:1) und Fällung der produkthaltigen Fraktionen aus Methylenchlorid/DIPE. t_{Ret}(I)=25,6 min, R_{f} (X)=0,17.

### c) H-[Phe^{NN}Nle]-Boc:

Analog Beispiel 17a erhält man ausgehend von 830 mg (1,85 mMol) N-Trifluoracetyl-[Phe^{NN}Nle]-Boc die Titelverbindung nach Fällung aus Methylenchlorid/DIPE. t_{Ret}(I)= 15,4 min, R_{f} (K)=0,54.

### d) N-Trifluoracetyl-[Phe^{NN}Nle]-Boc:

Analog Beispiel 16 erhält man ausgehend von 1 g 3,86 (mMol) 2(R)-[ 1′-(S)-(Trifluoracetyl-amino)-2′-phenylethyl] -oxiran aus Beispiel 16d und 720 mg (3,86 mMol) tert-Butyl-3-butyl-carbazat nach chromatographischer Reinigung auf Kieselgel mit Methylenchlorid/Ether (20:1) die Titelverbindung. t_{Ret}(I)=25,3 min, R_{f} (Q)=0,43.

### e) tert-Butyl-3-butyl-carbazat:

Analog Beispiel 4b erhält man aus 18,0 g (136,2 mMol) tert-Butylcarbazat und 12,3 ml (136,2 (mMol) n-Butanal das entsprechende tert-Butoxycarbonyl-hydrazon (25g, 99 %) als Rohprodukt, welches wie in Beispiel 4a beschrieben in Gegenwart von 10 g 5% Platin auf Kohle und 4 atm Wasserstoffdruck hydriert wird. Chromatographische Reinigung des Rohproduktes auf Kieselgel mit Hexan/Essigsäureethylester (1:1) ergibt die Titelverbindung. R_{f} (N)=0,44, ¹H-NMR (200 MHz, CD₃OD). 0,92 (t, J=7Hz, 3H) 1,43 (s, 9H) 1,30 bis 1,50 (m, 4H) 2,75 (t, J=7Hz, 2H).

### f) 2(R,S)-(N-(2-Morpholinoethyl)-carbamoyl)-3-methyl-buttersäure:

Analog Beipiel 9b erhält man aus 7 g (43,7 mMol) racemischem Isopropylmalonsäure-monomethylester (Chem. Ber. 119, 1196 (1986)), 6,3 ml (48,1 mMol) Aminoethyl-morpholin, 6,6 ml (43,7 mMol) Cyanphosphonsäure-diethylester und 12,8 ml (91,8 mMol) Triethylamin in DMF 2(R,S)-(N-(2-Morpholinoethyl)-carbamoyl)-3-methyl-buttersäure-methylester (Isopropylmalonsäure-N-mor-pholinoethylamid-methylester). Dieser wird während 5h in einem Gemisch von 28 ml 2N Natronlauge und 28 ml Dioxan bei RT gerührt, mit 2N Salzsäure angesäuert und vollständig eingedampft. Der Rückstand wird mit Ethanol digeriert, abfiltriert und ergibt nach Eindampfen des Filtrats die Titelverbindung. ¹H-NMR (200 MHz, CD₃OD): 0,95 und 1,00 (2d, J=7H, 6H) 2,25 (m, 4H) 2,70 (m, 6H) 2,75 (d, J=8Hz, 1H) 3,45 (m, 2H) 3,75 (m, 4H).

### Beispiel 62: Z-(L)-Val-[Phe^{NN}Nle]←(N-(3-(tetrazol-1-yl)-propionyl)-Val):

Analog Beispiel 37 erhält man aus 100 mg (0,18 mMol) Z-(L)-Val-[Phe^{NN}Nle]-H·2 HCl (aus Beispiel 61 a), 65 mg (0,27 mMol) N-(3-(Tetrazol-1-yl)-propionyl)-(L)-valin aus Beispiel 44a, 119 mg (0,27 mMol) BOP, 36 mg (0,27 mMol) HOBt und 2,1 ml 0,3M N-Methylmorpholin in DMF die Titelverbindung nach Fällung aus Methylenchlorid/DIPE und Lyophilisation aus Dioxan/tert-Butanol (2 Diastereomere im HPLC unterscheidbar). FAB-MS: (M+H)⁺=708, t_{Ret}(I)=20,3 und 20,6 min, R_{f} (D)=0,43.

### Beispiel 63: Z-(L)-Val-[Phe^{NN}Nle]←(N-(2(R,S)-(N-(2-pyridylmethyl)carbamoyl)-3-methyl)-butyryl) (Dibenzolsulfonat):

Analog Beispiel 37 erhält man aus 95 mg (0,17 mMol) Z-(L)-Val-[Phe^{NN}Nle]-H-2 HCl aus Beispiel 61a, 60 mg (0,26 mMol) (R,S)-Isopropylmalonsäure-N-(2-picolyl)-monoamid, 113 mg (0,26 mMol) BOP, 35 mg (0,26 mMol) HOBt und 2,0 ml 0,3M NMM in DMF die Titelverbindung nach chromatographischer Reinigung auf Kieselgel mit Methylenchlorid/Methanol (15:1) als freies Amin. Dieses wird in Methylenchlorid gelöst, mit 2 Aequivalenten Benzolsulfonsäure versetzt und durch Zugabe von DIPE ausgefällt. Lyophilisation aus tert-Butanol ergibt das Dibenzolsulfonat-Salz (2 Diastereomere im HPLC unterscheidbar). FAB-MS: (M+H)⁺=703, t_{Ret}(I)=17,7 und 18,0 min, R_{f} (D)=0,54.

### a) Isopropylmalonsäure-N-(2-picolyl)monoamid:

Eine Lösung von 15 g (93,6 mMol) Isopropylmalonsäure-monomethylester (Herstellung: Chem. Ber. 119, 1196 (1986)) in 150 ml THF wird mit 10,6 ml (103 mMol) N-Methylmorpholin und anschliessend tropfenweise mit 13,5 ml (103 mmol) Isobutylchloroformiat versetzt. Nach 30 min fügt man 15,3 ml (150 mMol) 2-Picolylamin zu und rührt die entstandene Suspension während 2h. Das Reaktionsgemisch wird verdünnt mit 1N Natronlauge und Wasser und mit Methylenchlorid gewaschen, die organische Phase durch Watte filtriert und eingedampft. Kristallisation des Rückstandes liefert Isopropylmalonsäure-N-(2-picolylamid)-methylester, welcher wie in Beispiel 61 f beschrieben in 2N Natronlauge und Dioxan zur Titelverbindung hydrolisiert wird. t_{Ret}(II)= 16,0 min.

### Beispiel 64: Z-(L)-Val-[Phe^{NN}(p-F)Phe]←(N-(3-(tetrazol-1-yl)-propionyl)-(L)-Val)(Benzolsulfonat):

Analog Beispiel 37 erhält man aus 100 mg (0,16 mMol) Z-(L)-Val-[Phe^{NN}(p-F)Phe]-H aus Beispiel 22a, 59 mg (0,25 mMol) N-(3-(Tetrazol-1-yl)-propionyl)-(L)-valin aus Beispiel 44a, 109 mg (0,25 mMol) BOP, 33 mg (0,25 mMol) HOBt und 1,19 ml 0,3M N-Methylmorpholin in DMF die Titelverbindung nach Ausfällen aus Methylenchlorid/DIPE als freies Amin. Dieses wird in Methylenchlorid/Methanol gelöst, mit 1 Aequivalent Benzolsulfonsäure versetzt und durch Zugabe von Hexan gefällt. Lyophilisation aus tert-Butanol ergibt die Titelverbindung als Benzolsulfonat-Salz. FAB-MS: (M+H)⁺=760, t_{Ret}(I)=21,6 min, R_{f} (B)=0,49.

### Beispiel 65: Methylsulfonyl-[Phe^{NN}Phe]←(N-phenylacetyl-(L)-Val):

132 mg (0,28 mMol) Methylsulfonyl-[Phe^{NN}Phe]-H·2 HCl wird analog Beispiel 7 mit 197 mg (0,84 mMol) N-Phenylacetyl-(L)-valin, (Herstellung: Mem. Tokyo Univ. Agric. 20, 51 ( 1978)), 317 mg (0,84 mMol) HBTU und 0,23 ml ( 1,67 mMol) Triethylamin in DMF umgesetzt und ergibt nach Fällung aus Methanol durch Zugabe von Ether die Titelverbindung. FAB-MS: (M+H)⁺=581, t_{Ret}(I)=20,2 min, R_{f} (B)=0,64.

### a) Methylsulfonyl-[Phe^{NN}Phe]-H·2 HCl:

Analog Beispiel 2a erhält man ausgehend von 130 mg (0,28 mMol) Methylsulfonyl-[Phe^{NN}Phe]-Boc die Titelverbindung nach Lyophilisation. FAB-MS: (M+H)⁺=364, t_{Ret}(II)=28,5 min, R_{f} (K)=0,56.

### b) Methylsulfonyl-[Phe^{NN}Phe]-Boc:

Analog Beispiel 16a erhält man ausgehend von 1,1 g (4,56 mMol) 2(R)-[1′(S)-(Methylsulfonylamino)-2′-phenylethyl]oxiran und 1,11 g (5,02 mMol) tert-Butyl-3-ben-zyl-carbazat (Herstellung: J. Chem Soc. Perkin 1,1712 (1975)) die Titelverbindung als Diastereomerengemisch im Verhältnis 4:1. Durch Kristallisation aus Methylen-chlorid/Hexan wird das Verhältnis zugunsten des 2S-Diastereomeren auf 10:1 verbessert. FAB-MS: (M+H)⁺=464, t_{Ret}(I)=21,3 min, R_{f} (N)=0,26.

### c) 2(R)-[1′(S)-(Methylsulfonylamino)-2′-phenylethyl]oxiran:

Eine Lösung von 1 g (6,8 mMol)11-Phenyl-3-buten-2(S)-amin aus Beispiel 16b in 10 ml Methylenchlorid wird bei 0° mit 2,36 g (13,6 mMol) Methansulfonsäureanhydrid und 1,88 ml (13,6 mMol) Triethylamin versetzt und während 1h gerührt. Das Reaktionsgemisch wird mit Wasser und ges. Natriumbicarbonatlösung gewaschen, die organische Phase wird durch Watte filtriert und eingedampft und ergibt 2(S)-Methyl-sulfonylamino-1-phenyl-3-buten. 1 g (4,4 mMol) dieses Rohprodukts wird in 30 ml Methylenchlorid gelöst, bei RT mit 3,05 g (17,7 mMol) 4-Chlorperbenzoesäure versetzt und während 18h gerührt. Die Reaktionslösung wird 5x mit 10% wässriger Natrium-sulfitlösung gewaschen, durch Watte filtriert und vollständig eingedampft. Das Rohprodukt enthält gemäss ¹H-NMR die beiden (2R) und (2S)-Epimeren im Verhältnis 4:1. ¹H-HMR (200 MHz, CD₃OD): 2,30 und 2,52, (2 s, zusammen 3H) 2,6 bis 3,2 (m, 5H) 3,55 (m, 1H) 7,32 (m, 5H).

### Beispiel 66: Methoxycarbonyl-(L)-Val-[Phe^{NN}Leu]←(N-methoxycarbonyl-(L)-Val):

Analog Beispiel 37 erhält man aus 200 mg (0,55 mMol) H-[Phe^{NN}Leu]-H·3 HCl (aus Beispiel 13a), 291 mg (1,66 mMol) N-Methoxycarbonyl-(L)-valin (Herstellung: Chem. Lett. 705, (1980)), 735 mg (1,66 mMol) BOP, 225 mg (1,66 mMol) HOBt und 11 ml 0,3M NMM in DMF die Titelverbindung nach Fällung aus Methylenchlorid/DIPE und Lyophilisation aus Dioxan. FAB-MS: (M+H)+=566, t_{Ret}(I)=18,6 min, R_{f} (U)=0,33.

### Beispiel 67: Methoxycarbonyl-(L)-Val-[Phe^{NN}(p-F)Phe]←(N-methoxycarbonyl-(L)-Val):

Analog Beispiel 37 erhält man aus 200 mg (0,48 mMol) H-[Phe^{NN}(p-F)Phe]-H·3 HCl (aus Beispiel 42b), 255 mg (1,45 mMol) N-Methoxycarbonyl-(L)-valin (Herstellung: Chem. Lett. 705, (1980)), 643 mg (1,45 mMol) BOP, 196 mg (1,45 mMol) HOBt und 9,7 ml 0,3M NMM in DMF die Titelverbindung nach Fällung aus Methylenchlorid/DIPE und Lyophilisation aus Dioxan. FAB-MS: (M+H)⁺=618, t_{Ret}(I)=19,5 min, R_{f} (U)=0,22.

### Beispiel 68: Methoxycarbonyl-(L)-Val-[Phe^{NN}(p-CN)Phe]←(N-methoxycarbonyl-(L)-Val):

Analog Beispiel 37 erhält man aus 200 mg (0,48 mMol) H-[Phe^{NN}(p-CN)Phe]-H·3 HCl (aus Beispiel 47a), 250 mg ( 1,43 mMol) N-Methoxycarbonyl-(L)-valin (Herstellung: Chem. Lett. 705, (1980)), 631 mg (1,43 mMol) BOP, 193 mg (1,43 mMol) HOBt und 9,5 ml 0,3M NMM in DMF die Titelverbindung nach chromatographischer Reinigung auf Kieselgel mit Methylenchlorid/Methanol (15:1) und Lyophilisation der produkthaltigen Fraktionen aus Dioxan. FAB-MS: (M+H)⁺=625, t_{Ret}(I)=18 min, R_{f} (U)=0,31.

### Beispiel 69: Z-(L)-Val-[(p-F)Phe^{NN}(p-F)Phe]←(N-(2(R,S)-(N-(2-morpholinoethyl)-carbamoyl)-3-methyl)-butyryl):

Analog Beispiel 18) werden 23,0 mg (0,089 mMol) 2(R,S)-(N-(2-Morpholinoethyl)-carbamoyl)-3-methyl-buttersäure (Beispiel 61 f) und 45 mg (0,081 mMol) Z-(L)-Val-[(p-F)Phe^{NN}(p-F)Phe]-H (Beispiel 32) mit 33,8 mg (0,089 mMol) HBTU in 0,76 ml NMM/CH₃CN 0,25 M zur Titelverbindung umgesetzt und mit DMF/DIPE umgefällt: DC R_{f}(P)=0,42; FAB-MS (M+H)⁺=795.

### Beispiel 70: Z-(L)-Val-[(p-F)Phe^{NN}(p-F)Phe]←(N-(2(R,S)-(N-(2-pyridylmethyl)-carbamoyl)-3-methyl)-butyryl):

Analog Beispiel 18) werden 21,0 mg (0,089 mMol) rac. Isopropylmalonsäure-N-(2-picolyl)amid (Beispiel 63 a) und 45 mg (0,081 mMol) Z-(L)-Val-[(p-F)Phe^{NN}(p-F)Phe]-H (Beispiel 32) mit 33,8 mg (0,089 mMol) HBTU in 0,76 ml NMM/CH₃CN 0,25M zur Titelverbindung umgesetzt und mit DMF/DIPE umgefällt: DC R_{f}(P)=0,52; FAB-MS (M+H)⁺=773.

### Beispiel 71:

Analog zu einem der vorgenannten Verfahren können folgende Verbindungen hergestellt werden:
a) Z-(L)-Val-[(p-F)Phe^{NN}(p-F)Phe]←((L)-Val)←(N-morpholinocarbonyl-Gly);
b) N-Morpholinocarbonyl-(L)-Val-[(p-F)Phe^{NN}(p-F)Phe]←((L)-Val)←(N-morpholinocarbonyl-Gly);
c) N-(Chinolin-2-carbonyl)-(L)-Asn-[Phe^{NN}(p-F)Phe]←((L)-Val)-Z

### Beispiel 72: Gelatine-Lösung:

Eine sterilfiltrierte wässrige Lösung mit 20 % Cyclodextrinen als Lösungsvermittler von einer der in den vorausgehenden Beispielen genannten Verbindungen der Formel I als Wirkstoff wird unter Erwärmen mit einer sterilen Gelatinelösung, welche als Wirkstoff wird unter Erwärmen mit einer sterilen Gelatinelösung, welche als Konservierungsmittel Phenol enthält, unter aseptischen Bedingungen so vermischt, dass 1,0 ml Lösung die folgende Zusammensetzung hat:

| | |
|---|---|
| Wirkstoff | 3 mg |
| Gelatine | 150,0 mg |
| Phenol | 4,7 mg |
| dest. Wasser mit 20 % Cyclodextrinen als Lösungsvermittler | 1,0 ml |

### Beispiel 73: Sterile Trockensubstanz zur Injektion:

Man löst 5 mg einer der in den vorausgehenden Beispielen genannten Verbindungen der Formel I als Wirkstoff in 1 ml einer wässrigen Lösung mit 20 mg Mannit und 20 % Cyclodextrinen als Lösungsvermittler. Die Lösung wird sterilfiltriert und unter aseptischen Bedingungen in eine 2 ml-Ampulle gefüllt, tiefgekühlt und lyophilisiert. Vor dem Gebrauch wird das Lyophilisat in 1 ml destilliertem Wasser oder 1 ml physiologischer Kochsalzlösung gelöst. Die Lösung wird intramuskulär oder intravenös angewendet. Diese Formulierung kann auch in Doppelkammerspritzampullen abgefüllt werden.

### Beispiel 74: Nasenspray:

In einer Mischung von 3,5 ml Myglyol 812® und 0,08 g Benzylalkohol werden 500 mg fein gemahlenes (<5,0 µm) Pulver einer der in den vorausgehenden Beispielen genannten Verbindungen der Formel I als Wirkstoff suspendiert. Diese Suspension wird in einen Behälter mit Dosierventil eingefüllt. Es werden 5,0 g Freon 12® unter Druck durch das Ventil in einen Behälter abgefüllt. Durch Schütteln wird das "Freon" in der Myglyol-Benzylalkoholmischung gelöst. Dieser Spraybehälter enthält ca. 100 Einzeldosen, die einzeln appliziert werden können.

### Beispiel 75: Lacktabletten

Für die Herstellung von 10 000 Tabletten enthaltend je 100 mg Wirkstoff werden folgende Bestandteile verarbeitet:

| | |
|---|---|
| Wirkstoff | 1000 g |
| Maisstärke | 680 g |
| Kolloidale Kieselsäure | 200 g |
| Magnesiumstearat | 20 g |
| Stearinsäure | 50 g |
| Natriumcarboxymethylstärke | 250 g |
| Wasser | quantum satis |

Ein Gemisch von einer der in den vorausgehenden Beispielen genannten Verbindungen der Formel I als Wirkstoff, 50 g Maisstärke und der kolloidalen Kieselsäure wird mit Stär-kekleister aus 250 g Maisstärke und 2,2 kg entmineralisiertem Wasser zu einer feuchten Masse verarbeitet. Diese wird durch ein Sieb von 3 mm Maschenweite getrieben und bei 45 ° während 30 min im Wirbelschichttrockner getrocknet. Das getrocknete Granulat wird durch ein Sieb von 1 mm Maschenweite gedrückt, mit einer vorher gesiebten Mischung (1 mm Sieb) von 330 g Maisstärke, des Magnesiumstearats, der Stearinsäure und der Natriumcarboxymethylstärke gemischt und zu schwach gewölbten Tabletten verpresst.

## Patentansprüche (Patentansprüche für folgende(n) Vertragsstaat(en): AT, BE, CH, DE, DK, FR, GB, IT, LI, LU, MC, NL, PT, SE)

1. Verbindungen der Formel 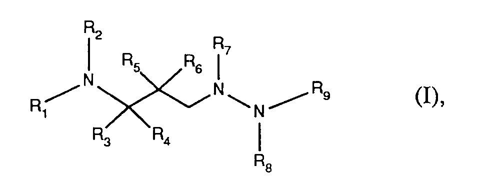 worin R₁ und R₉ unabhängig voneinander Wasserstoff, Acyl, unsubstituiertes oder substituiertes Alkyl, Alkenyl oder Alkinyl; Heterocyclyl; Sulfo, durch unsubstituiertes oder substituiertes Alkyl, Aryl, Alkoxy, das unsubstituiert oder substituiert ist, oder durch Aryloxy substituiertes Sulfonyl; Sulfamoyl, das am Stickstoff unsubstituiert oder substituiert ist; oder durch ein oder zwei unabhängig voneinander aus unsubstituiertem oder substituiertem Alkyl, aus unsubstituiertem oder substituiertem Cycloalkyl, aus Aryl, aus Hydroxy, aus unsubstituiertem oder substituiertem Alkoxy, aus Cycloalkoxy und aus Aryloxy ausgewählte Reste substituiertes Phosphoryl bedeuten, mit der Massgabe, dass höchstens einer der Reste R₁ und R₉ Wasserstoff bedeutet; und R₂ und R₈ jeweils unabhängig voneinander Wasserstoff oder einen der oben für R₁ und R₉ genannten Reste bedeuten; oder die Substituentenpaare R₁ und R₂ oder R₈ und R₉ jeweils unabhängig voneinander gemeinsam mit dem Stickstoffatom, an das sie gebunden sind, einen heterocyclischen Ring, bestehend aus dem bindenden Stickstoff zusammen mit einem aus Ethylen, Trimethylen, Tetramethylen und Pentamethylen ausgewählten Rest, worin ein Kohlenstoffatom durch Stickstoff, Sauerstoff, Schwefel oder mit Sauerstoff ein-oder zweifach substituierten Schwefel ersetzt sein kann und welcher ungesättigt sein kann, oder Napthalin- 1,8-dicarbonylimido bilden können;
R₃ und R₄ unabhängig voneinander Wasserstoff, unsubstituiertes oder substituiertes Alkyl oder Cycloalkyl; Aryl; Heterocyclyl; oder unsubstituiertes oder substituiertes Alkenyl bedeuten; oder R₃ und R₄ gemeinsam unsubstituiertes oder substituiertes Alkylen, Alkyliden oder benzokondensiertes Alkylen bedeuten;
R₅ Hydroxy bedeutet; R₆ Wasserstoff bedeutet;
oder R₅ und R₆ gemeinsam Oxo bedeuten;
und R₇ unsubstituiertes oder substituiertes Alkyl oder Cycloalkyl; Aryl; Heterocyclyl;
oder unsubstituiertes oder substituiertes Alkenyl bedeutet;
sowie die Salze der genannten Verbindungen, sofern salzbildende Gruppen vorliegen; mit
Ausnahme der Verbindung der Formel I, worin R₁, R₂, R₃, R₄, R₆ und R₈ jeweils Wasserstoff bedeuten, R₅ Hydroxy bedeutet, R₇ einen Rest der Formel -[CH₂-(C=O)-NH-(N-CH₃)-CH₂-COOH bedeutet und R₉ tert-Butoxycarbonyl bedeutet.

2. Eine Verbindung der Formel I gemäss Anspruch 1, worin
R₁ und R₉ unabhängig voneinander Wasserstoff; Niederalkanoyl; Arylniederalkanoyl, worin Niederalkanoyl unsubstituiert oder substituiert ist durch Carbamoyl oder durch einen oder zwei aus Niederalkyl, Carboxyniederalkyl, Niederalkoxycarbonyl-niederalkyl, Diniederalkylamino-niederalkyl, Amino-carboxy-niederalkyl, Hydroxyniederalkyl und Diniederalkoxyniederalkyl ausgewählte Reste am Stickstoff substituiertes Carbamoyl; Heterocyclylniederalkanoyl; Halogenniederalkanoyl, welches bis zu 3 Halogenatome enthält; (N-Hetemcyclylniederalkyl-carbamoyl)-niederalkanoyl; Niederalkoxycarbonyl; Arylniederalkoxycarbonyl; Heterocyclylniederalkoxycarbonyl; Niederalkylsulfonyl; N-Heterocyclylniederalkyl-N-niederalkyl-carbamoyl; oder einen Acylrest einer Aminosäure, deren Aminofunktion frei oder durch einen der übrigen bisher für R₁ und R₉ genannten Reste acyliert ist; bedeuten, mit der Massgabe, dass höchstens einer der Reste R₁ und R₉ Wasserstoff bedeutet;
R₂, R₄, R₆ und R₈ Wasserstoff bedeuten,
R₃ Niederalkyl, Cycloalkylniederalkyl oder Arylniederalkyl bedeutet,
R₅ Hydroxy bedeutet, und
R₇ unsubstituiertes oder substituiertes Niederalkyl bedeutet,
oder ein Salz davon, sofern wenigstens eine salzbildende Gruppe vorliegt, wobei in den oben genannten Definitionen der Ausdruck "Nieder" oder "nieder" bedeutet, dass der jeweilige Rest bis zu einschliesslich 7 Kohlenstoffatome hat.

3. Eine Verbindung der Formel I gemäss Anspruch 1, worin
R₁ und R₉ unabhängig voneinander Wasserstoff; Niederalkanoyl; Arylniederalkanoyl, worin Aryl Phenyl, Indenyl, Indanyl, Naphthyl, Anthryl, Phenanthryl, Acenaphthyl oder Fluorenyl bedeutet und unsubstituiert oder durch Niederalkyl, Halogenniederalkyl, Phenyl, 1- oder 2-Naphthyl, Hydroxy, Niederalkoxy, Carbamoylniederalkoxy, N-Niederalkylcarbamoylniederalkoxy, N,N-Diniederalkylcarbamoylniederalkoxy, Amino, Mono- oder Diniederalkylamino, Niederalkanoylamino, Halogen, Carboxy, Niederalkoxycarbonyl, Phenyl-, Naphthyl- oder Fluorenylniederalkoxycarbonyl, Niederalkanoyl, Sulfo, Niederalkylsulfonyl, Phosphono, Hydroxyniederalkoxy-phosphoryl, Diniederalkoxyphosphoryl, Carbamoyl, Mono- oder Diniederalkylcarbamoyl, Sulfamoyl, Mono- oder Diniederalkylaminosulfonyl, Nitro und/oder Cyano ein- oder bis zu dreifach substituiert sein kann, wobei Phenyl bis zu dreimal vorliegen kann, und worin Niederalkanoyl unsubstituiert oder substituiert durch Carbamoyl oder durch mit einem oder zwei aus Niederalkyl, Carboxyniederalkyl, Niederalkoxycarbonyl-niederalkyl, Diniederalkylamino-niederalkyl, Amino-carboxy-niederalkyl, Hydroxyniederalkyl und Diniederalkoxyniederalkyl ausgewählte Reste am Stickstoff substituiertes Carbamoyl ist; Heterocyclylniederalkanoyl, worin Niederalkanoyl unsubstituiert ist und worin Heterocyclyl aus Thienyl, Furyl, Pyranyl, Pyrrolyl, Imidazolyl, Pyrazolyl, Oxazolyl, Isoxazolyl, Thiazolyl, Furazanyl, Pyridyl, Pyrazinyl, Pyrimidinyl, Pyridazinyl, Azepinyl, Indolyl, Benzimidazolyl, 1H-Indazolyl, Chinolyl, Isochinolyl, Chinoxalinyl, Chinazolinyl, Cinnolyl, Purinyl, Pteridinyl, Naphthyridinyl, 4H-Chinolizinyl, 3,1-Benzfuranyl, 4,1-Benzoxazinyl, 4,1-Benzthiazinyl, Cyclohexa[b]pyrrolyl, Cyclohepta[b]pyrrolyl, Cyclohexa[d]pyrazolyl, Cyclohexa[b]pyridyl, Cyclohexa[b]pyrazinyl, Cyclohexa[b]pyrimidinyl, Cyclohexa[b]- 1,4-oxazinyl, Cyclohexa[b]-1,4-thiazinyl, Pyrrolidinyl, Pyrrolinyl, Imidazolidyl, 2-Imidazolinyl, 2,3-Dihydropyridyl, Piperidyl, Piperazinyl, 2,3,5,6-Tetrahydropyrazinyl, Morpholinyl, Thiomorpholinyl, S,S-Dioxo-thiomorpholinyl, Indolinyl, Isoindolinyl, 4,5,6,7-Tetrahydroindolyl, 1,2,3,4-Tetrahydrochinolyl, 1,2,3,4-Tetrahydroisochinolyl, Chromanyl, Thiochromanyl, 1,2,3,4-Tetrahydro-3,1-benzdiazinyl, 3,4-Dihydro-3H-4,1-benzoxazinyl und 3,4-Dihydro-3H-4,1-benzthiazinyl ausgewählt ist, wobei die genannten Heterocyclyl-Reste unsubstituiert oder durch Niederalkyl, Phenyl, 1-oder 2-Naphthyl, Phenylniederalkyl, Hydroxyniederalkyl, Niederalkoxyniederalkyl, Phenoxy- oder Naphthoxyniederalkyl, Phenylniederalkoxy- oder Naphthylniederalkoxy-niederalkyl, Niederalkanoyloxy-niederalkyl, Phenyl- oder Napthyl-niederalkanoyloxy-niederalkyl, Niederalkoxycarbonyloxy-niederalkyl, Phenyl-, Napthyl- oder Fluorenylniederalkoxycarbonyloxy-niederalkyl, Aminoniederalkyl, Carboxyniederalkyl, Hydroxy, Niederalkoxy, Phenyl- oder Naphthylniederalkoxy, Amino, Niederalkylamino, Diniederalkylamino, Carboxy, Niederalkoxycarbonyl, Phenyl- oder Napthyl-niederalkoxycarbonyl, Halogen, Niederalkanoyl, Niederalkylsulfonyl, Phosphono, Hydroxy-niederalkoxy-phosphoryl oder Dialkoxy-phosphoryl, Carbamoyl, Mono- oder Diniederalkylcarbamoyl, Hydroxy- oder Carboxyniederalkylcarbamoyl, Sulfamoyl, durch Nitro, Oxo und/oder durch Cyano substituiert sind; eterocyclylniederalkanoyl wie Halogenniederalkanoyl, welches bis zu drei Halogenatome enthält; (N-Heterocyclylniederalkylcarbamoyl)-niederalkanoyl, worin Heterocyclyl aus Pyrrolyl, Furyl, Thienyl, Imidazolyl, Pyrazolyl, Oxazolyl, Thiazolyl, Pyridyl, Pyrazinyl, Pyrimidinyl, Indolyl, Chinolyl, Isochinolyl, Chinoxalinyl, β-Carbolinyl und einem benzanellierten, cyclopenta-, cyclohexa- oder cyclohepta-anellierten Derivat dieser Reste, die auch ganz oder teilweise gesättigt sein können, aus Morpholin und aus Thiomorpholin ausgewählt ist; Niederalkoxycarbonyl; Arylniederalkoxycarbonyl, worin Aryl Phenyl, Biphenylyl, 1- oder 2-Naphthyl, Fluorenyl oder durch Niederalkyl, Hydroxy, Niederalkoxy, Halogen und/oder Nitro mono- oder mehrfach substituiertes Phenyl ist; Heterocyclylniederalkoxycarbonyl, worin Heterocyclyl aus Pyrrolyl, Furyl, Thienyl, Imidazolyl, Pyrazolyl, Oxazolyl, Thiazolyl, Pyridyl, Pyrazinyl, Pyrimidinyl, Indolyl, Chinolyl, Isochinolyl, Chinoxalinyl, β-Carbolinyl und einem benzanellierten, cyclopenta-, cyclohexa- oder cyclohepta-anellierten Derivat dieser Reste, die auch ganz oder teilweise gesättigt vorliegen können, Niederalkylsulfonyl; N-Heterocyclylniederalkyl-N-niederalkyl-carbamoyl, worin Heterocyclyl ausgewählt ist aus Thienyl, Furyl, Pyranyl, Pyrrolyl, Imidazolyl, Pyrazolyl, Oxazolyl, Isoxazolyl, Thiazolyl, Furazanyl, Pyridyl, Pyrazinyl, Pyrimidinyl, Pyridazinyl, Azepinyl, Indolyl, Benzimidazolyl, 1H-Indazolyl, Chinolyl, Isochinolyl, Chinoxalinyl, Chinazolinyl, Cinnolyl, Purinyl, Pteridinyl, Naphthyridinyl, 4H-Chinolizinyl, 3,1-Benzfuranyl, Benz[e]indolyl, 4,1-Benzoxazinyl, 4,1-Benzthiazinyl, Carbazolyl, β-Carbolinyl, Phenazinyl, Phenanthridyl, Acridyl, Phenoxazinyl, Phenothiazinyl, 1-Azaacenaphtenyl, Cyclohexa[b]pyrrolyl, Cyclohepta[b]pyrrolyl, Cyclohexa[d]pyrazolyl, Cyclohexa[b]pyridyl, Cyclohexa[b]pyrazinyl, Cyclohexa[b]pyrimidinyl, Cyclohexa[b]-1,4-oxazinyl, Cyclohexa[b]-1,4-thiazinyl, Pyrrolidinyl, Pyrrolinyl, Imidazolidinyl, 2-Imidazolinyl, 2,3-Dihydropyridyl, Piperidyl, Piperazinyl, 2,3,5,6-Tetrahydropyrazinyl, Morpholinyl, Thiomorpholinyl, S,S-Dioxothiomorpholinyl, Indolinyl, Isoindolinyl, 4,5,6,7-Tetrahydroindolyl, 1,2,3,4-Tetrahydrochinolyl, 1,2,3,4-Tetrahydroisochinolyl, Chromanyl, Thiochromanyl, 1,2,3,4-Tetrahydro-3,1-benzdiazinyl, 3,4-Dihydro-3H-4,1-benzoxazinyl, 3,4-Dihydro-3H-4,1-benzthiazinyl, 2,3,4,5-Tetrahydro-1H-5,1-benzazepinyl und 5,6-Dihydrophenanthridinyl, wobei die genannten Reste unsubstituiert oder durch Niederalkyl, Phenyl, 1- oder 2-Naphthyl, Phenylniederalkyl, Hydroxyniederalkyl, Niederalkoxyniederalkyl, Phenoxy- oder Naphthoxyniederalkyl, Phenylniederalkoxy-oder Naphthylniederalkoxy-niederalkyl, Niederalkanoyloxy-niederalkyl, Phenyl-oder Napthyl-niederalkanoyloxy-niederalkyl, Niederalkoxycarbonyloxy-niederalkyl, Phenyl-, Napthyl- oder Fluorenylniederalkoxycarbonyloxy-niederalkyl, Aminoniederalkyl, Carboxyniederalkyl, Hydroxy, Niederalkoxy, Phenyl- oder Naphthylniederalkoxy, Amino, Niederalkylamino, Diniederalkylamino, Carboxy, Niederalkoxycarbonyl, Phenyl-oder Napthylniederalkoxycarbonyl, Halogen, Niederalkanoyl, Niederalkylsulfonyl, Phosphono, Hydroxy-niederalkoxyphosphoryl oder Dialkoxy-phosphoryl, Carbamoyl, Mono-oder Diniederalkylcarbamoyl, Hydroxy- oder Carboxyniederalkylcarbamoyl, Sulfamoyl, durch Nitro, Oxo und/oder durch Cyano substituiert sind; oder einen Acylrest einer Aminosäure, deren Aminofunktion frei oder durch einen der übrigen bisher für R₁ und R₉ genannten Reste acyliert ist, bedeuten, wobei die Aminosäuren aus Glycin, Alanin, Valin, Norvalin, Leucin, Isoleucin, Norleucin, Serin, Homoserin, Threonin, Methionin, Cystein, Prolin, trans-3- und trans-4-Hydroxyprolin, Phenylalanin, Tyrosin, 4-Aminophenylalanin, 4-Chlorphenylalanin, 4-Carboxyphenylalanin, β-Phenylserin, Phenylglycin, α-Naphthylalanin, Cyclohexylalanin, Cyclohexylglycin, Tryptophan, Asparaginsäure, Asparagin, Aminomalonsäure, Aminomalonsäure-monoamid, Glutaminsäure, Glutamin, Histidin, Arginin, Lysin, δ-Hydroxylysin, Omithin, 3-Aminopropansäure, α,γ-Diaminobuttersäure und α,β-Diaminopropionsäure ausgewählt sind, worin (ausser Glycin) jeder der genannten Aminosäurereste in der D-, L- oder (D,L)-Form vorliegt; mit der Massgabe, dass höchstens einer der Reste R₁ und R₉ Wasserstoff bedeutet,
R₂, R₄, R₆ und R₈ Wasserstoff bedeuten,
R₃ Niederalkyl; Cycloalkylniederalkyl, worin Cycloalkyl 3 bis 7 Kohlenstoffatome hat und unsubstituiert oder durch Niederalkyl, Halogenniederalkyl, Hydroxy, Niederalkoxy, Carbamoylniederalkoxy, N-Niederalkylcarbamoylniederalkoxy, N,N-Diniederalkylcarbamoylniederalkoxy, Amino, Mono- oder Diniederalkylamino, Halogen, Carboxy, Niederalkoxycarbonyl, Phenyl-, Naphthyl- oder Fluorenylniederalkoxycarbonyl, Niederalkanoyl, Niederalkylsulfonyl, Phosphono, Hydroxy-niederalkoxy-phosphoryl oder Diniederalkoxyphosphoryl, Carbamoyl, Mono- oder Diniederalkylaminocarbamoyl, Sulfamoyl, Mono- oder Diniederalkylsulfamoyl, Nitro und/oder Cyano ein-oder bis zu dreifach substituiert ist; oder Arylniederalkyl bedeutet, worin Aryl Phenyl, Indenyl, Indanyl, Naphthyl, Anthryl, Phenanthryl, Acenaphthyl oder Fluorenyl bedeutet und unsubstituiert oder durch Niederalkyl, Halogenniederalkyl, Phenyl, 1-oder 2-Naphthyl, Hydroxy, Niederalkoxy, Carbamoylniederalkoxy, N-Niederaikylcarbamoylniederalkoxy, N,N-Diniederalkylcarbamoylniederalkoxv, Amino, Mono- oder Diniederalkylamino, Niederalkanoylamino, Halogen, Carboxy, Niederalkoxycarbonyl, Benzyl-, Naphthyl- oder Fluorenylniederalkoxycarbonyl, Niederalkanoyl, Sulfo, Niederalkylsulfonyl, Phosphono, Hydroxy-niederalkoxy-phosphoryl, Diniederalkoxyphosphoryl, Carbamoyl, Mono-oder Diniederalkylcarbamoyl, Sulfamoyl, Mono- oder Diniederalkylsulfamoyl, Nitro und/oder Cyano ein- oder bis zu dreifach substituiert sein kann, wobei Phenyl bis zu dreimal vorliegen kann,
R₅ Hydroxy bedeutet, und
R₇ unsubstituiertes Niederalkyl; oder Cycloalkylniederalkyl, wie zuletzt für Cycloalkylniederalkyl R₃ beschrieben; oder Arylniederalkyl, wie zuletzt für Arylniederalkyl R₃ beschrieben; bedeutet
oder ein Salz davon, sofern wenigstens eine salzbildende Gruppe vorliegt, wobei in den oben genannten Definitionen der Ausdruck "Nieder" oder "nieder" bedeutet, dass der jeweilige Rest bis zu einschliesslich 7 Kohlenstoffatome hat.

4. Verbindungen der Formel I gemäss Anspruch 1, worin
R₁ und R₉ unabhängig voneinander
- Wasserstoff,
- Niederalkoxycarbonyl,
- 2-Halogenniederalkoxycarbonyl,
- Aryloxycarbonyl, worin Aryl 6 bis 14 Kohlenstoffatome hat,
- Arylniederalkoxycarbonyl, worin Aryl 6 bis 14 Kohlenstoffatome hat,
- Heterocyclylniederalkoxycarbonyl, worin Heterocyclyl aus Pyrrolyl, Furyl, Thienyl, Imidazolyl, Pyrazolyl, Oxazolyl, Thiazolyl, Pyridyl, Pyrazinyl, Pyrimidinyl, Indolyl, Chinolyl, Isochinolyl, Chinoxalinyl, β-Carbolinyl und einem benzanellierten, cyclopenta-, cyclohexa- oder cyclohepta-anellierten Derivat dieser Reste, die auch ganz oder teilweise gesättigt vorliegen können, ausgewählt ist,
- 2-Triniederalkylsilylniederalkoxycarbonyl,
- 2-Triarylsilylniederalkoxycarbonyl, worin Aryl Phenyl oder 1- oder 2-Naphthyl ist,
- das über die Carboxygruppe gebundene Radikal einer einer Aminosäure ausgewählt aus Glycin, Alanin, Valin, Norvalin, Leucin, Isoleucin, Norleucin, Serin, Homoserin, Threonin, Methionin, Cystein, Prolin, trans-3- und trans-4-Hydroxyprolin, Phenylalanin, Tyrosin, 4-Aminophenylalanin, 4-Chlorphenylalanin, 4-Carboxyphenylalanin, β-Phenylserin, Phenylglycin, α-Naphthylalanin, Cyclohexylalanin, Cyclohexylglycin, Tryptophan, Indolin-2-carbonsäure, 1,2,3,4-Tetrahydroisochinolin-3-carbonsäure, Asparaginsäure, Asparagin, Aminomalonsäure, Aminomalonsäure-monoamid, Glutaminsäure, Glutamin, Histidin, Arginin, Lysin, δ-Hydroxylysin, Ornithin, α,γ-Diaminobuttersäure und α,β-Diaminopropionsäure, wobei jede dieser Aminosäuren in der D-, L- oder (D,L)-Form vorliegen kann, und
die α-Aminogruppe unsubstituiert oder ein- oder zweifach N-alkyliert durch Niederalkyl, durch Aminoniederalkyl, durch Phenyl- oder Naphthylaminoniederalkyl, oder durch Niederalkyl am Stickstoff substituiertes Piperazinylcarbonylniederalkyl, oder N-acyliert durch Niederalkanoyl, durch Arylniederalkanoyl, worin Aryl ausgewählt ist aus Phenyl, Indenyl, Indanyl, Naphthyl, Anthryl, Phenanthryl, Acenaphthyl und Fluorenyl, und unsubstituiert oder durch Niederalkyl, Halogenniederalkyl, Phenyl, 1- oder 2-Naphthyl, Hydroxy, Niederalkoxy, Carbamoylniederalkoxy, N-Niederalkylcarbamoylniederalkoxy oder N,N-Diniederalkylcarbamoylniederalkoxy, Amino, Mono- oder Diniederalkylamino, Niederalkanoylamino, Halogen, Carboxy, Niederalkoxycarbonyl, Phenyl-, Naphthyl- oder Fluorenylniederalkoxycarbonyl, Niederalkanoyl, Sulfo, Niederalkylsulfonyl, Phosphono, Hydroxy-niederalkoxy-phosphoryl oder Diniederalkoxyphosphoryl, Carbamoyl, Mono-oder Diniederalkylcarbamoyl, Sulfamoyl, Mono- oder Diniederalkylaminosulfonyl, Nitro und/oder Cyano ein- oder bis zu dreifach substituiert sein kann; wobei Phenyl bis zu dreimal vorliegen kann, und Niederalkanoyl unsubstituiert oder substituiert durch Niederalkyl, Heterocyclyl ausgewählt aus Pyrrolyl, Furyl, Thienyl, Imidazolyl, Pyrazolyl, Oxazolyl, Thiazolyl, Pyridyl, Pyrazinyl, Pyrimidinyl, Indolyl, Chinolyl, Isochinolyl, Chinoxalinyl, β-Carbolinyl und einem benz-, cyclopenta-, cyclohexa-oder cyclohepta-anellierten Derivat dieser Reste, die auch ganz oder teilweise gesättigt vorliegen können, Hydroxy, Niederalkoxy, Niederalkanoyloxy, Acetoacetoxy, Amino-oder Benzyloxycarbonylamino-niederalkanoyloxy, Arylniederalkanoyloxy, worin Aryl 6 - 10 Kohlenstoffatome hat, Niederalkoxycarbonyloxy, Mono- oder Diniederalkyl-aminocarbonyloxy, Aryloxycarbonyloxy, worin Aryl 6 - 10 Kohlenstoffatome hat, Arylniederalkoxycarbonyloxy mit Aryl von 6 - 14 Kohlenstoffatomen, Sulfonyloxy, Niederalkylsulfonyloxy, Phenylsulfonyloxy, 2- oder 4-Toluolsulfonyloxy oder 1 - oder 2- Naphthylsulfonyloxy, Carboxy, verestertes Carboxy ausgewählt aus Niederalkoxycarbonyl, Aryloxycarbonyl, worin Aryl 6 - 10 Kohlenstoffatome hat, Arylniederalkoxycarbonyl, worin Aryl 6 - 12 Kohlenstoffatome hat, Niederalkanoyl, Niederalkylsulfonyl, Hydroxy-niederalkoxyphosphoryl und Diniederalkoxyphosphoryl, Carbamoyl, durch einen oder zwei aus Niederalkyl, Carboxyniederalkyl, Niederalkoxycarbonyl-niederalkyl, Diniederalkylaminoniederalkyl, Aminocarboxyniederalkyl, Hydroxyniederalkyl und aus Diniederalkoxyniederalkyl ausgewählte Reste oder einen aus Ethylen, Trimethylen, Tetramethylen und Pentamethylen ausgewählten Rest, worin ein Kohlenstoffatom durch Stickstoff, Sauerstoff, Schwefel oder mit Sauerstoff ein- oder zweifach substituierten Schwefel ersetzt sein kann und das ungesättigt sein kann, substituiertes Carbamoyl, ferner Sulfamoyl, Phosphono, Benzfuranyl, Oxo und/oder Cyano sein kann und unverzweigt oder verzweigt ist; durch Heterocyclylniederalkanoyl ausgewählt aus Thienyl-, Furyl-, Pyranyl-, Pyrrolyl-, Imidazolyl-, Pyrazolyl-, Oxazolyl-, Isoxazolyl-, Thiazolyl-, Furazanyl-, Pyridyl-, Pyrazinyl-, Pyrimidinyl-, Pyridazinyl-, Azepinyl-, Indolyl-, Benzimidazolyl-, 1H-Indazolyl-, Chinolyl-, Isochinolyl-, Chinoxalinyl-, Chinazolinyl-, Cinnolyl-, Purinyl-, Pteridinyl-, Naphthyridinyl-, 4H-Chinolizinyl-, 3,1Benzfuranyl, Benz[e]indolyl-, 4,1-Benzoxazinyl-, 4,1-Benzthiazinyl-, Carbazolyl-, β-Carbolinyl-, Phenazinyl-, Phenanthridyl-, Acridyl-, Phenoxazinyl-, Phenothiazinyl-, 1-Azaacenaphtenyl-, Cyclohexa[b]pyrrolyl-, Cyclohepta[b]pyrrolyl-, Cyclohexa[d]pyrazolyl-, Cyclohexa[b]pyridyl-, Cyclohexa[b]pyrazinyl-, Cyclohexa[b]pyrimidinyl-, Cyclohexa[b]-1,4-oxazinyl-, Cyclohexa[b]-1,4-thiazinyl-, Pyrrolidinyl, Pyrrolinyl-, Imidazolidinyl-, 2-Imidazolinyl-, 2,3-Dihydropyridyl-, Piperidyl-, Piperazinyl-, 2,3,5,6-Tetrahydropyrazinyl-, Morpholiny-, Thiomorpholinyl-, S,S-Dioxo-thiomorpholinyl, Indolinyl-, Isoindolinyl-, 4,5,6,7-Tetrahydroindolyl-, 1,2,3,4-Tetrahydrochinolyl-, 1,2,3,4-Tetrahydroisochinolyl-, Chroman-, Thiochroman-, 1,2,3,4-Tetrahydro-3,1-benzdiazinyl-, 3,4-Dihydro-3H-4,1-benzoxazinyl-, 3,4-Dihydro-3H-4,1benzthiazinyl-, 2,3,4,5-Tetrahydro-1H-5,1-benzazepinyl- und 5,6-Dihydrophenanthridinyl-niederalkanoyl, wobei die genannten Heterocyclyl-Reste unsubstituiert oder durch Niederalkyl, Phenyl, 1- oder 2-Naphthyl, Phenylniederalkyl, Hydroxyniederalkyl, Niederalkoxyniederalkyl, Phenoxy- oder Naphthoxyniederalkyl, Phenylniederalkoxy- oder Naphthylniederalkoxy-niederalkyl, Niederalkanoyloxy-niederalkyl, Phenyl- oder Napthyl-niederalkanoyloxy-niederalkyl, Niederalkoxycarbonyloxy-niederalkyl, Phenyl-, Napthyl- oder Fluorenylniederalkoxycarbonyloxy-niederalkyl, Aminoniederalkyl, Carboxyniederalkyl, Hydroxy, Niederalkoxy, Phenyl- oder Naphthylniederalkoxy, Amino, Niederalkylamino, Diniederalkylamino, Carboxy, Niederalkoxycarbonyl, Phenyl- oder Napthyl-niederalkoxycarbonyl, Halogen, Niederalkanoyl, Niederalkylsulfonyl, Phosphono, Hydroxy-niederalkoxy-phosphoryl, Dialkoxy-phosphoryl, Carbamoyl, Mono- oder Di-niederalkylcarbamoyl, Hydroxy- oder Carboxyniederalkylcarbamoyl, Sulfamoyl, durch Nitro, Oxo und/oder durch Cyano substituiert sind; durch Heterocyclylniederalkenoyl, worin Heterocyclyl ausgewählt ist aus Pyrrolyl, Furyl, Thienyl, Imidazolyl, Pyrazolyl, Oxazolyl, Thiazolyl, Pyridyl, Pyrazinyl, Pyrimidinyl, Indolyl, Chinolyl, Isochinolyl, Chinoxalinyl, β-Carbolinyl und einem benzanellierten, cyclopenta-, cyclohexa- oder cyclohepta-anellierten Derivat dieser Reste, die auch ganz oder teilweise gesättigt sein können, durch Niederalkoxycarbonyl, durch Arylniederalkoxycarbonyl, worin Aryl 6 - 14 Kohlenstoffatome hat; durch Heterocyclylniederalkoxycarbonyl, worin Heterocyclyl ausgewählt ist aus Pyrrolyl, Furyl, Thienyl, Imidazolyl, Pyrazolyl, Oxazolyl, Thiazolyl, Pyridyl, Pyrazinyl, Pyrimidinyl, Indolyl, Chinolyl, Isochinolyl, Chinoxalinyl, β-Carbolinyl und einem benzanellierten, cyclopenta-, cyclohexa- oder cyclohepta-anellierten Derivat dieser Reste, die auch ganz oder teilweise gesättigt und unsubstituiert oder substituiert durch Niederalkyl sein können; durch Carboxyniederalkanoyl, durch Niederalkoxycarbonylniederalkanoyl, durch Hydroxyniederalkoxy-niederalkanoyl, durch Aminoniederalkanoyl oder durch Benzyloxycarbonylaminoniederalkanoyl, worin die Aminogruppe nicht in α- oder β-Stellung gebunden ist, durch Carbamoyl, durch Phenylniederalkylaminocarbonyl, durch N-Diniederalkylaminoniederalkyl-N-niederalkylaminocarbonyl, durch N-Dihydroxyniederalkyl-N-niederalkylaminocarbonyl, durch 2- oder 3-Pyridylniederalkylaminocarbonyl, durch N-2-Pyridylniederalkyl-N-niederalkylaminocarbonyl, durch Sulfonyl, durch Niederalkylsulfonyl, durch Arylsulfonyl, worin Aryl 6 - 10 Kohlenstoffatome hat und unsubstituiert oder durch Niederalkyl oder Niederalkoxy substituiert ist; durch Heterocyclylsulfonyl, worin Heterocyclyl ausgewählt ist aus Pyrrolyl, Furyl, Thienyl, Imidazolyl, Pyrazolyl, Oxazolyl, Thiazolyl, Pyridyl, Pyrazinyl, Pyrimidinyl, Indolyl, Chinolyl, Isochinolyl, Chinoxalinyl, β-Carbolinyl und einem benzaneliierten, cyclopenta-, cyclohexa- oder cyclohepta-anellierten Derivat dieser Reste, die auch ganz oder teilweise gesättigt sein können; durch Sulfamoyl, oder durch mit Heterocyclylniederalkyl, worin Heterocyclyl wie zuletzt definiert ist, und/oder mit Niederalkyl substituiertes Sulfamoyl vorliegen kann,
eine Carboxygruppe der Seitenkette frei oder in veresterter Form als Niederalkylestergruppe, als Arylestergruppe oder als Arylniederalkylestergruppe, wobei Aryl Phenyl, 4-Nitrophenyl, Naphthyl oder Biphenylyl bedeutet, oder in amidierter Form als Carbamoyl-, als Niederalkylcarbamoyl-, als Diniederalkylaminocarbamoyl-, als Mono- oder Di-(hydroxyniederalkyl)carbamoyl- oder als Mono- oder Di-(carboxyniederalkyl)carbamoylgruppe vorliegt,
eine Aminogruppe der Seitenkette frei oder in alkylierter Form als Mono-oder Diniederalkylamino oder in acylierter Form als Niederalkanoylamino, als Aminoniederalkanoylamino, als Arylniederalkanoylamino, worin Aryl 6 - 14 Kohlenstoffatome hat und unsubstituiert oder durch Niederalkyl, Hydroxy, Niederalkoxy, Carboxy, Carbamoyl oder Sulfamoyl substituiert ist, als Niederalkoxycarbonylamino-, als Arylmethoxycarbonylaminogruppe, worin Aryl 6 - 14 Kohlenstoffatome hat, als Piperidyl-1-carbonyl, Morpholinocarbonyl, Thiomorpholinocarbonyl oder als S,S-Dioxothiomorpholinocarbonyl vorliegt und/oder
eine Hydroxygruppe der Seitenkette frei oder in veretherter oder veresterter Form als Niederalkoxy-, als Arylniederalkoxy-, als Niederalkanoyloxy-, oder als Niederalkyloxycarbonyloxygruppe vorliegt,
- Niederalkylsulfonyl,
- 2- oder 3-Pyrrolyl-, 2-Thienyl-, 2-Furyl-, 1-Pyrazolyl-, 2-, 3- oder 4-Pyridyl-, 2-, 3- oder 5-Indolyl-, (1-Methyl-, 2-Methyl-, 5-Methoxy-, 5-Benzyloxy-, 5-Chlor- oder 4,5-Dimethyl-indol-2-yl)-, (1-Benzylindol-2-yl oder -3-yl)-, 4,5,6,7-Tetrahydroindol-2-yl-, (2-, 3- oder 4-Chinolyl oder 4-Hydroxychinol-2-yl)-, (1-, 3- oder 4-Isochinolyl oder 1-Oxo1,2-dihydroisochinol-3-yl)-, 3-Pyrrolidinyl-, (3- oder 4-Hydroxypyrrolidin-2-yl)-, 5-Oxopyrrolidin-2-yl-, (2- oder 3-Morpholinyl)-, (2- oder 3-Thiomorpholinyl)-, (S,S-Dioxothiomorpholin-2- oder -3-yl)-, (2- oder 3-Indolinyl)-, (1,2,3,4-Tetrahydrochinol-2-, -3- oder -4-yl)- oder (1,2,3,4-Tetrahydroisochinol-1-, -2- oder -3-yl)-methylsulfonyl
- unsubstituiertes oder durch Niederalkyl ein- oder zweifach substituiertes Phenyl-oder 1- oder 2-Napthyl-sulfonyl,
- Niederalkoxysulfonyl, oder
- Benzyloxysulfonyl oder 1- oder 2-Naphthyloxysulfonyl
bedeuten, so dass höchstens einer der Reste R₁ oder R₉ Wasserstoff bedeuten kann, und
R₂ und R₈ unabhängig voneinander Wasserstoff oder die gleichen Reste wie R₁ und R₉ bedeuten,
oder die Substituentenpaare R₁ und R₉ oder R₂ und R₈ jeweils unabhängig voneinander gemeinsam mit dem bindenden Stickstoff und einem aus Ethylen, Trimethylen, Tetramethylen und Pentamethylen ausgewählten Rest, worin ein Kohlenstoffatom durch Stickstoff, Sauerstoff, Schwefel oder mit Sauerstoff ein- oder zweifach substituierten Schwefel ersetzt sein kann und das ungesättigt sein kann, einen heterocyclischen Ring bilden, R₃
- Cycloalkylniederalkyl, worin Cycloalkyl 3 - 7 Kohlenstoffatome hat und unsubstituiert oder durch Niederalkyl, Halogenniederalkyl, Hydroxy, Niederalkoxy, Carbamoylniederalkoxy, N-Niederalkylcarbamoylniederalkoxy oder N,N-Diniederalkylcarbamoylniederalkoxy, Amino, Mono- oder Diniederalkylamino, Halogen, Carboxy, Niederalkoxycarbonyl, Phenyl-, Naphthyl- oder Fluorenylniederalkoxycarbonyl, Niederalkanoyl, Niederalkylsulfonyl, Phosphono, Hydroxy-niederalkoxy-phosphoryl oder Diniederalkoxyphosphoryl, Carbamoyl, Mono- oder Diniederalkylaminocarbamoyl, Sulfamoyl, Mono-oder Diniederalkylsulfamoyl, Nitro und/oder Cyano ein- oder bis zu dreifach substituiert ist und an Niederalkyl gebunden ist, oder
- Arylniederalkyl mit Aryl ausgewählt aus Phenyl, Indenyl, Indanyl, Naphthyl, Anthryl, Phenanthryl, Acenaphthyl und Fluorenyl, das unsubstituiert oder durch Niederalkyl, Halogenniederalkyl, Phenyl, 1- oder 2-Naphthyl, Hydroxy, Niederalkoxy, Carbamoylniederalkoxy, N-Niederalkylcarbamoylniederalkoxy oder N,N-Diniederalkylcarbamoylniederalkoxy, Amino, Mono- oder Diniederalkylamino, Niederalkanoylamino, Halogen, Carboxy, Niederalkoxycarbonyl, Phenyl-, Naphthyl- oder Fluorenylniederalkoxycarbonyl, Niederalkanoyl, Sulfo, Niederalkylsulfonyl, Phosphono, Hydroxy-niederalkoxy-phosphoryl oder Diniederalkoxyphosphoryl, Carbamoyl, Mono- oder Diniederalkylcarbamoyl, Sulfamoyl, Mono- oder Diniederalkylsulfamoyl, Nitro und/oder Cyano ein-oder bis zu dreifach substituiert sein kann, wobei Phenyl bis zu dreimal vorliegen kann, und mit Niederalkyl, welches unsubstituiert oder substituiert ist durch Niederalkyl, Heterocyclyl ausgewählt aus Pyrrolyl, Furyl, Thienyl, Imidazolyl, Pyrazolyl, Oxazolyl, Thiazolyl, Pyridyl, Pyrazinyl, Pyrimidinyl, Indolyl, Chinolyl, Isochinolyl, Chinoxalinyl, β-Carbolinyl oder einem benzanellierten, cyclopenta-, cyclohexa- oder cyclohepta-anellierten Derivat dieser Reste, die auch ganz oder teilweise gesättigt vorliegen können, Hydroxy, Niederalkoxy, Niederalkanoyloxy, Acetoacetoxy, Amino- oder Benzyloxycarbonylamino-niederalkanoyloxy, Arylniederalkanoyloxy, worin Aryl 6 - 10 Kohlenstoffatome hat, Niederalkoxycarbonyloxy, Mono- oder Diniederalkyl-aminocarbonyloxy, Aryloxycarbonyloxy, worin Aryl 6 - 10 Kohlenstoffatome hat, Arylniederalkoxycarbonyloxy mit Aryl von 6 - 12 Kohlenstoffatomen, Sulfonyloxy, Niederalkylsulfonyloxy, Phenylsulfonyloxy, 2- oder 4-Toluolsulfonyloxy oder 1- oder 2- Naphthylsulfonyloxy, Amino, Mono-oder Diniederalkylamino, N-Niederalkoxy-N-niederalkylamino, Mono- oder Di-(Phenyl- oder Naphthylniederalkyl)amino, Niederalkanoylamino, Carboxy, verestertes Carboxy ausgewählt aus Niederalkoxycarbonyl, Aryloxycarbonyl, worin Aryl 6 - 10 Kohlenstoffatome hat, Arylniederalkoxycarbonyl, worin Aryl 6 - 12 Kohlenstoffatome hat, Niederalkanoyl, Niederalkylsulfonyl, Hydroxy-niederalkoxyphosphoryl und Diniederalkoxyphosphoryl, Carbamoyl, durch einen oder zwei aus Niederalkyl, aus Carboxyniederalkyl oder Niederalkoxycarbonyl-niederalkyl, aus Diniederalkylaminoniederalkyl, aus Aminocarboxyniederalkyl, aus Hydroxyniederalkyl, und aus Diniederalkoxyniederalkyl ausgewählte Reste oder einen aus Ethylen, Trimethylen, Tetramethylen und Pentamethylen ausgewählten Rest, worin ein Kohlenstoffatom durch Stickstoff, Sauerstoff, Schwefel oder mit Sauerstoff ein-oder zweifach substituierten Schwefel ersetzt sein kann und das ungesättigt sein kann, substituiertes Carbamoyl, ferner Sulfamoyl, Phosphono, Benzofuranyl, Oxo und/oder Cyano und unverzweigt oder verzweigt ist,
R₄ Wasserstoff bedeutet,
R₅ Hydroxy bedeutet und
R₆ Wasserstoff bedeutet,
oder R₅ und R₆ zusammen Oxo bedeuten, und
R₇
- Niederalkyl,
- Cycloalkylniederalkyl, worin Cycloalkyl 3 - 7 Kohlenstoffatome hat und unsubstituiert oder durch Niederalkyl, Halogenniederalkyl, Hydroxy, Niederalkoxy, Carbamoylniederalkoxy, N-Niederalkylcarbamoylniederalkoxy oder N,N-Diniederalkylcarbamoylniederalkoxy, Amino, Mono- oder Diniederalkylamino, Halogen, Carboxy, Niederalkoxycarbonyl, Phenyl-, Naphthyl- oder Fluorenylniederalkoxycarbonyl, Niederalkanoyl, Niederalkylsulfonyl, Phosphono, Hydroxy-niederalkoxy-phosphoryl oder Diniederalkoxyphosphoryl, Carbamoyl, Mono- oder Diniederalkylaminocarbamoyl, Sulfamoyl, Mono-oder Diniederalkylsulfamoyl, Nitro und/oder Cyano ein- oder bis zu dreifach substituiert ist und an Niederalkyl gebunden ist,
- Bicycloalkylniederalkyl, worin Bicylcoalkyl 5 - 10, Kohlenstoffatome enthält,
- Tricycloalkylniederalkyl, worin Tricycloalkyl 8 - 10 Kohlenstoffatome enthält,
- Arylniederalkyl mit Aryl ausgewählt aus Phenyl, Indenyl, Indanyl, Naphthyl, Anthryl, Phenanthryl, Acenaphthyl und Fluorenyl, das unsubstituiert oder durch Niederalkyl, Halogenniederalkyl, Phenyl, 1- oder 2-Naphthyl, Hydroxy, Niederalkoxy, Carbamoylniederalkoxy, N-Niederalkylcarbamoylniederalkoxy, N,N-Diniederalkylcarbamoylniederalkoxy, Amino, Mono- oder Diniederalkylamino, Niederalkanoylamino, Halogen, Carboxy, Niederalkoxycarbonyl, Benzyl-, Naphthyl- oder Fluorenylniederalkoxycarbonyl, Niederalkanoyl, Sulfo, Niederalkylsulfonyl, Phosphono, Hydroxy-niederalkoxy-phosphoryl, Diniederalkoxyphosphoryl, Carbamoyl, Mono- oder Diniederalkylcarbamoyl, Sulfamoyl, Mono- oder Diniederalkylsulfamoyl, Nitro und/oder Cyano ein-oder bis zu dreifach substituiert sein kann, wobei Phenyl bis zu dreimal vorliegen kann, und mit Niederalkyl, welches unsubstituiert oder substituiert ist durch Niederalkyl, Heterocyclyl ausgewählt aus Pyrrolyl, Furyl, Thienyl, Imidazolyl, Pyrazolyl, Oxazolyl, Thiazolyl, Pyridyl, Pyrazinyl, Pyrimidinyl, Indolyl, Chinolyl, Isochinolyl, Chinoxalinyl, β-Carbolinyl und einem benzanellierten, cyclopenta-, cyclohexa- oder cyclohepta-anellierten Derivat dieser Reste, die auch ganz oder teilweise gesättigt vorliegen können, Hydroxy, Niederalkoxy, Niederalkanoyloxy, Acetoacetoxy, Amino- oder Benzyloxycarbonylamino-niederalkanoyloxy, Arylniederalkanoyloxy, worin Aryl 6 - 10 Kohlenstoffatome hat, Niederalkoxycarbonyloxy, Mono- oder Diniederalkyl-aminocarbonyloxy, Aryloxycarbonyloxy, worin Aryl 6 - 10 Kohlenstoffatome hat, Arylniederalkoxycarbonyloxy mit Aryl von 6 - 12 Kohlenstoffatomen, Sulfonyloxy, Niederalkylsulfonyloxy, Phenylsulfonyloxy, 2-oder 4-Toluolsulfonyloxy, 1- oder 2- Naphthylsulfonyloxy, Amino, Mono- oder Diniederalkylamino, N-Niederalkoxy-N-niederalkylamino, Mono- oder Di-(Phenyl- oder Naphthylniederalkyl)amino, Niederalkanoylamino, Carboxy, verestertes Carboxy ausgewählt aus Niederalkoxycarbonyl, Aryloxycarbonyl, worin Aryl 6 - 10 Kohlenstoffatome hat, Arylniederalkoxycarbonyl, worin Aryl 6 - 12 Kohlenstoffatome hat, Niederalkanoyl, Niederalkylsulfonyl, Hydroxy-niederalkoxyphosphoryl und Diniederalkoxyphosphoryl, Carbamoyl, durch einen oder zwei aus Niederalkyl, aus Carboxyniederalkyl, aus Niederalkoxycarbonyl-niederalkyl, aus Diniederalkylaminoniederalkyl, aus Aminocarboxyniederalkyl, aus Hydroxyniederalkyl und aus Diniederalkoxyniederalkyl ausgewählte Reste oder einen aus Ethylen, Trimethylen, Tetramethylen und Pentamethylen ausgewählten Rest, worin ein Kohlenstoffatom durch Stickstoff, Sauerstoff, Schwefel oder mit Sauerstoff ein- oder zweifach substituierten Schwefel ersetzt sein kann und das ungesättigt sein kann, substituiertes Carbamoyl, ferner Sulfamoyl, Phosphono, Benzfuranyl, Oxo (welches nicht an das Kohlenstoffatom gebunden ist, das mit dem den Rest R₇ bindenden Stickstoffatom verknüpft und/oder Cyano und unverzweigt oder verzweigt ist,
- Heterocyclylniederalkyl, worin Heterocyclyl vorzugsweise aus Pyrrolyl, Furyl, Thienyl, Imidazolyl, Pyrazolyl, Oxazolyl, Thiazolyl, Pyridyl, Pyrazinyl, Pyrimidinyl, Indolyl, Chinolyl, Isochinolyl, Chinoxalinyl, β-Carbolinyl und einem benzanellierten, cyclopenta-, cyclohexa- oder cyclohepta-anellierten Derivat dieser Reste, die auch ganz oder teilweise gesättigt sein können, ausgewählt ist, z.B. 4-Pyrrolidinylmethyl, 1-Imidazolylmethyl, 2-Pyridylmethyl, 3-Pyridylmethyl, Chinolin-2-ylmethyl oder Indol-2-ylmethyl,
- Hydroxyniederalkyl,
- Niederalkoxyniederalkyl,
- Phenoxyniederalkyl oder Nitrophenoxyniederalkyl,
- Naphthoxyniederalkyl,
- Niederalkanoyloxyniederalkyl,
- Acetoacetoxyniederalkyl,
- Arylmercaptoniederalkyl, worin Aryl 6 - 10 Kohlenstoffatome hat,
- Aminoniederalkyl,
- Mono- oder Diniederalkylaminoniederalkyl,
- Phenyl- oder Naphthylaminoniederalkyl,
- Niederalkanoylamino-niederalkyl,
- durch Niederalkyl am Stickstoff substituiertes Piperazinylcarbonylniederalkyl,
- Niederalkoxycarbonylamino-niederalkyl,
- Phenylniederalkoxycarbonylamino-niederalkyl,
- Aminocarbonylamino-niederalkyl,
- N-Phenylniederalkyl-N-niederalkylaminocarbonylamino-niederalkyl,
- Halogenniederalkyl,
- Carboxyniederalkyl,
- Niederalkoxycarbonylniederalkyl,
- 2-Halogenniederalkoxycarbonyl-niederalkyl,
- Phenyl- oder Naphthylniederalkyloxycarbonyl-niederalkyl,
- Heterocyclylniederalkoxycarbonyl-niederalkyl, worin Heterocyclyl ausgewählt ist aus Pyrrolyl, Furyl, Thienyl, Imidazolyl, Pyrazolyl, Oxazolyl, Thiazolyl, Pyridyl, Pyrazinyl, Pyrimidinyl, Indolyl, Chinolyl, Isochinolyl, Chinoxalinyl, β-Carbolinyl und einem benzanellierten, cyclopenta-, cyclohexa- oder cyclohepta-anellierten Derivat dieser Reste, wobei die genannten Reste auch ganz oder teilweise gesättigt vorliegen können,
- Niederalkylsulfonylniederalkyl,
- Arylsulfonylniederalkyl, worin Aryl 6 - 10 Kohlenstoffatome hat,
- Carbamoylniederalkyl,
- Niederalkylcarbamoyiniederalkyl,
- Diniederalkylcarbamoylniederalkyl,
- Hydroxyniederalkylcarbamoyl- oder Di-(hydroxyniederalkyl)carbamoyl-niederalkyl,
- N-Niederalkoxyniederalkoxyniederalkyl-carbamoyl-niederalkyl,
- Carboxyniederalkylcarbamoyl- oder Di-(carboxyniederalkyl)carbamoyl-niederalkyl,
- durch einen aus Ethylen, Trimethylen, Tetramethylen und Pentamethylen ausgewählten Rest, worin ein Kohlenstoffatom durch Stickstoff, Sauerstoff, Schwefel oder mit Sauerstoff ein- oder zweifach substituierten Schwefel ersetzt sein kann, am Stickstoff substituiertes Carbamoylniederalkyl, wobei der so gebildete Rest auch ganz oder teilweise ungesättigt sein kann,
- N-Niederalkyl-N-heterocyclylniederalkylcarbamoylniederalkyl, worin Heterocyclyl aus Pyrrolyl, Furyl, Thienyl, Imidazolyl, Pyrazolyl, Oxazolyl, Thiazolyl, Pyridyl, Pyrazinyl, Pyrimidinyl, Indolyl, Chinolyl, Isochinolyl, Chinoxalinyl, β-Carbolinyl und einem benz-anellierten, cyclopenta-, cyclohexa- oder cyclohepta-anellierten Derivat dieser Reste, die auch ganz oder teilweise gesättigt sein können, ausgewählt ist,
- Sulfamoylniederalkyl,
- N-(Phenyl- oder Naphthylniederalkyl)sulfamoylniederalkyl,
- am Stickstoffatom von einem aus Ethylen, Trimethylen, Tetramethylen und Pentamethylen ausgewählten Rest, worin ein Kohlenstoffatom durch Stickstoff, mit Niederalkyl substituierten Stickstoff, Sauerstoff, Schwefel oder mit Sauerstoff ein-oder zweifach substituierten Schwefel ersetzt sein kann, substituiertes Sulfamoylniederalkyl, wobei der so gebildete Rest auch ganz oder teilweise ungesättigt sein kann,
- Oxoniederalkyl (wobei Oxo nicht an das Kohlenstoffatom gebunden ist, welches mit dem R₇ bindenden Stickstoffatom verknüpft ist),
- Cyanoniederalkyl,
- Hydroxy-carboxy-niederalkyl,
- α-Naphthoxy-carboxy-niederalkyl,
- Hydroxy-niederalkoxycarbonyl-niederalkyl,
- α-Naphthoxy-niederalkoxycarbonylniederalkyl,
- Niederalkylcarbonyl-halogen-niederalkyl,
- α-Naphthoxyethoxycarbonyl-niederalkyl,
- α-Naphthoxy-benzyloxycarbonyl-niederalkyl,
- verestertes Hydroxy-niederalkoxycarbonyl-niederalkyl, wobei die Hydroxygruppe durch Niederalkanoyl, Cycloalkylniederalkanoyl, worin Cycloalkyl 3 - 7 Kohlenstoffatome hat, Bicycloalkylniederalkanoyl, worin Bicylcoalkyl 5 - 10 Kohlenstoffatome enthält, Tricycloalkylniederalkanoyl, worin Tricycloalkyl 8 - 10 Kohlenstoffatome enthält, Arylniederalkanoyl, worin Aryl 6 - 14 Kohlenstoffatome hat und unsubstituiert oder durch Niederalkyl, Hydroxy, Niederalkoxy, Carbamoylniederalkoxy, N-Niederalkylcarbamoylniederalkoxy oder N,N-Diniederalkylcarbamoylniederalkoxy, Amino, Mono- oder Diniederalkylamino, Halogen, Carboxy, Niederalkoxycarbonyl, Phenyl-, Naphthyl- oder Fluorenylniederalkoxycarbonyl, Niederalkanoyl, Niederalkylsulfonyl, Phosphono, Hydroxyniederalkoxy-phosphoryl, Diniederalkoxyphosphoryl, Carbamoyl, Sulfamoyl, Nitro und/oder Cyano ein- oder bis zu dreifach substituiert sein kann, Niederalkoxycarbonyl, 2-Halogenniederalkoxycarbonyl, oder Phenyl- oder Fluorenyl-niederalkoxycarbonyl, verestert ist,
- Dihydroxy-carboxy-niederalkyl,
- Dihydroxy-niederalkoxycarbonyl-niederalkyl,
- durch Niederalkanoyl, Niederalkoxycarbonyl, Phenyl- oder Fluorenyl-niederalkoxycarbonyl,- Niederalkylsulfonyl oder Toluolsulfonyl verestertes Dihydroxy-niederalkoxycarbonyl-niederalkyl,
- α-Naphthoxy-diniederalkylamino-niederalkyl,
- α-Naphthoxy-carbamoyl-niederalkyl,
- α-Naphthoxy-oxo-niederalkyl (wobei Oxo nicht an das Kohlenstoffatom gebunden ist, welches mit dem R₇ bindenden Stickstoffatom verknüpft ist), oder
- α-Naphthoxy-cyanoniederalkyl bedeutet,
sowie die Salze der genannten Verbindungen, sofern salzbildende Gruppen vorliegen, wobei in den oben genannten Definitionen der Ausdruck "Nieder'' oder "nieder" bedeutet, dass der jeweilige Rest bis zu einschliesslich 7 Kohlenstoffatome hat.

5. Verbindungen der Formel I gemäss Anspruch 1, worin R₁ Niederalkoxycarbonyl, Phenylniederalkoxycarbonyl, das über die Carboxygruppe gebundene monovalente Radikal einer aliphatischen Aminosäure ausgewählt aus Valin, Alanin, Leucin und Isoleucin oder das über die Carboxygruppe gebundene Radikal einer am Aminostickstoff durch einen der Reste Phenylniederalkanoyl, Morpholinylniederalkanoyl, Thiomorpholinylniederalkanoyl, S,S-Dioxothiomorpholinylniederalkanoyl, Pyridylniederalkanoyl, Niederalkoxycarbonyl oder Phenylniederalkoxycarbonyl acylierten, wie oben definierten aliphatischen Aminosäure, wobei alle genannten Aminosäuren in der D-, D,L- oder L-Form vorliegen, bedeutet, R₂ Wasserstoff, R₃ Phenylniederalkyl, R₄ Wasserstoff, R₅ Hydroxy, R₆ Wasserstoff, R₇ Niederalkyl, Cyclohexylniederalkyl oder Phenylniederalkyl, R₈ Wasserstoff und R₉ einen der für R₁ genannten Reste bedeuten und die die Reste R₃ und R₅ tragenden asymmetrischen Kohlenstoffatome in der S-Konfiguration vorliegen, sowie die pharmakologisch verwendbaren Salze dieser Verbindungen, wobei in den oben genannten Definitionen der Ausdruck "Nieder" oder ''nieder'' bedeutet, dass derjeweilige Rest bis zu einschliesslich 7 Kohlenstoffatome hat.

6. Verbindungen der Formel I gemäss Anspruch 1, worin R₁ tert-Butoxycarbonyl, Benzyloxycarbonyl, das über die Carboxygruppe gebundene monovalente Radikal der Aminosäure Valin oder das über die Carboxygruppe gebundene Radikal des am Aminostickstoff durch einen der Reste Phenylacetyl, 3-Pyridylacetyl, Morpholinocarbonyl, Thiomorpholinocarbonyl, tert-Butoxycarbonyl oder Benzyloxycarbonyl acylierten Alanins bedeutet, R₂ Wasserstoff, R₃ Benzyl, R₄ Wasserstoff, R₅ Hydroxy, R₆ Wasserstoff, R₇ Isobutyl, Cyclohexylmethyl oder Benzyl, R₈ Wasserstoff und R₉ einen der für R₁ genannten Reste bedeuten und die die Reste R₃ und R₅ tragenden asymmetrischen Kohlenstoffatome in der S-Konfiguration vorliegen, sowie die pharmakologisch verwendbaren Salze dieser Verbindungen.

7. Die Verbindung der Formel I gemäss Anspruch 1, worin R₁ und R₉ jeweils das über die Carboxygruppe gebundene, am Aminostickstoff durch Benzyloxycarbonyl acylierte monovalente Radikal der Aminosäure (L)-Valin bedeuten, R₂ und R₈ Wasserstoff bedeuten, R₃ Benzyl bedeutet, R₄ Wasserstoff bedeutet, R₅ Hydroxy bedeutet, R₆ Wasserstoff bedeutet und R₇ Benzyl bedeutet und die die Reste R₃ und R₅ tragenden asymmetrischen Kohlenstoffatome in der S-Konfiguration vorliegen, sowie die pharmakologisch verwendbaren Salze dieser Verbindung.

8. Die Verbindung der Formel I gemäss Anspruch 1, worin R₁ und R₉ jeweils das über die Carboxygruppe gebundene, am Aminostickstoff durch 4-Thiomorpholinocarbonyl acylierte monovalente Radikal der Aminosäure (L)-Valin bedeuten, R₂ und R₈ Wasserstoff bedeuten, R₃ Benzyl bedeutet, R₄ Wasserstoff bedeutet, R₅ Hydroxy bedeutet, R₆ Wasserstoff bedeutet und R₇ Isobutyl bedeutet und die die Reste R₃ und R₅ tragenden asymmetrischen Kohlenstoffatome in der S-Konfiguration vorliegen, sowie die pharmakologisch verwendbaren Salze dieser Verbindung.

9. Eine Verbindung der Formel I gemäss Anpruch 1, ausgewählt aus den Verbindungen mit den Bezeichnungen
Boc-[Phe^{NN}Phe]-Boc,
Boc-(L)-Val-[Phe^{NN}Phe]←((L)Val-Boc),
Boc-[Phe^{NN}Cha]-Boc,
H-(L)-Val-[Phe^{NN}Phe]←((L)-Val)-H,
N-Thiomorpholinocarbonyl-(L)-Val-[Phe^{NN}Phe]←(N-thiomorpholinocarbonyl-(L)-Val),
N-Morpholinocarbonyl-(L)-Val [Phe^{NN}Phe]←(N-morpholinocarbonyl-(L)-Val),
Phenylacetyl-(L)-Val-[Phe^{NN}Phe]←(N-phenylacetyl-(L)-Val),
N-(3-Pyridylacetyl)-(L)-Val-[Phe^{NN}Phe]←(N-(3-pyridylacetyl)-(L)-Val),
Boc-(L)-Val-[Phe^{NN}Cha]←((L)-Val)-Boc,
Z-(L)-Val-[Phe^{NN}Cha]←((L)-Val)-Z,
Boc-[Phe^{NN}Leu]-Boc
Z-(L)-Val-[Phe^{NN}Leu]←((L)-Val)-Z, H-(L)-Val-[Phe^{NN}Cha]←((L)-Val)-H und
N-(3-Pyridylacetyl)-(L)-Val-[Phe^{NN}Leu]←(N-(3-pyridylacetyl)-(L)-Val), oder ein Salz davon;
worin Boc für tert-Butoxycarbonyl steht, Z für Benzyloxycarbonyl steht, der Rest -[Phe^{NN}Phe]- das zweiwertige Radikal von
3(S)-Amino-4-phenyl-1-(N-benzylhydrazino)-butan-2(S)-ol bedeutet und die Formel 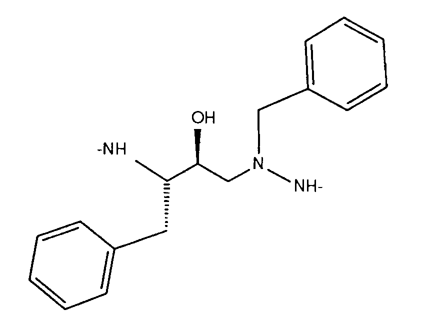 hat, der Rest -[Phe^{NN}Cha] das zweiwertige Radikal von 3(S)-Amino-4-phenyl-1-(N-cyclohexylmethylhydrazino)-butan-2(S)-ol bedeutet und die Formel 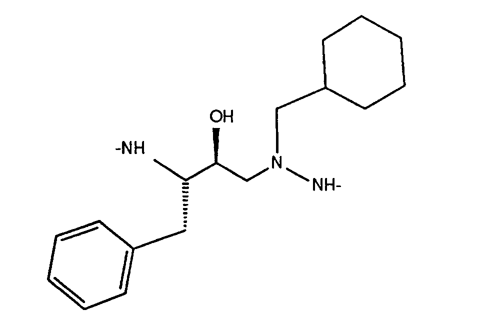 hat, der Rest -[Phe^{NN}Leu]- das zweiwertige Radikal von 3(S)-Amino-4-phenyl-1-(N-iso-butylhydrazino)-butan-2(S)-ol bedeutet und die Formel 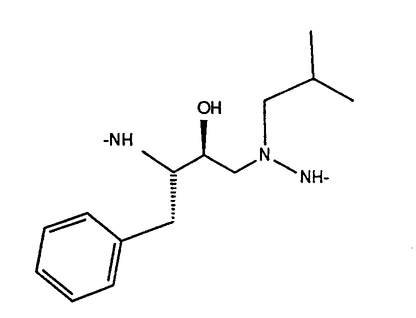 hat, und ein Pfeil "←" die Umkehrung der Bindungsrichtung in Abweichung von der üblichen Peptidnomenklatur, worin links der Amino- und rechts der Carboxyterminus vorliegen, bedeutet.

10. Eine Verbindung der Formel I gemäss Anpruch 1, ausgewählt aus den Verbindungen mit den Bezeichnungen
Z-(L)-Val-[(p-F)Phe^{NN}(p-F)Phe]←(N-(N-(2-pyridylmethyl)-N-methyl-aminocarbonyl)(L)-Val),
Z-(L)-Val-[(p-F)Phe^{NN}(p-F)Phe]←(N-(2(R,S)-carbamoyl-3-phenyl-propionyl)(L)-Val),
Acetyl-(L)-Val-[Phe^{NN}Cha]←(N-acetyl-(L)-Val),
Acetyl-Ile-[Phe^{NN}Cha]←(N-acetyl-Ile),
N-(2-Pyridylmethyl)-N-methyl-aminocarbonyl-(L)-Val-[Phe^{NN}(p-F)-Phe]←(N-(N-(2-pyridylmethyl)-N-methylaminocarbonyl)-(L)-Val),
Z-(L)-Val-[Phe^{NN}(p-F)Phe]←((L)-Val)-Z,
Z-(L)-Val-[Phe^{NN}(p-CN)Phe]←((L)-Val)-Z,
Z-(L)-Ile-[Phe^{NN}Leu]←((L)-Ile)-Z,
Isobutoxycarbonyl (L)-Val-[Phe^{NN}Leu]←(N-isobutoxycarbonyl-(L)-Val),
Acetyl-Val-[Phe^{NN}Leu]←(N-(2(R,S)-carbamoyl-3-phenyl-propionyl)-Val),
N-Trifluoracetyl-[Phe^{NN}Leu]←(N-(2(R,S)-carbamoyl-3-phenylpropionyl)-(L)Val),
Z-(L)-Val-[Phe^{NN}Nle]←(N-(2(R,S)-(N-(2-morpholinoethyl)carbamoyl)-3-methyl)-butyryl),
Z-(L)-Val-[Phe^{NN}Nle]←(N-(2(R,S)-(N-(2-pyridylmethyl)-carbamoyl)-3-methyl)-butyryl),
Methoxycarbonyl-(L)-Val-[Phe^{NN}Leu]←(N-methoxycarbonyl-(L)-Val),
Methoxycarbonyl-(L)-Val-[Phe^{NN}(p-F)Phe]←(N-methoxycarbonyl-(L)-Val) und
Methoxycarbonyl-(L)-Val-[Phe^{NN}(p-CN)Phe]←(N-methoxycarbonyl-(L)-Val),
oder einem Salz davon, worin worin Boc für tert-Butoxycarbonyl steht, Z für Benzyloxycarbonyl steht, der Rest -[Phe^{NN}Phe]- das zweiwertige Radikal von 3(S)-Amino-4-phenyl-1-(N-benzylhydrazino)-butan-2(S)-ol bedeutet und die Formel 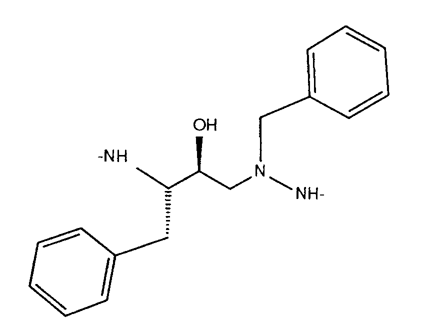 hat, der Rest -[Phe^{NN}Cha] das zweiwertige Radikal von 3(S)-Amino-4-phenyl-1-(N-cyclohexylmethylhydrazino)-butan-2(S)-ol bedeutet und die Formel 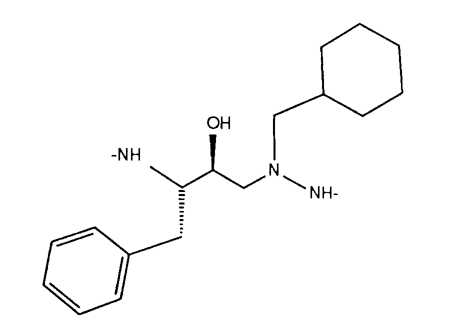 hat, der Rest -[Phe^{NN}Leu] das zweiwertige Radikal von 3(S)-Amino-4-phenyl-1-(N-isobutylhydrazino)-butan-2(S)-ol bedeutet und die Formel 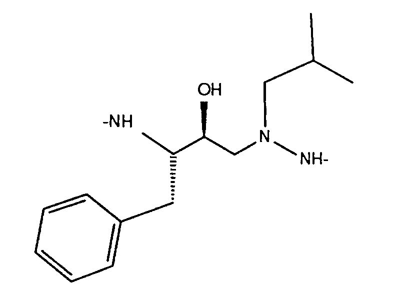 hat, der Rest mit der Bezeichnung -[Phe^{NN}Nle] das Radikal von 3(S)-Amino-4-phenyl-1-(N-n-butylhydrazino)-butan-2(S)-ol bedeutet und die Formel 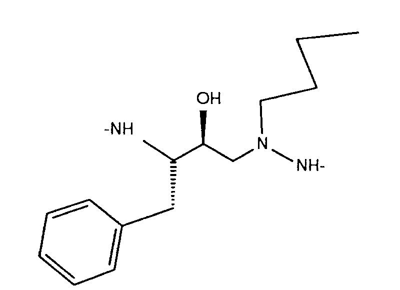 hat, der Rest mit der Bezeichnung -[Phe^{NN}(p-F)Phe] das zweiwertige Radikal von 3(S)-Amino-4-phenyl-1-(N-(p-fluorphenylmethyl)-hydrazino)-butan-2(S)-ol bedeutet und die Formel 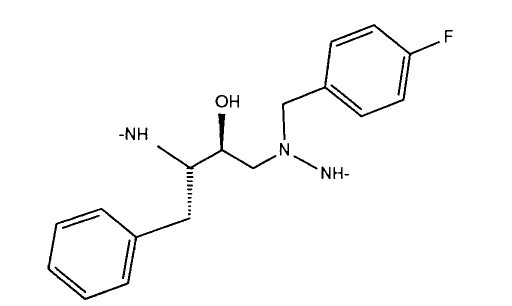 hat, der Rest mit der Bezeichnung -[(p-F)Phe^{NN}(p-F)Phe] das zweiwertige Radikal von 3(S)-Amino-4-(p-fluorphenyl)-1-(N-(p-fluorphenylmethyl)-hydrazino)-butan-2(S)-ol bedeutet und die Formel 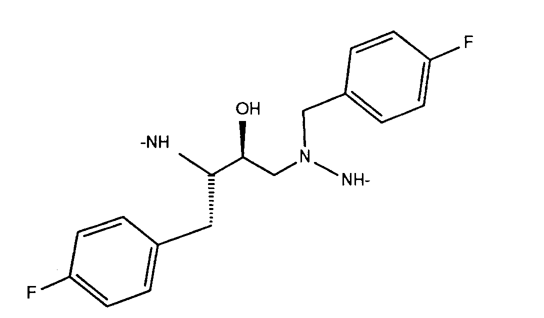 hat, der Rest mit der Bezeichnung -[Phe^{NN}(p-CN)Phe] das zweiwertige Radikal von 3(S)-Amino-4-phenyl-1-(N-(p-cyanophenylmethyl)-hydrazino)-butan-2(S)-ol bedeutet und die Formel 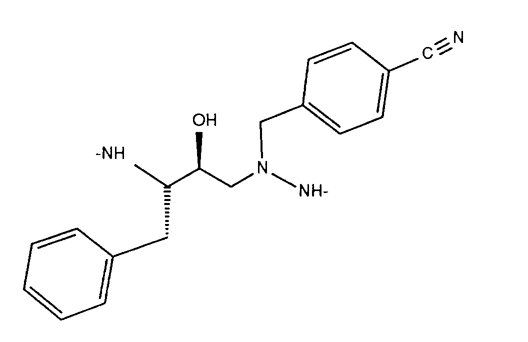 hat,
und ein Pfeil "←" die Umkehrung der Bindungsrichtung in Abweichung von der üblichen Peptidnomenklatur, worin links der Amino- und rechts der Carboxyterminus vorliegen, bedeutet.

11. Verbindungen der Formel I gemäss einem der Ansprüche 1 - 10 zur Anwendung in einem Verfahren zur therapeutischen Behandlung des menschlichen oder tierischen Körpers.

12. Pharmazeutische Präparate enthaltend Verbindungen der Formel I gemäss einem der Ansprüche 1 - 10 oder ein pharmazeutisch verwendbares Salz einer solchen Verbindung mit mindestens einer salzbildenden Gruppe zusammen mit pharmazeutisch verwendbarem Trägermaterial.

13. Verwendung einer der in den Ansprüchen 1 - 10 genannten Verbindungen der Formel I oder eines pharmazeutisch verwendbaren Salzes einer solchen Verbindung mit mindestens einer salzbildenden Gruppe zur Herstellung von pharmazeutischen Präparaten zur Anwendung zur Behandlung von AIDS.

14. Verfahren zur Herstellung einer Verbindung der Formel 1 gemäss Anspruch 1, worin die Substituenten die in Anspruch 1 genannten Bedeutungen haben, und deren Salzen, dadurch gekennzeichnet, dass man
a) ein Hydrazinderivat der Formel 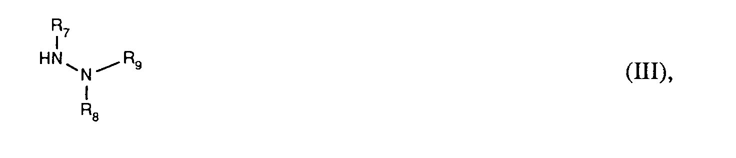 worin die Reste die oben genannten Bedeutungen haben, an ein Epoxid der Formel 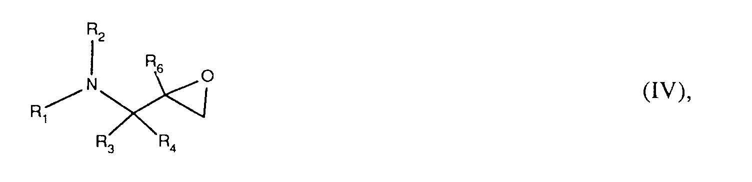 worin die Reste die oben genannten Bedeutungen haben, addiert, wobei freie funktionelle Gruppen mit Ausnahme der an der Reaktion beteiligten gegebenenfalls in geschützter Form vorliegen, und gegebenenfalls vorhandene Schutzgruppen abspaltet, oder
b) zur Herstellung von Verbindungen der Formel I, worin R₁ und R₉ Acyl, Sulfo, durch unsubstituiertes oder substituiertes Alkyl, Aryl, Alkoxy, das unsubstituiert oder substituiert ist, oder Aryloxy substituiertes Sulfonyl, Sulfamoyl, das am Stickstoff unsubstituiert oder substituiert ist, oder durch ein oder zwei unabhängig voneinander aus substituiertem oder unsubstituiertem Alkyl, unsubstituiertem oder substituiertem Cycloalkyl, Aryl, Hydroxy, unsubstituiertem oder substituiertem Alkoxy, Cycloalkoxy und Aryloxy ausgewählte Reste substituiertes Phosphoryl bedeuten, R₂ und R₈ Wasserstoff, unsubstituiertes oder substituiertes Alkyl, Alkenyl oder Alkinyl, oder Heterocyclyl bedeuten und die übrigen Reste die genannten Bedeutungen haben, eine Aminoverbindung der Formel 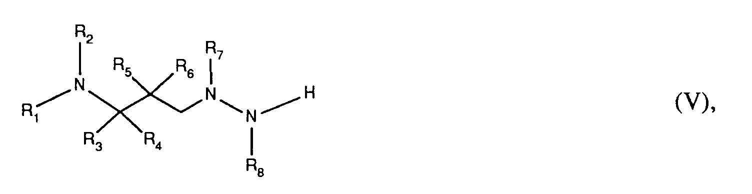 worin die Reste die gerade genannten Bedeutungen haben, mit einer Säure der Formel
R₉-OH (VI)
oder reaktionsfähigen Säurederivaten davon, worin R₉ die gerade genannten Bedeutungen hat, kondensiert, wobei freie funktionelle Gruppen mit Ausnahme der an der Reaktion beteiligten gegebenenfalls in geschützter Form vorliegen, und gegebenenfalls vorhandene Schutzgruppen abspaltet, oder
c) zur Herstellung von Verbindungen der Formel I, worin R₁ und R₉ Acyl, Sulfo, durch unsubstituiertes oder substituiertes Alkyl, Aryl, Alkoxy, das unsubstituiert oder substituiert ist, oder Aryloxy substituiertes Sulfonyl, Sulfamoyl, das am Stickstoff unsubstituiert oder substituiert ist, oder durch ein oder zwei unabhängig voneinander aus substituiertem oder unsubstituiertem Alkyl, unsubstituiertem oder substituiertem Cycloalkyl, Aryl, Hydroxy, unsubstituiertem oder substituiertem Alkoxy, Cycloalkoxy und Aryloxy ausgewählte Reste substituiertes Phosphoryl bedeuten, R₂ und R₈ Wasserstoff, unsubstituiertes oder substituiertes Alkyl, Alkenyl oder Alkinyl, oder Heterocyclyl bedeuten und die übrigen Reste die genannten Bedeutungen haben, eine Aminoverbindung der Formel 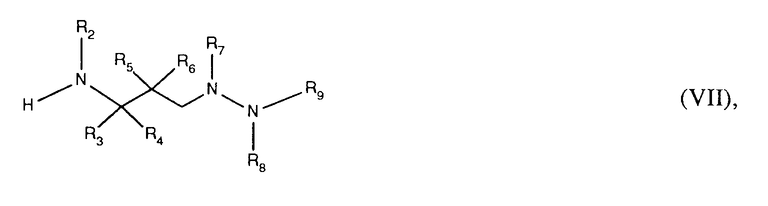 worin die Reste die gerade genannten Bedeutungen haben, mit einer Säure der Formel
R₁-OH (VIII)
oder reaktionsfähigen Säurederivaten davon, worin R₁ die gerade genannten Bedeutungen hat, kondensiert, wobei freie funktionelle Gruppen mit Ausnahme der an der Reaktion beteiligten gegebenenfalls in geschützter Form vorliegen, und gegebenenfalls vorhandene Schutzgruppen abspaltet, oder
d) zur Herstellung von Verbindungen der Formel I, worin R₁ und R₉ zwei gleiche Reste ausgewählt aus Acyl, Sulfo, durch unsubstituiertes oder substituiertes Alkyl, Aryl, Alkoxy, das unsubstituiert oder substituiert ist, oder Aryloxy substituiertes Sulfonyl, Sulfamoyl, das am Stickstoff unsubstituiert oder substituiert ist, und durch ein oder zwei unabhängig voneinander aus substituiertem oder unsubstituiertem Alkyl, unsubstituiertem oder substituiertem Cycloalkyl, Aryl, Hydroxy, unsubstituiertem oder substituiertem Alkoxy, Cycloalkoxy und Aryloxy ausgewählte Reste substituiertes Phosphoryl bedeuten, R₂ und R₈ Wasserstoff, unsubstituiertes oder substituiertes Alkyl, Alkenyl oder Alkinyl, oder Heterocyclyl bedeuten und die übrigen Reste die genannten Bedeutungen haben, eine Diaminoverbindung der Formel 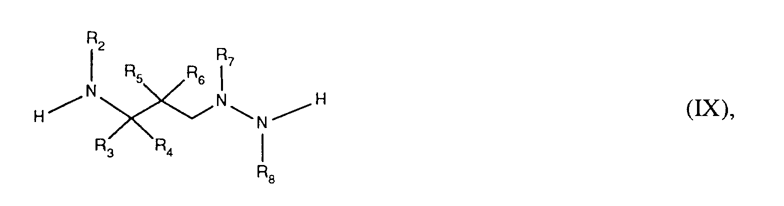 worin die Reste die gerade genannten Bedeutungen haben, mit einer zur Einführung der identischen Reste R₁ und R₉ geeigneten Säure oder reaktionsfähigen Säurederivaten davon, worin R₁ und R₉ die gerade genannten Bedeutungen haben, kondensiert, wobei freie funktionelle Gruppen mit Ausnahme der an der Reaktion beteiligten gegebenenfalls in geschützter Form vorliegen, und gegebenenfalls vorhandene Schutzgruppen abspaltet, oder
e) zur Herstellung einer Verbindung der Formel I, worin an Stelle von R₇ der Rest R₇'' vorliegt, welcher unsubstituiertes oder substituiertes Alkyl oder Cycloalkyl bedeutet, in einer Verbindung der Formel I', 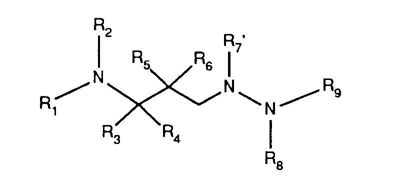 worin R₇' Wasserstoff bedeutet und die übrigen Reste die genannten Bedeutungen haben, den Rest R₇'' einführt durch Substitution mit einer Verbindung der Formel XII,
R₇''-X (XII),
worin X eine Abgangsgruppe ist und R₇'' unsubstituiertes oder substituiertes Alkyl oder Cycloalkyl bedeutet, wobei freie funktionelle Gruppen mit Ausnahme der an der Reaktion beteiligten gegebenenfalls in geschützter Form vorliegen, und gegebenenfalls vorhandene Schutzgruppen abspaltet, oder
f) in einer Verbindung der Formel I, worin die Substituenten die oben genannten Bedeutungen haben mit der Massgabe, dass in der betreffenden Verbindung der Formel I mindestens eine funktionelle Gruppe durch Schutzgruppen geschützt ist, vorhandene Schutzgruppen abspaltet
und gewünschtenfalls eine nach einem der vorstehenden Verfahren a) bis **f**) erhältliche Verbindung der Formel I mit mindestens einer salzbildenden Gruppe in ihr Salz oder ein erhältliches Salz in die freie Verbindung oder in ein anderes Salz überführt und/oder gegebenenfalls erhätliche Isomerengemische auftrennt und/oder eine erfindungsgemässe Verbindung der Formel I in eine andere erfindungsgemässe Verbindung der Formel I umwandelt.

15. Verbindungen der Formel 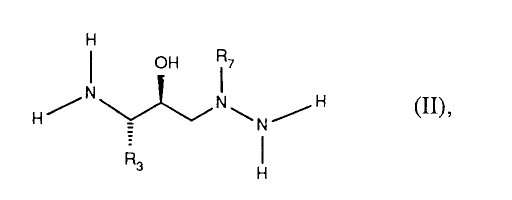 worin R₃ unsubstituiertes oder substituiertes Alkyl oder Cycloalkyl; Aryl; Heterocyclyl; oder unsubstituiertes oder substituiertes Alkenyl bedeutet; und R₇ unsubstituiertes oder substituiertes Alkyl oder Cycloalkyl; Aryl; Heterocyclyl; oder unsubstituiertes oder substituiertes Alkenyl bedeutet, sowie die Salze der genannten Verbindungen, sofern salzbildende Gruppen vorliegen.

## Patentansprüche (Patentansprüche für folgende(n) Vertragsstaat(en): ES, GR)

1. Verfahren zur Herstellung von Verbindungen der Formel 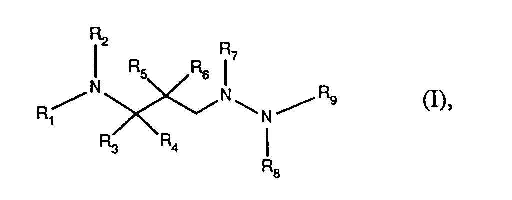 worin R₁ und R₉ unabhängig voneinander Wasserstoff, Acyl, unsubstituiertes oder substituiertes Alkyl, Alkenyl oder Alkinyl; Heterocyclyl; Sulfo, durch unsubstituiertes oder substituiertes Alkyl, Aryl, Alkoxy, das unsubstituiert oder substituiert ist, oder durch Aryloxy substituiertes Sulfonyl; Sulfamoyl, das am Stickstoff unsubstituiert oder substituiert ist; oder durch ein oder zwei unabhängig voneinander aus unsubstituiertem oder substituiertem Alkyl, aus unsubstituiertem oder substituiertem Cycloalkyl, aus Aryl, aus Hydroxy, aus unsubstituiertem oder substituiertem Alkoxy, aus Cycloalkoxy und aus Aryloxy ausgewählte Reste substituiertes Phosphoryl bedeuten, mit der Massgabe, dass höchstens einer der Reste R₁ und R₉ Wasserstoff bedeutet; und R₂ und R₈ jeweils unabhängig voneinander Wasserstoff oder einen der oben für R₁ und R₉ genannten Reste bedeuten; oder die Substituentenpaare R₁ und R₂ oder R₈ und R₉ jeweils unabhängig voneinander gemeinsam mit dem Stickstoffatom, an das sie gebunden sind, einen heterocyclischen Ring, bestehend aus dem bindenden Stickstoff zusammen mit einem aus Ethylen, Trimethylen, Tetramethylen und Pentamethylen ausgewählten Rest, worin ein Kohlenstoffatom durch Stickstoff, Sauerstoff, Schwefel oder mit Sauerstoff ein-oder zweifach substituierten Schwefel ersetzt sein kann und welcher ungesättigt sein kann, oder Napthalin-1,8-dicarbonylimido bilden können;
R₃ und R₄ unabhängig voneinander Wasserstoff, unsubstituiertes oder substituiertes Alkyl oder Cycloalkyl; Aryl; Heterocyclyl; oder unsubstituiertes oder substituiertes Alkenyl bedeuten; oder R₃ und R₄ gemeinsam unsubstituiertes oder substituiertes Alkylen, Alkyliden oder benzokondensiertes Alkylen bedeuten;
R₅ Hydroxy bedeutet; R₆ Wasserstoff bedeutet;
oder R₅ und R₆ gemeinsam Oxo bedeuten;
und R₇ unsubstituiertes oder substituiertes Alkyl oder Cycloalkyl; Aryl; Heterocyclyl;
oder unsubstituiertes oder substituiertes Alkenyl bedeutet;
sowie Salzen der genannten Verbindungen, sofern salzbildende Gruppen vorliegen; mit
Ausnahme der Verbindung der Formel I, worin R₁, R₂, R₃, R₄, R₆ und R₈ jeweils Wasserstoff bedeuten, R₅ Hydroxy bedeutet, R₇ einen Rest der Formel -[CH₂-(C=O)-NH-(N-CH₃)-CH₂-COOH bedeutet und R₉ tert-Butoxycarbonyl bedeutet; dadurch gekennzeichnet, dass man
a) ein Hydrazinderivat der Formel 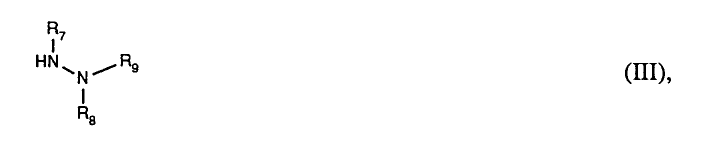 worin die Reste die oben genannten Bedeutungen haben, an ein Epoxid der Formel 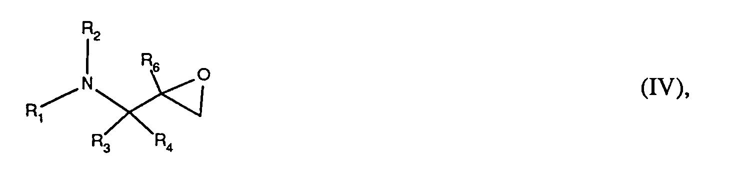 worin die Reste die oben genannten Bedeutungen haben, addiert, wobei freie funktionelle Gruppen mit Ausnahme der an der Reaktion beteiligten gegebenenfalls in geschützter Form vorliegen, und gegebenenfalls vorhandene Schutzgruppen abspaltet, oder
b) zur Herstellung von Verbindungen der Formel I, worin R₁ und R₉ Acyl, Sulfo, durch unsubstituiertes oder substituiertes Alkyl, Aryl, Alkoxy, das unsubstituiert oder substituiert ist, oder Aryloxy substituiertes Sulfonyl, Sulfamoyl, das am Stickstoff unsubstituiert oder substituiert ist, oder durch ein oder zwei unabhängig voneinander aus substituiertem oder unsubstituiertem Alkyl, unsubstituiertem oder substituiertem Cycloalkyl, Aryl, Hydroxy, unsubstituiertem oder substituiertem Alkoxy, Cycloalkoxy und Aryloxy ausgewählte Reste substituiertes Phosphoryl bedeuten, R₂ und R₈ Wasserstoff, unsubstituiertes oder substituiertes Alkyl, Alkenyl oder Alkinyl, oder Heterocyclyl bedeuten und die übrigen Reste die genannten Bedeutungen haben, eine Aminoverbindung der Formel 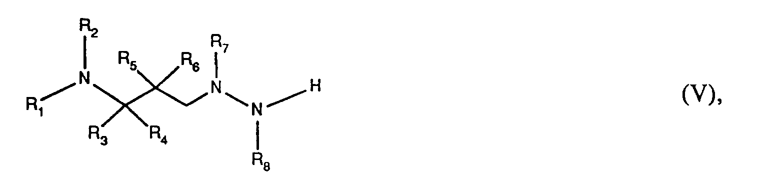 worin die Reste die gerade genannten Bedeutungen haben, mit einer Säure der Formel
R₉-OH (VI)
oder reaktionsfähigen Säurederivaten davon, worin R₉ die gerade genannten Bedeutungen hat, kondensiert, wobei freie funktionelle Gruppen mit Ausnahme der an der Reaktion beteiligten gegebenenfalls in geschützter Form vorliegen, und gegebenenfalls vorhandene Schutzgruppen abspaltet, oder
c) zur Herstellung von Verbindungen der Formel I, worin R₁ und R₉ Acyl, Sulfo, durch unsubstituiertes oder substituiertes Alkyl, Aryl, Alkoxy, das unsubstituiert oder substituiert ist, oder Aryloxy substituiertes Sulfonyl, Sulfamoyl, das am Stickstoff unsubstituiert oder substituiert ist, oder durch ein oder zwei unabhängig voneinander aus substituiertem oder unsubstituiertem Alkyl, unsubstituiertem oder substituiertem Cycloalkyl, Aryl, Hydroxy, unsubstituiertem oder substituiertem Alkoxy, Cycloalkoxy und Aryloxy ausgewählte Reste substituiertes Phosphoryl bedeuten, R₂ und R₈ Wasserstoff, unsubstituiertes oder substituiertes Alkyl, Alkenyl oder Alkinyl, oder Heterocyclyl bedeuten und die übrigen Reste die genannten Bedeutungen haben, eine Aminoverbindung der Formel 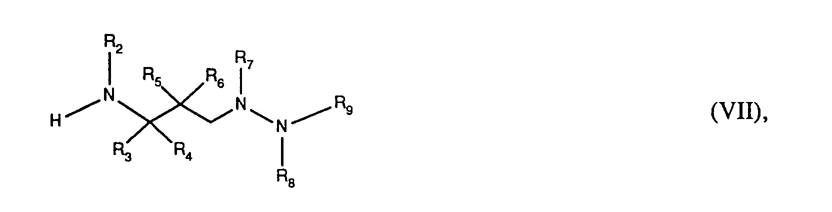 worin die Reste die gerade genannten Bedeutungen haben, mit einer Säure der Formel
R₁-OH (VIII)
oder reaktionsfähigen Säurederivaten davon, worin R₁ die gerade genannten Bedeutungen hat, kondensiert, wobei freie funktionelle Gruppen mit Ausnahme der an der Reaktion beteiligten gegebenenfalls in geschützter Form vorliegen, und gegebenenfalls vorhandene Schutzgruppen abspaltet, oder
d) zur Herstellung von Verbindungen der Formel I, worin R₁ und R₉ zwei gleiche Reste ausgewählt aus Acyl, Sulfo, durch unsubstituiertes oder substituiertes Alkyl, Aryl, Alkoxy, das unsubstituiert oder substituiert ist, oder Aryloxy substituiertes Sulfonyl, Sulfamoyl, das am Stickstoff unsubstituiert oder substituiert ist, und durch ein oder zwei unabhängig voneinander aus substituiertem oder unsubstituiertem Alkyl, unsubstituiertem oder substituiertem Cycloalkyl, Aryl, Hydroxy, unsubstituiertem oder substituiertem Alkoxy, Cycloalkoxy und Aryloxy ausgewählte Reste substituiertes Phosphoryl bedeuten, R₂ und R₈ Wasserstoff, unsubstituiertes oder substituiertes Alkyl, Alkenyl oder Alkinyl, oder Heterocyclyl bedeuten und die übrigen Reste die genannten Bedeutungen haben, eine Diaminoverbindung der Formel 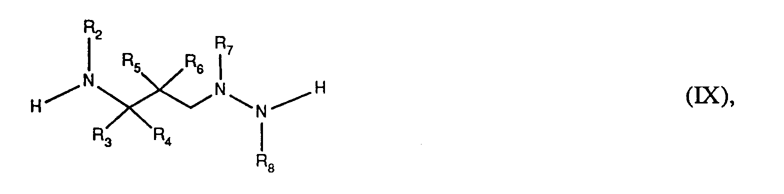 worin die Reste die gerade genannten Bedeutungen haben, mit einer zur Einführung der identischen Reste R₁ und R₉ geeigneten Säure oder reaktionsfähigen Säurederivaten davon, worin R₁ und R₉ die gerade genannten Bedeutungen haben, kondensiert, wobei freie funktionelle Gruppen mit Ausnahme der an der Reaktion beteiligten gegebenenfalls in geschützter Form vorliegen, und gegebenenfalls vorhandene Schutzgruppen abspaltet, oder
e) zur Herstellung einer Verbindung der Formel I, worin an Stelle von R₇ der Rest R₇'' vorliegt, welcher unsubstituiertes oder substituiertes Alkyl oder Cycloalkyl bedeutet, in einer Verbindung der Formel I', 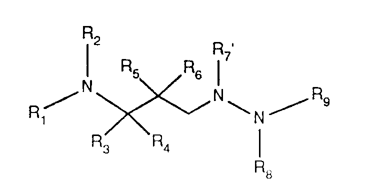 worin R₇' Wasserstoff bedeutet und die übrigen Reste die genannten Bedeutungen haben, den Rest R₇'' einführt durch Substitution mit einer Verbindung der Formel XII,
R₇''-X (XII)
worin X eine Abgangsgruppe ist und R₇'' unsubstituiertes oder substituiertes Alkyl oder Cycloalkyl bedeutet, wobei freie funktionelle Gruppen mit Ausnahme der an der Reaktion beteiligten gegebenenfalls in geschützter Form vorliegen, und gegebenenfalls vorhandene Schutzgruppen abspaltet, oder
f) in einer Verbindung der Formel I, worin die Substituenten die oben genannten Bedeutungen haben mit der Massgabe, dass in der betreffenden Verbindung der Formel I mindestens eine funktionelle Gruppe durch Schutzgruppen geschützt ist, vorhandene Schutzgruppen abspaltet
und gewünschtenfalls eine nach einem der vorstehenden Verfahren a) bis f) erhältliche Verbindung der Formel I mit mindestens einer salzbildenden Gruppe in ihr Salz oder ein erhältliches Salz in die freie Verbindung oder in ein anderes Salz überführt und/oder gegebenenfalls erhältliche Isomerengemische auftrennt und/oder eine erfindungsgemässe Verbindung der Formel I in eine andere erfindungsgemässe Verbindung der Formel I umwandelt.

2. Verfahren gemäss Anspruch 1 zur Herstellung einer Verbindung der Formel I, worin
R₁ und R₉ unabhängig voneinander Wasserstoff; Niederalkanoyl; Arylniederalkanoyl, worin Niederalkanoyl unsubstituiert oder substituiert ist durch Carbamoyl oder durch einen oder zwei aus Niederalkyl, Carboxyniederalkyl, Niederalkoxycarbonyl-niederalkyl, Diniederalkylamino-niederalkyl, Amino-carboxy-niederalkyl, Hydroxyniederalkyl und Diniederalkoxyniederalkyl ausgewählte Reste am Stickstoff substituiertes Carbamoyl; Heterocyclylniederalkanoyl; Halogenniederalkanoyl, welches bis zu 3 Halogenatome enthält; (N-Heterocyclylniederalkyl-carbamoyl)-niederalkanoyl; Niederalkoxycarbonyl; Arylniederalkoxycarbonyl; Heterocyclylniederalkoxycarbonyl; Niederalkylsulfonyl; N-Heterocyclylniederalkyl-N-niederalkyl-carbamoyl; oder einen Acylrest einer Aminosäure, deren Aminofunktion frei oder durch einen der übrigen bisher für R₁ und R₉ genannten Reste acyliert ist; bedeuten, mit der Massgabe, dass höchstens einer der Reste R₁ und R₉ Wasserstoff bedeutet;
R₂, R₄, R₆ und R₈ Wasserstoff bedeuten,
R₃ Niederalkyl, Cycloalkylniederalkyl oder Arylniederalkyl bedeutet,
R₅ Hydroxy bedeutet, und
R₇ unsubstituiertes oder substituiertes Niederalkyl bedeutet,
oder einem Salz davon, sofern wenigstens eine salzbildende Gruppe vorliegt, wobei in den oben genannten Definitionen der Ausdruck "Nieder" oder "nieder" bedeutet, dass der jeweilige Rest bis zu einschliesslich 7 Kohlenstoffatome hat; dadurch gekennzeichnet, dass man entsprechend substituierte Ausgangsmaterialien verwendet.

3. Verfahren gemäss Anspruch 1 zur Herstellung einer Verbindung der Formel I, worin
R₁ und R₉ unabhängig voneinander Wasserstoff; Niederalkanoyl; Arylniederalkanoyl, worin Aryl Phenyl, Indenyl, Indanyl, Naphthyl, Anthryl, Phenanthryl, Acenaphthyl oder Fluorenyl bedeutet und unsubstituiert oder durch Niederalkyl, Halogenniederalkyl, Phenyl, 1- oder 2-Naphthyl, Hydroxy, Niederalkoxy, Carbamoylniederalkoxy, N-Niederalkylcarbamoylniederalkoxy, N,N-Diniederalkylcarbamoylniederalkoxy, Amino, Mono- oder Diniederalkylamino, Niederalkanoylamino, Halogen, Carboxy, Niederalkoxycarbonyl, Phenyl-, Naphthyl- oder Fluorenylniederalkoxycarbonyl, Niederalkanoyl, Sulfo, Niederaikylsulfonyl, Phosphono, Hydroxyniederalkoxy-phosphoryl, Diniederalkoxyphosphoryl, Carbamoyl, Mono- oder Diniederalkylcarbamoyl, Sulfamoyl, Mond- oder Diniederalkylaminosulfonyl, Nitro und/oder Cyano ein- oder bis zu dreifach substituiert sein kann, wobei Phenyl bis zu dreimal vorliegen kann, und worin Niederalkanoyl unsubstituiert oder substituiert durch Carbamoyl oder durch mit einem oder zwei aus Niederalkyl, Carboxyniederalkyl, Niederalkoxycarbonyl-niederalkyl, Diniederalkylamino-niederalkyl, Amino-carboxy-niederalkyl, Hydroxyniederalkyl und Diniederalkoxyniederalkyl ausgewählte Reste am Stickstoff substituiertes Carbamoyl ist; Heterocyclylniederalkanoyl, worin Niederalkanoyl unsubstituiert ist und worin Heterocyclyl aus Thienyl, Furyl, Pyranyl, Pyrrolyl, Imidazolyl, Pyrazolyl, Oxazolyl, Isoxazolyl, Thiazolyl, Furazanyl, Pyridyl, Pyrazinyl, Pyrimidinyl, Pyridazinyl, Azepinyl, Indolyl, Benzimidazolyl, 1H-Indazolyl, Chinolyl, Isochinolyl, Chinoxalinyl, Chinazolinyl, Cinnolyl, Purinyl, Pteridinyl, Naphthyridinyl, 4H-Chinolizinyl, 3,1-Benzfuranyl, 4,1-Benzoxazinyl, 4,1-Benzthiazinyl, Cyclohexa[b]pyrrolyl, Cyclohepta[b]pyrrolyl, Cyclohexa[d]pyrazolyl, Cyclohexa[b]pyridyl, Cyclohexa[b]pyrazinyl, Cyclohexa[b]pyrimidinyl, Cyclohexa[b]-1,4-oxazinyl, Cyclohexa[b]-1,4-thiazinyl, Pyrrolidinyl, Pyrrolinyl, Imidazolidyl, 2-Imidazolinyl, 2,3-Dihydropyridyl, Piperidyl, Piperazinyl, 2,3,5,6-Tetrahydropyrazinyl, Morpholinyl, Thiomorpholinyl, S,S-Dioxo-thiomorpholinyl, Indolinyl, Isoindolinyl, 4,5,6,7-Tetrahydroindolyl, 1,2,3,4-Tetrahydrochinolyl, 1,2,3,4-Teträhydroisochinolyl, Chromanyl, Thiochromanyl, 1,2,3,4-Tetrahydro-3,1-benzdiazinyl, 3,4-Dihydro-3H-4,1-benzoxazinyl und 3,4-Dihydro-3H-4,1-benzthiazinyl ausgewählt ist, wobei die genannten Heterocyclyl-Reste unsubstituiert oder durch Niederalkyl, Phenyl, 1-oder 2-Naphthyl, Phenylniederalkyl, Hydroxyniederalkyl, Niederalkoxyniederalkyl, Phenoxy- oder Naphthoxyniederalkyl, Phenylniederalkoxy- oder Naphthylniederalkoxy-niederalkyl, Niederalkanoyloxy-niederalkyl, Phenyl- oder Napthyl-niederalkanoyloxy-niederalkyl, Niederalkoxycarbonyloxy-niederalkyl, Phenyl-, Napthyl- oder Fluorenylniederalkoxycarbonyloxy-niederalkyl, Aminoniederalkyl, Carboxyniederalkyl, Hydroxy, Niederalkoxy, Phenyl- oder Naphthylniederalkoxy, Amino, Niederalkylamino, Diniederalkylamino, Carboxy, Niederalkoxycarbonyl, Phenyl- oder Napthyl-niederalkoxycarbonyl, Halogen, Niederalkanoyl, Niederalkylsulfonyl, Phosphono, Hydroxy-niederalkoxy-phosphoryl oder Dialkoxy-phosphoryl, Carbamoyl, Mono- oder Diniederalkylcarbamoyl, Hydroxy- oder Carboxyniederalkylcarbamoyl, Sulfamoyl, durch Nitro, Oxo und/oder durch Cyano substituiert sind; eterocyclylniederalkanoyl wie Halogenniederalkanoyl, welches bis zu drei Halogenatome enthält; (N-Heterocyclylniederalkyl-carbamoyl)-niederalkanoyl, worin Heterocyclyl aus Pyrrolyl, Furyl, Thienyl, Imidazolyl, Pyrazolyl, Oxazolyl, Thiazolyl, Pyridyl, Pyrazinyl, Pyrimidinyl, Indolyl, Chinolyl, Isochinolyl, Chinoxalinyl, β-Carbolinyl und einem benzanellierten, cyclopenta-, cyclohexa- oder cyclohepta-anellierten Derivat dieser Reste, die auch ganz oder teilweise gesättigt sein können, aus Morpholin und aus Thiomorpholin ausgewählt ist; Niederalkoxycarbonyl; Arylniederalkoxycarbonyl, worin Aryl Phenyl, Biphenylyl, 1- oder 2-Naphthyl, Fluorenyl oder durch Niederalkyl, Hydroxy, Niederalkoxy, Halogen und/oder Nitro mono- oder mehrfach substituiertes Phenyl ist; Heterocyclylniederalkoxycarbonyl, worin Heterocyclyl aus Pyrrolyl, Furyl, Thienyl, Imidazolyl, Pyrazolyl, Oxazolyl, Thiazolyl, Pyridyl, Pyrazinyl, Pyrimidinyl, Indolyl, Chinolyl, Isochinolyl, Chinoxalinyl, β-Carbolinyl und einem benzanellierten, cyclopenta-, cyclohexa- oder cyclohepta-anellierten Derivat dieser Reste, die auch ganz oder teilweise gesättigt vorliegen können, Niederalkylsulfonyl; N-Heterocyclylniederalkyl-N-niederalkyl-carbamoyl, worin Heterocyclyl ausgewählt ist aus Thienyl, Furyl, Pyranyl, Pyrrolyl, Imidazolyl, Pyrazolyl, Oxazolyl, Isoxazolyl, Thiazolyl, Furazanyl, Pyridyl, Pyrazinyl, Pyrimidinyl, Pyridazinyl, Azepinyl, Indolyl, Benzimidazolyl, 1H-Indazolyl, Chinolyl, Isochinolyl, Chinoxalinyl, Chinazolinyl, Cinnolyl, Purinyl, Pteridinyl, Naphthyridinyl, 4H-Chinolizinyl, 3,1-Benzfuranyl, Benz[e]indolyl, 4,1-Benzoxazinyl, 4,1-Benzthiazinyl, Carbazoyl, β-Carbolinyl, Phenazinyl, Phenanthridyl, Acridyl, Phenoxazinyl, Phenothiazinyl, 1-Azaacenaphtenyl, Cyclohexa[b]pyrrolyl, Cyclohepta[b]pyrrolyl, Cyclohexa[d]pyrazolyl, Cyclohexa[b]pyridyl, Cyclohexa[b]pyrazinyl, Cyclohexa[b]pyrimidinyl, Cyclohexa[b]-1,4-oxazinyl, Cyclohexa[b]-1,4-thiazinyl, Pyrrolidinyl, Pyrrolinyl, Imidazolidinyl, 2-Imidazolinyl, 2,3-Dihydropyridyl, Piperidyl, Piperazinyl, 2,3,5,6-Tetrahydropyrazinyl, Morpholinyl, Thiomorpholinyl, S,S-Dioxothiomorpholinyl, Indolinyl, Isoindolinyl, 4,5,6,7-Tetrahydroindolyl, 1,2,3,4-Tetrahydrochinolyl, 1,2,3,4-Tetrahydroisochinolyl, Chromanyl, Thiochromanyl, 1,2,3,4-Tetrahydro-3,1-benzdiazinyl, 3,4-Dihydro-3H-4,1-benzoxazinyl, 3,4-Dihydro-3H-4,1-benzthiazinyl, 2,3,4,5-Tetrahydro-1H-5,1-benzazepinyl und 5,6-Dihydrophenanthridinyl, wobei die genannten Reste unsubstituiert oder durch Niederalkyl, Phenyl, 1- oder 2-Naphthyl, Phenylniederalkyl, Hydroxyniederalkyl, Niederalkoxyniederalkyl, Phenoxy- oder Naphthoxyniederalkyl, Phenylniederalkoxy-oder Naphthylniederalkoxy-niederalkyl, Niederalkanoyloxy-niederalkyl, Phenyl-oder Napthyl-niederalkanoyloxy-niederalkyl, Niederalkoxycarbonyloxy-niederalkyl, Phenyl-, Napthyl- oder Fluorenylniederalkoxycarbonyloxy-niederalkyl, Aminoniederalkyl, Carboxyniederalkyl, Hydroxy, Niederalkoxy, Phenyl- oder Naphthylniederalkoxy, Amino, Niederalkylamino, Diniederalkylamino, Carboxy, Niederalkoxycarbonyl, Phenyl-oder Napthylniederalkoxycarbonyl, Halogen, Niederalkanoyl, Niederalkylsulfonyl, Phosphono, Hydroxy-niederalkoxyphosphoryl oder Dialkoxy-phosphoryl, Carbamoyl, Mono-oder Diniederalkylcarbamoyl, Hydroxy- oder Carboxyniederalkylcarbamoyl, Sulfamoyl, durch Nitro, Oxo und/oder durch Cyano substituiert sind; oder einen Acylrest einer Aminosäure, deren Aminofunktion frei oder durch einen der übrigen bisher für R₁ und R₉ genannten Reste acyliert ist, bedeuten, wobei die Aminosäuren aus Glycin, Alanin, Valin, Norvalin, Leucin, Isoleucin, Norleucin, Serin, Homoserin, Threonin, Methionin, Cystein, Prolin, trans-3- und trans-4-Hydroxyprolin, Phenylalanin, Tyrosin, 4-Aminophenylalanin, 4-Chlorphenylalanin, 4-Carboxyphenylalanin, β-Phenylserin, Phenylglycin, α-Naphthylalanin, Cyclohexylalanin, Cyclohexylglycin, Tryptophan, Asparaginsäure, Asparagin, Aminomalonsäure, Aminomalonsäure-monoamid, Glutaminsäure, Glutamin, Histidin, Arginin, Lysin, δ-Hydroxylysin, Ornithin, 3-Aminopropansäure, α,γ-Diaminobuttersäure und α,β-Diaminopropionsäure ausgewählt sind, worin (ausser Glycin) jeder der genannten Aminosäurereste in der D-, L- oder (D,L)-Form vorliegt; mit der Massgabe, dass höchstens einer der Reste R₁ und R₉ Wasserstoff bedeutet,
R₂, R₄, R₆ und R₈ Wasserstoff bedeuten,
R₃ Niederalkyl; Cycloalkylniederalkyl, worin Cycloalkyl 3 bis 7 Kohlenstoffatome hat und unsubstituiert oder durch Niederalkyl, Halogenniederalkyl, Hydroxy, Niederalkoxy, Carbamoylniederalkoxy, N-Niederalkylcarbamoylniederalkoxy, N,N-Diniederalkylcarbamoylniederalkoxy, Amino, Mono- oder Diniederalkylamino, Halogen, Carboxy, Niederalkoxycarbonyl, Phenyl-, Naphthyl- oder Fluorenylniederalkoxycarbonyl, Niederalkanoyl, Niederalkylsulfonyl, Phosphono, Hydroxy-niederalkoxy-phosphoryl oder Diniederalkoxyphosphoryl, Carbamoyl, Mono- oder Diniederalkylaminocarbamoyl, Sulfamoyl, Mono- oder Diniederalkylsulfamoyl, Nitro und/oder Cyano ein-oder bis zu dreifach substituiert ist; oder Arylniederalkyl bedeutet, worin Aryl Phenyl, Indenyl, Indanyl, Naphthyl, Anthryl, Phenanthryl, Acenaphthyl oder Fluorenyl bedeutet und unsubstituiert oder durch Niederalkyl, Halogenniederalkyl, Phenyl, 1-oder 2-Naphthyl, Hydroxy, Niederalkoxy, Carbamoylniederalkoxy, N-Niederalkylcarbamoylniederalkoxy, N,N-Diniederalkylcarbamoylniederalkoxy, Amino, Mono- oder Diniederalkylamino, Niederalkanoylamino, Halogen, Carboxy, Niederalkoxycarbonyl, Benzyl-, Naphthyl- oder Fluorenylniederalkoxycarbonyl, Niederalkanoyl, Sulfo, Niederalkylsulfonyl, Phosphono, Hydroxy-niederalkoxy-phosphoryl, Diniederalkoxyphosphoryl, Carbamoyl, Mono-oder Diniederalkylcarbamoyl, Sulfamoyl, Mono- oder Diniederalkylsulfamoyl, Nitro und/oder Cyano ein- oder bis zu dreifach substituiert sein kann, wobei Phenyl bis zu dreimal vorliegen kann,
R₅ Hydroxy bedeutet, und
R₇ unsubstituiertes Niederalkyl; oder Cycloalkyiniederalkyl, wie zuletzt für Cycloalkylniederalkyl R₃ beschrieben; oder Arylniederalkyl, wie zuletzt für Arylniederalkyl R₃ beschrieben; bedeutet
oder von einem Salz davon, sofern wenigstens eine salzbildende Gruppe vorliegt, wobei in den oben genannten Definitionen der Ausdruck "Nieder" oder "nieder" bedeutet, dass der jeweilige Rest bis zu einschliesslich 7 Kohlenstoffatome hat; dadurch gekennzeichnet, dass man entsprechend substituierte Ausgangsmaterialien verwendet.

4. Verfahren gemäss Anspruch 1 zur Herstellung einer Verbindung der Formel I, worin R₁ und R₉ unabhängig voneinander
- Wasserstoff,
- Niederalkoxycarbonyl,
- 2-Halogenniederalkoxycarbonyl,
- Aryloxycarbonyl, worin Aryl 6 bis 14 Kohlenstoffatome hat,
- Arylniederalkoxycarbonyl, worin Aryl 6 bis 14 Kohlenstoffatome hat,
- Heterocyclylniederalkoxycarbonyl, worin Heterocyclyl aus Pyrrolyl, Furyl, Thienyl, Imidazolyl, Pyrazolyl, Oxazolyl, Thiazolyl, Pyridyl, Pyrazinyl, Pyrimidinyl, Indolyl, Chinolyl, Isochinolyl, Chinoxalinyl, β-Carbolinyl und einem benzanellierten, cyclopenta-, cyclohexa- oder cyclohepta-anellierten Derivat dieser Reste, die ganz oder teilweise gesättigt vorliegen können, ausgewählt ist,
- 2-Triniederalkylsilylniederalkoxycarbonyl,
- 2-Triarylsilylniederalkoxycarbonyl, worin Aryl Phenyl oder 1- oder 2-Naphthyl ist,
- das über die Carboxygruppe gebundene Radikal einer einer Aminosäure ausgewählt aus Glycin, Alanin, Valin, Norvalin, Leucin, Isoleucin, Norleucin, Serin, Homoserin, Threonin, Methionin, Cystein, Prolin, trans-3- und trans-4-Hydroxyprolin, Phenylalanin, Tyrosin, 4-Aminophenylalanin, 4-Chlorphenylalanin, 4-Carboxyphenylalanin, β-Phenylserin, Phenylglycin, α-Naphthylalanin, Cyclohexylalanin, Cyclohexylglycin, Tryptophan, Indolin-2-carbonsäure, 1,2,3,4-Tetrahydroisochinolin-3-carbonsäure, Asparaginsäure, Asparagin, Aminomalonsäure, Aminomalonsäure-monoamid, Glutaminsäure, Glutamin, Histidin, Arginin, Lysin, δ-Hydroxylysin, Ornithin, α,γ-Diaminobuttersäure und α,β-Diaminopropionsäure, wobei jede dieser Aminosäuren in der D-, L- oder (D,L)-Form vorliegen kann, und
die α-Aminogruppe unsubstituiert oder ein- oder zweifach N-alkyliert durch Niederalkyl, durch Aminoniederalkyl, durch Phenyl- oder Naphthylaminoniederälkyl, oder durch Niederalkyl am Stickstoff substituiertes Piperazinylcarbonylniederalkyl, oder N-acyliert durch Niederalkanoyl, durch Arylniederalkanoyl, worin Aryl ausgewählt ist aus Phenyl, Indenyl, Indanyl, Naphthyl, Anthryl, Phenanthryl, Acenaphthyl und Fluorenyl, und unsubstituiert oder durch Niederalkyl, Halogenniederalkyl, Phenyl, 1- oder 2-Naphthyl, Hydroxy, Niederalkoxy, Carbamoylniederalkoxy, N-Niederalkylcarbamoylniederalkoxy oder N,N-Diniederalkylcarbamoylniederalkoxy, Amino, Mono- oder Diniederalkylamino, Niederalkanoylamino, Halogen, Carboxy, Niederalkoxycarbonyl, Phenyl-, Naphthyl- oder Fluorenylniederalkoxycarbonyl, Niederalkanoyl, Sulfo, Niederalkylsulfonyl, Phosphono, Hydroxy-niederalkoxy-phosphoryl oder Diniederalkoxyphosphoryl, Carbamoyl, Mono-oder Diniederalkylcarbamoyl, Sulfamoyl, Mono- oder Diniederalkylaminosulfonyl, Nitro und/oder Cyano ein- oder bis zu dreifach substituiert sein kann; wobei Phenyl bis zu dreimal vorliegen kann, und Niederalkanoyl unsubstituiert oder substituiert durch Niederalkyl, Heterocyclyl ausgewählt aus Pyrrolyl, Furyl, Thienyl, Imidazolyl, Pyrazolyl, Oxazolyl, Thiazolyl, Pyridyl, Pyrazinyl, Pyrimidinyl, Indolyl, Chinolyl, Isochinolyl, Chinoxalinyl, β-Carbolinyl und einem benz-, cyclopenta-, cyclohexa-oder cyclohepta-anellierten Derivat dieser Reste, die auch ganz oder teilweise gesättigt vorliegen können, Hydroxy, Niederalkoxy, Niederalkanoyloxy, Acetoacetoxy, Amino-oder Benzyloxycarbonylamino-niederalkanoyloxy, Arylniederalkanoyloxy, worin Aryl 6 - 10 Kohlenstoffatome hat, Niederalkoxycarbonyloxy, Mono- oder Diniederalkyl-aminocarbonyloxy, Aryloxycarbonyloxy, worin Aryl 6 - 10 Kohlenstoffatome hat, Arylniederalkoxycarbonyloxy mit Aryl von 6 - 14 Kohlenstoffattomen, Sulfonyloxy, Niederalkylsulfonyloxy, Phenylsulfonyloxy, 2- oder 4-Toluolsulfonyloxy oder 1- oder 2- Naphthylsulfonyloxy, Carboxy, verestertes Carboxy ausgewählt aus Niederalkoxycarbonyl, Aryloxycarbonyl, worin Aryl 6 - 10 Kohlenstoffatome hat, Arylniederalkoxycarbonyl, worin Aryl 6 - 12 Kohlenstoffatome hat, Niederalkanoyl, Niederalkylsulfonyl, Hydroxy-niederalkoxyphosphoryl und Diniederalkoxyphosphoryl, Carbamoyl, durch einen oder zwei aus Niederalkyl, Carboxyniederalkyl, Niederalkoxycarbonyl-niederalkyl, Diniederalkylaminoniederalkyl, Aminocarboxyniederalkyl, Hydroxyniederalkyl und aus Diniederalkoxyniederalkyl ausgewählte Reste oder einen aus Ethylen, Trimethylen, Tetramethylen und Pentamethylen ausgewählten Rest, worin ein Kohlenstoffatom durch Stickstoff, Sauerstoff, Schwefel oder mit Sauerstoff ein- oder zweifach substituierten Schwefel ersetzt sein kann und das ungesättigt sein kann, substituiertes Carbamoyl, ferner Sulfamoyl, Phosphono, Benzfuranyl, Oxo und/oder Cyano sein kann und unverzweigt oder verzweigt ist; durch Heterocyclylniederalkanoyl ausgewählt aus Thienyl-, Furyl-, Pyranyl-, Pyrrolyl-, Imidazolyl-, Pyrazolyl-, Oxazolyl-, Isoxazolyl-, Thiazolyl-, Furazanyl-, Pyridyl-, Pyrazinyl-, Pyrimidinyl-, Pyridazinyl-, Azepinyl-, Indolyl-, Benzimidazolyl-, 1H-Indazolyl-, Chinolyl-, Isochinolyl-, Chinoxalinyl-, Chinazolinyl-, Cinnolyl-, Purinyl-, Pteridinyl-, Naphthyridinyl-, 4H-Chinolizinyl-, 3,1Benzfuranyl, Benz[e]indolyl-, 4,1-Benzoxazinyl-, 4,1-Benzthiazinyl-, Carbazolyl-, β-Carbolinyl-, Phenazinyl-, Phenanthridyl-, Acridyl-, Phenoxazinyl-, Phenothiazinyl-, 1-Azaacenaphtenyl-, Cyclohexa[b]pyrrolyl-, Cyclohepta[b]pyrrolyl-, Cyclohexa[d]pyrazolyl-, Cyclohexa[b]pyridyl-, Cyclohexa[b]pyrazinyl-, Cyclohexa[b]pyrimidinyl, Cyclohexa[b]-1,4-oxazinyl-, Cyclohexa[b]-1,4-thiazinyl-, Pyrrolidinyl, Pyrrolinyl-, Imidazolidinyl-, 2-Imidazolinyl-, 2,3-Dihydropyridyl-, Piperidyl-, Piperazinyl-, 2,3,5,6-Tetrahydropyrazinyl-, Morpholinyl-, Thiomorpholinyl-, S,S-Dioxo-thiomorpholinyl, Indolinyl-, Isoindolinyl-, 4,5,6,7-Tetrahydroindolyl-, 1,2,3,4-Tetrahydrochinolyl-, 1,2,3,4-Tetrahydroisochinolyl-, Chroman-, Thiochroman-, 1,2,3,4-Tetrahydro-3,1-benzdiazinyl-, 3,4-Dihydro-3H-4,1-benzoxazinyl-, 3,4-Dihydro-3H-4,1benzthiazinyl-, 2,3,4,5-Tetrahydro-1H-5,1-benzazepinyl- und 5,6-Dihydrophenanthridinyl-niederalkanoyl, wobei die genannten Heterocyclyl-Reste unsubstituiert oder durch Niederalkyl, Phenyl, 1- oder 2-Naphthyl, Phenylniederalkyl, Hydroxyniederalkyl, Niederalkoxyniederalkyl, Phenoxy- oder Naphthoxyniederalkyl, Phenylniederalkoxy- oder Naphthylniederalkoxy-niederalkyl, Niederalkanoyloxy-niederalkyl, Phenyl- oder Napthyl-niederalkanoyloxy-niederalkyl, Niederalkoxycarbonyloxy-niederalkyl, Phenyl-, Napthyl- oder Fluorenylniederalkoxycarbonyloxy-niederalkyl, Aminoniederalkyl, Carboxyniederalkyl, Hydroxy, Niederalkoxy, Phenyl- oder Naphthylniederalkoxy, Amino, Niederalkylamino, Diniederalkylamino, Carboxy, Niederalkoxycarbonyl, Phenyl- oder Napthyl-niederalkoxycarbonyl, Halogen, Niederalkanoyl, Niederalkylsulfonyl, Phosphono, Hydroxy-niederalkoxy-phosphoryl, Dialkoxy-phosphoryl, Carbamoyl, Mono- oder Di-niederalkylcarbamoyl, Hydroxy- oder Carboxyniederalkylcarbamoyl, Sulfamoyl, durch Nitro, Oxo und/oder durch Cyano substituiert sind; durch Heterocyclylniederalkenoyl, worin Heterocyclyl ausgewählt ist aus Pyrrolyl, Furyl, Thienyl, Imidazolyl, Pyrazolyl, Oxazolyl, Thiazolyl, Pyridyl, Pyrazinyl, Pyrimidinyl, Indolyl, Chinolyl, Isochinolyl, Chinoxalinyl, β-Carbolinyl und einem benzanellierten, cyclopenta-, cyclohexa- oder cyclohepta-anellierten Derivat dieser Reste, die auch ganz oder teilweise gesättigt sein können, durch Niederalkoxycarbonyl, durch Arylniederalkoxycarbonyl, worin Aryl 6 - 14 Kohlenstoffatome hat; durch Heterocyclylniederalkoxycarbonyl, worin Heterocyclyl ausgewählt ist aus Pyrrolyl, Furyl, Thienyl, Imidazolyl, Pyrazolyl, Oxazolyl, Thiazolyl, Pyridyl, Pyrazinyl, Pyrimidinyl, Indolyl, Chinolyl, Isochinolyl, Chinoxalinyl, β-Carbolinyl und einem benzanellierten, cyclopenta-, cyclohexa- oder cyclohepta-anellierten Derivat dieser Reste, die auch ganz oder teilweise gesättigt und unsubstituiert oder substituiert durch Niederalkyl sein können; durch Carboxyniederalkanoyl, durch Niederalkoxycarbonylniederalkanoyl, durch Hydroxyniederälkoxy-niederalkanoyl, durch Aminoniederalkanoyl oder durch Benzyloxycarbonylaminoniederalkanoyl, worin die Aminogruppe nicht in α- oder β-Stellung gebunden ist, durch Carbamoyl, durch Phenylniederalkylaminocarbonyl, durch N-Diniederalkylaminoniederalkyl-N-niederalkylaminocarbonyl, durch N-Dihydroxyniederalkyl-N-niederalkyaminocarbonyl, durch 2- oder 3-Pyridylniederalkylaminocarbonyl, durch N-2-Prridylniederalkyl-N-niederalkylaminocarbonyl, durch Sulfonyl, durch Niederalkylsulfonyl, durch Arylsulfonyl, worin Aryl 6 - 10 Kohlenstoffatome hat und unsubstituiert oder durch Niederalkyl oder Niederalkoxy substituiert ist; durch Heterocyclylsulfonyl, worin Heterocyclyl ausgewählt ist aus Pyrrolyl, Furyl, Thienyl, Imidazolyl, Pyrazolyl, Oxazolyl, Thiazolyl, Pyridyl, Pyrazinyl, Pyrimidinyl, Indolyl, Chinolyl, Isochinolyl, Chinoxalinyl, β-Carbolinyl und einem benzanellierten, cyclopenta-, cyclohexa- oder cyclohepta-anellierten Derivat dieser Reste, die auch ganz oder teilweise gesättigt sein können; durch Sulfamoyl, oder durch mit Heterocyclylniederalkyl, worin Heterocyclyl wie zuletzt definiert ist, und/oder mit Niederalkyl substituiertes Sulfamoyl vorliegen kann,
eine Carboxygruppe der Seitenkette frei oder in veresterter Form als Niederalkylestergruppe, als Arylestergruppe oder als Arylniederalkylestergruppe, wobei Aryl Phenyl, 4-Nitrophenyl, Naphthyl oder Biphenylyl bedeutet, oder in amidierter Form als Carbamoyl-, als Niederalkylcarbamoyl-, als Diniederalkylaminocarbamoyl-, als Mono- oder Di-(hydroxyniederalkyl)carbamoyl- oder als Mono- oder Di-(carboxyniederalkyl)carbamoylgruppe vorliegt,
eine Aminogruppe der Seitenkette frei oder in alkylierter Form als Mono-oder Diniederalkylamino oder in acylierter Form als Niederalkanoylamino, als Aminoniederalkanoylamino, als Arylniederalkanoylamino, worin Aryl 6 - 14 Kohlenstoffatome hat und unsubstituiert oder durch Niederalkyl, Hydroxy, Niederalkoxy, Carboxy, Carbamoyl oder Sulfamoyl substituiert ist, als Niederalkoxycarbonylamino-, als Arylmethoxycarbonylaminogruppe, worin Aryl 6 - 14 Kohlenstoffatome hat, als Piperidyl-1-carbonyl, Morpholinocarbonyl, Thiomorpholinocarbonyl oder als S,S-Dioxothiomorpholinocarbonyl vorliegt und/oder
eine Hydroxygruppe der Seitenkette frei oder in veretherter oder veresterter Form als Niederalkoxy-, als Arylniederalkoxy-, als Niederalkanoyloxy-, oder als Niederalkyloxycarbonyloxygruppe vorliegt,
- Niederalkylsulfonyl,
- 2- oder 3-Pyrrolyl-, 2-Thienyl-, 2-Furyl-, 1-Pyrazolyl-, 2-, 3- oder 4-Pyridyl-, 2-, 3- oder 5-Indolyl- , (1-Methyl-, 2-Methyl-, 5-Methoxy-, 5-Benzyloxy-, 5-Chlor- oder 4,5-Dimethyl-indol-2-yl)-, (1-Benzylindol-2-yl oder -3-yl)-, 4,5,6,7-Tetrahydroindol-2-yl-, (2-, 3- oder 4-Chinolyl oder 4-Hydroxychinol-2-yl)-, (1-, 3- oder 4-Isochinolyl oder 1-Oxo-1,2-dihydroisochinol-3-yl)-, 3-Pyrrolidinyl-, (3- oder 4-Hydroxypyrrolidin-2-yl)-, 5-Oxopyrrolidin-2-yl-, (2- oder 3-Morpholinyl)-, (2- oder 3-Thiomorpholinyl)-, (S,S-Dioxothiomorpholin-2- oder -3-yl)-, (2- oder 3-Indolinyl)-, (1,2,3,4-Tetrahydrochinol-2-, -3- oder -4-yl)- oder (1,2,3,4-Tetrahydroisochinol-1-, -2- oder -3-yl)-methylsulfonyl
- unsubstituiertes oder durch Niederalkyl ein- oder zweifach substituiertes Phenyl-oder 1- oder 2-Napthyl-sulfonyl,
- Niederalkoxysulfonyl, oder
- Benzyloxysulfonyl oder 1- oder 2-Naphthyloxysulfonyl
bedeuten, so dass höchstens einer der Reste R₁ oder R₉ Wasserstoff bedeuten kann, und
R₂ und R₈ unabhängig voneinander Wasserstoff oder die gleichen Reste wie R₁ und R₉ bedeuten,
oder die Substituentenpaare R₁ und R₉ oder R₂ und R₈ jeweils unabhängig voneinander gemeinsam mit dem bindenden Stickstoff und einem aus Ethylen, Trimethylen, Tetramethylen und Pentamethylen ausgewählten Rest, worin ein Kohlenstoffatom durch Stickstoff, Sauerstoff, Schwefel oder mit Sauerstoff ein- oder zweifach substituierten Schwefel ersetzt sein kann und das ungesättigt sein kann, einen heterocyclischen Ring bilden,
R₃
- Cycloalkylniederalkyl, worin Cycloalkyl 3 - 7 Kohlenstoffatome hat und unsubstituiert oder durch Niederalkyl, Halogenniederalkyl, Hydroxy, Niederalkoxy, Carbamoylniederalkoxy, N-Niederalkylcarbamoylniederalkoxy oder N,N-Diniederalkylcarbamoylniederalkoxy, Amino, Mono- oder Diniederalkylamino, Halogen, Carboxy, Niederalkoxycarbonyl, Phenyl-, Naphthyl- oder Fluorenylniederalkoxycarbonyl, Niederalkanoyl, Niederalkylsulfonyl, Phosphono, Hydroxy-niederalkoxy-phosphoryl oder Diniederalkoxyphosphoryl, Carbamoyl, Mono- oder Diniederalkylaminocarbamoyl, Sulfamoyl, Mono-oder Diniederalkylsulfamoyl, Nitro und/oder Cyano ein- oder bis zu dreifach substituiert ist und an Niederalkyl gebunden ist, oder
- Arylniederalkyl mit Aryl ausgewählt aus Phenyl, Indenyl, Indanyl, Naphthyl, Anthryl, Phenanthryl, Acenaphthyl und Fluorenyl, das unsubstituiert oder durch Niederalkyl, Halogenniederalkyl, Phenyl, 1- oder 2-Naphthyl, Hydroxy, Niederalkoxy, Carbamoylniederalkoxy, N-Niederalkylcarbamoylniederalkoxy oder N,N-Diniederalkylcarbamoylniederalkoxy, Amino, Mono- oder Diniederalkylamino, Niederalkanoylamino, Halogen, Carboxy, Niederalkoxycarbonyl, Phenyl-, Naphthyl- oder Fluorenylniederalkoxycarbonyl, Niederalkanoyl, Sulfo, Niederalkylsulfonyl, Phosphono, Hydroxy-niederalkoxy-phosphoryl oder Diniederalkoxyphosphoryl, Carbamoyl, Mono- oder Diniederalkylcarbamoyl, Sulfamoyl, Mono- oder Diniederalkylsulfamoyl, Nitro und/oder Cyano ein-oder bis zu dreifach substituiert sein kann, wobei Phenyl bis zu dreimal vorliegen kann, und mit Niederalkyl, welches unsubstituiert oder substituiert ist durch Niederalkyl, Heterocyclyl ausgewählt aus Pyrrolyl, Furyl, Thienyl, Imidazolyl, Pyrazolyl, Oxazolyl, Thiazolyl, Pyridyl, Pyrazinyl, Pyrimidinyl, Indolyl, Chinolyl, Isochinolyl, Chinoxalinyl, β-Carbolinyl oder einem benzanellierten, cyclopenta-, cyclohexa- oder cyclohepta-anellierten Derivat dieser Reste, die auch ganz oder teilweise gesättigt vorliegen können, Hydroxy, Niederalkoxy, Niederalkanoyloxy, Acetoacetoxy, Amino- oder Benzyloxycarbonylamino-niederalkanoyloxy, Arylniederalkanoyloxy, worin Aryl 6 - 10 Kohlenstoffatome hat, Niederalkoxycarbonyloxy, Mono- oder Diniederalkyl-aminocarbonyloxy, Aryloxycarbonyloxy, worin Aryl 6 - 10 Kohlenstoffatome hat, Arylniederalkoxycarbonyloxy mit Aryl von 6 - 12 Kohlenstoffatomen, Sulfonyloxy, Niederalkylsulfonyloxy, Phenylsulfonyloxy, 2- oder 4-Toluolsulfonyloxy oder 1- oder 2- Naphthylsulfonyloxy, Amino, Mono-oder Diniederalkylamino, N-Niederalkoxy-N-niederalkylamino, Mono- oder Di-(Phenyl- oder Naphthylniederalkyl)amino, Niederalkanoylamino, Carboxy, verestertes Carboxy ausgewählt aus Niederalkoxycarbonyl, Aryloxycarbonyl, worin Aryl 6 - 10 Kohlenstoffatome hat, Arylniederalkoxycarbonyl, worin Aryl 6 - 12 Kohlenstoffatome hat, Niederalkanoyl, Niederalkylsulfonyl, Hydroxy-niederalkoxyphosphoryl und Diniederalkoxyphosphoryl, Carbamoyl, durch einen oder zwei aus Niederalkyl, aus Carboxyniederalkyl oder Niederalkoxycarbonyl-niederalkyl, aus Diniederalkylaminoniederalkyl, aus Aminocarboxyniederalkyl, aus Hydroxyniederalkyl, und aus Diniederalkoxyniederalkyl ausgewählte Reste oder einen aus Ethylen, Trimethylen, Tetramethylen und Pentamethylen ausgewählten Rest, worin ein Kohlenstoffatom durch Stickstoff, Sauerstoff, Schwefel oder mit Sauerstoff ein-oder zweifach substituierten Schwefel ersetzt sein kann und das ungesättigt sein kann, substituiertes Carbamoyl, ferner Sulfamoyl, Phosphono, Benzofuranyl, Oxo und/oder Cyano und unverzweigt oder verzweigt ist,
R₄ Wasserstoff bedeutet,
R₅ Hydroxy bedeutet und
R₆ Wasserstoff bedeutet,
oder R₅ und R₆ zusammen Oxo bedeuten, und
R₇
- Niederalkyl,
- Cycloalkylniederalkyl, worin Cycloalkyl 3 - 7 Kohlenstoffatome hat und unsubstituiert oder durch Niederalkyl, Halogenniederalkyl, Hydroxy, Niederalkoxy, Carbamoylniederalkoxy, N-Niederalkylcarbamoylniederalkoxy oder N,N-Diniederaikylcarbamoylniederalkoxy, Amino, Mono- oder Diniederalkylamino, Halogen, Carboxy, Niederalkoxycarbonyl, Phenyl-, Naphthyl- oder Fluorenylniederalkoxycarbonyl, Niederalkanoyl, Niederalkylsulfonyl, Phosphono, Hydroxy-niederalkoxy-phosphoryl oder Diniederalkoxyphosphoryl, Carbamoyl, Mono- oder Diniederalkylaminocarbamoyl, Sulfamoyl, Mono-oder Diniederalkylsulfamoyl, Nitro und/oder Cyano ein- oder bis zu dreifach substituiert ist und an Niederalkyl gebunden ist,
- Bicycloalkylniederalkyl, worin Bicylcoalkyl 5 - 10, Kohlenstoffatome enthält,
- Tricycloalkylniederalkyl, worin Tricycloalkyl 8 - 10 Kohlenstoffatome enthält,
- Arylniederalkyl mit Aryl ausgewählt aus Phenyl, Indenyl, Indanyl, Naphthyl, Anthryl, Phenanthryl, Acenaphthyl und Fluorenyl, das unsubstituiert oder durch Niederalkyl, Halogenniederalkyl, Phenyl, 1- oder 2-Naphthyl, Hydroxy, Niederalkoxy, Carbamoylniederalkoxy, N-Niederalkylcarbamoylniederalkoxy, N,N-Diniederalkylcarbamoylniederalkoxy, Amino, Mono- oder Diniederalkylamino, Niederalkanoylamino, Halogen, Carboxy, Niederalkoxycarbonyl, Benzyl-, Naphthyl- oder Fluorenylniederalkoxycarbonyl, Niederalkanoyl, Sulfo, Niederalkylsulfonyl, Phosphono, Hydroxy-niederalkoxy-phosphoryl, Diniederalkoxyphosphoryl, Carbamoyl, Mono- oder Diniederalkylcarbamoyl, Sulfamoyl, Mono- oder Diniederalkylsulfamoyl, Nitro und/oder Cyano ein-oder bis zu dreifach substituiert sein kann, wobei Phenyl bis zu dreimal vorliegen kann, und mit Niederalkyl, welches unsubstituiert oder substituiert ist durch Niederalkyl, Heterocyclyl ausgewählt aus Pyrrolyl, Furyl, Thienyl, Imidazolyl, Pyrazolyl, Oxazolyl, Thiazolyl, Pyridyl, Pyrazinyl, Pyrimidinyl, Indolyl, Chinolyl, Isochinolyl, Chinoxalinyl, β-Carbolinyl und einem benzanellierten, cyclopenta-, cyclohexa- oder cyclohepta-anellierten Derivat dieser Reste, die auch ganz oder teilweise gesättigt vorliegen können, Hydroxy, Niederalkoxy, Niederalkanoyloxy, Acetoacetoxy, Amino- oder Benzyloxycarbonylamino-niederalkanoyloxy, Arylniederalkanoyloxy, worin Aryl 6 - 10 Kohlenstoffatome hat, Niederalkoxycarbonyloxy, Mono- oder Diniederalkyl-aminocarbonyloxy, Aryloxycarbonyloxy, worin Aryl 6 - 10 Kohlenstoffatome hat, Arylniederalkoxycarbonyloxy mit Aryl von 6 - 12 Kohlenstoffatomen, Sulfonyloxy, Niederalkylsulfonyloxy, Phenylsulfonyloxy, 2-oder 4-Toluolsulfonyloxy, 1- oder 2- Naphthylsulfonyloxy, Amino, Mono- oder Diniederalkylamino, N-Niederalkoxy-N-niederalkylamino, Mono- oder Di-(Phenyl- oder Naphthylniederalkyl)amino, Niederalkanoylamino, Carboxy, verestertes Carboxy ausgewählt aus Niederalkoxycarbonyl, Aryloxycarbonyl, worin Aryl 6 - 10 Kohlenstoffatome hat, Arylniederalkoxycarbonyl, worin Aryl 6 - 12 Kohlenstoffatome hat, Niederalkanoyl, Niederalkylsulfonyl, Hydroxy-niederalkoxyphosphoryl und Diniederalkoxyphosphoryl, Carbamoyl, durch einen oder zwei aus Niederalkyl, aus Carboxyniederalkyl, aus Niederalkoxycarbonyl-niederalkyl, aus Diniederalkylaminoniederalkyl, aus Aminocarboxyniederalkyl, aus Hydroxyniederalkyl und aus Diniederalkoxyniederalkyl ausgewählte Reste oder einen aus Ethylen, Trimethylen, Tetramethylen und Pentamethylen ausgewählten Rest, worin ein Kohlenstoffatom durch Stickstoff, Sauerstoff, Schwefel oder mit Sauerstoff ein- oder zweifach substituierten Schwefel ersetzt sein kann und das ungesättigt sein kann, substituiertes Carbamoyl, ferner Sulfamoyl, Phosphono, Benzfuranyl, Oxo (welches nicht an das Kohlenstoffatom gebunden ist, das mit dem den Rest R₇ bindenden Stickstoffatom verknüpft ist) und/oder Cyano und unverzweigt oder verzweigt ist, - Heterocyclylniederalkyl, worin Heterocyclyl vorzugsweise aus Pyrrolyl, Furyl, Thienyl, Imidazolyl, Pyrazolyl, Oxazolyl, Thiazolyl, Pyridyl, Pyrazinyl, Pyrimidinyl, Indolyl, Chinolyl, Isochinolyl, Chinoxalinyl, β-Carbolinyl und einem benzanellierten, cyclopenta-, cyclohexa- oder cyclohepta-anellierten Derivat dieser Reste, die auch ganz oder teilweise gesättigt sein können, ausgewählt ist, z.B. 4-Pyrrolidinylmethyl, 1-Imidazolylmethyl, 2-Pyridylmethyl, 3-Pyridylmethyl, Chinolin-2-ylmethyl oder Indol-2-ylmethyl,
- Hydroxyniederalkyl,
- Niederalkoxyniederalkyl,
- Phenoxyniederalkyl oder Nitrophenoxyniederalkyl,
- Naphthoxyniederalkyl,
- Niederalkanoyloxyniederalkyl,
- Acetoacetoxyniederalkyl,
- Arylmercaptoniederalkyl, worin Aryl 6 - 10 Kohlenstoffatome hat,
- Aminoniederalkyl,
- Mono- oder Diniederalkylaminoniederalkyl,
- Phenyl- oder Naphthylaminoniederalkyl,
- Niederalkanoylamino-niederalkyl,
- durch Niederalkyl am Stickstoff substituiertes Piperazinylcarbonylniederalkyl,
- Niederaikoxycarbonylamino-niederalkyl,
- Phenylniederalkoxycarbonylamino-niederalkyl,
- Aminocarbonylamino-niederalkyl,
- N-Phenylniederalkyl-N-niederalkylaminocarbonylamino-niederalkyl,
- Halogenniederalkyl,
- Carboxyniederalkyl,
- Niederalkoxycarbonylniederalkyl,
- 2-Halogenniederalkoxycarbonyl-niederalkyl,
- Phenyl- oder Naphthylniederalkyloxycarbonyl-niederalkyl,
- Heterocyclylniederalkoxycarbonyl-niederalkyl, worin Heterocyclyl ausgewählt ist aus Pyrrolyl, Furyl, Thienyl, Imidazolyl, Pyrazolyl, Oxazolyl, Thiazolyl, Pyridyl, Pyrazinyl, Pyrimidinyl, Indolyl, Chinolyl, Isochinolyl, Chinoxalinyl, β-Carbolinyl und einem benzanellierten, cyclopenta-, cyclohexa- oder cyclohepta-anellierten Derivat dieser Reste, wobei die genannten Reste auch ganz oder teilweise gesättigt vorliegen können,
- Niederalkylsulfonylniederalkyl,
- Arylsulfonylniederalkyl, worin Aryl 6 - 10 Kohlenstoffatome hat,
- Carbamoylniederalkyl,
- Niederalkylcarbamoylniederalkyl,
- Diniederalkylcarbamoylniederalkyl,
- Hydroxyniederalkylcarbamoyl- oder Di-(hydroxyniederalkyl)carbamoyl-niederalkyl,
- N-Niederalkoxyniederalkoxyniederalkyl-carbamoyl-niederalkyl,
- Carboxyniederalkylcarbamoyl- oder Di-(carboxyniederalkyl)carbamoyl-niederalkyl,
- durch einen aus Ethylen, Trimethylen, Tetramethylen und Pentamethylen ausgewählten Rest, worin ein Kohlenstoffatom durch Stickstoff, Sauerstoff, Schwefel oder mit Sauerstoff ein- oder zweifach substituierten Schwefel ersetzt sein kann, am Stickstoff substituiertes Carbamoylniederalkyl, wobei der so gebildete Rest auch ganz oder teilweise ungesättigt sein kann,
- N-Niederalkyl-N-heterocyclylniederalkylcarbamoylniederalkyl, worin Heterocyclyl aus Pyrrolyl, Furyl, Thienyl, Imidazolyl, Pyrazolyl, Oxazolyl, Thiazolyl, Pyridyl, Pyrazinyl, Pyrimidinyl, Indolyl, Chinolyl, Isochinolyl, Chinoxalinyl, β-Carbolinyl und einem benzanellierten, cyclopenta-, cyclohexa- oder cyclohepta-anellierten Derivat dieser Reste, die auch ganz oder teilweise gesättigt sein können, ausgewählt ist,
- Sulfamoylniederalkyl,
- N-(Phenyl- oder Naphthylniederalkyl)sulfamoylniederalkyl,
- am Stickstoffatom von einem aus Ethylen, Trimethylen, Tetramethylen und Pentamethylen ausgewählten Rest, worin ein Kohlenstoffatom durch Stickstoff, mit Niederalkyl substituierten Stickstoff, Sauerstoff, Schwefel oder mit Sauerstoff ein-oder zweifach substituierten Schwefel ersetzt sein kann, substituiertes Sulfamoylniederalkyl, wobei der so gebildete Rest auch ganz oder teilweise ungesättigt sein kann,
- Oxoniederalkyl (wobei Oxo nicht an das Kohlenstoffatom gebunden ist, welches mit dem R₇ bindenden Stickstoffatom verknüpft ist),
- Cyanoniederalkyl,
- Hydroxy-carboxy-niederalkyl,
- α-Naphthoxy-carboxy-niederalkyl,
- Hydroxy-niederalkoxycarbonyl-niederalkyl,
- α-Naphthoxy-niederalkoxycarbonylniederalkyl,
- Niederalkylcarbonyl-halogen-niederalkyl,
- α-Naphthoxyethoxycarbonyl-niederalkyl,
- α-Naphthoxy-benzyloxycarbonyl-niederalkyl,
- verestertes Hydroxy-niederalkoxycarbonyl-niederalkyl, wobei die Hydroxygruppe durch Niederalkanoyl, Cycloalkylniederalkanoyl, worin Cycloalkyl 3 - 7 Kohlenstoffatome hat, Bicycloalkylniederalkanoyl, worin Bicylcoalkyl 5 - 10 Kohlenstoffatome enthält, Tricycloalkylniederalkanoyl, worin Tricycloalkyl 8 - 10 Kohlenstoffatome enthält, Arylniederalkanoyl, worin Aryl 6 - 14 Kohlenstoffatome hat und unsubstituiert oder durch Niederalkyl, Hydroxy, Niederalkoxy, Carbamoylniederalkoxy, N-Niederalkylcarbamoylniederalkoxy oder N,N-Diniederalkylcarbamoylniederalkoxy, Amino, Mono- oder Diniederalkylamino, Halogen, Carboxy, Niederalkoxycarbonyl, Phenyl-, Naphthyl- oder Fluorenylniederalkoxycarbonyl, Niederalkanoyl, Niederalkylsulfonyl, Phosphono, Hydroxyniederalkoxy-phosphoryl, Diniederalkoxyphosphoryl, Carbamoyl, Sulfamoyl, Nitro und/oder Cyano ein- oder bis zu dreifach substituiert sein kann, Niederalkoxycarbonyl, 2-Halogenniederalkoxycarbonyl, oder Phenyl- oder Fluorenyl-niederalkoxycarbonyl, verestert ist,
- Dihydroxy-carboxy-niederalkyl,
- Dihydroxy-niederalkoxycarbonyl-niederalkyl,
- durch Niederalkanoyl, Niederalkoxycarbonyl, Phenyl- oder Fluorenyl-niederalkoxycarbonyl,- Niederalkylsulfonyl oder Toluolsulfonyl verestertes Dihydroxy-niederalkoxycarbonyl-niederalkyl,
- α-Naphthoxy-diniederalkylamino-niederalkyl,
- α-Naphthoxy-carbamoyl-niederalkyl,
- α-Naphthoxy-oxo-niederalkyl (wobei Oxo nicht an das Kohlenstoffatom gebunden ist, welches mit dem R₇ bindenden Stickstoffatom verknüpft ist), oder
- α-Naphthoxy-cyanoniederalkyl bedeutet,
sowie Salzen der genannten Verbindungen, sofern salzbildende Gruppen vorliegen, wobei in den oben genannten Definitionen der Ausdruck "Nieder" oder "nieder" bedeutet, dass der jeweilige Rest bis zu einschliesslich 7 Kohlenstoffatome hat; dadurch gekennzeichnet, dass man entsprechend substituierte Ausgangsmaterialien verwendet.

5. Verfahren gemäss Anspruch 1 zur Herstellung einer Verbindung der Formel I, worin R₁ Niederalkoxycarbonyl, Phenylniederalkoxycarbonyl, das über die Carboxygruppe gebundene monovalente Radikal einer aliphatischen Aminosäure ausgewählt aus Valin, Alanin, Leucin und Isoleucin oder das über die Carboxygruppe gebundene Radikal einer am Aminostickstoff durch einen der Reste Phenylniederalkanoyl, Morpholinylniederalkanoyl, Thiomorpholinylniederalkanoyl, S,S-Dioxothiomorpholinylniederalkanoyl, Pyridylniederalkanoyl, Niederalkoxycarbonyl oder Phenylniederalkoxycarbonyl acylierten, wie oben definierten aliphatischen Aminosäure, wobei alle genannten Aminosäuren in der D-, D,L- oder L-Form vorliegen, bedeutet, R₂ Wasserstoff, R₃ Phenylniederalkyl, R₄ Wasserstoff, R₅ Hydroxy, R₆ Wasserstoff, R₇ Niederalkyl, Cyclohexylniederalkyl oder Phenylniederalkyl, R₈ Wasserstoff und R₉ einen der für R₁ genannten Reste bedeuten und die die Reste R₃ und R₅ tragenden asymmetrischen Kohlenstoffatome in der S-Konfiguration vorliegen, sowie pharmakologisch verwendbaren Salzen dieser Verbindungen, wobei in den oben genannten Definitionen der Ausdruck "Nieder" oder "nieder" bedeutet, dass derjeweilige Rest bis zu einschliesslich 7 Kohlenstoffatome hat; dadurch gekennzeichnet, dass man entsprechend substituierte Ausgangsmaterialien verwendet.

6. Verfahren gemäss Anspruch 1 zur Herstellung einer Verbindung der Formel I, worin R₁ tert-Butoxycarbonyl, Benzyloxycarbonyl, das über die Carboxygruppe gebundene monovalente Radikal der Aminosäure Valin oder das über die Carboxygruppe gebundene Radikal des am Aminostickstoff durch einen der Reste Phenylacetyl, 3-Pyridylacetyl, Morpholinocarbonyl, Thiomorpholinocarbonyl, tert-Butoxycarbonyl oder Benzyloxycarbonyl acylierten Alanins bedeutet, R₂ Wasserstoff, R₃ Benzyl, R₄ Wasserstoff, R₅ Hydroxy, R₆ Wasserstoff, R₇ Isobutyl, Cyclohexylmethyl oder Benzyl, R₈ Wasserstoff und R₉ einen der für R₁ genannten Reste bedeuten und die die Reste R₃ und R₅ tragenden asymmetrischen Kohlenstoffatome in der S-Konfiguration vorliegen, sowie pharmakologisch verwendbaren Salzen dieser Verbindungen; dadurch gekennzeichnet, dass man entsprechend substituierte Ausgangsmaterialien verwendet.

7. Verfahren gemäss Anspruch 1 zur Herstellung einer Verbindung der Formel I, worin R₁ und R₉ jeweils das über die Carboxygruppe gebundene, am Aminostickstoff durch Benzyloxycarbonyl acylierte monovalente Radikal der Aminosäure (L)-Valin bedeuten, R₂ und R₈ Wasserstoff bedeuten, R₃ Benzyl bedeutet, R₄ Wasserstoff bedeutet, R₅ Hydroxy bedeutet, R₆ Wasserstoff bedeutet und R₇ Benzyl bedeutet und die die Reste R₃ und R₅ tragenden asymmetrischen Kohlenstoffatome in der S-Konfiguration vorliegen, sowie pharmakologisch verwendbaren Salzen dieser Verbindung; dadurch gekennzeichnet, dass man entsprechend substituierte Ausgangsmaterialien verwendet.

8. Verfahren gemäss Anspruch 1 zur Herstellung einer Verbindung der Formel I, worin R₁ und R₉ jeweils das über die Carboxygruppe gebundene, am Aminostickstoff durch 4-Thiomorpholinocarbonyl acylierte monovalente Radikal der Aminosäure (L)-Valin bedeuten, R₂ und R₈ Wasserstoff bedeuten, R₃ Benzyl bedeutet, R₄ Wasserstoff bedeutet, R₅ Hydroxy bedeutet, R₆ Wasserstoff bedeutet und R₇ Isobutyl bedeutet und die die Reste R₃ und R₅ tragenden asymmetrischen Kohlenstoffatome in der S-Konfiguration vorliegen, sowie pharmakologisch verwendbaren Salzen dieser Verbindung; dadurch gekennzeichnet, dass man entsprechend substituierte Ausgangsmaterialien verwendet.

9. Verfahren gemäss Anspruch 1 zur Herstellung einer Verbindung der Formel I, ausgewählt aus den Verbindungen mit den Bezeichnungen
Boc-[Phe^{NN}Phe]-Boc,
Boc-(L)-Val-[Phe^{NN}Phe]←(L)-Val-Boc),
Boc-[Phe^{NN}Cha]-Boc,
H-(L)-Val-[Phe^{NN}Phe]←(L)-Val)-H,
N-Thiomorpholinocarbonyl-(L)-Val-[Phe^{NN}Phe]←(N-thiomorpholinocarbonyl-(L)-Val),
N-Morpholinocarbonyl-(L)-Val-[Phe^{NN}Phe]←(N-morpholinocarbonyl-(L)-Val),
Phenylacetyl-(L)-Val-[Phe^{NN}Phe]←(N-phenylacetyl-(L)-Val),
N-(3-Pyridylacetyl)-(L)-Val-[Phe^{NN}Phe]←(N-(3-pyridylacetyl)-(L)-Val),
Boc-(L)-Val-[Phe^{NN}Cha]←((L)-Val)-Boc,
Z-(L)-Val-[Phe^{NN}Cha]←((L)-Val)-Z,
Boc-[Phe^{NN}Leu]-Boc
Z-(L)-Val-[Phe^{NN}Leu]←(L)-Val)-Z, H-(L)-Val-[Phe^{NN}Cha]←((L)-Val)-H und
N-(3-Pyridylacetyl)-(L)-Val-[Phe^{NN}Leu]←(N-(3-pyridylacetyl)-(L)-Val), oder von einem Salz davon;
worin Boc für tert-Butoxycarbonyl steht, Z für Benzyloxycarbonyl steht, der Rest -[Phe^{NN}Phe]- das zweiwertige Radikal von 3(S)-Amino-4-phenyl-1-(N-benzylhydrazino)-butan-2(S)-ol bedeutet und die Formel 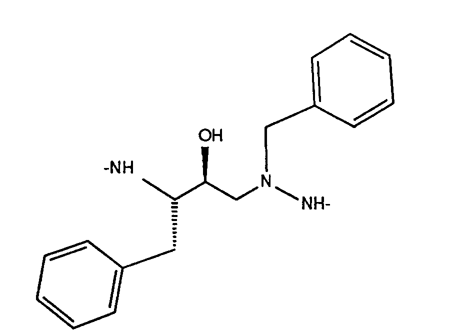 hat, der Rest -[Phe^{NN}Cha] das zweiwertige Radikal von 3(S)-Amino-4-phenyl-1-(N-cyclohexylmethylhydrazino)-butan-2(S)-ol bedeutet und die Formel 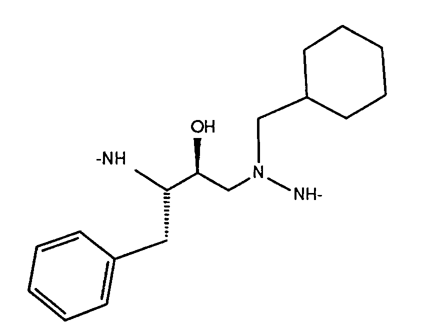 hat, der Rest -[Phe^{NN}Leu]- das zweiwertige Radikal von 3(S)-Amino-4-phenyl-1-(N-isobutylhydrazino)-butan-2(S)-ol bedeutet und die Formel 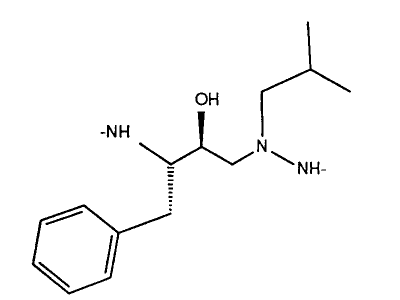 hat, und ein Pfeil "←" die Umkehrung der Bindungsrichtung in Abweichung von der üblichen Peptidnomenklatur, worin links der Amino- und rechts der Carboxyterminus vorliegen, bedeutet; dadurch gekennzeichnet, dass man entsprechend substituierte Ausgangsmaterialien verwendet.

10. Verfahren gemäss Anspruch 1 zur Herstellung einer Verbindung der Formel I, ausgewählt aus den Verbindungen mit den Bezeichnungen
Z-(L)-Val-[(p-F)Phe^{NN}(p-F)Phe]←(N-(N-(2-pyridylmethyl)-N-methylaminocarbonyl)(L)-Val),
Z-(L)-Val-[(p-F)Phe^{NN}(p-F)Phe]←(N-(2(R,S)-carbamoyl-3-phenyl-propionyl)(L)-Val),
Acetyl-(L)-Val-[Phe^{NN}Cha]←(N-acetyl-(L)-Val),
Acetyl-Ile-[Phe^{NN}Cha]←(N-acetyl-Ile),
N-(2-Pyridylmethyl)-N-methyl-aminocarbonyl(L)-Val-[Phe^{NN}(p-F)-Phe]←(N-(N-(2-pyridylmethyl)-N-methyl-aminocarbonyl)-(L)-Val),
Z-(L)-Val-[Phe^{NN}(p-F)Phe]←((L)-Val)-Z,
Z-(L)-Val-[Phe^{NN}(p-CN)Phe]←((L)-Val)-Z,
Z-(L)-Ile-[Phe^{NN}Leu]←((L)-Ile)-Z,
Isobutoxycarbonyl-(L)-Val[Phe^{NN}Leu]←(N-isobutoxycarbonyl-(L)-Val),
Acetyl-Val-[Phe^{NN}Leu]←(N-(2(R,S)-carbamoyl-3-phenyl-propionyl)-Val),
N-Trifluoracetyl-[Phe^{NN}Leu]←(N-(2(R,S)-carbamoyl-3-phenyl-propionyl)-(L)-Val),
Z-(L)-Val-[Phe^{NN}Nle]←(N-(2(R,S)-(N-(2-morpholinoethyl)carbamoyl)-3-methyl)-butyryl),
Z-(L)-Val-[Phe^{NN}Nle]←(N-(2(R,S)-(N-(2-pyridylmethyl)-carbamoyl)-3-methyl)-butyryl),
Methoxycarbonyl-(L)-Val-[Phe^{NN}Leu]←(N-methoxycarbonyl-(L)-Val),
Methoxycarbonyl-(L)-Val[Phe^{NN}(p-F)Phe]←(N-methoxycarbonyl-(L)-Val) und
Methoxycarbonyl-(L)-Val [Phe^{NN}(p-CN)Phe]←(N-methoxycarbonyl-(L)-Val),
oder von einem Salz davon, worin worin Boc für tert-Butoxycarbonyl steht, Z für Benzyloxycarbonyl steht, der Rest -[Phe^{NN}Phe]- das zweiwertige Radikal von 3(S)-Amino-4-phenyl-1-(N-benzylhydrazino)-butan-2(S)-ol bedeutet und die Formel 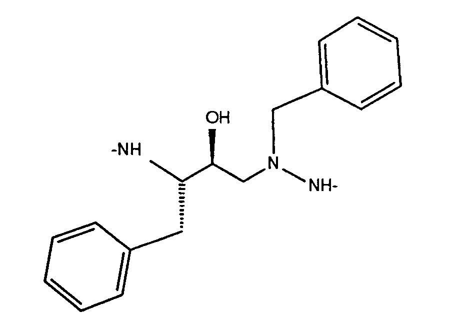 hat, der Rest -[Phe^{NN}Cha] das zweiwertige Radikal von 3(S)-Amino-4-phenyl-1-(N-cyclohexylmethyihydrazino)-butan-2(S)-ol bedeutet und die Formel 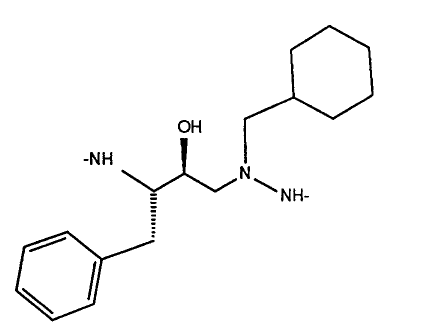 hat, der Rest -[Phe^{NN}Leu] das zweiwertige Radikal von 3(S)-Amino-4-phenyl-1-(N-isobutylhydrazino)-butan-2(S)-ol bedeutet und die Formel 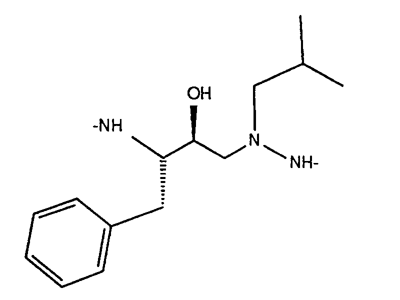 hat, der Rest mit der Bezeichnung -[Phe^{NN}Nle] das Radikal von 3(S)-Amino-4-phenyl-1-(N-n-butylhydrazino)-butan-2(S)-ol bedeutet und die Formel 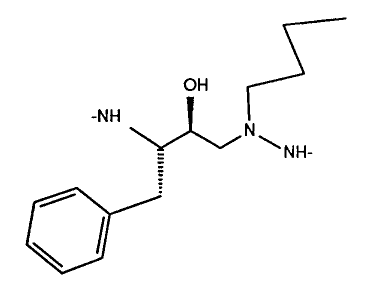 hat, der Rest mit der Bezeichnung -[Phe^{NN}(p-F)Phe] das zweiwertige Radikal von 3(S)-Amino-4-phenyl-1-(N-(p-fluorphenylmethyl)-hydrazino-butan-2(S)-ol bedeutet und die Formel 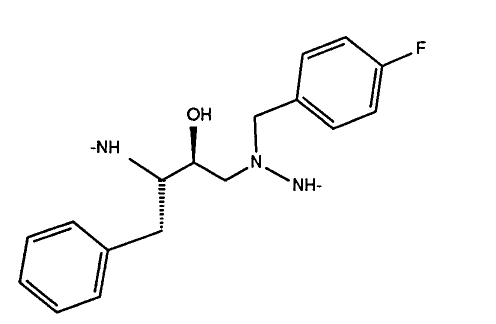 hat, der Rest mit der Bezeichnung -[(p-F)Phe^{NN}(p-F)Phe] das zweiwertige Radikal von 3(S)-Amino-4-(p-fluorphenyl)-1-(N-(p-fluorphenylmethyl)-hydrazino)-butan-2(S)-ol bedeutet und die Formel 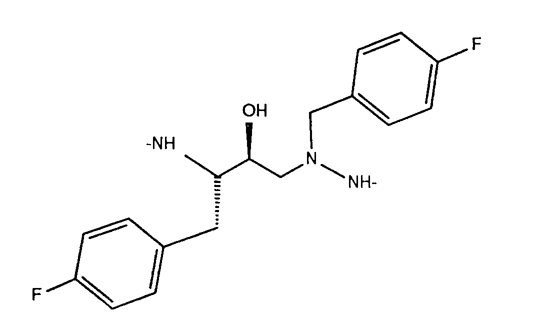 hat, der Rest mit der Bezeichnung -[Phe^{NN}(p-CN)Phe] das zweiwertige Radikal von 3(S)-Amino-4-phenyl-1-(N-(p-cyanophenylmethyl)-hydrazino)-butan-2(S)-ol bedeutet und die Formel 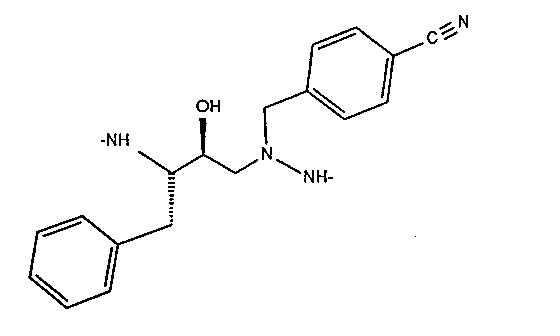 hat,
und ein Pfeil "←" die Umkehrung der Bindungsrichtung in Abweichung von der üblichen Peptidnomenklatur, worin links der Amino- und rechts der Carboxyterminus vorliegen, bedeutet; dadurch gekennzeichnet, dass man entsprechend substituierte Ausgangsmaterialien verwendet.

## Claims (Claims for the following Contracting State(s): AT, BE, CH, DE, DK, FR, GB, IT, LI, LU, MC, NL, PT, SE)

1. A compound of formula 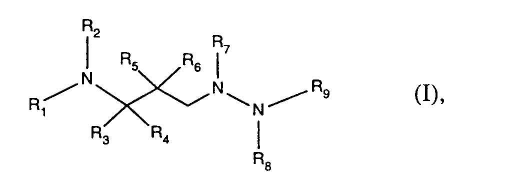 wherein R₁ and R₉ are each independently of the other hydrogen; acyl; unsubstituted or substituted alkyl, alkenyl or alkynyl; heterocyclyl; sulfo; sulfonyl substituted by unsubstituted or substituted alkyl, aryl, alkoxy, which is unsubstituted or substituted, or by aryloxy; sulfamoyl that is unsubstituted or substituted at the nitrogen atom; or phosphoryl substituted by one or two radicals selected independently of one another from unsubstituted or substituted alkyl, from unsubstituted or substituted cycloalkyl, from aryl, from hydroxy, from unsubstituted or substituted alkoxy, from cycloalkoxy and from aryloxy; with the proviso that not more than one of the radicals R₁ and R₉ is hydrogen; and R₂ and R₈ are each independently of the other hydrogen or one of the radicals mentioned above for R₁ and R₉; or the pairs of substituents R₁ and R₂, and R₈ and R₉, each independently of the other, may form, together with the nitrogen atom to which they are bonded, a heterocyclic ring consisting of the bonding nitrogen atom together with a radical selected from ethylene, trimethylene, tetramethylene and pentamethylene, in which a carbon atom may have been replaced by nitrogen, oxygen, sulfur or by sulfur mono-or di-substituted by oxygen and which may be unsaturated, or naphthalene-1,8-dicarbonylimido;
R₃ and R₄ are each independently of the other hydrogen; unsubstituted or substituted alkyl or cycloalkyl; aryl; heterocyclyl; or unsubstituted or substituted alkenyl; or R₃ and
R₄ together form unsubstituted or substituted alkylene, alkylidene or benzo-fused alkylene;
R₅ is hydroxy; R₆ is hydrogen;
or R₅ and R₆ together are oxo;
and R₇ is unsubstituted or substituted alkyl or cycloalkyl; aryl; heterocyclyl; or unsubstituted or substituted alkenyl;
or a salt of such a compound where salt-forming groups are present;
with the exception of the compound of formula I wherein R₁, R₂, R₃, R₄, R₆ and R₈ are each hydrogen, R₅ is hydroxy, R₇ is a radical of the formula -[CH₂-(C=O)-NH-(N-CH₃)-CH₂-COOH, and R₉ is tert-butoxycarbonyl.

2. A compound of formula I according to claim 1, wherein
R₁ and R₉ are each independently of the other hydrogen; lower alkanoyl; aryl-lower alkanoyl wherein lower alkanoyl is unsubstituted or substituted by carbamoyl or by carbamoyl substituted at the nitrogen atom by one or two radicals selected from lower alkyl, carboxy-lower alkyl, lower alkoxycarbonyl-lower alkyl, di-lower alkylamino-lower alkyl, amino-carboxy-lower alkyl, hydroxy-lower alkyl and di-lower alkoxy-lower alkyl; heterocyclyl-lower alkanoyl; halo-lower alkanoyl containing up to 3 halogen atoms; (N-heterocyclyl-lower alkylcarbamoyl)-lower alkanoyl; lower alkoxycarbonyl; aryl-lower alkoxycarbonyl; heterocyclyl-lower alkoxycarbonyl; lower alkylsulfonyl; N-heterocyclyl-lower alkyl-N-lower alkylcarbamoyl; or an acyl residue of an amino acid the amino function of which is free or has been acylated by one of the other radicals mentioned hitherto for R₁ and R₉; with the proviso that not more than one of the radicals R₁ and R₉ is hydrogen;
R₂, R₄, R₆ and R₈ are hydrogen;
R₃ is lower alkyl, cycloalkyl-lower alkyl or aryl-lower alkyl,
R₅ is hydroxy, and
R₇ is unsubstituted or substituted lower alkyl,
or a salt thereof where at least one salt-forming group is present, the term lower" in the above definitions indicating that the radical in question has up to and including 7 carbon atoms.

3. A compound of formula I according to claim 1, wherein
R₁ and R₉ are each independently of the other hydrogen; lower alkanoyl; aryl-lower alkanoyl wherein aryl is phenyl, indenyl, indanyl, naphthyl, anthryl, phenanthryl, acenaphthyl or fluorenyl and may be unsubstituted or mono- to tri-substituted by lower alkyl, halo-lower alkyl, phenyl, 1- or 2-naphthyl, hydroxy, lower alkoxy, carbamoyl-lower alkoxy, N-lower alkylcarbamoyl-lower alkoxy, N,N-di-lower alkylcarbamoyl-lower alkoxy, amino, mono- or di-lower alkylamino, lower alkanoylamino, halogen, carboxy, lower alkoxycarbonyl, phenyl-, naphthyl- or fluorenyl-lower alkoxycarbonyl, lower alkanoyl, sulfo, lower alkylsulfonyl, phosphono, hydroxy-lower alkoxyphosphoryl, di-lower alkoxyphosphoryl, carbamoyl, mono- or di-lower alkylcarbamoyl, sulfamoyl, mono-or di-lower alkylaminosulfonyl, nitro and/or by cyano, wherein phenyl may be present up to three times, and wherein lower alkanoyl is unsubstituted or substituted by carbamoyl or by carbamoyl substituted at the nitrogen atom by one or two radicals selected from lower alkyl, carboxy-lower alkyl, lower alkoxycarbonyl-lower alkyl, di-lower alkylamino-lower alkyl, amino-carboxy-lower alkyl, hydroxy-lower alkyl and di-lower alkoxy-lower alkyl; heterocyclyl-lower alkanoyl wherein lower alkanoyl is unsubstituted and wherein heterocyclyl is selected from thienyl, furyl, pyranyl, pyrrolyl, imidazolyl, pyrazolyl, oxazolyl, isoxazolyl, thiazolyl, furazanyl, pyridyl, pyrazinyl, pyrimidinyl, pyridazinyl, azepinyl, indolyl, benzimidazolyl, 1H-indazolyl, quinolyl, isoquinolyl, quinoxalinyl, quinazolinyl, cinnolyl, purinyl, pteridinyl, naphthyridinyl, 4H-quinolizinyl, 3,1-benzofuranyl, 4,1-benzoxazinyl, 4,1-benzothiazinyl, cyclohexa[b]pyrrolyl, cyclohepta[b]pyrrolyl, cyclohexa[d]pyrazolyl, cyclohexa[b]pyridyl, cyclohexa[b]pyrazinyl, cyclohexa[b]pyrimidinyl, cyclohexa[b]-1,4-oxazinyl, cyclohexa[b]-1,4-thiazinyl, pyrrolidinyl, pyrrolinyl, imidazolidyl, 2-imidazolinyl, 2,3-dihydropyridyl, piperidyl, piperazinyl, 2,3,5,6-tetrahydropyrazinyl, morpholinyl, thiomorpholinyl, S,S-dioxo-thiomorpholinyl, indolinyl, isoindolinyl, 4,5,6,7-tetrahydroindolyl, 1,2,3,4-tetrahydroquinolyl, 1,2,3,4-tetrahydroisoquinolyl, chromanyl, thiochromanyl, 1,2,3,4-tetrahydro-3,1-benzodiazinyl, 3,4-dihydro-3H-4,1-benzoxazinyl and 3,4-dihydro-3H-4,1-benzothiazinyl, the mentioned heterocyclyl radicals being unsubstituted or substituted by lower alkyl, phenyl, 1-or 2-naphthyl, phenyl-lower alkyl, hydroxy-lower alkyl, lower alkoxy-lower alkyl, phenoxy-or naphthoxy-lower alkyl, phenyl-lower alkoxy- or naphthyl-lower alkoxy-lower alkyl, lower alkanoyloxy-lower alkyl, phenyl- or naphthyl-lower alkanoyloxy-lower alkyl, lower alkoxycarbonyloxy-lower alkyl, phenyl-, naphthyl- or fluorenyl-lower alkoxycarbonyloxy-lower alkyl, amino-lower alkyl, carboxy-lower alkyl, hydroxy, lower alkoxy, phenyl-or naphthyl-lower alkoxy, amino, lower alkylamino, di-lower alkylamino, carboxy, lower alkoxycarbonyl, phenyl- or naphthyl-lower alkoxycarbonyl, halogen, lower alkanoyl, lower alkylsulfonyl, phosphono, hydroxy-lower alkoxyphosphoryl or dialkoxyphosphoryl, carbamoyl, mono- or di-lower alkylcarbamoyl, hydroxy- or carboxy-lower alkylcarbamoyl, sulfamoyl, nitro, oxo and/or by cyano; heterocyclyl-lower alkanoyl, such as halo-lower alkanoyl containing up to three halogen atoms; (N-heterocyclyl-lower alkylcarbamoyl)-lower alkanoyl wherein heterocyclyl is selected from pyrrolyl, furyl, thienyl, imidazolyl, pyrazolyl, oxazolyl, thiazolyl, pyridyl, pyrazinyl, pyrimidinyl, indolyl, quinolyl, isoquinolyl, quinoxalinyl, β-carbolinyl and a benzo-fused, cyclopenta-, cyclohexa- or cyclohepta-fused derivative of those radicals, which may also be fully or partially saturated, from morpholine and from thiomorpholine; lower alkoxycarbonyl; aryl-lower alkoxycarbonyl wherein aryl is phenyl, biphenylyl, 1- or 2-naphthyl, fluorenyl, or phenyl mono- or poly-substituted by lower alkyl, hydroxy, lower alkoxy, halogen and/or by nitro; heterocyclyl-lower alkoxycarbonyl wherein heterocyclyl is selected from pyrrolyl, furyl, thienyl, imidazolyl, pyrazolyl, oxazolyl, thiazolyl, pyridyl, pyrazinyl, pyrimidinyl, indolyl, quinolyl, isoquinolyl, quinoxalinyl, β-carbolinyl and a benzo-fused, cyclopenta-, cyclohexa- or cyclohepta-fused derivative of those radicals, which may also be fully or partially saturated; lower alkylsulfonyl; N-heterocyclyl-lower alkyl-N-lower alkylcarbamoyl wherein heterocyclyl is selected from thienyl, furyl, pyranyl, pyrrolyl, imidazolyl, pyrazolyl, oxazolyl, isoxazolyl, thiazolyl, furazanyl, pyridyl, pyrazinyl, pyrimidinyl, pyridazinyl, azepinyl, indolyl, benzimidazolyl, 1H-indazolyl, quinolyl, isoquinolyl, quinoxalinyl, quinazolinyl, cinnolyl, purinyl, pteridinyl, naphthyridinyl, 4H-quinolizinyl, 3,1-benzofuranyl, benz[e]indolyl, 4,1-benzoxazinyl, 4,1-benzothiazinyl, carbazolyl, β-carbolinyl, phenazinyl, phenanthridyl, acridyl, phenoxazinyl, phenothiazinyl, 1-azaacenaphthenyl, cyclohexa[b]pyrrolyl, cyclohepta[b]pyrrolyl, cyclohexa[d]pyrazolyl, cyclohexa[b]pyridyl, cyclohexa[b]pyrazinyl, cyclohexa[b]pyrimidinyl, cyclohexa[b]-1,4-oxazinyl, cyclohexa[b]-1,4-thiazinyl, pyrrolidinyl, pyrrolinyl, imidazolidinyl, 2-imidazolinyl, 2,3-dihydropyridyl, piperidyl, piperazinyl, 2,3,5,6-tetrahydropyrazinyl, morpholinyl, thiomorpholinyl, S,S-dioxothiomopholinyl, indolinyl, isoindolinyl, 4,5,6,7-tetrahydroindolyl, 1,2,3,4-tetrahydroquinolyl, 1,2,3,4-tetrahydroisoquinolyl, chromanyl, thiochromanyl, 1,2,3,4-tetrahydro-3,1-benzodiazinyl, 3,4-dihydro-3H-4,1-benzoxazinyl, 3,4-dihydro-3H-4,1-benzothiazinyl, 2,3,4,5-tetrahydro-1H-5,1-benzazepinyl and 5,6-dihydrophenanthridinyl, the mentioned radicals being unsubstituted or substituted by lower alkyl, phenyl, 1- or 2-naphthyl, phenyl-lower alkyl, hydroxy-lower alkyl, lower alkoxy-lower alkyl, phenoxy- or naphthoxy-lower alkyl, phenyl-lower alkoxy- or naphthyl-lower alkoxy-lower alkyl, lower alkanoyloxy-lower alkyl, phenyl-or naphthyl-lower alkanoyloxy-lower alkyl, lower alkoxycarbonyloxy-lower alkyl, phenyl-, naphthyl- or fluorenyl-lower alkoxycarbonyloxy-lower alkyl, amino-lower alkyl, carboxy-lower alkyl, hydroxy, lower alkoxy, phenyl- or naphthyl-lower alkoxy, amino, lower alkylamino, di-lower alkylamino, carboxy, lower alkoxycarbonyl, phenyl- or naphthyl-lower alkoxycarbonyl, halogen, lower alkanoyl, lower alkylsulfonyl, phosphono, hydroxy-lower alkoxyphosphoryl or dialkoxyphosphoryl, carbamoyl, mono- or di-lower alkylcarbamoyl, hydroxy- or carboxy-lower alkylcarbamoyl, sulfamoyl, nitro, oxo and/or by cyano; or an acyl radical of an amino acid the amino function of which is free or has been acylated by one of the other radicals mentioned hitherto for R₁ and R₉, the amino acids being selected from glycine, alanine, valine, norvaline, leucine, isoleucine, norleucine, serine, homoserine, threonine, methionine, cysteine, proline, trans-3- and trans-4-hydroxyproline, phenylalanine, tyrosine, 4-aminophenylalanine, 4-chlorophenylalanine, 4-carboxyphenylalanine, β-phenylserine, phenylglycine, α-naphthylalanine, cyclohexylalanine, cyclohexylglycine, tryptophan, aspartic acid, asparagine, aminomalonic acid, aminomalonic acid monoamide, glutamic acid, glutamine, histidine, arginine, lysine, δ-hydroxylysine, ornithine, 3-aminopropanoic acid, α,γ-diaminobutyric acid and α,β-diaminopropionic acid, wherein each of the mentioned amino acid residues (with the exception of glycine) is in the D-, L- or (D,L)-form; with the proviso that not more than one of the radicals R₁ and R₉ is hydrogen,
R₂, R₄, R₆ and R₈ are hydrogen,
R₃ is lower alkyl; cycloalkyl-lower alkyl wherein cycloalkyl has from 3 to 7 carbon atoms and is unsubstituted or mono- to tri-substituted by lower alkyl, halo-lower alkyl, hydroxy, lower alkoxy, carbamoyl-lower alkoxy, N-lower alkylcarbamoyl-lower alkoxy, N,N-di-lower alkylcarbamoyl-lower alkoxy, amino, mono- or di-lower alkylamino, halogen, carboxy, lower alkoxycarbonyl, phenyl-, naphthyl- or fluorenyl-lower alkoxycarbonyl, lower alkanoyl, lower alkylsulfonyl, phosphono, hydroxy-lower alkoxyphosphoryl or di-lower alkoxyphosphoryl, carbamoyl, mono- or di-lower alkylaminocarbamoyl, sulfamoyl, mono- or di-lower alkylsulfamoyl, nitro and/or by cyano; or is aryl-lower alkyl wherein aryl is phenyl, indenyl, indanyl, naphthyl, anthryl, phenanthryl, acenaphthyl or fluorenyl and may be unsubstituted or mono- to tri-substituted by lower alkyl, halo-lower alkyl, phenyl, 1- or 2-naphthyl, hydroxy, lower alkoxy, carbamoyl-lower alkoxy, N-lower alkylcarbamoyl-lower alkoxy, N,N-di-lower alkylcarbamoyl-lower alkoxy,amino,mono- or di-lower alkylamino, lower alkanoylamino, halogen, carboxy, lower alkoxycarbonyl, benzyl-, naphthyl- or fluorenyl-lower alkoxycarbonyl, lower alkanoyl, sulfo, lower alkylsulfonyl, phosphono, hydroxy-lower alkoxyphosphoryl, di-lower alkoxyphosphoryl, carbamoyl, mono- or di-lower alkylcarbamoyl, sulfamoyl, mono-or di-lower alkylsulfamoyl, nitro and/or by cyano, wherein phenyl may be present up to three times,
R₅ is hydroxy, and
R₇ is unsubstituted lower alkyl; or cycloalkyl-lower alkyl as last described for cycloalkyl-lower alkyl R₃; or aryl-lower alkyl as last described for aryl-lower alkyl R₃;
or a salt thereof where at least one salt-forming group is present, the term "lower" in the above definitions indicating that the radical in question has up to and including 7 carbon atoms.

4. A compound of formula I according to claim 1, wherein R₁ and R₉ are each independently of the other
-hydrogen,
- lower alkoxycarbonyl,
- 2-halo-lower alkoxycarbonyl,
- aryloxycarbonyl wherein aryl has from 6 to 14 carbon atoms,
- aryl-lower alkoxycarbonyl wherein aryl has from 6 to 14 carbon atoms,
- heterocyclyl-lower alkoxycarbonyl wherein heterocyclyl is selected from pyrrolyl, furyl, thienyl, imidazolyl, pyrazolyl, oxazolyl, thiazolyl, pyridyl, pyrazinyl, pyrimidinyl, indolyl, quinolyl, isoquinolyl, quinoxalinyl, β-carbolinyl and a benzo-fused, cyclopenta-, cyclohexa- or cyclohepta-fused derivative of those radicals, which may also be fully or partially saturated,
- 2-tri-lower alkylsilyl-lower alkoxycarbonyl,
- 2-triarylsilyl-lower alkoxycarbonyl wherein aryl is phenyl or 1- or 2-naphthyl,
- the residue, bonded via the carboxy group, of an amino acid selected from glycine, alanine, valine, norvaline, leucine, isoleucine, norleucine, serine, homoserine, threonine, methionine, cysteine, proline, trans-3- and trans-4-hydroxyproline, phenylalanine, tyrosine, 4-aminophenylalanine, 4-chlorophenylalanine, 4-carboxyphenylalanine, β-phenylserine, phenylglycine, α-naphthylalanine, cyclohexylalanine, cyclohexylglycine, tryptophan, indoline-2-carboxylic acid, 1,2,3,4-tetrahydroisoquinoline-3-carboxylic acid, aspartic acid, asparagine, aminomalonic acid, aminomalonic acid monoamide, glutamic acid, glutamine, histidine, arginine, lysine, δ-hydroxylysine, ornithine, α,γ-diaminobutyric acid and α,β-diaminopropionic acid, wherein each of those amino acids may be in the D-, L- or (D,L)-form, and
the α-amino group may be unsubstituted or N-alkylated one or two times by lower alkyl, by amino-lower alkyl, by phenyl- or naphthyl-amino-lower alkyl, or by piperazinylcarbonyl-lower alkyl substituted at the nitrogen atom by lower alkyl, or N-acylated by lower alkanoyl, by aryl-lower alkanoyl wherein aryl is selected from phenyl, indenyl, indanyl, naphthyl, anthryl, phenanthryl, acenaphthyl and fluorenyl and may be unsubstituted or mono- to tri-substituted by lower alkyl, halo-lower alkyl, phenyl, 1-or 2-naphthyl, hydroxy, lower alkoxy, carbamoyl-lower alkoxy, N-lower alkylcarbamoyl-lower alkoxy or N,N-di-lower alkylcarbamoyl-lower alkoxy, amino, mono- or di-lower alkylamino, lower alkanoylamino, halogen, carboxy, lower alkoxycarbonyl, phenyl-, naphthyl- or fluorenyl-lower alkoxycarbonyl, lower alkanoyl, sulfo, lower alkylsulfonyl, phosphono, hydroxy-lower alkoxyphosphoryl or di-lower alkoxyphosphoryl, carbamoyl, mono- or di-lower alkylcarbamoyl, sulfamoyl, mono- or di-lower alkylaminosulfonyl, nitro and/or by cyano; wherein phenyl may be present up to three times, and wherein lower alkanoyl is branched or unbranched and may be unsubstituted or substituted by lower alkyl; heterocyclyl selected from pyrrolyl, furyl, thienyl, imidazolyl, pyrazolyl, oxazolyl, thiazolyl, pyridyl, pyrazinyl, pyrimidinyl, indolyl, quinolyl, isoquinolyl, quinoxalinyl, β-carbolinyl and a benzo-, cyclopenta-, cyclohexa- or cyclohepta-fused derivative of those radicals, which may also be fully or partially saturated; hydroxy; lower alkoxy; lower alkanoyloxy; acetoacetoxy; amino- or benzyloxycarbonylamino-lower alkanoyloxy; aryl-lower alkanoyloxy wherein aryl has from 6 to 10 carbon atoms; lower alkoxycarbonyloxy; mono- or di-lower alkylaminocarbonyloxy; aryloxycarbonyloxy wherein aryl has from 6 to 10 carbon atoms; aryl-lower alkoxycarbonyloxy wherein aryl has from 6 to 14 carbon atoms; sulfonyloxy; lower alkylsulfonyloxy; phenylsulfonyloxy; 2- or 4-toluenesulfonyloxy or 1- or 2- naphthylsulfonyloxy; carboxy; esterified carboxy selected from lower alkoxycarbonyl, aryloxycarbonyl wherein aryl has from 6 to 10 carbon atoms, aryl-lower alkoxycarbonyl wherein aryl has from 6 to 12 carbon atoms; lower alkanoyl; lower alkylsulfonyl; hydroxy-lower alkoxyphosphoryl and di-lower alkoxyphosphoryl; carbamoyl; carbamoyl substituted by one or two radicals selected from lower alkyl, carboxy-lower alkyl, lower alkoxycarbonyl-lower alkyl, di-lower alkylamino-lower alkyl, aminocarboxy-lower alkyl, hydroxy-lower alkyl and di-lower alkoxy-lower alkyl, or by one radical selected from ethylene, trimethylene, tetramethylene and pentamethylene, in which a carbon atom may have been replaced by nitrogen, oxygen, sulfur or by sulfur mono- or di-substituted by oxygen and which may be unsaturated; also sulfamoyl; phosphono; benzofuranyl; oxo and/or by cyano; by heterocyclyl-lower alkanoyl selected from thienyl-, furyl-, pyranyl-, pyrrolyl-, imidazolyl-, pyrazolyl-, oxazolyl-, isoxazolyl-, thiazolyl-, furazanyl-, pyridyl-, pyrazinyl-, pyrimidinyl-, pyridazinyl-, azepinyl-, indolyl-, benzimidazolyl-, 1H-indazolyl-, quinolyl-, isoquinolyl-, quinoxalinyl-, quinazolinyl-, cinnolyl-, purinyl-, pteridinyl-, naphthyridinyl-, 4H-quinolizinyl-, 3,1-benzofuranyl-, benz[e]indolyl-, 4,1-benzoxazinyl-, 4,1-benzothiazinyl-, carbazolyl-, β-carbolinyl-, phenazinyl-, phenanthridyl-, acridyl-, phenoxazinyl-, phenothiazinyl-, 1-azaacenaphthenyl-, cyclohexa[b]pyrrolyl-, cyclohepta[b]pyrrolyl-, cyclohexa[d]pyrazolyl-, cyclohexa[b]pyridyl-, cyclohexa[b]pyrazinyl-, cyclohexa[b]pyrimidinyl-, cyclohexa[b]-1,4-oxazinyl-, cyclohexa[b]-1,4-thiazinyl-, pyrrolidinyl-, pyrrolinyl-, imidazolidinyl-, 2-imidazolinyl-, 2,3-dihydropyridyl-, piperidyl-, piperazinyl-, 2,3,5,6-tetrahydropyrazinyl-, morpholinyl-, thiomorpholinyl-, S,S-dioxothiomorpholinyl-, indolinyl-, isoindolinyl-, 4,5,6,7-tetrahydroindolyl-, 1,2,3,4-tetrahydroquinolyl-, 1,2,3,4-tetrahydroisoquinolyl-, chroman-, thiochroman-, 1,2,3,4-tetrahydro-3,1-benzodiazinyl-, 3,4-dihydro-3H-4,1-benzoxazinyl-, 3,4-dihydro-3H-4,1-benzothiazinyl-, 2,3,4,5-tetrahydro-1H-5,1-benzazepinyl- and 5,6-dihydrophenanthridinyl-lower alkanoyl, the mentioned heterocyclyl radicals being unsubstituted or substituted by lower alkyl, phenyl, 1- or 2-naphthyl, phenyl-lower alkyl, hydroxy-lower alkyl, lower alkoxy-lower alkyl, phenoxy- or naphthoxy-lower alkyl, phenyl-lower alkoxy- or naphthyl-lower alkoxy-lower alkyl, lower alkanoyloxy-lower alkyl, phenyl- or naphthyl-lower alkanoyloxy-lower alkyl, lower alkoxycarbonyloxy-lower alkyl, phenyl-, naphthyl- or fluorenyl-lower alkoxycarbonyloxy-lower alkyl, amino-lower alkyl, carboxy-lower alkyl, hydroxy, lower alkoxy, phenyl- or naphthyl-lower alkoxy, amino, lower alkylamino, di-lower alkylamino, carboxy, lower alkoxycarbonyl, phenyl- or naphthyl-lower alkoxycarbonyl, halogen, lower alkanoyl, lower alkylsulfonyl, phosphono, hydroxy-lower alkoxyphosphoryl, dialkoxyphosphoryl, carbamoyl, mono- or di-lower alkylcarbamoyl, hydroxy-or carboxy-lower alkylcarbamoyl, sulfamoyl, nitro, oxo and/or by cyano; by heterocyclyl-lower alkenoyl wherein heterocyclyl is selected from pyrrolyl, furyl, thienyl, imidazolyl, pyrazolyl, oxazolyl, thiazolyl, pyridyl, pyrazinyl, pyrimidinyl, indolyl, quinolyl, isoquinolyl, quinoxalinyl, β-carbolinyl and a benzo-fused, cyclopenta-, cyclohexa- or cyclohepta-fused derivative of those radicals, which may also be fully or partially saturated; by lower alkoxycarbonyl; by aryl-lower alkoxycarbonyl wherein aryl has from 6 to 14 carbon atoms; by heterocyclyl-lower alkoxycarbonyl wherein heterocyclyl is selected from pyrrolyl, furyl, thienyl, imidazolyl, pyrazolyl, oxazolyl, thiazolyl, pyridyl, pyrazinyl, pyrimidinyl, indolyl, quinolyl, isoquinolyl, quinoxalinyl, β-carbolinyl and a benzo-fused, cyclopenta-, cyclohexa- or cyclohepta-fused derivative of those radicals, which may also be fully or partially saturated and unsubstituted or substituted by lower alkyl; by carboxy-lower alkanoyl; by lower alkoxycarbonyl-lower alkanoyl; by hydroxy-lower alkoxy-lower alkanoyl; by amino-lower alkanoyl or by benzyloxycarbonylamino-lower alkanoyl wherein the amino group is not bonded in the α- or β-position; by carbamoyl; by phenyl-lower alkylaminocarbonyl; by N-di-lower alkylamino-lower alkyl-N-lower alkylaminocarbonyl; by N-dihydroxy-lower alkyl-N-lower alkylaminocarbonyl; by 2- or 3-pyridyl-lower alkylaminocarbonyl; by N-2-pyridyl-lower alkyl-N-lower alkylaminocarbonyl; by sulfonyl; by lower alkylsulfonyl; by arylsulfonyl wherein aryl has from 6 to 10 carbon atoms and is unsubstituted or substituted by lower alkyl or by lower alkoxy; by heterocyclylsulfonyl wherein heterocyclyl is selected from pyrrolyl, furyl, thienyl, imidazolyl, pyrazolyl, oxazolyl, thiazolyl, pyridyl, pyrazinyl, pyrimidinyl, indolyl, quinolyl, isoquinolyl, quinoxalinyl, β-carbolinyl and a benzo-fused, cyclopenta-, cyclohexa- or cyclohepta-fused derivative of those radicals, which may also be fully or partially saturated; by sulfamoyl or by sulfamoyl substituted by heterocyclyl-lower alkyl, wherein heterocyclyl is as last defined, and/or by lower alkyl,
a carboxy group of the side chain is present in free form or in esterified form in the form of a lower alkyl ester group, an aryl ester group or an aryl-lower alkyl ester group, wherein aryl is phenyl, 4-nitrophenyl, naphthyl or biphenylyl, or is present in amidated form in the form of a carbamoyl, lower alkylcarbamoyl, di-lower alkylaminocarbamoyl, mono-or di-(hydroxy-lower alkyl)carbamoyl or mono- or di-(carboxy-lower alkyl)carbamoyl group, an amino group of the side chain is present in free form or in alkylated form in the form of a mono- or di-lower alkylamino group or is present in acylated form in the form of a lower alkanoylamino group, an amino-lower alkanoylamino group, an aryl-lower alkanoylamino group wherein aryl has from 6 to 14 carbon atoms and is unsubstituted or substituted by lower alkyl, hydroxy, lower alkoxy, carboxy, carbamoyl or by sulfamoyl, a lower alkoxycarbonylamino group, an arylmethoxycarbonylamino group wherein aryl has from 6 to 14 carbon atoms, a piperidyl-1-carbonyl group, a morpholinocarbonyl group, a thiomorpholinocarbonyl group or an S,S-dioxothiomorpholinocarbonyl group and/or
a hydroxy group of the side chain is present in free form or in etherified or esterified form in the form of a lower alkoxy, aryl-lower alkoxy, lower alkanoyloxy or lower alkyloxycarbonyloxy group,
- lower alkylsulfonyl,
- 2- or 3-pyrrolyl-, 2-thienyl-, 2-furyl-, 1-pyrazolyl-, 2-, 3- or 4-pyridyl-, 2-, 3- or 5-indolyl-, (1-methyl-, 2-methyl-, 5-methoxy-, 5-benzyloxy-, 5-chloro- or 4,5-dimethylindol-2-yl)-, (1-benzylindol-2-yl or -3-yl)-, 4,5,6,7-tetrahydroindol-2-yl-, (2-, 3- or 4-quinolyl or 4-hydroxyquinol-2-yl)-, (1-, 3- or 4-isoquinolyl or 1-oxo-1,2-dihydroisoquinol-3-yl)-, 3-pyrrolidinyl-, (3- or 4-hydroxypyrrolidin-2-yl)-, 5-oxopyrrolidin-2-yl-, (2- or 3-morpholinyl)-, (2- or 3-thiomorpholinyl)-, (S,S-dioxothiomorpholin-2- or -3-yl)-, (2- or 3-indolinyl)-, (1,2,3,4-tetrahydroquinol-2-, -3- or -4-yl)- or (1,2,3,4-tetrahydroisoquinol-1-, -2- or -3-yl)-methylsulfonyl,
- phenyl- or 1- or 2-naphthyl-sulfonyl unsubstituted or mono- or di-substituted by lower alkyl,
- lower alkoxysulfonyl, or
- benzyloxysulfonyl or 1- or 2-naphthyloxysulfonyl,
such that not more than one of the radicals R₁ and R₉ may be hydrogen, and
R₂ and R₈ are each independently of the other hydrogen or the same radicals as R₁ and R₉, or the pairs of substituents R₁ and R₉, and R₂ and R₈, each independently of the other, form together with the bonding nitrogen atom and a radical selected from ethylene, trimethylene, tetramethylene and pentamethylene in which a carbon atom may have been replaced by nitrogen, oxygen, sulfur or by sulfur mono- or di-substituted by oxygen and which may be unsaturated, a heterocyclic ring,
R₃ is
- cycloalkyl-lower alkyl wherein cycloalkyl has from 3 to 7 carbon atoms and is unsubstituted or mono- to tri-substituted by lower alkyl, halo-lower alkyl, hydroxy, lower alkoxy, carbamoyl-lower alkoxy, N-lower alkylcarbamoyl-lower alkoxy or N,N-di-lower alkylcarbamoyl-lower alkoxy, amino, mono- or di-lower alkylamino, halogen, carboxy, lower alkoxycarbonyl, phenyl-, naphthyl- or fluorenyl-lower alkoxycarbonyl, lower alkanoyl, lower alkylsulfonyl, phosphono, hydroxy-lower alkoxyphosphoryl or di-lower alkoxyphosphoryl, carbamoyl, mono- or di-lower alkylaminocarbamoyl, sulfamoyl, mono-or di-lower alkylsulfamoyl, nitro and/or by cyano and is bonded to lower alkyl, or - aryl-lower alkyl wherein aryl is selected from phenyl, indenyl, indanyl, naphthyl, anthryl, phenanthryl, acenaphthyl and fluorenyl and may be unsubstituted or mono-to tri-substituted by lower alkyl, halo-lower alkyl, phenyl, 1- or 2-naphthyl, hydroxy, lower alkoxy, carbamoyl-lower alkoxy, N-lower alkylcarbamoyl-lower alkoxy or N,N-di-lower alkylcarbamoyl-lower alkoxy, amino, mono- or di-lower alkylamino, lower alkanoylamino, halogen, carboxy, lower alkoxycarbonyl, phenyl-, naphthyl- or fluorenyl-lower alkoxycarbonyl, lower alkanoyl, sulfo, lower alkylsulfonyl, phosphono, hydroxy-lower alkoxyphosphoryl or di-lower alkoxyphosphoryl, carbamoyl, mono- or di-lower alkylcarbamoyl, sulfamoyl, mono- or di-lower alkylsulfamoyl, nitro and/or by cyano, wherein phenyl may be present up to three times, and wherein lower alkyl is unbranched or branched and is unsubstituted or substituted by lower alkyl; heterocyclyl selected from pyrrolyl, furyl, thienyl, imidazolyl, pyrazolyl, oxazolyl, thiazolyl, pyridyl, pyrazinyl, pyrimidinyl, indolyl, quinolyl, isoquinolyl, quinoxalinyl, β-carbolinyl and a benzo-fused, cyclopenta-, cyclohexa- or cyclohepta-fused derivative of those radicals, which may also be fully or partially saturated; hydroxy; lower alkoxy; lower alkanoyloxy; acetoacetoxy; amino- or benzyloxycarbonylamino-lower alkanoyloxy; aryl-lower alkanoyloxy wherein aryl has from 6 to 10 carbon atoms; lower alkoxycarbonyloxy; mono- or di-lower alkylaminocarbonyloxy; aryloxycarbonyloxy wherein aryl has from 6 to 10 carbon atoms; aryl-lower alkoxycarbonyloxy wherein aryl has from 6 to 12 carbon atoms; sulfonyloxy; lower alkylsulfonyloxy; phenylsulfonyloxy; 2- or 4-toluenesulfonyloxy or 1- or 2-naphthylsulfonyloxy; amino; mono- or di-lower alkylamino; N-lower alkoxy-N-lower alkylamino; mono- or di-(phenyl- or naphthyl-lower alkyl)amino; lower alkanoylamino; carboxy; esterified carboxy selected from lower alkoxycarbonyl, aryloxycarbonyl wherein aryl has from 6 to 10 carbon atoms, aryl-lower alkoxycarbonyl wherein aryl has from 6 to 12 carbon atoms; lower alkanoyl; lower alkylsulfonyl; hydroxy-lower alkoxyphosphoryl and di-lower alkoxyphosphoryl; carbamoyl; carbamoyl substituted by one or two radicals selected from lower alkyl, carboxy-lower alkyl or lower alkoxycarbonyl-lower alkyl, di-lower alkylamino-lower alkyl, aminocarboxy-lower alkyl, hydroxy-lower alkyl and di-lower alkoxy-lower alkyl, or by one radical selected from ethylene, trimethylene, tetramethylene and pentamethylene, in which a carbon atom may have been replaced by nitrogen, oxygen, sulfur or by sulfur mono- or di-substituted by oxygen and which may be unsaturated; also sulfamoyl; phosphono; benzofuranyl; oxo and/or by cyano,
R₄ is hydrogen,
R₅ is hydroxy and
R₆ is hydrogen,
or R₅ and R₆ together are oxo, and
R₇ is
- lower alkyl,
- cycloalkyl-lower alkyl wherein cycloalkyl has from 3 to 7 carbon atoms, is unsubstituted or mono- to tri-substituted by lower alkyl, halo-lower alkyl, hydroxy, lower alkoxy, carbamoyl-lower alkoxy, N-lower alkylcarbamoyl-lower alkoxy or N,N-di-lower alkylcarbamoyl-lower alkoxy, amino, mono- or di-lower alkylamino, halogen, carboxy, lower alkoxycarbonyl, phenyl-, naphthyl- or fluorenyl-lower alkoxycarbonyl, lower alkanoyl, lower alkylsulfonyl, phosphono, hydroxy-lower alkoxyphosphoryl or di-lower alkoxyphosphoryl, carbamoyl, mono- or di-lower alkylaminocarbamoyl, sulfamoyl, mono- or di-lower alkylsulfamoyl, nitro and/or by cyano and is bonded to lower alkyl,
- bicycloalkyl-lower alkyl wherein bicycloalkyl contains from 5 to 10 carbon atoms,
- tricycloalkyl-lower alkyl wherein tricycloalkyl contains from 8 to 10 carbon atoms,
- aryl-lower alkyl wherein aryl is selected from phenyl, indenyl, indanyl, naphthyl, anthryl, phenanthryl, acenaphthyl and fluorenyl and may be unsubstituted or mono-to tri-substituted by lower alkyl, halo-lower alkyl, phenyl, 1- or 2-naphthyl, hydroxy, lower alkoxy, carbamoyl-lower alkoxy, N-lower alkylcarbamoyl-lower alkoxy, N,N-di-lower alkylcarbamoyl-lower alkoxy, amino, mono- or di-lower alkylamino, lower alkanoylamino, halogen, carboxy, lower alkoxycarbonyl, benzyl-, naphthyl- or fluorenyl-lower alkoxycarbonyl, lower alkanoyl, sulfo, lower alkylsulfonyl, phosphono, hydroxy-lower alkoxyphosphoryl, di-lower alkoxyphosphoryl, carbamoyl, mono- or di-lower alkylcarbamoyl, sulfamoyl, mono- or di-lower alkylsulfamoyl, nitro and/or by cyano, wherein phenyl may be present up to three times, and wherein lower alkyl is unbranched or branched and is unsubstituted or substituted by lower alkyl; heterocyclyl selected from pyrrolyl, furyl, thienyl, imidazolyl, pyrazolyl, oxazolyl, thiazolyl, pyridyl, pyrazinyl, pyrimidinyl, indolyl, quinolyl, isoquinolyl, quinoxalinyl, β-carbolinyl and a benzo-fused, cyclopenta-, cyclohexa- or cyclohepta-fused derivative of those radicals, which may also be fully or partially saturated; hydroxy; lower alkoxy; lower alkanoyloxy; acetoacetoxy; amino- or benzyloxycarbonylamino-lower alkanoyloxy; aryl-lower alkanoyloxy wherein aryl has from 6 to 10 carbon atoms; lower alkoxycarbonyloxy; mono- or di-lower alkylaminocarbonyloxy; aryloxycarbonyloxy wherein aryl has from 6 to 10 carbon atoms; aryl-lower alkoxycarbonyloxy wherein aryl has from 6 to 12 carbon atoms; sulfonyloxy; lower alkylsulfonyloxy; phenylsulfonyloxy; 2- or 4-toluenesulfonyloxy; 1- or 2-naphthylsulfonyloxy; amino; mono- or di-lower alkylamino; N-lower alkoxy-N-lower alkylamino; mono- or di-(phenyl- or naphthyl-lower alkyl)amino; lower alkanoylamino; carboxy; esterified carboxy selected from lower alkoxycarbonyl, aryloxycarbonyl wherein aryl has from 6 to 10 carbon atoms, aryl-lower alkoxycarbonyl wherein aryl has from 6 to 12 carbon atoms; lower alkanoyl; lower alkylsulfonyl; hydroxy-lower alkoxyphosphoryl and di-lower alkoxyphosphoryl; carbamoyl; carbamoyl substituted by one or two radicals selected from lower alkyl, carboxy-lower alkyl, lower alkoxycarbonyl-lower alkyl, di-lower alkylamino-lower alkyl, aminocarboxy-lower alkyl, hydroxy-lower alkyl and di-lower alkoxy-lower alkyl, or by one radical selected from ethylene, trimethylene, tetramethylene and pentamethylene, in which a carbon atom may have been replaced by nitrogen, oxygen, sulfur or by sulfur mono- or di-substituted by oxygen and which may be unsaturated; also sulfamoyl; phosphono; benzofuranyl; oxo (which is not bonded to the carbon atom that is linked to the nitrogen atom bonding the radical R₇) and/or by cyano, - heterocyclyl-lower alkyl wherein heterocyclyl is preferably selected from pyrrolyl, furyl, thienyl, imidazolyl, pyrazolyl, oxazolyl, thiazolyl, pyridyl, pyrazinyl, pyrimidinyl, indolyl, quinolyl, isoquinolyl, quinoxalinyl, β-carbolinyl and a benzo-fused, cyclopenta-, cyclohexa- or cyclohepta-fused derivative of those radicals, which may also be fully or partially saturated, e.g. 4-pyrrolidinylmethyl, 1-imidazolylmethyl, 2-pyridylmethyl, 3-pyridylmethyl, quinolin-2-ylmethyl or indol-2-ylmethyl,
- hydroxy-lower alkyl,
- lower alkoxy-lower alkyl,
- phenoxy-lower alkyl or nitrophenoxy-lower alkyl,
- naphthoxy-lower alkyl,
- lower alkanoyloxy-lower alkyl,
- acetoacetoxy-lower alkyl,
- arylmercapto-lower alkyl wherein aryl has from 6 to 10 carbon atoms,
- amino-lower alkyl,
- mono- or di-lower alkylamino-lower alkyl,
- phenyl- or naphthyl-amino-lower alkyl,
- lower alkanoylamino-lower alkyl,
- piperazinylcarbonyl-lower alkyl substituted at the nitrogen atom by lower alkyl,
- lower alkoxycarbonylamino-lower alkyl,
- phenyl-lower alkoxycarbonylamino-lower alkyl,
- aminocarbonylamino-lower alkyl,
- N-phenyl-lower alkyl-N-lower alkylaminocarbonylamino-lower alkyl,
- halo-lower alkyl,
- carboxy-lower alkyl,
- lower alkoxycarbonyl-lower alkyl,
- 2-halo-lower alkoxycarbonyl-lower alkyl,
- phenyl- or naphthyl-lower alkyloxycarbonyl-lower alkyl,
- heterocyclyl-lower alkoxycarbonyl-lower alkyl wherein heterocyclyl is selected from pyrrolyl, furyl, thienyl, imidazolyl, pyrazolyl, oxazolyl, thiazolyl, pyridyl, pyrazinyl, pyrimidinyl, indolyl, quinolyl, isoquinolyl, quinoxalinyl, β-carbolinyl and a benzo-fused, cyclopenta-, cyclohexa- or cyclohepta-fused derivative of those radicals, it being possible for the said radicals also to be fully or partially saturated,
- lower alkylsulfonyl-lower alkyl,
- arylsulfonyl-lower alkyl wherein aryl has from 6 to 10 carbon atoms,
- carbamoyl-lower alkyl,
- lower alkylcarbamoyl-lower alkyl,
- di-lower alkylcarbamoyl-lower alkyl,
- hydroxy-lower alkylcarbamoyl- or di(hydroxy-lower alkyl)carbamoyl-lower alkyl,
- N-lower alkoxy-lower alkoxy-lower alkylcarbamoyl-lower alkyl,
- carboxy-lower alkylcarbamoyl- or di(carboxy-lower alkyl)carbamoyl-lower alkyl,
- carbamoyl-lower alkyl substituted at the nitrogen atom by a radical selected from ethylene, trimethylene, tetramethylene and pentamethylene, wherein a carbon atom may have been replaced by nitrogen, oxygen, sulfur or by sulfur mono- or di-substituted by oxygen, it being possible for the radical so formed also to be fully or partially unsaturated,
- N-lower alkyl-N-heterocyclyl-lower alkylcarbamoyl-lower alkyl wherein heterocyclyl is selected from pyrrolyl, furyl, thienyl, imidazolyl, pyrazolyl, oxazolyl, thiazolyl, pyridyl, pyrazinyl, pyrimidinyl, indolyl, quinolyl, isoquinolyl, quinoxalinyl, β-carbolinyl and a benzo-fused, cyclopenta-, cyclohexa- or cyclohepta-fused derivative of those radicals, which may also be fully or partially saturated,
- sulfamoyl-lower alkyl,
- N-(phenyl- or naphthyl-lower alkyl)sulfamoyl-lower alkyl,
- sulfamoyl-lower alkyl substituted at the nitrogen atom by a radical selected from ethylene, trimethylene, tetramethylene and pentamethylene, wherein a carbon atom may have been replaced by nitrogen, by nitrogen substituted by lower alkyl, by oxygen, by sulfur or by sulfur mono- or di-substituted by oxygen, it being possible for the radical so formed also to be fully or partially unsaturated,
- oxo-lower alkyl (wherein oxo is not bonded to the carbon atom that is linked to the nitrogen atom bonding R₇),
- cyano-lower alkyl,
- hydroxy-carboxy-lower alkyl,
- α-naphthoxy-carboxy-lower alkyl,
- hydroxy-lower alkoxycarbonyl-lower alkyl,
- α-naphthoxy-lower alkoxycarbonyl-lower alkyl,
- lower alkylcarbonyl-halo-lower alkyl,
- α-naphthoxyethoxycarbonyl-lower alkyl,
- α-naphthoxybenzyloxycarbonyl-lower alkyl,
- esterified hydroxy-lower alkoxycarbonyl-lower alkyl wherein the hydroxy group is esterified by lower alkanoyl; by cycloalkyl-lower alkanoyl wherein cycloalkyl has from 3 to 7 carbon atoms; by bicycloalkyl-lower alkanoyl wherein bicycloalkyl contains from 5 to 10 carbon atoms; by tricycloalkyl-lower alkanoyl wherein tricycloalkyl contains from 8 to 10 carbon atoms; by aryl-lower alkanoyl wherein aryl has from 6 to 14 carbon atoms and may be unsubstituted or mono- to tri-substituted by lower alkyl, hydroxy, lower alkoxy, carbamoyl-lower alkoxy, N-lower alkylcarbamoyl-lower alkoxy or N,N-di-lower alkylcarbamoyl-lower alkoxy, amino, mono- or di-lower alkylamino, halogen, carboxy, lower alkoxycarbonyl, phenyl-, naphthyl- or fluorenyl-lower alkoxycarbonyl, lower alkanoyl, lower alkylsulfonyl, phosphono, hydroxy-lower alkoxy-phosphoryl, di-lower alkoxyphosphoryl, carbamoyl, sulfamoyl, nitro and/or by cyano; by lower alkoxycarbonyl; by 2-halo-lower alkoxycarbonyl; or by phenyl- or fluorenyl-lower alkoxycarbonyl,
- dihydroxy-carboxy-lower alkyl,
- dihydroxy-lower alkoxycarbonyl-lower alkyl,
- dihydroxy-lower alkoxycarbonyl-lower alkyl esterified by lower alkanoyl, lower alkoxycarbonyl, phenyl- or fluorenyl-lower alkoxycarbonyl, lower alkylsulfonyl or by toluenesulfonyl,
- α-naphthoxy-di-lower alkylamino-lower alkyl,
- α-naphthoxy-carbamoyl-lower alkyl,
- α-naphthoxy-oxo-lower alkyl (wherein oxo is not bonded to the carbon atom that is linked to the nitrogen atom bonding R₇), or
- α-naphthoxy-cyano-lower alkyl,
or a salt of such a compound where salt-forming groups are present, the term "lower" in the above definitions indicating that the radical in question has up to and including 7 carbon atoms.

5. A compound of formula I according to claim 1, wherein R₁ is lower alkoxycarbonyl, phenyl-lower alkoxycarbonyl, the monovalent residue, bonded via the carboxy group, of an aliphatic amino acid selected from valine, alanine, leucine and isoleucine, or the residue, bonded via the carboxy group, of an aliphatic amino acid as defined above that is acylated at the amino nitrogen atom by one of the radicals phenyl-lower alkanoyl, morpholinyl-lower alkanoyl, thiomorpholinyl-lower alkanoyl, S,S-dioxothiomorpholinyl-lower alkanoyl, pyridyl-lower alkanoyl, lower alkoxycarbonyl and phenyl-lower alkoxycarbonyl, all of the amino acids mentioned being in the D-, D,L- or L-form, R₂ is hydrogen, R₃ is phenyl-lower alkyl, R₄ is hydrogen, R₅ is hydroxy, R₆ is hydrogen, R₇ is lower alkyl, cyclohexyl-lower alkyl or phenyl-lower alkyl, R₈ is hydrogen and R₉ is one of the radicals mentioned for R₁, and the asymmetric carbon atoms carrying the radicals R₃ and R₅ are in the S-configuration, or a pharmacologically acceptable salt of such a compound, the term "lower" in the above definitions indicating that the radical in question has up to and including 7 carbon atoms.

6. A compound of formula I according to claim 1, wherein R₁ is tert-butoxycarbonyl, benzyloxycarbonyl, the monovalent residue, bonded via the carboxy group, of the amino acid valine, or the residue, bonded via the carboxy group, of alanine acylated at the amino nitrogen atom by one of the radicals phenylacetyl, 3-pyridylacetyl, morpholinocarbonyl, thiomorpholinocarbonyl, tert-butoxycarbonyl and benzyloxycarbonyl, R₂ is hydrogen, R₃ is benzyl, R₄ is hydrogen, R₅ is hydroxy, R₆ is hydrogen, R₇ is isobutyl, cyclohexylmethyl or benzyl, R₈ is hydrogen and R₉ is one of the radicals mentioned for R₁, and the asymmetric carbon atoms carrying the radicals R₃ and R₅ are in the S-configuration, or a pharmacologically acceptable salt of such a compound.

7. The compound of formula I according to claim 1, wherein R₁ and R₉ are each the monovalent residue, bonded via the carboxy group and acylated at the amino nitrogen atom by benzyloxycarbonyl, of the amino acid (L)-valine, R₂ and R₈ are hydrogen, R₃ is benzyl, R₄ is hydrogen, R₅ is hydroxy, R₆ is hydrogen and R₇ is benzyl, and the asymmetric carbon atoms carrying the radicals R₃ and R₅ are in the S-configuration, or a pharmacologically acceptable salt of that compound.

8. The compound of formula I according to claim 1, wherein R₁ and R₉ are each the monovalent residue, bonded via the carboxy group and acylated at the amino nitrogen atom by 4-thiomorpholinocarbonyl, of the amino acid (L)-valine, R₂ and R₈ are hydrogen, R₃ is benzyl, R₄ is hydrogen, R₅ is hydroxy, R₆ is hydrogen and R₇ is isobutyl, and the asymmetric carbon atoms carrying the radicals R₃ and R₅ are in the S-configuration, or a pharmacologically acceptable salt of that compound.

9. A compound of formula I according to claim 1 selected from the compounds designated
Boc-[Phe^{NN}Phe]-Boc,
Boc-(L)-Val-[Phe^{NN}Phe]←((L)-Val-Boc),
Boc-[Phe^{NN}Cha]-Boc,
H-(L)-Val-[Phe^{NN}Phe]←((L)-Val)-H,
N-thiomorpholinocarbonyl-(L)-Val-[Phe^{NN}Phe]←(N-thiomorpholinocarbonyl-(L)-Val),
N-morpholinocarbonyl-(L)-Val-[Phe^{NN}Phe]←(N-morpholinocarbonyl-(L)-Val),
phenylacetyl-(L)-Val-[Phe^{NN}Phe]←(N-phenylacetyl-(L)-Val),
N-(3-pyridylacetyl)-(L)-Val-[Phe^{NN}Phe]←(N-(3-pyridylacetyl)-(L)-Val),
Boc-(L)-Val-[Phe^{NN}Cha]←((L)-Val)-Boc,
Z-(L)-Val-[Phe^{NN}Cha]←((L)-Val)-Z,
Boc-[Phe^{NN}Leu]-Boc,
Z-(L)-Val-[Phe^{NN}Leu]←((L)-Val)-Z, H-(L)-Val-[Phe^{NN}Cha]←((L)-Val)-H and
N-(3-pyridylacetyl)-(L)-Val-[Phe^{NN}Leu]←(N-(3-pridylacetyl)-(L)-Val), or a salt thereof;
wherein Boc is tert-butoxycarbonyl, Z is benzyloxycarbonyl, the residue - [Phe^{NN}Phe]- is the divalent radical of 3(S)-amino-4-phenyl-1-(N-benzylhydrazino)-butan-2(S)-ol and has the formula 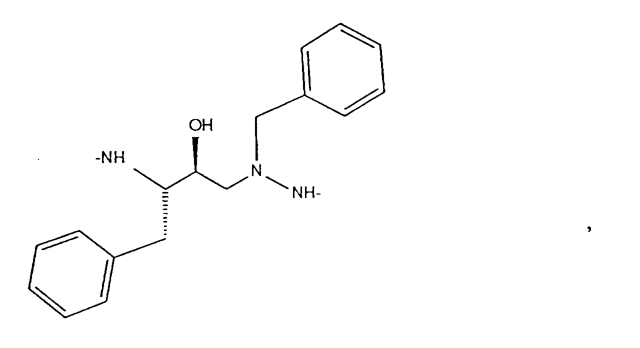 the residue -[Phe^{NN}Cha]- is the divalent radical of 3(S)-amino-4-phenyl-1-(N-cyclohexylmethylhydrazino)-butan-2(S)-ol and has the formula 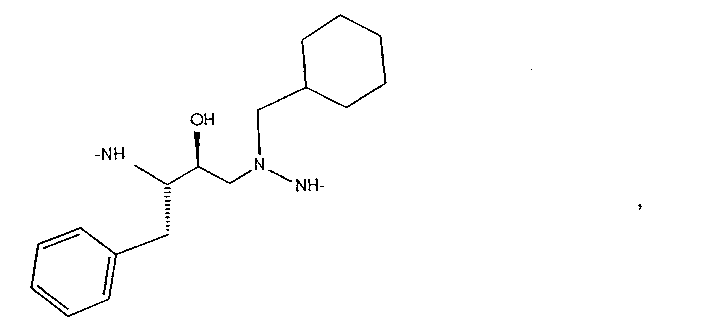 the residue -[Phe^{NN}Leu]- is the divalent radical of 3(5)-amino-4-phenyl-1-(N-isobutylhydrazino)-butan-2(S)-ol and has the formula 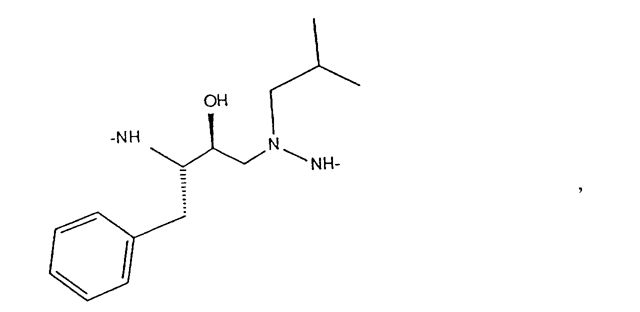 and an arrow "←" denotes the reversal of the bonding direction in a departure from customary peptide nomenclature in which the amino terminus is on the left and the carboxy terminus on the right.

10. A compound of formula I according to claim 1 selected from the compounds designated
Z-(L)-Val-[(p-F)Phe^{NN}(p-F)Phe]←(N-(N-(2-pyridylmethyl)-N-methylaminocarbonyl)-(L)-Val),
Z-(L)-Val-[(p-F)Phe^{NN}(p-F)Phe]←(N-(2(R,S)-carbamoyl-3-phenyl-propionyl)(L)-Val),
acetyl-(L)-Val-[Phe^{NN}Cha]←(N-acetyl-(L)-Val),
acetyl-Ile-[Phe^{NN}Cha]←(N-acetyl-Ile),
N-(2-pyridylmethyl)-N-methyl-aminocarbonyl-(L)-Val-[Phe^{NN}(p-F)-Phe]←(N-(N-(2-pyridylmethyl)-N-methyl-aminocarbonyl)-(L)-Val),
Z-(L)-Val-[Phe^{NN}(p-F)Phe]←((L)-Val)-Z,
Z-(L)-Val-[Phe^{NN}(p-CN)Phe]←((L)-Val)-Z,
Z-(L)-Ile-[Phe^{NN}Leu]←((L)-Ile)-Z,
isobutoxycarbonyl-(L)-Val-[Phe^{NN}Leu]←(N-isobutoxycarbonyl-(L)-Val),
acetyl-Val-[Phe^{NN}Leu]←(N-(2(R,S)-carbamoyl-3-phenyl-propionyl)-Val),
N-trifluoroacetyl-[Phe^{NN}Leu]←(N-(2(R,S)-carbamoyl-3-phenyl-propionyl)-(L)-Val),
Z-(L)-Val-[Phe^{NN}Nle]←(N-(2(R,S)-(N-(2-morpholinoethyl)-carbamoyl)-3-methyl)-butyryl),
Z-(L)-Val-[Phe^{NN}Nle]←(N-(2(R,S)-(N-(2-pyridylmethyl)-carbamoyl)-3-methyl)-butyryl),
methoxycarbonyl-(L)-Val-[Phe^{NN}Leu]←(N-methoxycarbonyl-(L)-Val),
methoxycarbonyl-(L)-Val-[Phe^{NN}(p-F)Phe]←(N-methoxycarbonyl-(L)-Val) and
methoxycarbonyl-(L)-Val-[Phe^{NN}(p-CN)Phe]←(N-methoxycarbonyl-(L)-Val),
or a salt thereof, wherein Boc is tert-butoxycarbonyl, Z is benzyloxycarbonyl, the residue -[Phe^{NN}Phe]- is the divalent radical of 3(S)-amino-4-phenyl-1-(N-benzylhydrazino)butan-2(S)-ol and has the formula 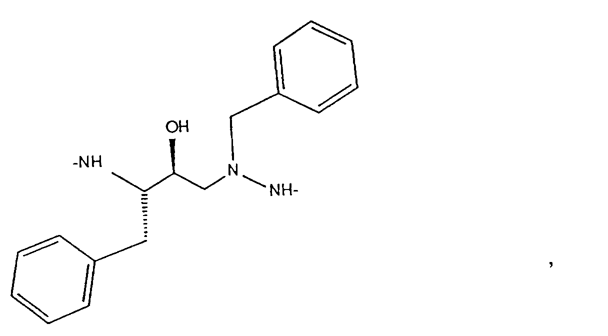 the residue -[Phe^{NN}Cha]- is the divalent radical of 3(S)-amino-4-phenyl-1-(N-cyclohexylmethylhydrazino)-butan-2(S)-ol and has the formula 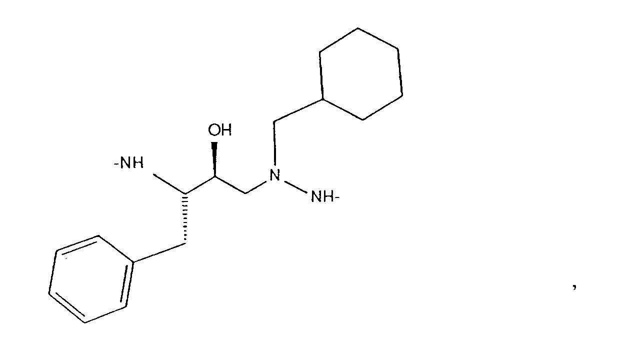 the residue -[Phe^{NN}Leu]- is the divalent radical of 3(S)-amino-4-phenyl-1-(N-isobutylhydrazino)-butan-2(S)-ol and has the formula 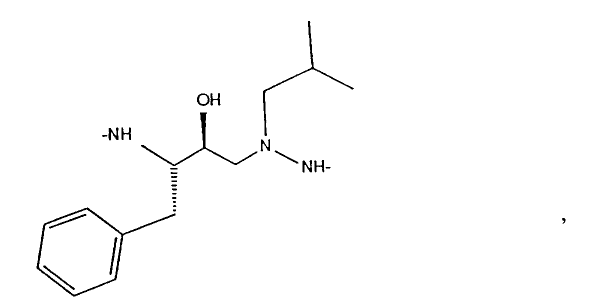 the residue designated -[Phe^{NN}Nle]- is the radical of 3(S)-amino-4-phenyl-1-(N-n-butylhydrazino)-butan-2(S)-ol and has the formula 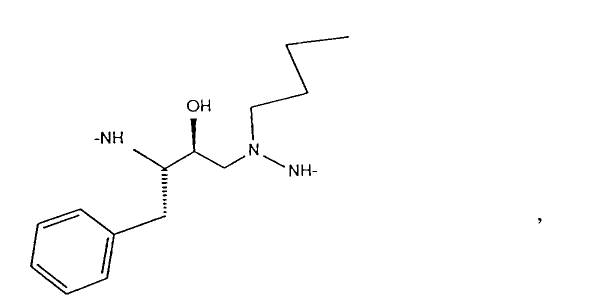 the residue designated -[Phe^{NN}(p-F)Phe]- is the divalent radical of 3(S)-amino-4-phenyl-1-(N-(p-fluorophenylmethyl)-hydrazino)-butan-2(S)-ol and has the formula 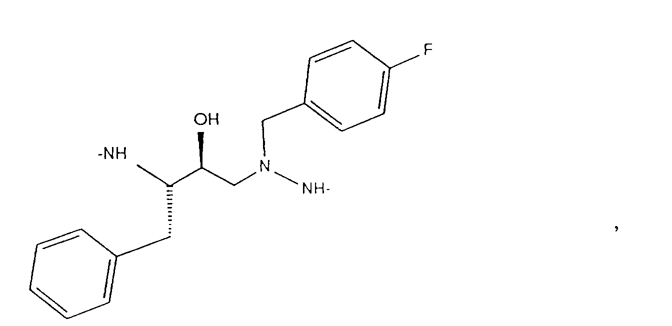 the residue designated -[(p-F)Phe^{NN}(p-F)Phe]- is the divalent radical of 3(S)-amino-4-(p-fluorophenyl)-1-(N-(p-fluorophenylmethyl)-hydrazino)-butan-2(S)-ol and has the formula 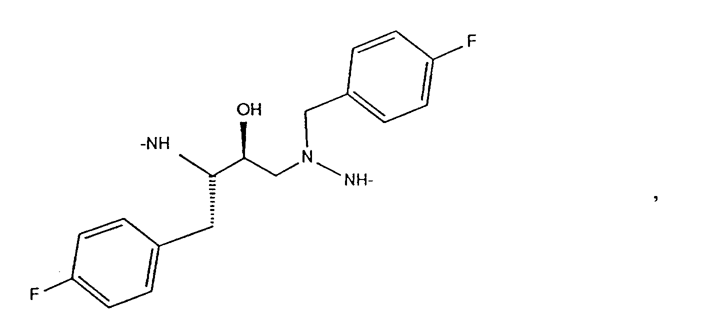 the residue designated -[Phe^{NN}(p-CN)Phe]- is the divalent radical of 3(S)-amino-4-phenyl-1-(N-(p-cyanophenylmethyl)-hydrazino)-butan-2(S)-ol and has the formula 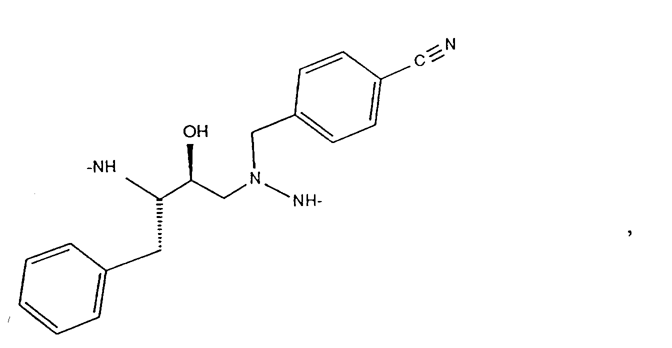 and an arrow "←" denotes the reversal of the bonding direction in a departure from customary peptide nomenclature in which the amino terminus is on the left and the carboxy terminus on the right.

11. A compound of formula I according to any one of claims 1 to 10 for use in a method for the therapeutic treatment of the human or animal body.

12. A pharmaceutical composition comprising a compound of formula I according to any one of claims 1 to 10 or a pharmaceutically acceptable salt of such a compound having at least one salt-forming group together with a pharmaceutically acceptable carrier.

13. The use of any one of the compounds of formula I mentioned in claims 1 to 10 or a pharmaceutically acceptable salt of such a compound having at least one salt-forming group in the preparation of a pharmaceutical composition for use in the treatment of AIDS.

14. A process for the preparation of a compound of formula I according to claim 1, wherein the substituents are as defined in claim 1, or a salt thereof, which comprises
a) adding a hydrazine derivative of the formula 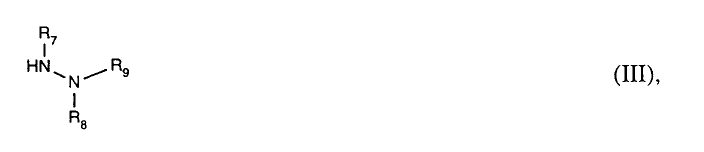 wherein the radicals are as defined above, to an epoxide of the formula 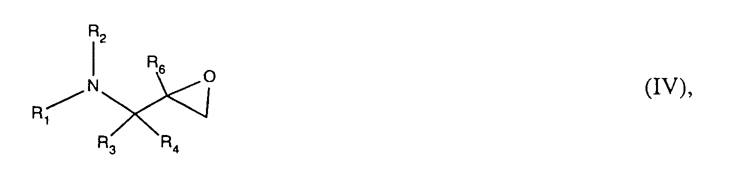 wherein the radicals are as defined above, and free functional groups, with the exception of those participating in the reaction, are optionally in protected form, and removing any protecting groups present, or
b) for the preparation of a compound of formula I wherein R₁ and R₉ are acyl; sulfo; sulfonyl substituted by unsubstituted or substituted alkyl, aryl, alkoxy, which is unsubstituted or substituted, or by aryloxy; sulfamoyl that is unsubstituted or substituted at the nitrogen atom; or phosphoryl substituted by one or two radicals selected independently of one another from substituted or unsubstituted alkyl, unsubstituted or substituted cycloalkyl, aryl, hydroxy, unsubstituted or substituted alkoxy, cycloalkoxy and aryloxy; R₂ and R₈ are hydrogen, unsubstituted or substituted alkyl, alkenyl or alkynyl, or heterocyclyl, and the remaining radicals are as defined, condensing an amino compound of the formula 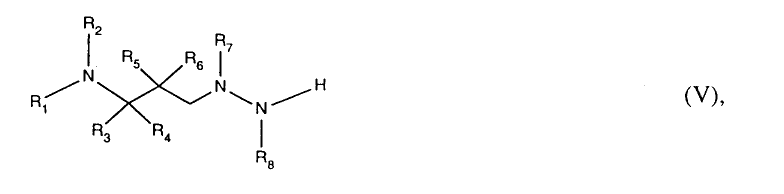 wherein the radicals are as defined immediately above, with an acid of the formula
R₉-OH (VI)
or with a reactive acid derivative thereof, wherein R₉ is as defined immediately above, and free functional groups, with the exception of those participating in the reaction, are optionally in protected form, and removing any protecting groups present, or
c) for the preparation of a compound of formula I wherein R₁ and R₉ are acyl; sulfo; sulfonyl substituted by unsubstituted or substituted alkyl, aryl, alkoxy, which is unsubstituted or substituted, or by aryloxy; sulfamoyl that is unsubstituted or substituted at the nitrogen atom; or phosphoryl substituted by one or two radicals selected independently of one another from substituted or unsubstituted alkyl, unsubstituted or substituted cycloalkyl, aryl, hydroxy, unsubstituted or substituted alkoxy, cycloalkoxy and aryloxy; R₂ and R₈ are hydrogen, unsubstituted or substituted alkyl, alkenyl or alkynyl, or heterocyclyl, and the remaining radicals are as defined, condensing an amino compound of the formula 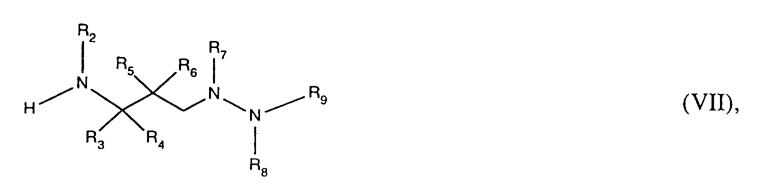 wherein the radicals are as defined immediately above, with an acid of the formula
R₁-OH (VIII),
or with a reactive acid derivative thereof, wherein R₁ is as defined immediately above, and free functional groups, with the exception of those participating in the reaction, are optionally in protected form, and removing any protecting groups present, or
d) for the preparation of a compound of formula I wherein R₁ ad R₉ are two identical radicals selected from acyl; sulfo; sulfonyl substituted by unsubstituted or substituted alkyl, aryl, alkoxy, which is unsubstituted or substituted, or by aryloxy; sulfamoyl that is unsubstituted or substituted at the nitrogen atom; and phosphoryl that is substituted by one or two radicals selected independently of one another from substituted or unsubstituted alkyl, unsubstituted or substituted cycloalkyl, aryl, hydroxy, unsubstituted or substituted alkoxy, cycloalkoxy and aryloxy; R₂ and R₈ are hydrogen, unsubstituted or substituted alkyl, alkenyl or alkynyl, or heterocyclyl, and the remaining radicals are as defined, condensing a diamino compound of formula 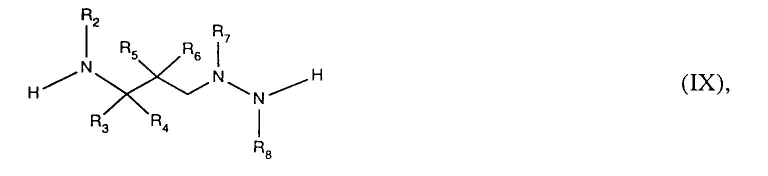 wherein the radicals are as defined immediately above, with an acid suitable for introducing the identical radicals R₁ and R₉, or with a reactive acid derivative thereof, wherein R₁ and R₉ are as defined immediately above, and free functional groups, with the exception of those participating in the reaction, are optionally in protected form, and removing any protecting groups present, or
e) for the preparation of a compound of formula I wherein in place of the radical R₇ there is a radical R₇'' which is unsubstituted or substituted alkyl or cycloalkyl, in a compound of formula I' 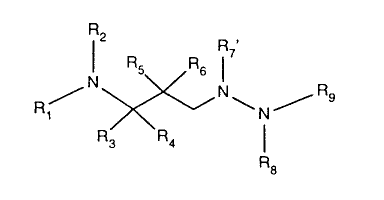 wherein R₇' is hydrogen and the remaining radicals are as defined, introducing the radical R₇'' by substitution with a compound of formula XII,
R₇''-X (XII)
wherein X is a leaving group and R₇'' is unsubstituted or substituted alkyl or cycloalkyl, and free functional groups, with the exception of those participating in the reaction, are optionally in protected form, and removing any protecting groups present, or
f) in a compound of formula I wherein the substituents are as defined above, with the proviso that in the compound of formula I in question at least one functional group is protected by protecting groups, removing the protecting groups,
and, if desired, converting a compound of formula I obtainable in accordance with any one of processes a) to f) above having at least one salt-forming group into a salt or converting an obtainable salt into the free compound or into a different salt and/or separating any isomeric mixtures that are obtainable and/or converting a compound of formula I according to the invention into a different compound of formula I according to the invention.

15. A compound of formula 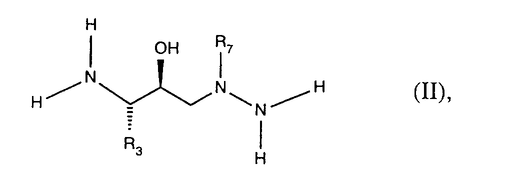 wherein R₃ is unsubstituted or substituted alkyl or cycloalkyl; aryl; heterocyclyl; or unsubstituted or substituted alkenyl; and R₇ is unsubstituted or substituted alkyl or cycloalkyl; aryl; heterocyclyl; or unsubstituted or substituted alkenyl, or a salt of such a compound where salt-forming groups are present.

## Claims (Claims for the following Contracting State(s): ES, GR)

1. A process for the preparation of a compound of formula 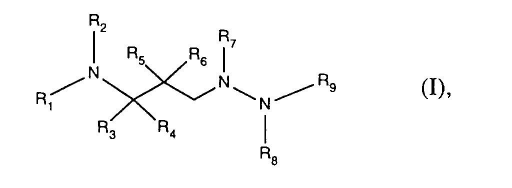 wherein R₁ and R₉ are each independently of the other hydrogen; acyl; unsubstituted or substituted alkyl, alkenyl or alkynyl; heterocyclyl; sulfo; sulfonyl substituted by unsubstituted or substituted alkyl, aryl, alkoxy, which is unsubstituted or substituted, or by aryloxy; sulfamoyl that is unsubstituted or substituted at the nitrogen atom; or phosphoryl substituted by one or two radicals selected independently of one another from unsubstituted or substituted alkyl, from unsubstituted or substituted cycloalkyl, from aryl, from hydroxy, from unsubstituted or substituted alkoxy, from cycloalkoxy and from aryloxy; with the proviso that not more than one of the radicals R₁ and R₉ is hydrogen; and R₂ and R₈ are each independently of the other hydrogen or one of the radicals mentioned above for R₁ and R₉; or the pairs of substituents R₁ and R₂, and R₈ and R₉, each independently of the other, may form, together with the nitrogen atom to which they are bonded, a heterocyclic ring consisting of the bonding nitrogen atom together with a radical selected from ethylene, trimethylene, tetramethylene and pentamethylene, in which a carbon atom may have been replaced by nitrogen, oxygen, sulfur or by sulfur mono-or di-substituted by oxygen and which may be unsaturated, or naphthalene-1,8-dicarbonylimido;
R₃ and R₄ are each independently of the other hydrogen; unsubstituted or substituted alkyl or cycloalkyl; aryl; heterocyclyl; or unsubstituted or substituted alkenyl; or R₃ and
R₄ together form unsubstituted or substituted alkylene, alkylidene or benzo-fused alkylene;
R₅ is hydroxy; R₆ is hydrogen;
or R₅ and R₆ together are oxo;
and R₇ is unsubstituted or substituted alkyl or cycloalkyl; aryl; heterocyclyl; or unsubstituted or substituted alkenyl;
or a salt of such a compound where salt-forming groups are present;
with the exception of the compound of formula I wherein R₁, R₂, R₃, R₄, R₆ and R₈ are each hydrogen, R₅ is hydroxy, R₇ is a radical of the formula -[CH₂-(C=O)-NH-(N-CH₃)-CH₂-COOH, and R₉ is tert-butoxycarbonyl, which process comprises
a) adding a hydrazine derivative of the formula 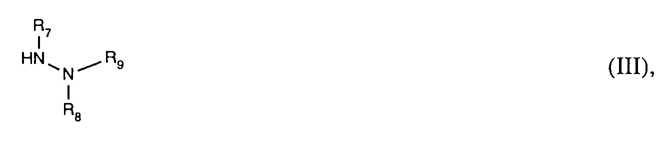 wherein the radicals are as defined above, to an epoxide of the formula 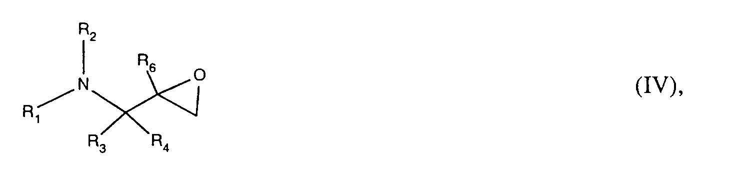 wherein the radicals are as defined above, and free functional groups, with the exception of those participating in the reaction, are optionally in protected form, and removing any protecting groups present, or
b) for the preparation of a compound of formula I wherein R₁ and R₉ are acyl; sulfo; sulfonyl substituted by unsubstituted or substituted alkyl, aryl, alkoxy, which is unsubstituted or substituted, or by aryloxy; sulfamoyl that is unsubstituted or substituted at the nitrogen atom; or phosphoryl substituted by one or two radicals selected independently of one another from substituted or unsubstituted alkyl, unsubstituted or substituted cycloalkyl, aryl, hydroxy, unsubstituted or substituted alkoxy, cycloalkoxy and aryloxy; R₂ and R₈ are hydrogen, unsubstituted or substituted alkyl, alkenyl or alkynyl, or heterocyclyl, and the remaining radicals are as defined, condensing an amino compound of the formula 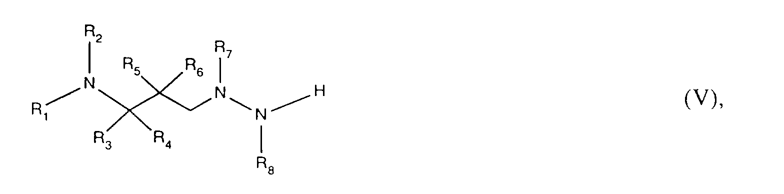 wherein the radicals are as defined immediately above, with an acid of the formula
R₉-OH (VI)
or with a reactive acid derivative thereof, wherein R₉ is as defined immediately above, and free functional groups, with the exception of those participating in the reaction, are optionally in protected form, and removing any protecting groups present, or
c) for the preparation of a compound of formula I wherein R₁ and R₉ are acyl; sulfo; sulfonyl substituted by unsubstituted or substituted alkyl, aryl, alkoxy, which is unsubstituted or substituted, or by aryloxy; sulfamoyl that is unsubstituted or substituted at the nitrogen atom; or phosphoryl substituted by one or two radicals selected independently of one another from substituted or unsubstituted alkyl, unsubstituted or substituted cycloalkyl, aryl, hydroxy, unsubstituted or substituted alkoxy, cycloalkoxy and aryloxy; R₂ and R₈ are hydrogen, unsubstituted or substituted alkyl, alkenyl or alkynyl, or heterocyclyl, and the remaining radicals are as defined, condensing an amino compound of the formula 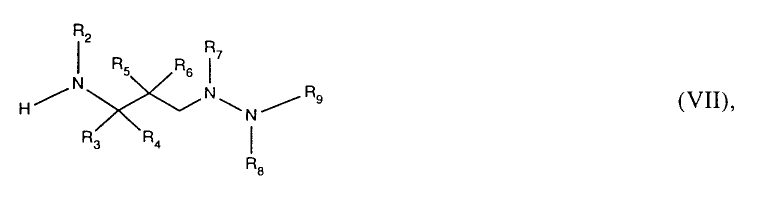 wherein the radicals are as defined immediately above, with an acid of the formula
R₁-OH (VIII),
or with a reactive acid derivative thereof, wherein R₁ is as defined immediately above, and free functional groups, with the exception of those participating in the reaction, are optionally in protected form, and removing any protecting groups present, or
d) for the preparation of a compound of formula I wherein R₁ and R₉ are two identical radicals selected from acyl; sulfo; sulfonyl substituted by unsubstituted or substituted alkyl, aryl, alkoxy, which is unsubstituted or substituted, or by aryloxy; sulfamoyl that is unsubstituted or substituted at the nitrogen atom; and phosphoryl that is substituted by one or two radicals selected independently of one another from substituted or unsubstituted alkyl, unsubstituted or substituted cycloalkyl, aryl, hydroxy, unsubstituted or substituted alkoxy, cycloalkoxy and aryloxy; R₂ and R₈ are hydrogen, unsubstituted or substituted alkyl, alkenyl or alkynyl, or heterocyclyl, and the remaining radicals are as defined, condensing a diamino compound of formula 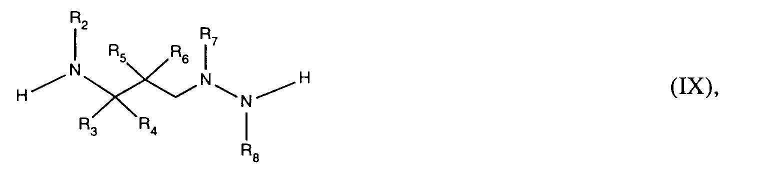 wherein the radicals are as defined immediately above, with an acid suitable for introducing the identical radicals R₁ and R₉, or with a reactive acid derivative thereof, wherein R₁ and R₉ are as defined immediately above, and free functional groups, with the exception of those participating in the reaction, are optionally in protected form, and removing any protecting groups present, or
e) for the preparation of a compound of formula I wherein in place of the radical R₇ there is a radical R₇'' which is unsubstituted or substituted alkyl or cycloalkyl, in a compound of formula I' 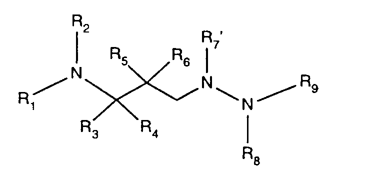 wherein R₇' is hydrogen and the remaining radicals are as defined, introducing the radical R₇'' by substitution with a compound of formula XII,
R₇''-X (XII)
wherein X is a leaving group and R₇'' is unsubstituted or substituted alkyl or cycloalkyl, and free functional groups, with the exception of those participating in the reaction, are optionally in protected form, and removing any protecting groups present, or
f) in a compound of formula I wherein the substituents are as defined above, with the proviso that in the compound of formula I in question at least one functional group is protected by protecting groups, removing the protecting groups,
and, if desired, converting a compound of formula I obtainable in accordance with any one of processes a) to f) above having at least one salt-forming group into a salt or converting an obtainable salt into the free compound or into a different salt and/or separating any isomeric mixtures that are obtainable and/or converting a compound of formula I according to the invention into a different compound of formula I according to the invention.

2. A process according to claim 1 for the preparation of a compound of formula I wherein
R₁ and R₉ are each independently of the other hydrogen; lower alkanoyl; aryl-lower alkanoyl wherein lower alkanoyl is unsubstituted or substituted by carbamoyl or by carbamoyl substituted at the nitrogen atom by one or two radicals selected from lower alkyl, carboxy-lower alkyl, lower alkoxycarbonyl-lower alkyl, di-lower alkylamino-lower alkyl, amino-carboxy-lower alkyl, hydroxy-lower alkyl and di-lower alkoxy-lower alkyl; heterocyclyl-lower alkanoyl; halo-lower alkanoyl containing up to 3 halogen atoms; (N-heterocyclyl-lower alkylcarbamoyl)-lower alkanoyl; lower alkoxycarbonyl; aryl-lower alkoxycarbonyl; heterocyclyl-lower alkoxycarbonyl; lower alkylsulfonyl; N-heterocyclyl-lower alkyl-N-lower alkylcarbamoyl; or an acyl residue of an amino acid the amino function of which is free or has been acylated by one of the other radicals mentioned hitherto for R₁ and R₉; with the proviso that not more than one of the radicals R₁ and R₉ is hydrogen;
R₂, R₄, R₆ and R₈ are hydrogen;
R₃ is lower alkyl, cycloalkyl-lower alkyl or aryl-lower alkyl,
R₅ is hydroxy, and
R₇ is unsubstituted or substituted lower alkyl,
or a salt thereof where at least one salt-forming group is present, the term "lower" in the above definitions indicating that the radical in question has up to and including 7 carbon atoms, which process comprises using appropriately substituted starting materials.

3. A process according to claim 1 for the preparation of a compound of formula I wherein
R₁ and R₉ are each independently of the other hydrogen; lower alkanoyl; aryl-lower alkanoyl wherein aryl is phenyl, indenyl, indanyl, naphthyl, anthryl, phenanthryl, acenaphthyl or fluorenyl and may be unsubstituted or mono- to tri-substituted by lower alkyl, halo-lower alkyl, phenyl, 1- or 2-naphthyl, hydroxy, lower alkoxy, carbamoyl-lower alkoxy, N-lower alkylcarbamoyl-lower alkoxy, N,N-di-lower alkylcarbamoyl-lower alkoxy, amino, mono- or di-lower alkylamino, lower alkanoylamino, halogen, carboxy, lower alkoxycarbonyl, phenyl-, naphthyl- or fluorenyl-lower alkoxycarbonyl, lower alkanoyl, sulfo, lower alkylsulfonyl, phosphono, hydroxy-lower alkoxyphosphoryl, di-lower alkoxyphosphoryl, carbamoyl, mono- or di-lower alkylcarbamoyl, sulfamoyl, mono-or di-lower alkylaminosulfonyl, nitro and/or by cyano, wherein phenyl may be present up to three times, and wherein lower alkanoyl is unsubstituted or substituted by carbamoyl or by carbamoyl substituted at the nitrogen atom by one or two radicals selected from lower alkyl, carboxy-lower alkyl, lower alkoxycarbonyl-lower alkyl, di-lower alkylamino-lower alkyl, amino-carboxy-lower alkyl, hydroxy-lower alkyl and di-lower alkoxy-lower alkyl; heterocyclyl-lower alkanoyl wherein lower alkanoyl is unsubstituted and wherein heterocyclyl is selected from thienyl, furyl, pyranyl, pyrrolyl, imidazolyl, pyrazolyl, oxazolyl, isoxazolyl, thiazolyl, furazanyl, pyridyl, pyrazinyl, pyrimidinyl, pyridazinyl, azepinyl, indolyl, benzimidazolyl, 1H-indazolyl, quinolyl, isoquinolyl, quinoxalinyl, quinazolinyl, cinnolyl, purinyl, pteridinyl, naphthyridinyl, 4H-quinolizinyl, 3,1-benzofuranyl, 4,1-benzoxazinyl, 4,1-benzothiazinyl, cyclohexa[b]pyrrolyl, cyclohepta[b]pyrrolyl, cyclohexa[d]pyrazolyl, cyclohexa[b]pyridyl, cyclohexa[b]pyrazinyl, cyclohexa[b]pyrimidinyl, cyclohexa[b]-1,4-oxazinyl, cyclohexa[b]-1,4-thiazinyl, pyrrolidinyl, pyrrolinyl, imidazolidyl, 2-imidazolinyl, 2,3-dihydropyridyl, piperidyl, piperazinyl, 2,3,5,6-tetrahydropyrazinyl, morpholinyl, thiomorpholinyl, S,S-dioxo-thiomorpholinyl, indolinyl, isoindolinyl, 4,5,6,7-tetrahydroindolyl, 1,2,3,4-tetrahydroquinolyl, 1,2,3,4-tetrahydroisoquinolyl, chromanyl, thiochromanyl, 1,2,3,4-tetrahydro-3,1-benzodiazinyl, 3,4-dihydro-3H-4,1-benzoxazinyl and 3,4-dihydro-3H-4,1-benzothiazinyl, the mentioned heterocyclyl radicals being unsubstituted or substituted by lower alkyl, phenyl, 1-or 2-naphthyl, phenyl-lower alkyl, hydroxy-lower alkyl, lower alkoxy-lower alkyl, phenoxy-or naphthoxy-lower alkyl, phenyl-lower alkoxy- or naphthyl-lower alkoxy-lower alkyl, lower alkanoyloxy-lower alkyl, phenyl- or naphthyl-lower alkanoyloxy-lower alkyl, lower alkoxycarbonyloxy-lower alkyl, phenyl-, naphthyl- or fluorenyl-lower alkoxycarbonyloxy-lower alkyl, amino-lower alkyl, carboxy-lower alkyl, hydroxy, lower alkoxy, phenyl-or naphthyl-lower alkoxy, amino, lower alkylamino, di-lower alkylamino, carboxy, lower alkoxycarbonyl, phenyl- or naphthyl-lower alkoxycarbonyl, halogen, lower alkanoyl, lower alkylsulfonyl, phosphono, hydroxy-lower alkoxyphosphoryl or dialkoxyphosphoryl, carbamoyl, mono- or di-lower alkylcarbamoyl, hydroxy- or carboxy-lower alkylcarbamoyl, sulfamoyl, nitro, oxo and/or by cyano; heterocyclyl-lower alkanoyl, such as halo-lower alkanoyl containing up to three halogen atoms; (N-heterocyclyl-lower alkylcarbamoyl)-lower alkanoyl wherein heterocyclyl is selected from pyrrolyl, furyl, thienyl, imidazolyl, pyrazolyl, oxazolyl, thiazolyl, pyridyl, pyrazinyl, pyrimidinyl, indolyl, quinolyl, isoquinolyl, quinoxalinyl, β-carbolinyl and a benzo-fused, cyclopenta-, cyclohexa- or cyclohepta-fused derivative of those radicals, which may also be fully or partially saturated, from morpholine and from thiomorpholine; lower alkoxycarbonyl; aryl-lower alkoxycarbonyl wherein aryl is phenyl, biphenylyl, 1- or 2-naphthyl, fluorenyl, or phenyl mono- or poly-substituted by lower alkyl, hydroxy, lower alkoxy, halogen and/or by nitro; heterocyclyl-lower alkoxycarbonyl wherein heterocyclyl is selected from pyrrolyl, furyl, thienyl, imidazolyl, pyrazolyl, oxazolyl, thiazolyl, pyridyl, pyrazinyl, pyrimidinyl, indolyl, quinolyl, isoquinolyl, quinoxalinyl, β-carbolinyl and a benzo-fused, cyclopenta-, cyclohexa- or cyclohepta-fused derivative of those radicals, which may also be fully or partially saturated; lower alkylsulfonyl; N-heterocyclyl-lower alkyl-N-lower alkylcarbamoyl wherein heterocyclyl is selected from thienyl, furyl, pyranyl, pyrrolyl, imidazolyl, pyrazolyl, oxazolyl, isoxazolyl, thiazolyl, furazanyl, pyridyl, pyrazinyl, pyrimidinyl, pyndazinyl, azepinyl, indolyl, benzimidazolyl, 1H-indazolyl, quinolyl, isoquinolyl, quinoxalinyl, quinazolinyl, cinnolyl, purinyl, pteridinyl, naphthyridinyl, 4H-quinolizinyl, 3,1-benzofuranyl, benz[e]indolyl, 4,1-benzoxazinyl, 4,1-benzothiazinyl, carbazolyl, β-carbolinyl, phenazinyl, phenanthridyl, acridyl, phenoxazinyl, phenothiazinyl, 1-azaacenaphthenyl, cyclohexa[b]pyrrolyl, cyclohepta[b]pyrrolyl, cyclohexa[d]pyrazolyl, cyclohexa[b]pyridyl, cyclohexa[b]pyrazinyl, cyclohexa[b]pyrimidinyl, cyclohexa[b]-1,4-oxazinyl, cyclohexa[b]-1,4-thiazinyl, pyrrolidinyl, pyrrolinyl, imidazolidinyl, 2-imidazolinyl, 2,3-dihydropyridyl, piperidyl, piperazinyl, 2,3,5,6-tetrahydropyrazinyl, morpholinyl, thiomorpholinyl, S,S-dioxothiomorpholinyl, indolinyl, isoindolinyl, 4,5,6,7-tetrahydroindolyl, 1,2,3,4-tetrahydroquinolyl, 1,2,3,4-tetrahydroisoquinolyl, chromanyl, thiochromanyl, 1,2,3,4-tetrahydro-3,1-benzodiazinyl, 3,4-dihydro-3H-4,1-benzoxazinyl, 3,4-dihydro-3H-4,1-benzothiazinyl, 2,3,4,5-tetrahydro-1H-5,1-benzazepinyl and 5,6-dihydrophenanthridinyl, the mentioned radicals being unsubstituted or substituted by lower alkyl, phenyl, 1- or 2-naphthyl, phenyl-lower alkyl, hydroxy-lower alkyl, lower alkoxy-lower alkyl, phenoxy- or naphthoxy-lower alkyl, phenyl-lower alkoxy- or naphthyl-lower alkoxy-lower alkyl, lower alkanoyloxy-lower alkyl, phenyl-or naphthyl-lower alkanoyloxy-lower alkyl, lower alkoxycarbonyloxy-lower alkyl, phenyl-, naphthyl- or fluorenyl-lower alkoxycarbonyloxy-lower alkyl, amino-lower alkyl, carboxy-lower alkyl, hydroxy, lower alkoxy, phenyl- or naphthyl-lower alkoxy, amino, lower alkylamino, di-lower alkylamino, carboxy, lower alkoxycarbonyl, phenyl- or naphthyl-lower alkoxycarbonyl, halogen, lower alkanoyl, lower alkylsulfonyl, phosphono, hydroxy-lower alkoxyphosphoryl or dialkoxyphosphoryl, carbamoyl, mono- or di-lower alkylcarbamoyl, hydroxy- or carboxy-lower alkylcarbamoyl, sulfamoyl, nitro, oxo and/or by cyano; or an acyl radical of an amino acid the amino function of which is free or has been acylated by one of the other radicals mentioned hitherto for R₁ and R₉, the amino acids being selected from glycine, alanine, valine, norvaline, leucine, isoleucine, norleucine, serine, homoserine, threonine, methionine, cysteine, proline, trans-3- and trans-4-hydroxyproline, phenylalanine, tyrosine, 4-aminophenylalanine, 4-chlorophenylalanine, 4-carboxyphenylalanine, β-phenylserine, phenylglycine, α-naphthylalanine, cyclohexylalanine, cyclohexylglycine, tryptophan, aspartic acid, asparagine, aminomalonic acid, aminomalonic acid monoamide, glutamic acid, glutamine, histidine, arginine, lysine, δ-hydroxylysine, ornithine, 3-aminopropanoic acid, α,γ-diaminobutyric acid and α,β-diaminopropionic acid, wherein each of the mentioned amino acid residues (with the exception of glycine) is in the D-, L- or (D,L)-form; with the proviso that not more than one of the radicals R₁ and R₉ is hydrogen,
R₂, R₄, R₆ and R₈ are hydrogen,
R₃ is lower alkyl; cycloalkyl-lower alkyl wherein cycloalkyl has from 3 to 7 carbon atoms and is unsubstituted or mono- to tri-substituted by lower alkyl, halo-lower alkyl, hydroxy, lower alkoxy, carbamoyl-lower alkoxy, N-lower alkylcarbamoyl-lower alkoxy, N,N-di-lower alkylcarbamoyl-lower alkoxy, amino, mono- or di-lower alkylamino, halogen, carboxy, lower alkoxycarbonyl, phenyl-, naphthyl- or fluorenyl-lower alkoxycarbonyl, lower alkanoyl, lower alkylsulfonyl, phosphono, hydroxy-lower alkoxyphosphoryl or di-lower alkoxyphosphoryl, carbamoyl, mono- or di-lower alkylaminocarbamoyl, sulfamoyl, mono- or di-lower alkylsulfamoyl, nitro and/or by cyano; or is aryl-lower alkyl wherein aryl is phenyl, indenyl, indanyl, naphthyl, anthryl, phenanthryl, acenaphthyl or fluorenyl and may be unsubstituted or mono- to tri-substituted by lower alkyl, halo-lower alkyl, phenyl, 1- or 2-naphthyl, hydroxy, lower alkoxy, carbamoyl-lower alkoxy, N-lower alkylcarbamoyl-lower alkoxy, N,N-di-lower alkylcarbamoyl-lower alkoxy, amino, mono- or di-lower alkylamino, lower alkanoylamino, halogen, carboxy, lower alkoxycarbonyl, benzyl-, naphthyl- or fluorenyl-lower alkoxycarbonyl, lower alkanoyl, sulfo, lower alkylsulfonyl, phosphono, hydroxy-lower alkoxyphosphoryl, di-lower alkoxyphosphoryl, carbamoyl, mono- or di-lower alkylcarbamoyl, sulfamoyl, mono-or di-lower alkylsulfamoyl, nitro and/or by cyano, wherein phenyl may be present up to three times,
R₅ is hydroxy, and
R₇ is unsubstituted lower alkyl; or cycloalkyl-lower alkyl as last described for cycloalkyl-lower alkyl R₃; or aryl-lower alkyl as last described for aryl-lower alkyl R₃;
or a salt thereof where at least one salt-forming group is present, the term "lower" in the above definitions indicating that the radical in question has up to and including 7 carbon atoms, which process comprises using appropriately substituted starting materials.

4. A process according to claim 1 for the preparation of a compound of formula I wherein
R₁ and R₉ are each independently of the other
- hydrogen,
- lower alkoxycarbonyl,
- 2-halo-lower alkoxycarbonyl,
- aryloxycarbonyl wherein aryl has from 6 to 14 carbon atoms,
- aryl-lower alkoxycarbonyl wherein aryl has from 6 to 14 carbon atoms,
- heterocyclyl-lower alkoxycarbonyl wherein heterocyclyl is selected from pyrrolyl, furyl, thienyl, imidazolyl, pyrazolyl, oxazolyl, thiazolyl, pyridyl, pyrazinyl, pyrimidinyl, indolyl, quinolyl, isoquinolyl, quinoxalinyl, β-carbolinyl and a benzo-fused, cyclopenta-, cyclohexa- or cyclohepta-fused derivative of those radicals, which may also be fully or partially saturated,
- 2-tri-lower alkylsilyl-lower alkoxycarbonyl,
- 2-triarylsilyl-lower alkoxycarbonyl wherein aryl is phenyl or 1- or 2-naphthyl,
- the residue, bonded via the carboxy group, of an amino acid selected from glycine, alanine, valine, norvaline, leucine, isoleucine, norleucine, serine, homoserine, threonine, methionine, cysteine, proline, trans-3- and trans-4-hydroxyproline, phenylalanine, tyrosine, 4-aminophenylalanine, 4-chlorophenylalanine, 4-carboxyphenylalanine, β-phenylserine, phenylglycine, α-naphthylalanine, cyclohexylalanine, cyclohexylglycine, tryptophan, indoline-2-carboxylic acid, 1,2,3,4-tetrahydroisoquinoline-3-carboxylic acid, aspartic acid, asparagine, aminomalonic acid, aminomalonic acid monoamide, glutamic acid, glutamine, histidine, arginine, lysine, δ-hydroxylysine, ornithine, α,γ-diaminobutyric acid and α,β-diaminopropionic acid, wherein each of those amino acids may be in the D-, L- or (D,L)-form, and
the α-amino group may be unsubstituted or N-alkylated one or two times by lower alkyl, by amino-lower alkyl, by phenyl- or naphthyl-amino-lower alkyl, or by piperazinylcarbonyl-lower alkyl substituted at the nitrogen atom by lower alkyl, or N-acylated by lower alkanoyl, by aryl-lower alkanoyl wherein aryl is selected from phenyl, indenyl, indanyl, naphthyl, anthryl, phenanthryl, acenaphthyl and fluorenyl and may be unsubstituted or mono- to tri-substituted by lower alkyl, halo-lower alkyl, phenyl, 1-or 2-naphthyl, hydroxy, lower alkoxy, carbamoyl-lower alkoxy, N-lower alkylcarbamoyl-lower alkoxy or N,N-di-lower alkylcarbamoyl-lower alkoxy, amino, mono- or di-lower alkylamino, lower alkanoylamino, halogen, carboxy, lower alkoxycarbonyl, phenyl-, naphthyl- or fluorenyl-lower alkoxycarbonyl, lower alkanoyl, sulfo, lower alkylsulfonyl, phosphono, hydroxy-lower alkoxyphosphoryl or di-lower alkoxyphosphoryl, carbamoyl, mono- or di-lower alkylcarbamoyl, sulfamoyl, mono- or di-lower alkylaminosulfonyl, nitro and/or by cyano; wherein phenyl may be present up to three times, and wherein lower alkanoyl is branched or unbranched and may be unsubstituted or substituted by lower alkyl; heterocyclyl selected from pyrrolyl, furyl, thienyl, imidazolyl, pyrazolyl, oxazolyl, thiazolyl, pyridyl, pyrazinyl, pyrimidinyl, indolyl, quinolyl, isoquinolyl, quinoxalinyl, β-carbolinyl and a benzo-, cyclopenta-, cyclohexa- or cyclohepta-fused derivative of those radicals, which may also be fully or partially saturated; hydroxy; lower alkoxy; lower alkanoyloxy; acetoacetoxy; amino- or benzyloxycarbonylamino-lower alkanoyloxy; aryl-lower alkanoyloxy wherein aryl has from 6 to 10 carbon atoms; lower alkoxycarbonyloxy; mono- or di-lower alkylaminocarbonyloxy; aryloxycarbonyloxy wherein aryl has from 6 to 10 carbon atoms; aryl-lower alkoxycarbonyloxy wherein aryl has from 6 to 14 carbon atoms; sulfonyloxy; lower alkylsulfonyloxy; phenylsulfonyloxy; 2- or 4-toluenesulfonyloxy or 1- or 2- naphthylsulfonyloxy; carboxy; esterified carboxy selected from lower alkoxycarbonyl, aryloxycarbonyl wherein aryl has from 6 to 10 carbon atoms, aryl-lower alkoxycarbonyl wherein aryl has from 6 to 12 carbon atoms; lower alkanoyl; lower alkylsulfonyl; hydroxy-lower alkoxyphosphoryl and di-lower alkoxyphosphoryl; carbamoyl; carbamoyl substituted by one or two radicals selected from lower alkyl, carboxy-lower alkyl, lower alkoxycarbonyl-lower alkyl, di-lower alkylamino-lower alkyl, aminocarboxy-lower alkyl, hydroxy-lower alkyl and di-lower alkoxy-lower alkyl, or by one radical selected from ethylene, trimethylene, tetramethylene and pentamethylene, in which a carbon atom may have been replaced by nitrogen, oxygen, sulfur or by sulfur mono- or di-substituted by oxygen and which may be unsaturated; also sulfamoyl; phosphono; benzofuranyl; oxo and/or by cyano; by heterocyclyl-lower alkanoyl selected from thienyl-, furyl-, pyranyl:, pyrrolyl-, imidazolyl-, pyrazolyl-, oxazolyl-, isoxazolyl-, thiazolyl-, furazanyl-, pyridyl-, pyrazinyl-, pyrimidinyl-, pyridazinyl-, azepinyl-, indolyl-, benzimidazolyl-, 1H-indazolyl-, quinolyl-, isoquinolyl-, quinoxalinyl-, quinazolinyl-, cinnolyl-, purinyl-, pteridinyl-, naphthyridinyl-, 4H-quinolizinyl-, 3,1-bensofuranyl-, bens[e]indolyl-, 4,1-benzoxazinyl-, 4,1-benzothiazinyl-, carbazolyl-, β-carbolinyl-, phenazinyl-, phenanthridyl-, acridyl-, phenoxazinyl-, phenothiazinyl-, 1-azaacenaphthenyl-, cyclohexa[b]pyrrolyl-, cyclohepta[b]pyrrolyl-, cyclohexa[d]pyrazolyl-, cyclohexa[b]pyridyl-, cyclohexa[b]pyrazinyl-, cyclohexa[b]pyrimidinyl-, cyclohexa[b]-1,4-oxazinyl-, cyclohexa[b]-1,4-thiazinyl-, pyrrolidinyl-, pyrrolinyl-, imidazolidinyl-, 2-imidazolinyl-, 2,3-dihydropyridyl-, piperidyl-, piperazinyl-, 2,3,5,6-tetrahydropyrazinyl-, morpholinyl-, thiomorpholinyl-, S,S-dioxothiomorpholinyl-, indolinyl-, isoindolinyl-, 4,5,6,7-tetrahydroindolyl-, 1,2,3,4-tetrahydroquinolyl-, 1,2,3,4-tetrahydroisoquinolyl-, chroman-, thiochroman-, 1,2,3,4-tetrahydro-3,1-benzodiazinyl-, 3,4-dihydro-3H-4,1-benzoxazinyl-, 3,4-dihydro-3H-4,1-benzothiazinyl-, 2,3,4,5-tetrahydro-1H-5,1-benzazepinyl- and 5,6-dihydrophenanthridinyl-lower alkanoyl, the mentioned heterocyclyl radicals being unsubstituted or substituted by lower alkyl, phenyl, 1- or 2-naphthyl, phenyl-lower alkyl, hydroxy-lower alkyl, lower alkoxy-lower alkyl, phenoxy- or naphthoxy-lower alkyl, phenyl-lower alkoxy- or naphthyl-lower alkoxy-lower alkyl, lower alkanoyloxy-lower alkyl, phenyl- or naphthyl-lower alkanoyloxy-lower alkyl, lower alkoxycarbonyloxy-lower alkyl, phenyl-, naphthyl- or fluorenyl-lower alkoxycarbonyloxy-lower alkyl, amino-lower alkyl, carboxy-lower alkyl, hydroxy, lower alkoxy, phenyl- or naphthyl-lower alkoxy, amino, lower alkylamino, di-lower alkylamino, carboxy, lower alkoxycarbonyl, phenyl- or naphthyl-lower alkoxycarbonyl, halogen, lower alkanoyl, lower alkylsulfonyl, phosphono, hydroxy-lower alkoxyphosphoryl, dialkoxyphosphoryl, carbamoyl, mono- or di-lower alkylcarbamoyl, hydroxy-or carboxy-lower alkylcarbamoyl, sulfamoyl, nitro, oxo and/or by cyano; by heterocyclyl-lower alkenoyl wherein heterocyclyl is selected from pyrrolyl, furyl, thienyl, imidazolyl, pyrazolyl, oxazolyl, thiazolyl, pyridyl, pyrazinyl, pyrimidinyl, indolyl, quinolyl, isoquinolyl, quinoxalinyl, β-carbolinyl and a benzo-fused, cyclopenta-, cyclohexa- or cyclohepta-fused derivative of those radicals, which may also be fully or partially saturated; by lower alkoxycarbonyl; by aryl-lower alkoxycarbonyl wherein aryl has from 6 to 14 carbon atoms; by heterocyclyl-lower alkoxycarbonyl wherein heterocyclyl is selected from pyrrolyl, furyl, thienyl, imidazolyl, pyrazolyl, oxazolyl, thiazolyl, pyridyl, pyrazinyl, pyrimidinyl, indolyl, quinolyl, isoquinolyl, quinoxalinyl, β-carbolinyl and a benzo-fused, cyclopenta-, cyclohexa- or cyclohepta-fused derivative of those radicals, which may also be fully or partially saturated and unsubstituted or substituted by lower alkyl; by carboxy-lower alkanoyl; by lower alkoxycarbonyl-lower alkanoyl; by hydroxy-lower alkoxy-lower alkanoyl; by amino-lower alkanoyl or by benzyloxycarbonylamino-lower alkanoyl wherein the amino group is not bonded in the α- or β-position; by carbamoyl; by phenyl-lower alkylaminocarbonyl; by N-di-lower alkylamino-lower alkyl-N-lower alkylaminocarbonyl; by N-dihydroxy-lower alkyl-N-lower alkylaminocarbonyl; by 2- or 3-pyridyl-lower alkylaminocarbonyl; by N-2-pyridyl-lower alkyl-N-lower alkylaminocarbonyl; by sulfonyl; by lower alkylsulfonyl; by arylsulfonyl wherein aryl has from 6 to 10 carbon atoms and is unsubstituted or substituted by lower alkyl or by lower alkoxy; by heterocyclylsulfonyl wherein heterocyclyl is selected from pyrrolyl, furyl, thienyl, imidazolyl, pyrazolyl, oxazolyl, thiazolyl, pyridyl, pyrazinyl, pyrimidinyl, indolyl, quinolyl, isoquinolyl, quinoxalinyl, β-carbolinyl and a benzo-fused, cyclopenta-, cyclohexa-or cyclohepta-fused derivative of those radicals, which may also be fully or partially saturated; by sulfamoyl or by sulfamoyl substituted by heterocyclyl-lower alkyl, wherein heterocyclyl is as last defined, and/or by lower alkyl,
a carboxy group of the side chain is present in free form or in esterified form in the form of a lower alkyl ester group, an aryl ester group or an aryl-lower alkyl ester group, wherein aryl is phenyl, 4-nitrophenyl, naphthyl or biphenylyl, or is present in amidated form in the form of a carbamoyl, lower alkylcarbamoyl, di-lower alkylaminocarbamoyl, mono-or di-(hydroxy-lower alkyl)carbamoyl or mono- or di-(carboxy-lower alkyl)carbamoyl group,
an amino group of the side chain is present in free form or in alkylated form in the form of a mono- or di-lower alkylamino group or is present in acylated form in the form of a lower alkanoylamino group, an amino-lower alkanoylamino group, an aryl-lower alkanoylamino group wherein aryl has from 6 to 14 carbon atoms and is unsubstituted or substituted by lower alkyl, hydroxy, lower alkoxy, carboxy, carbamoyl or by sulfamoyl, a lower alkoxycarbonylamino group, an arylmethoxycarbonylamino group wherein aryl has from 6 to 14 carbon atoms, a piperidyl-1-carbonyl group, a morpholinocarbonyl group, a thiomorpholinocarbonyl group or an S,S-dioxothiomorpholinocarbonyl group and/or
a hydroxy group of the side chain is present in free form or in etherified or esterified form in the form of a lower alkoxy, aryl-lower alkoxy, lower alkanoyloxy or lower alkyloxycarbonyloxy group,
- lower alkylsulfonyl,
- 2- or 3-pyrrolyl-, 2-thienyl-, 2-furyl-, 1-pyrazolyl-, 2-, 3- or 4-pyridyl-, 2-,3- or 5-indolyl-, (1-methyl-, 2-methyl-, 5-methoxy-, 5-benzyloxy-, 5-chloro- or 4,5-dimethylindol-2-yl)-, (1-benzylindol-2-yl or -3-yl)-, 4,5,6,7-tetrahydroindol-2-yl-, (2-,3- or 4-quinolyl or 4-hydroxyquinol-2-yl)-, (1-, 3- or 4-isoquinolyl or 1-oxo-1,2-dihydroisoquinol-3-yl)-, 3-pyrrolidinyl-, (3- or 4-hydroxypyrrolidin-2-yl)-, 5-oxopyrrolidin-2-yl-, (2- or 3-morpholinyl)-, (2- or 3-thiomorpholinyl)-, (S,S-dioxothiomorpholin-2- or -3-yl)-, (2- or 3-indolinyl)-, (1,2,3,4-tetrahydroquinol-2-, -3- or -4-yl)- or (1,2,3,4-tetrahydroisoquinol-1-, -2- or -3-yl)-methylsulfonyl,
- phenyl- or 1- or 2-naphthyl-sulfonyl unsubstituted or mono- or di-substituted by lower alkyl,
- lower alkoxysulfonyl, or
- benzyloxysulfonyl or 1- or 2-naphthyloxysulfonyl,
such that not more than one of the radicals R₁ and R₉ may be hydrogen, and
R₂ and R₈ are each independently of the other hydrogen or the same radicals as R₁ and R₉, or the pairs of substituents R₁ and R₉, and R₂ and R₈, each independently of the other, form together with the bonding nitrogen atom and a radical selected from ethylene, trimethylene, tetramethylene and pentamethylene in which a carbon atom may have been replaced by nitrogen, oxygen, sulfur or by sulfur mono- or di-substituted by oxygen and which may be unsaturated, a heterocyclic ring,
R₃ is
- cycloalkyl-lower alkyl wherein cycloalkyl has from 3 to 7 carbon atoms and is unsubstituted or mono- to tri-substituted by lower alkyl, halo-lower alkyl, hydroxy, lower alkoxy, carbamoyl-lower alkoxy, N-lower alkylcarbamoyl-lower alkoxy or N,N-di-lower alkylcarbamoyl-lower alkoxy, amino, mono- or di-lower alkylamino, halogen, carboxy, lower alkoxycarbonyl, phenyl-, naphthyl- or fluorenyl-lower alkoxycarbonyl, lower alkanoyl, lower alkylsulfonyl, phosphono, hydroxy-lower alkoxyphosphoryl or di-lower alkoxyphosphoryl, carbamoyl, mono- or di-lower alkylaminocarbamoyl, sulfamoyl, mono-or di-lower alkylsulfamoyl, nitro and/or by cyano and is bonded to lower alkyl, or
- aryl-lower alkyl wherein aryl is selected from phenyl, indenyl, indanyl, naphthyl, anthryl, phenanthryl, acenaphthyl and fluorenyl and may be unsubstituted or mono-to tri-substituted by lower alkyl, halo-lower alkyl, phenyl, 1- or 2-naphthyl, hydroxy, lower alkoxy, carbamoyl-lower alkoxy, N-lower alkylcarbamoyl-lower alkoxy or N,N-di-lower alkylcarbamoyl-lower alkoxy, amino, mono- or di-lower alkylamino, lower alkanoylamino, halogen, carboxy, lower alkoxycarbonyl, phenyl-, naphthyl- or fluorenyl-lower alkoxycarbonyl, lower alkanoyl, sulfo, lower alkylsulfonyl, phosphono, hydroxy-lower alkoxyphosphoryl or di-lower alkoxyphosphoryl, carbamoyl, mono- or di-lower alkylcarbamoyl, sulfamoyl, mono- or di-lower alkylsulfamoyl, nitro and/or by cyano, wherein phenyl may be present up to three times, and wherein lower alkyl is unbranched or branched and is unsubstituted or substituted by lower alkyl; heterocyclyl selected from pyrrolyl, furyl, thienyl, imidazolyl, pyrazolyl, oxazolyl, thiazolyl, pyridyl, pyrazinyl, pyrimidinyl, indolyl, quinolyl, isoquinolyl, quinoxalinyl, β-carbolinyl and a benzo-fused, cyclopenta-, cyclohexa- or cyclohepta-fused derivative of those radicals, which may also be fully or partially saturated; hydroxy; lower alkoxy; lower alkanoyloxy; acetoacetoxy; amino- or benzyloxycarbonylamino-lower alkanoyloxy; aryl-lower alkanoyloxy wherein aryl has from 6 to 10 carbon atoms; lower alkoxycarbonyloxy; mono- or di-lower alkylaminocarbonyloxy; aryloxycarbonyloxy wherein aryl has from 6 to 10 carbon atoms; aryl-lower alkoxycarbonyloxy wherein aryl has from 6 to 12 carbon atoms; sulfonyloxy; lower alkylsulfonyloxy; phenylsulfonyloxy; 2- or 4-toluenesulfonyloxy or 1- or 2-naphthylsulfonyloxy; amino; mono- or di-lower alkylamino; N-lower alkoxy-N-lower alkylamino; mono- or di-(phenyl- or naphthyl-lower alkyl)amino; lower alkanoylamino; carboxy; esterified carboxy selected from lower alkoxycarbonyl, aryloxycarbonyl wherein aryl has from 6 to 10 carbon atoms, aryl-lower alkoxycarbonyl wherein aryl has from 6 to 12 carbon atoms; lower alkanoyl; lower alkylsulfonyl; hydroxy-lower alkoxyphosphoryl and di-lower alkoxyphosphoryl; carbamoyl; carbamoyl substituted by one or two radicals selected from lower alkyl, carboxy-lower alkyl or lower alkoxycarbonyl-lower alkyl, di-lower alkylamino-lower alkyl, aminocarboxy-lower alkyl, hydroxy-lower alkyl and di-lower alkoxy-lower alkyl, or by one radical selected from ethylene, trimethylene, tetramethylene and pentamethylene, in which a carbon atom may have been replaced by nitrogen, oxygen, sulfur or by sulfur mono- or di-substituted by oxygen and which may be unsaturated; also sulfamoyl; phosphono; benzofuranyl; oxo and/or by cyano,
R₄ is hydrogen,
R₅ is hydroxy and
R₆ is hydrogen,
or R₅ and R₆ together are oxo, and
R₇ is
- lower alkyl,
- cycloalkyl-lower alkyl wherein cycloalkyl has from 3 to 7 carbon atoms, is unsubstituted or mono- to tri-substituted by lower alkyl, halo-lower alkyl, hydroxy, lower alkoxy, carbamoyl-lower alkoxy, N-lower alkylcarbamoyl-lower alkoxy or N,N-di-lower alkylcarbamoyl-lower alkoxy, amino, mono- or di-lower alkylamino, halogen, carboxy, lower alkoxycarbonyl, phenyl-, naphthyl- or fluorenyl-lower alkoxycarbonyl, lower alkanoyl, lower alkylsulfonyl, phosphono, hydroxy-lower alkoxyphosphoryl or di-lower alkoxyphosphoryl, carbamoyl, mono- or di-lower alkylaminocarbamoyl, sulfamoyl, mono- or di-lower alkylsulfamoyl, nitro and/or by cyano and is bonded to lower alkyl,
- bicycloalkyl-lower alkyl wherein bicycloalkyl contains from 5 to 10 carbon atoms,
- tricycloalkyl-lower alkyl wherein tricycloalkyl contains from 8 to 10 carbon atoms,
- aryl-lower alkyl wherein aryl is selected from phenyl, indenyl, indanyl, naphthyl, anthryl, phenanthryl, acenaphthyl and fluorenyl and may be unsubstituted or mono-to tri-substituted by lower alkyl, halo-lower alkyl, phenyl, 1- or 2-naphthyl, hydroxy, lower alkoxy, carbamoyl-lower alkoxy, N-lower alkylcarbamoyl-lower alkoxy, N,N-di-lower alkylcarbamoyl-lower alkoxy, amino, mono- or di-lower alkylamino, lower alkanoylamino, halogen, carboxy, lower alkoxycarbonyl, benzyl-, naphthyl- or fluorenyl-lower alkoxycarbonyl, lower alkanoyl, sulfo, lower alkylsulfonyl, phosphono, hydroxy-lower alkoxyphosphoryl, di-lower alkoxyphosphoryl, carbamoyl, mono- or di-lower alkylcarbamoyl, sulfamoyl, mono- or di-lower alkylsulfamoyl, nitro and/or by cyano, wherein phenyl may be present up to three times, and wherein lower alkyl is unbranched or branched and is unsubstituted or substituted by lower alkyl; heterocyclyl selected from pyrrolyl, furyl, thienyl, imidazolyl, pyrazolyl, oxazolyl, thiazolyl, pyridyl, pyrazinyl, pyrimidinyl, indolyl, quinolyl, isoquinolyl, quinoxalinyl, β-carbolinyl and a benzo-fused, cyclopenta-, cyclohexa- or cyclohepta-fused derivative of those radicals, which may also be fully or partially saturated; hydroxy; lower alkoxy; lower alkanoyloxy; acetoacetoxy; amino- or benzyloxycarbonylamino-lower alkanoyloxy; aryl-lower alkanoyloxy wherein aryl has from 6 to 10 carbon atoms; lower alkoxycarbonyloxy; mono- or di-lower alkylaminocarbonyloxy; aryloxycarbonyloxy wherein aryl has from 6 to 10 carbon atoms; aryl-lower alkoxycarbonyloxy wherein aryl has from 6 to 12 carbon atoms; sulfonyloxy; lower alkylsulfonyloxy; phenylsulfonyloxy; 2- or 4-toluenesulfonyloxy; 1- or 2-naphthylsulfonyloxy; amino; mono- or di-lower alkylamino; N-lower alkoxy-N-lower alkylamino; mono- or di-(phenyl- or naphthyl-lower alkyl)amino; lower alkanoylamino; carboxy; esterified carboxy selected from lower alkoxycarbonyl, aryloxycarbonyl wherein aryl has from 6 to 10 carbon atoms, aryl-lower alkoxycarbonyl wherein aryl has from 6 to 12 carbon atoms; lower alkanoyl; lower alkylsulfonyl; hydroxy-lower alkoxyphosphoryl and di-lower alkoxyphosphoryl; carbamoyl; carbamoyl substituted by one or two radicals selected from lower alkyl, carboxy-lower alkyl, lower alkoxycarbonyl-lower alkyl, di-lower alkylamino-lower alkyl, aminocarboxy-lower alkyl, hydroxy-lower alkyl and di-lower alkoxy-lower alkyl, or by one radical selected from ethylene, trimethylene, tetramethylene and pentamethylene, in which a carbon atom may have been replaced by nitrogen, oxygen, sulfur or by sulfur mono- or di-substituted by oxygen and which may be unsaturated; also sulfamoyl; phosphono; benzofuranyl; oxo (which is not bonded to the carbon atom that is linked to the nitrogen atom bonding the radical R₇) and/or by cyano,
- heterocyclyl-lower alkyl wherein heterocyclyl is preferably selected from pyrrolyl, furyl, thienyl, imidazolyl, pyrazolyl, oxazolyl, thiazolyl, pyridyl, pyrazinyl, pyrimidinyl, indolyl, quinolyl, isoquinolyl, quinoxalinyl, β-carbolinyl and a benzo-fused, cyclopenta-, cyclohexa- or cyclohepta-fused derivative of those radicals, which may also be fully or partially saturated, e.g. 4-pyrrolidinylmethyl, 1-imidazolylmethyl, 2-pyridylmethyl, 3-pyridylmethyl, quinolin-2-ylmethyl or indol-2-ylmethyl,
- hydroxy-lower alkyl,
- lower alkoxy-lower alkyl,
- phenoxy-lower alkyl or nitrophenoxy-lower alkyl,
- naphthoxy-lower alkyl,
- lower alkanoyloxy-lower alkyl,
- acetoacetoxy-lower alkyl,
- arylmercapto-lower alkyl wherein aryl has from 6 to 10 carbon atoms,
- amino-lower alkyl,
- mono- or di-lower alkylamino-lower alkyl,
- phenyl- or naphthyl-amino-lower alkyl,
- lower alkanoylamino-lower alkyl,
- piperazinylcarbonyl-lower alkyl substituted at the nitrogen atom by lower alkyl,
- lower alkoxycarbonylamino-lower alkyl,
- phenyl-lower alkoxycarbonylamino-lower alkyl,
- aminocarbonylamino-lower alkyl,
- N-phenyl-lower alkyl-N-lower alkylaminocarbonylamino-lower alkyl,
- halo-lower alkyl,
- carboxy-lower alkyl,
- lower alkoxycarbonyl-lower alkyl,
- 2-halo-lower alkoxycarbonyl-lower alkyl,
- phenyl- or naphthyl-lower alkyloxycarbonyl-lower alkyl,
- heterocyclyl-lower alkoxycarbonyl-lower alkyl wherein heterocyclyl is selected from pyrrolyl, furyl, thienyl, imidazolyl, pyrazolyl, oxazolyl, thiazolyl, pyridyl, pyrazinyl, pyrimidinyl, indolyl, quinolyl, isoquinolyl, quinoxalinyl, β-carbolinyl and a benzo-fused, cyclopenta-, cyclohexa- or cyclohepta-fused derivative of those radicals, it being possible for the said radicals also to be fully or partially saturated,
- lower alkylsulfonyl-lower alkyl,
- arylsulfonyl-lower alkyl wherein aryl has from 6 to 10 carbon atoms,
- carbamoyl-lower alkyl,
- lower alkylcarbamoyl-lower alkyl,
- di-lower alkylcarbamoyl-lower alkyl,
- hydroxy-lower alkylcarbamoyl- or di(hydroxy-lower alkyl)carbamoyl-lower alkyl,
- N-lower alkoxy-lower alkoxy-lower alkylcarbamoyl-lower alkyl,
- carboxy-lower alkylcarbamoyl- or di(carboxy-lower alkyl)carbamoyl-lower alkyl,
- carbamoyl-lower alkyl substituted at the nitrogen atom by a radical selected from ethylene, trimethylene, tetramethylene and pentamethylene, wherein a carbon atom may have been replaced by nitrogen, oxygen, sulfur or by sulfur mono- or di-substituted by oxygen, it being possible for the radical so formed also to be fully or partially unsaturated,
- N-lower alkyl-N-heterocyclyl-lower alkylcarbamoyl-lower alkyl wherein heterocyclyl is selected from pyrrolyl, furyl, thienyl, imidazolyl, pyrazolyl, oxazolyl, thiazolyl, pyridyl, pyrazinyl, pyrimidinyl, indolyl, quinolyl, isoquinolyl, quinoxalinyl, β-carbolinyl and a benzo-fused, cyclopenta-, cyclohexa- or cyclohepta-fused derivative of those radicals, which may also be fully or partially saturated,
- sulfamoyl-lower alkyl,
- N-(phenyl- or naphthyl-lower alkyl)sulfamoyl-lower alkyl,
- sulfamoyl-lower alkyl substituted at the nitrogen atom by a radical selected from ethylene, trimethylene, tetramethylene and pentamethylene, wherein a carbon atom may have been replaced by nitrogen, by nitrogen substituted by lower alkyl, by oxygen, by sulfur or by sulfur mono- or di-substituted by oxygen, it being possible for the radical so formed also to be fully or partially unsaturated,
- oxo-lower alkyl (wherein oxo is not bonded to the carbon atom that is linked to the nitrogen atom bonding R₇),
- cyano-lower alkyl,
- hydroxy-carboxy-lower alkyl,
- α-naphthoxy-carboxy-lower alkyl,
- hydroxy-lower alkoxycarbonyl-lower alkyl,
- α-naphthoxy-lower alkoxycarbonyl-lower alkyl,
- lower alkylcarbonyl-halo-lower alkyl,
- α-naphthoxyethoxycarbonyl-lower alkyl,
- α-naphthoxybenzyloxycarbonyl-lower alkyl,
- esterified hydroxy-lower alkoxycarbonyl-lower alkyl wherein the hydroxy group is esterified by lower alkanoyl; by cycloalkyl-lower alkanoyl wherein cycloalkyl has from 3 to 7 carbon atoms; by bicycloalkyl-lower alkanoyl wherein bicycloalkyl contains from 5 to 10 carbon atoms; by tricycloalkyl-lower alkanoyl wherein tricycloalkyl contains from 8 to 10 carbon atoms; by aryl-lower alkanoyl wherein aryl has from 6 to 14 carbon atoms and may be unsubstituted or mono- to tri-substituted by lower alkyl, hydroxy, lower alkoxy, carbamoyl-lower alkoxy, N-lower alkylcarbamoyl-lower alkoxy or N,N-di-lower alkylcarbamoyl-lower alkoxy, amino, mono- or di-lower alkylamino, halogen, carboxy, lower alkoxycarbonyl, phenyl-, naphthyl- or fluorenyl-lower alkoxycarbonyl, lower alkanoyl, lower alkylsulfonyl, phosphono, hydroxy-lower alkoxy-phosphoryl, di-lower alkoxyphosphoryl, carbamoyl, sulfamoyl, nitro and/or by cyano; by lower alkoxycarbonyl; by 2-halo-lower alkoxycarbonyl; or by phenyl- or fluorenyl-lower alkoxycarbonyl,
- dihydroxy-carboxy-lower alkyl,
- dihydroxy-lower alkoxycarbonyl-lower alkyl,
- dihydroxy-lower alkoxycarbonyl-lower alkyl esterified by lower alkanoyl, lower alkoxycarbonyl, phenyl- or fluorenyl-lower alkoxycarbonyl, lower alkylsulfonyl or by toluenesulfonyl,
- α-naphthoxy-di-lower alkylamino-lower alkyl
- α-naphthoxy-carbamoyl-lower alkyl,
- α-naphthoxy-oxo-lower alkyl (wherein oxo is not bonded to the carbon atom that is linked to the nitrogen atom bonding R₇), or
- α-naphthoxy-cyano-lower alkyl,
or a salt of such a compound where salt-forming groups are present, the term "lower" in the above definitions indicating that the radical in question has up to and including 7 carbon atoms, which process comprises using appropriately substituted starting materials.

5. A process according to claim 1 for the preparation of a compound of formula I wherein R₁ is lower alkoxycarbonyl, phenyl-lower alkoxycarbonyl, the monovalent residue, bonded via the carboxy group, of an aliphatic amino acid selected from valine, alanine, leucine and isoleucine, or the residue, bonded via the carboxy group, of an aliphatic amino acid as defined above that is acylated at the amino nitrogen atom by one of the radicals phenyl-lower alkanoyl, morpholinyl-lower alkanoyl, thiomorpholinyl-lower alkanoyl, S,S-dioxothiomorpholinyl-lower alkanoyl, pyridyl-lower alkanoyl, lower alkoxycarbonyl and phenyl-lower alkoxycarbonyl, all of the amino acids mentioned being in the D-, D,L- or L-form, R₂ is hydrogen, R₃ is phenyl-lower alkyl, R₄ is hydrogen, R₅ is hydroxy, R₆ is hydrogen, R₇ is lower alkyl, cyclohexyl-lower alkyl or phenyl-lower alkyl, R₈ is hydrogen and R₉ is one of the radicals mentioned for R₁, and the asymmetric carbon atoms carrying the radicals R₃ and R₅ are in the S-configuration, or a pharmacologically acceptable salt of such a compound, the term "lower" in the above definitions indicating that the radical in question has up to and including 7 carbon atoms, which process comprises using appropriately substituted starting materials.

6. A process according to claim 1 for the preparation of a compound of formula I wherein R₁ is tert-butoxycarbonyl, benzyloxycarbonyl, the monovalent residue, bonded via the carboxy group, of the amino acid valine, or the residue, bonded via the carboxy group, of alanine acylated at the amino nitrogen atom by one of the radicals phenylacetyl, 3-pyridylacetyl, morpholinocarbonyl, thiomorpholinocarbonyl, tert-butoxycarbonyl and benzyloxycarbonyl, R₂ is hydrogen, R₃ is benzyl, R₄ is hydrogen, R₅ is hydroxy, R₆ is hydrogen, R₇ is isobutyl, cyclohexylmethyl or benzyl, R₈ is hydrogen and R₉ is one of the radicals mentioned for R₁, and the asymmetric carbon atoms carrying the radicals R₃ and R₅ are in the S-configuration, or a pharmacologically acceptable salt of such a compound, which process comprises using appropriately substituted starting materials.

7. A process according to claim 1 for the preparation of a compound of formula I wherein R₁ and R₉ are each the monovalent residue, bonded via the carboxy group and acylated at the amino nitrogen atom by benzyloxycarbonyl, of the amino acid (L)-valine, R₂ and R₈ are hydrogen, R₃ is benzyl, R₄ is hydrogen, R₅ is hydroxy, R₆ is hydrogen and R₇ is benzyl, and the asymmetric carbon atoms carrying the radicals R₃ and R₅ are in the S-configuration, or a pharmacologically acceptable salt of that compound, which process comprises using appropriately substituted starting materials.

8. A process according to claim 1 for the preparation of a compound of formula I wherein R₁ and R₉ are each the monovalent residue, bonded via the carboxy group and acylated at the amino nitrogen atom by 4-thiomorpholinocarbonyl, of the amino acid (L)-valine, R₂ and R₈ are hydrogen, R₃ is benzyl, R₄ is hydrogen, R₅ is hydroxy, R₆ is hydrogen and R₇ is isobutyl, and the asymmetric carbon atoms carrying the radicals R₃ and R₅ are in the S-configuration, or a pharmacologically acceptable salt of that compound, which process comprises using appropriately substituted starting materials.

9. A process according to claim 1 for the preparation of a compound of formula I selected from the compounds designated
Boc-[Phe^{NN}Phe]-Boc,
Boc-(L)-Val-[Phe^{NN}Phe]←((L)-Val-Boc),
Boc-[Phe^{NN}Cha]-Boc,
H-(L)-Val-[Phe^{NN}Phe]←((L)-Val)-H,
N-thiomorpholinocarbonyl-(L)-Val-[Phe^{NN}Phe]←(N-thiomorpholinocarbonyl-(L)-Val),
N-morpholinocarbonyl-(L)-Val-[Phe^{NN}Phe]←(N-morpholinocarbonyl-(L)-Val),
phenylacetyl-(L)-Val-[Phe^{NN}Phe]←(N-phenylacetyl-(L)-Val),
N-(3-pyridylacetyl)-(L)-Val-[Phe^{NN}Phe]←(N-(3-pyridylacetyl)-(L)-Val),
Boc-(L)-Val-[Phe^{NN}Cha]←((L)-Val)-Boc,
Z-(L)-Val-[Phe^{NN}Cha]←((L)-Val)-Z,
Boc-[Phe^{NN}Leu]-Boc,
Z-(L)-Val-[Phe^{NN}Leu]←((L)-Val)-Z, H-(L)-Val-[Phe^{NN}Cha]←((L)-Val)-H and
N-(3-pyridylacetyl)-(L)-Val-[Phe^{NN}Leu]←(N-(3-pyridylacetyl)-(L)-Val), or a salt thereof;
wherein Boc is tert-butoxycarbonyl, Z is benzyloxycarbonyl, the residue - [Phe^{NN}Phe]- is the divalent radical of 3(S)-amino-4-phenyl-1-(N-benzylhydrazino)-butan-2(S)-ol and has the formula 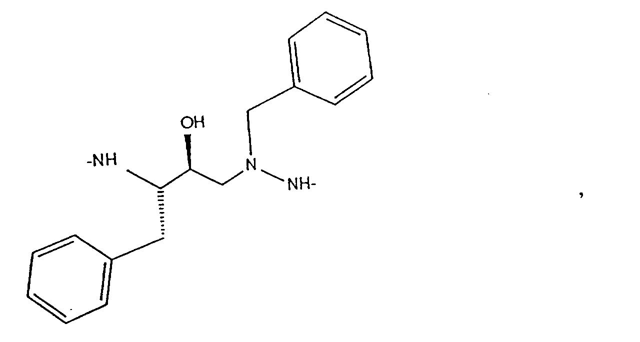 the residue -[Phe^{NN}Cha]- is the divalent radical of 3(S)-amino-4-phenyl-1-(N-cyclohexylmethylhydrazino)-butan-2(S)-ol and has the formula 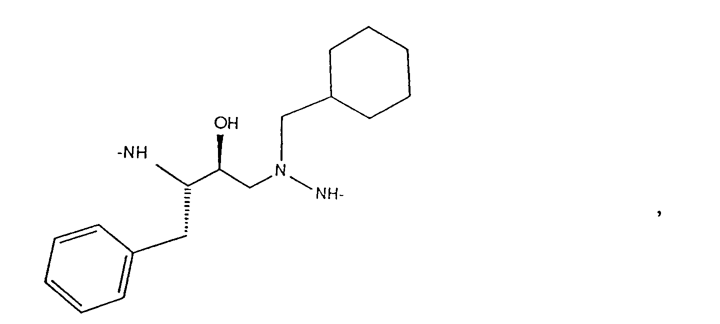 the residue -[Phe^{NN}Leu]- is the divalent radical of 3(S)amino-4-phenyl-1-(N-isobutylhydrazino)-butan-2(S)-ol and has the formula 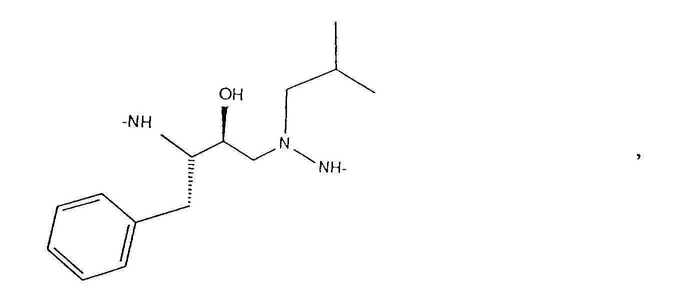 and an arrow "←" denotes the reversal of the bonding direction in a departure from customary peptide nomenclature in which the amino terminus is on the left and the carboxy terminus on the right, which process comprises using appropriately substituted starting materials.

10. A process according to claim 1 for the preparation of a compound of formula I selected from the compounds designated
Z-(L)-Val-[(p-F)Phe^{NN}(p-F)Phe]←(N-(N-(2-pyridylmethyl)-N-methylaminocarbonyl)-(L)-Val),
Z-(L)-Val-[(p-F)Phe^{NN}(p-F)Phe]←(N-(2(R,S)-carbamoyl-3-phenyl-propionyl)(L)-Val),
acetyl-(L)-Val-[Phe^{NN}Cha]←(N-acetyl-(L)-Val),
acetyl-Ile-[Phe^{NN}Cha]←(N-acetyl-Ile),
N-(2-pyridylmethyl)-N-methyl-aminocarbonyl-(L)-Val-[Phe^{NN}(p-F)-Phe]←(N-(N-(2-pyridylmethyl)-N-methyl-aminocarbonyl)-(L)-Val),
Z-(L)-Val-[Phe^{NN}(p-F)Phe]←((L)-Val)-Z,
Z-(L)-Val-[Phe^{NN}(p-CN)Phe]←((L)-Val)-Z,
Z-(L)-Ile-[Phe^{NN}Leu]←((L)-Ile)-Z,
isobutoxycarbonyl-(L)-Val-[Phe^{NN}Leu]←(N-isobutoxycarbonyl-(L)-Val),
acetyl-Val-[Phe^{NN}Leu]←(N-(2(R,S)-carbamoyl-3-phenyl-propionyl)-Val),
N-trifluoroacetyl-[Phe^{NN}Leu]←(N-(2(R,S)-carbamoyl-3-phenyl-propionyl)-(L)-Val),
Z-(L)-Val-[Phe^{NN}Nle]←(N-(2(R,S)-(N-(2-morpholinoethyl)-carbamoyl)-3-methyl)-butyryl),
Z-(L)-Val-[Phe^{NN}Nle]←(N-(2(R,S)-(N-(2-pyridylmethyl)-carbamoyl)-3-methyl)-butyryl),
methoxycarbonyl-(L)-Val-[Phe^{NN}Leu]←(N-methoxycarbonyl-(L)-Val),
methoxycarbonyl-(L)-Val-[Phe^{NN}(p-F)Phe]←(N-methoxycarbonyl-(L)-Val) and
methoxycarbonyl-(L)-Val-[Phe^{NN}(p-CN)Phe]←(N-methoxycarbonyl-(L)-Val),
or a salt thereof, wherein Boc is tert-butoxycarbonyl, Z is benzyloxycarbonyl, the residue -[Phe^{NN}Phe]- is the divalent radical of 3(S)-amino-4-phenyl-1-(N-benzylhydrazino)-butan-2(S)-ol and has the formula 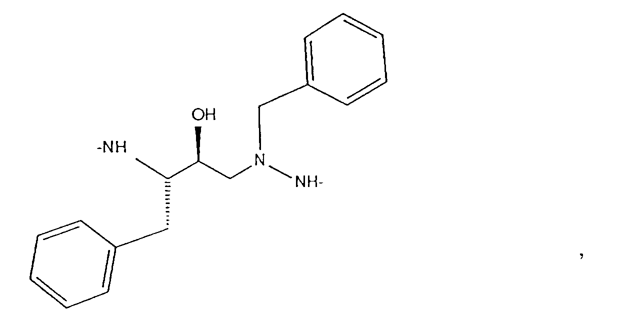 the residue -[Phe^{NN}Cha]- is the divalent radical of 3(S)-amino-4-phenyl-1-(N-cyclohexylmethylhydrazino)-butan-2(S)-ol and has the formula 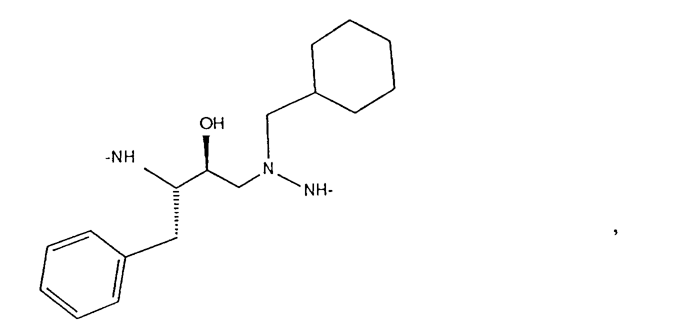 the residue -[Phe^{NN}Leu]- is the divalent radical of 3(S)-amino-4-phenyl-1-(N-isobutylhydrazino)-butan-2(S)-ol and has the formula 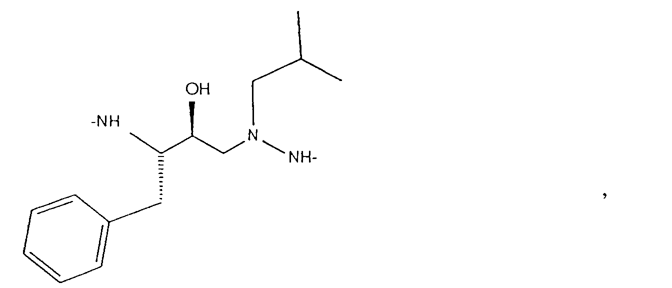 the residue designated -[Phe^{NN}Nle]- is the radical of 3(S)-amino-4-phenyl-1-(N-n-butylhydrazino)-butan-2(S)-ol and has the formula 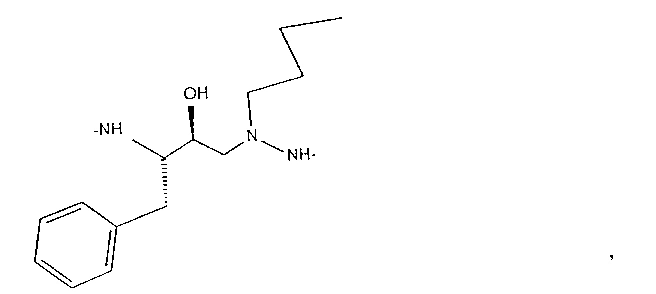 the residue designated -[Phe^{NN}(p-F)Phe]- is the divalent radical of 3(S)-amino-4-phenyl-1-(N-(p-fluorophenylmethyl)-hydrazino)-butan-2(S)-ol and has the formula 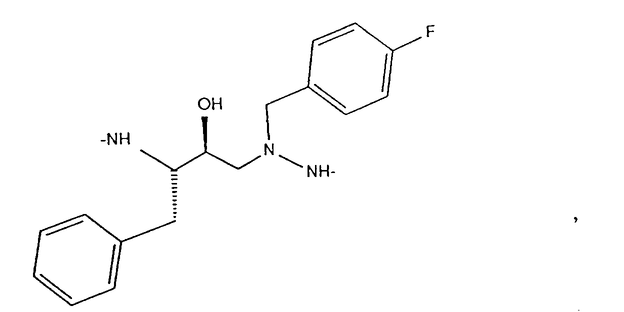 the residue designated -[(p-F)Phe^{NN}(p-F)Phe]- is the divalent radical of 3(S)-amino-4-(p-fluorophenyl)-1-(N-(p-fluorophenylmethyl)-hydrazino)-butan-2(S)-ol and has the formula 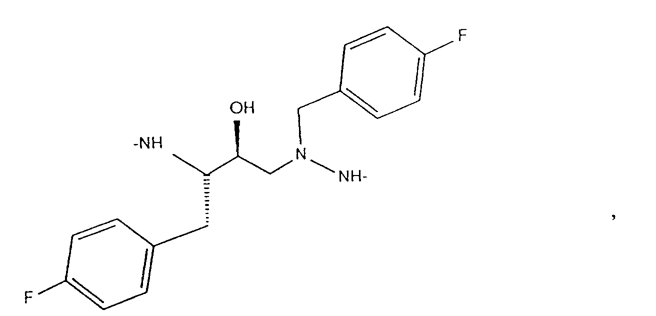 the residue designated -[Phe^{NN}(p-CN)Phe]- is the divalent radical of 3(S)-amino-4-phenyl-1-(N-(p-cyanophenylmethyl)-hydrazino)-butan-2(S)-ol and has the formula 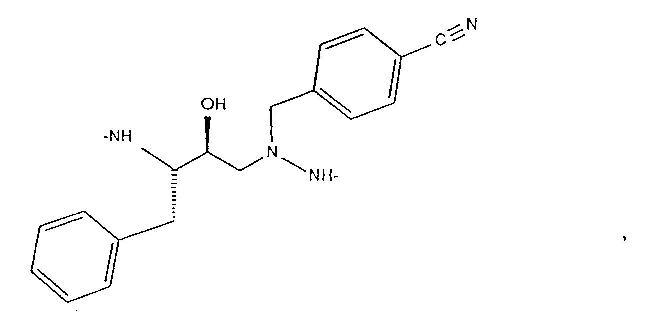 and an arrow "←" denotes the reversal of the bonding direction in a departure from customary peptide nomenclature in which the amino terminus is on the left and the carboxy terminus on the right, which process comprises using appropriately substituted starting materials.

## Revendications (Revendications pour l'(les) Etat(s) contractant(s) suivant(s): AT, BE, CH, DE, DK, FR, GB, IT, LI, LU, MC, NL, PT, SE)

1. Composés de formule 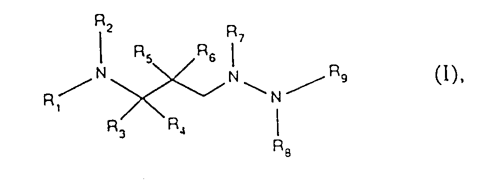 dans laquelle R₁ et R₉ représentent indépendamment l'un de l'autre un hydrogène, un acyle, un alkyle, un alcényle ou un alcynyle non substitué ou substitué; un hétérocyclyle; un sulfo, un sulfonyle substitué par un alkyle non substitué ou substitué, un aryle, un alcoxy, qui est non substitué ou substitué, ou par un aryloxy; un sulfamoyle, qui est non substitué ou substitué sur l'atome d'azote; ou un phosphoryle substitué par un ou deux radicaux choisis indépendamment l'un de l'autre parmi un alkyle non substitué ou substitué, un cycloalkyle non substitué ou substitué, un aryle, un hydroxy, un alcoxy non substitué ou substitué, un cycloalcoxy et un aryloxy, à la condition qu'au plus un des radicaux R₁ et R₉ représente un hydrogène; et R₂ et R₈ représentent, indépendamment l'un de l'autre, un hydrogène ou l'un des radicaux mentionnés ci-dessus pour R₁ et R₉ ; ou les paires de substituants R₁ et R₂ ou R₈ et R₉ représentent, chacun indépendamment l'un de l'autre avec l'atome d'azote auquel ils sont liés, un noyau hétérocyclique qui est constitué de l'atome d'azote liant avec un radical choisi parmi l'éthylène, le triméthylène, le tétraméthylène et le pentaméthylène, dans lequel un atome de carbone peut être remplacé par un atome d'azote, d'oxygène, de soufre ou par un atome de soufre mono- ou bisubstitué par un oxygène et qui peut être insaturé, ou peuvent former le radical naphtalène- 1,8-dicarbonylimido;
R₃ et R₄ représentent indépendamment l'un de l'autre un hydrogène, un alkyle ou un cycloalkyle non substitué ou substitué; un aryle; un hétérocyclyle; ou un alcényle non substitué ou substitué; ou R₃ et R₄ représentent ensemble un alkylène non substitué ou substitué, un alkylidène ou un alkylène benzocondensé;
R₅ représente un hydroxy; R₆ représente un hydrogène;
ou R₅ et R₆ représentent ensemble un oxo;
et R₇ représente un alkyle ou un cycloalkyle non substitué ou substitué; un aryle;
un hétérocyclyle; ou un alcényle non substitué ou substitué;
ainsi que les sels des composés mentionnés, dans la mesure où des groupes salificateurs sont présents; à l'exception du composé de formule I, dans laquelle R₁, R₂ , R₃, R₄, R₆ et R₈ représentent chacun un hydrogène, R₅ représente un hydroxy, R₇ représente un radical de formule -[CH₂-(C=O)-NH-(N-CH₃)-CH₂-COOH] et R₉ représente un tert-butoxycarbonyle.

2. Composé de formule I selon la revendication 1, dans laquelle
R₁ et R₉ représentent indépendamment l'un de l'autre un hydrogène; un alcanoyle inférieur; un aryl-alcanoyle inférieur, dans lequel l'alcanoyle inférieur est non substitué ou substitué par un carbamoyle ou par un carbamoyle substitué sur l'atome d'azote par un ou deux radicaux choisis parmi un alkyle inférieur, un carboxyalkyle inférieur, un alcoxy inférieur-carbonyl-alkyle inférieur, un dialkyl inférieur-amino-alkyle inférieur, un amino-carboxy-alkyle inférieur, un hydroxyalkyle inférieur ou un dialcoxy inférieur-alkyle inférieur; un hétérocyclylalcanoyle inférieur; un halogèno-alcanoyle inférieur qui contient un à trois atomes d'halogène; un (N-hétérocyclyl-alkyl inférieur-carbamoyl)-alcanoyle inférieur; un alcoxy inférieur-carbonyle; un arylalcoxy inférieur-carbonyle; un hétérocyclylalcoxy inférieur-carbonyle; un alkyl inférieur-sulfonyle, un N-(hétérocyclylalkyl inférieur)-N-alkyl inférieur-carbamoyle; ou un radical liée par le groupe acyle d'un acide aminé dont la fonction amino est libre ou acylée par l'un des autres radicaux R₁ et R₉ mentionnés jusqu'à présent; sous réserve qu'au plus un des radicaux R₁ et R₉ représente un hydrogène;
R₂, R₄, R₆ et R₈ représentent un hydrogène,
R₃ représente un alkyle inférieur, un cycloalkyl-alkyle inférieur ou un aryl-alkyle inférieur,
R₅ représente un hydroxy, et
R₇ représente un alkyle inférieur non substitué ou substitué,
ou un de ses sels, dans la mesure où au moins un groupe salificateur est présent, où dans les définitions mentionnées ci-dessus l'expression "inférieur" signifie que le radical en question comporte jusqu'à 7 atomes de carbone compris.

3. Composé de formule I selon la revendication 1, dans laquelle
R₁ et R₉ représentent indépendamment l'un de l'autre un hydrogène; un alcanoyle inférieur; un arylalcanoyle inférieur dans lequel aryle représente le phényle, l'indényle, l'indanyle, le naphtyle, l'anthryle, le phénanthryle, l'acénaphtyle ou le fluorényle et peut être non substitué ou mono- à trisubstitué par un alkyle inférieur, un halogèno-alkyle inférieur, un phényle, un 1- ou un 2-naphtyle, un hydroxy, un alcoxy inférieur, un carbamoyl-alcoxy inférieur, un N-alkyl inférieur-carbamoyl-alcoxy inférieur, un N,N-dialkyl inférieur-carbamoylalcoxy inférieur, un amino, un mono- ou di-alkyl inférieur-amino, un alcanoyl inférieur-amino, un halogène, un carboxy, un alcoxy inférieur-carbonyle, un phényl-, naphtyl-ou fluorénylalcoxy inférieur-carbonyle, un alcanoyle inférieur, un sulfo, un alkyl inférieur-sulfonyle, un phosphono, un hydroxy-alcoxy inférieur-phosphoryle, un dialcoxy inférieur-phosphoryle, un carbamoyle, un mono- ou di-alkyl inférieur-carbamoyle, un sulfamoyle, un mono- ou di-alkyl inférieur-aminosulfonyle, un nitro et/ou un cyano, où le phényle peut être présent jusqu'à trois fois, et où l'alcanoyle inférieur est non substitué ou substitué par un carbamoyle ou par un carbamoyle substitué sur l'atome d'azote par un ou deux radicaux choisis parmi les groupements alkyle inférieur, carboxyalkyle inférieur, alcoxy inférieur-carbonylalkyle inférieur, dialkyl inférieur-amino-alkyle inférieur, amino-carboxy-alkyle inférieur, hydroxyalkyle inférieur et dialcoxy inférieur-alkyle inférieur; hétérocyclylalcanoyle inférieur, où l'alcanoyle inférieur est non substitué et où l'hétérocyclyle est choisi parmi les radicaux thiényle, furyle, pyranyle, pyrrolyle, imidazolyle, pyrazolyle, oxazolyle, isoxazolyle, thiazolyle, furazanyle, pyridyle, pyrazinyle, pyrimidinyle, pyridazinyle, azépinyle, indolyle, benzimidazolyle, 1H-indazolyle, quinoléyle, isoquinoléyle, quinoxalinyle, quinazolinyle, cinnolyle, purinyle, ptéridinyle, naphtyridinyle, 4H-quinolizinyle, 3,1-benzofuranyle, 4,1-benzoxazinyle, 4,1-benzothiazinyle, cyclohexa[b]pyrrolyle, cyclohepta[b]pyrrolyle, cyclohexa[b]pyrazolyle, cyclohexa[b]pyridyle, cyclohexa[b]pyrazinyle, cyclohexa[b]pyrimidinyle, cyclohexa[b]-1,4-oxazinyle, cyclohexa[b]-1,4-thiazinyle, pyrrolidinyle, pyrrolinyle, imidazolidyle, 2-imidazolinyle, 2,3-dihydropyridyle, pipéridyle, pipérazinyle, 2,3,5,6-tétrahydropyrazinyle, morpholinyle, thiomorpholinyle, S,S-dioxothiomorpholinyle, indolinyle, isoindolinyle, 4,5,6,7-tétrahydroindolyle, 1,2,3,4-tétrahydroquinoléyle, 1,2,3,4-tétrahydroisoquinoléyle, chromanyle, thiochromanyle, 1,2,3,4-tétrahydro-3,1-benzodiazinyle, 3,4-dihydro-3H-4,1-benzoxazinyle et 3,4-dihydro-3H-4,1-benzothiazinyle, les radicaux hétérocyclyle mentionnés étant non substitués ou substitués par un alkyle inférieur, un phényle, un 1- ou 2-naphtyle, un phénylalkyle inférieur, un hydroxyalkyle inférieur, un alcoxy inférieur-alkyle inférieur, un phénoxy- ou naphtoxyalkyle inférieur, un phénylalcoxy inférieur- ou naphtylalcoxy inférieur-alkyle inférieur, un alcanoyloxy inférieur-alkyle inférieur, un phényl- ou naphtyl-alcanoyloxy inférieur-alkyle inférieur, un alcoxy inférieur-carbonyloxy-alkyle inférieur, un phényl-, naphtyl- ou fluorényl-alcoxy inférieur-carbonyloxy-alkyle inférieur, un aminoalkyle inférieur, un carboxyalkyle inférieur, un hydroxy, un alcoxy inférieur, un phényl- ou naphtyl-alcoxy inférieur, un amino, un alkyl inférieur-amino, un di-alkyle inférieur-amino, un carboxy, un alcoxy inférieur-carbonyle, un phényl- ou naphtyl-alcoxy inférieur-carbonyle, un halogène, un alcanoyle inférieur, un alkyl inférieur-sulfonyle, un phosphono, un hydroxy-alcoxy inférieur-phosphoryle ou un dialcoxy-phosphoryle, un carbamoyle, un mono-ou di-alkyl inférieur-carbamoyle, un hydroxy- ou carboxy-alkyl inférieur-carbamoyle, un sulfamoy'le, par un nitro, un oxo et/ou par un cyano; un hétérocyclylalcanoyle inférieur comme un halogèno-alcanoyle inférieur, qui contient jusqu'à trois atomes d'halogène; un (N-hétérocyclylalkyl inférieur-carbamoyl)-alcanoyle inférieur, où hétérocyclyle est choisi parmi pyrrolyle, furyle, thiényle, imidazolyle, pyrazolyle, oxazolyle, thiazolyle, pyridyle, pyrazinyle, pyrimidinyle, indolyle, quinoléyle, isoquinoléyle, quinoxalinyle, β-carbolinyle et un dérivé benzocondensé, cyclopenta-, cyclohexa- ou cyclohepta-condensé de ces radicaux, qui peuvent également être entièrement ou partiellement saturés, parmi morpholine et parmi thiomorpholine; un alcoxy inférieur-carbonyle; un arylalcoxy inférieur-carbonyle, où aryle est un phényle, biphénylyle, 1- ou 2-naphtyle, un fluorényle ou un phényle mono- ou polysubstitué par un alkyle inférieur, un hydroxy, un alcoxy inférieur, un halogène et/ou un nitro; un hétérocyclylalcoxy inférieur-carbonyle, où hétérocyclyle est choisi parmi pyrrolyle, furyle, thiényle, imidazolyle, pyrazolyle, oxazolyle, thiazolyle, pyridyle, pyrazinyle, pyrimidinyle, indolyle, quinoléyle, isoquinoléyle, quinoxalinyle, β-carbolinyle et un dérivé benzocondensé, cyclopenta-, cyclohexa-ou cyclohepta-condensé de ces radicaux, qui peuvent également être entièrement ou partiellement saturés, un alkyle inférieur-sulfonyle; un N-hétérocyclylalkyle inférieur-N-alkyle inférieur-carbamoyle, où hétérocyclyle est choisi parmi thiényle, furyle, pyranyle, pyrrolyle, imidazolyle, pyrazolyle, oxazolyle, isoxazolyle, thiazolyle, furazanyle, pyridyle, pyrazinyle, pyrimidinyle, pyridazinyle, azépinyle, indolyle, benzimidazolyle, 1H-indazolyle, quinoléyle, isoquinoléyle, quinoxalinyle, quinazolinyle, cinnolyle, purinyle, ptéridinyle, naphtyridinyle, 4H-quinolizinyle, 3,1-benzofuranyle, benz[e]indolyle, 4,1-benzoxazinyle, 4,1-benzothiazinyle, carbazlolyle, β-carbolinyle, phénazinyle, phénanthridyle, acridyle, phénoxazinyle, phénothiazinyle, 1-azaacénaphtényle, cyclohexa[b]pyrrolyle, cyclohepta[b]pyrrolyle, cyclohexa[d]pyrazolyle, cyclohexa[b]pyridyle, cyclohexa[b]pyrazinyle, cyclohexa[b]pyrimidinyle, cyclohexa[b]-1,4-oxazinyle, cyclohexa[b]-1,4-thiazinyle, pyrrolidinyle, pyrrolinyle, imidazolidinyle, 2-imidazolinyle, 2,3-dihydropyridyle, pipéridyle, pipérazinyle, 2,3,5,6-tétrahydropyrazinyle, morpholinyle, thiomorpholinyle, S,S-dioxothiomorpholinyle, indolinyle, isoindolinyle, 4,5,6,7-tétrahydroindolyle, 1,2,3,4-tétrahydroquinoléyle, 1,2,3,4-tétrahydroisoquinoléyle, chromanyle, thiochromanyle, 1,2,3,4-tétrahydro-3,1-benzodiazinyle, 3,4-dihydro-3H-4,1-benzoxazinyle, 3,4-dihydro-3H-4,1-benzothiazinyle, 2,3,4,5-tétrahydro-1H-5,1-benzazépinyle et 5,6-dihydrophénanthridinyle, où les radicaux mentionnés sont non substitués ou substitués par un alkyle inférieur, un phényle, un 1-ou 2-naphtyle, un phénylalkyle inférieur, un hydroxyalkyle inférieur, un alcoxy inférieur-alkyle inférieur, un phénoxy- ou naphtoxy-alkyle inférieur, un phénylalcoxy inférieur- ou naphtylalcoxy inférieur-alkyle inférieur, un alcanoyloxy inférieur-alkyle inférieur, un phényl- ou naphtyl-alcanoyloxy inférieur-alkyle inférieur, un alcoxy inférieur-carbonyloxy-alkyle inférieur, un phényl-, naphtyl- ou fluorényl-alcoxy inférieur-carbonyloxy-alkyle inférieur, un aminoalkyle inférieur, un carboxyalkyle inférieur, un hydroxy, un alcoxy inférieur, un phényl- ou naphtylalcoxy inférieur, un amino, un alkyle inférieur-amino, un dialkyl inférieur-amino, un carboxy, un alcoxy inférieur-carbonyle, un phényl- ou naphtyl-alcoxy inférieur-carbonyle, un halogène, un alcanoyle inférieur, un alkyl inférieur-sulfonyle, un phosphono, un hydroxy-alcoxy inférieur-phosphoryle ou un dialcoxy-phosphoryle, un carbamoyle, un mono-ou dialkyle inférieur-carbamoyle, un hydroxy- ou carboxy-alkyle inférieur-carbamoyle, un sulfamoyle, par un nitro, un oxo et/ou par un cyano; ou un radical acyle d'un acide aminé, dont la fonction amino est libre ou est acylée par un des autres radicaux mentionnés jusqu'ici pour R₁ et R₉, où les acides aminés sont choisis parmi la glycine, l'alanine, la valine, la norvaline, la leucine, l'isoleucine, la norleucine, la sérine, l'homosérine, la thréonine, la méthionine, la cystéine, la proline, la trans-3- et la trans-4-hydroxyproline, la phénylalanine, la tyrosine, la 4-aminophénylalanine, la 4-chlorophénylalanine, la 4-carboxyphénylalanine, la β-phénylsérine, la phénylglycine, l'α-naphtylalanine, la cyclohexylalanine, la cyclohexylglycine, le tryptophane, l'acide aspartique, l'asparagine, l'acide aminomalonique, le monoamide de l'acide aminomalonique, l'acide glutamique, la glutamine, l'histidine, l'arginine, la lysine, la δ-hydroxylysine, l'ornithine, l'acide 3-aminopropanoïque, l'acide α,γ-diaminobutyrique et l'acide α,β-diaminopropionique, où (en-dehors de la glycine) chacun des radicaux acide aminé mentionnés se présente sous forme D, L ou (D,L); sous réserve qu'au plus un des radicaux R₁ et R₉ représente un hydrogène,
R₂, R₄, R₆ et R₈ représentent un hydrogène,
R₃ représente un alkyle inférieur; un cycloalkyl-alkyle inférieur, dans lequel le cycloalkyle comporte de 3 à 7 atomes de carbone et est non substitué ou est mono-ou jusqu'à trisubstitué par un alkyle inférieur, un halogène-alkyle inférieur, un hydroxy, un alcoxy inférieur, un carbamoylalcoxy inférieur, un N-alkyl inférieur-carbamoylalcoxy inférieur, un N,N-dialkyl inférieur-carbamoylalcoxy inférieur, un amino, un mono- ou dialkyl inférieur-amino, un halogène, un carboxy, un alcoxy inférieur-carbonyle, un phényl-, naphtyl- ou fluorénylalcoxy inférieur-carbonyle, un alcanoyle inférieur, un alkyl inférieur-sulfonyle, un phosphono, un hydroxy-alcoxy inférieur-phosphoryle ou un dialcoxy inférieur-phosphoryle, un carbamoyle, un mono- ou dialkyl inférieur-aminocarbamoyle, un sulfamoyle, un mono- ou dialkyl inférieur-sulfamoyle, un nitro et/ou un cyano; ou un arylalkyle inférieur, où l'aryle représente un phényle, indényle, indanyle, naphtyle, anthryle, phénanthryle, acénaphtyle ou fluorényle et est non substitué ou peut être mono-ou jusqu'à trisubstitué par un alkyle inférieur, un halogèno-alkyle inférieur, un phényle, un 1- ou 2-naphtyle, un hydroxy, un alcoxy inférieur, un carbamoylalcoxy inférieur, un N-alkyl inférieur-carbamoylalcoxy inférieur, un N,N-dialkyl inférieurcarbamoylalcoxy inférieur, un amino, un mono- ou dialkyl inférieur-amino, un alcanoyl inférieur-amino, un halogène, un carboxy, un alcoxy inférieur-carbonyle, un benzyl-, naphtyl- ou fluorényl-alcoxy inférieur-carbonyle, un alcanoyle inférieur, un sulfo, un alkyl inférieur-sulfonyle, un phosphono, un hydroxy-alcoxy inférieur-phosphoryle, un dialcoxy inférieur-phosphoryle, un carbamoyle, un mono- ou dialkyl inférieur-carbamoyle, un sulfamoyle, un mono-ou dialkyl inférieur-sulfamoyle, un nitro et/ou un cyano, le phényle pouvant être présent jusqu'à trois exemplaires,
R₅ représente un hydroxy, et
R₇ représente un alkyle inférieur non substitué; ou un cycloalkyl-alkyle inférieur, comme indiqué en dernier lieu pour cycloalkyl-alkyle inférieur R₃ ; ou un arylalkyle inférieur, comme indiqué en dernier pour arylalkyle inférieur R₃ ;
ou un de ses sels, dans la mesure où au moins un groupe salificateur est présent, et où dans les difinitions données ci-dessus l'expression "inférieur" signifie que le radical en question comporte jusqu'à 7 atomes de carbone compris.

4. Composés de formule I selon la revendication 1 dans laquelle
R₁ et R₉ représentent indépendamment l'un de l'autre
- un hydrogène,
- un alcoxy inférieur-carbonyle,
- un 2-halogèno-alcoxy inférieur-carbonyle,
- un aryloxycarbonyle, où l'aryle a de 6 à 14 atomes de carbone,
- un arylalcoxy inférieur-carbonyle, où l'aryle a de 6 à 14 atomes de carbone,
- un hétérocyclyl-alcoxy inférieur-carbonyle, où l'hétérocyclyle est choisi parmi les radicaux pyrrolyle, furyle, thiényle, imidazolyle, pyrazolyle, oxazolyle, thiazolyle, pyridyle, pyrazinyle, pyrimidinyle, indolyle, quinoléyle, isoquinoléyle, quinoxalinyle, β-carbolinyle et un dérivé benzocondensé, cyclopenta-, cyclohexa- ou cyclohepta-condensé de ces radicaux, qui peuvent également être entièrement ou partiellement saturés,
- un 2-trialkyl inférieur-silyl-alcoxy inférieur-carbonyle,
- un 2-triarylsilylalcoxy inférieur-carbonyle, où l'aryle est un phényle ou un 1-ou 2-naphtyle,
- le radical, lié par l'intermédiaire du groupe carboxy, d'un acide aminé choisi parmi la glycine, l'alanine, la valine, la norvaline, la leucine, l'isoleucine, la norleucine, la sérine, l'homo-sérine, la thréonine, la méthionine, la cystéine, la proline, la trans-3- et la trans-4-hydroxyproline, la phénylalanine, la tyrosine, la 4-amino-phénylalanine, la 4-chlorophénylalanine, la 4-carboxyphénylalanine, la β-phénylsérine, la phénylglycine, l'α-naphtylalanine, la cyclohexyl-alanine, la cyclohexylglycine, le tryptophane, l'acide indoline-2-carboxylique, l'acide 1,2,3,4-tétrahydroisoquinoléine-3-carboxylique, l'acide aspartique, l'asparagine, l'acide aminomalonique, le monoamide de l'acide aminomalonique, l'acide glutamique, la glutamine, l'histidine, l'arginine, la lysine, la δ-hydroxylysine, l'ornithine, l'acide α,γ-diaminobutyrique et l'acide α,β-diamino-propionique, chacun de ces acides aminés pouvant se présenter sous forme D, L ou (D,L), et
le groupe α-amino est non substitué ou mono- ou bi- N-alkylé par un alkyle inférieur, par un amino-alkyle inférieur, par un phényl- ou un naphtylamino-alkyle inférieur, ou un pipérazinylcarbonylalkyle inférieur substitué sur l'atome d'azote par un alkyle inférieur, ou N-acylé par un alcanoyle inférieur, par un arylalcanoyle inférieur, où l'aryle est choisi parmi les groupements phényle, indényle, indanyle, naphtyle, anthryle, phénanthryle, acénaphtyle et fluorényle, et est non substitué ou peut être mono- ou jusqu'à trisubstitué par un alkyle inférieur, un halogèno-alkyle inférieur, un phényle, un 1- ou 2-naphtyle, un hydroxy, un alcoxy inférieur, un carbarnoylalcoxy inférieur, un N-alkyl inférieur-carbamoylalcoxy inférieur ou un N,N-dialkyl inférieur-carbamoylalcoxy inférieur, un amino, un mono-ou un di-alkyl inférieur-amino, un alcanoyl inférieur-amino, un halogène, un carboxy, un alcoxy inférieur-carbonyle, un phényl-, naphtyl- ou fluorénylalcoxy inférieur-carbonyle, un alcanoyle inférieur, un sulfo, un alkyl inférieur-sulfonyle, un phosphono, un hydroxy-alcoxy inférieur-phosphoryle ou un dialcoxy inférieur-phosphoryle, un carbamoyle, un mono- ou di-alkyl inférieur-carbamoyle, un sulfamoyle, un mono- ou di-alkyl inférieur-amino-sulfonyle, un nitro et/ou un cyano; où le phényle peut être présent jusqu'à trois exemplaires, et un alcanoyle inférieur non substitué ou substitué par un alkyle inférieur, un hétérocyclyle choisi parmi les radicaux pyrrolyle, furyle, thiényle, imidazolyle, pyrazolyle, oxazolyle, thiazolyle, pyridyle, pyrazinyle, pyrimidinyle, indolyle, quinoléyle, isoquinoléyle, quinoxalinyle, β-carbolinyle et un dérivé benzo-, cyclopenta-, cyclohexa-ou cyclohepta-condensé de ces radicaux, qui peuvent également être entièrement ou partiellement saturés, un hydroxy, un alcoxy inférieur, un alcanoyloxy inférieur, un acétoacétoxy, un amino- ou benzyloxycarbonylainino-alcanoyloxy inférieur, un arylalcanoyloxy inférieur, où être entièrement ou partiellement condensés, un hydroxy, un alcoxy inférieur, un alcanoyloxy inférieur, un acétoacétoxy, un amino- ou benzyloxycarbonylarnino-alcanoyloxy inférieur, un arylalcanoyloxy inférieur, où l'aryle a de 6 à 10 atomes de carbone, un alcoxy inférieur-carbonyloxy, un mono- ou dialkyl inférieur-aminocarbonyloxy, un aryloxycarbonyloxy, où aryle a de 6 à 10 atomes de carbone, un arylalcoxy inférieur-carbonyloxy où aryle a de 6 à 10 atomes de carbone, un arylalcoxy inférieur carbonyloxy où l'aryle a de 6 à 14 atomes de carbone, un sulfonyloxy, un alkyl inférieur-sulfonyloxy, un phénylsulfonyloxy, un 2- ou 4-toluènesulfonyloxy ou un 1- ou 2-naphtylsulfonyloxy, carboxy, un carboxy estérifié choisi parmi un alcoxy inférieur-carbonyle, un aryloxycarbonyle, où l'aryle a de 6 à 10 atomes de carbone, un arylalcoxy inférieur-carbonyle, où l'aryle a de 6 à 12 atomes de carbone, un alcanoyle inférieur, un alkyl inférieur-sulfonyle, un hydroxy-alcoxy inférieur-phosphoryle et un di-alcoxy inférieur-phosphoryle, un carbamoyle, un carbamoyle substitué par un ou deux radicaux choisis parmi les groupements alkyle inférieur, carboxyalkyle inférieur, alcoxy inférieur carbonyl-alkyle inférieur, un dialkyl inférieur-amino-alkyle inférieur, aminocarboxyalkyle inférieur, hydroxyalkyle inférieur et parmi dialcoxy inférieur-alkyle inférieur ou un radical choisi parmi l'éthylène, le triméthylène, le tétraméthylène, le pentaméthylène, dans lequel un atome de carbone peut être remplacé par un atome d'azote, un atome d'oxygène, un atome de soufre , ou un atome de soufre mono- ou di-substitué par un atome d'oxygène et qui peut être insaturé, ou encore peut être un sulfamoyle, un phosphono, un benzofuranyle, un oxo et/ou cyano et est non ramifié ou ramifié ; par un hétérocyclylalcanoyle inférieur choisi parmi les radicaux thiényl-, furyl-, pyranyl-, pyrrolyl-, imidazolyl-, pyrazolyl-, oxazolyl-, isoxazolyl-, thiazolyl-, furazanyl-, pyridyl-, pyrazinyl-, pyrimidinyl-, pyridazinyl-, azépinyl-, indolyl-, benzimidazolyl-, 1H-indazolyl-, quinoléyl-, isoquinoléyl-, quinoxalinyl-, quinazolinyl-, cinnolyl-, purinyl-, ptéridinyl-, naphtyridinyl-, 4H-quinolizinyl-, 3,1-benzofuranyl, benz[e]indolyl-, 4,1-benzoxazinyl-, 4,1-benzothiazinyl-, carbazolyl-, β-carbolinyl-, phénazinyl-, phénanthridyl-, acridyl-, phénoxazinyl-, phénothiazinyle, 1-azaacénaphtényl-, cyclohexa[b]pyrrolyl-, cyclohepta[b]pyrrolyl-, cyclohexa[b]pyrazolyl-, cyclohexa[b]pyridyl-, cyclohexa[b]pyrazinyl-, cyclohexa[b]pyrimidinyl-cyclohexa[b]-1,4-oxazinyl-, cyclohexa[b]-1,4-thiazinyl-, pyrrolidinyl-, pyrrolinyl-, imidazolidinyl-, 2-imidazolinyl-, 2,3-dihydropyridyl-, pipéridyl-, pipérazinyl-, 2,3,5,6-tétrahydropyrazinyl-, morpholinyl-, thiomorpholinyl-, S,S-dioxothiomorpholinyl-, indolinyl-, isoindolinyl-, 4,5,6,7-tétrahydroindolyl-, 1,2,3,4-tétrahydroquinoléyl-, 1,2,3,4-tétrahydroisoquinoléyl-, chroman-, thiochroman-, 1,2,3,4-tétrahydro-3,1-benzodiazinyl-, 3,4-dihydro-3H-4,1-benzoxazinyl-, 3,4-dihydro-3H-4,1-benzothiazinyl-, 2,3,4,5-tétrahydro-1H-5,1-benzazépinyl- et 5,6-dihydrophénanthridinyl-alcanoyle inférieur, où les radicaux hétérocyclyles mentionnés sont non substitués ou substitués par un alkyle inférieur, un phényle, un 1- ou 2-naphtyle, un phénylalkyle inférieur, un hydroxyalkyle inférieur, un alkoxy inférieur-alkyle inférieur un phénoxy- ou un naphtoxyalkyle inférieur, un phénylalcoxy inférieur- ou un naphtylalcoxy inférieur-alkyle inférieur, un alcanoyloxy inférieur-alkyle inférieur, un phényl- ou un naphtyl-alcanoyloxy inférieur-alkyle inférieur, un alcoxy inférieur- carbonyloxy-alkyle inférieur, un phényl-, un naphtyl- ou un fluorénylalcoxy inférieur-carbonyloxy-alkyle inférieur, un aminoalkyle inférieur, un carboxyalkyle inférieur, un hydroxy, un alcoxy inférieur, un phényl- ou un naphtylalcoxy inférieur, un amino, un alkyl inférieur-amino, un dialkyl inférieur-amino, un carboxy, un alkoxy inférieur-carbonyle, un phényl- ou un naphtyl-alcoxy inférieur-carbonyle, un halogène, un alcanoyle inférieur, un alkyl inférieur-sulfonyle, un phosphono, un hydroxy-alcoxy inférieur-phosphoryle, un dialoxy-phosphoryle, un carbamoyle, un mono- ou di-alkyl inférieur-carbamoyle, un hydroxy- ou carboxyalkyl inférieur-carbamoyle, un sulfamoyle, par un nitro, un oxo et/ou par un cyano ; par un hétérocyclylalcénoyle inférieur, où hétérocyclyle est choisi parmi les radicaux pyrrolyle, furyle, thiényle, imidazolyle, pyrazolyle, oxazolyle, thiazolyle, pyridyle, pyrazinyle, pyrimidinyle, indolyle, quinoléyle, isoquinoléyle, quinoxalinyle, β-carbolinyle et un dérivé benzocondensé, cyclopenta-, cyclohexa- ou cyclohepta- condensé de ces radicaux, qui peuvent également être entièrement ou partiellement saturés, par un arylalcoxy inférieur-carbonyle, où aryle a de 6 à 14 atomes de carbone ; par un hétérocyclylalcoxy inférieur-carbonyle, où hétérocyclyle est choisi parmi pyrrolyle, furyle, thiényle, imidazolyle, pyrazolyle, oxazolyle, thiazolyle, pyridyle, pyrazinyle, pyrimidinyle, indolyle, quinoléyle, isoquinoléyle, quinoxalinyle, β-carbolinyle et un dérivé benzocondensé, cyclopenta-, cyclohexa- ou cyclohepta condensé de ces radicaux, qui peuvent être aussi entièrement ou partiellement saturés et non substitués ou substitués par un alkyle inférieur ; par carboxyalcanoyle inférieur, par alcoxy inférieur-carbonylalkanoyle inférieur, par hydroxyalcoxy inférieur-alcanoyle inférieur, par aminoalcanoyle inférieur ou par benzyloxycarbonylaminoalcanoyle inférieur, où le groupe amino n'est pas lié en position α- ou β-, est substitué par carbamoyle, par phénylalkyl inférieur-alkylaminocarbonyle, par N-dialkyl inférieur-aminoalkyl inférieur-N-alkyl inférieur-aminocarbonyle, par N-dihydroxyalkyl inférieur-N-alkyl inférieur-aminocarbonyle, par 2- ou 3-pyridylalkyl inférieur-aminocarbonyle, par N-2-pyridyl-alkyl inférieur-N-alkyl inférieur-aminocarbonyle, par sulfonyle, par alkyl inférieur-sulfonyle, par arylsulfonyle, où aryle a de 6 à 10 atomes de carbone et est non substitué ou substitué par alkyle inférieur ou alcoxy inférieur ; par hétéroxycyclylsulfonyle, où l'hétérocyclyle est choisi parmi les radicaux pyrrolyle, furyl, thiényle, imidazolyle, pyrazolyle, oxazolyle, thiazolyle, pyridyle, pyrazinyle, pyrimidinyle, indolyle, quinoléyle, isoquinoléyle, quinoxalinyle, β-carbonylyle et un dérivé benzocondensé, cyclopenta-, cyclohexa- ou cyclohepta-condensé de ces radicaux, qui peuvent être entièrement ou partiellement saturés; par sulfamoyle, ou par un sulfamoyle substitué par un hétérocyclylalkyle inférieur où l'hétérocyclyle est défini comme ci-dessus, et/ou par un alkyle inférieur, un groupe carboxy de la chaîne latérale se présente libre ou sous forme estérifiée comme groupe alkyl inférieur-ester, comme groupe aryl-ester ou comme groupe aryl-alkyl inférieur-ester, où l'aryle représente un phényle, un 4-nitrophényle, un naphtyle ou un biphénylyle, ou sous forme amidée comme un groupe carbamoyle, comme un groupe alkyle inférieur-carbamoyle, comme un groupe dialkyle inférieur-aminocarbamoyle, comme un groupe mono- ou di-(hydroxyalkyl inférieur)carbamoyle ou comme un groupe mono- ou di-(carboxyalkyl inférieur)carbamoyle,
un groupe amino de la chaîne latérale se présente libre ou sous forme alkylée comme mono- ou dialkyl inférieur-amino ou sous forme acylée comme alcanoyl inférieur-amino, comme aminoalcanoyl inférieur-amino, comme arylalcanoyl inférieur-amino, où l'aryle a de 6 à 14 atomes de carbone et est non substitué ou substitué par un alkyle inférieur, un hydroxy, un alcoxy inférieur, un carboxy, un carbamoyle ou un sulfamoyle, comme groupe alcoxy inférieur-carbonylamino, ou comme groupe aryl-méthoxycarbonylamino, où l'aryle a de 6 à 14 atomes de carbone, comme pipéridyl-1-carbonyle, morpholinocarbonyle, thiomorpholinocarbonyle ou comme S,S-dioxiothiomorpholinocarbonyle, et/ou un groupe hydroxy de la chaîne latérale se présente libre ou sous forme éthérifiée ou estérifiée comme groupe alcoxy inférieur, comme groupe arylalcoxy inférieur, comme groupe alcanoyloxy inférieur, ou comme groupe alkyloxy inférieur-carbonyloxy,
-alkyl inférieur-sulfonyle,
-2-ou-3-pyrrolyl, 2-thiényl-, 2-furyl-, 1-pyrazolyl-, 2-,3- ou 4-pyridyl-, 2-, 3- ou 5-indolyl-, (1-méthyl-, 2-méthyl-, 5-méthoxy-, 5-benzyloxy-, 5-chloro- ou 4,5-di-méthyl-indol-2-yl)-, (1-benzylindol-2-yl ou -3-yl)-, 4,5,6,7-tétrahydroindol-2-yl-, (2-, 3- ou 4-quinoléyl ou 4-hydroxyquinol-2-yl)-, 1-, 3- ou 4-isoquinoléyle ou 1-oxo-1,2-dihydroisoquinol-3-yl)-, 3-pyrrolidinyl-, (3- ou 4-hydropyrrolidin-2-yl)-, 5-oxo-pyrrolidin-2-yl-, (2- ou 3-morpholinyl)-, (2- ou 3-morpholinyl)-, (2- ou 3-thiomorpholinyl)-, (S,S-dioxothio-morpholin-2- ou -3-yl)-, (2- ou 3-indolinyl), (1,2,3,4-tétrahydroquinol-2-, -3- ou -4-yl)- ou (1,2,3,4-tétrahydroisoquinol-1-, -2- ou -3-yl)-méthylsulfonylphényl- ou 1- ou 2-naphtyl-sulfonyle non substitué ou mono-ou di-substitué par un alkyle inférieur,
-alcoxy inférieur-sulfonyle, ou
-benzyloxysulfonyle ou 1- ou 2- naphtyloxysulfonyle,
si bien qu'au plus un des radicaux R₁ ou R₉ peut représenter un hydrogène, et
R₂ et R₈ représentent indépendamment l'un de l'autre un hydrogène ou les mêmes radicaux que R₁ et R₉,
ou la paire de substituants R₁ et R₉ ou R₂ et R₈ forment un noyau hétérocyclique à chaque fois indépendamment l'un de l'autre ensemble avec l'azote de liaison et un radical choisi parmi l'éthylène, le triméthylène, le tétraméthylène et le pentaméthylène, où un atome de carbone peut être remplacé par un atome d'azote, un atome d'oxygène, un atome de soufre ou un atome de soufre mono- ou disubstitué par de l'oxygène et qui peut être insaturé,
R₃ représente
-un cycloalkyle inférieur, où le cycloalkyl a de 3 à 7 atomes de carbone et est non substitué ou mono- ou jusqu'à trisubstitué par un alkyle inférieur, un halogènoalkyle inférieur, un hydroxy, un alcoxy inférieur, un carbamoylalcoxy inférieur, un N-alkyl inférieur-carbamoylalcoxy inférieur ou un N,N-dialkyl inférieur-carbamoylalcoxy inférieur, un amino, un mono- ou un di-alkyl inférieur-amino, un halogène, un carboxy, un alcoxy inférieur-carbonyle, un phényl-, un naphtyl- ou un fluorénylalcoxy inférieur-carbonyle, un alcanoyle inférieur, un alkyl inférieur-sulfonyle, un phosphono, un hydroxy-alcoxy inférieur-phosphoryle ou un dialcoxy inférieur-phosphoryle, un carbamoyle, un mono- ou un di-alkyl inférieur-aminocarbamoyle, un sulfamoyle, un mono- ou un di-alkyl inférieur-sulfamoyle, un nitro et/ou un cyano et est lié à un alkyle inférieur, ou
-un arylalkyle inférieur avec l'aryle choisi parmi les radicaux phényle, indényle, indanyle, naphtyle, anthryle, phénantryle, acénaphtyle et fluorényle, qui est non substitué ou peut être mono- ou jusqu'à trisubstitué par un alkyle inférieur, un halogèno-alkyle inférieur, un phényle, un 1- ou 2-naphtyle, un hydroxy, un alcoxy inférieur, un carbamoylalcoxy inférieur, un N-alkyl inférieur-carbamoylalcoxy inférieur ou N,N-dialkyl inférieur carbamoylalcoxy inférieur, un amino, un mono-ou di-alkyl inférieur-amino, un alkanoyl inférieur-amino, un halogène, un carboxy, un alcoxy inférieur-carbonyle, un phényl-, naphtyl- ou fluorénylalcoxy inférieur-carbonyle, un alcanoyle inférieur, un sulfo, un alkyl inférieur-sulfonyle, un phosphono, un hydroxy-alcoxy inférieur-phosphoryle ou un dialcoxy inférieur-phosphoryle, un carbamoyle, un mono- ou dialkyl inférieur-carbamoyle, un sulfamoyle, un mono- ou dialkyl inférieur-sulfamoyle, un nitro et/ou un cyano, où le phényle peut être présent jusqu'à trois exemplaires, et par un alkyle inférieur, qui est non substitué ou substitué par un alkyle inférieur, un hétérocyclyle choisi parmi les radicaux pyrrolyle, furyle, thiényle, imidazolyle, pyrazolyle, oxazolyle, thiazolyle, pyridyle, pyrazinyle, pyrimidinyle, indolyle, quinoléyle, isoquinoléyle, quinoxalinyle, β-carbolinyle ou un dérivé benzocondensé, cyclopenta-, cyclohexa- ou cyclohepta-condensé de ces radicaux, qui peuvent également se présenter sous forme entièrement ou partiellement saturée, un hydroxy, un alcoxy inférieur, un alcanoyloxy inférieur, un acétoacétoxy, un amino- ou benzyloxycarbonylaminoalkanoyloxy inférieur, un arylalcanoyloxy inférieur, où l'aryle a de 6 à 10 atomes de carbone, un alcoxy inférieur-carbonyloxy, un mono- ou dialkyl inférieur-aminocarbonyloxy, un aryloxycarbonyloxy, où l'aryle a de 6 à 10 atomes de carbone, un arylalcoxy inférieur-carbonyloxy dans lequel l'aryle a de 6 à 12 atomes de carbone, un sulfonyloxy, un alkyl inférieur-sulfonyloxy, un phénylsulfonyloxy, un 2- ou 4-toluènesulfonyloxy ou un 1- ou 2-naphtylsulfonyloxy, un amino, un mono- ou un dialkyl inférieur-amino, un N-alcoxy inférieur-N-alkyl inférieur amino, un mono- ou un di-(phényl- ou naphtylalkyle inférieur)amino, un alcanoyl inférieur-amino, un carboxy, un carboxy estérifié choisi parmi alcoxy inférieur-carbonyle, aryloxycarbonyle, où aryle a de 6 à 10 atomes de carbone, un arylalcoxy inférieur-carbonyle, où aryle à de 6 à 12 atomes de carbone, un alcanoyle inférieur, un alkyl inférieur-sulfonyle, un hydroxy-alcoxy inférieur-phosphoryle et un dialcoxy inférieur-phosphoryle, un carbamoyle, par un ou deux radicaux choisis parmi les radicaux alkyle inférieur, carboxyalkyle inférieur ou alcoxy inférieur-carbonyl-alkyle inférieur, dialkyl inférieur-aminoalkyle inférieur, aminocarboxyalkyle inférieur, parmi le groupement hydroxyalkyle inférieur, et parmi dialcoxy inférieur-alkyle inférieur ou un radical choisi parmi éthylène, triméthylène, tétraméthylène et pentaméthylène, où un atome de carbone peut être remplacé par un atome d'azote, un atome d'oxygène, un atome de soufre ou un atome de soufre mono- ou disubstitué par de l'oxygène et qui peut être insaturé, un carbamoyle substitué, ou encore un sulfamoyle, un phosphono, un benzofuranyle, un oxo et/ou un cyano et est non ramifié ou ramifié,
R₄ représente un hydrogène,
R₅ représente un hydroxy et
R₆ représente un hydrogène,
ou R₅ et R₆ représentent ensemble un oxo, et
R₇ représente
-un alkyle inférieur,
-un cycloalkylalkyle inférieur, où cycloalkyle a de 3 à 7 atomes de carbone et est non substitué ou est mono- ou jusqu'à trisubstitué par un alkyle inférieur, un halogènoalkyle inférieur, un hydroxy, un alcoxy inférieur, un carbamoylalcoxy inférieur, un N-alkyl inférieur-carbamoylalcoxy inférieur ou un N,N-dialkyl inférieur-carbamoylalcoxy inférieur, un amino, mono- ou dialkyl inférieur-amino, un halogène, un carboxy, un alcoxy inférieur-carbonyle, un phényl-, un naphtyl- ou un fluorénylalcoxy inférieur-carbonyle, un alcanoyle inférieur, un alkyl inférieur-sulfonyle, un phosphono, un hydroxy-alcoxy inférieur-phosphoryle ou un di-alcoxy inférieur-phosphoryle, un carbamoyle, un mono- ou di-alkyl inférieur-aminocarbamoyle, un sulfamoyle, un mono- ou dialkyl inférieur-sulfamoyle, un nitro et/ou un cyano et est lié à un alkyle inférieur,
- un bicycloalkyl-alkyle inférieur, où bicycloalkyle comprend de 5 - 10 atomes de carbone,
- un tricycloalkyl-alkyle inférieur, où tricycloalkyle contient de 8 à 10 atomes de carbone,
- un arylalkyle inférieur dans lequel l'aryle est choisi parmi les groupements phényle, indényle, indanyle, naphtyle, anthryle, phénanthryl, acénaphtyle, et fluorényle, qui est non substitué ou peut être mono- ou jusqu'à trisubstitué par un alkyle inférieur, un halogènealkyle inférieur, un phényle, un 1- ou 2-naphtyle, un hydroxy, un alcoxy inférieur, un carbamoylalcoxy inférieur, un N-alkyl inférieur-carbarmoylalcoxy inférieur, un N,N-dialkyl inférieur-carbamoylalcoxy inférieur, un amino, un mono- ou di-alkyle inférieur-amino, un alcanoyle inférieur-amino, un halogène, un carboxy, un alcoxy inférieur-carbonyle, un benzyl-, un naphtyl- ou un fluorényl-alcoxy inférieur-carbonyle, un alcanoyle inférieur, un sulfo, un alkyl inférieur-sulfonyle, un phosphono, un hydroxy-alkyloxy inférieur-phosphoryle, un dialcoxy inférieur-phosphoryle, un carbamoyle, un mono- ou un dialkyl inférieur-carbamoyle, un sulfamoyle, un mono- ou un dialkyl inférieur-sulfamoyle, un nitro et/ou un cyano, où phényle peut être présent jusqu'à trois exemplaires, et avec un alkyle inférieur, qui est non substitué ou substitué par un alkyle inférieur, hétérocyclyle choisi parmi les groupements pyrrolyle, furyle, thiényle, imidazolyle, pyrazolyle, oxazolyle, thiazolyle, pyridyle, pyrazinyle, pyrimidinyle, indolyle, quinoléyle, isoquinoléyle, quinoxalinyle, β-carbolinyle et un dérivé benzocondensé, cyclopenta-, cyclohexa-, ou cyclohepta-condensé de ces radicaux , qui peuvent également être entièrment ou partielement saturés , un hydroxy, un alcoxy inférieur, un alcanoyloxy inférieur, un acétoacétoxy, un amino- ou un benzyloxycarbonylamino-alcanoyloxy inférieur, un arylalcanoyloxy inférieur, où l'aryle a de 6 à 10 atomes de carbone, un alcoxy inférieur-carbonyloxy, un mono- ou di-alkyl inférieur-aminocarbonyloxy, un aryloxycarbonyloxy, où l'aryle a de 6 à 10 atomes de carbone, un arylalcoxy inférieur-carbonyloxy, où l'aryle a de 6 à 12 atomes de carbone, un sulfonyloxy, un alkyl inférieur-sulfonyloxy, un phénylsulfonyloxy, un 2- ou 4-toluènesulfonyloxy, un 1- ou 2-naphtylsulfonyloxy, un amino, un mono- ou dialkyl inférieur-amino, un N-alcoxy inférieur-N-alkyl inférieur-amino, un mono- ou un di-(phényl- ou naph thyl-alkyl inférieur)amino, un alcanoyl inférieur-amino, un carboxy, un carboxy estérifié choisi parmi alcoxy inférieur-carbonyle, aryloxycarbonyle, où l'aryle a de 6 à 10 atomes de carbone, un arylalcoxy inférieur-carbonyle, où l'aryle a de 6 à 12 atomes de carbone, un alcanoyle inférieur, un alkyl inférieur-sulfonyle, un hydroxy-alcoxy inférieur-phosphoryle et un dialcoxy inférieur-phosphoryle, un carbamoyle, un carbamoyle substitué par un ou deux radicaux choisis parmi alkyle inférieur, parmi carboxyalkyle inférieur, parmi alcoxy inférieur carbonyl-alkyle inférieur, parmi dialkyl inférieur-aminoalkyle inférieur, parmi aminocarbonylalkyle inférieur, parmi hydroxyalkyle inférieur et parmi dialcoxy inférieur-alkyle inférieur ou un radical choisi parmi éthylène, triméthylène, tétraméthylène, et pentaméthylène, où un atome de carbone peut être remplacé par un atome d'azote, un atome d'oxygène, un atome de soufre ou un atome de soufre mono- ou disubstitué par de l'oxygène et peut être insaturé, un carbamoyle substitué, ou encore un sulfamoyle, un phosphono, un benzofuranyle, un oxo(qui n'est pas lié à l'atome de carbone qui est relié à l'atome d'azote liant le radical R₇) et/ou un cyano et est non ramifié ou ramifié,
-un héterocyclylalkyle inférieur, où héterocyclyle est de préférence choisi parmi les radicaux pyrrolyle, furyle, thiényle, imidazolyle, pyrazolyle, oxazolyle, thiazolyle, pyridyle, pyrazinyle, pyrimidinyle, indolyle, quinoléyle, isoquinoléyle, quinoxalinyle, β-carbolinyle et un dérivé benzocondensé, cyclopenta-, cyclohexa- ou cyclohepta- condensé de ces radicaux, qui peuvent également être partiellement ou entièrement saturés, par exemple un 4-pyrrolidinylméthyle, 1-imidazolylméthyle, 2-pyridylméthyle, 3-pyridylméthyle, quinoléine-2-ylméthyl ou indol-2-ylméthyl,
- un hydroxyalkyle inférieur,
- un alcoxy inférieur-alkyle inférieur,
- un phénoxyalkyle inférieur ou un nitrophénoxyalkyle inférieur,
- un naphtoxyalkyle inférieur,
- un alcanoyloxyalkyle inférieur,
- un acétoacétoxyalkyle inférieur,
- un arylmercaptoalkyle inférieur,où aryle a de 6 à 10 atomes de carbone,
- un aminoalkyle inférieur,
- un mono- ou dialkyl inférieur-aminoalkyle inférieur,
- un phényl- ou naphtylaminoalkyle inférieur,
- un alkanoyl inférieur-amino-alkyle inférieur,
- un pipérazinylcarbonylalkyle inférieur substitué sur l'atome d'azote par un alkyle inférieur,
- un alcoxy inférieur-carbonylamino-alkyle inférieur,
- un phénylalcoxy inférieur-carbonylamino-alkyle inférieur,
- un aminocarbonylamino-alkyle inférieur,
- un N-phénylalkyl inférieur-N-alkyl inférieur-aminocarbonylamino-alkyle inférieur,
- un halogènealkyle inférieur,
- un carboxyalkyle inférieur,
- un alcoxy inférieur-carbonylalkyle inférieur,
- un 2-halogènealcoxy inférieur- carbonyl-alkyle inférieur,
- un phényl- ou un naphtylalkyl inférieur-oxycarbonyl -alkyle inférieur,
- un hétérocyclylalcoxy inférieur-carbonyl-alkyle inférieur, où l'hétérocyclyle est choisi parmi pyrrolyle, furyle, thiényle, imidazolyle, pyrazolyle, oxazolyle, thiazolyle, pyridyle, pyrazinyle, pyrimidinyle, indolyle, quinoléyle, isoquinoléyle, quinoxalinyle, β-carbolinyle et un dérivé benzocondensé, cyclopenta-, cyclohexa- ou cyclohepta- condensé de ces radicaux,
où les radicaux mentionnés peuvent également être entièrement ou partiellement saturés,
- un alkyl inférieur-sulfonylalkyle inférieur,
- un arylsulfonylalkyle inférieur, où aryle a de 6 à 10 atomes de carbone,
- un carbamoylalkyle inférieur,
- un alkyl inférieur-carbamoylalkyle inférieur,
- un dialkyl inférieur-carbamoylalkyle inférieur,
- un hydroxyalkyl inférieur-carbamoyl- ou di-(hydroxyalkyle inférieur)carbamoyl-alkyle inférieur,
- un N-alcoxy inférieur-alcoxy inférieur-alkyl inférieur-carbamoyl-alkyle inférieur,
- un carboxyalkyl inférieur-carbamoyl- ou di-(carboxy-alkyl inférieur)carbamoyl-alkyle inférieur,
- un carbamoylalkyle inférieur substitué sur l'atome d'azote par un radical choisi parmi éthylène, triméthylène, tétraméthylène et pentaméthylène, où un atome de carbone peut être remplacé par un atome d'azote, un atome d'oxygène, un atome de soufre ou un atome de soufre mono- ou disubstitué par de l'oxygène, un carbamoylalkyle inférieur substitué sur l'atome d'azote, le radical ainsi formé pouvant également être entièrement ou partiellement insaturé,
- un N-alkyl inférieur-N-hétérocyclylalkyl inférieur-carbamoylalkyle inférieur, où hétérocyclyle est choisi parmi pyrrolyle, furyle, thiényle, imidazolyle, pyrazolyle, oxazolyle, thiazolyle, pyridyle, pyrazinyle, pyrimidinyle, indolyle, quinoléyle, isoquinoléyle, quinoxalinyle, β-carbolinyle et un dérivé benzocondensé, cyclopenta-, cyclohexa- ou cyclohepta-condensé de ces radicaux, qui peuvent également être entièrement ou partiellement saturés,
- un sulfamoylalkyle inférieur,
- un N-(phényl- ou naphtylalkyl inférieur)sulfamoyl-alkyle inférieur, - un sulfamoylalkyle inférieur substitué sur l'atome d'azote par un radical choisi parmi éthylène, triméthylène, tétraméthylène et pentaméthylène, où un atome de carbone peut être remplacé par un atome d'azote, un atome d'azote substitué par un alkyle inférieur, un atome d'oxygène, un atome de soufre ou un atome de soufre mono- ou disubstitué par de l'oxygène, le radical ainsi formé pouvant également être entièrement ou partiellement insaturé,
- un oxoalkyle inférieur (où oxo n'est pas lié à l'atome de carbone qui est relié à l'atome d'azote liant R₇),
- un cyanoalkyle inférieur,
- un hydroxycarboxy-alkyle inférieur,
- un α-naphtoxy-carboxy-alkyle inférieur,
- un hydroxy-alcoxy inférieur-carbonyl-alkyle inférieur,
- un α-naphtoxy-alcoxy inférieur-carbonylalkyle inférieur,
- un alkyl inférieur-carbonyl-halogène-alkyle inférieur,
- un α-naphtoxyéthoxycarbonyl-alkyle inférieur,
- un α-naphtoxy-benzyloxycarbonyl-alkye inférieur,
- un hydroxy estérifié-alcoxy inférieur-carbonyl-alkyle inférieur, où le groupe hydroxy est estérifié par un alcanoyle inférieur, un cycloalkylalcanoyle inférieur, où cycloalkyle a de 3 à 7 atomes de carbone, un bicycloalkylalcanoyle inférieur, où bicycloalkyle contient de 5 à 10 atomes de carbone, un tricycloalkylalcanoyle inférieur, où tricycloalkyle contient de 8 à 10 atomes de carbone, un arylalcannoyle inférieur, où aryle a de 6 à 12 atomes de carbone et est non substitué ou peut être mono- ou jusqu'à trisubstitué par un alkyle inférieur, un hydroxy, un alcoxy inférieur, un carbamoylalcoxy inférieur, un N-alkyl inférieur-carbamoylalcoxy inférieur ou un N,N-dialkyl inférieur-carbamoylalcoxy inférieur, un amino, un mono- ou di-alkyl inférieur-amino, un halogène, un carboxy, un alcoxy inférieur-carbonyle, un phényl-, naphtyl- ou fluorénylalcoxy inférieur-carbonyle, un alcanoyle inférieur, un alkyl inférieur-sulfonyle, un phosphono, un hydroxyalcoxy inférieur-phosphoryle, un dialcoxy inférieur-phosphoryle, un carbamoyle, un sulfamoyle, un nitro et/ou un cyano, un alcoxy inférieur-carbonyle, un 2-halogèno-alcoxy inférieur-carbonyle, ou un phényl-ou fluorényl-alcoxy inférieur,
- un dihydroxy-carboxy-alkyle inférieur,
- un dihydroxy-alcoxy inférieur-carbonyl-alkyle inférieur,
- un dihydroxy-alcoxy inférieur-carbonyl-alkyle inférieur estérifié par un alcanoyle inférieur, un alcoxy inférieur-carbonyle, un phényl- ou un fluorénylalcoxy inférieur-carbonyle, un alkyl inférieur-sulfonyle ou un toluènesulfonyle,
- un α-naphtoxy-dialkyl inférieur-amino-alkyle inférieur,
- un α-naphtoxy-carbamoyl-alkyle inférieur,
- un α-naphtoxy-oxo-alkyle inférieur (où oxo n'est pas lié à l'atome de crbone qui est relié à l'atome d'azote liant R₇), ou
- un α-naphtoxy-cyanoalkyle inférieur,
ainsi que les sels des composés mentionnés, dans la mesure où des groupes salificateurs sont présents, et où dans les définitions données ci-dessus l'expression "inférieur" signifie que le radical en question comporte jusqu'à 7 atomes de carbone compris.

5. Composés de formule I selon la revendication 1, dans laquelle R₁ représente un alcoxy inférieur-carbonyle, un phényl-alcoxy inférieur-carbonyle, le radical monovalent, lié par l'intermédiaire du groupe carboxy, d'un acide aminé aliphatique choisi parmi la valine, l'alanine, la leucine et l'isoleucine ou le radical, lié par l'intermédiaire du groupe carboxy, d'un acide aminé aliphatique acylé sur l'atome d'azote de l'amino par l'un des radicaux phénylalcanoyle inférieur, morpholinylalcanoyle inférieur, thiomorpholinylalcanoyle inférieur, S,S-dioxothiomorpholinylalcanoyle inférieur, pyridylalcanoyle inférieur, alcoxy inférieur-carbonyle ou phénylalcoxy inférieur-carbonyle, et tel que défini ci-dessus, tous les acides aminés mentionnés se présentant sous forme D, D,L ou L, R₂ représente un hydrogène, R₃ représente un phénylalkyle inférieur, R₄ représente un hydrogène, R₅ représente un hydroxy, R₆ représente un hydrogène, R₇ représente un alkyle inférieur, un cyclohexylalkyle inférieur ou un phénylalkyle inférieur, R₈ représente un hydrogène et R₉ représente l'un des radicaux mentionnés pour R₁ et les atomes de carbone asymétriques portant les radicaux R₃ et R₅ se présentant sous la configuration S, ainsi que les sels pharmacologiquement acceptables de ces composés, où dans les définitions données ci-dessus l'expression "inférieur" signifie que le radical en question comporte jusqu'à 7 atomes de carbone compris.

6. Composés de formule I selon la revendication 1, dans laquelle R₁ représente un tert-butoxycarbonyle, un benzyloxycarbonyle, le radical monovalent de l'acide aminé valine, lié par l'intermédiaire du groupe carboxy, ou le radical, lié par l'intermédiaire du groupe carboxy, de l'alanine acylée sur l'atome d'azote de l'amino par l'un des radicaux phénylacétyle, 3-pyridylacétyle, morpholinocarbonyle, thiomorpholinocarbonyle, tert-butoxycarbonyle ou benzyloxycarbonyle, R₂ représente un hydrogène, R₃ un benzyle, R₄ un hydrogène, R₅ un hydroxy, R₆ un hydrogène, R₇ un isobutyle, un cyclohexylméthyle ou un benzyle, R₈ représente un hydrogène et R₉ représente l'un des radicaux mentionnés pour R₁ et les atomes de carbone asymétriques portant les radicaux R₃ et R₅ se présentent sous la configuration S, ainsi que les sels pharmacologiquement acceptables de ces composés.

7. Le composé de formule I selon la revendication 1, dans laquelle R₁ et R₉ représentent chacun le radical monovalent, lié par l'intermédiaire du groupe carboxyle, acylé sur l'atome d'azote de l'amino par un benzyloxycarbonyle, de l'acide aminé (L)-valine, R₂ et R₈ représentent un hydrogène, R₃ représente un benzyle, R₄ représente un hydrogène, R₅ représente un hydroxy, R₆ représente un hydrogène et R₇ représente un benzyle et les atomes de carbone asymétriques portant les radicaux R₃ et R₅ se présentent sous la configuration S, ainsi que les sels pharmacologiquement acceptables de ces composés.

8. Le composé de formule I selon la revendication 1, dans laquelle R₁ et R₉ représentent chacun le radical monovalent de l'acide aminé (L)-valine, lié par l'intermédiaire du groupe carboxy, acylé sur l'atome d'azote de l'amino par un 4-thiomorpholinocarbonyle, R₂ et R₈ représentent un hydrogène, R₃ représente un benzyle, R₄ représente un hydrogène, R₅ représente un hydroxy, R₆ représente un hydrogène et R₇ représente un isobutyle et les atomes de carbone asymétriques portant les radicaux R₃ et R₅ se présentent sous la configuration S, ainsi que les sels pharmacologiquement acceptables de ce composé.

9. Composé de formule I selon la revendication 1, choisi parmi les composés désignés par
Boc-[Phe^{NN}Phe]-Boc,
Boc-(L)-Val-[Phe^{NN}Phe]←((L)-Val-Boc),
Boc-[Phe^{NN}Cha]-Boc,
H-(L)-Val-[Phe^{NN}Phe]←((L)-Val-H,
N-thiomorpholinocarbonyl-(L)-Val-[Phe^{NN}Phe]←(N-thio-morpholinocarbonyl-(L)-Val),
N-morpholinocarbonyl-(L)-Val[Phe^{NN}Phe]←(N-morpholino-carbonyl-(L)-Val),
Phénylacétyl-(L)-Val-[Phe^{NN}Phe]←(N-phénylacétyl-(L)-Val),
N-(3-pyridylacétyl)-(L)-Val-[Phe^{NN}Phe]←(N-(3-pyridylacétyl)-(L)-Val),
Boc-(L)-Val-[Phe^{NN}Cha]←((L)-Val)-Boc,
Z-(L)-Val-[Phe^{NN}Cha]←((L)-Val)-Z,
Boc-[Phe^{NN}Leu]-Boc,
Z-(L)-Val-[Phe^{NN}Leu]←((L)-Val)-Z, H-(L)-Val-[Phe^{NN}Cha]¬((L)-Val)-H et
N-(3-pyridylacétyl)-(L)-Val-[Phe^{NN}Leu]←(N-(3-pyridyl-acétyl)-(L)-Val), ou un de ses sels;
où Boc représente un tert-butoxycarbonyle, Z représente un benzyloxycarbonyle, le radical -[Phe^{NN}Phe]- représente le radical bivalent du 3(S)-amino-4-phényl-1-(N-benzylhydrazino)-butan-2(S)-ol et a la formule 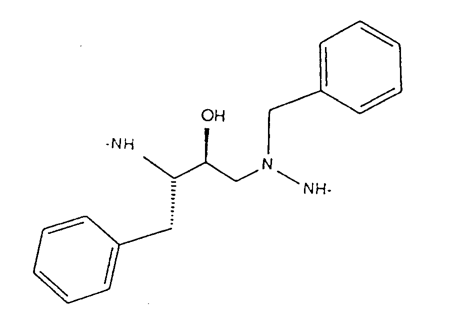 le radical -[Phe^{NN}Cha] représente le radical bivalent du 3(S)-amino-4-phényl-1-(N-cyclohexylméthylhydra-zino)-butan-2(S)-ol et a la formule 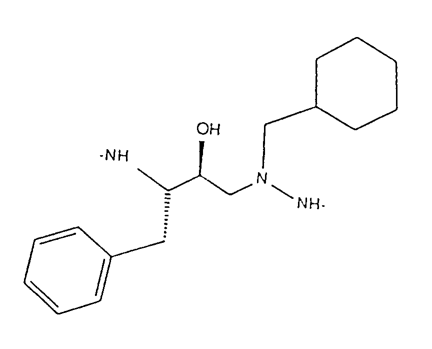 le radical -[Phe^{NN}Leu]- représente le radical bivalent du 3(S)-amino-4-phényl-1-(N-isobutylhydrazino)-butan-2(S)-ol et a la formule 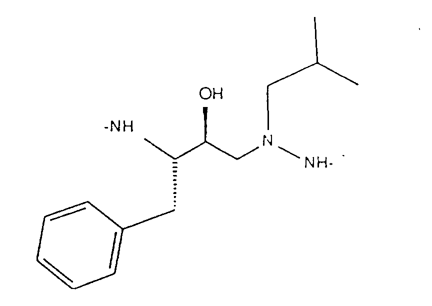 et une flèche "←" représente l'inversion de la direction de liaison en s'écartant de la nomenclature habituelle des peptides, dans laquelle l'extrémité amino se situe à gauche et l'extrémité carboxy se situe à droite.

10. Composé de formule I selon la revendication 1, choisi parmi les composés ayant les désignations
Z-(L)-Val-[(p-F)Phe^{NN}(p-F)Phe]←(N-(N-(2-pyridylmethyl)-N-methyl-aminocarbonyl)-(L)-Val,
Z-(L)-Val-[(p-F)Phe^{NN}(p-F)Phe]←(N-(2(R,S)-carbamoyl-3-phényl-propionyl)(L)-Val),
Acétyl-(L)-Val-[Phe^{NN}Cha]←(N-acétyl-(L)-Val),
Acétyl-Ile-[Phe^{NN}Cha]←(N-acétyl-Ile),
N-(2-pyridylméthyl)-N-méthyl-aminocarbonyl-(L)-Val-[Phe^{NN}(p-F)-Phe]←(N-(N-(2-pyridylméthyl)-N-méthyl-aminocarbonyl)-(L)-Val),
Z-(L)-Val-[Phe^{NN}(p-F)Phe]←((L)-Val)-Z,
Z-(L)-Val-[Phe^{NN}(p-CN)Phe]←((L)-Val)-Z,
Z-(L)-Ile-[Phe^{NN}Leu]←((L)-Ile)-Z,
Isobutoxycarbonyl-(L)-Val-[Phe^{NN}Leu]←(N-isobutoxycarbonyl-(L)-Val),
Acétyl-Val-[Phe^{NN}Leu]¬(N-(2(R,S)-carbamoy-3-phényl-propionyl)-Val),
N-trifluoroacétyl-[Phe^{NN}Leu]←(N-(2-(R,S)-carbamoyl-3-phényl-propionyl)-(L)-Val),
Z-(L)-Val-[Phe^{NN}Nle]←(N-(2(R,S)-N-(2-moropholinoéthyl)-carbamoyl)-3-méthyl)-butyryl),
Z-(L)-Val-[Phe^{NN}Nle]←(N-(2(R,S)-(N-(2-pyridylméthyl)-carbamoyl)-3-méthyl)-butyryl),
Méthoxycarbonyl-(L)-Val-[Phe^{NN}Leu]←(N-méthoxycarbonyl-(L)-Val),
Méthoxycarbonyl-(L)-Val-[Phe^{NN}(p-F)Phe]←(N-méthoxy-carbonyl-(L)-Val) et
Méthoxycarbonyl-(L)-Val-[Phe^{NN}(p-CN)Phe]←(N-méthoxy-carbonyl-(L)-Val),
ou un de ses sels, dans lequel Boc représente un tert-butoxycarbonyle, Z représente un benzyloxycarbonyle, le radical -[Phe^{NN}Phe]- représente le radical bivalent du 3(S)-amino-4-phényl-1-(N-benzylhydrazino)-butan-2(S)-ol et a la formule 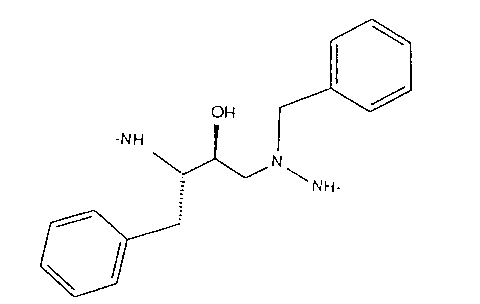 le radical -[Phe^{NN}Cha] est le radical bivalent du 3(S)-amino-4-phényl-1-(N-cyclohexylméthyl-hydrazino)-butan-2(S)-ol et a la formule 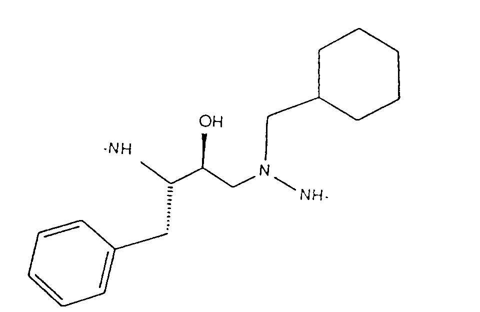 le radical -[Phe^{NN}Leu] est le radical bivalent du 3(S)-amino-4-phényl-1-(N-isobutylhydrazino)-butan-2(S)-ol et a la formule 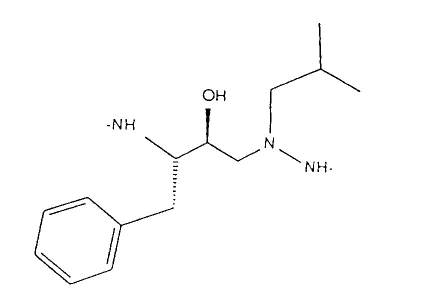 le radical ayant la désignation -[Phe^{NN}Nle] représente le radical du 3(S)-amino-4-phényl-1-(N-n-butyl-hydrazino)-butan-2(S)-ol et a la formule 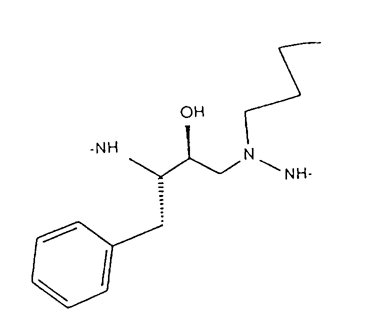 le radical ayant la désignation -[Phe^{NN}(p-F)Phe] représente le radical bivalent du 3(S)-amino-4-phényl-1-(N-(p-fluorophénylméthyl)-hydrazino)-butan-2(S)-ol et a la formule 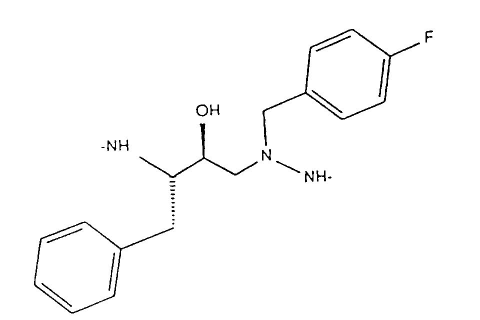 le radical ayant la désignation -[(p-F)Phe^{NN}(p-F)Phe] représente le radical bivalent du 3(S)-amino-4-(p-fluorophényl)-1-(N-(p-fluorophénylméthyl)-hydrazino)-butan-2(S)-ol et a la formule 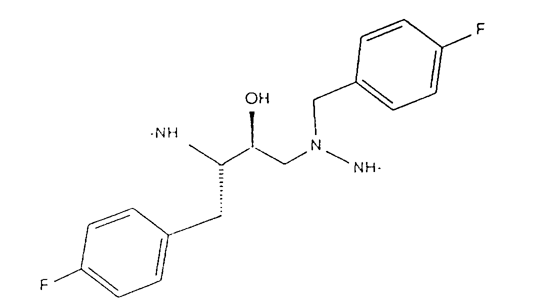 le radical ayant la désignation -[Phe^{NN}(p-CN)Phe] représente le radical bivalent du 3(S)-amino-4-phényl-1-(N-(p-cyanophénylméthyl)-hydrazino)-butan-2(S)-ol et a la formule 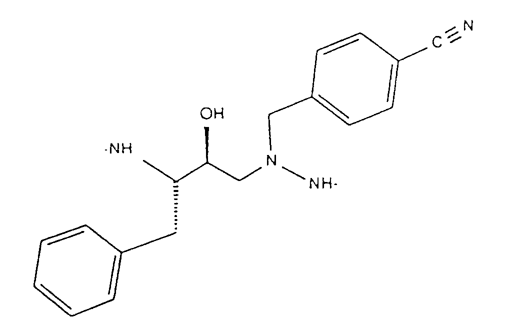 et une flèche "←" représente l'inversion de la direction de liaison en s'écartant de la nomenclature habituelle des peptides, où l'extrémité amino se situe à gauche et l'extrémité carboxy se situe à droite.

11. Composés de formule I selon l'une des revendications 1-10 aux fins d'application dans un procédé de traitement thérapeutique du corps humain ou animal.

12. Préparations pharmaceutiques contenant des composés de formule I selon l'une des revendications 1-10 ou un sel pharmaceutiquement acceptable d'un tel composé ayant au moins un groupe salificateur, avec un support pharmaceutiquement acceptable.

13. Application d'un des composés de formule I mentionnés dans les revendications 1-10 ou d'un sel pharmaceutiquement acceptable d'un tel composé ayant au moins un groupe salificateur, à la préparation de préparations pharmaceutiques destinées à être utilisées dans le traitement du SIDA.

14. Procédé de préparation d'un composé de formule I selon la revendication 1, dans laquelle les substituants ont les significations mentionnées dans la revendication 1, et de leurs sels, caractérisé en ce que
a) on ajoute un dérivé d'hydrazine de formule 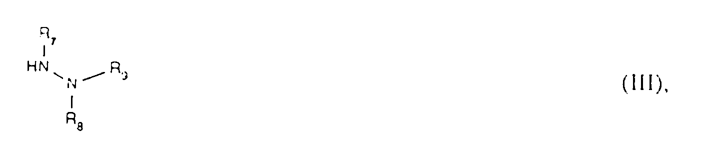 dans laquelle les radicaux ont les significations mentionnées ci-dessus, à un époxyde de formule 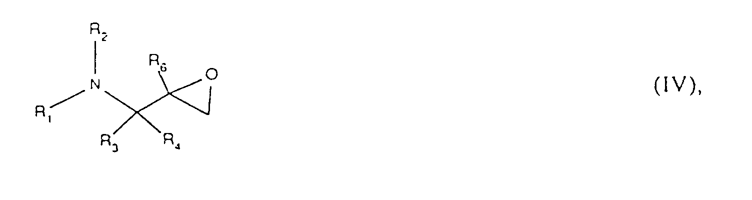 dans laquelle les radicaux ont les significations mentionnées ci-dessus, opération dans laquelle les groupes fonctionnels libres à l'exception de ceux qui participent à la réaction se présentent le cas échéant sous forme protégée, et on sépare les groupes protecteurs éventuellement présents, ou
b) pour préparer des composés de formule I dans laquelle R₁ et R₉ représentent un acyle, un sulfo, un sulfonyle substitué par un alkyle non substitué ou substitué, un aryle, un alcoxy, qui est non substitué ou substitué, ou un aryloxy, un sulfamoyle, qui est non substitué ou substitué sur l'atome d'azote, ou un phosphoryle substitué par un ou deux radicaux choisis indépendamment l'un de l'autre parmi alkyle substitué ou non substitué, cycloalkyle non substitué ou substitué, aryle, hydroxy, alcoxy non substitué ou substitué, cycloalcoxy et aryloxy, R₂ et R₈ représentent un hydrogène, un alkyle non substitué ou substitué, un alcényle ou un alcynyle, ou un hétérocyclyle, et les autres radicaux ont les significations mentionnées, on condense un composé amino de formule 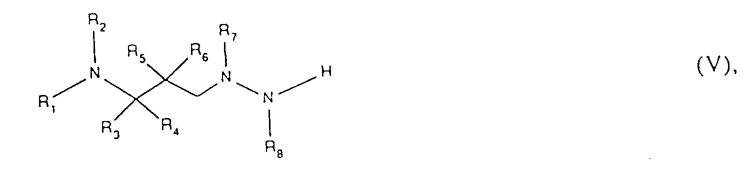 dans laquelle les radicaux ont les significations déjà mentionnées, avec un acide de formule
R₉-OH (VI)
ou un de ses dérivés acides réactifs, où R₉ a les significations déjà mentionnées, les groupes fonctionnels libres, à l'exception de ceux qui participent à la réaction, se présentant le cas échéant sous forme protégée, et on sépare les groupes protecteurs éventuellement présents, ou
c) pour préparer des composés de formule I, dans laquelle R₁ et R₉ représentent un acyle, un sulfo, un sulfonyle substitué par un alkyle non substitué ou substitué, un aryle, un alcoxy, qui est non substitué ou substitué, ou un aryloxy, un sulfamoyle, qui est non substitué ou substitué sur l'atome d'azote, ou un phosphoryle substitué par un ou deux radicaux choisis indépendamment l'un de l'autre parmi alkyle substitué ou non substitué, cycloalkyle non substitué un substitué, aryle, hydroxy, alcoxy non substitué ou non substitué, cycloalcoxy et aryloxy, R₂ et R₈ représentent un hydrogène, un alkyle, alcényle ou alcynyle non substitué ou substitué, un hétérocyclyle et les autres radicaux ont les significations mentionnées, on condense un composé amino de formule. 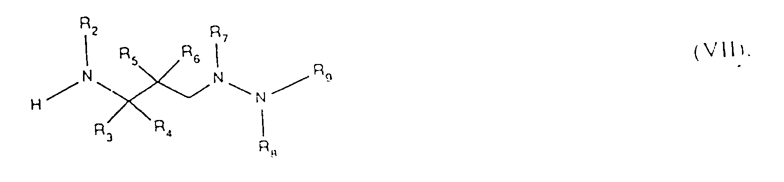 dans laquelle les radicaux ont les significations déjà mentionnées, avec un acide de formule
R₁-OH (VIII)
ou un de ses dérivés acides réactifs, où R₁ a les significations déjà mentionnées, opération dans laquelle les groupes fonctionnels libres, à l'exception de ceux qui participent à la réaction, se présentent le cas échéant sous forme protégée, et on sépare les groupes protecteurs éventuellement présents, ou
d) pour préparer des composés de formule I, dans laquelle R₁ et R₉ représentent deux radicaux identiques choisis parmi acyle, sulfo, sulfonyle substitué par un alkyle non substitué ou substitué, un aryle, un alcoxy, qui est non substitué ou substitué, ou un aryloxy, sulfamoyle, qui est non substitué ou substitué sur l'atome d'azote, et phosphoryle substitué par un ou deux radicaux choisis indépendamment l'un de l'autre parmi alkyle substitué ou non substitué, cycloalkyle non substitué ou substitué, aryle, hydroxy, alcoxy non substitué ou substitué, cycloalcoxy, cycloalcoxy et aryloxy, R₂ et R₈ représentent un hydrogène, un alkyle, alcényle ou alcynyle non substitué ou substitué, ou un hétérocyclyle et les autres radicaux ont les significations mentionnées, on condense un composé diamino de formule 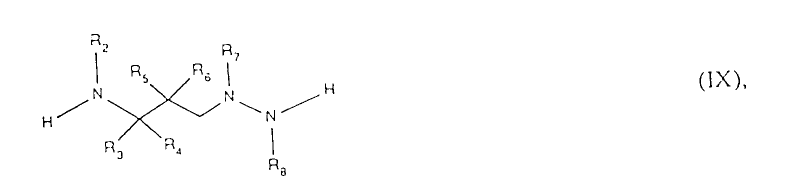 dans laquelle les radicaux ont les significations déjà mentionnées, avec un acide approprié pour l'introduction des radicaux identiques R₁ et R₉ ou un de ses dérivés acides réactifs, où R₁ et R₉ ont les significations déjà mentionnées, les groupes fonctionnels libres, à l'exception de ceux qui participent à la réaction, se présentent le cas échéant sous forme protégée, et on sépare les groupes protecteurs éventuellement présents, ou
e) pour préparer un composé de formule I, dans laquelle à la place de R₇ on trouve le radical R₇'', qui représente un alkyle ou un cycloalkyle non substitué ou substitué, on introduit le radical R₇'' dans un composé de formule I', 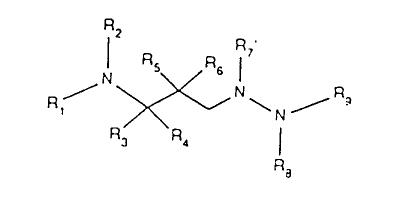 dans laquelle R₇' représente un hydrogène et les autres radicaux ont les significations mentionnées, par substitution avec un composé de formule XII,
R₇''-X (XII)
dans laquelle X représente un groupe sortant et R₇'' représente un alkyle ou cycloalkyle non substitué ou substitué, les groupes fonctionnels présents, à l'exception de ceux qui participent à la réaction, se présentant le cas échéant sous forme protégée, et on sépare les groupes protecteurs éventuellement présents, ou
f) dans un composé de formule I, dans laquelle les substituants ont les significations mentionnées, sous réserve que dans le composé de formule I en question au moins un groupe fonctionnel est protégé par des groupes protecteurs, on sépare les groupes protecteurs présents,
et si on le désire on transforme un composé de formule I que l'on peut obtenir selon l'un des procédés a) à f) ci-dessus et ayant au moins un groupe salificateur, en un sel, ou un sel obtenu en le composé libre ou en un autre sel, et/ou on sépare des mélanges d'isomères que l'on peut le cas échéant obtenir et/ou on transforme un composé de formule I selon l'invention en un autre composé de formule I selon l'invention.

15. Composés de formule 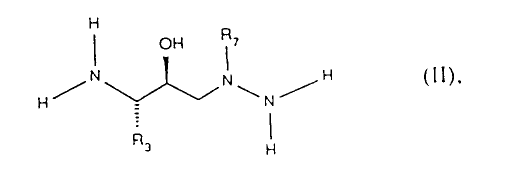 dans laquelle R₃ représente un alkyle ou cycloalkyle non substitué ou substitué; un aryle; un hétérocyclyle; ou un alcényle non substitué ou substitué; et R₇ représente un alkyle ou cycloalkyle non substitué ou substitué; un aryle; un hétérocyclyle; ou un alcényle non substitué ou substitué, ainsi que les sels des composés mentionnés, dans la mesure où des groupes salificateurs sont présen

## Revendications (Revendications pour l'(les) Etat(s) contractant(s) suivant(s): ES, GR)

1. Procédé de préparation de composés de formule 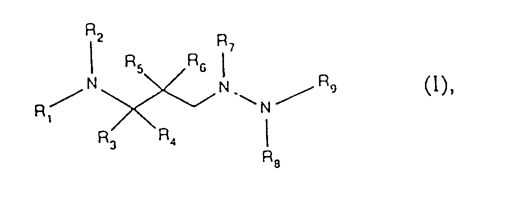 dans laquelle R₁ et R₉ représentent indépendamment l'un de l'autre un hydrogène, un acyle, un alkyle, un alcényle ou un alcynyle non substitué ou substitué; un hétérocyclyle; un sulfo, un sulfonyle substitué par un alkyle non substitué ou substitué, un aryle, un alcoxy, qui est non substitué ou substitué, ou par un aryloxy; un sulfamoyle, qui est non substitué ou substitué sur l'atome d'azote; ou un phosphoryle substitué par un ou deux radicaux choisis indépendamment l'un de l'autre parmi un alkyle non substitué ou substitué, un cycloalkyle non substitué ou substitué, un aryle, un hydroxy, un alcoxy non substitué ou substitué, un cycloalcoxy et un aryloxy, à la condition qu'au plus un des radicaux R₁ et R₉ représente un hydrogène; et R₂ et R₈ représentent, indépendamment l'un de l'autre un hydrogène ou un des radicaux mentionnés ci-dessus pour R₁ et R₉ ; ou les paires de substituants R₁ et R₂ ou R₈ et R₉ représentent chacun indépendamment l'un de l'autre avec l'atome d'azote auquel ils sont liés un noyau hétérocyclique qui est constitué de l'atome d'azote liant avec un radical choisi parmi l'éthylène, le triméthylène, le tétraméthylène et le pentaméthylène, dans lequel un atome de carbone peut être remplacé par un atome d'azote, d'oxygène, de soufre ou par un atome de soufre mono- ou bisubstitué par un oxygène et qui peut être insaturé, ou peuvent former le radical naphtalène-1,8-dicarbonylimido;
R₃ et R₄ représentent indépendamment l'un de l'autre un hydrogène, un alkyle ou cycloalkyle non substitué ou substitué; un aryle; un hétérocyclyle; ou un alcényle non substitué ou substitué; ou R₃ et R₄ représentent ensemble un alkylène non substitué ou substitué, un alkylidène ou un alkylène benzocondensé;
R₅ représente un hydroxy; R₆ représente un hydrogène;
ou R₅ et R₆ représentent ensemble un oxo;
et R₇ représente un alkyle ou cycloalkyle non substitué ou substitué; un aryle; un hétérocyclyle; ou un alcényle non substitué ou substitué;
ainsi que des sels des composés mentionnés, dans la mesure où des groupes salificateurs sont présents; à l'exception du composé de formule I, dans laquelle R₁, R₂ , R₃, R₄, R₆ et R₈ représentent chacun un hydrogène, R₅ représente un hydroxy, R₇ représente un radical de formule -[CH₂-(C=O)-NH-(N-CH₃)-CH₂-COOH et R₉ représente un tert-butoxycarbonyle;
caractérisé en ce que
a) on ajoute un dérivé d'hydrazine de formule 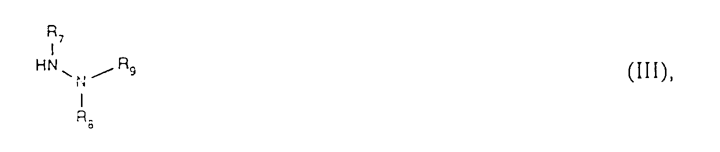 dans laquelle les radicaux ont les significations mentionnées ci-dessus, à un époxyde de formule 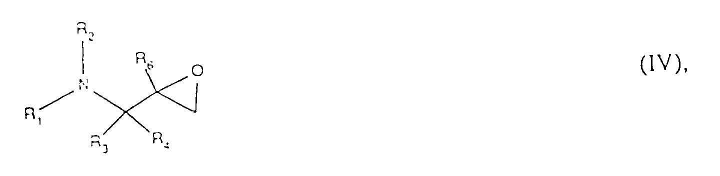 dans laquelle les radicaux ont les significations mentionnées ci-dessus, opération dans laquelle les groupes fonctionnels libres à l'exception de ceux qui participent à la réaction se présentent le cas échéant sous forme protégée, et on sépare les groupes protecteurs éventuellement présents, ou
b) pour préparer des composés de formule I dans laquelle R₁ et R₉ représentent un acyle, un sulfo, un sulfonyle substitué par un alkyle non substitué ou substitué, un aryle, un alcoxy, qui est non substitué ou substitué, ou un aryloxy, un sulfamoyle, qui est non substitué ou substitué sur l'atome d'azote, ou un phosphoryle substitué par un ou deux radicaux choisis indépendamment l'un de l'autre parmi un alkyle substitué ou non substitué, un cycloalkyle non substitué ou substitué, un aryle, un hydroxy, un alcoxy non substitué ou substitué, cycloalcoxy et aryloxy, R₂ et R₈ représentent un hydrogène, un alkyle non substitué ou substitué, un alcényle ou un alcynyle, ou un hétérocyclyle, et les autres radicaux ont les significations mentionnées, on condense un composé amino de formule 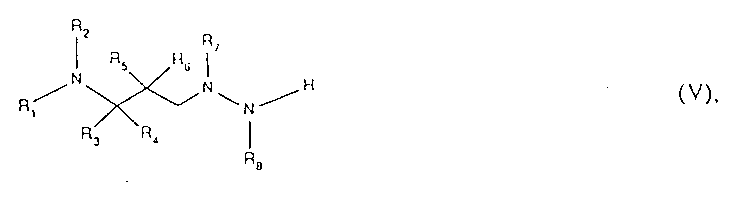 dans laquelle les radicaux ont les significations déjà mentionnées, avec un acide de formule
R₉-OH (VI)
ou un de ses dérivés acides réactifs, où R₉ a les significations déjà mentionnées, les groupes fonctionnels libres, à l'exception de ceux qui participent à la réaction, se présentant le cas échéant sous forme protégée, et on sépare les groupes protecteurs éventuellement présents, ou
c) pour préparer des composés de formule I, dans laquelle R₁ et R₉ représentent un acyle, un sulfo, un sulfonyle substitué par un alkyle non substitué ou substitué, un aryle, un alcoxy, qui est non substitué ou substitué, ou un aryloxy, un sulfamoyle, qui est non substitué ou substitué sur l'atome d'azote, ou un phosphoryle substitué par un ou deux radicaux choisis indépendamment l'un de l'autre parmi un alkyle substitué ou non substitué, un cycloalkyle non substitué ou substitué, un aryle, un hydroxy, un alcoxy non substitué ou substitué, un cycloalcoxy et un aryloxy, R₂ et R₈ représentent un hydrogène, un alkyle, alcényle ou alcynyle non substitué ou substitué, ou un hétérocyclyle et les autres radicaux ont les significations mentionnées, on condense un composé amino de formule 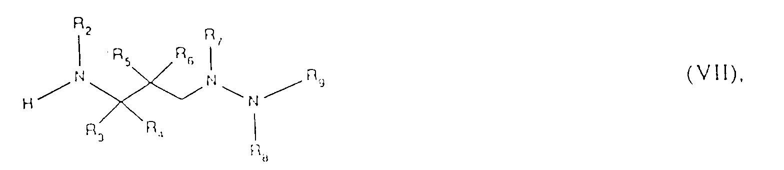 dans laquelle les radicaux ont les significations déjà mentionnées, avec un acide de formule
R₁-OH (VIII)
ou un de ses dérivés acides réactifs, où R₁ a les significations déjà mentionnées, opération dans laquelle les groupes fonctionnels libres, à l'exception de ceux qui participent à la réaction, se présentent le cas échéant sous forme protégée, et on sépare les groupes protecteurs éventuellement présents, ou
d) pour préparer des composés de formule I, dans laquelle R₁ et R₉ représentent deux radicaux identiques choisis parmi acyle, sulfo, sulfonyle substitué par un alkyle non substitué ou substitué, un aryle, un alcoxy, qui est non substitué ou substitué, ou un aryloxy, sulfamoyle, qui est non substitué ou substitué sur l'atome d'azote, et phosphoryle substitué par un ou deux radicaux choisis indépendamment l'un de l'autre parmi alkyle substitué ou non substitué, cycloalkyle non substitué ou substitué, aryle, hydroxy, alcoxy non substitué ou substitué, cycloalcoxy, cycloalcoxy et aryloxy, R₂ et R₈ représentent un hydrogène, un alkyle, alcényle ou alcynyle non substitué ou substitué, ou un hétérocyclyle et les autres radicaux ont les significations mentionnées, on condense un composé diamino de formule 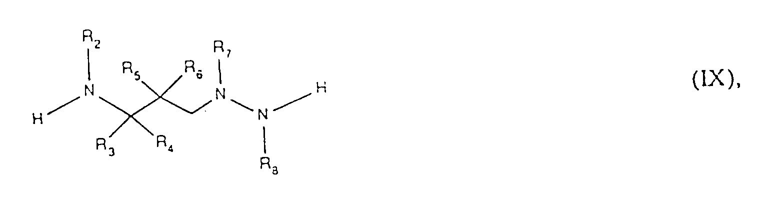 dans laquelle les radicaux ont les significations déjà mentionnées, avec un acide approprié pour l'introduction des radicaux identiques R₁ et R₉ ou un de ses dérivés acides réactifs, où R₁ et R₉ ont les significations déjà mentionnées, les groupes fonctionnels libres, à l'exception de ceux qui participent à la réaction, se présentent le cas échéant sous forme protégée, et on sépare les groupes protecteurs éventuellement présents, ou
e) pour préparer un composé de formule I, dans laquelle à la place de R₇ on trouve le radical R₇'', qui représente un alkyle ou un cycloalkyle non substitué ou substitué, on introduit le radical R₇'' dans un composé de formule I', 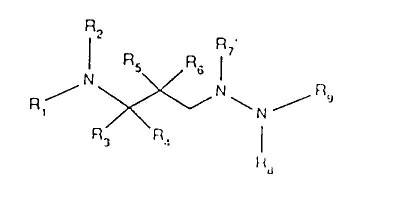 dans laquelle R₇' représente un hydrogène et les autres radicaux ont les significations mentionnées,
par substitution avec un composé de formule XII,
R₇''-X (XII)
dans laquelle X représente un groupe sortant et R₇'' représente un alkyle ou cycloalkyle non substitué ou substitué, les groupes fonctionnels présents, à l'exception de ceux qui participent à la réaction, se présentant le cas échéant sous forme protégée, et on sépare les groupes protecteurs éventuellement présents, ou
f) dans un composé de formule I, dans laquelle les substituants ont les significations mentionnées, sous réserve que dans le composé de formule I en question au moins un groupe fonctionnel est protégé par des groupes protecteurs, on sépare les groupes protecteurs présents,
et si on le désire on transforme un composé de formule I que l'on peut obtenir selon l'un des procédés a) à f) ci-dessus et ayant au moins un groupe salificateur, en un sel, ou un sel obtenu en le composé libre ou en un autre sel, et/ou on sépare des mélanges d'isomères que l'on peut le cas échéant obtenir et/ou on transforme un composé de formule I selon l'invention en un autre composé de formule I selon l'invention.

2. Procédé selon la revendication 1 de préparation d'un composé de formule I, dans laquelle
R₁ et R₉ représentent indépendamment l'un de l'autre un hydrogène; un alcanoyle inférieur; un aryl-alcanoyle inférieur, dans lequel l'alcanoyle inférieur est non substitué ou substitué par un carbamoyle ou par un carbamoyle substitué sur l'atome d'azote par un ou deux radicaux choisis parmi les groupements alkyle inférieur, carboxyalkyle inférieur, alcoxy inférieur-carbonyl-alkyle inférieur, dialkyl inférieur-amino-alkyle inférieur, amino-carboxy-alkyl inférieur, hydroxyalkyle inférieur et dialcoxy inférieur-alkyle inférieur; un hétérocyclylalcanoyle inférieur; un halogèno-alcanoyle inférieur qui contient jusqu'à trois atomes d'halogène; un (N-hétérocyclylalkyl inférieur-carbamoyl)-alcanoyle inférieur; un alcoxy inférieur-carbonyle; un arylalcoxy inférieur-carbonyle; un hétérocyclylalcoxy inférieur-carbonyle; un alkyl inférieur-sulfonyle, un N-hétérocyclylalkyl inférieur-N-alkyl inférieur-carbamoyle; ou un radical lié par le groupe acyle d'un acide aminé dont la fonction amino est libre ou acylée par l'un des radicaux R₁ et R₉ mentionnés jusqu'à présent; sous réserve qu'au plus un des radicaux R₁ et R₉ représente un hydrogène;
R₂, R₄, R₆ et R₈ représentent un hydrogène,
R₃ représente un alkyle inférieur, un cycloalkyl-alkyle inférieur ou un aryl-alkyle inférieur,
R₅ représente un hydroxy, et
R₇ représente un alkyle inférieur non substitué ou substitué,
ou d'un de ses sels, dans la mesure où au moins un groupe salificateur est présent, où dans les définitions mentionnées ci-dessus l'expression "inférieur" signifie que le radical en question comporte jusqu'à 7 atomes de carbone compris; caractérisé en ce qu'on utilise des produits de départ substitués de façon correspondante.

3. Procédé selon la revendication 1 de préparation d'un composé de formule I, dans laquelle
R₁ et R₉ représentent indépendamment l'un de l'autre un hydrogène; un alcanoyle inférieur; un arylalcanoyle inférieur dans lequel l'aryle représente un groupement phényle, indényle, indanyle, naphtyle, anthryle, phénanthryle, acénaphtyle ou fluorényle et peut être non substitué ou mono- à trisubstitué par un alkyle inférieur, un halogèno-alkyle inférieur, un phényle, un 1- ou un 2-naphtyle, un hydroxy, un alcoxy inférieur, un carbamoylalcoxy inférieur, un N-alkyl inférieur-carbamoyl-alcoxy inférieur, un N,N-dialkyl inférieur-carbamoylalcoxy inférieur, un amino, un mono- ou di-alkyl inférieur-amino, un alcanoyl inférieur-amino, un halogène, un carboxy, un alcoxy inférieur-carbonyle, un phényl-, naphtyl-ou fluorénylalcoxy inférieur-carbonyle, un alcanoyle inférieur, un sulfo, un alkyl inférieur-sulfonyle, un phosphono, un hydroxyalcoxy inférieur-phosphoryle, un dialcoxy inférieur-phosphoryle, un carbamoyle, un mono- ou di-alkyl inférieur-carbamoyle, un sulfamoyle, un mono- ou di-alkyle inférieur-aminosulfonyle, un nitro et/ou un cyano, où le phényle peut être présent jusqu'à trois fois, et où un alcanoyle inférieur est non substitué ou substitué par un carbamoyle ou par un carbamoyle substitué sur l'atome d'azote par un ou deux radicaux choisis parmi les groupements alkyle inférieur, carboxyalkyle inférieur, alcoxy inférieur-carbonylalkyle inférieur, dialkyl inférieur-amino-alkyle inférieur, amino-carboxy-alkyle inférieur, hydroxyalkyle inférieur et dialcoxy inférieur-alkyle inférieur; un hétérocyclylalcanoyle inférieur, où l'alcanoyle inférieur est non substitué et où l'hétérocyclyle est choisi parmi les radicaux thiényle, furyle, pyranyle, pyrrolyle, imidazolyle, pyrazolyle, oxazolyle, isoxazolyle, thiazolyle, furazanyle, pyridyle, pyrazinyle, pyrimidinyle, pyridazinyle, azépinyle, indolyle, benzimidazolyle, 1H-indazolyle, quinoléyle, isoquinoléyle, quinoxalinyle, quinazolinyle, cinnolyle, purinyle, ptéridinyle, naphtyridinyle, 4H-quinolizinyle, 3,1-benzofuranyle, 4,1-benzoxazinyle, 4,1-benzothiazinyle, cyclohexa[b]pyrrolyle, cyclohepta[b]pyrrolyle, cyclohexa[b]pyrazolyle, cyclohexa[b]pyridyle, cyclohexa[b]pyrazinyle, cyclohexa[b]pyrimidinyle, cyclohexa[b]-1,4-oxazinyle, cyclohexa[b]-1,4-thiazinyle, pyrrolidinyle, pyrrolinyle, imidazolidyle, 2-imidazolinyle, 2,3-dihydropyridyle, pipéridyle, pipérazinyle, 2,3,5,6-tétrahydropyrazinyle, morpholinyle, thiomorpholinyle, S,S-dioxothiomorpholinyle, indolinyle, isoindolinyle, 4,5,6,7-tétrahydroindolyle, 1,2,3,4-tétrahydroquinoléyle, 1,2,3,4-tétrahydroisoquinoléyle, chromanyle, thiochromanyle, 1,2,3,4-tétrahydro-3,1-benzodiazinyle, 3,4-dihydro-3H-4,1-benzoxazinyle et 3,4-dihydro-3H-4,1-benzothiazinyle, les radicaux hétérocyclyle mentionnés étant non substitués ou substitués par un alkyle inférieur, un phényle, un 1- ou 2-naphtyle, un phénylalkyle inférieur, un hydroxyalkyle inférieur, un alcoxy inférieur-alkyle inférieur, un phénoxy- ou naphtoxyalkyle inférieur, un phénylalcoxy inférieur- ou naphtylalcoxy inférieur-alkyle inférieur, un alcanoyloxy inférieur-alkyle inférieur, un phényl- ou naphtyl-alcanoyloxy inférieur-alkyle inférieur, un alcoxy inférieurcarbonyloxy-alkyle inférieur, un phényl-, naphtyl- ou fluorényl-alcoxy inférieur-carbonyloxy-alkyle inférieur, un aminoalkyle inférieur, un carboxyalkyle inférieur, un hydroxy, un alcoxy inférieur, un phényl- ou naphtyl-alcoxy inférieur, un amino, un alkyl inférieur-amino, un di-alkyl inférieur-amino, un carboxy, un alcoxy inférieur-carbonyle, un phényl- ou naphtyl-alcoxy inférieur-carbonyle, un halogène, un alcanoyle inférieur, un alkyl inférieur-sulfonyle, un phosphono, un hydroxy-alcoxy inférieur-phosphoryle ou un dialcoxy-phosphoryle, un carbamoyle, un mono-ou di-alkyl inférieur-carbamoyle, un hydroxy- ou carboxy-alkyl inférieur-carbamoyle, un sulfamoyle, par un nitro, un oxo et/ou par un cyano; un hétérocyclylalcanoyle inférieur comme un halogèno-alcanoyle inférieur, qui contient jusqu'à trois atomes d'halogène; un (N-hétérocyclylalkyl inférieur-carbamoyl)-alcanoyle inférieur, où hétérocyclyle est choisi parmi pyrrolyle, furyle, thiényle, imidazolyle, pyrazolyle, oxazolyle, thiazolyle, pyridyle, pyrazinyle, pyrimidinyle, indolyle, quinoléyle, isoquinoléyle, quinoxalinyle, β-carbolinyle et un dérivé benzocondensé, cyclopenta-, cyclohexa- ou cyclohepta-condensé de ces radicaux, qui peuvent également être entièrement ou partiellement saturés, parmi morpholine et parmi thiomorpholine; un alcoxy inférieur-carbonyle; un arylalcoxy inférieur-carbonyle, où aryle est un phényle, biphénylyle, 1- ou 2-naphtyle, un fluorényle ou un phényle mono- ou polysubstitué par un alkyle inférieur, un hydroxy, un alcoxy inférieur, un halogène et/ou un nitro; un hétérocyclylalcoxy inférieur-carbonyle, où hétérocyclyle est choisi parmi pyrrolyle, furyle, thiényle, imidazolyle, pyrazolyle, oxazolyle, thiazolyle, pyridyle, pyrazinyle, pyrimidinyle, indolyle, quinoléyle, isoquinoléyle, quinoxalinyle, β-carbolinyle et un dérivé benzocondensé, cyclopenta-, cyclohexa- ou cyclohepta-condensé de ces radicaux, qui peuvent également être entièrement ou partiellement saturés, un alkyle inférieur-sulfonyle; un N-hétérocyclylalkyle inférieur-N-alkyle inférieur-carbamoyle, où hétérocyclyle est choisi parmi thiényle, furyle, pyranyle, pyrrolyle, imidazolyle, pyrazolyle, oxazolyle, isoxazolyle, thiazolyle, furazanyle, pyridyle, pyrazinyle, pyrimidinyle, pyridazinyle, azépinyle, indolyle, benzimidazolyle, 1H-indazolyle, quinoléyle, isoquinoléyle, quinoxalinyle, quinazolinyle, cinnolyle, purinyle, ptéridinyle, naphtyridinyle, 4H-quinolizinyle, 3,1-benzofuranyle, benz[e]indolyle, 4,1-benzoxazinyle, 4,1-benzothiazinyle, carbazolyle, β-carbolinyle, phénazinyle, phénanthridyle, acridyle, phénoxazinyle, phénothiazinyle, 1-azaacénaphtényle, cyclohexa[b]pyrrolyle, cyclohepta[b]pyrrolyle, cyclohexa[d]pyrazolyle, cyclohexa[b]pyridyle, cyclohexa[b]pyrazinyle, cyclohexa[b]pyrimidinyle, cyclohexa[b]-1,4-oxazinyle, cyclohexa[b]-1,4-thiazinyle, pyrrolidinyle, pyrrolinyle, imidazolidinyle, 2-imidazolinyle, 2,3-dihydropyridyle, pipéridyle, pipérazinyle, 2,3,5,6-tétrahydropyrazinyle, morpholinyle, thiomorpholinyle, S,S-dioxothiomorpholinyle, indolinyle, isoindolinyle, 4,5,6,7-tétrahydroindolyle, 1,2,3,4-tétrahydroquinoléyle 1,2,3,4-tétrahydroisoquinoléyle, chromanyle, thiochromanyle, 1,2,3,4-tétrahydro-3,1-benzodiazinyle, 3,4-dihydro-3H-4,1-benzoxazinyle, 3,4-dihydro-3H-4,1-benzothiazinyle, 2,3,4,5-tétrahydro-1H-5,1-benzazépinyle et 5,6-dihydrophénanthridinyle, où les radicaux mentionnés sont non substitués ou substitués par un alkyle inférieur, un phényle, un 1-ou 2-naphtyle, un phénylalkyle inférieur, un hydroxyalkyle inférieur, un alcoxy inférieur-alkyle inférieur, un phénoxy- ou naphtoxy-alkyle inférieur, un phénylalcoxy inférieur- ou naphtylalcoxy inférieur-alkyle inférieur, un alcanoyloxy inférieur-alkyle inférieur, un phényl- ou naphtyl-alcanoyloxy inférieur-alkyle inférieur, un alcoxy inférieur-carbonyloxy-alkyle inférieur, un phényl-, naphtyl- ou fluorényl-alcoxy inférieur-carbonyloxy-alkyle inférieur, un aminoalkyle inférieur, un carboxyalkyle inférieur, un hydroxy, un alcoxy inférieur, un phényl- ou naphtylalcoxy inférieur, un amino, un alkyl inférieur-amino, un dialkyle inférieur-amino, un carboxy, un alcoxy inférieur-carbonyle, un phényl- ou naphtyl-alcoxy inférieur-carbonyle, un halogène, un alcanoyle inférieur, un alkyl inférieur-sulfonyle, un phosphono, un hydroxy-alcoxy inférieur-phosphoryle ou un dialcoxy-phosphoryle, un carbamoyle, un mono-ou dialkyl inférieur-carbamoyle, un hydroxy- ou carboxy-alkyl inférieur-carbamoyle, un sulfamoyle, par un nitro, un oxo et/ou par un cyano; ou un radical acyle d'un acide aminé, dont la fonction amino est libre ou est acylée par un des autres radicaux mentionnés jusqu'ici pour R₁ et R₉ , où les acides aminés sont choisis parmi la glycine, l'alanine, la valine, la norvaline, la leucine, l'isoleucine, la norleucine, la sérine, l'homosérine, la thréonine, la méthionine, la cystéine, la proline, la trans-3- et la trans-4-hydroxyproline, la phénylalanine, la tyrosine, la 4-aminophénylalanine, la 4-chlorophénylalanine, la 4-carboxyphénylalanine, la β-phénylsérine, la phénylglycine, l'a-naphtylalanine, la cyclohexylalanine, la cyclohexylglycine, le tryptophane, l'acide aspartique, l'asparagine, l'acide aminomalonique, le monoamide de l'acide aminomalonique, l'acide glutamique, la glutamine, l'histidine, l'arginine, la lysine, la δ-hydroxylysine, l'omithine, l'acide 3-aminopropanoïque, l'acide α,γ-diaminobutyrique et l'acide α,β-diaminopropionique, où (en-dehors de la glycine) chacun des radicaux acide aminé mentionnés se présente sous forme D, L ou (D,L); sous réserve qu'au plus un des radicaux R₁ et R₉ représente un hydrogène,
R₂, R₄, R₆ et R₈ représentent un hydrogène,
R₃ représente un alkyle inférieur; un cycloalkyl-alkyle inférieur, dans lequel le cycloalkyle comporte de 3 à 7 atomes de carbone et est non substitué ou est mono-ou jusqu'à trisubstitué par un alkyle inférieur, un halogèno-alkyle inférieur, un hydroxy, un alcoxy inférieur, un carbamoylalcoxy inférieur, un N-alkyle inférieur-carbamoylalcoxy inférieur, un N,N-dialkyle inférieur-carbamoylalcoxy inférieur, un amino, un mono- ou dialkyl inférieur-amino, un halogène, un carboxy, un alcoxy inférieur-carbonyle, un phényl-, naphtyl- ou fluorénylalcoxy inférieur-carbonyle, un alcanoyle inférieur, un alkyl inférieur-sulfonyle, un phosphono, un hydroxy-alcoxy inférieur-phosphoryle ou un dialcoxy inférieur-phosphoryle, un carbamoyle, un mono- ou dialkyl inférieur-aminocarbamoyle, un sulfamoyle, un mono- ou dialkyl inférieur-sulfamoyle, un nitro et/ou un cyano; ou un arylalkyle inférieur, où l'aryle représente un phényle, indényle, indanyle, naphtyle, anthryle, phénanthryle, acénaphtyle ou fluorényle et est non substitué ou peut être mono-ou jusqu'à trisubstitué par un alkyle inférieur, un halogèno-alkyle inférieur, un phényle, un 1- ou 2-naphtyle, un hydroxy, un alcoxy inférieur, un carbamoylalcoxy inférieur, un N-alkyl inférieur-carbamoylalcoxy inférieur, un N,N-dialkyl inférieurcarbamoylalcoxy inférieur, un amino, un mono- ou dialkyl inférieur-amino, un alcanoyl inférieur-amino, un halogène, un carboxy, un alcoxy inférieur-carbonyle, un benzyl-, naphtyl- ou fluorényl-alcoxy inférieur-carbonyle, un alcanoyle inférieur, un sulfo, un alkyl inférieur-sulfonyle, un phosphono, un hydroxy-alcoxy inférieur-phosphoryle, un dialcoxy inférieur-phosphoryle, un carbamoyle, un mono- ou dialkyl inférieur-carbamoyle, un sulfamoyle, un mono-ou dialkyl inférieur-sulfamoyle, un nitro et/ou un cyano, le phényle pouvant être présent jusqu'à trois exemplaires,
R₅ représente un hydroxy, et
R₇ représente un alkyle inférieur non substitué; ou un cycloalkyl-alkyle inférieur, comme indiqué en dernier lieu pour cycloalkyl-alkyle inférieur R₃ ; ou un arylalkyle inférieur, comme indiqué en dernier pour arylalkyle inférieur R₃ ;
ou d'un de ses sels, dans la mesure où au moins un groupe salificateur est présent, et où dans les difinitions données ci-dessus l'expression "inférieur" signifie que le radical en question comporte jusqu'à 7 atomes de carbone compris; caractérisé en ce qu'on utilise des produits de départ substitués de façon correspondante.

4. Procédé selon la revendication 1 de préparation d'un composé de formule I, dans laquelle
R₁ et R₉ représentent indépendamment l'un de l'autre
- un hydrogène,
- un alcoxy inférieur-carbonyle,
- un 2-halogèno-alcoxy inférieur-carbonyle,
- un aryloxycarbonyle, où l'aryle a de 6 à 14 atomes de carbone,
- un arylalcoxy inférieur-carbonyle, où l'aryle a de 6 à 14 atomes de carbone,
- un hétérocyclylalcoxy inférieur-carbonyle, où l'hétérocyclyle est choisi parmi les groupements pyrrolyle, furyle, thiényle, imidazolyle, pyrazolyle, oxazolyle, thiazolyle, pyridyle, pyrazinyle, pyrimidinyle, indolyle, quinoléyle, isoquinoléyle, quinoxalinyle, β-carbolinyle et un dérivé benzocondensé, cyclopenta-, cyclohexa- ou cyclohepta-condensé de ces radicaux, qui peuvent également être entièrement ou partiellement saturés,
- un 2-trialkyl inférieur-silylalcoxy inférieur-carbonyle,
- un 2-triarylsilylalcoxy inférieur-carbonyle, où l'aryle est un phényle ou un 1-ou 2-naphtyle,
- le radical, lié par l'intermédiaire du groupe carboxy, d'un acide aminé choisi parmi la glycine, l'alanine, la valine, la norvaline, la leucine, l'isoleucine, la norleucine, la sérine, l'homo-sérine, la thréonine, la méthionine, la cystéine, la proline, la trans-3- et la trans-4-hydroxy-proline, la phénylalanine, la tyrosine, la 4-amino-phénylalanine, la 4-chlorophénylalanine, la 4-carboxyphénylalanine, la β-phénylsérine, la phénylglycine, l'α-naphtylalanine, la cyclohexyl-alanine, la cyclohexylglycine, le tryptophane, l'acide indoline-2-carboxylique, l'acide 1,2,3,4-tétrahydroisoquinoléine-3-carboxylique, l'acide aspartique, l'asparagine, l'acide aminomalonique, le monoamide de l'acide aminomalonique, l'acide glutamique, la glutamine, l'histidine, l'arginine, la lysine, la δ-hydroxylysine, l'ornithine, l'acide α,γ-diaminobutyrique et l'acide α,β-diamino-propionique, chacun de ces acides aminés pouvant se présenter sous forme D, L ou (D,L), et
le groupe α-amino est non substitué ou mono- ou bi- N-alkylé par un alkyle inférieur, par un aminoalkyle inférieur, par un phényl- ou un naphtylaminoalkyle inférieur, ou un pipérazinylcarbonylalkyle inférieur substitué sur l'atome d'azote par un alkyle inférieur, ou N-acylé par un alcanoyle inférieur, par un arylalcanoyle inférieur, où l'aryle est choisi parmi les groupements phényle, indényle, indanyle, naphtyle, anthryle, phénanthryle, acénaphtyle et fluorényle, et est non substitué ou peut être mono- ou jusqu'à trisubstitué par un alkyle inférieur, un halogènoalkyle inférieur, un phényle, un 1- ou 2-naphtyle, un hydroxy, un alcoxy inférieur, un carbamoylalcoxy inférieur, un N-alkyl inférieur-carbamoylalcoxy inférieur ou un N,N-dialkyl inférieur-carbamoylalcoxy inférieur, un amino, un mono-ou un dialkyl inférieur-amino, un alcanoyl inférieur-amino, un halogène, un carboxy, un alcoxy inférieur-carbonyle, un phényl-, naphtyl- ou fluorénylalcoxy inférieur-carbonyle, un alcanoyle inférieur, un sulfo, un alkyl inférieur-sulfonyle, un phosphono, un hydroxy-alcoxy inférieur-phosphoryle ou un dialcoxy inférieur-phosphoryle, un carbamoyle, un mono- ou di-alkyl inférieur-carbamoyle, un sulfamoyle, un mono- ou di-alkyl inférieur-amino-sulfonyle, un nitro et/ou un cyano; où le phényle peut être présent jusqu'à trois exemplaires, et un alcanoyle inférieur non substitué ou substitué par un alkyle inférieur, un hétérocyclyle choisi parmi les groupements pyrrolyle, furyle, thiényle, imidazolyle, pyrazolyle, oxazolyle, thiazolyle, pyridyle, pyrazinyle, pyrimidinyle, indolyle, quinoléyle, isoquinoléyle, quinoxalinyle, β-carbolinyle et un dérivé benzo-, cyclopenta-, cyclohexa- ou cyclohepta-condensé de ces radicaux, qui peuvent également être entièrement ou partiellement saturés, un hydroxy, un alcoxy inférieur, un alcanoyloxy inférieur, un acétoacétoxy, un amino- ou benzyloxycarbonylaminoalcanoyloxy inférieur, un arylalcanoyloxy inférieur, où être entièrement ou partiellement condensés, un hydroxy, un alcoxy inférieur, un alcanoyloxy inférieur, un acétoacétoxy, un amino- ou berzyloxycarbonylamino-alcanoyloxy inférieur, un arylalcanoyloxy inférieur, où l'aryle a de 6 à 10 atomes de carbone, un alcoxy inférieur-carbonyloxy, un mono- ou dialkyl inférieur-aminocarbonyloxy, un aryloxycarbonyloxy, où l'aryle a de 6 à 10 atomes de carbone, un arylalcoxy inférieur-carbonyloxy où l'aryle a de 6 à 14 atomes de carbone, un sulfonyloxy, un alkyl inférieur-sulfonyloxy, un phénylsulfonyloxy, un 2- ou 4-toluènesulfonyloxy ou un 1- ou 2-naphtylsulfonyloxy, carboxy, un carboxy estérifié choisi parmi un alcoxy inférieur-carbonyle, un aryloxycarbonyle, où l'aryle a de 6 à 10 atomes de carbone, un aryl-alcoxy inférieur-carbonyle, où l'aryle a de 6 à 12 atomes de carbone, un alcanoyle inférieur, un alkyl inférieur-sulfonyle, un hydroxy-alcoxy inférieur-phosphoryle et un di-alcoxy inférieur-phosphoryle, un carbamoyle, un carbamoyle substitué par un ou deux radicaux choisis parmi les groupements alkyle inférieur, carboxyalkyle inférieur, alcoxy inférieur carbonyl-alkyle inférieur, un dialkyl inférieur-amino-alkyle inférieur, aminocarboxyalkyle inférieur, hydroxyalkyle inférieur et parmi dialcoxy inférieur-alkyle inférieur ou un radical choisi parmi éthylène, triméthylène, tétraméthylène, pentaméthylène, dans lequel un atome de carbone peut être remplacé par un atome d'azote, un atome d'oxygène, un atome de soufre , ou un atome de soufre mono-ou di-substitué par un atome d'oxygène et qui peut être insaturé, ou encore peut être un sulfamoyle, un phosphono, un benzofuranyle, un oxo et/ou cyano et est non ramifié ou ramifié ; par un hétérocyclylalcanoyle inférieur choisi parmi les radicaux thiényl-, furyl-, pyranyl-, pyrrolyl-, imidazolyl-, pyrazolyl-, oxazolyl-, isoxazolyl-, thiazolyl-, furazanyl-, pyridyl-, pyrazinyl-, pyrimidinyl-, pyridazinyl-, azépinyl-, indolyl-, benzimidazolyl-, 1H-indazolyl-, quinoléyl-, isoquinoléyl-, quinoxalinyl-, quinazolinyl-, cinnolyl-, purinyl-, ptéridinyl-, naphthyridinyl-, 4H-quinolizinyl-, 3,1-benzofuranyl benz[e]indolyl-, 4,1-benzoxazinyl-, 4,1-benzothiazinyl-, carbazolyl-, β-carbolinyl-, phénazinyl-, phénanthridyl-, acridyl-, phénoxazinyl-, phénothiazinyle, 1-azaacénaphtényl-, cyclohexa[b]pyrrolyl-, cyclohepta[b]pyrrolyl-, cyclohexa[d]pyrazolyl-, cyclohexa[b]pyridyl-, cyclohexa[b]pyrazinyl-, cyclohexa[b]pyrimidinyl- cyclohexa[b]-1,4-oxazinyl-, cyclohexa[b]-1,4-thiazinyl-, pyrrolidinyl-, pyrrolinyl-, imidazolidinyl-, 2-imidazolinyl-, 2,3-dihydropyridyl-, pipéridyl-, pipérazinyl-, 2,3,5,6-tétrahydropyrazinyl-, morpholinyl-, thiomorpholinyl-, S,S-dioxo-thiomorpholinyl-, indolinyl-, isoindolinyl-, 4,5,6,7-tétrahydroindolyl-, 1,2,3,4-tétrahydroquinoléyl-, 1,2,3,4-tétrahydroisoquinoléyl-, chroman-, thiochroman-, 1,2,3,4-tétrahydro-3,1-benzodiazinyl-, 3,4-dihydro-3H-4,1-benzoxazinyl-, 3,4-dihydro-3H-4,1-benzothiazinyl-, 2,3,4,5-tétrahydro-1H-5,1-benzazépinyl et 5,6-dihydrophénanthridinyl-alcanoyle inférieur, où les radicaux hétérocyclyles mentionnés sont non substitués ou substitués par un alkyle inférieur, un phényle, un 1- ou 2-naphtyle, un phénylalkyle inférieur, un hydroxyalkyle inférieur, un alkoxy inférieur-alkyle inférieur, un phénoxy- ou un naphtoxyalkyle inférieur, un phénylalcoxy inférieur- ou un naphtylalcoxy inférieur-alkyle inférieur, un alcanoyloxy inférieur-alkyle inférieur, un phényl- ou un naphtyl-alcanoyloxy inférieur-alkyle inférieur, un alcoxy inférieur-carbonyloxy-alkyle inférieur, un phényl-, un naphtyl- ou un fluorénylalcoxy inférieur-carbonyloxy-alkyle inférieur, un aminoalkyle inférieur, un carboxyalkyle inférieur, un hydroxy, un alcoxy inférieur, un phényl- ou un naphtylalcoxy inférieur, un amino, un alkyl inférieur-amino, un dialkyl inférieur-amino, un carboxy, un alkoxy inférieur- carbonyle, un phényl- ou un naphtyl-alcoxy inférieur-carbonyle, un halogène, un alcanoyle inférieur, un alkyl inférieur-sulfonyle, un phosphono, un hydroxy-alcoxy inférieur-phosphoryle, un dialcoxy-phosphoryle, un carbamoyle, un mono-ou di-alkyl inférieur-carbamoyle, un hydroxy- ou carboxyalkyl inférieur-carbamoyle, un sulfamoyle, par un nitro, un oxo et/ou par un cyano ; par un hétérocyclylalcénoyle inférieur, où l'hétérocyclyle est choisi parmi les radicaux un pyrrolyle, furyle, thiényle, imidazolyle, pyrazolyle, oxazolyle, thiazolyle, pyridyle, pyrazinyle, pyrimidinyle, indolyle, quinoléyle, isoquinoléyle, quinoxalinyle, β-carbolinyle et un dérivé benzocondensé, cyclopenta-, cyclohexa- ou cyclohepta- condensé de ces radicaux, qui peuvent également être entièrement ou partiellement saturés, par un alcoxy inférieur-carbonyle par un arylalcoxy inférieur-carbonyle, où l'aryle a de 6 à 14 atomes de carbone ; par un hétérocyclylalcoxy inférieur-carbonyle, où l'hétérocyclyle est choisi parmi les radicaux pyrrolyle, furyle, thiényle, imidazolyle, pyrazolyle, oxazolyle, thiazolyle, pyridyle, pyrazinyle, pyrimidinyle, indolyle, quinoléyle, isoquinoléyle, quinoxalinyle, β-carbolinyle et un dérivé benzocondensé, cyclopenta-, cyclohexa- ou cyclohepta-condensé de ces radicaux, qui peuvent être aussi entièrement ou partiellement saturés et non substitués ou substitués par alkyle inférieur ; par carboxyalcanoyle inférieur, par alcoxy inférieur-carbonylalkanoyle inférieur, par hydroxyalcoxy inférieur-alcanoyle inférieur, par aminoalcanoyle inférieur ou par benzyloxycarbonylaminoalcanoyle inférieur, où le groupe amino n'est pas lié en position α- ou β-, est substitué par un carbamoyle, par un phénylalkyl inférieur-aminocarbonyle, par un N-dialkyl inférieur-amino-alkyle inférieur-N-alkyl inférieur-aminocarbonyle, par un N-dihydroxyalkyl inférieur-N-alkyl inférieur-aminocarbonyle, par 2- ou 3-pyridylalkyl inférieur-aminocarbonyle, par N-2-pyridyl-alkyl inférieur-N-alkyl inférieur-aminocarbonyle, par sulfonyle, par alkyl inférieur-sulfonyle, par arylsulfonyle, où l'aryle a de 6 à 10 atomes de carbone et est non substitué ou substitué par alkyle inférieur ou alcoxy inférieur ; par hétéroxycyclylsulfonyle, où l'hétérocyclyle est choisi parmi les radicaux pyrrolyle, furyle, thiényle, imidazolyle, pyrazolyloxazolyle, thiazolyle, pyridyle, pyrazinyle, pyrimidinyle, indolyle, quinoléyle, isoquinoléyle, quinoxalinyle, β-carbonylyle et un dérivé benzocondensé, cyclopenta-, cyclohexa- ou cyclohepta-condensé de ces radicaux, qui peuvent être entièrement ou partiellement saturés ; par un sulfamoyle, ou par un sulfamoyle substitué par un hétérocyclylalkyle inférieur où l'hétérocyclyle est défini comme ci-dessus, et/ou par un alkyle inférieur, un groupe carboxy de la chaîne latérale se présente libre ou sous forme estérifiée comme groupe alkyl inférieur-ester, comme groupe arylester ou comme groupe aryl-alkyl inférieur-ester, où l'aryle représente un phényle, un 4-nitrophényle, un naphtyle ou un biphénylyle, ou sous forme amidée comme un groupe carbamoyle, comme un groupe alkyle inférieur-carbamoyle, comme un groupe dialkyl inférieur-amino-carbamoyle, comme un groupe mono- ou di-(hydroxyalkyl inférieur)carbamoyle ou comme un groupe mono- ou di-(carboxyalkyl inférieur)carbamoyle,
un groupe amino de la chaîne latérale se présente libre ou sous forme alkylée comme mono- ou dialkyle inférieur-amino ou sous forme acylée comme alcanoyle inférieur-amino, comme aminoalcanoyle inférieur-amino, comme arylalcanoyle inférieur amino, où l'aryle a de 6 à 14 atomes de carbone et est non substitué ou substitué par un alkyle inférieur, un hydroxy, un alcoxy inférieur, un carboxy, un carbamoyle ou un sulfamoyle, comme groupe alcoxy inférieur-carbonylamino, ou comme groupe aryl-méthoxycarbonylamino, où l'aryle a de 6 à 14 atomes de carbone, comme pipéridyl-1-carbonyle, morpholinocarbonyle, thiomorpholinocarbonyle ou comme S,S-dioxiothiomorpholinocarbonyle, et/ou un groupe hydroxy de la chaîne latérale se présente libre ou sous forme éthérifiée ou estérifiée comme groupe alcoxy inférieur, comme groupe arylalcoxy inférieur, comme groupe alcanoyloxy inférieur, ou comme groupe alkyloxy inférieur-carbonyloxy,
-alkyl inférieur-sulfonyle,
-2-ou-3-pyrrolyl, 2-thiényl-, 2-furyl-, 1-pyrazolyl-, 2-,3- ou 4-pyridyl-, 2-, 3- ou 5-indolyl-, (1-méthyl-, 2-méthyl-, 5-méthoxy-, 5-benzyloxy-, 5-chloro- ou 4,5-di-méthyl-indol-2-yl)-, (1-benzylindol-2-yl ou -3-yl)-, 4,5,6,7-tétrahydroindol-2-yl-, (2-, 3- ou 4-quinoléyl ou 4-hydroxyquinol-2-yl)-, 1-, 3- ou 4-isoquinoléyle ou 1-oxo-1,2-dihydroisoquinol-3-yl), 3-pyrrolidinyl-, (3- ou 4-hydropyrrolidin-2-yl)-, 5-oxo-pyrrolidin-2-yl-, (2- ou 3-morpholinyl)-, (2- ou 3-thiomorpholinyl)-, (S,S-dioxothio-morpholin-2- ou -3-yl)-, (2- ou 3-indolinyl), (1,2,3,4-tétrahydroquinol-2-, -3- ou -4-yl)- ou (1,2,3,4-tétrahydroisoquinol-1-, -2- ou -3-yl)-méthylsulfonylphényl- ou 1- ou 2-naphtyl-sulfonyle non substitué ou mono-ou di-substitué par un alkyle inférieur,
-alcoxy inférieur-sulfonyle, ou
-benzyloxysulfonyle ou 1- ou 2- naphtyloxysulfonyle,
si bien qu'au plus un des radicaux R₁ ou R₉ peut représenter un hydrogène, et
R₂ et R₈ représentent indépendamment l'un de l'autre un hydrogène ou les mêmes radicaux que R₁ et R₉,
ou la paire de substituants R₁ et R₉ ou R₂ et R₈ forment un noyau hétérocyclique à chaque fois indépendamment l'un de l'autre ensemble avec l'azote de liaison et un radical choisi parmi éthylène, triméthylène tétraméthylène et pentaméthylène, ou un atome de carbone peut être remplacé par un atome d'azote, un atome d'oxygène, un atome de soufre ou un atome de soufre mono- ou disubstitué par de l'oxygène et qui peut être insaturé,
R₃ représente
-un cycloalkyl inférieur, où le cycloalkyle a de 3 à 7 atomes de carbone et est non substitué ou mono- ou jusqu'à trisubstitué par un alkyle inférieur, un halogènoalkyle inférieur, un hydroxy, un alcoxy inférieur, un carbamoylalcoxy inférieur, un N-alkyl inférieur-carbamoylalcoxy inférieur ou un N,N-dialkyl inférieur-carbamoylalcoxy inférieur, un amino, un mono- ou un di-alkyl inférieur-amino, un halogène, un carboxy, un alcoxy inférieur-carbonyle, un phényl-, un naphtyl- ou un fluorénylalcoxy inférieur-carbonyle, un alcanoyle inférieur, un alkyl inférieur-sulfonyle, un phosphono, un hydroxy-alcoxy inférieur-phosphoryle ou un dialcoxy inférieur-phosphoryle, un carbamoyle, un mono- ou un di-alkyl inférieur-aminocarbamoyle, un sulfamoyle, un mono- ou un di-alkyl inférieur-sulfamoyle, un nitro et/ou un cyano et est lié à un alkyle inférieur, ou
-un arylalkyle inférieur avec l'aryle choisi parmi les radicaux phényle, indényle, indanyle, naphtyle, anthryle, phénantryle, acénaphtyle et fluorényle, qui est non substitué ou peut être mono- ou jusqu'à trisubstitué par un alkyle inférieur, un halogèno-alkyle inférieur, un phényle, un 1- ou 2-naphtyle, un hydroxy, un alcoxy inférieur, un carbamoylalcoxy inférieur, un N-alkyl inférieur-carbamoylalcoxy inférieur ou N,N-dialkyl inférieur carbamoylalcoxy inférieur, un amino, un mono-ou di-alkyl inférieur-amino, un alkanoyl inférieur-amino, un halogène, un carboxy, un alcoxy inférieur-carbonyle, un phényl-, naphtyl- ou fluorénylalcoxy inférieur-carbonyle, un alcanoyle inférieur, un sulfo, un alkyl inférieur-sulfonyle, un phosphono, un hydroxy-alcoxy inférieur-phosphorylde ou un dialcoxy inférieur-phosphoryle, un carbarmoyle, un mono- ou dialkyl inférieur-carbamoyle, un sulfamoyle, un mono- ou dialkyl inférieur-sulfamoyle, un nitro et/ou un cyano, où le phényle peut être présent jusqu'à trois exemplaires, et par un alkyle inférieur, qui est non substitué ou substitué par un alkyle inférieur, un hétérocyclyle choisi parmi les radicaux pyrrolyle, furyle, thiényle, imidazolyle, pyrazolyle, oxazolyle, thiazolyle, pyridyle, pyrazinyle, pyrimidinyle, indolyle, quinoléyle, isoquinoléyle, quinoxalinyle, β-carbolinyle ou un dérivé henzocondensé, cyclopenta-, cyclohexa- ou cyclohepta-condensé de ces radicaux, qui peuvent également se présenter sous forme entièrement ou partiellement saturée, un hydroxy, un alcoxy inférieur, un alcanoyloxy inférieur, un acétoacétoxy, un amino- ou benzyloxycarbonylamino-alkanoyloxy inférieur, un arylalcanoyloxy inférieur, où l'aryle a de 6 à 10 atomes de carbone, un alcoxy inférieur-carbonyloxy, un mono- ou dialkyl inférieur-aminocarbonyloxy, un aryloxycarbonyloxy, où l'aryle a de 6 à 10 atomes de carbone, un arylalcoxy inférieur-carbonyloxy dans lequel l'aryle a de 6 à 12 atomes de carbone, un sulfonyloxy, un alkyl inférieur-sulfonyloxy, un phénylsulfonyloxy, un 2- ou 4-toluènesulfonyloxy ou un 1- ou 2-naphtylsulfonyloxy, un amino, un mono- ou un dialkyl inférieur-amino, un N-alcoxy inférieur-N-alkyl inférieur amino, un mono- ou un di-(phényl- ou naphtylalkyl inférieur)amino, un alcanoyl inférieur-amino, un carboxy, un carboxy estérifié choisi parmi alcoxy inférieur-carbonyle, aryloxycarbonyle, où l'aryle a de 6 à 10 atomes de carbone, un arylalcoxy inférieur-carbonyle, où l'aryle à de 6 à 12 atomes de carbone, un alcanoyle inférieur, un alkyl inférieur-sulfonyle, un hydroxy-alcoxy inférieur-phosphoryle et un dialcoxy inférieur-phosphoryle, un carbamoyle, par un ou deux radicaux choisis parmi les radicaux alkyle inférieur, parmi carboxyalkyle inférieur ou alcoxy inférieur-carbonyl-alkyle inférieur, dialkyl inférieur-aminoalkyle inférieur, aminocarboxyalkyle inférieur, hydroxyalkyle inférieur, et dialcoxy inférieur-alkyle inférieur ou un radical choisi parmi les groupements éthylène, triméthylène, tétraméthylène et pentaméthylène, où un atome de carbone peut être remplacé par un atome d'azote, un atome d'oxygène, un atome de soufre ou un atome de soufre mono- ou disubstitué par de l'oxygène et qui peut être insaturé, un carbamoyle substitué, ou encore un sulfamoyle, un phosphono, un benzofuranyle, un oxo et/ou un cyano et est non ramifié ou ramifié,
R₄ représente un hydrogène,
R₅ représente un hydroxy et
R₆ représente un hydrogène,
ou R₅ et R₆ représentent ensemble un oxo, et
R₇ représente
-un alkyle inférieur,
-un cycloalkylalkyle inférieur, où cycloalkyle a de 3 à 7 atomes de carbone et est non substitué ou est mono- ou jusqu'à trisubstitué par un alkyle inférieur, un halogènoalkyle inférieur, un hydroxy, un alcoxy inférieur, un carbamoylalcoxy inférieur, un N-alkyl inférieur-carbamoylalcoxy inférieur ou un N,N-dialkyl inférieur-carbamoylalcoxy inférieur, un amino, mono- ou dialkyl inférieur-amino, un halogène, un carboxy, un alcoxy inférieur-carbonyle, un phényl-, un naphtyl- ou un fluorénylalcoxy inférieur-carbonyle, un alcanoyle inférieur, un alkyl inférieur-sulfonyle, un phosphono, un hydroxy-alcoxy inférieur-phosphoryle ou un di-alcoxy inférieur-phosphoryle, un carbamoyle, un mono- ou di-alkyl inférieur-aminocarbamoyle, un sulfamoyle, un mono- ou dialkyl inférieur-sulfamoyle, un nitro et/ou un cyano et est lié à un alkyle inférieur,
- un bicycloalkyl -alkyle inférieur, où bicycloalkyle comprend de 5 - 10 atomes de carbone,
- un tricycloalkyl -alkyle inférieur, où tricycloalkyle contient de 8 à 10 atomes de carbone,
- un arylalkyle inférieur dans lequel l'aryle est choisi parmi les groupements phényle, indényle, indanyle, naphtyle, antrhyle, phénantryle, acénaphtyle et fluorényle, qui est non substitué ou peut être mono- ou jusqu'à trisubstitué par un alkyle inférieur, un halogènealkyle inférieur, un phényle, un 1- ou 2-naphtyle, un hydroxy, un alcoxy inférieur, un carbamoylalcoxy inférieur, un N-alkyl inférieur-carbamoylalcoxy inférieur, un N,N-dialkyl inférieur-carbamoylalcoxy inférieur, un amino, un mono- ou di-alkyl inférieur-amino, un alcanoyl inférieur-amino, un halogène, un carboxy, un alcoxy inférieur-carbonyle, un benzyl-, un naphtyl- ou un fluorényl-alcoxy inférieur-carbonyle, un alcanoyle inférieur, un sulfo, un alkyl inférieur-sulfonyle, un phosphono, un hydroxy-alkyloxy inférieur-phosphoryle, un dialcoxy inférieur-phosphoryle, un carbamoyle, un mono- ou un dialkyl inférieur-carbamoyle, un sulfamoyle, un mono- ou un dialkyl inférieur sulfamoyle, un nitro et/ou un cyano, où phényle peut être présent jusqu'à trois exemplaires, et avec un alkyle inférieur, qui est non substitué ou substitué par un alkyle inférieur, hétérocyclyle choisi parmi les groupements pyrrolyle, furyle, thiényle, imidazolyle, pyrazolyle, oxazolyle, thiazolyle, pyridyle, pyrazinyle, pyrimidinyle, indolyle, quinoléyle, isoquinoléyle, quinoxalinyle, β-carbolinyle et un dérivé benzocondensé, cyclopenta-, cyclohexa-, ou cyclohepta-condensé de ces radicaux , qui peuvent également être entièrment ou partielement saturés , un hydroxy, un alcoxy inférieur, un alcanoyloxy inférieur, un acétoacétoxy, un amino- ou un benzyloxycarbonylamino-alcanoyloxy inférieur, un arylalcanoyloxy inférieur, où l'aryle a de 6 à 10 atomes de carbone, un alcoxy inférieur-carbonyloxy, un mono- ou di-alkyl inférieur-aminocarbonyloxy, un aryloxycarbonyloxy, où l'aryle a de 6 à 10 atomes de carbone, un arylalcoxy inférieur-carbonyloxy, où l'aryle a de 6 à 12 atomes de carbone, un sulfonyloxy, un alkyl inférieur-sulfonyloxy, un phénylsulfonyloxy, un 2- ou 4-toluènesulfonyloxy, un 1- ou 2-naphtylsulfonyloxy, un amino, un mono- ou dialkyl inférieur-amino, un N-alcoxy inférieur-N-alkyl inférieur-amino, un mono- ou un di-(phényl- ou naph thyl-alkyl inférieur)amino, un alcanoyl inférieur-amino, un carboxy, un carboxy estérifié choisi parmi alcoxy inférieur-carbonyle, aryloxycarbonyle, où l'aryle a de 6 à 10 atomes de carbone, un arylalcoxy inférieur-carbonyle, où l'aryle a de 6 à 12 atomes de carbone, un alcanoyle inférieur, un alkyl inférieur-sulfonyle, un hydroxy-alcoxy inférieur-phosphoryle et un dialcoxy inférieur-phosphoryle, un carbamoyle, un carbamoyle substitué par un ou deux radicaux choisis parmi alkyle inférieur, parmi carboxyalkyle inférieur, parmi alcoxy inférieur carbonyl-alkyle inférieur, parmi dialkyl inférieur-aminoalkyle inférieur, parmi aminocarboxyalkyle inférieur, parmi hydroxyalkyle inférieur et parmi dialcoxy inférieur-alkyle inférieur ou un radical choisi parmi l'éthylène, le triméthylène, le tétraméthylène, et le pentaméthylène, où un atome de carbone peut être remplacé par un atome d'azote, un atome d'oxygène, un atome de soufre ou un atome de soufre mono- ou disubstitué par de l'oxygène et peut être insaturé, un carbamoyle substitué, ou encore un sulfamoyle, un phosphono, un benzofuranyle, un oxo (qui n'est pas lié à l'atome de carbone qui est relié à l'atome d'azote liant le radical R₇) et/ou un cyano et est non ramifié ou ramifié,
-un héterocyclylalkyle inférieur, où l'héterocyclyle est de préférence choisi parmi les radicaux pyrrolyle, furyle, thiényle, imidazolyle, pyrazolyle, oxazolyle, thiazolyle, pyridyle, pyrazinyle, pyrimidinyle, indolyle, quinoléyle, isoquinoléyle, quinoxalinyle, β-carbolinyle et un dérivé benzocondensé, cyclopenta-, cyclohexa- ou cyclohepta- condensé de ces radicaux, qui peuvent également être partiellement ou entièrement saturés, par exemple un 4-pyrrolidinylméthyle, 1-imidazolylméthyle, 2-pyridylméthyle, 3-pyridylméthyle, quinoléine-2-ylméthyle ou indol-2-ylméthyle,
- un hydroxyalkyle inférieur,
- un alcoxy inférieur-alkyle inférieur,
- un phénoxyalkyle inférieur ou un nitrophénoxyalkyle inférieur,
- un naphtoxyalkyle inférieur,
- un alcanoyloxy inférieur-alkyle inférieur,
- un acétoacétoxyalkyle inférieur,
- un arylmercaptoalkyle inférieur, où aryle a de 6 à 10 atomes de carbone,
- un aminoalkyle inférieur,
- un mono- ou dialkyl inférieur-aminoalkyle inférieur,
- un phényl- ou naphtylaminoalkyle inférieur,
- un alkanoyl inférieur-amino-alkyle inférieur,
- un pipérazinylcarbonylalkyle inférieur substitué sur l'atome d'azote par un alkyle inférieur,
- un alcoxy inférieur-carbonylamino-alkyle inférieur,
- un phénylalcoxy inférieur-carbonylamino-alkyle inférieur,
- un aminocarbonylamino-alkyle inférieur,
- un N-phénylalkyl inférieur-N-alkyl inférieur-aminocarbonylamino-alkyle inférieur,
- un halogènoalkyle inférieur,
- un carboxyalkyle inférieur,
- un alcoxy inférieur-carbonylalkyle inférieur,
- un 2-halogèncalcoxy inférieur- carbonyl-alkyle inférieur,
- un phényl- ou un naphtylalkyl inférieur-oxycarbonyl -alkyle inférieur,
- un hétérocyclylalcoxy inférieur-carbonyl-alkyle inférieur, où l'hétérocyclyle est choisi parmi les radicaux pyrrolyle, furyle, thiényle, imidazolyle, pyrazolyle, oxazolyle, thiazolyle, pyridyle, pyrazinyle, pyrimidinyle, indolyle, quinoléyle, isoquinoléyle, quinoxalinyle, β-carbolinyle et un dérivé benzocondensé, cyclopenta-, cyclohexa- ou cyclohepta- condensé de ces radicaux, où les radicaux mentionnés peuvent également être entièrement ou partiellement saturés,
- un alkyl inférieur-sulfonylalkyle inférieur,
- un arylsulfonylalkyle inférieur, où l'aryle a de 6 à 10 atomes de carbone,
- un carbamoylalkyle inférieur,
- un alkyl inférieur-carbamoylalkyle inférieur,
- un dialkyl inférieur-carbamoylalkyle inférieur,
- un hydroxyalkyl inférieur-carbamoyl- ou di-(hydroxy-alkyl inférieur) carbamoyl-alkyle inférieur,
- un N-alcoxy inférieur-alcoxy inférieur-alkyl inférieur-carbamoyl-alkyle inférieur,
- un carboxyalkyl inférieur-carbamoyl- ou di-(carboxy-alkyl inférieur) carbamoyl-alkyle inférieur,
- un carbamoylalkyle inférieur substitué sur l'atome d'azote par un radical choisi parmi l'éthylène, le triméthylène, le tétraméthylène et le pentaméthylène, où un atome de carbone peut être remplacé par un atome d'azote, un atome d'oxygène, un atome de soufre ou un atome de soufre mono- ou disubstitué par de l'oxygène, un carbamoylalkyle inférieur substitué sur l'atome d'azote, le radical ainsi formé pouvant également être entièrement ou partiellement insaturé,
- un N-alkyl inférieur-N-hétérocyclylalkyl inférieur-carbamoylalkyle inférieur, où l'hétérocyclyle est choisi parmi les radicaux pyrrolyle, furyle, thiényle, imidazolyle, pyrazolyle, oxazolyle, thiazolyle, pyridyle, pyrazinyle, pyrimidinyle, indolyle, quinoléyle, isoquinoléyle, quinoxalinyle, β-carbolinyle et un dérivé benzocondensé, cyclopenta-, cyclohexa- ou cyclohepta-condensé de ces radicaux, qui peuvent également être entièrement ou partiellement saturés,
- un sulfamoylalkyle inférieur,
- un N-(phényl- ou naphtylalkyl inférieur)sulfamoyl-alkyle inférieur,
- un sulfamoylalkyle inférieur substitué sur l'atome d'azote par un radical choisi parmi l'éthylène, le triméthylène, le tétraméthylène et le pentaméthylène, où un atome de carbone peut être remplacé par un atome d'azote, un atome d'azote substitué par un alkyle inférieur, un atome d'oxygène, un atome de soufre ou un atome de soufre mono- ou disubstitué par de l'oxygène, le radical ainsi formé pouvant également être entièrement ou partiellement insaturé,
- un oxoalkyle inférieur (où oxo n'est pas lié à l'atome de carbone qui est relié à l'atome d'azote liant R₇),
- un cyanoalkyle inférieur,
- un hydroxy-carboxy-alkyle inférieur,
- un α-naphtoxy-carboxy-alkyle inférieur,
- un hydroxy-alcoxy inférieur-carbonyl-alkyle inférieur,
- un α-naphtoxy-alcoxy inférieur-carbonylalkyle inférieur,
- un alkyl inférieur-carbonyl-halogèno-alkyle inférieur,
- un α-naphtoxyéthoxycarbonyl-alkyle inférieur,
- un α-naphtoxy-benzyloxycarbonyl-alkyle inférieur,
- un hydroxy estérifié-alcoxy inférieur-carbonyl-alkyle inférieur, où le groupe hydroxy est estérifié par un alcanoyle inférieur, un cycloalkylalcanoyle inférieur, où cycloalkyle a de 3 à 7 atomes de carbone, un bicycloalkylalcanoyle inférieur, où le bicycloalkyle contient de 5 à 10 atomes de carbone, un tricycloalkylalcanoyle inférieur, où le tricycloalkyle contient de 8 à 10 atomes de carbone, un arylalcannoyle inférieur, où l'aryle a de 6 à 12 atomes de carbone et est non substitué ou peut être mono- ou jusqu'à trisubstitué par un alkyle inférieur, un hydroxy, un alcoxy inférieur, un carbamoylalcoxy inférieur, un N-alkyl inférieur-carbamoylalcoxy inférieur ou un N,N-dialkyl inférieur-carbamoylalcoxy inférieur, un amino, un mono- ou di-alkyl inférieur-amino, un halogène, un carboxy, un alcoxy inférieur-carbonyle, un phényl-, naphtyl- ou fluorénylalcoxy inférieur-carbonyle, un alcanoyle inférieur, un alkyl inférieur-sulfonyle, un phosphono, un hydroxyalcoxy inférieur-phosphoryle, un dialcoxy inférieur-phosphoryle, un carbamoyle, un sulfamoyle, un nitro et/ou un cyano, un alcoxy inférieur-carbonyle, un 2-halogèno alcoxy inférieur-carbonyle, ou un phényl-ou fluorényl-alcoxy inférieur,
- un dihydroxy-carboxy-alkyle inférieur,
- un dihydroxy-alcoxy inférieur-carbonyl-alkyle inférieur,
- un dihydroxy-alcoxy inférieur-carbonyl-alkyle inférieur estérifié par un alcanoyle inférieur, un alcoxy inférieur-carbonyle, un phényl- ou un fluorénylalcoxy inférieur-carbonyle, un alkyl inférieur-sulfonyle ou un toluènesulfonyle,
- un α-naphtoxy-dialkyl inférieur-amino-alkyle inférieur,
- un α-naphtoxy-carbamoyl-alkyle inférieur,
- un α-naphtoxy-oxo-alkyle inférieur (où oxo n'est pas lié à l'atome de carbone qui est relié à l'atome d'azote liant R₇), ou
- un α-naphtoxy-cyanoalkyle inférieur,
ainsi que des sels des composés mentionnés, dans la mesure où des groupes salificateurs sont présents, et où dans les définitions données ci-dessus l'expression "inférieur" signifie que le radical en question comporte jusqu'à 7 atomes de carbone compris.

5. Procédé selon la revendication 1 de préparation d'un composé de formule I, dans laquelle R₁ représente un alcoxy inférieur-carbonyle, un phényl-alcoxy inférieur-carbonyle, le radical monovalent, lié par l'intermédiaire du groupe carboxy, d'un acide aminé aliphatique choisi parmi la valine, l'alanine, la leucine et l'isoleucine ou le radical, lié par l'intermédiaire du groupe carboxy, d'un acide aminé aliphatique acylé sur l'atome d'azote de l'amino par l'un des radicaux phénylalcanoyle inférieur, morpholinylalcanoyle inférieur, thiomorpholinylalcanoyle inférieur, S,S-dioxothiomorpholinylalcanoyle inférieur, pyridylalcanoyle inférieur, alcoxy inférieur-carbonyle ou phénylalcoxy inférieur-carbonyle, et tel que défini ci-dessus, tous les acides aminés mentionnés se présentant sous forme D, D,L ou L, R₂ représente un hydrogène, R₃ représente un phénylalkyle inférieur, R₄ représente un hydrogène, R₅ représente un hydroxy, R₆ représente un hydrogène, R₇ représente un alkyle inférieur, un cyclohexylalkyle inférieur ou un phénylalkyle inférieur, R₈ représente un hydrogène et R₉ représente l'un des radicaux mentionnés pour R₁ et les atomes de carbone asymétriques portant les radicaux R₃ et R₅ se présentant sous la configuration S, ainsi que des sels pharmacologiquement acceptables de ces composés, où dans les définitions données ci-dessus l'expression "inférieur" signifie que le radical en question comporte jusqu'à 7 atomes de carbone compris; caractérisé en ce qu'on utilise des produits de départ substitués de façon correspondante.

6. Procédé selon la revendication 1 de préparation d'un composé de formule I, dans laquelle R₁ représente un tert-butoxycarbonyle, un benzyloxycarbonyle, le radical monovalent de l'acide aminé valine, lié par l'intermédiaire du groupe carboxy, ou le radical, lié par l'intermédiaire du groupe carboxy, de l'alanine acylée sur l'atome d'azote de l'amino par l'un des radicaux phénylacétyle, 3-pyridylacétyle, morpholinocarbonyle, thiomorpholinocarbonyle, tert-butoxycarbonyle ou benzyloxycarbonyle, R₂ représente un hydrogène, R₃ un benzyle, R₄ un hydrogène, R₅ un hydroxy, R₆ un hydrogène, R₇ un isobutyle, un cyclohexylméthyle ou un benzyle, R₈ représente un hydrogène et R₉ représente l'un des radicaux mentionnés pour R₁ et les atomes de carbone asymétriques portant les radicaux R₃ et R₅ se présentent sous la configuration S, ainsi que des sels pharmacologiquement acceptables de ces composés; caractérisé en ce qu'on utilise des produits de départ substitués de façon correspondante.

7. Procédé selon la revendication 1 de préparation d'un composé de formule I, dans laquelle R₁ et R₉ représentent chacun le radical monovalent, lié par l'intermédiaire du groupe carboxyle, acylé sur l'atome d'azote de l'amino par un benzyloxycarbonyle, de l'acide aminé (L)-valine, R₂ et R₈ représentent un hydrogène, R₃ représente un benzyle, R₄ représente un hydrogène, R₅ représente un hydroxy, R₆ représente un hydrogène et R₇ représente un benzyle et les atomes de carbone asymétriques portant les radicaux R₃ et R₅ se présentent sous la configuration S, ainsi que des sels pharmacologiquement acceptables de ces composés; caractérisé en ce qu'on utilise des produits de départ substitués de façon correspondante.

8. Procédé selon la revendication 1 de préparation d'un composé de formule I, dans laquelle R₁ et R₉ représentent chacun le radical monovalent de l'acide aminé (L)-valine, lié par l'intermédiaire du groupe carboxy, acylé sur l'atome d'azote de l'amino par un 4-thiomorpholinocarbonyle, R₂ et R₈ représentent un hydrogène, R₃ représente un benzyle, R₄ représente un hydrogène, R₅ représente un hydroxy, R₆ représente un hydrogène et R₇ représente un isobutyle et les atomes de carbone asymétriques portant les radicaux R₃ et R₅ se présentent sous la configuration S, ainsi que des sels pharmacologiquement acceptables de ce composé; caractérisé en ce qu'on utilise des produits de départ substitués de façon correspondante.

9. Procédé selon la revendication 1 de préparation d'un composé de formule I, choisi parmi les composés désignés par
Boc-[Phe^{NN}Phe]-Boc,
Boc-(L)-Val-[Phe^{NN}Phe]←((L)-Val-Boc),
Boc-[Phe^{NN}Cha]-Boc,
H-(L)-Val-[Phe^{NN}Phe]←((L)-Val-H,
N-thiomorpholinocarbonyl-(L)-Val-[Phe^{NN}Phe]←(N-thio-morpholinocarbonyl-(L)-Val),
N-morpholinocarbonyl-(L)-Val[Phe^{NN}Phe]←(N-morpholino-carbonyl-(L)-Val),
Phénylacétyl-(L)-Val-[Phe^{NN}Phe]←(N-phénylacétyl-(L)-Val),
N-(3-pyridylacétyl)-(L)-Val-[Phe^{NN}Phe]←(N-(3-pyridylacétyl)-(L)-Val),
Boc-(L)-Val-[Phe^{NN}Cha]←((L)-Val)-Boc,
Z-(L)-Val-[Phe^{NN}Cha]←((L)-Val)-Z,
Boc-[Phe^{NN}Leu]-Boc,
Z-(L)-Val-[Phe^{NN}Leu]←((L)-Val)-Z, H-(L)-Val-[Phe^{NN}Cha]¬((L)-Val)-H et
N-(3-pyridylacétyl)-(L)-Val-[Phe^{NN}Leu]←(N-(3-pyridyl-acétyl)-(L)-Val), ou d'un de ses sels;
où Boc représente un tert-butoxycarbonyle, Z représente un benzyloxycarbonyle, le radical -[Phe^{NN}Phe]- représente le radical bivalent de 3(S)-amino-4-phényl-1-(N-benzylhydrazino)-butan-2(S)-ol et a la formule 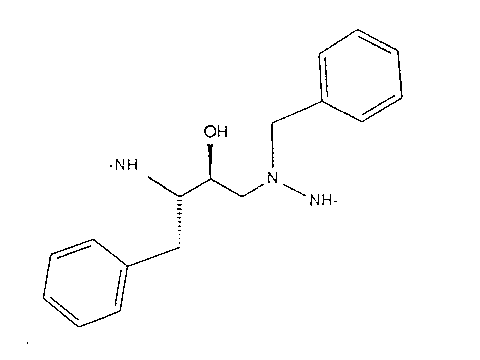 le radical -[Phe^{NN}Cha] représente le radical bivalent du 3(S)-amino-4-phényl-1-(N-cyclohexylméthylhydra-zino)-butan-2(S)-ol et a la formule 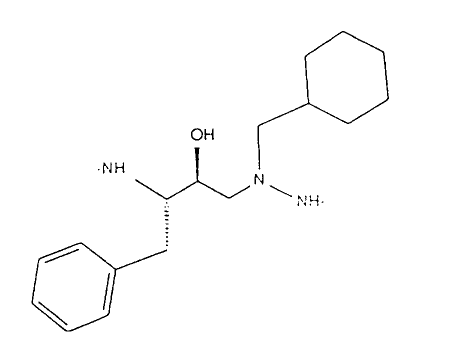 le radical -[Phe^{NN}Leu]- représente le radical bivalent du 3(S)-amino-4-phényl-1-(N-isobutylhydrazino)-butan-2(S)-ol et a la formule 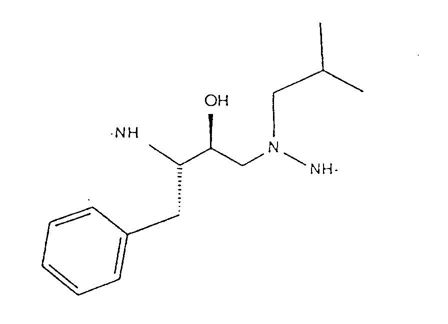 et une flèche "←" représente l'inversion de la direction de liaison en s'écartant de la nomenclature habituelle des peptides, dans laquelle l'extrémité amino se situe à gauche et l'extrémité carboxy se situe à droite; caractérisé en ce qu'on utilise des produits de départ substitués de façon correspondante.

10. Procédé selon la revendication 1 de préparation d'un composé de formule I, choisi parmi les composés ayant les désignations
Z-(L)-Val-[(p-F)Phe^{NN}(p-F)Phe]←(N-(N-(2-pyridylméthyl)-N-méthyl-aminocarbonyl)-(L)-Val,
Z-(L)-Val-[(p-F)Phe^{NN}(p-F)Phe]←(N-(2(R,S)-carbamoyl-3-phényl-propionyl)(L)-Val),
Acétyl-(L)-Val-[Phe^{NN}Cha]←(N-acétyl-(L)-Val),
Acétyl-Ile-[Phe^{NN}Cha]←(N-acétyl-Ile),
N-(2-pyridylmethyl)-N-méthyl-aminocarbonyl-(L)-Val-[Phe^{NN}(p-F)-Phe]←(N-(N-(2-pyridylméthyl)-N-méthyl-aminocarbonyl)-(L)-Val),
Z-(L)-Val-[Phe^{NN}(p-F)Phe]←((L)-Val)-Z,
Z-(L)-Val-[Phe^{NN}(p-CN)Phe]←((L)-Val)-Z,
Z-(L)-Ile-[Phe^{NN}Leu]←((L)-Ile)-Z,
Isobutoxycarbonyl-(L)-Val-[Phe^{NN}Leu]←(N-isobutoxycarbonyl-(L)-Val),
Acétyl-Val-[Phe^{NN}Leu]←(N-(2(R,S)-carbamoyl-3-phenyl-propionyl)-Val),
N-trifluoroacétyl-[Phe^{NN}Leu]←(N-(2-(R,S)-carbamoyl-3-phényl-propionyl)-(L)-Val),
Z-(L)-Val-[Phe^{NN}Nle]←(N-(2(R,S)-(N-(2-morpholinoéthyl)-carbamoyl)-3-méthyl)-butyryl),
Z-(L)-Val-[Phe^{NN}Nle]←(N-(2(R,S)-(N-(2-pyridylmethyl)-carbamoyl)-3-méthyl)-butyryl),
Méthoxycarbonyl-(L)-Val-[Phe^{NN}Leu]←(N-méthoxycarbonyl-(L)-Val),
Méthoxycarbonyl-(L)-Val-[Phe^{NN}(p-F)Phe]←(N-méthoxy-carbonyl-(L)-Val) et
Méthoxycarbonyl-(L)-Val-[Phe^{NN}(p-CN)Phe]←(N-méthoxy-carbonyl-(L)-Val),
ou d'un de ses sels, dans lequel Boc représente un tert-butoxycarbonyle, Z représente un benzyloxycarbonyle, le radical -[Phe^{NN}Phe]- représente le radical bivalent du 3(S)-amino-4-phényl-1-(N-benzylhydrazino)-butan-2(S)-ol et a la formule 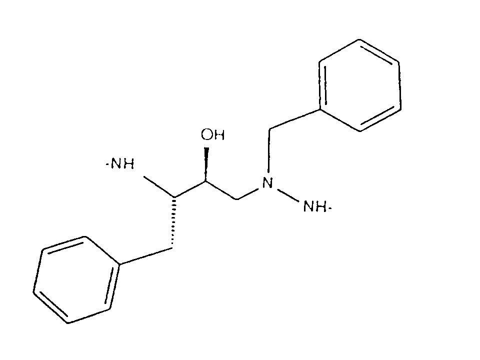 le radical -[Phe^{NN}Cha] est le radical bivalent du 3(S)-amino-4-phényl-1-(N-cyclohexylméthyl-hydrazino)-butan-2(S)ol et a la formule 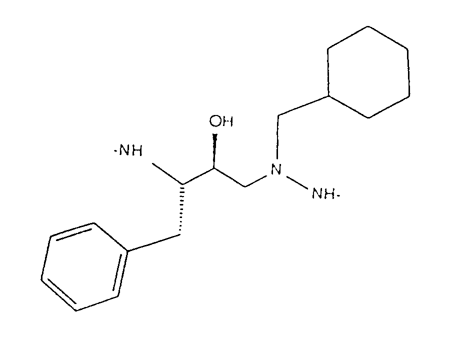 le radical -[Phe^{NN}Leu] est le radical bivalent du 3(S)-amino-4-phényl-1-(N-isobutylhydrazino)-butan-2(S)-ol et a la formule 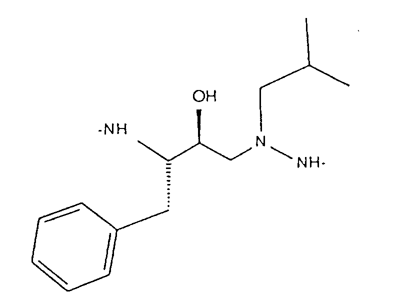 le radical ayant la désignation -[Phe^{NN}Nle] représente le radical du 3(S)-amino-4-phényl-1-(N-n-butyl-hydrazino)-butan-2(S)-ol et a la formule 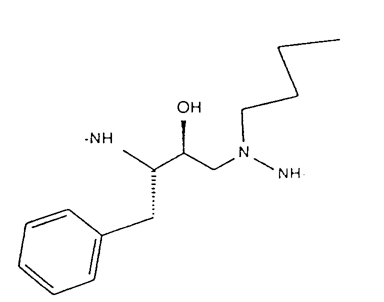 le radical ayant la désignation -[Phe^{NN}(p-F)Phe] représente le radical bivalent du 3(S)-amino-4-phényl-1-(N-(p-fluorophénylméthyl)-hydrazino)-butan-2(S)-ol et a la formule 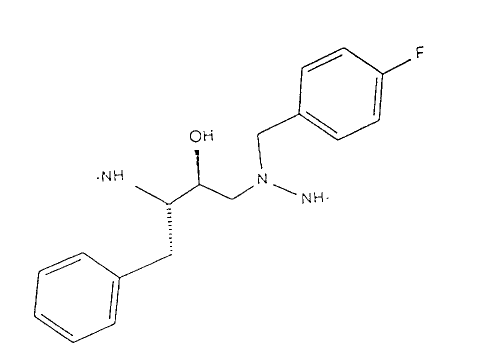 le radical ayant la désignation -[(p-F)Phe^{NN}(p-F)Phe] représente le radical bivalent du 3(S)-amino-4-(p-fluorophényl)-1-(N-(p-fluorophénylméthyl)-hydrazino)-butan-2(S)-ol et a la formule 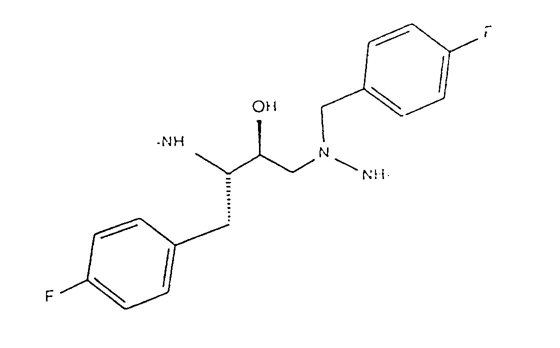 le radical ayant la désignation -[Phe^{NN}(p-CN)Phe] représente le radical bivalent du 3(S)-amino-4-phényl-1-(N-(p-cyanophénylméthyl)-hydrazino)-butan-(S)-ol et a la formule 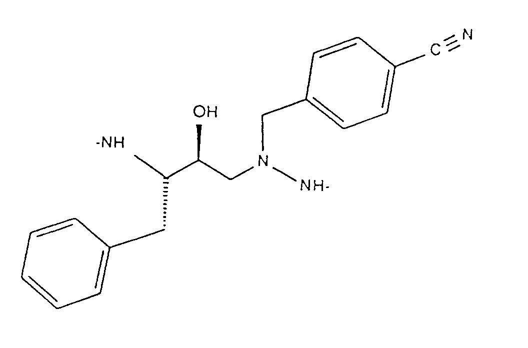 et une flèche "←" représente l'inversion de la direction de liaison en s'écartant de la nomenclature habituelle des peptides, où l'extrémité amino se situe à gauche et l'extrémité carboxy se situe à droite; caractérisé en ce qu'on utilise des produits de départ substitués de façon correspondante.
